# EUROPEAN PATENT SPECIFICATION

(11) **EP 2 909 335 B1**
(45) Date of publication and mention of the grant of the patent: **30.05.2018**
(21) Application number: 13779570.4
(22) Date of filing: 17.10.2013
(51) Int. Cl.: C12Q 1/68

(54) **PROGNOSTIC OF DIET IMPACT ON OBESITY-RELATED CO-MORBIDITIES**
PROGNOSE DER AUSWIRKUNG EINER DIÄT AUF ADIPOSITASBEDINGTE KOMORBIDITÄTEN
PRONOSTIC DE L'IMPACT D'UN RÉGIME ALIMENTAIRE SUR DES COMORBIDITÉS LIÉES À L'OBÉSITÉ

(30) Priority: 17.10.2012 EP 12306285
(43) Date of publication of application: 26.08.2015
(73) Proprietor: Institut National de la Recherche Agronomique, 75007 Paris (FR); Université Pierre et Marie Curie (Paris 6), 75005 Paris (FR); Institut De Recherche Pour Le Développement (IRD), 13000 Marseille (FR); Assistance Publique - Hôpitaux de Paris, 75004 Paris (FR)
(72) Inventor: EHRLICH, Stanislav, 91400 Orsay (FR); DORE, Joël, 94400 Vitry Sur Seine (FR); LE CHATELIER (EPOUSE JANNIERE), Emmanuelle, 78114 Magny Les Hameaux (FR); CLEMENT (EPOUSE LAUSCH), Karine, 75003 Paris (FR); ZUCKER, Jean-Daniel, 75011 Paris (FR)
(74) Representative: Regimbeau
(86) International application number: PCT/EP2013/071764
(87) International publication number: WO 2014/060537

(56) References cited:
- WO-A2-2008/076696
- WO-A2-2011/107482
- QIN JUNJIE ET AL: "A human gut microbial gene catalogue established by metagenomic sequencing", NATURE, NATURE PUBLISHING GROUP, UNITED KINGDOM, vol. 464, no. 7285, 4 March 2010 (2010-03-04), pages 59-65, XP008132800, ISSN: 1476-4687, DOI: 10.1038/NATURE08821
- PETER J. TURNBAUGH ET AL: "A core gut microbiome in obese and lean twins", NATURE, vol. 457, no. 7228, 22 January 2009 (2009-01-22), pages 480-484, XP055006664, ISSN: 0028-0836, DOI: 10.1038/nature07540
- TURNBAUGH PETER J ET AL: "An obesity-associated gut microbiome with increased capacity for energy harvest", NATURE: INTERNATIONAL WEEKLY JOURNAL OF SCIENCE, NATURE PUBLISHING GROUP, UNITED KINGDOM, vol. 444, no. 7122, 1 December 2006 (2006-12-01), pages 1027-1031, XP002492885, ISSN: 0028-0836, DOI: 10.1038/NATURE05414
- P. J. TURNBAUGH ET AL: "The core gut microbiome, energy balance and obesity", THE JOURNAL OF PHYSIOLOGY, vol. 587, no. 17, 2 June 2009 (2009-06-02) , pages 4153-4158, XP055095908, ISSN: 0022-3751, DOI: 10.1113/jphysiol.2009.174136
- VACHARAKSA A ET AL: "Gut Microbiota: Metagenomics to Study Complex Ecology", CURRENT BIOLOGY, CURRENT SCIENCE, GB, vol. 20, no. 13, 13 July 2010 (2010-07-13) , pages R569-R571, XP027189188, ISSN: 0960-9822 [retrieved on 2010-07-12]

### SEQUENCE LISTING

<110> INSTITUT NATIONAL DE LA RECHERCHE AGRONOMIQUE
   UNIVERSITE PIERRE ET MARIE CURIE UPMC
   INSTITUT DE RECHERCHE POUR LE DEVELOPPEMENT IRD
   LE CHATELIER, Emmanuelle
   EHRLICH, Stanislav
   DORE, Joel
   CLEMENT, Karine
   zUCKER, Jean Daniel
<120> PROGNOSTIC OF DIET IMPACT ON OBESITY-RELATED CO-MORBIDITIES.
<130> 364100D31429
<150> EP 12306285.3
   <151> 2012-10-17
<160> 450
<170> PatentIn version 3.5
<210> 1
   <211> 1713
   <212> DNA
   <213> Unknown
<220>
   <223> Unknown gut bacteria
<400> 1
   atgacaccat cagaatggag tttccgtatg aaacacacgt tgaaaatact gatcccgatc        60
   ctgctgatcc ttgcgctgct gatcggcgcg tgctggtttt tcctcatcgc ccgccgcgat       120
   ctgacggaga gcgtgtttac ctactggggc aatcattttt ataacaacgg ccgctacggc       180
   cgcgccatca cctgctacaa gctcgccatg cacttcgccc cgaaggacgc cgagcttgcc       240
   atctggctct cgaacgctta caaacggagc ggcaactata cgaaggccga atataccctc       300
   gtcaacgcca tcacgcagag ccccgacgcg gccgacctct acatcgccct gtccaagacc       360
   tacgtggagc aggacaacct ccttgacgcc gagaccatgc tctgccgcat caccaacgac       420
   gccgtccgca cccagatcga cgccctgcgc cccgccgcac ccgtgatcga gccggagagc       480
   ggcacctata cggaatacat cgatgtcacc atcaccggga ccgagggcac cgtctacgcc       540
   gtctgcaaca gcgatttccc ggccgaggag acggacatct ataccggccc catctccctg       600
   accgcgggcg agagcaagat cgtcgcgctg agcgtggcgg aaaacggcct cgtgagcgac       660
   gcggtctacg ccggctatac cgtcggcagc gtcgtggagc cggtcacgct ggcggacgcg       720
   gggctggaca gctatgtccg tgagctgctc ggcaagacgg ctggcagcac gctgatgacg       780
   gacgagctct gggccatcga ggagctggac ctgcccgata ccgtcgcctc gctggaggat       840
   ctgccgtact tcaccggcct gcgcacgctc gcgctgcacc atagcagcgc gtcgatggac       900
   ctgtcggtcc tcgcccagct gccgacgctg cgcacgctgg acctgtccgg ctgcacgctc       960
   tcgtcggcgg cgatgagcac catcgtctca ctgccggagc tgacgagcct gaacctctcc      1020
   ggctgcgccg tcatcgacat caacgccctg attggcctgc agaagctgga attcctcgac      1080
   ctttccaaca acaccatctc cgacctcacc gccctgtccg ccctgcaggc gctcaaggag      1140
   ctgcatctga ccaacaaccc catcacctcg ctcgccaacc tcaaaaactg cacgcagctg      1200
   gagatcctct acgcgaacca gtgctccatc acgcgcatcg cgggcctggc cgaccatacg      1260
   gcgctcaagg agctgtatct ggccaacaac cagatcgcgg atatctccgt cctcgcctcc      1320
   tgcacggcgc tgcagacgct ggacctgtcg ttcaacgcgg tcacggatat ctccatcgtt      1380
   tccgagctca ggcagctggt cgacctcaac gtgagcaaca accagatcac cgtcttcccg      1440
   gctgtggacg ccgatacgcc gctctggcac gtcgatatca gccacaacca gatcgaggat      1500
   ctgaccggcc ttgcgggcaa cctctccgtc aacttcatca acgccgacta taataagatc      1560
   aagtccatcg caaagctcga ggcgtgcgtc atgctcgtta agatgaacct ctgggataac      1620
   cccgtgaaca ccgacgaggt caagaagctg caggacgtcg gcatcatcat caactataac      1680
   cccgaataca aggaaacgga cacagaagcc tga                                   1713
<210> 2
   <211> 1746
   <212> DNA
   <213> Unknown
<220>
   <223> Unknown gut bacteria
<400> 2
   atgatcaaac gtttggcggc ctgtatccgg gaatacaaaa ggccgacgat cctgaccccc 60
   gtgtgcatgg tgggcgaggt cgtatgcgag tgcacgatcc cgctgctgac ggcggatctg 120
   atcaacgcga tccagaacgg ctgcacgatg cagaccattc tgtccttcgg actcaagctg 180
   ttcgtcatcg ccatgctgtc gctggggttc ggatcgttgt ccggctggtt cgcggcagag 240
   gccgcggcgg gctttgcgaa gaatctgcgg catgatctgt attatcaggt gcagggcttt 300
   tcctttgcga acatcgaccg gttttcgacc tcgtcgctcg tcacgcggct gacgacggac 360
   gtcacgaaca tccagaacgc gttcatgatg tgcatccgca tcgcggtgcg cgcgccgatg 420
   atgctggtgt tcgcggtggt gatgtccgtc cgcgtgggca agcagctggc gctcatcttc 480
   gccgggatcg tgccggtgct ggggttcgcg ctgtttctga tgatccggag cgcgctgccg 540
   ctgttcaagg cggtcttcaa gaaatacgac gcgctcaacc actccgtgca ggagaacgtg 600
   caggccatgc gcgtggtcaa gagcttcgtg cgcgaggact atgagacgga gaaattctcc 660
   gccgcctccg actccgtgcg gaaggatttc ctcaaggcgg agaaaatcct ggcgctcaac 720
   tcccctgcca tgcagttctg cgtgtacacg tcgatgatcc tcatctcgta tttcgcggcg 780
   aagctcatcg tcacgtcggg cggaacgtat ctgggcgtgg gaagtctgac gagcatgatc 840
   acgtattcca tgcagatcct catgagcctg atgatgctct cgatgatctt cgtgatgctg 900
   acgatggcgc aggcctccgg caagcgcatc tgcgaggtgc tcgacgagac gagcagcctg 960
   cagaacccgg cagagccggt ctatgatgtg aagaacggcg acgtggattt tgaccatgtc 1020
   aactttaagt attcggcggc ggcgaagcgc agcgcgcttt cggacgttga tttccatgtg 1080
   agatccggcc agaccgtcgg catcattggc ggcacggggt cttctaagac gagtctggtg 1140
   cagctcatct gccggctgta cgacgtgacg gacggcagcg tgcgggtcgg cggcgtggac 1200
   gtgcggcagt acgatctgga gacgctgcgc aaccaggtcg cggtcgtgct gcagaagaac 1260
   gtgctgttct ccggcacgat caaggagaat ctgcgctggg gcgacgcgaa cgcgtcggac 1320
   gaggagctgc agcgggcctg cgagctggcg tgcgcggatg aattcatcca gcagatgccg 1380
   gacaaatacg acacctatat cgagcagggc ggcacgaacg tcggcggcgg gcagaagcag 1440
   cggctgtgca tcgcgcgggc gctgctgaaa aagccgaaga ttttgatcct cgacgactcg 1500
   acctccgctg tcgacacgaa gaccgacgcg aagatccgcg cggcgctgcg ctcggagatc 1560
   ccggagacga cgaagttcat catcgcgcag cggatctcgt ccatccagaa cgcggatctc 1620
   atcctcgtgc tcgagggcgg gcagatcgac gccatgggca cgcacgagca gctgctggct 1680
   tcgaaccgga tctaccggga agtctatgaa tctcagaaca aggcgggagg tgctgacaat 1740
   ggctaa 1746
<210> 3
   <211> 1251
   <212> DNA
   <213> Unknown
<220>
   <223> Unknown gut bacteria
<400> 3
   atgaaaatca aaaatgttgt ctgcgcacca ggacgtaccg gcttctattt tgacgatcag      60
   cgcgcgatca agcgcggcgc ggggcacgac gggatgttct atatcggtca gcctgtcacc     120
   gagggctttt ccgccgtccg tcaggcaggc gaggcgatct cggtcatgct gatcctggag     180
   gacggccagg tcgcttacgg cgactgcgcg gcggtacagt attccggcgc aggcgggcgc     240
   gatccgctgt ttttggcgaa ggattttatt cctattattg agcagtacat caaaccgtgt      300
   ctggttggcc aggaagcgga caatttccgt gcgcttgccg caaggatgga agccatcgag     360
   gtcggcggca agcggctgca tacggcgatc cgctatggtg tttctcaggc gctgcttgac     420
   gccgttgcaa aggcgagcgg gcgcatgatg tgtgaggtcg tggcggatga atacggctgc     480
   acggtatcgg atcacctgat tccgatcttt acccagtccg gggacgaccg gtatgataat     540
   gcggacaaaa tgatcatcaa gggcgcgcag gtgctcccgc acgcattgat caacaacggg     600
   gagacgaagc tcggcgcgca cggcgaaaag cttcaggagt atgtggcgtg gcttcgtgac     660
   cggatcttgg cgcagcgtct ggacgagcac tatgccccgg tcctgcatat cgatgtctac     720
   ggtacgatcg gcgcagcctt cggaaatcat aactacgcag ccatggcgga ttatctggcg     780
   gtcctggagc agaccgcaaa gccgttccac ctgcggatcg aaggaccgat ggactgcgac     840
   tgtgaccgcg aaacgcagat ggaggcgctc gcggggctga ccgcggagct ggaccggcgc     900
   ggcatcgatg ttgagctggt cgctgacgaa tggtgcaata cgctcgaaga tatcaagctt     960
   ttcgccgatc acaaagccgg tcacatgatc cagatcaaaa cgccggatct cggcggcatc    1020
   aacaatacca tcgaggcagt cctctactgc aaggaaaagg gcatcggtgc atatcagggc    1080
   ggcacctgca atgagaccga ccgctctgcg caggtctgcg tgcagtgcgc catggcgaca    1140
   cagccggcac agatcctcgc aaagcccggc atgggtgtgg acgaaggcta tatgatcgtc    1200
   tataacgaga tgaaccggat cctcgcgctg cggaaggcaa aggcagaata a             1251
<210> 4
   <211> 990
   <212> DNA
   <213> Unknown
<220>
   <223> Unknown gut bacteria
<400> 4
   atgcagaagg agcaggttat catgcgcgga atcccgtctt tgatcacaga gatccgaaag      60
   aaagtcttta ccgaggtcgc ccggatggca tactccggcg actacaccga tatggaggat     120
   atccccttca agatcgtccc cggccagagc ccgctgcacc gcgaatcggt ctttcttgag     180
   cgcgccatcg cgggcgaacg cgtgcgtctt gcgatgggtc tgtccctgca gcccgtacag     240
   acgcgcacgc ttctgaccga gggcatggac caggccgcca tcgccgagca gtattacgag     300
   ccgccgctcg tcaacatcat cccctacgcc tgccacgcct gcccgacgcg gcagtaccgt     360
   gtcaacgagc tgtgtcaggg ctgtctcgcc tcctcgtgcc agcgcgtgtg cccgaagggc     420
   gcgatcaaat ttgtcaacgg caagagccgc atcgaccaga aactgtgcat caagtgcggc     480
   aaatgcgcca gatcctgtcc gtacaacgcc atcacctacc tcgagcgccc gtgtcaggcc     540
   gcctgcggca tggacgccat cggcgtcgac gaatacggca aggcctgcat cgactatgac     600
   cgctgcgtct cctgcggcca gtgccttgtg agctgcccgt tcggcgcgat cgtcgacaag     660
   agccagatct atcaggtcat ccgctccatg ctcgagggca acgaggtcgt ggccatcgtc     720
   gcgccgtcgt tcgtcggcca gttcggcaag gacgtgaccg tgccgaagtt cctcacggcc     780
   atgaagcagc tcggctttgc cgatgtggtc gaggtcgcgg tcggcgcgga tatctgcacg     840
   atcgaggaag cgcgggactt tatggaaaag gtcccggagg agcagcggtt catggcgacc     900
   tcctgctgcc cgtcgtggcg gatgatggcc agacggctgt tccccgccga atccggcaac     960
   atttccatga cctacacgcc gatggtctat                                     990
<210> 5
   <211> 1221
   <212> DNA
   <213> Unknown
<220>
   <223> Unknown gut bacteria
<400> 5
   aaaaagatca tcgcattgct gcttgcagcg atcatggtgc tcggtctggc tgcctgcgca      60
   tccaagacgg agcccgagac caccaccgaa ccggaaacca ccaccgaaac cgctcccgag     120
   accacggaag aacccgccgc tgaggagccc gcagctgaag aagagacccc ggctgaagag     180
   cccgctgccg acgctgctta ctccgtcgcc atgatcaccg actacggtga taccaccgac     240
   cagtccttca accagaccac ctacgaggcc tgcaaggagt tctgcgaggc caacggcctg     300
   cagtttaact atttcaagcc cgccggtgac tccgacgccg agcgtgtcgc catgatcgag     360
   tccgcgatcg acgagggcta caacgttgtt gtgatgcccg gctacgcttt cgcaggcgcc     420
   atcaaggaaa ctgccgacat ctatcccgag gtcaccttca ttgcgctgga cgtcagtgcc     480
   ggcgatctgg gcgatgacta caccctgccc gccaacctct actgcgctgt ctatcaggaa     540
   gagctgtgcg gctacatggc cggctacgca gctgtgaagc tccgctacac caagcgcgga     600
   ctcctgggcg gcatggctgt ccccgctgtt gtccgttacg gcttcggcta tgtgcagggc     660
   gttgacgctg ctgccaagga gctcggcatc acggtcgacc tcaagtacgc ttacggcaac     720
   cagttcgttg gcgacgctga catcaccgct gcaatggaca cctggtatca gggcggcacg     780
   gagattgtct tcgcctgcgg cggcggcatc tatacctccg cggctgaggc tgctgccaag     840
   gtcggcggca agatcatcgg cgtcgacgtt gaccaggccg gcatcatcga cggcgattac     900
   ggcgaaggca tgaccgttac ctccgcaatg aagggccttg ctcccaccgt caagcatctg     960
   ctctccgaag tcgtggccgg caactttgcc aactacggcg gcaagatcga aacgctgggc    1020
   ctcgtctccg ccaacccgga agagaactac gtccagatcc cgaccgcttc cacgcagttc    1080
   gaagacggca agttcaccca ggctgactac gaagcgctcg tcgctgccat gttcgccggc    1140
   gatgtcaccg tctccaacga catcacggct atgcccgctg tcaccaacgt caccgttgag    1200
   gacttcggca acctgaagta a                                              1221
<210> 6
   <211> 1446
   <212> DNA
   <213> Unknown
<220>
   <223> Unknown gut bacteria
<400> 6
   atgaataagc gggcgatcgt ctatgtgctg ggcgcggtgc tgctgatcgg ggccgcgctg      60
   atgctgccgc cgctgctcgt ggcgctgatc taccatgagg taagcggctg gtatttcctc     120
   tgggtgatgc tgggggcggc ggtgctcggc gcgctggccc tgcggctggg cggcggcaag     180
   cgggccgtca tgtacgcaaa agaagggctg atcgcggtgg cgctcagctg gatcgtgctt     240
   tcgctcgtcg gcgcgctgcc gttcacgctt tcggggcaga tcccgtttta tctcgacgcc     300
   gtgttcgaga tgatctccgg ctttaccacc accggctcga gcatcctgcc cgcggtggag     360
   gagctcgaca agtgcatgct gttctggcgc agcttttcgc actggatcgg cggcatgggc     420
   attctggtgt tcatgctggc catcgtccgg atggacggcg gccaggccat ccacctgctg     480
   cgggccgaga gccccgggcc gacggtctcc aagatggtgc cgcggatggc cgattcgtcc     540
   aagatcctct acgggatcta ttttggcctg acggtgctgc agatcctctt ttatctggcc     600
   ggcggcgagc cgctgcttga ctcgctctgc aatgccttcg gcacggccgg caccggcggc     660
   tttgccatcc gcaacgattc cttcgcctcc tacagcgcct acacgcagac ggtcacgacc     720
   gtcttcatgg ccctgttcgg cgtcaatttt tcgatctatt tcttcctgct gcgccggaaa     780
   ttcgacctgg cgtggaagaa tacggagctg cgctggtacg tgtcgatcat tctcggctcg     840
   acgctgctca tcacctgcaa catcatgaag ctctacggcg gcgatttcgg cttcagcctg     900
   catcacgcgg cttttgccgt ctcctccgtc atcaccacga cgggctttgg cacggagaac     960
   tttgacctgt ggccggagtt ttcccgcgtg atcctggtgc tcctcatgat cgtcggtgcg    1020
   agcgccgggt cgacgggcgg cggactgaag gtctcgcgcg tggtcatcct gatgcgcgcg    1080
   gccaaggcgg agctgcgcaa gatcctgcac ccgcacaccg tgaaggtcat caccgtcgac    1140
   ggcaagcccg tctccggcga gacggtccgg gccgtgagca cctattttat cctgtacgtg    1200
   ctcatcgctg cggcgtcggt gctgctcgtc tcgctcgacg gctacgacgg ctcgaccacg    1260
   ctcacggccg tcatggcgac gttcaacaac atcggtcccg gcctcggcct ctgcggcccg    1320
   gcgggcaact tcgcgttctt ttctccgttc gcgaaggtga tcctgtgcct cgatatgctg    1380
   ctcgggcggc tggagctgta cccgatgctg gtgctgctga tgccgagcac gtggagaaag    1440
   aaataa                                                               1446
<210> 7
   <211> 657
   <212> DNA
   <213> Unknown
<220>
   <223> Unknown gut bacteria
<400> 7
   ttggacgcga aattactgga cctgctggag cagatcgcaa aggcgatcgc agcacaattc      60
   ggcagcaact gcgaagtggt gatccacgag ctttccggca aaagcgccta ttcctcgatc     120
   gtcgcgatcg aaaacgggca tgtgaccggc cgcaaggtgg gcgacgggcc ctcccatgtg     180
   gtgctggagc agctggggca cgaggatgac agcgcaaagg atcagcttgc gtatctgacg     240
   cgcacaaagg acggaaagat cctgaaatcc tcctctgtgt atatccgtga cgagtccgga     300
   aaggtctcgg ggatcctggg gatcaactat gacatttcca tgatgcagct gtttgaaaac     360
   tccctgcatg acttcatctc agccgatcag catgcgagcc gcgagcccga gcgcatcacc     420
   ctcaacgtgg ccgatctgct cgacgacctc atccggcagg cggacgaact ggttggaaaa     480
   ccggtcgcgc tgatgaccaa agacgacaag gtcaaggcca tccgctatct cagtaattcc     540
   ggcgctcttc tgattaccaa atccggcgat aaaatagcga agcatttcgg catatccaag     600
   tacactctct attcatacct agataacagt aaaacaggag gaacaaacga actatga        657
<210> 8
   <211> 1176
   <212> DNA
   <213> Unknown
<220>
   <223> Unknown gut bacteria
<400> 8
   gaagacgccg tcaacgagat cttccccgag atctacgaag ctgccattgt caagcaggag 60
   ctcaaggccg tcggccagcc ctccgttacc gatatgcaga cccccgaaga cggcagcgtc 120
   gtgctgaccg tttccactga gctctacccc gaggtcaccc tcggcgagta caagggcatc 180
   gaggtcccca aggagaccgt ttccatctcc aaggccgaag tcgacgcaga gctcggccgc 240
   atgcaggacc gcaacgcccg catcgagacc gttgaccgcg aagccaagac cggcgacacc 300
   gtcattctgg actttgaagg ctttgtcgac ggcaagccct tcgacggcgg caaggccgag 360
   ggctataccc tgacgctcgg ctccggcgcg ttcatccccg gcttcgaaga tcagctcgtc 420
   ggcctgaagg ccggtgaaga gaaggccgtc gaggtcacct tccccgagag ctacaccaag 480
   gagctggccg gcaaggccgc gaccttccag tgcaagatcc acgaggtcaa ggagaccatc 540
   aagcccgagc tcgacgacga gttcgccaag gacgtctccg aatacgacac gctggccgcc 600
   ctcaagaaga gcatcaaggc cgacctcgcc aagaagcgtc aggaagctgt tgaccgtgac 660
   tttgagaacg aggctgtcgt tctggccggc aagaacatga cctgcgacat ccccgcctgc 720
   atgatcgacg agcaggtcga caagcacctc gagcagtttg cctatcagct gcagtcccag 780
   ggcatgaaga tggaagacta cgtcaagatg atcggcggcg acgtcaaggg catgcgcgat 840
   tccatgcgcc cgatggcgga gcagaccgtc cgcagcaaca tcctgctgag cgagatcgtc 900
   catcaggaga acatcgacgt gaccgacgag gaagtcgaag aagagctgaa gaagctggcc 960
   gagcagtaca agatggagct ggacaagatc aaggaaatga tcgacgtcga ggccgtcaag 1020
   tccgatctca agggccgcaa ggctgtgaag ctgatcgtcg acaatgccaa gcccgtcgag 1080
   aaggaaaagg aaaagaaggc cgccaagaaa tctgccaaga aggaagacga ggctgccgaa 1140
   gctcccgcag aagaggctcc caaggctgag gaataa 1176
<210> 9
   <211> 1653
   <212> DNA
   <213> Unknown
<220>
   <223> Unknown gut bacteria
<400> 9
   cagcagatga tccaggagaa gatccacgaa tacgggctga ccggcgtagt catctgttcg      60
   tgctccccca gaatgcacga gcagacgttc cgcaagacct gtgaaaaagc aggtctcaac     120
   ccctatatgg tccagatcgc caacatccgc gagcagtgct cctggatcca caagtacaag     180
   gaggcgggca cggaaaaggc catcatcctc ggccgggccg ccatcgccaa ggtacatctg     240
   aacgcgccgc tgaccgcggg cagcagcccc gtgaccaagc gggcgctcgt catcggcggc     300
   ggcatcgctg gcatccagac ggcgctcgac atcgccgagg ccggatttga ggtcgatatc     360
   gtggaaaagc agccgaccat cggcggcaag atgacccaga tcgacaagac cttcccgacg     420
   ctcgactgcg ccgcgtgcat cctgaccccg aagatggtcg actgcgcaca gaacgagaag     480
   atccacatct attcctactc cgagatcgaa tccgtgggcg gcttcgtcgg caacttccac     540
   gtgaagatcc gccgcaaagc gcgcttcgtc aaggaagacg tctgcaccgg ctgcggactt     600
   tgcaccgaga agtgcccgca gaagaaggtc ccgaacgaat tcaacctcgg catggacaac     660
   cgccgcgcca tctatatccc cttcgcacag gccgtgccga aggtcgcgac catcgatccc     720
   aactactgca gcatgctcaa aaacggcaag tgcggtgtgt gcgcaaaggt ctgttccgcc     780
   ggcgcgatcg actacaagca gaccgacacc ttcatcgagc aggaatacgg cgccatcgtc     840
   gccgcgacgg gcttcaaccc catcgacctt tccaaattcg atgaattcgc ctacagccag     900
   agcccggacg tcgtctcgag cctggaattt gagcgcctca tgaacgccgc gggcccgacg     960
   agcggcacgc tcctgcgccc gtccgacggc acgcacccga agaccatcgt cttcgtccag    1020
   tgcgtcggct cccgctgtga cggtgtggag aagggcaagc cctactgctc caagatctgc    1080
   tgcatgtaca cggcgaagca cgccatgctc tgccgcgaaa aatacccgga caccgacgtg    1140
   tacgtcttct acatcgacgt ccggaccccc ggcaaaaact ttgacgagtt ctaccgcaga    1200
   gccgtcgagc agtacggcgt gcattacatc aagggcatgg tcggcaaggt cacgccggag    1260
   ggcggcaagc tcaaggtgca ggcgtccgac ctgctcgaga accggcagct gcacatcgac    1320
   gccgacatgg tcgtcctcgc ggccgccatc gagcctgaca agtccgcccg gccgctcgct    1380
   acgatgctga cggcctccat ggacaccaac gactttttca ccgaggccca tccgaagctc    1440
   cgcccggtcg aaagcccgac ggcaggcgtg ttcctgtccg gcatgtgcca ggggccgaag    1500
   gatatccccg agacggtcgc acaggcgtcc gccgcggcgg cgaaggtcat cggccttctg    1560
   tgcaaggaca gcctgacctg caacccgtgc gtggcgcagc cggatgagat gatgtgcaac    1620
   ggctgctcca actgtgaaaa ggtctgcccc tac                                 1653
<210> 10
   <211> 843
   <212> DNA
   <213> Unknown
<220>
   <223> Unknown gut bacteria
<400> 10
   atggaaaaga aaaaaggcct ctccctgcag acgatcctga ccatcgcggg catcgccctc    60
   atctacgtgc tcgccttcct gctcgaccgc atcccgggcg tgcccatcat gctcacgacg   120
   gttttgcgca agggcgcgat ctattccctc gtcgccgtct ccatgaacct gctcaacggc   180
   tttacgggcc tgttctccct cggacaggcg ggcttcatgc tggtcggcgc ctatgccttc   240
   gccgccatca acatctcgag cggcatccgc tcgacggtgt atcagtacta tgatccgctg   300
   ctcaaattct ccctgaccga gacgctgcag aacgccctcg gcaatacggc gggcaccgtc   360
   ctcggcgtgt tcgtttcgct gctgctggcg ggcctgctcg ccgcggggct ggcgtacctc   420
   atcggcctgc ccgtcctgaa gctcaagagc gactatctgg ccatcgccac gctcggcttc   480
   gccgagatca tccgcgcgct ggtccagctc gaggcgttcg cgcccgtcgc caacggctcg   540
   cagctgctgc gcggcttccc gtatttctcc gataccttca tgccgttcgt gatcgtcggc   600
   atctgcatcg ccatcgtcgt gctgctgatc aactcgacct acggccgcgc cttcaaggcc   660
   atccgcgatg atgagatcgc cgccgaggcc atgggcatca accttgccca ccacaagcgc   720
   atggcattca tcatctcctc gttctttgcc ggcatctccg gcgcgctgct ggccatgtac   780
   cagaccacgg tccaggccaa cacgttcaaa tcggccatga cgtatgagat cctgctcatc   840
   gtc                                                                 843
<210> 11
   <211> 978
   <212> DNA
   <213> Unknown
<220>
   <223> Unknown gut bacteria
<400> 11
   atgatcgtgg atattaccta ccccattgag acctggccga agcagaatga gctgacgcac    60
   atcaccgagg gcatcaagca gtccaccggc ggctcggtct gcaacaccat ttcggacctc    120
   gcaagactcg atccccagct tccgctcgtg gcctccggct ttgccgggca tgacgccgag    180
   ggcgatttcg tcctgcagga gatgggaaaa tacaagaata tcgacctttc catggtcaag    240
   cgcaacggcc gcacgtcctt taccgcggtc atgtccaaca accagacgaa ggagcgcacc    300
   ttcttccagc acgcgggcgc gaacgcctac tacggcgagg acgacatcga ctgggataag    360
   ctcgacgtcg atatcttcca cattggctat atcctgctgc tgccgcatct ggacgagccg    420
   gacgcggaat acggcacgaa gatggcgcgg cttctgcacc gggcgcagaa ggccgggatg    480
   aagacgtcca tcgacgtcgt ctccgaggcg ggcgagcgct ttgcgcggct cgtgaccccg    540
   gcgctcaaat acaccgacta ctgcatcatc aacgagcttg agacccagca gaccacgggc    600
   gtgctgctcc gcggagaaga cggcgtgctg catgtggaaa acatgaaggc cgcgctgcag    660
   aagctccacg agctgggcgt tgcgaagtgg gccgtcatcc actgcccgga gatcggctgc    720
   ggcatggacg agaacggcaa ttactatgag tttgagagcc tgaagctccc gaagggctat    780
   atcaagggca ccgtcggcgc gggcgacgcg ttctgcgcgg gcgttctgta cgcggcggag    840
   acggatatgc cgctgccgga ggcgctgaag ctcggcgctt gcagcgcggc ggcctccctc    900
   agcgaggtca gcgcgtccga cggcgtgaag acgaaggatg aagtcctgaa gctgtacgag    960
   ctgtacggca agaaataa                                                  978
<210> 12
   <211> 567
   <212> DNA
   <213> Unknown
<220>
   <223> Unknown gut bacteria
<400> 12
   atgacaacct tctccgagca gttccctatc gtatttcagg ccctgaatgt gggctttttg      60
   cagacgctca agctgttttt tgtgacgctc atcggcgcga tcccgctggg tctgatcatc     120
   tccttcggct ccatgagccg ctggtcgccg ctgggctttc tgcgcccgta tctgaaaaat     180
   ctccccaagg acggcaagct cacctggtgg cagaagacgc agctatggtt cgcggtcgac     240
   ttccggccca tccgcctgat tgtgcgtttc gtcatctggg tcgtgcgcgg cacgccgctg     300
   atgctgcagc tgctggtgtt ctactacttc cccggccttg tgctgcacaa gaatatctgg     360
   ggcagcggcg aggcagggcg cttcctcgca tcgtcgatcg cgttcatctt caactacgcg     420
   tgctatttct ccgagatgtt ccgcggcggc atccagggcg tcccgaaggg gcagcaggag     480
   gctgggcagg tgctcggcat gacgagcacg cagatcttct tcaaggtgac gctgctgcag     540
   atggtcaagc gcatcgtgcc gcccatg                                         567
<210> 13
   <211> 1449
   <212> DNA
   <213> Unknown
<220>
   <223> Unknown gut bacteria
<400> 13
   atgcctgaaa tcaaactcga acacgtcacc aagcgctggg ccaagttcta cgctgtggaa      60
   gacctgaatc ttaccattga gaataacgcg ttcgtcacgc tgctcggtcc ctccggctgc     120
   ggcaagacca cgacgctccg catgatcgcg ggtctggaga ccccgacgtc gggccgcatc     180
   acgatcggcg acaaggtcgt cttcgacagc gacctcggta tcaacgtccc ggccaacaag     240
   cgcaaggtcg gcttcctgtt ccagaactat gcgctctggc ccaacatgac ggtctaccag     300
   aacatctcct tcggcctctc caacatcaaa gaggagctcc cgaagatcga cttcgaggcc     360
   aaaaatgctg cgcgtctggc tcagatcctc cagactccgt ccgatgtgac cgcggttctg     420
   gacgagtgcc gcgacaagga cggcaagctg gaagaaaaga aggccgtcct caagctcatc     480
   gacgccttta cgctctcgca gtacacggcg aagaagctct tcggctatca tctggagaac     540
   ggggccgatc gcgccgccgc gatcgccgca ctgcaggcaa aggtcgacgc agcccataag     600
   gccgcgaagg acgccggctg cacgctggac gaggagttcc gcttctgcag ggacggcagc     660
   gtcgtcaagc agacgagaaa gctcacgaag gaggagatcg acctctccgt ccgccgcgtc     720
   agccgcatcg tcaagattgg catgttcatg gaccggtatg cggccgagct ctccggcggc     780
   cagcagcagc gtgtcgccat cgcaagaacg ctcgcgccgg agcccacggt tttgttcatg     840
   gacgaaccgc tgtcgaacct cgacgcgaag ctgcggctcg agatgcgcta tgagctgcag     900
   cgcctgcacg tggagaccgg ctcgaccttc gtctacgtca cgcacgacca gatggaggcc     960
   atgacgctcg ccacgcagat ctgcctcatc aataacggcg ttctgcagca gtatgaagcg    1020
   ccgctggagg tctatcatca cccggcgaac ctctttgtcg ccgacttcgt cggcaacccg    1080
   tccatcaact ttgtcgaagc caagggcaag caggcggccg acggctcgct gaccctcacg    1140
   gtcctcggtg ggctgacagc cagattccgc ccggcaaagg cgctcgagct ttccaagtgg    1200
   ttcgcagacc gcgacgcagc tgccgccaag aaagccgccg acctcaagga gaaggccgcg    1260
   cagaagggct acgtcgaaaa gggcaacaag gatgaggtct tccgctatca tatcgcaaag    1320
   gtcgacgagg aagacgactc ccttgcggag cttcccgaga tcacgaacga agattttgtc    1380
   ctcggcatcc gcccggagtt cctcgacatc gcggacgaag ggaacctccg cggcgagatc    1440
   tacggcgcc                                                            1449
<210> 14
   <211> 1020
   <212> DNA
   <213> Unknown
<220>
   <223> Unknown gut bacteria
<400> 14
   atgggcggcg acaacgcccc ggatcagatc gcgctcggtg cgatccaggc cgcgaaggac      60
   tttggctgcg aggccgtact ggtcggccgc ggcgaggcca ttttgcaggc gctcaaggac     120
   caagggatcg agacgcttcc caagggcgtg gagatcgcca acgccgacga cgtcgtcgat     180
   atgcacgacg acccggcgac ggtcgtgaaa aagaaaaagg attcctccat ggtcgtcgga     240
   ctgaccatgc tcaaagaggg cggcggcgac gcgttcgtct ccgccggctc gacgggcgca     300
   ctgctctctg cggcgacgct gatcgtccgg cgcgtcaagg gcatccgccg cgcggccatg     360
   ggcccgcaga tcccgacgaa gaccggccgg gagtgcgtcc tcatcgactg cggtgcaacg     420
   gcggactgca cgccggagtt tctgctgcag tttgccttca tgggctcgta ttacgcggaa     480
   aaggtcctcg ggatcgaaaa cccgcgcgtg gcgcttctca acatcggcgc agaggactcc     540
   aagggcggcg agctgcagaa ggccgtttac ccgctgctga agcaggcggg cgaggccggg     600
   aagatcaact tcaccggcaa catcgaagcg cgtgacgtgc cgctgggcgg cgcggacgtg     660
   gtcgtttccg acggcttctc cggcaatatc ctgctcaagg ggatcgaggg catggcgctt     720
   ttcatggcct ccatgatgaa ggacatgttc aagaaaaatc tgctgaccaa gctggccgcg     780
   cttttgtgca tggacggcgt caaggcgttc aagaagaaaa tggactaccg cgagaccggc     840
   ggcaccgcgc tgatcggcct caacaagcct gtcatcaagg cgcacggctc gtccgacgcg     900
   ctggccatcc gcaacgccat ccggcaggcc atcggcgccg tcgaggcgga tgtcgccggg     960
   acactggcag caaatatcga ccatatggtc gttccaaagg agtatcaaca tgctgagtga    1020
<210> 15
   <211> 1347
   <212> DNA
   <213> Unknown
<220>
   <223> Unknown gut bacteria
<400> 15
   atggcgaccc gtcttccgta ttccgatttt gccgatatcc tgccggaaat gaacaaggcc      60
   ggctactatt cggtggaatg ctggggcggc gctacgttcg actcctgcct gcgttacctg     120
   ggcgaggatc cctggcagag actgcgcgac atccgcaagg ccatgcccga caccaagctg     180
   cagatgctgc ttcgcggcca gaacctgctc ggctacaagc actatcatga cgacgtcgtc     240
   gagaagttcg tcgagctgtc catcaagaac ggcatcgaca tcctgcgtat ctttgacgca     300
   ctgaacgact tccgcaacat cgccaccgcg ctggaagcta ccaagaaata cgcgaccaag     360
   aacaccgtcg cctccggctg tatctcctac acccagagcc cggtccacac ggtcgaaata     420
   tatgtcgaga tgtgcaagga gctcaagaac atgggcttcg acaccatctg cctgaaggat     480
   atggccggca ccatgagccc gtacgaggcc gagcacctca tcaagggcat caaggacgcc     540
   gtcggcgatg ttcccctcat cctgcacacc cactgcacca ccggcatggc ttacatgacc     600
   gtgacgaagg ccatcgagtc cggcatcgac gtgatcgact gcgcgacctc ctgcttctcc     660
   aacggcacca gccagccctc caccgagacc atgttctacg ccctcggcca gtacggcatc     720
   gagactggcc tcaatgagga cgtcatcaac aaggtcaacg actacttcaa gcccatcaag     780
   cagaagtacg tcgattccgg ccgcatcagc gccaagtcca tggcgaccga cgcgcaggcg     840
   ctggtctaca aggtcccggg cggcatgctc tccaacatga tcgccaacct gaccgacatg     900
   aaggccatgg ataagttcga cgccgcgctc aaggagatcc ccgaagtccg caaggatctg     960
   ggctatcccc cgctggtcac cccgctgagc cagatggtcg gcaaccaggc cgttaccaac    1020
   gtcctgatgg gcgagcgcta caagatcgtc tccaaggaag tgcagaacta tttcaagggc    1080
   cagtacggca tcgcgcccgc gcctgtctct gaagagctgc aggccaagat cctcggcgag    1140
   ggcggcaagc ccgtcgactg ccgcatcgaa gacgccaagc gcaccggcga agacttcaag    1200
   aaagccaagg aagccctcgg cgatctggcg aagagcgaag aggatgtcat gagctacatc    1260
   tgctatccgg atcaggccat gaagttcttc gaagaccgca aggccaagga agaaaacgtc    1320
   tgcacctacg tcatcgaaga agcataa                                        1347
<210> 16
   <211> 1329
   <212> DNA
   <213> Unknown
<220>
   <223> Unknown gut bacteria
<400> 16
   ctgcgtctgc gcggcacgcg cggaaaagcg ctgaaaacaa aggagcacgg aacagtgaac 60
   acttatatag taaagggaga tatctgcttc agccggagca tggatgagat cgtctgtctg 120
   gagcagggca gcatcgttgt ggaaaacggc cgcatcaccg gcgtctttca gacgctgccg 180
   gcgcgctatg ccgcgctgcc ggtgctcgat tacggccatc ggctgatcct gcccggtatg 240
   tccgacctgc atgtccacgc gccgcagttc gccttccgcg ggctcggcat ggatatggag 300
   ctgctggagt ggctcaacac ctataccttc ccggaggaat ccaaatacaa ggacctggac 360
   tacgccgaaa gggcgtatgg cagcttcgcc tcgcatctgc tgcgcagcac gaccacgcgc 420
   gcggccatct tcgccaccat ccatgtgccc gcgacggagc ttctcatgca gaagctggac 480
   gcgatggggc tggaatgctt cgtgggcaag gtcaatatga accgcaactc gccggtctac 540
   ctgcgcgaga tctccaccaa ccaggccctg cgcgccaccg agcgctgcat ctgcgagacg 600
   aaggcgcgct atgcccatgt caggcccatc ctgaccccgc gcttcatccc gtcgtgcacg 660
   gacgatctga tgttcgggct ggcgcggctg cgggtgaaat acgacctgcc ggtgcagagc 720
   catctgtcgg aaaactattc ggagatcgcc tggatccgcg agctgtgccc gcgctcgaag 780
   aactacggcg acgcgtatga gcagttcggc atgttcggcg gcggatatcc gtgcatcatg 840
   gcacactgcg tccactcgaa cgaggacgag caggcgctga tgcagaaaaa cggcgtgttc 900
   atcgcccatt cgccggaatc gaacatgaac ctcgcctccg gcgtcgcgcc gatcaataaa 960
   ttcctcgata acggtctgca ggtcggcctt gcgacggata tcgccggcgg cagccacgag 1020
   agcatgctcc gcgccatgac ccacgccatc caggcctcga agctccgctg gcggctgtat 1080
   gaccaggacg tgaagcccct gtcgtttgac cgcgcgttct atcttgccac catgggcggc 1140
   ggcgcgttct tcggcaaggt cggctcgttc ctggacggct acgaatttga cgccgtcgtg 1200
   ctggacgacg cggcgctgga ccatccgcag gagctttcgg tccgcgcccg gctcgagcgc 1260
   gcggcctatc tggccgacga ccggaacctc acggccaaat ttgtcgcggg gcagaaaatt 1320
   ctcggttga 1329
<210> 17
   <211> 882
   <212> DNA
   <213> Unknown
<220>
   <223> Unknown gut bacteria
<400> 17
   atgaagaaaa aacgctattt ttcccgcacg gtctgctttc tgctctgcgc ggtgttcgcg      60
   ctgttgtttt tgctgccgac ggttctgacc atcacgaact ccttcatgac gcagagcgag     120
   atcacggcca gctacggcca ggtctttgaa aacgccaacg acggcaagag ctacatctcc     180
   aagaccattg aactcaaatt catccccgac aaggtcagct tcacccagta tttcaccgtc     240
   ctgctcaaaa gcccggacta tctgctgaaa ttctggaact ccgtcatcct gaccgcgccg     300
   atcgtcttcg cgcagctcat ggtcgcgtcc atcgcggcct atggctttac ccgctggcgc     360
   gggaaggtgc gcgacagtct gttcttcttc tatgtgatcc tgatgctcat gccgtatcag     420
   gtcacgctgg tccccaacta tctggtgagc gactggctga agatcctcaa tacccggtgg     480
   gcgatcttcc tgcccggcgc gtttgcgccg ttctcggtct ttctgctcac caaattcatg     540
   cgccgcatcc ccatggctgt catcgaatcg gccaagatcg acggcgcgaa cgagtggcat     600
   atcttctgga acatctgcct gccgcagtgc cggagcgccc tctattccat tgcgatcctg     660
   gtgttcatcg acaactggaa catggtcgaa cagccgctga tcctgctcaa cgacgtggag     720
   aagcagccgc tttcgatgtt cctgtccagc atcaacgccg gcgagatcgg catcgccttt     780
   gccgccgccg tcatctatat gatccccggt ctgctgctgt ttctgcacgg cgaggcgtat     840
   ctggtggaag gcatcgcaca ttctggctct gtgaaaggat aa                        882
<210> 18
   <211> 1347
   <212> DNA
   <213> Unknown
<220>
   <223> Unknown gut bacteria
<400> 18
   atgacccaca cgataggcat gatctcgctt ggctgcgcca aaaatcaggt caatgccgag      60
   catatgctct ggctgctgcg gcaggcaggg tatgagatca gccccgaccc cgacggtgcg     120
   gagctcgtca tcgtcaacac ctgcggcttt atcgagtccg caaagaccga ggccatcgac     180
   aacattctcg ccatggcgca gctcaaggcg gagggccgga tccagaagat cctggtcacc     240
   ggctgtcttg cgcagcgcta tcaggaggag attctgcagg aaatgccgga ggtcgacggc     300
   gtgcttggca ccggctccta ctttgacgtt gccaacgcgg tcgggcaggt gctgagcggc     360
   aaacgctaca agcgcttcga cgatatcaat gcagaccagt cggaggatgg ccgtatcctg     420
   accacgccgg agtattatgc ctatctgaag atcgccgagg gctgcgacaa ccactgtgcc     480
   tactgcatca tcccgaagct gcgcggcaag ctgcgcagcc gcccgatcga ggagctcgtc     540
   aaggaggcgg agcagcttgc ggccgacggc gtcaaggagc tgatcgtcgt cgcgcaggat     600
   accagccgct acggtctcga tctctacggc gagcggaagc tcgcggagct gctgcggcag     660
   ctgtgccgga tcgacgggct tgtctggatc cgcgtgcact atctctatcc ggatgagatg     720
   tcacaggagc tgattgacgt gctggcgaac gagccgaaga tcgtcaagta tctggatatc     780
   ccgatccagc acatcaacga cgatatcctc cgccgcatga accgccgcgg caacggcgaa     840
   tacgtcaagg agctgttcgc caagctccgc cgcgagatcc caggccttgt gctccgcacg     900
   agccttatca ccggccttcc cggcgagggc gaggcggaat ttgcccagct ctgtgacttt     960
   ttgaaggaat acaagctcga gcgcgtcggt gcgtttgcgt tctccccgga ggagggcacg    1020
   cgcgcggcag agatggaata tcctgatacc gagatcgccc ggcagcgcgc ggacatggtc    1080
   gccgagatcc agtcccgcat catggacgat tacaacgaat cgtgcatcgg caagaccatg    1140
   caggtcctgt gcgagggcta cgatcccgat gaagagagct attacggccg gacctttgcc    1200
   gactcgccgg atattgacgg gaagatctgg ttcacggcgg aaaaacatat caagaccggt    1260
   gactttgccg acgtccgtgt ggtggacgcc tatgacggtg agcttgtcgg tgtgctggag    1320
   gaagaataca atgacgactg cgagtaa                                        1347
<210> 19
   <211> 1383
   <212> DNA
   <213> Unknown
<220>
   <223> Unknown gut bacteria
<400> 19
   ggcaagtcga ccctcggccg caccatcctc cagctctacc atcagaccta cggccgcacg 60
   atgtactatg gaagatcgct ggacgatctg gcgccgaagt acgtcatcga caccgtcaaa 120
   aacctcaaat cacgccgggc caagctccac gagctggagc agaagcgcga taagctcgag 180
   gccgagtacg ccaagctctc cgaaacggag cagtatgcca agcacaccga gctcgacacc 240
   gcgcgcaagc tggccgagga cgcgctgctc gacatcacaa agatcgtcgg cggcttcctc 300
   gtggtcgatg atctcgatcc cgtgatcgcg gcctattcca aggactacgg catcgcgcgc 360
   aagatctccg cgctcgagga aaggcggcag gcgctgcagg tcgacgtcga cgacgcgcag 420
   tacgcgaaga agaccgccga agaggcgggc aagtccgaaa agtccggcca gctcgaaaag 480
   gcacaggaaa agctcaagca gtgtgacgat cagatcgccg cgctgcggca gcagctggac 540
   gctggccgcc aggagatcga ggcgctgcgc cagccgtatg ccagcgatcc cgaattccag 600
   aagtatgagg cctaccgcga cgacggtatc gaccttgccc gactggagta caacgagatg 660
   cgccgcctgc gcagcgatat gcagctcatt ttccaggatc cgtattcctc gctgaacccg 720
   cgcatgagcg tcggccagat catcagcgag ggcatgcagg cgcacaacat gatcaagaaa 780
   aaggacgccc gtatgcagca gctcgtcctg aacatcatgg acgaatgcgg cctcgcaccg 840
   tacttcctgc accgcttccc gcaccagttc tccggcggcc agcggcagcg catcggcatc 900
   gcccgcgcgc tcgccacgaa cccgaagttc gtcgtctgcg acgaggctgt ttccgcgctg 960
   gacgtgtcta ttcaggccca gatcatcaac ctgctgctcg acctcaagga gcaccagaac 1020
   ctgacctaca tgttcatcac gcacgacctg tccgtcgtca agtacatctc cgaccgcgtc 1080
   ggcgtcatgt acctcggcaa catggtcgag ctggcggagg ccgacgagat cttccaccac 1140
   ccgatccatc cgtacaccga ggccctgctc aacgccatcc cgacgacgga taccgtcggc 1200
   gcgaaggagc tctccatcct cgaaggcgat atccccagcc cggtcaaccc gccgaagggc 1260
   tgcaaattcc acacgcgctg caagtactgc acagagatct gcacgcaggt cgtcccggcg 1320
   tgggaagagg tccggccgaa ccatttcgtc gcctgccatc ataagctcga agtcaaggag 1380
   taa 1383
<210> 20
   <211> 1194
   <212> DNA
   <213> Unknown
<220>
   <223> Unknown gut bacteria
<400> 20
   atggtcagtg aaaaaatggt cgcctggggc agccagggct gcgtcatccg ggatctcgcc        60
   gcctacggcg agcagcgcgc cgccgtcgtc ggcgcggaaa acgtgttcaa ctttaccatc       120
   ggcaatcctt ccgtcccggc gccgtcctgc gtccggcaga cgatcgagca cctgctgcag       180
   accgtcccgg ccgagcagct gcacgcctat acggccgcgc cgggccttgc gtccgtccgc       240
   gcgaagatgg cggcgtatct cgagaagacg ttcggcgtcg gctaccgcgc ggaggacgtg       300
   tatatgacct ccggcgcatc ctcggcgctg gcgatgctgg cctatgcgct gctctcgccg       360
   ggcgacgagg tgctgacgct cagcccgtat ttctcggagt acaagctcta cgccgaggcc       420
   gcgggcggca cgctcacggc cgtgccctcg aacccggagg atttccagat cgatctggac       480
   gccttctccg ctgccatcag cgagaagacc gccgtcgtcc tcctgaactc cccgaacaac       540
   ccttccggtg tcatcctctc ccgccggagc atcacaaagc tggcggagct gctggagaaa       600
   aaatcagccg aatacggcca ccgcatctac atcgtcgccg acgagcccta ccgcgagctg       660
   gcctatgacg gcgaggaggt gccgttcatc cccgccatct acaaggacac catctactgc       720
   tattccttca gcaagaccct ctcgctgccc ggtgagcgca tcggcttcct ggccatccat       780
   ccggagctga ccgactacgc gcgcgtccgc gcggccatct acggcgcggg ccgggcgctc       840
   ggctatatca acgccagcac gatgttccag ctcgtagcgg cggaatgcgt cgggcagacg       900
   gcggatatct ccgtctacaa gcagaaccgg gatctgatcc atacggcgct tctggatatg       960
   ggctatacct gcgtgaagcc ttcgggcgcg ttctatcttt tcctcaaaac gccggagccg      1020
   gacgcgaccg cgttctgcaa aaaggcgatg gcgctcgacc tgctggtcgt gccgggcgac      1080
   gaattcggct gcccgggcta cgtccgcctg gcctactgcg tccccaaaac catgctcgag      1140
   cgcagcctcc cccgcttccg ccagctcgcc gaccaatacg gcctcaaaaa ataa            1194
<210> 21
   <211> 498
   <212> DNA
   <213> Unknown
<220>
   <223> Unknown gut bacteria
<400> 21
   gcctatgaca accggccgct caccttcgac gaatttacag accgcatcca tcaggccatc        60
   tacgtctccg ccacgcccgc ggactatgag cgcagccgcg ccgggcagat cgccgagcag       120
   gtgatccggc ccacgggcct tctcgacccc gaggtcttcg tgcgccccat tgaggggcag       180
   atcgacgatc tcatcggcga gatcaacgcc acgaccgaca agggcgagcg cacgctcgtc       240
   acgacgctga cgaagaagat ggccgaggat ctgaccaaat atctgaccga aaacggcatc       300
   cgcgtgcgct acatgcactc ggacatcggc gcgatggaac gcatggagat catccgcgat       360
   ctgcgcatgg cgaagttcga tgtactggtc ggcatcaacc tgctgcgcga ggggctcgac       420
   ctgcccgagg tctcgctcat cgccattctt gacgcagaca aggaggggtt cctgcgctcc       480
   gagacgagcc tcatccag                                                     498
<210> 22
   <211> 1932
   <212> DNA
   <213> Unknown
<220>
   <223> Unknown gut bacteria
<400> 22
   gaccacggcg tcatgtacgg cacgatcgac ttttaccgcg cagcaaagaa ggccgggatc      60
   aagcccatcg tcggctgcga ggtctatgtc gcccgccgca cgcgcttcga caaggtccac     120
   ggcgtcgaca aagaacccta ccatctcgtc ctcctgtgtg aaaacgagac cggctaccgc     180
   aatctcagct atatggtctc gctcggcttc acggagggct tttataaccg cccgcgcgtg     240
   gatatggagc tgctgcgcga gcatcacgag ggcctgatcg ccctgtccgc ctgtctggct     300
   gggcgcatcc cgcagtcgat catgcaggac gacctgcagg ccgccgagca ggcggcgcgc     360
   gagtatgccg aagttttcgg gccggaccat ttttatctgg agctgcagga tcacggcatc     420
   ccggagcagc ggaccgtcaa cgcacagatc cgcaagctcg ccgaaaaact gtctctgccg     480
   ctcgtcgtga cgaacgacgc acactatctg cgcaagtcgg acgcgaaaat gcaggacgtg     540
   ctgctctgca tccagaccgg caagaccgcc gacgacccga accgcatgaa atttgagacg     600
   gaggaattct atctcaagag cgaagaggag ctgcgcgagc tgttcccgga tgtgccggag     660
   gcgtttgaca atacagtaaa aattgcggag cgctgcaatg tggagtttac gttcggcaag     720
   tatttcctgc cggaattcaa gctcccggag ggcgtgacga gcctttcgta tcttaagcag     780
   ctctgccgcg agggctttga cgagctctac gggacagagc acccggaata tatgcagcag     840
   ctcgactacg agatcgacat gatcgagaag atgggcttta ccgattactt cctcatcgtg     900
   tgggatttcg tgcgctttgc caaatcggcc gggatcccgg tcggccccgg gcgcggcagc     960
   gccgcgggct cgatggtcac ctactgcctg catatcaccg agatcgaccc catgaaatac    1020
   gggctttatt tcgagcgttt cctcaacccg gcgcgcgtga cgatgcccga tatcgatatg    1080
   gacttcggtg acacccggcg cgccgaggtc gtcgactacg tccggcgcaa atacggcgag    1140
   gaccatgtcg cacagatcgt caccttcggt accatggctg cgcgcggctc catccgcgac    1200
   gtcggccgcg tgatgaactt cacctacgcc gagacggacg cggtggccaa gctcgtgccg    1260
   ccgacgccgc acatgacgct ggcggacgcg ctgaagcagt cgccgcagct gcggcagatg    1320
   tacgactctg acgaacgggt cagggaactg gtcgacacgg caaaggccat cgagggcatg    1380
   ccgcgcaaca cctcgacgca cgccgcgggc gtggtcatca ccaagctccc ggtctatgac      1440
   tatgtgccgc tggcgacgaa cgacggcacg atcgttaccg aatacaccat gacgacgctg      1500
   gaggagctcg ggctcctcaa aatggacttc ctcggcctgc gcaacatcac cgtcatcgac      1560
   gatgccatcc gcgatatccg taagaccgag ccggacttct ccatggcccg cgtcaccgac      1620
   aacgacccca aggtcatgga catgctcacg cagggccgga ccgccggtgt gttccagatg      1680
   gaatcgaccg gcatgacggg cgtgtgcatg ggcctgaagc cgcagtcgat cgaagatctg      1740
   tccatcatcg tcgccgccta ccggccgggc ccgatggaat cgatcccgcg cctgatcgcc      1800
   tgcaagcagg atccgtcgct cgtcaaatac aagcacccga tgctgcagcc catcctctcc      1860
   gtcacctacg gctgcatcct ctatcaggag caggtcatcg agatcttccg caagcttgcc      1920
   gggtattcgc tc                                                          1932
<210> 23
   <211> 1323
   <212> DNA
   <213> Unknown
<220>
   <223> Unknown gut bacteria
<400> 23
   atgcaggcgc agcggatgac ggagcccggc gatacggtcg tatgcgcagt gtcgggcggc 60
   gcggattctg tgagcatgct gcacgtgctg ctgtcactgc aggacacgct cgacatccgg 120
   gtagaagccg cgcacttcaa ccatcacctg cgcggcgcgg aatccgaccg cgacgaggcc 180
   tttgtccggc agctgtgcgc ctcgctcggc gtgccgctgc acacgggcgg cggcgacgtg 240
   caggcgcggg cggcgcagac gcatgaatct ctggaagagg ctgcgcgcaa gctccgctac 300
   gcgttctttg acacactccc cggccttgtc gccacggcgc acacgcagga cgacaatctc 360
   gagactgtgc tcctgaacct cacgcgcggg accggccttg ccgggctgac cggcatcccg 420
   ccgaagcgcg gacggctcat ccggccgatg ctggtctgca cgcgggcgca gatcgaggcg 480
   tgtcttgctg agcacggatt ttcttatgtg accgacagca ccaatctcct gccggacgcc 540
   cggcgcaacc ggctgcgcca gcgcgtgatc ccgcttctgc gggaggaaaa ccccgccgtc 600
   gcacagaccg tcttccggac ctgcgcgctg ctccgtaagg atgacgcata tcttgatacg 660
   cgggcgcgcg aggcgctgca ggctgctcaa ttgccggacg gtgtcagccg cagtgcactt 720
   tccgcttggc cggaggctgt gcgcacccgc gcagtccggc tgctgctcga gtcgatccat 780
   gcgcccaatc tgacgcagcg gcatatcgat gcggtcgacc ggctgctgtt cgcttcgtgc 840
   ccctccgctt ctgtctcgct gccgggcggc tttgccgcgc ggctggaata tgacaggctg 900
   tacctgacgg accgctcccc tgccgcttcc ttttcacccg tgacactttc gccgggcgag 960
   acagtgctga tcccggcgct cgggctgcgc gtcgaatgcc ggatcgaaga aaattttcag 1020
   gaaaaagcaa aaaccctctc cacttttgca gtcaaatatg ataccataga gcaaactccc 1080
   cggatcacga tccgtccgcg tcaggcgcat gacgtcatgc gcacggcagg cggcacgaaa 1140
   tcgctcaaaa agctgctgat cgaccgcaag gtccccgcag cacgacgtgc gctcatgccg 1200
   gtggccgctg acgcatccgg tgttctgggc gtttatggga tcggcgtcga tctgggccgc 1260
   gccgcggctc cgggcgaacg cgcggtcatc atcacaatcg aagaaacaga gaaagaggat 1320
   tag 1323
<210> 24
   <211> 1836
   <212> DNA
   <213> Unknown
<220>
   <223> Unknown gut bacteria
<400> 24
   gcgccggagg accgcttccc ggccgcgctg gatgatgcgc tcgtcagcta tcagtatctg 60
   ctctcgaagg ggtatccggc caaccagatc ctgctgtgcg gcgagtctgc gggcggcgga 120
   ctgatctact gcctgtgcct gcggctcaag cagcggaagc tgccgctgcc atgcgggctg 180
   atcggcatct cgccgtggac cgatctgacc ggctccggcc agtcctatat cgacacccgc 240
   gacatcgacc cctccatgac gccggagctg ctgcagtttt acgcggcgtg ctataccgac 300
   catccggagg acccgctgtg ttcgccgctg ttcggcgatc tgacgggctt tccgccgtcg 360
   ctgctgttcg tgggcgggga cgaggtcatg ctcgacgaca cgcgggtgct gcacgaaaag 420
   ctgctggccg cgggctgccg gagcaggctc atcgtcgcgc cggagcgctg gcacgcgtat 480
   gtgctgtatt atctcaatga gaatatgtcg gatttcgaca cgatcaacca gtttatgacg 540
   cgtgtgctct ccccggcccg gaagctgcgg tggatgcggc tggacaacgc cgcgaagatc 600
   taccccgcgg ccaagcggcg caactggacg aactacttcc gcctctccgc cacgctgaca 660
   gagccggtcg acgtgcaggt gctgcgcgcg gcgctggacg ttacggtgcg gcgcttcccg 720
   tcgatcgcgg tgcggctgcg ccggggcgtg ttctggtatt atctcgagga gatcagcaag 780
   gcgcccgcca tcgaggagga caagagctac ccgctcgtcc acgtgccgtt cgacgacgtg 840
   cgcaggtgcg cgttccgcgt gctggtgtat aagaaccggc tggccgtgga gtttttccac 900
   gccgtgaccg acggcacggg cggcctcatc ttcctcaaga cgctgacggc ggaatatctg 960
   agccagaaat acggcatcca gatcccggcg gagcgcggcg tgctcggccg gctggaggat 1020
   ccgggtcccg aggagctgga ggacagcttc ctgcactatg ccggtaacgt caccgccagc 1080
   cgcaaggagg ccaccgccta tcagctctcc ggcacgccgg agccggacgg ctttctggat 1140
   ctcacgacgc tgatgctgcc ggtggacgcc gtaaaggcca aggcgaaaga gtttggcgtc 1200
   agcgtgacgg agttcatcgc ggccgtcatg atgaaggcca tcagcgacct gcagaccgag 1260
   aaggtgccgc ggcggatgcg ccgccggccc gtcaaggtcc tgctgccggt caacctgcgc 1320
   gggctgttcc cgagcaggac gcttcggaac ttcgcgtcct acgtgacgcc ggagatcgac 1380
   ccgcggctgg gcgactattc gctcgcggag atctgcaaga tcgtgcgcta ccgcatgggt 1440
   ctggagaacg acgcgcggat gatggccgcc aagatcgcca cgaacgtcgc gagcgaacgc 1500
   tcggccgtgc tgcgggcgat gccgctgttt atcaaaaata tcgccatgaa ggccgttttc 1560
   gacctggtgg gcgaatgcaa atcgtgcctg tgcctgtcca atctcgggct ggtacagctg 1620
   ccggacgcga tggcgccgta tgtcgcgcgg atggatttca tcatcggcgt acaggcaaag 1680
   gcgccgcaca actgcggcgt cgtcagctgg gacggcacga tgtatatcaa catgatccgc 1740
   aacatccgcg agccggagct ggaatcccat ttttaccgtg tcctgcacac gctggggctg 1800
   cccgtcaagg tcgagagcaa ccagcgctgg acgtga 1836
<210> 25
   <211> 1359
   <212> DNA
   <213> Unknown
<220>
   <223> Unknown gut bacteria
<400> 25
   atgggaaaag ggggatttgc gttgcggttg cggatgaaac ggatgctgct ggccgtgctg 60
   gccgcggcaa tgtcgctggt cctgtccggc tgctttgtca agaccgtcga cgagctgtac 120
   acgctcccta agcactctga tgaatataac cggctggaac aggccatcga tcaggtcatg 180
   tccagccaga acgcgtccta cgccgcgccg gtctccggca tgaaccagca gtcggtccag 240
   ctggccgatc tggacggcga cgggacggaa gaggcggtcg ctttcctgaa aacgacgggc 300
   gacaagccgc tcaaggcctg catcttttcc cgcgtcgatg aggattatca gctcatggac 360
   gtgatcgagg gcgacggtac cagctttgcg agcgtggaat acgtgccgct gaccggcggc 420
   cccggtgttg agctggtcat cgggcggcag ctttccacgg acgtgctgca gtcgctgtgc 480
   gcgtattcgt acaccggcgg gcacgtggtc gagctgctga gcacgaatta ctcggaatac 540
   cgctgcgtcg atctcgacga agacgggcgg acggatctgt tcgtcctccg ctttgacgcg 600
   gagcagccgc agggcgtggc cgagctctac cgctggcagg gcgaccagat ggagcgcgag 660
   ccggaggcgt atctgacggc cggcgcgtcg ccggtcaagc gcattgtgtc cggtgatctg 720
   atggaagatg tcccggctgt ctttgtggcg agcgcctatg aagagaacgg gcttgcgacc 780
   gacgtgtttg ccttccggga cggcgtgttc cgcaacatta ccgctgccga cccgggcagc 840
   gcgctttcca ccgtgcgcag ctattacgtc tacgccaccg atgtggacag cgacggactc 900
   atcgagctgc cgcagatcct tccgctgccg tcggctgcgg gctcggagga gaactacagc 960
   gtcatcagat ggtataattt agacctgcag ggccgcagca caccgaagat gacgacctat 1020
   cacagctttt ccggcggctg gttcttatcc ctgccggacc ggtggcagaa ccggctctgc 1080
   atcacccgca gcgaagaggt cggcggtgtc cgcggctacg tcttctccga gtggaaggat 1140
   gagacgcaca cggagctgat cttcaccatc tatgccttct ccggcgacga ccgggcgcag 1200
   ctcgcttcgt ctgacggccg gttcctcctt gccgagaagg gcgatatcac atacgcagcg 1260
   cagcttgcag cagggaagtg ggcaggcgag ctttccgagc agacgctcag aggcatgttc 1320
   cactttattc atattgactg gaattcggga gagacgtga 1359
<210> 26
   <211> 1101
   <212> DNA
   <213> Unknown
<220>
   <223> Unknown gut bacteria
<400> 26
   atggactttt taggaattct cagcgagacc tttgctcgct ccggctttgc taatctcagc      60
   tggcagcagg gcgtcatgct gctcatttcc tttgtgctgc tgtactgcgc cattgtaaag     120
   ggttttgagc cgctgctgct gctgcccatc gcttttggta tgatgctggc caacctgcct     180
   gataccggta ttatgaccga tgccaccgaa gccgccaccc ttgcggtaaa gaatgcttcg     240
   gccgcactgg ccgccgatcc taccaaccca acgcttgtct cggaactggc tgctgcccag     300
   tcccagctgg ccaatgttca ctggtatgat tccggcgttc tgcgtatcat ccaccgcggc     360
   gttaagtctt ccatcttccc ctgcctcatc ttcatgggcg taggcgcgat gaccgacttt     420
   ggcccgctgc tggccaaccc catctccctg ctgctcggcg ctgccgctca gctgggtatc     480
   tacattgcct tcatcttcgc caatgccatc accgtcggcg gcgagcacct gttcacggct     540
   gctcaggctg cttccatcgg tatcatcggc ggcgcggatg gtcctaccgc tatctttgtt     600
   accaataagc tggctcccga gctgctctct gccatcgcag tagctgccta ctcctacatg     660
   gctctgatcc cgctcatcca gccccccatc atgaaggctc ttaccaccaa gaaagagcgc     720
   gtcatcaaga tgggccagct gcgcaaggtt tccaaggcgg agaaggtcat cttccccatt     780
   gttgtttcct gcgttgttat catgctcatc cccgataccg cttctctgat cggctgcctg     840
   atgctgggta acctgttccg tgaggccggc tgcgttgagc gtctttccga caccgcgcag     900
   aacgctctct gcaacatcgt taccatcatg ctgggtacca ccgttggcgc taccgccgat     960
   ggtcagacct tcctttccct gcagaccatt ggtatcgtcg taatgggtct ggctgccttc    1020
   tgcttctcca ccgctggtgg tgtactgctg ggcaagctgc tgtgcctgct caccggcggc    1080
   aagatcaatc ctctgatcgg t                                              1101
<210> 27
   <211> 1281
   <212> DNA
   <213> Unknown
<220>
   <223> Unknown gut bacteria
<400> 27
   atggccataa ataccgacaa tgcgctgcgg cgcagcgtga tctatgaagt atatgtacgc 60
   agccacacac cggaggggac ctttaaggcg atagagcctg acctggaccg gatagcggca 120
   ctgggggtgg atattgtatg gttcatgccc attcacccta ttggggggct gaacaaaaaa 180
   ggaagcctgg gctgccccta tgccaaccgg gactatcgca ccgtgaaccc ggaatacggc 240
   acactggagg attttctgca cctggtggag gagatccata agcggggtat gaagtgcatc 300
   attgatgtgg tgtataacca cacctcgccc gattccacac tggtggcgga gcatccggac 360
   tacttctact ataagccgga tggctgccgc ggcaacaagg tgggcgactg gaccgatgtg 420
   gtggacctga actatgaaaa ccgcgcgctg tggcagtatc agattgattc gctgtgctac 480
   tgggcacaat atgtggatgg cttccgttgc gatgtagcca gcaccgtgcc ggtggccttt 540
   tggcaggaag cccgccgggc tgtggagcag atccggccgg gggccatctg gctgggagaa 600
   agcgtacacc tggcgcacat tcaggccttc cgggagatgg gcttctatgc cgcgaccgat 660
   acggaattgt tcacggcttt cgatatccta tacccctacg acctgtggcc gctgtatgag 720
   gatgtaactg agggacggct gccgctttcc cggtggtttg atgccgtgga ttaccaggag 780
   gtgagctttg agagcggata caataagctg cgctgcctgg aaaaccatga taccagccgc 840
   gcagcggatc gcttcccgga gcggaagaag ctgctggcct ggacagcgct gaattacttt 900
   atgaagggta ccatactact gtacgctggg caggaggtat gtgaaaagca cacgccctcc 960
   ctctttgaga aggagccggt ggactggaac agcggagagg atatcagcgg ttatctggcc 1020
   aagctcgcgg ccatcaagaa gaagctgccc accgatgagc tgttccgcat tggggcggat 1080
   gacacacagg gcatagtgac cgccagctat atcgggccag ccacacgggc tgtgggcggc 1140
   ttcccgctgg agggcaacgg cggcaccgtg gccgtacccc tgccggatgg cgcctacacc 1200
   gatgccctaa gtggcaaagc agtaacagta gccgatggca tgctgaccat cggggaagaa 1260
   gcggtcattc tattgggata a 1281
<210> 28
   <211> 1131
   <212> DNA
   <213> Unknown
<220>
   <223> Unknown gut bacteria
<400> 28
   atgtatcaac tgaaactgac agaaggagca gcccggcggg gagcgcttac caccgtacat 60
   ggtacggtgc agacgccggc ctttatgaat gtggcgacgg cggctgccat caagggcggt 120
   atttccgctt atgacctgaa ggagctgaag tgccaggtgg tgctgtgcaa tacctatcac 180
   ctgcatctgc gacctggtga taatgtggtg cgtcagctgg gagggctgca caggtttacc 240
   ggttggcagg gacccatcct gaccgacagc ggcgggttcc aggtgttttc gcttgcatcg 300
   ctacggcata tcgaggaaaa gggagtgacc tttgcctccc atatcgatgg acatcgtatt 360
   tttatgggac cagaggagag catgcagatc cagtcgcatt tgggttctac cattgcaatg 420
   gcatttgatg aatgtatcga aaacccatcg ccccgggatt atgtgcagaa atcggtagca 480
   cggacgaccc gatggttgga gcgctgccaa aaggaaatgg cccggctgaa cagcctggag 540
   gatactgtga atccacacca gctactgttt ggcatcaacc agggcagcac ctatgaggat 600
   attcgggtgc agcacatgaa ggatattgct gcgatgggac tggacggcta tgccatcggt 660
   ggcctggcag tgggcgagag cgccgaagag atgtatcata tcattgaggc tgtggagccc 720
   tttgccccga aggataagat ccgttaccta atgggtgttg gtacacctgt gaatatcttg 780
   gaatcggtac accggggtgt agatcttttt gactgtgtga tgccaagccg caatgcccga 840
   catggccatc tttttacctg ggaagggatc attaatatcc aaaatgctaa gtatgtaacg 900
   gatgaccgcc cggtggatgc cctttgcggg tgcccactgt gccgtaacca tacccgcgcc 960
   tacttgcgcc acctgatgcg tgccaatgaa atgcttggta tgcggctggc tgtgatgcat 1020
   aacctgtggt tctataatac actgatggaa aagataaggt tagcactgga tgagggacgg 1080
   tttgagagct tttaccgcga gcatgtgcca atactgggta atagaatata a 1131
<210> 29
   <211> 1251
   <212> DNA
   <213> Unknown
<220>
   <223> Unknown gut bacteria
<400> 29
   atgaaaaaag catttgcact ggcactggct gtgctgatgg tgtgcaccat ggcgtttgcg 60
   gtttccaccg ggaaccagac tggctacccc ggcggcacct ccgcggccga taacacctac 120
   atgcagtcca tcgtgcccgg acagtctatc gtattcaccc aggaagagct gggcctgact 180
   gaccagaact ggggcaagac caacggcagc tttgacccca agaagaatac cgttaccctg 240
   accgtccccg taggcagcga tctcatcgcc agccagggct gggtacggac cgatgagacc 300
   acctacaagt atgtggtgac caccaaggcc aatgagacct ctgtactgga taacaatgcc 360
   gatatcatca tttccggcgt aaaggtcacc gtttacggtg tttccgctcc ggcgctgaat 420
   gtgagctatg tgaaggatgt aaacggcgtg accaactata cctatgttac cagctttgat 480
   gagctggcga gcttctccgc caaggagccg ggcgacaact tctgccccat gaacgccaag 540
   agcgaggacc acatcctggc catgtgcttc gactacggcc gcaaggctga tgaggccgac 600
   gccgtatttg gcgcgggcgg actgaatgtc gccaacatgg ataagaccgg taaaatcctg 660
   tatactgtaa aggccgccac cgtagatggc cagtacatca ccagcggcag ctggaaccag 720
   tccgtcaccg aggataacgg cggcaaggtc actgtttctg tcccgctgaa ggccggcgac 780
   aagatctact ttggcaccgt gaattacacc accctttccc ccgccgccca gaaggcgctg 840
   aacgacaacc taaccaaggc cggcgcttcc atcaaggctt cctacggcta cggcgctgag 900
   caggtcatca tcaaccgccc ggcggagatc accgtggacg gcatggccaa gggctacaat 960
   gcctacatgg tccgcgagga cggctccctg cagagcctga gcgccaagtt taatgagggc 1020
   actggtctgc tgagctttgg cggcaccctg accggcccgg tcatcgtgac cgataaggca 1080
   ctgaccgcga ccaccaccag caatagcggc aacagcggct ccaatggctc caacagcaat 1140
   ggcggcagcc agaatcctgc caccggtgcc aatgacatcg taggcgtagc ggccgctctg 1200
   gggggtatca ccggcgtttc cgccgccgcc atttccctga agaaggacta a 1251
<210> 30
   <211> 1551
   <212> DNA
   <213> Unknown
<220>
   <223> Unknown gut bacteria
<400> 30
   atgttaggtt ctcaagtagt cattctgata accatagcgg tatacctgat tgccatggtg      60
   ctcatcggtg tatactttgg caaaaagggc agcggcaaca gcagcgatga cttttacctg     120
   ggcggccgca agatgggccc gctggtgacg gccatgagcg ctgaggccag cgatatgagc     180
   agctacctgc tgatgggtct gccgggactg gcatacctgt gcggcctgcc ggaggtttcc     240
   tggacctgca tcgggctggc gataggcacc tacctcaact ggcttatcgt agcgcggcgg     300
   ctgcggcgct attcggcccg cattggggcc atcaccgtgc cggacttttt ctcccgccgg     360
   tatggcgata agaaaaacct gctttccgcc attgcggcgg tggtaatcct ggttttcttt     420
   atcccctata ccgcttccgg ctttaaggcc atcggtacgc tgttcaacag cctgtttggg     480
   gtggattacc ataccgcgat gatcgtgggc gctattgtag tcattggcta tacagtgctg     540
   ggcggtttta tggcggtttc cttcaccgac ctgatccagt ccatctttat gaccattgcg     600
   ctgatcgttg tagtggtgtt tggcatccaa caggcgggcg gtgtggatac cgtcattgcc     660
   aatgccgagg cgctgcccgg ctacttgagc atgaccaagg gctatgatgt gactaccggc     720
   ggcgcggctt ccttcggggg gctcagcatt atctccacgc tggcgtgggg tctgggctac     780
   tttggtatgc ctcacatcct gctgcgcttt atggctatca aggaggaaaa gaagcttaac     840
   gattcccgtc ggatcgcgac cgtctgggtg gtgatctcga tgttcattgc ggtggctatt     900
   ggtgtgattg gttatagcgt ttcggtggcg ggcaaggtgc ccttcctgac caacagcagc     960
   gatgcagaga cgatcatcat taagctggcg gacctgatga gtcagcacgg taccctgctg    1020
   gccattgtgg cgggcgtaat cctttccggt attctggcgg cgacgatgtc caccgcggac    1080
   agccagctgc tgacagcggc ttccagcgtt tcgcaggatc tttctcagaa tgtgtttggc    1140
   atcaagctga gcagcaagac cgagatgatc gtggcacggg cgacagtact ggttattgcg    1200
   gccatcggta ttactctggc gtgggacccc aatagctcgg tattccgggt ggtttccttt    1260
   gcctgggcag gctttggcgc ggcttttggc ccggtggtgc tgttctcgct gttctggaag    1320
   cgcagcaaca agtggggcgc cctggccggt atggttgccg gcggcgtgat ggtatttgtg    1380
   tggaagtacc tgattgcccc gatgggcggc gcatgggcca tctacgagct gctgccggcc    1440
   tttgtggtgg cgtgcattgc cctagtggtg gtttctctgc tgaccgaggc cccggaaaag    1500
   gaaattgtag agacctacga aagcgtgtgt gccaagaagg taaaggcgta a             1551
<210> 31
   <211> 1146
   <212> DNA
   <213> Unknown
<220>
   <223> Unknown gut bacteria
<400> 31
   atgtcaaagc tgaataaacc gcaggccact gcccgtcccg cccgggaacc cttcctctat      60
   gtcacccgtc gggatcagat cccgctgggc cgggccattc tcatccgggt catcgccatc     120
   ctgctggcgc tcatcgtgtg cagtgtggtc acccgtgtcc tcaccgggga tgaccccctc     180
   tccatctacg ccaccatcct ccacggcgcg ttcggcaccg gccgtatgct ggtcaccctg     240
   catgatatcg ccattctgct gtgcatctcg ctggcggtca ccccggcctt ccgcatgcgc     300
   ttctggaata ccggcgccga aggccaggtg ctcatcggct gcctttccac cgcggtatgc     360
   atgctggtgc tgggcggcaa agtgcccgat ggtctgctca tcctcatcat ggcagtttcc     420
   gccatcctct ccggcgttat ctggggcatt atccccgcct tctttaaggc ccattggaac     480
   accaatgaga ccctcttcac ccttatgatg aactatgttg ccatccagct ggtggaatac     540
   ttcctcaagg tggcggataa gaccggctcc aatgttgtcg gccccgatct gctcacccac     600
   ggctggttcc cggaaatttt cggcgtcaag tatctgctca atatccttat cgtcgccatc     660
   gtctgcatcg ccatgcacat ctacctgcgg tacagcaagc acggctatga gatcgccgtt     720
   gtgggcgaaa gcgaaaatac cgcccattac atcggcatcg atgtcaaaaa ggtcatcatc     780
   cgcaccatgg ctctttccgg cgccatctgc ggcctggccg gtctgctgct ggtcggcggc     840
   tcctcccact ccatcgattc caacctcgtg gatggccgcg gcttcaccgc catcatggtt     900
   tcctggctcg ccaagttcga tcccttcacc atgatcctta ccacgctgct gctggtgttc     960
   ctggcccgcg gcgccgatga agtcacctcc aagtttggcc tcaacagcga tttttccgag    1020
   atccttaccg gcatcatctt gttcttcatc atcggctgtg agttctttat caattaccag    1080
   gtccatttca atcatcacaa aaaagaaaaa acggcgtcgg ccgccgtcag cggggagggg    1140
   aactga                                                               1146
<210> 32
   <211> 1200
   <212> DNA
   <213> Unknown
<220>
   <223> Unknown gut bacteria
<400> 32
   atgaacaaga aaatttcgct tggcgcggcc atcgcctata tggccatcgt agcggctgtg      60
   gccttttccc ttaccatgat ctactcgatg aacctgttca atgtgaagat gacaggcatc     120
   accgaacggg aaaaaatgta cagctctctg catgaggtag acctgtatgt gcgggaaaat     180
   tactacggct ccatcaatga ggaagcgctg caggaggcca ttgcccgcgg gtatatcgat     240
   ggcctggagg acagcaatgc ccgctacttc accgctgcgg aatatgaggc ctatacccaa     300
   tccggcagcg gcaaatatgt aggcgtgggt ctggtgaccg agatggattc ctccggctat     360
   ctgctggttc gggaggttta tcccgattct ccggcgcaga tggcgggcat caaggtgggg     420
   gcgcttatta ccaatgtaaa cggcacccgc gtgaccgaaa ataacaatga aagcatggcg     480
   ggccgcctgc gtggcgaagc cggcaccaag gtgactctga ccatccaaca ggatagccag     540
   gagaacaact acgagctgac cctgcgcgcc attgaggtgc ccacagtaaa aacgaccatg     600
   gtaagtgaag ataccgccta tatcagcttt accgagtgga gcggtaccac tgccgaccag     660
   ttcatcgccc agctgaatgc cttgaaggcc caggaagtaa agggcattct gctggatgta     720
   cgcggcgtta ccagcaatga gctttcctat gcggtccggc tgctggatcc gctgctgccc     780
   agcggtactg tggcctattc ccgcgctaag gacggcaacc tgaccgccgt ggcggaatcc     840
   gatgcgaaca gctgggatat tccgctggtg gtgctggccg atgagaccac cggtggcacc     900
   acggagctct ttgtgatggc gctgcaggac tttggccggt ccaaggtggt aggcgcccgc     960
   accgccggca acggcgatat caccgagatc ttcaagcttt cgggtggttc ggccatccag    1020
   gtcaccacgg cgcagtatgc cgggcccaag caaaagagct ttggtgatac cggtgtgggc    1080
   cccgactatg aagtggaact ggcagccagc gagagtaacc ggggtgacat cctgggcaac    1140
   gctgaactgg atgctcccta taagaaggca ctggaagtgc tggcagcggc catccggtaa    1200
<210> 33
   <211> 3576
   <212> DNA
   <213> Unknown
<220>
   <223> Unknown gut bacteria
<400> 33
   atgaagctga aagcggtgga aatgcagggg ttcaagtcct tccccgaccg cacaaagctg      60
   accttcgatg acgggatcac ggtcattgtg gggccaaacg gcagcggcaa aagcaatatc     120
   agcgacgcca tcaagtgggt gttcggcgag caatcagtga agagcctgcg cggcgccaag     180
   atggaggatg tgattttcgg cgggtccatc agccgcaagc cgaccggttt tgcatgggtg     240
   agcctgttta ttgataatac cgaccgcagc attgatatcg acagcgatga ggtggtcatc     300
   acccggcggc tgtaccgcag cggagaaagc gaataccgca tcaataataa cccggtgcgc     360
   ctgcgggata tcaacgagat gtttatggat acaggccttg gacgggatgg ctattcggtc     420
   attgagcagg gcaagatcgc ggagatcgtg agcgcgaaaa gcacccagcg gcgggagatt     480
   tttgaggaag ccgccgggat ctcccggttc cgctaccgca agaccgaagc ggagcgcaag     540
   ctgcagcagg ccgaggataa cctggtgcgc ctgcgggata tcatgcagga gctggaggac     600
   cgggtaggcc ccctgaaggc ccagagcgaa aaggccaagc agtatgtggt gctggccggg     660
   gaaaagaagt cgctggagat cagcctgtgg ctggagcagc tggccaaact gggccggcag     720
   ctggcggagc tggaggacaa gaccctgctg gctgctaatg accgcaccac cgcccaaagg     780
   cggctggatg agatcgagcg ggagctggcg gagatccagc acaaaacaca ggacgcgaac     840
   ctgtatattg agcataagcg cgagcgcatc aaggagtttg aggacgcgat ctccaacgct     900
   aaggtggagg cggcggtcaa gcagaatacc attgatcata ataacgatac catcgccgag     960
   ctggaaaagg agctggagcg cagcgagctt tccgccgggg agctggagga aaagctgact    1020
   gccctgcggg aggattacca aaaccgcctg gacgaggccc aaaagacccg cgaagccctg    1080
   gagggggggc aaaaggcgct ggaggctcag cggcaggagg agcaccgcct ggcggcggag    1140
   cagagcgcgc tggcgctgca aaaggaggag ctgcaggggc agatccaccg ggcggagctt    1200
   tcggccgaga cggcaggaac gctggccgag gaaacggtga cccgcctgga agcgctgcgc    1260
   ggacaggcca aggacaagga tgaaaatctg agccgcctgc gggaagaact ggccggacat    1320
   aaggcctttg ccgaagagct gcaggagcgg ctgcaaagcc tgcagaacag caagaacggc    1380
   tacctgtata agctgaaaag ccgcagtgat aagcaggccg aggcggagag ccgccagcgc    1440
   aacagcgaaa agctggcagg cgagaagctg cagcgtgcgc aggtgctctc cgacctggaa    1500
   aagaactacg agagctttaa taacagcgtt aagtttgtga tgaagcaggc cggcgcgggt    1560
   gccctgcggg gcatcgtggg tccggtatcc agcctgatca agaccgaaaa ccgctattcc 1620
   accgcggtgg agatcgcgct gggcgcggcg atccagaata tcgtggcgca ggatgagggc 1680
   gcggccaagc gagccatcgc gctgctgaag gagcggggcg ccggacgggc gactttcctg 1740
   ccgctgacca atctcaaggc gaacccggta agtgaaagcg acctggccgc ccgaggcggc 1800
   tatgtgggcc tggccagcga gctggtgcag tgcgatgacc gctaccgtgt ggtgatggat 1860
   tccctgctgg gacgcaccat tgtggcggag gacattgacg ccgcgactgc catcgccaag 1920
   gcgtacagct accgctaccg ggtggtgacg ctggatggcc aggtggtgaa tgccggcggc 1980
   agtcttaccg gcggttccgt caataagaac gcgtccattc tttcccgccg gggagagata 2040
   gacgccctga ccgcagaggc gaagaaatac gcaaagcagg cggaggagct ggaggcccag 2100
   ctgaccgagc tgcgccggga gaccgatacc attcaggcga cggtggacgg catagaggcc 2160
   gaggaaaaga ccgcgcggga ggatctcatt tccgcccaga cgctggtgga ccgcctggcc 2220
   gccaatgtgg aggaagccga gcgcctgaac gagcaggcgc tgcgggaata tgatgacctg 2280
   accgcccgta tggagcagct gcgccagcag aagatctccg gcggcgagct ggtgaagaaa 2340
   ctgaccgaag aggtggagcg gctgaccagg ctttcggccg agggtatggc tgcccacgaa 2400
   atgttgactg cggccatcac cgaagctgcc gaaaaggtga ctgccctgca gatggaggac 2460
   atcgcccggc agaaggactg cgaagccgtc cagatggcca ttgagcagat ggagggccag 2520
   caaagcggca gtgaggccgc cctggccgcc ttgcgggaac gacaggagca gctgaaaaag 2580
   gataatgagg ccatccggga ggagatcgag cagatcaccg ccggggcgca aagcggcagc 2640
   agcgagatcg aggagctgcg cagggatatc aaggacgccg atgcacagcg tgaccagctg 2700
   gaaatgcgcc gcaatatcct ctccaaggaa aacagcgatg tcatctcccg ccgggaggcg 2760
   gccgccagcg agctgctgcg cctgcaggaa aagagcgaag gtatgcagga gcagcagaac 2820
   agcatcagcg ccaagatgtg ggaggaatat gagctgaccc gcagcgaagc ggccgaacag 2880
   gccatcccgc tggaggatgt gggggcggcg caaaagcgcc tttccgagct gcgcagcaag 2940
   atacgggcgc tgggtgccgt gaatgtggca gccattgagg aatacgagga agtgagcggc 3000
   cggtaccgct ttatgaagga gcagatagac gacgccgaaa atgccaagaa ggagctgaca 3060
   cggctcatca ccgagctttc ggcccagatg agcgcgatct ttaccgagcg ttttgcgggc 3120
   atcaaccgct actttggcga gatcttccgg gagctctttg gcggcggaca tgccgagctg 3180
   cgcctgaccg atgccaccga tccgctggag acgggtatcg agatcatcgt gcagccgccc 3240
   ggcaagatca tcaagaatct ttccgccctt tccggcggcg agcaggcctt tgtggccatc 3300
   tgcatctact ttgccatcct gcgggtcaat cccgcaccgt tcgttctcat cgatgagata 3360
   gaggcggcac tggacgatgt gaatgtcggc aagtttgccg cctacctgca ccgcatgacc 3420
   gatcataccc agtttatttc catcacccac cgccgcggca ccatggaaca gggcgatgtg 3480
   ctgtacggcg tcaccatgga ggaggagggc atcagtaagc tgctgcggct ggatgtggcc 3540
   gagatggaaa agaaatttgg caagagcctg aactaa 3576
<210> 34
   <211> 1014
   <212> DNA
   <213> Unknown
<220>
   <223> Unknown gut bacteria
<400> 34
   atgagtaaag tgttcattcc tgcaggttac cacagctgcc tttccctgta cgatacccaa 60
   aaggccatca gcctgatcaa gcgggtgttt gaggatactc tctgcggaaa tctgaatctc 120
   agccgggtat cggccccgct gtttgtggag gcttccaccg gcttgaatga tgacctaaat 180
   ggagtggagc gccccgtttc ctttgatgtg ccggatatcg gccgggatgc acaggtggtg 240
   cactccctag ccaagtggaa gcgcctggcg ctgcaccgct acggcttccg tcccggcaag 300
   ggcctttata ccgatatgaa tgccatccgg cgggatgaaa cgctggataa cattcactcc 360
   atctatgtgg accagtggga ttgggaaaag gttatcacgc cagaggagcg taatgtggcc 420
   tatctgaagg ctacggtaca gagtattgtg gatgcgatcg tttccactca gcgcaccctg 480
   cgggccatat atccgcagct gaatacggtt ccggacctgg tgccggaggt caccttcatc 540
   accacacagg agctggaaga ccgttacccg gagctgaccc ctaaggagcg ggaaaatgcc 600
   attgcgaggg aatgcggcac tgtattccta atgcagattg gcggtaagct gcgcagcggc 660
   cagccccatg atggccgtgc cccagactac gatgactgga ccctaaatgg tgatctgctg 720
   gtttataacc gagtgctggg aacagcattc gagctgtcct ccatgggcat ccgtgtagat 780
   cctgcggcac tggaccgcca gctgaccctt tccggctgtg aaaaccgccg tacattgccc 840
   ttccaccgga tgctgctggc cggaaagctg ccgctgacca ttggtggcgg tattggccaa 900
   tcaagactgt gcatgttcct gatggggacc gcccatatcg gtgaagtgca gtcttcggtg 960
   tgggatgacg ataccatgca gaaatgcgaa gccgcgggta ttatcctgct gtaa 1014
<210> 35
   <211> 939
   <212> DNA
   <213> Unknown
<220>
   <223> Unknown gut bacteria
<400> 35
   atgcacatta cccctatgaa ccccaaagca aatcccccta ctgtttccgc ttcccagtcc 60
   cattcccgtc tgcttgccca cctgatggcg ctgatggttt ccgccatttg ggccaccacc 120
   tttgtttgca gcaaggagct ccttgcctat tacagcccgg cgcaggtgat gttcatgcgc 180
   tttgccattg cctactttgt gctgtggctg ctgcgccctc ggccgctgcc gtggcagggc 240
   aggggagagc tcaccttcct gctattgggc attttgggcg gcacgctcta tttctttacc 300
   gaaaacaccg ccctgaagca cacctttgcc gccaatgtca gcatcattgt ggcgatggcg 360
   cccatcctta cctccatcct ggcgcacttt tttaccaggg atgaaaagct ccacaccacc 420
   gtttggatcg gctttgccgt ggccatgagt ggtgtggtgc tggtggtgct caacggtgcg 480
   ctggtgctca agctcagccc ggtaggcgat ctgctggcct ttttggcggc agccagctgg 540
   gcctgctatt cagtccttat caagcggctt tccggccggg tggacagcac gctgctggcc 600
   cgccgggtca tcctgtacgg cgcggttacc tcggtgccgc tgctggctgc ccgcggggag 660
   ctttcaatcg actttacccc gctggcacag ccaaagccgc tgttctgcat cctgttcttg 720
   gcggtgctgg gcagtgccgt ctgctttgcc gtgtggaatg ttgtcatcga ccggctggga 780
   gtcgttcacg ccaataacta tatctatctc cagcccttta tcactatagt ggcctcccat 840
   ttcctgctgg gggaacccat gagctggacg gccattgtgg gggcgctgct tatcattggc 900
   ggcgttatca tctccggccg gcaaaaacgg ccgcagtaa 939
<210> 36
   <211> 1431
   <212> DNA
   <213> Unknown
<220>
   <223> Unknown gut bacteria
<400> 36
   atgagaaagt ttgataccaa ggtgcaatac ctgaaataca aggtgctgcg ggaggtggcc 60
   cggcaggcct ggaatgatac cctgctggat aatgtcatgt ccattccgcg ggagctggcg 120
   ccccgcaatg ccgagaccat ctactgctgt gtctacaagg agcgtgccat cctgagcgaa 180
   cgggtacgca tggccatggg cggcaataag cagaacccca atgtcatcga ggtcattgat 240
   atcgcctgcg atgagtgtcc catgggcggc tatgaggtca ccaattcctg tcgcggctgc 300
   ctagcccgcc ggtgcgagga tgcctgcccc aagggagcca tcacattcca caaccatgtg 360
   gctcacatcg ataaggccaa gtgtgtggac tgcggtgcct gtgcccgggt ctgcccttat 420
   tccgccattg tagaccgcaa gcgtccctgc gagaacgcct gcaagatcaa ggccatcacc 480
   atgaatgaga ataaggccgc cgctattgat aacaatacct gcatttcctg cggtgcctgc 540
   gtctatcagt gcccctttgg cgccatcacc gataaatcct atatcctcaa tgttattgat 600
   atgataaaaa agagcgggga caatgagaaa taccatgtct atgcggtcat tgccccctcc 660
   atctccagcc aattcaccta cgccaagctg gggcaggttg tcaccgggct gcatcggttg 720
   gggttttact cggtggcgga ggccgccctg ggcgccgata tggtggccga tgccgaaagc 780
   cgcgaactgg tggaaaaggg ctttctgacc agctcctgct gccccggatt tgtcagctat 840
   attgaaaagg ccttcccgga attgaaacct tatatctctc acaatctctc ccccatggcc 900
   accattgccc gctatctgaa ggagcatgat gcaacggcca aggttgtctt tatcggtccc 960
   tgcacggcca aaaaggggga ggccgccaag caggaagtcc gcccctatgt ggatgccgca 1020
   atgacctttg aggagttaca ggcgcttttt gacagccggg aaatcgatat cactgcgctg 1080
   gaggaaggaa tgctggatga ggcctcctac ttcggacgca tttttgcccg ctgcggcggt 1140
   ttggcggaag cggtggccca agggatcaag gagcatggcc acaccgattt cgagctgaaa 1200
   gcaacctcct gtgacggtat tgaacagtgc cgcattgccc tgttcaagaa gagtaaaaag 1260
   ctgctggatg ccaatttcat tgaaggaatg gcctgcaccg gtgggtgcat cggtggggca 1320
   ggatgcctga cccacggccc ccgcagccgt gccgaggtag ataaatacgg gatggaagcc 1380
   aacgataaaa ccattaccga ggcgttggca gctctggagc ctaagcccta a 1431
<210> 37
   <211> 951
   <212> DNA
   <213> Unknown
<220>
   <223> Unknown gut bacteria
<400> 37
   gtgcggctga aaccgaatac gctgattgta gtggcggggc ccacggcctc cggcaaaaca 60
   gcgctggcca tccaaatcgc gaaagcggtg gatggagaga tcgtttccgc cgatagtatg 120
   cagatctacc ggggagtgga cattgccacg gcaaagccca ccccggagga gctggcggcg 180
   gtgcctcacc accttatcgg ctttttgccg atggatcggg ctttttcggt ggcggattac 240
   gcggagttgg cccgcaagga gatcaatgat attctaagcc ggggcaaggt gccggtgctg 300
   tgcggcggta cggggcttta catcaaggcc attgtggaca atgtgcagta cagcgaggca 360
   atcaccggcg atgaagcgct gcgggagcgg ctgcggcagc tggcggagcg ggagggcaat 420
   gatgtcctgt ggcggcggct ggaagcaatg gaccccgaaa cggccaagcg tatccacccc 480
   aataatgtgg gccgggtcat tcgggccatt gaggtgatga cggtcagcgg aaagtccatc 540
   cgggaacacg aagccgaaag cctgcgggag ccatgtccct atcatgtcat tgagctgggg 600
   ctgcgctatc acgaccggga aacgctgtat ggacgcatta accggcgggt ggataaaatg 660
   gtggagatgg ggctgactga ggaggtaaaa gctgcccggg aacagggcct gaccgctacc 720
   gccgcgcagg ccatcggctg gaaggagctg tatagctggc tggacggcaa agaaacccag 780
   gaggaagcgc tggaaaagct gaagcaaagt acccgccgct acgctaagcg tcagctgacc 840
   tggtttggga cggatcagcg gatgcgctgg atcgagctgg acacgctggc cgcgggggaa 900
   acgccgctgg ataaggcaat ggagatactg gaaaaggagg gaatggcatg a 951
<210> 38
   <211> 768
   <212> DNA
   <213> Unknown
<220>
   <223> Unknown gut bacteria
<400> 38
   atggggaaaa tcatcgcgat catcaaccaa aagggcggcg tgggaaaaac cactacggcg        60
   gtcaatcttt cggcggcgct ggggcagctg ggctgccgtg tgctgctggc ggacctggac       120
   ccgcagggga acgccaccag cggcgttggg atcaataagc gggaagtgaa ggtttccagc       180
   taccatgtgc tgatggagga gagcggcgcg gccgaggcca tcctgcccac acggttcaag       240
   gggctttcca tcctgcccag caatatagac ctggccggcg ccgagataga gctggtggaa       300
   aaggaaaacc gggccaaatc tctgcgggcg gcgctgctgc gggtaaagga ggactatgac       360
   tttatcttta ttgactgtcc gccttctttg gggcttatca ccgtaaatgc cctgaccgcc       420
   agcgattcta tcctcattcc catccagtgt gaatactatg ccctggaggg tctgacccag       480
   ctgatgcaga cggtgcgtac cgtcaagcgg ctggataata gcgccattgc cgtggagggc       540
   gtgctgctga cgatgtatga cagccgcctg aacctgacca atcaggtggt gggggagatc       600
   aagaagtact ttgcggataa ggtgttccgc tccacggtgc cgagaagtgt gcgtatctcc       660
   gaagccccca gctatggcga gccgatctgc tactacgata aggcaaaccg cggcacgctg       720
   gcctaccagc gggtagcgga ggagctgctg gagcgcaacc ggaagtaa                    768
<210> 39
   <211> 564
   <212> DNA
   <213> Unknown
<220>
   <223> Unknown gut bacteria
<400> 39
   aagcgcatgg ggagaccatc actggcggag caacgccgtg tggaaatatg ccgggcgctg        60
   caaagctgtg tcatccgcaa cggatcgtat gagatgacct cggtgaagga cattgcgcag       120
   gaggcgggta tggccgccgg aatgctgcat cactattttg agagcaagga tgatattctg       180
   ctggtgacgg cggaaatggt gctgctggag ctgcagaacc acctgagcga cctgctgcgc       240
   atcaaggatg ccgcggagag agaaaagatg ctggaggagc tgctgggcga taacgagcag       300
   aaccggtttt acatcatgct gcaggccatg gctctggcca tgccggcggt gaatgagctg       360
   ctgcttggca agcaggagga gatgcgggat atcctggcag aacggctgga attccgcggg       420
   agtgacccgg aaaaggccaa agccgatgcg gatcggatga ccttcctgct gtgcagcgcg       480
   ctgaccaccc ccaagggcgt acagagcgcc gccgcgcggc agatgacagc ggatatcctg       540
   caggagcttt tcccattggg ctaa                                              564
<210> 40
   <211> 1404
   <212> DNA
   <213> Unknown
<220>
   <223> Unknown gut bacteria
<400> 40
   atggaaccta aaagagccca atggggcagc aagatcggct ttattctggc ggcagccggt      60
   tcggccgtcg gcctcggaaa catctggaag ttccccggca aggcttacga aggcggcggc     120
   ggagcgtaca tcctcatcta ccttgctatc gtactcctca ttggcatccc ggtcatgctc     180
   tcggaactga ccatcggccg cggcgcgcag gccaatgcca tcgatagcta cggcaagctg     240
   ggccaccctg gctacaagtg ggtcggctgg ttcggcgtta tcgtggcgtt tatcattacc     300
   agctactact gccatgtagg cggctgggtg ctgagctatg tggccagcta cgccaccgag     360
   cccaccaagg tttacgccga tccgctggga tatttctatg cgctgctggg ctacaatgcc     420
   gtcaccgggg aaacatggtt cccactgact gcagttatct tcgcgggtat ctttatgggc     480
   attacctgct tcatcatcgt caagggcgtg gagtccggca ttgagcgctt taataagatc     540
   ggtatgccgg cgctgtttgt catcctcatt attctgctta tccgggcgct taccatgccc     600
   ggcgccgggg aaggcctgca atatatgctg attccggatt ggagcaaagt caccttcagc     660
   accgtgcttt cggccctggg gcaggcgttc ttctcactca gccttggtat ggctattatg     720
   gttacctacg gctcctatgt taaaaaggaa gaaaacctcg ccaaaaacgc cgctatcatc     780
   tgtggtatgg ataccctggt agccctgatt gccggcttta tcattgttcc cgccgtattt     840
   gctaccctcg gcgcagaagg cgttggcaag ggcgcaggct ttgcgttctc ttcgctggcc     900
   ggtgtgttcc agcagatgcc cggcggtcag ttcttcggca tcctgttcta catgctgctg     960
   ctgttcgcgg cgctcacctc ctgcatttcc ctagtggagg gcgttgttgc cttcgctact    1020
   gagcgctttg gatggaaacg cacccccacg accatcattc tgtgcgtggt gatgttcctc    1080
   cttggtacca tttatacctg ctctcaggcc gcctttgata tcaagggtat ctggttcgac    1140
   ttcaagaacg gtcttaccac ccccatcttc ggcgacttta tggagttcct caccgaccgt    1200
   ctgatgattc ctgtgtgcgc actaggttcc tgcctgtttg tcggttgggc ctggaagccc    1260
   caaaacgcca tccgcgagat cgagctggac ggcaagccct tccgtctgaa gaaggcatac    1320
   agcttcctgg tgaagtatct ggctcccatc agcatcatta tcatcatcgt ggccagcttt    1380
   gccaccggca ctaccctcag ctaa                                           1404
<210> 41
   <211> 1032
   <212> DNA
   <213> Unknown
<220>
   <223> Unknown gut bacteria
<400> 41
   atggagctgg atcccagaaa aagcaggatc ctcacctgcg tggtggaaag ctacatccgc      60
   accggcgagc ccatcggctc caagatgatc gccgccatgc tggaggagac tgtctcctcc     120
   gctaccgtcc gcaatgacct ggcctacctc agcgagcagg gctacatcga gcagccccat     180
   acctccgccg gacgcatccc cactcaccgc ggtttgcgct actacctcga tcacctgatg     240
   aagccgcagc cgctggagga cgccgcccgc ggcgagattg acgccctgtt caattactgc     300
   gaccccgacc ccgaccgcgt gctggaaggc gccacccgga tgctggccaa ggaaaccggc     360
   tgctttgcca tttccaccac catctcccgc cagagcctct ttgtcaagcg cattgagatc     420
   attccggcca cggcccacac cgtcgtcatc ctgctgctgt gcagcaatgg catggtgaaa     480
   aacaaggtct gccgggtcaa tttcaatctg acccccaatg tggtggactt cttccagagc     540
   ttcgccaatg gccgtctggc cgggcgcagc ctcaatgaga tcaccgccca gtacatcaat     600
   tcgatggcct ttaccctggg tgagcacacc gatatctttg cgccgctttt ggccgctatt     660
   tacgagctgt gccgggaagc ctgcgaaggt gccctgtacc gcagcggcac ggggaatctg     720
   ctggaacatc cagagctgag aaatgtggcc gaccggctgt accgcctcct cgattcccgc     780
   gaggaaatgc tgcggatcat cggcagccgg gaaaatgacc tgtttgtcag cgtggggcgg     840
   gaaaatgccc gccccgaaat ggagaattcc tccatgatcg ttgcccgtta ccgcatcggt     900
   gaggattcca tcggcgccct ggggctggtt ggtcccgtcc gcatggatta cgcccggctc     960
   atcccccatg tggagtactt tgccgggatg ctgagccgga tgctgagcga tacactgaat    1020
   aacggtctgt aa                                                        1032
<210> 42
   <211> 870
   <212> DNA
   <213> Unknown
<220>
   <223> Unknown gut bacteria
<400> 42
   atgaaccgaa ctgaccgtcc tgcccgctgt gccgctgtgc acgatctttc cggcctgggc      60
   cgctgctccc tttccgttat tctgccggcc atgtcggtaa tgggggtgca ggtgtgcccg     120
   ctgccgactg cggtattatc cacccacacc gggggctttg gcaccccggc ccgtcgcgac     180
   ctaaccgact acctgaccga agcgctggcg cattaccgta cactgggtct gcccatggag     240
   tgcgtctaca ccggctattt gggctcggtg gcacaggttg cgctgtgcca ggggctcttt 300
   gcggaatggc ccgcggccat gaaggtggtg gaccccgtga tgggcgacca cggcaagctg 360
   taccacggct ttacgccgga actggtacag ggtatccggg agctggccac ccatgccgat 420
   gttatcaccc ccaacctgac cgaagcagca ctgctgctgg gtgaggagcc gaatacggcc 480
   cccctttcgc tggggcaggc ccgcagcctg ctggcacggc tttcggccct ggggccgggg 540
   gtagtggtta tcaccggggc agcgcttacc accggggaat atgccaacct gtgttatgat 600
   cgggaacgcg gcggctactg gaaatcggta agcgaatacc tgccggtgcg ttatccaggc 660
   acgggcgatc tgtttgccgc agtgctgaca gccggcctga tgcagggcga cagccctgct 720
   atggcgatgg accgggcaac gaggtatgtg gagatcaccg taaaaacgac ctttggctac 780
   ggcgccgatt ctcgcgaggg agtaatgctg gagcgcacac tgggctggct gacccaaaag 840
   gagctgccgc gaggctaccg cacgctgtaa 870
<210> 43
   <211> 1818
   <212> DNA
   <213> Unknown
<220>
   <223> Unknown gut bacteria
<400> 43
   atgcccggag gagaaaaaga catggatatt tttgcgcttc tgcggctggt actgggcctg      60
   gcctttttcc tgttcggtat gaatgtgatg tccagcaacc tggaaaagat ggcgggcggt     120
   aagctggagc agctgctgaa aacaatgaca gcgagcccct gggtgagcct gctgctgggc     180
   gcggccatta ccatagcggt gcagtcttcc tcggcggtca cggttatgct ggtgggtctg     240
   gtcaattccg ggatcatgaa atttggccag acgctgtatg tcatcttcgg cgcgaatatc     300
   ggcaccacgc tgacggcgtg gatcctctcc ctttccggca ttcaaagcgg gaatgtgtgg     360
   ctgaagatgc tgaagcccga aaatttctcg cccattttag ccatcatcgg cgtagtgatg     420
   atgatggtgt gtaaaaacga taagaaaaag agcattggta cggtgctggt gggctttaca     480
   gtgctgatgt acggcatggt ggtgatgagc gacgcggtaa gcccgctggc ggagatgccg     540
   gagttttcgg cgatcctagc ggggctgcgt aacccgttac tgggcctggt ggtggccacc     600
   ctgtttaccg ccgtgatcca aagctcggcg gcctcggtgg gtattctgca ggcgctggcg     660
   ctgaccggca gcctgaccta tgagatggtc attcccatgg tgatgggcct gaacattggc     720
   acctgcgcca cctcgctgat ttccagcatt gggacaagct acaaggccaa gcgggtggcg     780
   gtcatccact tttccaccaa ggtcattggt actgttatct gcctgccgtt gtacctgttg     840
   atcacctcgg tgcttcagct tgattttatc catattgcag tgacaccatg gaatattgcc     900
   atggtgcata ccatctataa cctggctatt accgccattt tgatgccatt taccaagtac      960
   ctggtgaagc tgggcaagtg gctggtgaag gccaaagacg aaacggcacc taaggactcc     1020
   atgcagtacg cgcctgacct gctgctgctc cgctcccctt cggtggcgct gcaggagtgt     1080
   gaccactata ccttccggat ggccgggacg gcaagggatt cgctggaacg ggcgatctcg     1140
   ctcatcggcg cctacaatga gcaggacgcc gaggtggtac tgaagcagga ggacacgctg     1200
   gatctgtatg aggaccggct ggatacctac ctggtgtacc tttcggcgca ggcgctttcc     1260
   cagcaggaca gccgccgcgt ggggaaaatg cagcacgcca tcggggactt tgagcggctg     1320
   ggcgaccacg ccgtaaacct ggtaaaggcc ggtaaggagt tggagatcaa atcgctgttt     1380
   ttctcggagg aggcccggcg ggagttgggg gtgctttccg ctgcggtgcg ggagattttg     1440
   cagttgacaa ccgattgcta tatcaatacc gaccatgaga tggcccacca tgtggagccg     1500
   ttggagcagg tcattgacgc actgaccaag gagatcaagc agaatcatct ggcgcgcctg     1560
   caggcgggcc gctgttccat cgagctggga tttatcctaa ccgacctgct gactgactac     1620
   gagcggattt ccgaccactg ctccaatatt gcggtggcgg tcatcgagct gcagcacgac     1680
   gcgctggact cccaccgcta cttgaacgaa atgaagcgtg acagcgatgc gttccgggag     1740
   atgtatgtga aattcaggga gaaatataca ctgccggaat cggcctatat tgccagacag     1800
   gcggcggaat accgctga                                                   1818
<210> 44
   <211> 1179
   <212> DNA
   <213> Unknown
<220>
   <223> Unknown gut bacteria
<400> 44
   atgatcgatc tgtttgaaaa atgccgcaaa ccgtccctgg ccagcgagct caaggaaaaa      60
   ggcatctacc cctacttcca cgcgctggaa agccgccagg ccccggaggt catcatggag     120
   ggccgccgcc gcatcatgct gggctccaat aactatctgg gtcttaccac ctgcccggag     180
   gtcatcgaag ccgggatcaa agcgtttgag cagtacggca ccggctgctc cggctcccgt     240
   ttcctcaatg gtacgctgga aatgcatttg gagctggaag cggagctcgc caaattcctg     300
   cacaaggagg ccgtcgtcac cttctctacc ggcttccagt ccaatctggg catcatcagt     360
   gccattgtcg gccggggcga tgttgtcatc tgcgataagg aaaaccatgc cagcatctac     420
   gatggctgca aattgagcta cggcaagatg ctgcgctttg accacgccga tatggaggat     480
   ctggagcgcc agctgcaaaa ggtgccgcaa aacgccggct gccttatcgt caccgatggt     540
   gtgttcagca tgggcggcga tattgccaag ctgccggaaa ttgtacggct ggccaagcag     600
   tacggcgccc gtgtcatggt ggatgacgcc cacggccttg gcgttctggg cgaaggcggc     660
   cggggcacgg ccagccactt tggcctggaa gatgaagtag atatctacat gggcaccttc     720
   agcaagtcgc tggccagcct ggggggctac atggcctcct cccatgaggt cgccgaatat     780
   gtccgccatg cttcccggcc gttcatcttc tcggcttcca ttacaccggc ttcctgcgcc     840
   accgctttag cggcgctgcg ctacctggag gcccaccccg agatcgtgga tcgtcttaat     900
   gccctttcgg cctatgcccg ggacggcctg cggaaaaaga aggtcaagat cattgaatcc     960
   accaccccca tcatcccgct gtatacctac accgtggaaa acaccctgac cgccgcccgc    1020
   cagctgtatg atgccggggt ctatgtcaac ccggtgctgc cgcccgctac cccccctgcc    1080
   gagtgtctgc tgcgcaccag ctacatggcc acccttaccg aaccgctcat cgatgaagcg    1140
   gtcgagatca ttggcagcgt gctgggggaa aaagtatga                            1179
<210> 45
   <211> 1083
   <212> DNA
   <213> Unknown
<220>
   <223> Unknown gut bacteria
<400> 45
   gtgaacggca tgatccccaa aaaagaatgg gcgcaggaca ccctgcggat gctgcttcta        60
   ttgggggcag cggtggccgt atgcagcgtg ctttcccgcg tgcacgatga caacaacccc       120
   tttgccgttc tcatctttgt gctggctgtc gccatagtgg cactggtcac caacggctat       180
   gtgtggggtg ttctggcctc ggtcatcggg gtgttctgcg tcaattacat gttcacctac       240
   cccttttgga gctttgatat gagcatcacg ggttacccgc tcaccttcgt ggtcatgctg      300
   gtggtctcgg tggtcatcag cacccttacc actcgggtaa agcaaaccga acgcctgcgg      360
   tatgagatcg aaagcgaaaa gcttcgggcg gatcttttgc gggctctctc ccacgatctg       420
   cgcacaccgc tcgcctccat tgagggtgcc gacagcctgc tgctggagca gcccgacctg       480
   agcgaagcgg agcgccgtac cctgctggag cagatccgtc aggagacccg gtggctgacc       540
   cgcatcaccg aaaatatgct ctcggtcacc cgtatggccg gggaacaggt aagcctgcac       600
   cgcaccgatg aggtagtgga ggagattgtg gacagcgccg tgatgaagct gcggcgcagc       660
   cgcaatgccc tgccggtcac ggtcatccgc ccgcaggaga tcctgaccgc cccgatggat       720
   gccaccttga tggagcaagt cattctgaac cttttggaaa acgcggtgaa ccacgccgag       780
   ggcgctaccc gcatccgggt ggagatcgcc gggcaggggg ataccgtgat gatcgctgtg       840
   gaggatgacg gcgcgggctt ccctccagag ctgctccccc agctttttga ccgcaagctg       900
   ctgccgcagc cgcgctcctg cgctgatggc cgccggggac tgggactggg gctcacgcta       960
   tgcagcgcca tcataaaagc acacggcggc cgcatgaccg ccgaaaaccg ccccgagggt      1020
   ggagcgcggg tgtgcctgtg gctgcctgcc ggaaccgaag atgaggagga aaccgaagga      1080
   tga                                                                    1083
<210> 46
   <211> 1086
   <212> DNA
   <213> Unknown
<220>
   <223> Unknown gut bacteria
<400> 46
   ttgttcaccg aaagtccatt gaattgcccc cgggggtata ctataatatt cggtagagtt 60
   ccgataagga ggacaatttt tgtgaaatac accggtatcc cctgcgcggc ctgcggcaaa 120
   aagtttaccg cggaagacga tgtggtcgtc tgcccggagt gcggcacgcc ctaccaccgc 180
   gcctgctata aagaactggg ccactgtgtt aatgaggccc gccatgccga gggctatgtg 240
   tggcagccgc ccaaggccgc tccggggcct tctgtgccgc tggaggagca gccgcatgcc 300
   ccccagaaca agacggacgg cgaaggctat gtgatgtgca gccgctgcgg caccgtgaac 360
   cctgccggac aggagcgctg cgacctgtgc ggctacccgc tgaaggagac cggcgaacgc 420
   atcccggctg gggaccgcga gaccgaggac ggcgggacct ttgcggaata tgtgcgcgac 480
   cagtacaatg tgaaccccaa tgaaaagctg ggcgatgaac tgactgcccg ggaggtggcc 540
   gcctatgtgg gacccaatgc cctgaacttc ctatacaaat tccgggcgat gctgcagcgc 600
   aagaccgctg tgagctttaa cctgggggcg ttcctgttca ccgggctgta ctgcttctac 660
   cgcaagatgt atgccctggg cggcattctg ctggccataa aggccgcctg ctatatcccc 720
   tttgccatca attatatccc ctacttccag gagttgatgg ccgcgggggc caccaccctc 780
   agcgagttta tgcacatcaa taccctttcc ccctactacc aggcgctggt ttccacctcc 840
   gccattgtgc agtacggcgg gctggtgctc agtgtgcttt gctccctgtt cttcaattat 900
   ttctatctca agaagatcac ccagcaggtc cgcatccagc gctaccgcgg ccacgcagcg 960
   gccggtaccc agcagtacta ccagaacctt tcccgcatgg gcggcaccag ttggctgctg 1020
   gtattcctca tcgtcatcgg gctgctttcg gtctcttctg tcgtcacttc cctttttgtg 1080
   atataa 1086
<210> 47
   <211> 1239
   <212> DNA
   <213> Unknown
<220>
   <223> Unknown gut bacteria
<400> 47
   atgcagatgc tcattaagaa cgcgaagctc cgtgaccggg aagagctggt caatatcctc 60
   atcgatggcg gcaagatcgt tgatatcggc gtgggactga ccgccgaggc cgagaccgtt 120
   attgacgccg agggcaacct gaccaccgct tcctacatcg atccccatat ccacctggat 180
   aaggtcaatg tactggatgt tctgcccaag aaccagagcg gcaccctgaa ggaagccatt 240
   gagatgctgt gggataaaaa gcgcaactat gtcaatgagg acattctgac ccgcggcggc 300
   gatgtggtgg agcgtgagat caagaacggt gtactgaata ttcgtaccca cattgatgtg 360
   gataccatca ccggcctgaa ggccaccgag ggcgtactgg ccctgaaaga caagtataag 420
   ggcgtagtgg atatgcagac cgttgctttc ccgcaggaag gcattatgaa ggatcccggc 480
   gccgataagc tgatgtggca ggccatggag atgggctgcg atgtagtagg cggtatgccc 540
   gctaacgaaa actgccctga cgacagccgc gcacatgtca agctgtgctt tgacatcgcc 600
   gaaaaatacg atgccgatat agatatgcat gtggatgaaa gcgacgaccc cttctaccgc 660
   acgctggaga tggtggccga tgagaccatc gcccgcggct ggcagggccg tgttaccgcc 720
   ggccatacct gcgccatggc ggcctatgac gaccactatg cggcctatgt tatcgaaaag 780
   gtgaaaaagg ccggcatcaa tgtcatcacc aaccccgtga ccaacctgat gctccagggc 840
   cgcttggata aacagcccat ccgccgcggt attacccgcg taaaggaact gctggctgcc 900
   ggtgtgacca tgagctttgg ccaggactgc gtcaatgata ccttctatcc ctacggcgcc 960
   ggcgatatgc tgcaggtggc cctgattacc ggccacgctg cccagctgag cctgcccgat 1020
   gagattgaaa agaccttcga tatgatcacc gatgacgccg ccaagatcct ccgtctgaag 1080
   gattacggct tgaaggtcgg ctgcaatgcc gatatcgtca tcaccgcttg ccgtgatgca 1140
   cgcgacgcca ttcgtctgac tccggagcgt cgctatgtca tcaagcgcgg acgcgtagtg 1200
   gcttccacca ccgtccagtc taaactcgcc atcttctaa 1239
<210> 48
   <211> 1221
   <212> DNA
   <213> Unknown
<220>
   <223> Unknown gut bacteria
<400> 48
   atgctatact ggctccataa accggcatac ctgccgggaa ccggcaacaa acccgagaag      60
   aaggagaaca ttatggcaga aaccaccaaa gaaaacaaag cccagcgggc caagaaatat     120
   cgccgcagaa gagtgactgc cgcagtagcc gcagccgcga tgtgcgcggt catcggtgtg     180
   ggggtaaacg gcgtagtgac cgcccgagcc aataccgccc tggcggatgc gctggctacg     240
   gccgccgatc tgcagcaggc gcagcaggaa agcgcccggc aggccctgcg ggaaagccgt     300
   actgctgccg aggcagacct gaccgcctat gtgactgaag ccagcgaaga ggctgaagcc     360
   gccctgaaaa aagccgctga ggaagccgcc aaggcggccg cggccaaggc cgcccagacc     420
   accaacacct ccaccaatga cagcaccgag ctgcccggca acggaggaaa actgagcagc     480
   ggtggccggg ctcttgccgc caccaaaacc ggcgggaact ttaaggccag cgtttatacc     540
   ggcaataccg gtgcttccaa cctgagctac taccagtcac tgaacagcga tgtgaaggcc     600
   tggcttaaga tccccggcac caacatcaat tacccggtgc tgcagaatgc cagcgaggac     660
   cactactacc tgcaccgcgg cctggaccgc ggccagagct actacggtgt gctgtggacc     720
   cagaccgcga ctcgctttgg cagcggcgac agcctttcca gcaacaatgt catctatggg     780
   cacaactgga agaactgtcg ctggaacgct gccccttcca cctactatgc cggtcagcag     840
   atgtttgaat cgctgctgag ctaccactac accagctggg cggagcagta tccctacatc     900
   tactattcca cccccagtga ggaaatggcg tttgttatct ttgcctgctt ctacaccgag     960
   ggcagcgatt ggtatatcaa tgccgagggt aatattgacg gtgtgatcag cggcgcgcag    1020
   agccgctccc gccacaattt tggtgtggat gtcaactcca gcgataagct gctgaccctt    1080
   tccacctgca cccgttacta cggcaatacc gataaccagc gctttgtggt gatggcgcgc    1140
   ctgctgcgcc cgggcgagga gatgggccct gtttccgtca cctataatcc caaccaccag    1200
   cagcccaatg tatggggcta a                                              1221
<210> 49
   <211> 1143
   <212> DNA
   <213> Unknown
<220>
   <223> Unknown gut bacteria
<400> 49
   atgaaaaagc agaatattat cattatcatc gtttttggcg cgctgatctt tgggctgggg 60
   ctgctggggt ggctcacccc ggatcaagcc ttttccgaaa atgagaaccg ctacctgcag 120
   gagctgccaa agctttccga agataccatg gtaaatggag attttggcga tgaggtggag 180
   gattatttaa gcgatcagtt ctggcagcgg gatagctgga ccgccgcccg cagcaaaatg 240
   aaaatgctgc tgcgcaacaa ggatatcggc ggggtgtacc tgtgtgagga tgggtattac 300
   atcgaaaagg tgacccaaac cgatgtggac gaagcaaggc tgcaaaagaa cctgcaaacg 360
   gtgaaggatt tctttacccg gtgcgaggaa aaaggactgg cggcagaaaa tctgaccttt 420
   atgccggtgc cgacccccgg ctataccctg cgggataagc tgccgcgtta tgccaccctc 480
   tttgatgagg atgcggtatt tgacgagatg aagctggtat ttgatgacca gctggtggac 540
   ctgcgaacgg actttgccaa ggcggcagag agtgagcagc tttattaccg caccgaccac 600
   cactggaaaa cggcaggcgc tctgctgggc taccagtgct accgcgcagc catggggctt 660
   tcgattcccg cagcggcgga ttacaccgta gagcggtatg agggcttccg cgggacgctg 720
   tacagcaagg tgctggataa aaacgcggcg accgataccg tagagctgta ccaccggaaa 780
   ggcgatgccg ccttgaccgt gacctacgat aacgccgatc atgccagctg ttatgataca 840
   gaaaaactcc agcaaaagga tatgtacgaa gtgttctttg gcgggaactg gcccgcggtg 900
   accattaccg gcggaccgga aaatggccgc cgcctgctgg tgctgaagga cagctatgcc 960
   aatgccctgc tgccctttgc cgcagcggat tacgaaaaga tcgtggcggt ggacctgcgg 1020
   tattatcttg gctccctgag tgagctgatg gagcaggaag gcattaccga tgtgctggtg 1080
   ctgtatagta cctccgcctt tatgagcgac accaagatag accggctggc actggcaaaa 1140
   taa 1143
<210> 50
   <211> 1602
   <212> DNA
   <213> Unknown
<220>
   <223> Unknown gut bacteria
<400> 50
   atgaagtaca tttttgtcac cggcggtgtg gtttcgggct tgggcaaagg catctgtgcg        60
   gcttccctgg gcagattgct gaagcagtgc ggcctgcggg tcaaaaatca gaagttcgac       120
   ccgtatttca atgttgatcc cggcaccatg agcccctttc agcacggtga ggtctttgtg       180
   accgaggatg gcgccgaaac cgacctggat ctgggccact atgagcggtt tgtggatgaa       240
   tccttggacg gcaatgccag cgtttcctcc ggcaaggtct tttggacggt gctgaaccgg       300
   gagcggcagg ggggctatct cggcggcacg gtgcagatca ttccccatat caccgatgag       360
   atcaagcggc ggatctatgc catggaggat ggcaacaccg atgtggtcat cagcgagatc       420
   ggcggcacgg taggggatat cgaaagccag ccctatctgg aggccatccg gcaagtagcg       480
   gtggaaaagg gccgggaaaa tgtgctgtat atccatgttc cgctcatcgt ttccatcccc       540
   ggcaccggag agctgaaaag caagccaacc cagcattctg tcaaggagct tctttcggtg       600
   ggcatccagc cggatattct ggtttgccgg acggaggcgc ccatcaccga ggaggtgcgg       660
   cataaaatcg ctctgttctg caatgtggag gagcggtgtg tcatccccaa tgtgacggcc       720
   catactctgt atgaagtacc gctgctgctg gaaaaagagg gcctgtgcga tgccgtctgc       780
   cgccggctgg gtctgggaga acggaccccc gatatgaccg aatggacagc catggtggag       840
   aagataaaag gggtgcaccg ccgggtggaa atcgccctgg tagggaaata taccggcctg       900
   cgggatgctt acctcagtgt ggcagaggcg ctgttccatg ctgggacggc ctgtgatgcc       960
   gaggtggaga tccgttgggt ggattccgaa aacttgaccc cggaaaacat agcggaaacc      1020
   ctggtcggct gcggcggcat tctggtgccg ggcggctttg gcgaccgggg aatagagggc      1080
   atgatcctgg cggcccagtt tgcccgggaa aaggggatcc cctactttgg catctgcctt      1140
   ggcatgcaga tcgccgtgat cgaatttgcc cggcatgtgg ccgactggca ggatgccaac      1200
   agcgctgaat tttccggttc taccgcgcac ccggtcattg ccctgatgcc ggagcaggtg      1260
   ggcgtgaccc aaaagggcgg caccatgcgg ctggggcggt atccctgtgt tttggcggag      1320
   gggacaaggg ccctttccct gtacggggaa aaggagataa gcgagcggca ccgccaccgg      1380
   tatgagttca acaatgactt ccggtgtgag ctgcagcagg acggcctgac actggccggt      1440
   acctcgccgg atggggcact ggtggagatc gtggagatca aggaccaccc atggtatgtg      1500
   gggtgccagt tccatccgga attcaagagc cggcccaacc gcccgcatcc gctgttttac       1560
   ggctttgtaa aagcggcgtt ggaggcagag gaaaagcggt ga                         1602
<210> 51
   <211> 531
   <212> DNA
   <213> Unknown
<220>
   <223> Unknown gut bacteria
<400> 51
   atgaagctga atctgagaga aatcatagaa attcccggcg gcagtgttcc gtttgcgcag      60
   gagctggaca cggccctgct cgactttccc tccgtccggg cctacaccgc gccgatcacg     120
   gccgagggca aggtggtcaa tacggccggc atcctcacgc tgcagggcaa actgcacgcg     180
   cagatgctgt gtgtgtgcga ccgctgcggc gcggagtttc cgcgcacggt cgacctcgac     240
   ctgcacgccg acatcacggc ggatgcgcag gacgacgatc ctgaggtgtt tacgctcgac     300
   ggagactggc tcgatgtgga cgaggtgctc tccaccagct tcatcctcag catggatacc     360
   aagtgcctgt gcaagcccga ctgtgccgga ctgtgcgacc gctgcggcgc gaacctgaac     420
   ctcgggccgt gccggtgtca aaaaccccgc gatccaagaa tggctgtatt agagcagctt     480
   ttggataata aaactgacga agaagaggaa gctgctcata acagcgactg a              531
<210> 52
   <211> 1779
   <212> DNA
   <213> Unknown
<220>
   <223> Unknown gut bacteria
<400> 52
   atgaaattga gcggaaccat tacaaaagtt tccggtccgc tggtcgtggc caatggcctt      60
   gccgatgcca acgtctccga cgttgtgcgt gtcggcgagc agcgcctgat cggcgagatc     120
   ctgaatatga ccggcgacag cgcgtccatc caggtctatg aggagacctc gggccttggc     180
   cccggcgcga aggtcgagac caccggtatg ccgctgtccg tggagcttgg ccccggcatg     240
   ctggagaata tctatgacgg tatccagcgc ccgctgcccg agatccgcga cctgaccggc 300
   tccaacatca cccgcggcgt cgaggtgccc gcgctcaacc gcgagcgcgt ctggcacttt 360
   gaccccgttg tgcagcccgg caccgccgtc accggcggcg acgtgatcgg caccgtccag 420
   gagacgacgg ccatcctgca caagatcatg gtgcccccgc agatgaaggg cacgatcaag 480
   agcatcaccg gcggcgactt cacggtcgac cagaccgtcg ctgtgctcac ggacgccaac 540
   ggcgtcgacc atgagctgaa catgatccag cgctggccgg tgcgtatcgc gcgcccctat 600
   gcgcagaagt tcgctccgaa caagcccatg aatagcggcc agcgtatcat cgacacgctc 660
   ttcccgatcg ccaagggcgg cacggccgcc gtgcccggcc cgttcggcag cggcaagacc 720
   gtcgtgcagc accagctcgc caagtggtcg gacgtggacg tcgtcgtcta catcggctgc 780
   ggcgaacgcg gcaacgagat gaccgacgtt ctcatggagt tcccggagct gaccgacccg 840
   cgcaacggcg agccgctgat gaagcgcacc gtgctcattg ccaacacctc cgatatgccc 900
   gtggcagcgc gtgaggcgtc catctatacc ggcatcacca tcgccgagta cttccgtgac 960
   atgggctatg acgtcgccgt tctggctgac tccacctccc gctgggccga ggccctgcgt 1020
   gagatgtccg gccgtctgga agagatgccc ggcgaagaag gctacccggc ctacctcgcg 1080
   tcccgcatcg cgcagttcta cgagcgcgcc ggctgcatcc gctgcatcgg cagcgagggc 1140
   gaccgccgcg gctccgtcac cgccatcggc gctgtctcgc ctccgggcgg cgacatttcc 1200
   gagccggtct cgcaggccac catgcgtatc gtcaaggtgt tctgggcgct cgattcgagc 1260
   ctggcgtatg cccgccactt cccggccatc aactggctga cgtcttactc gctgtatgtc 1320
   gactcgctca agccgtggta tgaggccacg ttcggcgagg aatatatggc caaccgcgac 1380
   aaggccatga gcatcctgca gcaggaatcc gacatgcagg agatcgtccg cctcgtcggc 1440
   caggacgcgc tgtccccggc tgaccgcctg acgatggaga cggccaagat gatccgtgag 1500
   ggcttcctgc agcagaacgc cttcgtcgac atcgacagct actccgagta ccgccgtcag 1560
   tttctgctgc tcggtctcat cctgcgctac gacgccgagt gccgcgacgc actcgagaag 1620
   aacgcaccga tgcacaagct ctttgccatc cctgcccgcg tcgacatcgg ccgcgccaag 1680
   agcgtgccga gcgacgagta cgagcaggtc tatgccaaga tcgccgcgga catgaccgcg 1740
   cagatcaaag agattatcgc cggaggtgag gagcagtga 1779
<210> 53
   <211> 951
   <212> DNA
   <213> Unknown
<220>
   <223> Unknown gut bacteria
<400> 53
   atgattgaaa ttgaaaagcc gcgcatcgaa tgccaggaat ccccggacga tatctcgtat 60
   ggcaagttcg tcgttgagcc tttggagcgt ggctacggca cgaccctggg taactctctg 120
   cgcagagtgc tgctctcttc actgcccggc actgccccga cgtccatccg gatcgccggc 180
   gtgcagcacg agttttcgac gatccccggt gtcaaggaag acgtcacgga gatcgtgctg 240
   aatgtgaagg agatcatcgc gcgcctgcac tgcgacggcc cgaagaccgt ctatatcgaa 300
   gcggcaggcg agtgtgaagt cacggccggc gacatcaagg cagacggcga ggtcgagatc 360
   ctcaaccccg agctgcacat tgccagcctc ggcccggacg gcgcgctctc catggagatc 420
   acgctcgccc acggcagagg ctacgtcccg gcggacaaga acaagagtgc gcagcaggtc 480
   atcggcacca tcccggtgga cagcatctat gcgccggtcc tgaaggtcaa ctacgctgtc 540
   gagaacaccc gtgtgggcaa ccagacggac ttcgacaagc tgaccatcga ggtctggacc 600
   gacaagacca tctcggcgcg cgacgcgctg agcctcggcg ccaagatcct gtgcgatcac 660
   ttcacgctct tcaccgatct gtctgacacg attggcaaca gctgcacggt ggtggagaaa 720
   gagccggagc gccccgacac cgtcatgaaa atgacgatcg aggaactgga tctctccgtg 780
   cgcagcttca actgcctgaa gcgcgcgaat atcaacaccg tcgaagacct cacgaacaag 840
   actgaggaag agatgatcaa ggtgcgcaac cttggccgca aatccctgga agaggtcgag 900
   cacaagctcg ctatgatggg cctgagcctg gcgagcgacg acaaccagta a 951
<210> 54
   <211> 3432
   <212> DNA
   <213> Unknown
<220>
   <223> Unknown gut bacteria
<400> 54
   gtgtcagaaa aggttttcaa caaggtcctt gccgccaacc gcggcgagat cgcggtgcgt        60
   atcttccgcg cgtgctatga cctcggcatc cacaccgtgg ccatgtactc caaggaagac       120
   gaactgagtc tgttccgcac ccgcgccgac gaggcgtatc tcgtcggtga aaacaagagc       180
   cctctgggtg cgtatctcga catccccgag atcatcgacc tggccaagcg ccgcggcgtc       240
   gacgccatcc acccgggcta cggtttcctg tccgaaaacg cggactttgc ccgcgcctgt       300
   gaagaggccg gcatcacgtt catcgggcct ccgagcgacg tgctcggcaa aatgggcgac       360
   aagctctcgg ccaagcagat cgccatggaa tgcggcgtgc cggtcatccc cggctgctcc       420
   gagcccctgc gcgacgggca ggaggcactc gagaaggcca tcagcttcgg cttccccgtc       480
   atcctcaagg cggcagccgg cggcggcggc cgcggcatgc gcctgtgcga gatgcccgag       540
   gacgtcatcc ccgcgttcga gctcgtcaag aacgaggcgc gcaaggcctt cggcaatgac       600
   gacatcttca tcgagaaata cctcgtgcag cccaagcaca tcgaggtgca gatcctcgcc       660
   gaccagtacg gcaacgtgcg ccacctcggc gagcgtgact gctcgctgca gcgccgctac 720
   cagaaggtcg tggagtttgc gcccgcgtgg agcgtgcccg agagcatccg cgagcagctg 780
   cacgccgacg ccgtgaagat cgccgaggcc gtcggctatg tcaacgccgg cacggtggag 840
   ttcctcgtcg accgcgacgg caaccactac ttcatcgaga tgaacccccg catccaggtc 900
   gagcacaccg tcaccgagat ggtcacgagc atcgacctcg tgcgcgcgca gatcctcatc 960
   gccgaggggc agccgatcag ccacccggag atcggtctcg gcgaccagaa caacctcaag 1020
   gtcaacggct acgccatcca gtgccgcgtg accaccgagg acccggccaa caacttcgcg 1080
   cccgacaacg gcaagatcga ggcctaccgc tccggcggcg gcttcggcgt gcgtctcgac 1140
   ggcggcaacg ccggcacggg ctcgatcatc tcgccgtatt atgactccct gctcgtcaag 1200
   gtcacgagct gggactgcac cttccccgcc gtctgccgca aggctacccg cgccatcaat 1260
   gaggagcacg tgcgcggcgt caagacgaac atcccgtttg tgacgaacat cctcacgcac 1320
   ccgacgttca tcgccggcaa gtgccacacg aagttcatcg acgagacgcc ggagctgttc 1380
   gagttcaccg aatcgcgcga ccgcgcgacc cgcgtgctca aatacatcgc caacatccag 1440
   gtcaacaatc ccgacgccga gcgccaccag tacgacacac cgcgcttccc gaaggcgcag 1500
   cgcgagatct cgaagcagga cgggctcaag ctcctgctcg acaccgacgg ccccgaggcc 1560
   gtcaaggact gggtcctcgg ccagaagaag ctgctcatca ccgacacgac gatgcgcgac 1620
   gcgcaccagt ccctgctgag cacccgcctg cgcacccgcg acatgctcaa gggcgccgac 1680
   ggcacggccg acatcctcgc cgactgcttc tcgctcgaga tgtggggcgg cgcgacgttc 1740
   gacaccgcct accgcttcct acatgagtcc ccgtgggagc gcctggagat gctgcgcgag 1800
   aagatcccga acatcccgtt ccagatgctg ctgcgcggct cgaacctcgt cggctacgcg 1860
   agctatccgg acaaccttgt gcgtgccttc atcgccgagt ccgcccgcga gggcatcgac 1920
   gtgttccgcg tgttcgactc gctcaactgg ctgccgaaca tggagaccgc catggacgag 1980
   gtgctgcgtc agggcaagtt cctcgagggc accgtgtgct acaccggcga tatcctcgac 2040
   ccgacgcgcg accgctacac actcgagtac tacgtcaact tcgccaagga gctcgagcgc 2100
   cgcggcgtgc acatgctggc catcaaggac atgtccggcc tgctcaagcc ctacgccgcc 2160
   aagaagctcg tgagcaccct caagcaggag atcggcatcc ccatccacct gcacacgcac 2220
   aacaccaccg acaaccagat cgcgacctat ctcatggcgg cccaggccgg cgtggatgtg 2280
   gtcgacaccg ccatcggccc gctggccaac ctcacgagcc agccgtcgat gaacgccgtc 2340
   gtcgagtccc tgcgcggtca ggagcgcgac accggctttg acccgcagcg cctgcaggag 2400
   ctggccgatt actgggccga cgtgcgcctg cgctacgaga gcttcgacaa gggcctcaaa 2460
   gtgcccgtca ccgatatcta ccgctacgag atccccggcg gccagtacac gaacctgcag 2520
   ccgcaggtcg agtcgctcgg cctcggccac cgcttcgcgg aggtcaagga gatgtaccgc 2580
   accgtcaaca tcatgctcgg cgacatcatc aaggtcacgc cgtcgtccaa aatggtcggc 2640
   gacctggcga tcttcatggt gcagaatgac ctcacgcccg aaaacatcgt cgagcgcggc 2700
   gagaccctcg ccttccccga cagcgtcgtg agctacttca agggcatgat gggccagccg 2760
   ccctgcggct tcccgaagga tctgcagcgc gtcgtgctca agggcgagaa gccgatcacc 2820
   ggccgccccg gcgacctgct gccgcccgtc gactgggatc agctgcgcag cgagatccgc 2880
   aagttctacc cgaactacga ggagccgcgc tccctcatct cctacgccat gtacccgaag 2940
   gtctatgagg agtacatccg ccaccgcaag gagtatggct acatcatgcg catgggcagc 3000
   cacgtgttct tcaacggcat ggccgtcggc gagatgaaca agatcaacat cgaggacggc 3060
   aagaccctca tgatcaagta catcggcctc ggcgaccgca acgacgacgg cacgcgcaac 3120
   gtgcagttcg agctcaacgg catgcgccgt gaggtcgccg tgcccgatcc gaccgcgacc 3180
   gacacggcca agaccgtcgt catggccgac ccgaacgaca agagccaggc cggcgcatcc 3240
   atccccggca tggtgtcgaa ggtcaacgtc aagcccggcg atcaggtcaa ggtcaacgac 3300
   gtgctcgccg tgatcgaggc catgaagatg gagacgagcg tcgtcgcccg catggacggc 3360
   acgatcgacg aagtctttgt caagggcggt cagacggtca aggccggcga gctgctgctg 3420
   acgatcaagt aa 3432
<210> 55
   <211> 1236
   <212> DNA
   <213> Unknown
<220>
   <223> Unknown gut bacteria
<400> 55
   atggataaca aaaagctcaa gagcagctgg gcagccgcct tcaccgtggc gtctgtctgg 60
   ttcggcaccc acgtgggcgc cggctttgcg accggcaacc aggtcgtgaa ctacttcgtc 120
   cagtatggct ggaccgcggc aatcttcccg ctgctggcca tgggcattct ggccgtcgtc 180
   atgtacatca tgatgaagtt tgccaagctc aacggtttcg acaactacaa ggacacctat 240
   cgcgccctgt atccgaagcc gtggatggag atcttcttcg agatctttta catcatcatc 300
   atccttgcgg cggtcgcgtc ctgcgttgac ggcgccggcg gcatcgtcaa gagcctgatc 360
   gacctgcccg atatcatctg caacctcgtc atcatcgccc tgctgatcct tctgtcgatc 420
   ttcggcgttg acctgatcat caaggcttcc accgttcttt ccaccgcgat cctgatcgtc 480
   accgcgatcc tggtcatcat gggcttcgtc gttccgatcg acgcggttgc ccaggagctg 540
   gccactgaga acgcggccta cgcttccatc ccggccatcg cggacgtcac ctcccagaag 600
   ggcctcgagg gcgtctggcg cggcgtgttc gtgtacgctg ctttccagtg cgtgtccgtc 660
   ccggccatga tcgctgcttc caccgagctg acccacaagg gcgtcaagcg cgccagcatc 720
   ctcggctggc tcatgaacgg cggcgcgctc gccgcttccg gcgccatgct gctgcgctgg 780
   tacccggtcc tctgcgccat caagagcgtt ggtgttgact ccgtcgccag catcgccgcc 840
   cgccacattg ccgacgtcat gaagctcccc aaccagagcg tcgtcaccct catgggcagc 900
   ggctacacct ggctgttcgt cgtgtacacc atcctgctgt tctgcgcgtt cgtgtccacg 960
   tccgtcacgc tcgtctactc catgatccag cgcttcgagg gccactgctt cccgaaggcc 1020
   atcaagagca aggctgtccg cagcgtcatc gtcggcctga tcgtcatcgg cctgtgctac 1080
   gcagtttccc tgctcggcct gtcgaccatc atctccaaga tctatggcta tgacggctac 1140
   tacgctctga tcgttgttgt gctcccggcg ttcatctggg gcatcccgaa gatcaagaag 1200
   ctgtctgccg acaagaaggc agagctgtcg gagtga 1236
<210> 56
   <211> 1362
   <212> DNA
   <213> Unknown
<220>
   <223> Unknown gut bacteria
<400> 56
   atgagtaaaa gctccatcgg taccgagccg gtgtacgacg cacgtacact cggcaagtcc      60
   agaatgttca ttctgggtct ccagcacatg ttcgccatgt tcggcgcgac cgtcctcgtc     120
   ccggcgctgt ccggcctcga cgtcgcaaca acgctgctgt tcgccggcct cggcacgctg     180
   ctgttccacc tgctgaccaa gggcaaggtc ccggccttcc tcggcagctc gttcgccttc     240
   atcggcggct acaacgccgt gcgcaccatc ggcacgaatc cggacggctc cgcgatctat     300
   aataatgacc tgctggccta cgcctgcttc ggcgtcgcca tcgcgggcct gatgtacatc     360
   atcctgtcga ccctgttcaa ggtcttcggc gtcaagaaag tcatgcgcta cttcccgccg     420
   atcgtcaccg gcccgatcat catcgccatc ggcctgacgc tctcctcttc cgccatcacc     480
   aactgccgtg ccaactggct tgtcgccgtc gtcgcgatcc tgatcgtcat cggcttcaac     540
   atgtggggca agggcatggc caagatcatc ccgatcctgc tcggcgtgct cggctcttac     600
   atcttcgcac tcatcgttga tcccgcggag cgcgcctccg tcgccgctgc cgtcagcagc     660
   gccaagtggt ttggcctgcc gatcttctgg aaaaacagcg tgttccacct cttcgccggc     720
   ccggtcgaca gcggcgtcct ctggtccgcc gtgctgacgg tcgtgcccct gtcccttgcc     780
   accatggtcg agcacatcgg cgacatctgc gccatctcct ccacctgcgg caagaacttc     840
   atggtcgacc ccggcttcca ccgcaccctg ctgggcgacg gcctggccac caccctcgct     900
   tccctcttcg gcgctccggc caacaccacc tacggtgaga acaccggcgt gcttgccctg     960
   tcccgcgtct acgatccccg cgtgatccgc atcgcggcct gcattgcgat cgtcgtgtcc    1020
   ttctgcccga aattcgcggc cctcgtgtcc gccatgccga cggccaccat cggcggcgtg    1080
   tccctcgtgc tctacggcat gatctccgct gtcggcgtgc gcaacgtcgt cgagaacaag    1140
   gtcgacttca ccaacacccg caacgtcatc atcgccgcgc tcatcctcgt gctcgccatc    1200
   ggcatcacct actccggcag catccagatc ggcatcgtat ccctgtcggg tctggctgtc    1260
   tcctccatcg tgggcatcct gctcaacgcc attctgcccg gcaaggacta caccttcgag    1320
   caggacgata agggcgcgac ctctgtggac ttcaaggtct ga                       1362
<210> 57
   <211> 1776
   <212> DNA
   <213> Unknown
<220>
   <223> Unknown gut bacteria
<400> 57
   atgtattaca gcagcggtaa ctacgaggcg tttgcaacgc cgaaaaaacc cgaaggcatc 60
   gacaacaagt cggcatatgt cgtcggcacc ggcctggccg gtctggcggc ggcctgcttc 120
   ctgatccgcg atgcacagat gcccggcgag cacatcacgc tctttgagca cctgccggtg 180
   gccggcggct cctgcgacgg catctacgac gcgaccaagg gcttcatcat gcgcggcggc 240
   cgtgagatgg ataaccactt cgagtgcatg tgggatctgt tcaagtccat cccgtcgatc 300
   gtgaacccgg gcgagacggt cttctccgag tactactacc tcaacaagga agacccgaac 360
   ttctccctgt gccgtgtcac cgagaagcag ggccaggacg cccacaccga ccgtaagtac 420
   ggcctgacgc ccggcgccgc cacccagctg ctgaagctgt tcatggcgac caacaagtcc 480
   ctcgaggaca agaagatcga tgacgtgttc gacgatgagt tctacgccac gaacttctgg 540
   acctactggc agaccatgtt cgcgttcgag aagtggcaca gcgccctcga gatgaagctc 600
   tacctgcagc gctacatcca ccacatcgac ggcctgccgg atctgagcgc gctgcgtttc 660
   acccgctaca accagtatga gtccatgatc ctcccgatgt gcaaatacat taccgaccac 720
   ggcggaaagg tcctcttcga caccacggtc acgaacatcg tctgcgactg caccgaggat 780
   aagaaggtcg ccaagaagat cgagtacacc cagggcggcg tggaaaaggt catcgagctg 840
   actgagaacg atctcgtcat ctgcacgaat ggctgccagg gcgatgcgtc cgcttacggc 900
   gatcagacgc acgctccggt catcaaggtc aagaacggcg aaggcccgtc catcgagatg 960
   tggaagaagc tcgccgcgca ggatccggcg ttcggccatc ccgagaagtt cttcaaggac 1020
   atcaaggaga ccagctggga gtcctggacc gtcgacacgg ccaacaagca gatcctcgac 1080
   gccatccaga agatctgcaa gcgtgacccg ctgtccggca aggtcgttac cggcggcatc 1140
   gtcacctgcc gcgattcgag ctggctcgtc agctggacca tcaaccgtca gggccagttc 1200
   caggagcagc cgaaggatca ctgcctgatc tgggtctacg gcctcaactg ctgggacgac 1260
   aagggcgact tcatcaagaa gaacatgtgc gactgcaccg gcatcgagct ggcggcagag 1320
   tggctgtacc acatcggcat ccccgaggat cagatcatgg atctggccac gaacgaatgc 1380
   aacaccacgc cgtgcatgat gccttacgtc accacgttct ttgagcccag agccgaaggc 1440
   gaccgcccga aggtcgtgcc cgatggttcc gtcaacctcg cgttcgtcgg ccagttcgcc 1500
   gataccccgc gcgacaccgt gttcaccacc gagtactcca tccgtaccgc gatggaagcg 1560
   gtctacacgc tctgcaatgt cgaccgcggc gtgccggaag tctggggcag cgtgtatgat 1620
   atccgcgacc tgctctacgc caccagcaag ctgctcgacg gcaagaagcc gctggagttc 1680
   ctgctcccgt cggttctgcc gctgctcgac ctcgtcaagg agccgctggt caacaacgtc 1740
   gtggtggatc tgctcaagaa gtataacatc gtctga 1776
<210> 58
   <211> 1080
   <212> DNA
   <213> Unknown
<220>
   <223> Unknown gut bacteria
<400> 58
   atggcattga cgcttatgaa gggtagcgaa gctatcgctg aggctgcgat ccgtgcaggc      60
   gcgcgttact tcttcggcta tccgatcact ccgcagacgg agattcctga atacatgtcc     120
   gcacgtatgc ccgaagtcgg cggctgcttc ctgcaggccg agagcgaagt tgcggctatc     180
   aacatgattt acggcgcagc cggtgctggt gcacgtgcgc tgacctcttc ctccagcccc     240
   ggtgtcagcc tgaagcagga aggcatttcc tactgcgcag gcgcgcagct gccggctgtc     300
   atcctgaacg tcatgcgcgg cggccccggc ctcggcaaca ttcaggcttc tcagggcgat     360
   tacttccagg ctgtcaaggg cggcggcaac ggtgactatc acctgctgac ctatgctccg     420
   gcatccgtgc aggaagcgat cgacatcatg atgtacgcct tcgacaaggc tgagaagtac     480
   cgtataccgg tcctcttcct ggctgacggc atcatcgctc agatgatgga gcccgtggag     540
   atgcccgaga tggtcgacta caagatcgac ccggagaaga agccttgggc ctgcaccggc     600
   tggaaaccgg gcgatgatcc ggcaaagcgc ggcgtcatca actccatcta catcgatacc     660
   gagaccctgt ccgtccacaa cgacgagctg caggcctgct atcgtgagat cattgccaac     720
   gagcagcagt gggaagagta caacctcgag ggtgctgagt atgtcatcac cgctttcggc     780
   acggttgcgc gtattgccaa gtctgcgatc gttgagctca aggagcaggg catcaacgtt     840
   ggtctggtcc gcccgatcac cgtttggccg ttcccgtacg acgccgtcaa gaaggctgcg     900
   gagcagccgg gcgtgaaggc tgtgctcgat gtcgagctga acgaaggcca gatgctcgag     960
   gacgtcaagc tcgccatcaa cggcgcaaag ccggtcgact tctttggcca cctcggcagc    1020
   cagatgccga ccaccgaaga gatcaaggcg aagattatca gcatgaagga gggcaagtaa    1080
<210> 59
   <211> 1101
   <212> DNA
   <213> Unknown
<220>
   <223> Unknown gut bacteria
<400> 59
   cgcccgacgt ctgagacgct cttctgccag cactggagcc acatcgtgca cagctggcgc 60
   gacctgccgt gcctgtataa ccagtggtgc agcgtcgtgc gctgggaaaa gaccacccgc 120
   ccgttcctgc gcgggcgtga gttcctctgg caggagggcc acaccctgca cgccactgag 180
   gccgaggccc gcaaggagac gctgcagatg ctcgaaattt atgccgagac ctgcgagcag 240
   gagctggcca tgcctgtcat ccgcggcaac aagaccgaca aggagaagtt cgccggcgcc 300
   gaaaacacct acaccatcga gtgcatgatg cacgaccaca aggcgctcca gtctggcacg 360
   agccactact tcggcgacgg gttcgcccgc gcgttcgaca tcacctacac cgacaagaac 420
   aaccagcagg tctacccgca ccagacctcg tggggcctgt ccacgcgcgt catcggcggc 480
   atcatcatga cccacggcga caacagcggc ctcgtcctgc cgccgcacat cgcgccgatc 540
   caggtcatcg tgctgcccat cgcggcgcac aagcccggcg tgaccgagaa ggccgaggag 600
   ctcgttgcgc gcctgaaggc cgccggcctg cgcgcgcagg gcgacttcag cgacaacagc 660
   cccggctgga agttcgccaa ctgggagatg aagggcgtgc cgctgcgcat cgagatcggc 720
   ccgaaggaca tcgaggccgg ccactgcgtc gccgtccgcc gcgacaacgg cgagaagatc 780
   accgtcgcgc tcgatgagct ggaggcgcgc atccctgagc tgctcgagac cgtgcagcag 840
   ggcctgttcg acaaggccaa gcgcaacctc gacgagcaca cctatgccgc gcactcgctg 900
   gccgaggcca aggaactgca ggagaaaaac ggcggcttca tcaagaacat gtggtgcggc 960
   gacctcgcct gcgagctcga gatgaaggac aaggccggca tgtccagccg ctgcatcccg 1020
   tttgagcagg agcacctcga cgatgtgtgc cccgtgtgcg gcaagcccgc caagtgcatg 1080
   atctactggg gcgtggcgta c 1101
<210> 60
   <211> 999
   <212> DNA
   <213> Unknown
<220>
   <223> Unknown gut bacteria
<400> 60
   atgaatctca aaggcaacga ttttctcaag ctgctcgact acacccccga gcagatcgac 60
   tatctgctcc ggctcgccgc ccagctcaag gccgagaaaa aggccggcat cccgcaccgc      120
   ctgtgcgagg gcaaaaacgt cgcgctcatc ttcgaaaaga cgagcacgcg cacgcgcagc      180
   agcttcgagg tcgcggcgca cgatctgggc atgggcagca cgtatctcga ctccaccggc      240
   tcgcagatcg gcaagaagga gagcatcgcc gacacggcgc gcgtgctctc gggcatgttc      300
   gacggcatcg agtaccgcgg cttcggccag tgcatcgtcg aggagctggc gcagtacgcc      360
   tccgtcccgg tctggaacgg cctgacggat gagttccacc cgacgcagat cctcgcggac      420
   tttctgacca tccgggagca cttcggctcg ctggagaaca tccacctcgt ctacatgggc      480
   gatgcgcgct tcaacatggg caactccctc atggtcggct gcgcgaagat gggcatgcac      540
   ttcacggcct gcgcacccaa gggctacttc cccgatgcgc agctcgtcgc cgagtgtcag      600
   gccatcgcgg cgcagacggg cgcgacgctc gacttcatct ccgaccccgc cgcggccgtg      660
   cagggcgcga acgtgatcta taccgacatc tgggtctcca tgggcgaacc cgagagcgtc      720
   tgggccgagc gcattgccgc gctcgcaccg tatcaggtca acgcggcgct catggccaag      780
   gccgacccca acgccatctt catgcactgc ctgccggcgt atcacgacaa aaagaccgcc      840
   atcggcaacg agatgtgcac gcgcttcggc cgcgaggcga tggaggtcac ggacgatgtg      900
   tttgaaagcg cgcagtcggt cgtgttccag gaggccgaga accgcatgca caccatcaag      960
   gccgtcatgg cggcgacgct tgcggagatc gagctatga                             999
<210> 61
   <211> 771
   <212> DNA
   <213> Unknown
<220>
   <223> Unknown gut bacteria
<400> 61
   gagcgcgtgc ccgcgctggt cgaggccggt gtggacgtgc tcgtgatcga ctcgtccgag    60
   ggctactccg agtggcagaa gcgcacgctc gagtggatcc gcgagcgcta cggcgacagc   120
   gtcaaggtcg gcgccggcaa cgtcgtggac gcggacggct tccgcttcct ggccgactgc   180
   ggcgcggact tcgtgaaagt cggcatcggc ggcggctcga tctgcatcac gcgtgagacc   240
   aagggcatcg gccgcggcca ggcgaccgcc gtcatcgagg tctgccgcgc acgcgacgag   300
   tacttccgcg agaccggcat ctacgtgccc gtctgctccg acggcggcat cgtgcacgat   360
   caccacatca cgctcgcgct ggccatgggc gcggacttcg tgatgctcgg ccgctacttc   420
   gcccgcttcg acgagagccc gtcggacaag gtcaacgtca acggcacgct catgaaggag   480
   tacaggggcg agggctccaa ccgcgcgcgc aactggcagc gctacgacct cggcggcagc   540
   aagaagctgt ccttcgagga gggcgtggac tcctacgtgc cctacgccgg caagctctcc   600
   gacaacgtcc agcagacgct ggcaaaggtc cgctcaacca tgtgcaactg cggcgcactg   660
   accatccgcg agctgcagga aaaggcgcgg ctgacgctcg tgtcctcggt gagcatcgtc   720
   gagggcggcg cgcacgacgt cgtgctcaag acctcgaaca agcccgtgtg a            771
<210> 62
   <211> 1026
   <212> DNA
   <213> Unknown
<220>
   <223> Unknown gut bacteria
<400> 62
   atgaaagaaa tgctgcagaa gctgcgcgag gcttccgtcg ccgccatttt gcaggcggac 60
   gacatcaccg cgctggaaaa cctgcgtgtg aagtacctcg gcaaaaaggg tgagctgacc 120
   gccatcctca agcagatggg ccgcctgtcg gcggaggagc gcccggccat gggccagctg 180
   gcaaaccagg tgcgcgctga gctcgaagaa gcgctcgacc agcgccgcga gacgctcaat 240
   gcgcacatgc tcgagatgcg cctcaaggcc gagacgctcg atgtgaccat gcccggaaaa 300
   gcgccggagc tcggccacca gcacccgatg tatatcgtgc tcgatgagat taaggacatc 360
   ttcgtcggca tgggcttcga gatcctcgaa ggccccgaga tcgaagaggc cgaatacaac 420
   ttcaccaagc tcaacaccag cgagggccac cctgcccgcg agtggtcgga cacgttctat 480
   ctgaccgagg acagcagcat cctcctgcgc acgcagacct ccccgatgca ggtgcgcgcc 540
   atggagagca agccgctgcc gatccgcatg gtcgcccctg gccgcgtcta ccgcaaggat 600
   gaggtggacg ccacgcactc gcccatgttc caccagatcg agggcatggt catcgacaag 660
   ggcgtcacga tggcggacct gaagggcacg ctcaacacgc tcgtgaaaaa gctctacggt 720
   gagaacaccg tcacccgctt ccgtccgcac cacttcccgt tcaccgagcc gcgctgcgag 780
   atggacgtgc agtgccacaa gtgcggcggc aagggctgcc ccacctgcaa gggtgagggc 840
   tggatcgaga tcctcggcgc cggcatgatc catccgaagg tgctcgaggg ctgcggcatc 900
   gacacggacg tctactccgg ctgggccttc ggcatgggcc tggagcgtct ggcgatgcgc 960
   cggttcaaga tcagcgacct gcgtctgatc ttcgaaaacg acgtgcgctt cctgcagcag 1020
   ttctga 1026
<210> 63
   <211> 1935
   <212> DNA
   <213> Unknown
<220>
   <223> Unknown gut bacteria
<400> 63
   gtgtatttca tgagttacga tgcattgttt tctcccatca agctcagagg gcttgagctg 60
   aaaaaccgtg tggtcctgcc gggtatgaac accaagatgg tcaaggacaa gaacgacgtg      120
   ggcgaggatc tgcccgccta ccatgcggcg cgcgcagccg gcggctgcgg cctgaacatt      180
   gtcgagctcg tctccatctg ccccgagtgc catgcatacc tgtacctcgg tctttataat      240
   gagcaccaca gagaccagct gcgcaagatc accgacgcga tccacgcggc cggcggcaaa      300
   gcaggcgtgc agatctggca cggcggcttc gtgcccgaag agttcttcga caagaccaac      360
   aagcttgaga cgcctgacac gctgaccgtc gagcgcatcc acgagatcgt caagcagttc      420
   ggctactccg ccaagctcgc tgtcgaggcc ggctttgacg cactcgagtt ccacggcgca      480
   cacacttatc tgccgcacga attcatgaac ccctcgctca acaagcgcac cgacgagtac      540
   ggcaaccaga gcctggagaa ccgctgccgc ttcaatctcg aggtcatccg cgagatgcgc      600
   aagaacatgc cggaagacat gccgctgctc atgcgcctgg acgccatcga cgagatgctc      660
   ccggccgtga ccacgcagga cgagaccgtg cagttcatca actgggccgc cgaggccggt      720
   gtcgatgcga tcgacctgtc ccgcggcaac gcccgcagcc ttgcgaccgt gtatgaagtg      780
   ccgccctaca atctggagcc cggcttcaac atggacaaca tcgccgccat caaggcgcgc      840
   gtgaacatcc cggtcatcgg cgtcggccgc atcgtcgacc ccgctctggc cgatcagctc      900
   atccgtgagg gcaagatcga catggtcgcc gtcggccgtg cgcagctcgc cgatcccgaa      960
   tggtgcaaca agtccatgga aggccgcgag gcggagatcc gccgctgcat cggctgcacc     1020
   gagggctgct atgacaaggt catcgacccg aaggccaagc acatcacctg cacccgcaac     1080
   ccggcgctct gcctggagta caagggcctc ccgaagccgg agaaagccaa gaacgtcatg     1140
   gtcatcggcg gcggcatcgg cggtctgatg gcagccgagt acctcaaggc tcgcggccac     1200
   aatccgaccg tctatgaagc caccgacgcc ccgggcggcc actttgtgct cgccggaaag     1260
   gccccgaaga agcaggcctt caccgacgcc gtcatgtggg acgctgagga gtgcaagcgt     1320
   atgggcatcg agatcaagac cggcgtgacc gtcacgcccg agctcatcaa ggaagtcaag     1380
   cccgatcacg tgatcgttgc caccggcgcg cactttgtcg ccccgaacat cccgggtctc     1440
   gacaccgcga aggacgtcta cgtggctgag gacgtgctgg ccggcaaggc catgccgcac     1500
   ggtgaaacca tcatcctcgg cggcggcggc gttggctgcg agaccgcgca gttcctcatc     1560
   gagcacggcg tcaccgatgt gcgcgtgatg gattccaagc gcgtcggcaa caagatgggc     1620
   atgctgcgca ccatgttcct cgacatcgag taccccggcg agaccatcaa gaagagccgc     1680
   ggctctaaaa tcaccgccgt cggcgatcac gagatcacct atgcgttcac gaacaaggcc     1740
   aagaagaccg tcgagaagac ccgccacttt gactccctcg tcatcgccac cggcatgcac     1800
   tcccgcccga cgcaggagct gaccgacgca tgcagcgaac tcggcatccc ctgcgacgtg     1860
   atcggcagcg ccaagaaggc tgacatgggc atcgaagcca ccgccgacgc ttacgccgcc     1920
   gccatgaacg tctga 1935
<210> 64
   <211> 1275
   <212> DNA
   <213> Unknown
<220>
   <223> Unknown gut bacteria
<400> 64
   atccaccagc acttcaagct ggtcgacgtc ttcaccgccg ctgagaacat cacgctcggc 60
   ctcgatgaaa agctcgatct caaggccacg gccgcgcgca tccgcagcat ctgcgagcgc 120
   tacggcttcg acctcgaccc gaaccagaag atctacgata tgtccgtgtc ccagaagcag 180
   acggtcgaga tcgtcaaggc gctctaccgc ggtgcggaca tcctcatcct cgacgagccg 240
   accgccgtgc tcaccccgca ggagacggac acgctcttcg ccgccctgcg cagcatgcgc 300
   gacgacggca aggccatcgt catcatcacg cacaagatgc acgaggtcga gacgctcagc 360
   gaccgcgtgg ccgtgctgcg ccacggccag ttcgtgggcg acatgctcac gaaggacacc 420
   aacgcgcagg aaatgacgaa catgatggtc ggccacgccg tgacgctcaa tattgcccgg 480
   cccgaccccg tcgaccccaa gccgcgcatc gaggtcaaaa acctcaccgt gcgcagcatc 540
   gacggcatcg tcaagctcga cgacgtgtcc ttcaccgcca actccggcga gatcctcggc 600
   atcgcgggca tctccggctg cggccagaag gagctgcttg aggccatcgc cggcctgcag 660
   ccgaccgaaa acggcagcat ctgctacgtc gaggacgacg gcacgcgcga ggagctgctc 720
   ggcaaagatc cgctgagcat cgccgagatg ggcgtggcgc tgtcctttgt gccggaggac 780
   cgcctcggca tgggccttgt cggcaacatg gacctgtcgg acaacatgat gctccgctcc 840
   ttccgcaagg gccgcggcat cctcaccgac cgcaagtcgc cgcgcaagct ggccgagacc 900
   gtcgtcgagg aactcgacgt caacactccg ggcgtgacca cgccggtgcg ccgcctgagc 960
   ggcggcaacg tgcagaaggt gctcgtcggc cgcgagatcg cctccgcccc caccgtgctg 1020
   ctcacggcct atgccgtgcg tggcctggac atcaacgcat cctacacgat ctatgacctc 1080
   atcaaccggc agaaggagcg cggcgtcgcc gtgatctacg tcggcgagga tctcgacgtg 1140
   ctgctcgagc tggccgaccg catcctcgtg ctctgcggcg gcaaggtcag cggcatcgtc 1200
   cccggccgcg gcgccacgaa gcgcgacgtc ggcatgatga tgacgcagtt gggagggaac 1260
   gaagcccatg cataa 1275
<210> 65
   <211> 270
   <212> DNA
   <213> Unknown
<220>
   <223> Unknown gut bacteria
<400> 65
   atgcgtgcaa tcgtaaccgt gatcggaaaa gaccgtgtcg gcatcatcgc cgacgtgacc        60
   gccctgctgg cgcagtacgg cgtgaacgtg ctggacatca gccagacggt attgcaggaa       120
   tacttcacga tgatcatgct cgtggacgca gcgcagtgca ccgtgccgtt tgcagacctc       180
   gcccgcacgc tcgacgaggc cggcacacag cgcggcctgc agatccgcgc ccagcgcgag       240
   gacatcttca acgccatgca ccgcatctga                                        270
<210> 66
   <211> 1542
   <212> DNA
   <213> Unknown
<220>
   <223> Unknown gut bacteria
<400> 66
   atgtcgttac caatagcact cggttgtatc atcgcagcgc ttgtggttgg ttgtatcctt        60
   ggcgggcttt accgcaaaaa ggtctcggag cgcgagatcg gcagtgcgga agagaaggcc       120
   aagcagatca taaatgatgc ctataagagt gccgagagca agcggcgcga gaccctgctc       180
   gaggccaagg aagagatcca caagagccgg acggagtacg aacgcgaggt caaggaacgc       240
   cgcgcggagc tgcagaagca ggagcgccgc ctccagcaga aggaggagac cctcgaccgc       300
   aaaacggaca cgctcgacaa aaagacggac gccctcaacc agaagatcgc cgcgatcgac       360
   accaagcaga atgagatcaa tcagctcaaa aagacccaga tggagctgct cgagaagatc       420
   tccggctata cggtcgagga ggccaaggcc ttcctcatca acagcctgaa ggacgacgtc       480
   gtgcatgagc aggcgctcat ggtcaaggag gtcgaggccc agtacaagga tgaggccgat       540
   acccgcgccc gcgagatcat cgccaccgcc atccagcgct gcgccgccga ccatgccagc       600
   gaagtgaccg tgtcggtcgt gccgctgccg aacgacgaga tgaagggccg catcatcggc       660
   cgcgagggcc gcaccatccg cgccatcgcg acgctccccg gctgcgagct catcatcgac       720
   gacacgcccg aggccatcac gctctcgagc ttcgaccccg tgcggcgcga agtcgcgcgc       780
   atcgcgctcg aaaagctcat tcaggacggc cgcattcacc ccgcccgcat cgaggaaatg       840
   gtcgccaagg ctcagaagga ggtcaacgcc accatcaagg ccgagggcga gcgcgccgtt       900
   ttcgagacga acatccacgg cctgcatccc gagcttatca agctgctcgg ccgcatgaag       960
   taccgcacca gttacggcca gaatgtgctc aaccactcca tcgaggtttc gcacattgcc      1020
   ggcctgctcg ctgccgagct cggcgtcgac gtggccaccg ccaagcgcgc cggcctcctg      1080
   cacgatatcg gcaaggccat cgaccacgag gtcgagggca gccacgtgac catcggcgtc      1140
   aacatcgctc gcaagtacaa ggagtccgag gaagccatcc acgccatcga gcccccccac      1200
   ggcgacgtgg aggcccacac cgtcgtcgcc tgcctcgttc aggcggccga cgccatctct      1260
   gccgcgcgcc ccggcgcccg gcgcgagaac atcgagaact atgtcaagcg tctcgaaaag      1320
   ctcgaggaag tcaccaagag cttccccggc atcgccaact gctttgccat tcaggccggc      1380
   cgcgagatcc gcatcatggt caagcccgag gaggtctcgg aggatcagat ggtgctgctc      1440
   gcgcgcgaca tcgccaagaa gatcgaggac gagctgacct accccggcca gatcaaggtc      1500
   aatctcctgc gcgagaccaa ggccatcgac tacgcgaagt aa                         1542
<210> 67
   <211> 1701
   <212> DNA
   <213> Unknown
<220>
   <223> Unknown gut bacteria
<400> 67
   atgctgacac cctgggcgga tacgctcgac cgcgcgcagc ccctgccgga gtatccccgg      60
   ccgcagatgg tgcgcgacag ctacctgaat ctcaacggcc cgtggagcta cgcgatcacg     120
   acgagcgcga acaagcccgc gcaggccgac ggcacgatcc ttgtgccctt ctcgcccgag     180
   agcgagctgt ccggcgtggg acacgtcctg cagccggagg aatacctgtg gtacatccgc     240
   acggtcacgc tgccggacgg attcaacgtc ggccgtgtgc tgctgcactt cggcgcgatc     300
   gaccagaccg cgaccgtgtg gtgcaacggc gtggagctgg ccacgcacac gggcggctac     360
   ctgccgttca cggtggacat caccgaggtg ctggccaagg aaaacacgat ccttgtgtgc     420
   gtgcgcgacg cgacgaacaa gtcccagctc ccgcgcggca agcagacgct gcacccgcac     480
   ggcatctggt acacgccgca gagcggcatc tggcagacgg tctggctcga gagcgtgccg     540
   cagaactaca tccgctccct gcacgtgacg ccgctgtttg acgcgcatca ggtggagatc     600
   accgtcgagg gcgaaggtca gtgcacgatc gacctcgagg gcaagcgctt cacgttcccc     660
   gcgggcgtgc cggcgcacgt gccggtgcag tcgtttcgcc cctggtcgcc ggagcacccg     720
   acgctctacc ccttcagcgt gacgctcggg gacgacacgg tctacagcta ctttgccatg     780
   cgcaagtgca gcgtcgagac ggacaaggac ggcgtgcgcc gcctgtttct caacggcaaa     840
   ccgtattttc acaacggcct gctcgaccag ggctactggc cggacggact ctacaccgcg     900
   ccgagcgacg acgcgctcgt ctacgacatc cagctggcca aggacatggg cttcaacatg     960
   ctgcgcaagc acatcaaggt cgagtgcgac cgctggtact atcactgcga ccggctgggc    1020
   atgctcgtct ggcaggacat gccctgcggc ggcaggcgct acgacccgct catcgtctcg    1080
   accccgctcg tgacgggctg gcatctcgac gacagctggt atcccctctt cggccgcacg    1140
   aacgtgacgg cgcgcaaggc gtttttgaac gagctgcgcg acatggtcac cacgctctac    1200
   aaccacccgt gcatcgccgt gtgggtgccg ttcaacgagg gctggggtca gtttgacagc    1260
   cagacggccg tgcaggtcat ccggtccgtg gacaagacgc gcacgatcga cccggccagc    1320
   ggctggcacg atcagggctt cggcgatgtg cgcagcctgc acgtgtattt ccagaagtac    1380
   aggttccagc ccgataagct cggccgcgcc acgctgctgt cggagttcgg cggctatgtc    1440
   caccgcgtcg agggccacgc catcggcggc aaaagcgtcg gctacaagac gtgcgacgcc    1500
   ccgctctcgc tcgagtacgc gctccaggcg ctctatgacg agcagatccg ccccgccatc    1560
   gcgcagggcc tcagcgccgc ggtgtacacg cagctgagcg acgtggagca cgagctcaac    1620
   ggtctcgtga cctatgaccg cagcatcgtg aagctcccgg tacagacact gcacaaaatc    1680
   ttttatatcg aaaacgagtg a                                              1701
<210> 68
   <211> 1884
   <212> DNA
   <213> Unknown
<220>
   <223> Unknown gut bacteria
<400> 68
   atgcgtcaga gaatcgaatc cttggagcag ttccgcgccc tgcaggccga ctacatggcc 60
   gcgcgcgacg cggaaaaacg taagatcctc gtctgctgcg gcacgggctg tgtcgccggc 120
   ggcaacctga acgtctacca cgagctcaaa aagcgcatgg acgagctggg catcccgtgc 180
   gaggtgtccc tgaccgagca ccacgccgac accatcggcc tgaaaaagtc cggctgccac 240
   ggcttttgcg agatgggccc gctggtgcgc atcgagccgg agggctggct gtacacgaag 300
   tgtcaggtca gcgacgtgga ggagatcatc cagaagacca tcctcggcgg cgagttcatc 360
   gagcgcctgg gctacaacca gaacggcgag ctgtatgagc gccaggagga catcccgttt 420
   tacaaaaagc agacccgccg cgtgctcgag cactgcggcc atatcgacgc ggaatccatc 480
   gaggagtacc tgtccatcgg cggctacggc gcgctggcca aggccctgtt cgagatgcag 540
   ccggacgaga tcgtccaggc cgtgagcgat tccggtctgc gcggccgcgg cggcgcgggc 600
   ttcccgaccg ggcgcaagtg ggcgcaggtc gcggcacata cggaagagcc cgtcaagtac 660
   atcgtctgca acggcgacga gggcgacccc ggcgccttca tggaccgctc ctgcatggag 720
   ggcgacccgc acaaggtcat cgagggcatg atcgtcgcgg gcgtggccac cggctccacc 780
   gagggctaca tctatgtccg cgccgagtac cccatggccg tgcaccggct caccgtcgcc 840
   atcgagcagg ccaaggagct cggcctgctg ggcgacaaca tcctcggcag cggcttcagc 900
   ttccatatgc acatcaactg cggcgcaggc gcgttcgtct gcggcgaggg ctcggccatg 960
   accgcgtcca tcgagggcaa ccgcggcatg ccgcgcacga agcccccgcg ctccgtcgac 1020
   aagggcctgt acggcaagcc cacctgcctg aacaacgtcg agacgtttgc caacgtgccc 1080
   gacatcatca aaaagggcgc ggactggttc aaatccgtcg gcacggaggg cagctccggc      1140
   acgaaggcct tcgccctcac gggcaacgtc gtcaacaccg gcctgatcga agtgcccatg      1200
   ggcacgacgc tgcgcgaggt cgtgtacgac atcggcggcg gcatcaaaaa cggcaaagcg      1260
   ttcaaggccg tgcagatcgg cggcccgtcc ggcggctgcc tgaccgaggc gcacatggac      1320
   ctgcccatgg actttgactc gctgaaaaag gccggcgcga tcatcggctc cggcggcctc      1380
   gtcgtcatgg acgaggacac gtgcatggtg tccatcgcgc agtttttcat gaacttcatc      1440
   tgcaacgagt cgtgcggcaa gtgcaccccg tgccgcgagg gcacgacccg tatgctcgac      1500
   attctcaccc ggatcacgaa gggcaacggc cgccccggcg acattgagga gctgcgctcc      1560
   ctcgccaaga tgatccagaa ctcctccctg tgcggtctcg gcaaaacggc gccgaacccc      1620
   gtgctgtcca cgctcgccaa cttcgaggac gaataccgcg agcatatctt cgacaagaag      1680
   tgccgcaccg gcagctgccg cagcctgacg acgtatgtca tcgacccggc catctgcaag      1740
   ggctgcacga agtgcgcgcg caactgcccg gccggcgcga tcaccggcga gctgaaaaag      1800
   ccgcaccaca tcgacacaga caaatgcatc aagtgcggca cctgcaagac tggctgcccg      1860
   ttcggtgcga tcaaggaggc gtga                                             1884
<210> 69
   <211> 1578
   <212> DNA
   <213> Unknown
<220>
   <223> Unknown gut bacteria
<400> 69
   ggcggcctgc acggcgtcgg cgcctcggtc gtcaatgcgc tgtccgagtg gctggaagtg 60
   aacgtgcaca aaaacgggaa gatgtatcag atgaagttct cccgcggcga tatcacgcag 120
   cccatgaccg ttgtcggcga gacggatcac accggcacga ccgtgcgctt caagcccgat 180
   cccatcatgt tcgaggacac ggtctacgac tatgagacgc tgcacgtgcg catgcgcgag 240
   caggcgtttc tgaacgccgg actgcgcatc accatcaccg accgacgcgg cgaagagccc 300
   gtgagcgagt cgatgtgcta cgagggcggc atccgcgagt tcgtggactt catcaacaaa 360
   aacaagacgg ccctgcaccc cgaggccatc tatatgtccg gcgagcgcag cgactccatg 420
   tgcgagatcg cgctgcagta taacgacggc tataacgagg tcatcgtgtc gttcgccaac 480
   aacatccaca cgccggaggg cggcatgcac gagaccggct tcaaggctgc gctcacccgc 540
   gcgctcaatg cctacggcaa aaagtccggt atcctcaagg agggcgacag catctccggc 600
   gaggacagcc gcgagggcct cacggctgtc atctccgtca agctcacgaa cccccagttc 660
   gagggacaga cgaaggccaa gctcggcaac tccgagatgc gcacgctcgt cgattccgtt 720
   gtgttcgacc gcctgtcgca gtttctggag gagaaccccg ccgtcggccg tatgatcatc 780
   gaaaaagcgc tcacggcctc gcgcgcccgc gaagccgcgc gccgcgcgcg cgaaaacatc 840
   cgccgtaaaa acgggctcga gggcttcaac atgcccgaca agctgcgcga ctgcaacgac 900
   cgcgacccgt cgctcaccga actctacatc gtcgagggcg acagcgcagg cggctcggcc 960
   acgcaggggc gcgactcgcg cttccaggcc attctgccgc tctggggcaa gatgctcaac 1020
   gtcgaaaagg cccgcgccga caaggtctac ggcaacgaca agctcacgcc cgtcatcacg 1080
   gcgctcggcg ccggactggg cgatgagttt gacatcaata agctccgcta tcacaaggtc 1140
   atcatcatgg ccgatgccga tgtcgacggc agccaaatcc gcacgctgct gctgacgttc 1200
   ttcttccgct tcatgcgtcc gctcatcgac ggcggctatg tctactccgc catcccgccg 1260
   ctgtataagc tcaaccgtgg caagaccacg cgcgtggcct tctccgacga ggagcgcgat 1320
   aaaatttccg ctgagatgcg cggcgacaac ccgaacgcga agatcgacat caaccgcttc 1380
   aagggcctcg gcgagatgga cccgcacgag ctctgggaga cgaccatgaa ccccgagacc 1440
   cgcacgctca agcagatcac gctcgaggac gccgtgcacg ccgacgaggt gttcaccgtg 1500
   ctcatgggcg aaaaggtcga gccgcgcaag gcgttcatcg agcaaaatgc caagtacgcg 1560
   gtcaatattg atatttaa 1578
<210> 70
   <211> 348
   <212> DNA
   <213> Unknown
<220>
   <223> Unknown gut bacteria
<400> 70
   gacgtgaccg gccgtgtgcg cacgtacgaa gccatcgtca agggcgagaa catcccgcag     60
   ccgggcgtgc cggagtcgtt caaggtgctc atgaaggagc tgcagtccct gtgcctggat    120
   gtgcgcatcc tcgacgagaa cggcgacgag atcgagctca aggacgacga ggacgattac    180
   atcccgggta tgcgcgatga gatgtcctac aagtccgacg atgatgagat caccggcagc    240
   ggcttcacga tcgaggacgt accggtggag gaagatgaca gtctcggcgc gttcggcgat    300
   gccggcgacg atgactacac ggacgattct gataaggagg aggaataa                 348
<210> 71
   <211> 1011
   <212> DNA
   <213> Unknown
<220>
   <223> Unknown gut bacteria
<400> 71
   atgaaatttg caatgtatgg ttccggcgca gccggcagcg tcttcgcttc ttacctgcgt     60
   aagggcggtg cggacattat cctcatcgac cgctacgaag cccacatgaa gaaggtcgcc      120
   gaggaaggca tgcacttcac catccatcag gaggaaaatg gcggctacaa ggattacgac      180
   taccagctca agggcttccg cacctacacc accgctgctg acgcggccgc ggcggagggc      240
   aaggtcgacg tcatcatcta catgaccaag gcgacccagc tcgagcaggc catccaggac      300
   tccatgcccg tcgtcggcga cacgaccgtc gccgtctccc tcatcaacgg cctcggcaac      360
   gatgacaacc tcttcaaggt cttcccgaaa gagcgctgca ttatcggctc cggcgtgctg      420
   ggcacggctc tgcccgaacc cgcacactgc gtgtccaccc cggccggcgg cgtgcagatg      480
   aacttcggcg gtgcggtccg ctcggagctc accgacgcag cctgcaccga gatgctcaag      540
   tgctaccgtg acggcggttg cgacgcttac tggcgcgacg gcgagccggg caccgacaac      600
   aacatctaca agttcatctg gaaaaaggtc tgcgtgaacg ccactgtcaa caccgtgtgc      660
   gcggtcctgc gtctgaagat cggcgaggtc gaggcggatc cgttcggcca gtggctgttc      720
   cacagcgtca tccgtgagac ctgcgcggtc gcgaccgcca agggcgtgcc gatggacgcc      780
   gatgagttca tcgcccacga tcacaaggac atcgtcacca acatcgcgga ctactatccg      840
   tccatgtgcc aggacatgct cttcaaccac cgccagaccg agatcgacgt gctcaacggc      900
   aagatcgccg agtacggtga gcagctgggc atcgacaccc cggtgtgcaa ggtcctgtcc      960
   caggtcgtgc gctgcatcca gggcaactac gacaagcagt acctgaagta a             1011
<210> 72
   <211> 297
   <212> DNA
   <213> Unknown
<220>
   <223> Unknown gut bacteria
<400> 72
   atgagaaccg cttacgatgt cattatccgt cccatcatca ccgagcagtc catggaagac       60
   ctcgacatca aaaagtacac gttcgaagtc gccaaggacg ccggcaagat cgagatcaaa      120
   aaggccatcg aggagatctt tgacgtgaag gtcatcaagg tcaccaccac gacgatccgt      180
   gccaaggaga agcgcgtcgg tgccaacccc tccggcatga ccgccaccgg caagaaggcc      240
   gttgtgaaac tgtccgctga ttcgaagaac atcgagatct tcgagggcat ggtgtaa         297
<210> 73
   <211> 1113
   <212> DNA
   <213> Unknown
<220>
   <223> Unknown gut bacteria
<400> 73
   atgcataaca aagtccggga accgctgttc cacatcgtca agcgcgacac gctgccctgg      60
   tatcaggcct ggggcatccg cgcggtcgcc atcgtgctgg cgctgctgct gtgcgcgctc     120
   atcacgacgc tgtgcaccca cctcaacccc atcaaggtgt tcggcaccat gttcggcggc     180
   gcgttcggct cgccgcgcaa gatctggatc ctggggcaga acctcgccat cctgctgtgc     240
   atttcgctgg ccgtgacgcc cgcgttcaag atgcgcttct ggaacatcgg cgccgagggc     300
   caggtgctcg ccggcggcct cgccgccgcg gcgtgcatga tctgcctcgg tgacaagctg     360
   cccaacggcc tgctcatcgt actcatcgtg atcgccggca tccttgcggg cgcggtctgg     420
   gccgtcatcc ccgccatctt caaggccaag tggaacacga acgagacgct cttcaccctc     480
   atgatgaact acgttgccac gcagctcgtg gcctatttca tcatcgtctg ggagtcgccg     540
   aagggctcgg gcaaggttgg catcatcaac cagagcaccg aggccggctg gctgccgcag     600
   atcggcggct acaagtacct gctgacgatc ctcgtcgtcg cggtgctgac cgtgctcgtc     660
   tatgtctacc tcaactacag caagcacggc tatgagatct ccgtcgtcgg cgagagcgag     720
   cgcaccgccc gctacgtcgg catcaaggtc ggcaaggtca tcgtgcgcac gatgctgctg     780
   tccggcgcgc tgtgcggcat cgcgggcgtg ctgctcgtgt ccggcacgga tcacacgatc     840
   acgaccacca tcgccggcgg gcagggcttc acggccgtca tggtctcgtg gttggccaag     900
   ttcaatcccc tcggcatgat cttcacgagc ttcctgctcg tgttcctcga ccgcggcgcg     960
   ggcgagatct cgaccgtgtt cggcctcaac cagtcgttcg ctgatatcct cagcggcatc    1020
   atcattttct tcatcatcgg ctgtgagttt ttcatcacct accggctgag cttccgcaag    1080
   ccggcaaaga aggaggcggc ggagcatgtt tga                                 1113
<210> 74
   <211> 1413
   <212> DNA
   <213> Unknown
<220>
   <223> Unknown gut bacteria
<400> 74
   atgtcgaaca gcaacaataa atataaaacc ctcgtgtcca acaccatgct catcagcctc      60
   ggcaccttcg gcagcaagct gctggtgttt ttcatggtgc gcttttacac gagctatctc     120
   acccccgcgg actacggcac cgccgacctc atcacgcaga cggccaatct gctcttcccg     180
   ctcatcagcc tcggcatcac ggacggcgtg ttccgcttcg ccgtggacca caccgagcag     240
   cggcgcaaca ttttctccgt cggcgtgtat acgatcctgg cgggcgctgt gctactcgtg     300
   gccattttgc cgctgctggg gctcttcccg cagtttgacg ggttcctctg gctgatcgcg     360
   ctctatacga tgtgctcgtg cttccactcg ctgtgcgcgc agtttgtgcg cgccgaggga     420
   cacacggcgc tctacgccgt gcagggcatg atcaataccg tgctcgtcat cggactgaat     480
   gtgctcttcc tcgtcggcct gcgcatgggc atcacgggct atgtgctctc cgtcgcgctg     540
   gcggatctgg cgtgcacgct gcttttgttc gtgcgcgagc ggctctggcg tcagctcatg     600
   ctccgcccgg gctgggcggc cgcgcgcgag atgctggcct acagcatccc catgatcccg     660
   acgacggtgt tctggtggat cacgagcgtg tccgaccgct acatggtcac ggcctacctc     720
   ggcgtggacg ccaacggtat ttacgccgtg tcgtacaaga tgccgacgat cctgtcgctc     780
   gtgtcgagca ttttcatgga ggcgtggcag ttttccgccg tgtccgaggc gcagggcagc     840
   cggcgcgagc acatccgctt cttctcgaag gtctggtgct cgttccagtc ggtgatgttc     900
   ctcgccggcg cgtgcatcat cgccttcgcc gtgccggaga tccacgtgct cacgaccgag     960
   cagtattacg gcgcgtggct gtatgtgccg gtgctcggca tggccatggt cttttccgcg    1020
   ttcgtgagct atctcggcag cgtctacatg gtcgaaaagt gcagcaagcg cacgttcacc    1080
   acgtccatga tcggcgcgct gctcaatatc gcgctcaact tcctgctcat cccgtcgaag    1140
   cttggcgtgc agggtgcggc catcgccacg tgtgtgagct attttgtcgt gtttgtcatc    1200
   cgggcggtgg atgcgcgcaa gttcatcccg ttccggtttt acgttgtcgg cgtcacggaa    1260
   aactgcatcc tgctgacggt gcagacgctc ttcatcgtcc tgcagcttcc gggctggaag    1320
   atcgcgcagg cagtgtgcat tttgctgctg ctcctgctca accgcaagcc gcttctggac    1380
   ggcgcgggca agatcgtccg cttccaccgc tga                                 1413
<210> 75
   <211> 687
   <212> DNA
   <213> Unknown
<220>
   <223> Unknown gut bacteria
<400> 75
   atggaagtca tgtctgatct gctcatcaaa ctctggcaaa acggcatctt gcagctcggt 60
   atctgggaga ccatttacat gacgctcatc tccacgttct ttgcctatct catcggcctg 120
   ccggtcggcc tcatcctgcg catgaccgac cgcgacggca tccaccccat cggctggctc 180
   aaccgcacgc tcggtatcat cgtcaacgcc ctgcgcagca tcccgttcat catcctcatg 240
   gtcgccatgc tgccggtggc aaaggccatc gtcggcacca gcctcggcaa ccgcgccgtg 300
   atcgtgacgc tcgtcatcgc cgccgccccg tatgtggcgc gcatggtcga gtccgccgcc 360
   aaggaagtgg acgccggtgt catcgaggcc gcgctgtcca tgggcgcgtc cacgctgcgc 420
   atcatctgga aggttatcat cccggaggcc aagcccgcgc tcatcaccgg cgccgtcatc 480
   tccaccgtga ccatcctcgg ctactccgcc atggccggca cgatcggcgg cggcggcctc 540
   ggccagattg ccatcaccta cggctaccag aagtacgacg atcagatcgt ctggctgtgc 600
   gtagcgctga cgatcatcat cgtgcagatc ctgcaggagt tcggcatgta catcgcccgc 660
   cgcaccgacc ggcgctcccg cacctga 687
<210> 76
   <211> 696
   <212> DNA
   <213> Unknown
<220>
   <223> Unknown gut bacteria
<400> 76
   atgctcatac acggcctgca aaagctgacg ctcctggatt accccggcaa ggtagcctgc 60
   accgtattca cagggggctg taatttccgc tgtcccttct gccacaacgc gagccttgta 120
   ttaaggccag acgagggcga gcacatccct gaggaagagc ttttcgccct gcttaaaaag 180
   cggcagggcg tgcttgaggg agtatgcata accggcggcg aacccaccct ggccttcggt 240
   ctggagacgc tgatggcaag gataaaggag atgggctatc ttgtcaagct ggataccaac 300
   ggcacccggc caaaggccgt aaagcggata atagacgcgg gccttgcgga catgatagcc 360
   atggacataa aaaacagcta tgaaaaatac cccgagacct gcggcatatc cggctatgac 420
   acggcgccga taagggaaag catacagctc ataatgtcct ccggcataga ctacgaattc 480
   cgcacaacgg taatgcggga attccacacc cccgatgata tccgcgccat agcgaaggaa 540
   attcaggccg caaagggcta cttccttcag gcctaccggg attccggcga catactgaag 600
   ggcggctgct cccgccccac ggacggcgag ctgcacgcgc tgctttcagc cgcaagggaa 660
   tacctcccca atgcggagct gagaggtgtg gaatag 696
<210> 77
   <211> 723
   <212> DNA
   <213> Unknown
<220>
   <223> Unknown gut bacteria
<400> 77
   atgaagatga gtagcctgct tgaaatacgc gatcttaaag taaactacgg cggcatagag      60
   gcgctgaagg gcgtttcctt cgacgtgaag gagggggaga tagtcaccct cataggcgcg     120
   aacggcgcgg gcaaatccac cacgctgcgg gccataatga gcgtcgtcaa ccccgcctcc     180
   ggagccatca gctacgaggg caaggacatc accaacgccg ccacacacga tatagttcag     240
   cgcggcatag agctggtgcc tgagggccgc cgggtgttcc cgaaccttac cgtccttgaa     300
   aacctgcgca taggagccta tctccgggag gacaaggacg ccatagagca cgacatagaa     360
   cacgtgtaca gcctgttccc aaggctgaag gagcgccact ggcagcaggc cggcaccccc     420
   tccggcggcg agcagcagat gctggccgtt ggccgcgcgc tcatggcaaa gccaaaggtt     480
   atgatgatgg acgaacccag ccttggcctt gcgcctctgg tggtaaagga tatattctcc     540
   ataatccgcc gcatcaatga cgacggcgtg accgtcctcc tcatagagca gaacgccaac     600
   gcggcgttga agatagccga ccggggctac gtcctccaga ccggcaacgt gaccatgacc     660
   ggcaccggca tggagctgct caacgacgag agcataaaag aggcctacct cggaaagaaa     720
   taa                                                                   723
<210> 78
   <211> 477
   <212> DNA
   <213> Unknown
<220>
   <223> Unknown gut bacteria
<400> 78
   atggaattca cccactttaa ccatcagggc cgggcccgca tggtagacgt gtccgaaaag        60
   gcggataccc tccgcaccgc cacagccaga gcccgcgtgc ttctcaatgc cgatacctac       120
   gcactcgtaa agtccggcgg cataaaaaag ggggatgtgc tcaccaccgc acagatagcc       180
   ggcataatgg gcgcaaaacg caccccggac gttataccca tgtgccatcc catagccctg       240
   accggcgcag acatatgctt tgagctggac gacgaaaacc acacaataga catattcgcc       300
   acgacgaaat gcaccggccc cacaggcgtt gagatggagg ccatgacggc agcaagcata       360
   gccgccctga cagtttacga tatgtgcaag gcggcgcagc gggatataat tattgaaagc       420
   atttgcctat tgcgcaaggc ggggggaaaa tccggtgagt tcaggaggaa ggaataa          477
<210> 79
   <211> 1056
   <212> DNA
   <213> Unknown
<220>
   <223> Unknown gut bacteria
<400> 79
   atggcggata tactgaacaa tatgataccc gtaaagcgcg ccggaaggca gacggtgctg        60
   ttttccaacc ctccggcgat aataagctcg gctaccgttg taggcccaat ggagggcaag       120
   ggaccgctgg ggccatactt tgatatggtg ctcaaggacg atacctgggg cgaggacagc       180
   tgggagaagg cggaacgcaa aatgttcgag cacactgtaa ggggggcgat ggacaaggtg        240
   aacctccaaa gcggcggcgt ggactgcctg ctgggcggcg accttctgaa ccagataatc       300
   tccgcaaact ttgcggccag agaactgaaa ttaccgttct taggcctgta tggcgcctgc       360
   tctaccatgg cggaatcact cctaataggt actatgctca tagacggagg gtatgccggc       420
   agggtggcct gcatagctac cagccatttt tccaccgccg aaaggcagta ccgcaaccct       480
   ctggagatgg gtggccagac tacgccgacg gcacagcgca cagtgaccgg cgcaggctgc       540
   agcatagccg caaatccgac tctggaaggc gacagactgt atggcaatgt atttgtaact 600
   gcgggtacga taggcaaggt tacggatctc ggcatcaccg atgtcaacaa tatgggagct 660
   gcaatggcgc ctgcagcggt ggacacgctg acagcgcacc ttagcgatac cggcagggga 720
   gtaatggact atgatttgat agtgaccggc gacctcggaa gattcggcag cgagatgttc 780
   agggagctgt gcctgcaaaa cggaatagac gtgggcaaca ggcaccttga ctgcggcaac 840
   ctgatattct ccggggagca gaaggtggac tgcggaggca gcggctgcgg ctgctgtgca 900
   agcgtgctta acgcatacct gcttgagcgc atagaaaagg gcgatctgag gcgcatattc 960
   ttcatggcga cgggggcgct gctgagccct accagcagca tgcagaacga cagcataccc 1020
   ggcatagccc acggtatagt gctggaaagg aagtaa 1056
<210> 80
   <211> 2667
   <212> DNA
   <213> Unknown
<220>
   <223> Unknown gut bacteria
<400> 80
   ctgtcaacag gcagggcggt attgctggtt gcgccaaacg aggtggcggc ggtaaagctg 60
   catgacgaca tcgcctgcta tgatataccc tgcgcccact tcccgaccag ggagataccc 120
   ctgtccggca agggctttgc cgcccgtgac agcatagagg aacgaagggt agcggtgctg 180
   tctgcacttg ccgccggcaa gaccatgacg gtggtgacat gcatacaggc gctgatgcag 240
   cgcaccgtgg ccccggagat aatcaaaacc agcctgcaca gctatgaggc ggggcagacc 300
   atagaaccac gggatatggt atcggagctt gttatggcgg gctatgagag ggttgacgta 360
   tgcgaggcgc cgggacaggt atgcctccgc ggcggctatg ttgacgtata tcctatcgcc 420
   gctgaaaatc ctgtgcgtat agagtttttt ggcgatgaga tagatacgct caggatatac 480
   gatccgctta cacagcgctc tgtggataat gtggaccaca tagacgtccc tccggctacc 540
   gagatgccca taacagacga ggcaagagcg cgagcgctga agcttctcaa aaagcgcaag 600
   gcggaggaac tggcctcggc gctggaggag gggggcaggc cggataattc agttgccctg 660
   ctgcctctgt tttaccccgg ggaagcgacg cttttcgatt acatagacaa ggactggata 720
   atactcatag acgagccggc ccgccttgag gaaagcggaa agatagccta tcaggagttc 780
   ctcgagggcg tatcctcgct tatgcaggcg ggagagatac agccggagca ggccaacctt 840
   acggtatccc ctcttgaaac catcaagcgc atggatacca gaaggaccgc catgatgttc 900
   gccctgaccc gcagctacgg ccttataggg cagaggtgca tattcagatt cgatacccgg 960
   ccggtttcga gatacgtagg ccgggaggag ctgctggccg aggatataga ggcgtggaaa 1020
   aagagccata cgagcgttct gatatacgcc ggtacccacg ccaaaaggct tcaggacaga 1080
   ctgctggatt caaaccacat agtacctata aaggaaaagc tggaccggga cattgtgccg 1140
   ggcgaggtgc tgataataga gcagcagctg aaccacggct tcgagtaccc ggagctttcc 1200
   cttgcggtag tcacggaaaa cgagctttac ggagccgaaa accgaaggcc tgcggacaat 1260
   aagaaaaagc gcaggaagcc gcagctggtg ttcagcgagc tgaacgtggg cgaccttgtg 1320
   gtccatgagc ttcatggcat aggtcggttc gtaggcgttg agacgctcac ggtaggcggc 1380
   gtacagaagg attatctgca tcttgtctat tccggcggcg ataagctgta catacctacg 1440
   gaccagctgg accgggtgca gaagtacatc ggcggcggag atgatgaggg tacgcagcgc 1500
   ctttcaaagc tgggcagcgg cgaatggcag aaaaccgtat ccaggacccg cgacagcgta 1560
   aagaagctgg ccttcgacct tgtgcgcctt tacggcgaga ggcagaaacg caaaggcttt 1620
   aagttttcgc cggatacccc ttggcagaga aagctggagg atagctttcc gtatgagcct 1680
   acgccggatc agctcacaag cattcaggag ataaaggcgg acatggagag cgacaaggtg 1740
   atggaccgcc tgctctgcgg cgatgtgggg tatggtaaga ccgaagtagc gctcagggct 1800
   gcctttaagg cagtgatgga cggcaagcag tgcgcaatgc ttgtgcctac aactatactt 1860
   gcccagcagc attataatac gatagccgcc aggttcgacg gtttccccgt gaaggtggag 1920
   cttctctccc gctttaagac ccccaaggag gaggaaaaga taatcgaggg gctgcaaagc 1980
   ggcagcgtgg acatggtggt gggcacccat aagctgctct caaagagcgt aaagttcaag 2040
   gatctgggcc tgctgataat agacgaggag cagcgcttcg gcgtaggcca taaggagcag 2100
   ataaagaaca tacgccagtc cgtggacgtg ctgacgcttt ccgccacgcc tatcccgcgg 2160
   acgctgcaca tgtccatgac gggcatacgc gatatgtccg tgatagagac gccgccctcc 2220
   cagcgctatc ccgtgcagac gtatgtaatg gaatacagcg attctgtaat ccgtgaggcc 2280
   atactgaagg agattggacg cggcgggcag gtatacttcg tatataacaa cgtccgcagt 2340
   atggagcagt tcgcaggaag gctcagggag ctggtgccgg aggccagagt gtgctttgct 2400
   aacgggcaga tgaacgagcg ggtgcttgaa aagaccatgc tggaattcat ggatcacaag 2460
   tacgacgttt tgctttgcag caccataata gagagcggac tggatatgca gaacgtgaac 2520
   acgataataa tatacgacgc ggacgccatg ggcctatccc agctttatca gctcaggggc 2580
   cgcgtgggcc gcggggtgcg gctcggctat gcctatctga catacaggcc gaacaaggcc 2640
   atgagcgaga ctgcggaaaa gcgcctt 2667
<210> 81
   <211> 1113
   <212> DNA
   <213> Unknown
<220>
   <223> Unknown gut bacteria
<400> 81
   cccaagggca tggtgctgcg cagcaccctg ctcgaatact ggcgcgaggt gcataaacga      60
   tacggctatg tggaaatctc cactcctata attctcaatc aggagctgtg gcacaggagc     120
   ggccactggg atcactacaa ggacaacatg tacaccaccg taatagacgg cgaggattat     180
   gcgataaagc ccatgaactg ccccggcggc atgctggtct acaagcttga gccccattcc     240
   taccgcgagc tgcccctgcg catggctgag ctcggcctcg tacaccgcca tgagctttcc     300
   ggcgcgctcc acggtctgtt ccgcgttcgc tgcttcaccc aggacgatgc ccacatcttc     360
   atgacctggg atcaaatgga gcatgagata cagaacgtcg ttcgtctgtt cgacgaagtt     420
   tactccacct tcggcctgac ctatcagatc gaggtttcca ccatgccgga ggatcatatc     480
   ggcgataagg agacctggga ttttgccacc gataccctca agaaggccat agagcatatg     540
   ggcaagagct acgtcataaa cgagggcgac ggcgccttct acggccccaa gcttgacttc     600
   catctttccg actccctggg ccgtacctgg cagtgcggca ctatccagct ggatatgcag     660
   atgcccgaaa gatttgatct tgaatacgta ggcgaggacg gccaaaagca ccgtcccgta     720
   atgatacacc gcgtcgtatt gggctccatc gagcgcttca taggcgttat caccgagcac     780
   ttcgcaggcg cgttccccac ctggctggct cccgtacagg tgaaggtgct gccccttacc     840
   gaccgtaccg cccagatatg cgacaatatc gcttccgagc ttgacgagct tggtctgcgt     900
   gtagaagttg atcaccgtaa cgaaaagctg ggctataaga tccgcgaagc acagctccag     960
   cgtatcccct atatgctggt cgtaggcgac cgcgacgttg aaaacggcgt tgtatccgtc    1020
   cgctcccgca agggcggcga cctgggcgca atgactctcg acgccttcaa ggaaaagata    1080
   ctggaagaag taaagaccag agcccgtgac tga                                 1113
<210> 82
   <211> 1641
   <212> DNA
   <213> Unknown
<220>
   <223> Unknown gut bacteria
<400> 82
   atggaagaga tacgcgcgcc aaagaacttt atcgaggaat tcatcgaaga ggacataaag 60
   gaaaagggac ttacccacat acagacccgc ttcccgccgg agcccaacgg ctacctgcac 120
   ataggccacg caaaggcgct ggccatcgac ttcaccatgg ctgaaaagtt cggcggcagc 180
   tgcaacctgc gctacgacga caccaacccc accaaggagg acgaggaatt cgtggacgcc 240
   atacaggagg acatagcctg gctgggcttc cactgggaca acgtatttta cgggagcgac 300
   tacttcgaag agtgctatga gctggcggtg aagctcatca aaaaggtcgt ggcctacgtg 360
   tgcgaccttt caaaggacga gatgagggaa taccggggaa cccttaccga gccgggcagg 420
   aactccccct ggcgggacag gagcgtcgaa gaaaacctcg acctgttcca gcgcatgaag 480
   gacggcgaat tccctgacgg cagcaggacg ctgcgcgcca agatagacat ggcgtcgccc 540
   aatctcaata tgcgcgatcc cgccatgtac aggataatac acatgaccca tcaccatcag 600
   ggggacaagt ggtgcatcta ccccatgtac gacttcgccc atcccataca ggacgcaata 660
   gagggcgtta cccactccct ctgctccctt gagtacgagg cccacaggcc gctgtataac 720
   tgggtcatag agcagtgcga attcgagcac aagccgaggc agatagagtt cgcccggctg 780
   aacctgacca ataccataat gagcaagcgc tacctccgcc ggctggtgga ggaaaagtac 840
   gtcgccggct gggacgaccc ccgcatgccg accctgtgcg cgctcaggcg caggggatat 900
   accccggagt ccatactgga cttcctgaat cgggtaggcg tggccaaggc ggattccgtg 960
   gttgagacag ccatgctcga acactgcgta agggaggacc ttaacgccca cgccccccgc 1020
   atgatggcca tactcgaccc cctgaagctc atcgttgaga actggccgga aggcaaaacc 1080
   gaggaggtgg aggtggagta cctgcccgac tgctccacag gcaagcgcag ggtaagcttc 1140
   ggccgggagc tttacataga gcgggaggat ttcaactgca acgcccccaa gaagtatttc 1200
   cggctcaagc ccgaatgcga ggtgcgtctc aagggcgcat acgtggtcag gtgttcccac 1260
   tgggacacag acgaaaacgg caatgtcacc acggtatact gcacctacga ccccgcgaca 1320
   aggggcgggg agtgcgagag aaagataaag ggcaccatac actggctcaa cgccaaggac 1380
   gcgaagcccg ccgagatacg cctttacgag gacctgctgc tgcctgaggc ggagggcgag 1440
   gagaaggaat ttacggagcg gctcaatccc aattcgctga cagtcgtcaa cgctctggtg 1500
   gagcccgagc ttgcccaaaa ggagccgggc taccgctgcc agttcatgcg caccggctac 1560
   ttctgcgccg accttgacta tactggggag cgccccgtgt tcaacaggat agtcggcctc 1620
   aaggacagct ttaaggcata a 1641
<210> 83
   <211> 3201
   <212> DNA
   <213> Unknown
<220>
   <223> Unknown gut bacteria
<400> 83
   gatttctccg tgacaaagct ggacggcgct cccgcaggtg aagtacagga cttttattat        60
   tacaataatg caaacgcaaa cagtgaaagc ggcagccaca acgtgaatga gcttcacggc       120
   ggcgccatgc tccttgccgt gctgctaagc ggcgtgctcg gcgccatatg ctacaggaag       180
   ggcatactca aaaagcccga gcccgatggg gaaggcgtca atgtctgcac gctgctcgtg       240
   ctggcgtttc ttatgcgctg cctgctgtcg ctggtatttt acggccacag cacggacata       300
   aactgcttta tggcatggag ctaccacctt gccgaggacg gattggcgaa cttctataac 360
   agcggtatgt tcgcggatta tccgcctggg tatatgtaca tactctggct taccggcagc 420
   atagccagag cgctcggcct tgaatacggc ggcgcgggat atgtgctgat aaccaagatg 480
   ccaagcatac tggcagatat attcgctgcg tatatgacct ataagctggc gaggaataag 540
   ctgacaggga gccagtcgac gcttttgtgc tgcatagtgg cgttcaaccc ggctatggcg 600
   ttcatatccg gcgcctgggg gcaggtggac atgatacctg ccataatgct gctgggcgtg 660
   atatatctat ttgatagcgg caaactggag cttgcgggcc ttctttacgg agcggctatc 720
   ataaccaagc cacagtctct catgataggg ccgctgcttg cggtggcata cttcgcgaag 780
   gtgtacgata agggctggag gtatgccctt cgtacattgg cggcggtagc gttggctgta 840
   atggccatat tcgctctggc atggccgttc aagggggcgc agcagccgct gtggttcatg 900
   gacaagctgc tgggtaccgc cacgtcctac ccctatgcaa gcgtagaagc cttcaacatg 960
   ccggcgctgc tggggggcaa ctgggcagat gtgaacgcac cgctgctggg cctgacctat 1020
   ggcaaatggg ggagcattat gatagcgctc tcatgcatag ccgcagccgc aggatacata 1080
   aggtcacggg ataaaaaata cagcctcact ctgtctgggg cgctgctgct ctccggtata 1140
   ttcaccttcg gccagtatat gcatgagaga tatattttcc cggtgctgcc gctgctcatt 1200
   gcggctttcc tgctgggcgg ggacaggggg gtgataaaga cgtatgtgat atatacctgc 1260
   gctctgctgc taaacgtcct tgccgccttt gtggtggtga aaagcgcagc gctcaggggc 1320
   gctgagtata atgtgataac tctgataggc tctgtgataa acctgatggc ctttgcaagc 1380
   atgattactg cctattggag catgactgta aggcgggaga tatatcccgc atattgtgag 1440
   gaaagaatgg aaaagacgga cggaaccgta tatgaaatac agggtgatac gcctgtcgtt 1500
   accgtgaggg acaggctgta ctgctttgcc ctgacgctgg tgtacgccat aatcgccctg 1560
   acgaacctcg gcacaacgca ggcgcctgag aattactgga taggcagcgt tggtgatacg 1620
   gcggccataa cccttgaaag ccctgaagag ataagcgaga taaggatatt cggcggaata 1680
   tatgagggaa gggcaactct ttccttcagc gacgggcaga gcatagaata cgttcaggag 1740
   aacgacgata tgttccgctg gataaccgtg agtggcgatg acgcggttta taccgataag 1800
   ataggcatag aggtaacgga gggcagggta tggataaatg agatcgccct gttcgacggc 1860
   gacggcaata taataaaatc cgctgcgtcc gaaggggcgg aggctcttgt gaacgagcct 1920
   gaggagatac cggcaactcc ttcttacctg aacggaatgt actttgacga actgtaccac 1980
   ggccgcacgg cctacgaaca cctgcacggc atagccccat acgagaactc tcatccgccg 2040
   ctcggcaaga tattcatcat gctgggaatt gccatattcg gcatgaacgc cttcggctgg 2100
   cgaataatag gaacgctgtt cggaatagcc atggtaccga taatgtacgc cttcggcaaa 2160
   aagctgttcc gccgcagcga ctatgcgctg ctttccgccg gcctgttcac cttcgacttc 2220
   atgcacttta cccagacccg catagccacc attgacgtat acggggtatt tttcataata 2280
   ctcatgtact acttcatgta tgactattac tgcatgaact tctttgacga cggcctgaag 2340
   gccacattga agccccttgc catagccggc ctgttctttg gcctgggcgc cgcaagcaag 2400
   tggatatgca tatacgcagg cggcggcctc gcggtgatac tatttacctc gctgggccag 2460
   agatacgccg agtacaggcg cttcaaaaac gcccccggca aggcggagcg ccgcagggtg 2520
   cagggcttct ggcggaatac cctaatgacg ctgctttggt gctgtgtatt ctatatcgcg 2580
   gtgcctgtgg tgatatatct gctgtcctac ataccctatg tgctctgtga gaatcattac 2640
   gggctcagcg gtatatggga cttccagaaa ttcatgttca actatcacag cggtcttacc 2700
   gctacccatc catacgaatc cccatggtgg cagtggcccc ttgaccttag gccggtgtgg 2760
   tattacataa gctacagggt tccggagggg atgacttcta ccatatccgc ctttggcaac 2820
   ccggcggtag ggtgggtgtg ctcagcctgc accggaatac tctgcgcgcg gctgatcgcg 2880
   ggcaggtcta agccggacaa gggcatattt gtgctgctga tcggtttggg ggcgaattac 2940
   ctgccctggg tgctggtaat tcgctgcacc ttcgcctatc acttttttgc cagcgttccg 3000
   tttatcgtat tcataaccgt atggctgata cgtgaatttg acgagcggca ccctaaactg 3060
   cgtcggatga aatgggcgta tctgggcctt gtggtgctgc tgttcgcgct gttctacccg 3120
   gtgatatccg gcgcgcctgc aagcatcgga tacgtaaggg cctttgagtg gctgcccggc 3180
   tggacattta tggggtattg a 3201
<210> 84
   <211> 1146
   <212> DNA
   <213> Unknown
<220>
   <223> Unknown gut bacteria
<400> 84
   atggaaatca ccgttcaaga gcttgctgct ctcgacccca agggatacgt gctgctggat        60
   atgcgtggtt ccgaggccag ggagtacggg tcaatacccg gctccgttgg agtttccccg       120
   gaggagctgt cccggtataa cggcgatagg ggcaaaaggg tgataatata ctgtgcccgc       180
   ggacaggtca gccttgacgc ggccatagcc ctgcgggaga gaagctatga cgcatacagc       240
   ctctccggcg gctatctcgc ctggctgcag gacgagatgg aacagcagga ggcggcagcg       300
   ctgtgccgtc aggtcgagga aagcattcgc aagcgcttcc acagcaaaat ctggtgctgc       360
   ttcacccggg ccatacggga gtatgagctt gtaaaagagg gggacaggat cgccgtgtgc       420
   atatccggcg gcaaggactc catgctcatg gcaaagctct ttcaggagtt aaagctccac       480
   aacaagttcc ccttcgaggt ggagtttctg gtgatggatc cgggctacag cccggagaac       540
   cgccgcgtaa tagaggaaaa cgccagaaag ctcaacatac ccatacacat attcgaatca       600
   aacatattcg agtcggttta ccatgttgaa aagtccccct gctacctctg cgccagaatg       660
   cgcagggggt atctctaccg ctacgcacag gagctgggct gcaacaagat agcattgggc       720
   caccactatg acgacgttat agagactata ctcatgggta tgctgtgggg ggcgcaggtg       780
   cagaccatga tgcccaagct ccacagcaca aacttcgagg gcatggagct tatacgcccc       840
   ctttactacg tccgggagga cgccatcaag gcgtggcggg actgcaacgg cctgcacttt       900
   atacagtgcg cctgcaagtt cacggacacc tgtaccacct gcacaaataa tgagacaaag       960
   tccaagcgca tggaggtaaa gaacctcata cgggaaatga aaaaaacaaa ccccggcgta      1020
   gaggcccata tattcaggag catggaaaac gtcagtctgg acgccgtcat agcctacaag      1080
   actaagggaa ggaaaattca ttttttagat aattatgaca accctgacga aggcattgaa      1140
   gaataa                                                                 1146
<210> 85
   <211> 654
   <212> DNA
   <213> Unknown
<220>
   <223> Unknown gut bacteria
<400> 85
   atgataagct tttccgacct tttcaccatc acaatgaacc ccgttcaggt aatactgacc      60
   ctgatagttg gcctgctcgt gggcctttac gtatggttca tatataaaaa gaccttcagc     120
   ggcgtcatgt attcccgcac ctacggcatg acgctggtaa tgatgaccat agttacggcg     180
   ctgatgctga tgctcatcaa caacagcctg accctcagcc ttggcatggt gggcgccctg     240
   tccatcgtcc gctaccgcac cgccataaag gatccccttg acaccgcctt catattctgg     300
   gcagtgggcg agggtatagc cgtgggcaca aagttctacg acgtggccat aatcgccggc     360
   ataataatag gcatattcgt gctgctgatc tcaggctcaa ggggcaaggg tggcgctcag     420
   agctacctcc ttataatcca ctacagcgag gccgcggcag acgccatacg caagatgacc     480
   aagcagcttc ccggcgcccg gctcaagagc aaggtagtgc gccccggcag catagagctt     540
   accatggagc tgcgcctcaa ggagaacgag acgggcttcg tagagaagtt cctccgggtg     600
   gagggcgtgt acgatgcaag ccttatcact catcagggag acatagtggc gtag           654
<210> 86
   <211> 1848
   <212> DNA
   <213> Unknown
<220>
   <223> Unknown gut bacteria
<400> 86
   atgcttcagg aaaagagctt gaatgatctc agggagatcg ccaagcttca gggcgtaaag      60
   tcactcacaa aataccgcaa gaacgagctt gtggacatca taatgaacgg cggagcgata     120
   ccctccgcgg tgcagcagcc agctgagccg ccggctcccc agctcgaagc ccagcctgat     180
   ccacagcccg ccgcccctgc atccgggcag ccaatacagg ccgcagaaca gccatctgag     240
   gaagcacacg tgcggcacag cgacgttctg cacgaagctc ctgcacagcc ccgctctcag     300
   tatatacctg cgtatcaggc acagcagccc cgtcagatgg gctatcagca gcagtaccgc     360
   aaaccctatc aacagaatta tcagcagcgg caggggtacg gctacaatca gaaccgttat     420
   cagcagcagg gctatcaggc atacaacagg gactactccc agcagagtta tcagccttct     480
   tataacaggg actattccca gcagggctac cagcagccct cctaccagca gggctatcag     540
   cagcagagct atcaggccgc ctatgagcag cagtccgagg aaagcttccg caggccagac     600
   cgtcccgagg gctactacaa caaagaatac ggcaccagca atcccgcagt tcccggcctt     660
   ctcgagggcg gagaatgcgg cgattgcgaa ggcgtacttg aagttctgcc cgatggctac     720
   ggcttcctcc gcagcgacaa ctatctgccg ggcaacaagg acatatacgt ctccatcgcc     780
   cagatacgcc gcttcggcct ccgcacaggc gatctcgtca ccggcaagac ccgtccaagc     840
   aaggagggcg agcgttttct cgcgctgctg tacataacag ccataaacgg cattgatccc     900
   gaggaggcca tgaagcgtaa gccctttgag gagcttgtgc ccatataccc caatgagcgt     960
   ctcactctgg aatatgacga cggcaccggc gcaaaggacc agaatctggc cattcgcctg    1020
   gtagacctca tagcccccat aggcaagggc cagcgcggcc ttatcgtcag ccagcccaag    1080
   agcggcaaga ctacgctgct caagaacata gccaacggaa taaccaacaa ctatcccgac    1140
   gttaccctta tagtgctgct catagacgag cggcccgagg aagtcacgga tatgcagcgc    1200
   agcataaagg gtgaggtact gtactccacc tttgatgagc tgcccgagca tcatacccgc    1260
   gtagccgaaa tggcgcagga gcgcgctatg cgcctggtgg agcagggcaa ggacgtggta    1320
   gtcctcatag actccataac ccgccttacc cgcgcctaca atattactat ccccgccacc    1380
   ggcaagactc tttccggcgg tatggatccg ggagctctgc ataagcccaa gcgcttcttc    1440
   ggtgctgccc gcaacatcga aaacggaggc agcctaacca taatcgctac cgccctcatc    1500
   gagaccggca gccgcatgga cgatatagtt tatgaggaat tcaagggcac aggcaacatg    1560
   gagatacacc ttgaccgcaa gctctccgaa aagcgtatat tccctgccat agacatctac    1620
   aagagcggta cccgccggga tgacctgctt ctctccccag acgaaatgga cgcagtgtac    1680
   atcatacgca aaatgctggg caacggcaat cccgcggaga tcaccgagca gctcatcgcc    1740
   atgcttgaaa agacggaaaa caacaaggaa ttcgtcgcca agatacgggc atggaacagc    1800
   gcctatgaga aggacggctt caccacaggc aagaccttca acaaataa                 1848
<210> 87
   <211> 894
   <212> DNA
   <213> Unknown
<220>
   <223> Unknown gut bacteria
<400> 87
   atgtgcgatc gtttttttat agtgaacccg gtgtccggct ccggcaagtc aaggaagctc      60
   tttgaggagc tgcttcccca gatagaaagg agcggcagga gcttcgagtg gcggtatacc     120
   accggcaagg gccacgcaag agagttggcg gaagaagcct ccggaaaagg ctgccaacgg     180
   gtgatatccg tgggcggcga cggcaccgca aacgaggtgg ccgcggcgct tataggttcg     240
   gatacagaga tgggaatact tccctttggc acaggcaacg actttgctaa ggcggtgggc     300
   ctgcccaccg atcccaatgc agcgctggag gcggcgctgt acggccaggt ccggccggtg     360
   gacgccgcaa tggcaaacga caagcccttt ataaacgtca gcggaacagg cttcgacgtg     420
   gacgtgataa tcaacaccga gaagtttaaa aagaggttca acggaatgct gccctacatg     480
   ctgggcatat tccagagcct tatgcacctc aagcccatca ccttcgagat aacggcggac     540
   ggggaaagct ttaccgagcg ggcgctgctc ttcaacgcct gcaacggcac ccatttcgcc     600
   ggaggaatgc acgttgcgcc catggcgagt ccctatgacg ggctgctcga cgtatgcata     660
   ctgcgggcca taagcatacc gcagtttctc ctgctgctgc cccgctatac caggggcgcc     720
   cacacagctt caaggcacgt gcgctacttc aaggcgaagg aaatagcggt aaggtgcgac     780
   agcgaaaaca taatcaacct tgacggggag ctggattcct ctacccccgt aacatttaag    840
   gtattgcccg gggcgataaa gcttgcgctt ccgggagaaa aagggaacaa atga            894
<210> 88
   <211> 792
   <212> DNA
   <213> Unknown
<220>
   <223> Unknown gut bacteria
<400> 88
   atgaacgaat taaggaacaa aaagggtttt atatgcgata tggacggcgt gatttaccac      60
   ggtaacaagc tgctgcccgg cgtaaaggag ttcgttgact ggctgtacag ggaaaacaag     120
   aacttcctct tcctcaccaa ctcctccgag cgctcaccca aggagcttca gcaaaagctc     180
   aagcgcatgg ggcttgatgt ggacgagagc catttctata caagtgcgtt ggcgaccgca     240
   cgcttcataa gctcccagtc gccggaaagc agcgcctacg taataggcgg cgcaggcctc     300
   ataatggcgc tgcacgacga aggcatcacc atgaacgacg ttgacccgga ttacgttata       360
   atcggcgagg gtaataccta caactacgag aacatactga aggcggtaca cctggtgatg       420
   aagggcgcaa agctcatcgg caccaacagc gaccttacag gcccctctga gaacgggata     480
   atccccgcat gccgggcaat gatcgcccct atcgaaatgg ccaccggcca gaacgcctat     540
   ttcataggca aacccaaccc tcttatgatg cggacaggcc ttaggatact gggcgtacac     600
   tcagaggacg cggtaatgat aggcgatcgc atggataccg acatgatcgc gggaatcgag     660
   actggccttg acacctgcct tgtgctctcc ggcgtgaccg accgcgccga catcaataag     720
   ttcccctacc gtccgcggct ggtgttaaac ggggtaggcg atatacctga caagtcaaag     780
   gaaatagtgt ga                                                          792
<210> 89
   <211> 684
   <212> DNA
   <213> Unknown
<220>
   <223> Unknown gut bacteria
<400> 89
   atgttcggca agaaggagct tcagagggaa cttgaacaga aggagcagca gatacaggag        60
   ctgaaccgca ggatcaagga ataccaggac aaaaacgacg cggtagtaag cgccctcacg       120
   gacgcgaagt ccgccgcgaa ccgcataatt ggcgcagcgg aagcaaagcg cgatgatatc       180
   atcgccgccg ccatgcgtga aaaggccgat atggaggccc aaagggacgg aatacttgac       240
   gaagcccggg cgcaggccgc ggctatcgtt gcagatgccg agaagagggc aaaggctgcc       300
   gaggagcagg ccaggtactt tcaggattat atcaggaaca ccgcagatac ggtgcgccgc       360
   caagccgaaa actacgccgg cttccttgaa aattacgagc atatcggcgt cggccagcag       420
   ccggagacgg aaaagataga tactccggac gagtaccgga accctgcgga gctcataaag       480
   agcatataca atatcgaggg ccgggagctt cccgatgagg tgacgaagcc cgacgcgggg       540
   caggagccag aggaggaaaa ggtatggacc gtggacgaag tcgtcagcga ggaaaagcat       600
   gacgatgacg acgctgacaa agcagatgac ggccttgaca gcgagctcaa cgacatactg       660
   aacgatatat taaagctgga ttga                                              684
<210> 90
   <211> 1113
   <212> DNA
   <213> Unknown
<220>
   <223> Unknown gut bacteria
<400> 90
   atgacagatc cgaggatcaa gacacttgca aaaaacctca taggcttttc ctgcgccgta        60
   aagcccggtg aaaagatact tatcgagggc aacggcgaat gtaccgagct ggtaaaagcg       120
   ttggtcaagg agacctatgc cgcgggcggc gtccccttcg tatggataaa gaagcccgag      180
   gtatgccggg agcttgcgct gggcgcgaca aaggagcagc aggagcttat ggcaaagata      240
   gactgcgagc tcatgcgcca catggacgcg tatatcggca taggcgcgaa gctcaacact      300
   aacgagatgt ccgacgtgcc gggggataag ctggccctca taagcgccgt atacggcagg      360
   ccgctgaaca acattcgcat acccaacacc aagtggtgcg tgctgcgcta tcccaacccc      420
   aactttgccc agatggcagg catgagcacc gaggccttcg aggacttcta ctttaacgtt      480
   tgcaacctcg actatgctaa gatggacaag gccatgcagc ccctcaagga gcttatggaa      540
   aagaccgaca gggtgcatat agtgggcccc ggcacagacc tccgcttctc cataaagggt      600
   atgcccgcca taaagtgctc cggccagatg aacattccgg acggcgagat atataccgct      660
   cccgtagacg gcagcgtaga gggaacaata acctacaaca ccccctccat aatggacggc      720
   ttcacatatg agaacgtcag cttgaccttc aaaaacggcc gcatagtgga atccacctcc      780
   aacgacaacg agcgcataaa ggccgtattc gacagggacg cgggcgcacg gggcgtgggc      840
   gaattcgcca taggcgtgaa cccctacgtc accttcccca tgagggacac cctcttcgac      900
   gagaaaatat ccggatcctt ccactttacc ccgggctgct gctacgacga ttgcagcaac      960
   ggcaacaagt cctgcataca ctgggatctc gtatgcatac agacccccga atacggcggc     1020
   ggcgagatgt acttcgacga cgtactgatc cgcaaggacg gcaggtttgt actgcccgag     1080
   ctggattgcc tcaatcccga gaaccttaaa taa                                  1113
<210> 91
   <211> 774
   <212> DNA
   <213> Unknown
<220>
   <223> Unknown gut bacteria
<400> 91
   atgcttgtaa acactcccat agcaaacctc tattcagggc cttcggttgg agagctggac 60
   gaccagatac tctacggaga ggaagccgat gtgctggaga gaagcaacgg ctttgccaag 120
   atacgcacgg aatacggcta tgaaggctgg atagaggaaa ggcatcttgc tatccggcgg 180
   tatgacggcg caataatgcg ggtcgtaact cccttctgca cagtgctgga tatacccaaa 240
   tatcagggca ggacggtggt gacgctgccc cggggcgcta aggtcgaaat gacgggtgag 300
   aataacagct ttgcccaggt gcggctgata ggcggagaaa agggctacat acgccgcagc 360
   catatagcgg ccctgccggg aaagctgagg gaggctgagc tgcgccgggc cgtgacggag 420
   actgccaaag catatcttgg ggtaccatac ctctggggtg gaagatcgtc cgtggggatg 480
   gattgcagcg gcctgtgcca tacggcatac tggctcaacg gcgtggccat ataccggaac 540
   tcacgcatgg cggagggctt tccggtgaag gcgatagacc cggccaatat gaattcgggt 600
   gaccttctct acttcaaggg acatgtggct atgtaccttg gcggcggcaa atacattcac 660
   gccacaggga gggccggtga cgacagggta gttataaaca gcctttttcc ggaggacacc 720
   ggttacaggg aggacctggc caatggcctc gtcgccgtgg gcagcgtatt ttga 774
<210> 92
   <211> 1026
   <212> DNA
   <213> Unknown
<220>
   <223> Unknown gut bacteria
<400> 92
   atggacgaca gacttataac ctcttccatg acaatggagg acgagcagat agaatacagc      60
   ctgcgcccca ggtttcttaa cgagtacata ggtcagcata acgtcaagga acacatgcgc     120
   atatacatag aggcggccaa gcacagaggg gagcccctgg accatgccct tttctatggc     180
   ccgccgggtc tcggcaagac cacgcttgcg gccatagtcg caaacgagat gggtggcaac     240
   ctgcgggtaa cgtcaggccc agcaatatcc catgcaggcg accttgcggc catacttaca     300
   aatctcgccc ccggcgacgt gctgttcata gacgagatac acaggctgaa ttccagcgtt     360
   gaggaagtgc tctatcctgc catggaggat tattccatag acataacaat aggcaagggt     420
   ccatccgccc actccatacg tctggatctt ccaaagttca ccctcatagg cgcgacgacg     480
   cgtatgggca tgctcacatc tcccctcagg gacaggttcg gcataaagga gcagcttgag     540
   ctgtacaacc cggaggagct gaaggaaata atcgagcgca gcgcggatat actcaacgtt     600
   aagatagaca cagagggcgc ccttgagata gcctcgcgct cccggggtac accgcgaata     660
   gttaaccgcc tgctaaaaag ggtgcgtgat tacgcacagg tgcggggcaa tggcgagata     720
   gatcttgaga cagcccgggc tggcctggat atgctggcca tagatgagat gggccttgac     780
   gctgtggaca ggcgcatgct cgccaccatg atagaaaagt tcggcggcag gcctgtaggc     840
   cttgatacca tagccgcggc cacaggcgac gacgccacca ccatcgagga cgtgtacgag     900
   ccatatctca tacagcttgg cttcatcgcg agaacgcccc ggggccgcgt ggtgatgccg     960
   ctgggatacc ggcatatggg ttacgcgccg ccggctgatg tgggccaaca gaagatgaat    1020
   ttttga                                                               1026
<210> 93
   <211> 1182
   <212> DNA
   <213> Unknown
<220>
   <223> Unknown gut bacteria
<400> 93
   cgggagcaga cccttaacca gctactggtg gagatggacg gctttgcggt aaacgaaggc 60
   ataatagtgc ttgccgccac taaccgccca gacatactcg atcccgcact gctgcgtcct 120
   ggcaggtttg acaggcagat aaccgtgggc tatcccgacg ttaagggacg ggaggccata 180
   ctcgaggttc acgccaaggg caagcccctc gccaaggacg tggatctggg ggtgctggca 240
   aagcgcacca gcggcatgac cggcgcggat cttgaaaacg tgcttaacga ggcggccata 300
   cttgcggccc gcttcaagaa gaaggagata accatggtgg agctggagga ggccataacc 360
   cgcacggttg tgggccccga aaagcgcagc cgtgtagtta ccgaggagga taagcgcatc 420
   accgcctatc acgaggcggg acacgccata gtagccctga agatggagca ctgcgatcct 480
   gtacacgagg tatccataat acccagaggt caggcggcag gctacaccat gaccatgccc 540
   gacaacgacg acagccacat ttcccggggc aagatacttg accagatagc catgagtatg 600
   ggaggccgtg tggccgagag catagccctt gacgatatat gcaccggcgc cataggagat 660
   ctcaagcacg ccagcgacct tgaccgccgc atggtgatag agttaggcat gagcgacgag 720
   ataggccccg tgttcctggg cggcgattcc gaggtgttca tagcaaagga ctggggacac 780
   cagcgcagct attccgagga ggtggctgcc aaggtggaca aggagatacg caggatactg 840
   gaggagcagt tcgagcgggc gaagcaggtg ctgctccacg agcgggaggc gcttgatcgg 900
   gtagtgaagt atcttataga gtacgagcgc ataaccggcg aggaattcga gaagatattc 960
   cataacgagc ctgtggagct ggtatcctat aaagagcgga aggaagctct tgaaaagccg 1020
   gacgaggagg aaaagccgga ggacgaatcc gatgacagga caggggatga gccggagcag 1080
   cgccatgagg gcagcggtga ggacagcccc gaggataagc ctgagaaaga ggcggaacag 1140
   aaattctacc ggcccggcgg attcgacggc gaggacaaat aa 1182
<210> 94
   <211> 1248
   <212> DNA
   <213> Unknown
<220>
   <223> Unknown gut bacteria
<400> 94
   atgaagatag caatgcctga gcttctggct cctgccggaa atatggaaag gctgacagcg      60
   gccatcgatt acggtgcgga cgcggtatat cttggggcgc agagcatgag cctgaggaat     120
   tatgccgata actttacgcc ggagcagctc agggcgggct gcgaatacgc ccacgggagg     180
   aacgccaagg tatacgtggc ctgcaatgca ttcgcgcggg acgaggacct taaggatttg     240
   ccggaactgc tggcaaccat ccggactgcc ggtgccgatg cgcttatagt caacgatccc     300
   ggcgtgatac gggtggcgcg taaatgcgtg ccggatatgc cgctgcactt gagtactcag     360
   gcgaacactt tgaattccga ggcggcgctg ttctggtacg agcagggggt aaagcgcata     420
   atacttgcca gggagctttc catcaagcag ataaaggcta tgcggcagag actgcctgag     480
   gaccttgagc ttgagatgtt cgtccacggc gccatgtgcg tgtcctattc cggaagatgc     540
   ctgctgagta actacataaa cggcagggac tcaaacaggg gcgaatgcgc ccagccctgc     600
   cgctggcagt atgaactaag ggaaaagggc tcaaagggcg agtactttac gatagagcag     660
   gacgagcgcg gcagctttat catgaactcc cgggatatgc gcctcataga gtacataccg     720
   gagatagtgg atgcaggcat aatgagcctc aagatagagg gccgcatgaa gtccatcgcc     780
   tatgtggcca ccgtggtgaa cgcctaccga ctggccattg acgagtataa gcgcagcatc     840
   gacgagggca gggaatataa ggtgcccgct tccgtgatgc gggagctgga acttgcgtcc     900
   catcggccct ataccaccgg ctttgcattt ggcgatcccg gcgccgaagg tcaggagacc     960
   cgcagcggcg gttatgtagc ggacggagcc atagccgcgg tggtgatatc ctatgacggc    1020
   aagactggga ccgcttttgt ggctcagcgc aacaagtttt atgacggcga ggagctttcc    1080
   atactttccc ccggcgacat aggccgcagc tttaaggtga gcggcataac aaacgaggct    1140
   ggcgaaaggg tgcagagtac gccgcatcca aaggagaagc tgtatatagg ctgcaaacag    1200
   gaattaaagc ccggcgacat actccgaaag cttaccgaaa aaagataa                 1248
<210> 95
   <211> 1386
   <212> DNA
   <213> Unknown
<220>
   <223> Unknown gut bacteria
<400> 95
   gacaggcagt ttcaccgcaa cagcgtgctc tcagaggtgg agcgcagggt aagcgacttt      60
   gttaccgcca tggagggcgt atacagaagg tacgacaaca gcaagttctt cttcgtgttc     120
   gaggcaaagc gcatagcgga gcttgagcag cagcgcttta cccttctcga cgaggtgcgc     180
   gagatagata cgggaaccgg ccagccggta acgctctcta tttccatagg cgtggcggac     240
   aggatagccg cctcggacga ggcggcccga aacggcatgc agctggtgct gggcagggga     300
   ggcgatcagg cggtggtcaa gcggggggca aactacgcct tctacggcgg caagcgccag     360
   gtaaccacaa agaactccag agtaaaggcg cggcttttcg ccgaggcgct gcatcagctg     420
   atggacaccg cggatgcggt tttcataatg ggccacagtc taccggatat ggactgcgta     480
   ggggcgggcc tcggcataat gcgctgcgcc aagagcataa gctgcccggc gtatttcgtg     540
   ctggacgaat ccaacgccag cattgatggg gcgatagcgg caatgaagga aaacaaggcc     600
   tacagggata ctataaagac gccggagcag gccgcccaga tgatgcgctc ctcctcgcta     660
   ttgataatcg tggacaccca gcgcaaaagc tcgctgctca gcgccgagct gctggaaaag     720
   gcgggcaagg cggtggtcat agaccatcac aggcgggctg tggactccat acagaaccct     780
   accctcaact atcttgaggc gggatcctcc tcggcctgcg agatggtgac agaggtgata     840
   cagtactttg acgacggcct caagcccacc accttcgagt gcggggcgct gcttgcgggc     900
   atcactatgg atacaaagca tttttccttc aataccggag cgcgcacctt tgaggcggca     960
   agctatttaa ggcgcaacgg cgcggacaac accaccgtga agatgatgtt ccaggacgat    1020
   atgcagacct accgcaacag ggccaaggta gtggaaaacg ccataataat ggagcagggg    1080
   atagccatat ccgcatgccc cgcgggcatg gaagccacca acctcatagc cgcacaggcg    1140
   gcggacgagc tggtgaacat aaagggtatt caggcttcct tcgtgcttgc ggagcggggc    1200
   caggtgatat acataagcgg cagaagcctc ggggacatca gcgtccagct tatacttgaa    1260
   aagctggggg gcggcgggca tcagtccatt gccggcgcgc agctgaagga cgtgactatg    1320
   gacgaggcca taagcaggct tacggaaagt ataaacagct atttaaagga ggcacacgag    1380
   aaatga                                                                1386
<210> 96
   <211> 1035
   <212> DNA
   <213> Unknown
<220>
   <223> Unknown gut bacteria
<400> 96
   atgaagctca cccttatacc tgcacagtac ctggagcaaa gcatccaata ccctgagctt      60
   tccctctgcc ggatagtttc ccaggaaaag ggcttgtacc gcatcataag cgccgaaggg     120
   gatcagctgg cgtcggtatc cggtaaattc cagcatgaag cagcctcccc ttcggattac     180
   cccgttgtgg gcaactatgt gatggcagcc ctaaacggcg gagcggccgt tataaaccgt     240
   gtgctgcccc gtaaaagcct gtttctccgt aaatcggcag gcacagccag gatggagcag     300
   gccgttgccg ctaatattga caccgtattc atctgcatgt cgctgaacag cgactttaac     360
   ctgcgccgcc tggaacgcta cttatccgtt gcgtgggaaa gcggttccct gcccgtagtg     420
   gtgcttacca aggccgacct ttgcgataat cccgcagtaa aggccgccca ggccgccagc     480
   atcgctatcg gggcagatgt acttgtcact tcttccatgg cgctggacgg ctataaccag      540
   atactgccct atatctccca aggaaagaca gttgccttcg taggttcctc cggcgtaggc     600
   aagtccaccc tcatcaaccg gctgctgggc gaggacaggc aggataccgg cggcctcagg     660
   aacgacgaca agggccgcca caccaccacc cacagggagc tgctgatgct tcccaacggc     720
   gcagcggtga tagacactcc cggcatgcgg gagcttggca tatgggacgc ctccattggc     780
   ctcagcacag ccttcagcga tatagaggag ctggccgccc aatgccgttt cggcgactgc     840
   acccataaaa acgagcccgg ctgcgcagtg cgttccgcgc tgcaaagggg cgagctgtca     900
   gaggagcgct ttttgtctta tgaaaagctg gcgttagaaa acgcctatgc agccggcccc      960
   acaggcagcc gcgccgcaaa ggagcgtaaa ttcaagagca tagcaaaatt caacaaaact    1020
   aaaccaaaga gataa                                                     1035
<210> 97
   <211> 870
   <212> DNA
   <213> Unknown
<220>
   <223> Unknown gut bacteria
<400> 97
   atggaaaacg tggatttgct cacacggata aaccagcatt acaattcttt cagcaagggg 60
   cagaagctgc ttgccaacta cataatagac aattacgaca gggcggcgtt cattaccgca 120
   tcgaagatgg gccgcactgt aggcgtaagt gaatctacgg ttgtccgctt tgcatatgct 180
   ttgggttatg acggatatcc tgcgctccag cgctcccttc aggaaatgat acgcaaccgt 240
   cttactacgg tccagcgtat acaactcaca tcggatcttt ctcaggacga ggtcctttcc 300
   actgtattga aatcagatat aagcaatatc agatccacta tagagtcggt aaataccgct 360
   accttcaata ccgtcataga ctacatgctc aaggcgcgca agatctatgt tgtaggtata 420
   aagagtgcgg cgccgttagc acaattcctg gggtattatc tcaacttcat actggacgat 480
   gtatgcatcg tcaacagtac cgtatatgaa acgatgctgc gcataagcga caaggacatg 540
   tgcataggaa taagcttccc cagatactcc tctaaggcgg tggaagcgct gagcttcgca 600
   aggagcaagg gcgcatatgt gacggccata acggacagca tgttttcccc tatagccgag 660
   cttgcggaca gcgtactggt agcgcgcagc gatatggctt cctttgccga ttctctggct 720
   gcccccttga gccttatcaa tgcccttata gtgggtgcgg gccttcgcag gaaggacgag 780
   gtgtacgaga gattggatca gctggaaggc atatggaaga gccacaaggt gtatatagcg 840
   tccaatacag acgataggga taacgaatga 870
<210> 98
   <211> 1017
   <212> DNA
   <213> Unknown
<220>
   <223> Unknown gut bacteria
<400> 98
   ttgaaaggag acggaataat gtctatcatc gacaccatca aagcaaaagc aaaagctgac 60
   gtaaagcaca tagtgcttcc cgagggaagt gaggagcgta ccgttcaggc cgcaaggctg 120
   ataaccgatc agggcatcgc caaggttacc cttatcggca aaagcgagga tatcaaggct 180
   gccgctgcca ggttcaacgt gaacgtggac gacattgcaa tgatcgaccc ggctgcctct      240
   gagctgtatc ccggatactg cgagaagttc gctgaaatgc gcgcaaagaa gggcgttacc      300
   cttgagcagg cgcagaagac catgctcgac cccctgtttt tcgccgctat gatggtttac      360
   gatgacaagg ctgacggctt cgtttccggc gctataaata caaccggcaa cacactccgc      420
   cccggcctcc agataataaa gaccgcaaag ggcgtatcca ccgtttccag ctgctttatc      480
   atggaagttc ccaacaaggc ctacggtgac catggcgtgc tggtatttgg cgactgcgcg      540
   gtaaatatcg cccctaccgc agagcaactg gccgccatag ccatcagctc cgcacagagc      600
   gcaaaggcgc tttgcggaat ggagccccgc gtagccatgc tttccttctc caccaagggc      660
   agcgccaagc acgagaacgt ggatcgcgta gcggaagcta ccaggctggt acacgagctc      720
   gctcccgagc ttaacgcgga cggcgaactt caggcggatg ctgctctggt cgaaaaagtc      780
   ggccagctca agagccccgg aagccctgtt gccggcaagg ccaatgtact gatcttcccc      840
   gacctgcagg cgggcaacat cggctataag ctggttcagc gtctggcagg cgcagaggcc      900
   ataggcccca tctgccaggg cttcgcaaag cccatcaacg acctgtcccg cggctgcagc      960
   gttgacgata tagtatccgt cgttgccatt accgccgttc aggcacagaa tatgtaa        1017
<210> 99
   <211> 1341
   <212> DNA
   <213> Unknown
<220>
   <223> Unknown gut bacteria
<400> 99
   atggaagaag aaaaagacat cttaactgaa gaaaataacg attcggaaat acccgtggaa 60
   gccgaaatga tagatcaggc ggttgaagag gcggcggagc acgccggcgg gggggacgac 120
   ggctttttcg gcaagtacat atgggatgag gaaacgggca ggctgtacga gataaccgag 180
   gatccaaaaa agaagtggga caggctgctc ccgctgctgc tgattgcgct gccagtaaca 240
   gcccttgcgt ttggcatcgc caagggttcc tcgcttctgc cgccggaatg ctacaacggc 300
   acctccatac tgtccgaaag caatgtggga gaacagcttg cccagcctaa agcttacgga 360
   gtaagcgacg ctgaagaggc aacggccgag aacagccccg acacagagca ggccatggcg 420
   ccggaacagt atacgccctg cgccatagtc atagacggca ggacaagggc ggtgctggcc 480
   agcaccgagg ccgcggaatc gctgctggag gaagtcagga gctacttcga cggcaaggtg 540
   acggaatccg gcgaccgtac caccgagctt tacagtgggg tagagatgca gccccagccc 600
   gatgcaaagc ccgaagacat ttccagctac gaccgggtat acgaaaagtt cacctcctca 660
   agcagtcctc tgaaggtcat taccactgtg acgagcagcg aaacgaagga gctgcccttc 720
   gactcaaagg tagagaaaga ccccacactg gtggccgaca ccggcattac cctcacctta 780
   ggcagggcgg gcagcagaac caccgtgaac catacggtat acataaacgg cgaaaagagc 840
   tccagccgca gcaagagcga aacagttacg gtggaacccc aggatacggt aatacttcag 900
   ggcaccgata aaaagagcgg cggcgagccg ggcaggcacg agggcaaaga gggcagaaag 960
   cccgaggcgc tcagctttgc ttcccccgta gcggagggag agataatttc aaactacgga 1020
   cagcgcgacg gcatacttca tcttggcctg gactacgagg ccaaggccgg agacccggtg 1080
   ctgtccagcg agggaggcac cgtggtctgc gccataaatc ggggcggcta cggcaacatc 1140
   atagagatag accacggcga agggttcgtc acccgctacg cccacctgag ccggttctcc 1200
   gtggctcccg gcgataaggt agccaagggc cagatcatag gcaaggccgg cgactcaggc 1260
   agctgtaccg aagcacatct tcactttgag ctgaggattg acggcatagc ctacaatcca 1320
   agatattact tagaaaagta a 1341
<210> 100
   <211> 1128
   <212> DNA
   <213> Unknown
<220>
   <223> Unknown gut bacteria
<400> 100
   atgcaagata aattcgactt caaatacgag cttctgcacg tttgcaagca gtccggcgcc 60
   cgccgcggca ggctgcacac cccgcacgga gtaatagaga ccccctgcta tatgccggta 120
   ggcacacagg ccacggtaaa ggccatgctt ccacgggacg tcaaggaagt gggctctatg 180
   ataatactgt caaacacata tcacctcttt gagaggcccg gtcacgagct tataaaagag 240
   gcgggcggcc tgcacgagtt catgcggtgg gataagccca tacttaccga cagcggcggc 300
   tttcaggtgt tcagcctcgc ctccgccaat aaaataaaag aggacggcgt ggagttccgc 360
   tcccttctgg acggacgtaa gcatttcttc acccccgagc ttgtaatgga gatagaggaa 420
   gcccttggcg cggatatcgc catggcgttc gacgaatgcg ccccttatcc ctccgaccgg 480
   gaatacacca tagctgcaat ggagcgcacc catcgctggg tcgtgcgctg caaggctgcc 540
   catacccgcc cggatcaggc tcttttcggc atagttcagg gcggcatgtt cgccgacctc 600
   aggatagaga gcgctaagtt tctcgcttcc ctcgacctgc cgggctatgg catcggcggt 660
   ctttcagtgg gcgagcccaa ggaaatgatg tacgatatgc tggagcagat gatgccctat 720
   atgcccgcaa acaagccccg ctaccttatg ggcgtgggca gccccgactg tctggtggag 780
   ggcgctgtac gcggcataga catgttcgac tgcgtactcc agacccgtat agcgagaaac 840
   ggccttgcgc tcacgggaag cggcaaaaaa atgctgcgaa acgcctcctt tgagcacgac 900
   ttcacgccta tcgaggaggg ctgcgactgc tatgcctgca agaacggctt caccagggca 960
   tatatacgcc accttgtaaa atgcaaggag atactggcgg cacagctcat aaccacccac 1020
   aacctgcgct tctctctgag gctcatggag caggtgcgca aggccataga gcaggacagg 1080
   ctgctggact tccgggaaga gcttaaagcg aaaggaacat tcgattga 1128
<210> 101
   <211> 2223
   <212> DNA
   <213> Unknown
<220>
   <223> Unknown gut bacteria
<400> 101
   atgagtttaa aactgaaacg tgtactggcc gtactggtgc tgatcgcgct ggtattcggc      60
   tggtatgtca ccttgttcgg catcggtgac aaaacaaagg acgatgccat cacttcggta     120
   aaatctgctt taaaatacgg cctggacatt gacggcggcg tctatgtcgt tatggaggct     180
   aagacagact tgaccggaga cgaactcaag aagctgatgg aacagacccg cgcggtactt     240
   gacaatcgtg tcaatcaaat gggtgtcgcg gaatcttccg tcactatcga gggcaacaag     300
   cgtctgagag ttgagattcc gggcatggag gatgcggaag acgccatcaa ggcgatcggt     360
   aaaaccgcac agttatactt cattctggca gacggcagcg tggtactgga cggcagccat     420
   gtcaaagacg cgcagattgc gacagatgga agctactaca aaattctgct cgactttgac     480
   agcgaaggcg cgaccttgtt cgaagaagga acccgcaagg cgtttaaccg tgaagtaacg     540
   tcaaccatcg aaggtgtgga gagcaatcaa atcgcgatcg tactggataa cgaaatcgtg     600
   gtacatccga atgtaaacag cgtcatcagc ggcggaagct gtgaaatgac aggaaactat     660
   acaaaggaag acgcttccat gaccgcggcg ctgattcgag gcggcgcgct tccggtagaa     720
   ctggaagaag tacagtcctc cgtacagacg gcgaccatcg gcgcacacgc gctggacaag     780
   tccatcgtgg caggcgcgat cggtctgggg attgtattcc tgttgatgct gatcttttac     840
   ggcatgctgg ggttggtcgc ggatatcgcg ctcctgctct atgttgtgat gttcctgtgg     900
   tcgatggtcg gtctgggtgt cgttatgacc ctgccgggta tcgcggctct gatcctgtcc     960
   atcggtatgg cagttgacgc gaacgtcatc atttttgcga gaatcaaaga agaaatctgc    1020
   gccggcaaaa cgatccgcgt cgcggtggat gccggcttta agaacgcgct gacaacggta    1080
   ctcgacgcgc agattacaac tctgatcgcg gcagtggtac tttatgaaat cggtacgacc    1140
   tccgtcaaag gtttcgcgct gaccttgatg atcggtattg tcatcagtat ttttacagcg    1200
   gttgttatca cgcagctgtt tatttccctg cttgccaata atccgaaatt cgcgaagaac    1260
   aagtactttg gcgtcaacga tgatctgacc ccgaagcaga tgttaaataa aactttctca    1320
   tttatcaagc atagaaaagt ttactacatc atcagctgcg cgatcatcat cgcgggcctg    1380
   ctgttcgcga cgatcggcgg tatgaattac ggtatcgact ttaccggcgg cacgaccatt     1440
   caggttgaaa tgggcaagga ggttccgatt gaggacgtcg aaaaatctct cgaagcctat     1500
   gaattgaacc cgcagattat ttatgcggga gaaggcaata cacagattgt catcaaaacg     1560
   attaaatctt tggacaacgc agcgcgtgga gaaatcatcg acacgctggg tacggacttc     1620
   ggcatcaccc aggaggatgt actggcttcc gagcagttcg gcccgtccgt cggaaacgag     1680
   ctgaagatga acgcgttgaa ggcagtaatc attgcttcaa tcggcatgct gatctatatt     1740
   atcttccgct tcaagtcctg gaaatacggc gtgacggcga ttgccggcgt tgtgcatgac     1800
   gtactggtgg ttatcgcttt ctacgcgatt ttcggctata cggtcaacaa tccgttcatc     1860
   gcggcgatct tgactttggt cggatattcc atcaacgata cgattgtaat ctttgaccgt     1920
   atcagagaaa acaaagccct gcatccaaag gacaacaacg agaccaatat cgacctgagt     1980
   atcaatcaga cgctgaatcg aaccatcatg acctcgctga ctacgctggt ggttatgatc     2040
   ccgctttgca tcatggtttc ctccgcgatc agagaattca tcattccgct gatggtcggc     2100
   gttatcgtag gatgctattc ctccatcttt gtttgcagcc cgctttacta cgatctctgc     2160
   aagaaggatg aaacctccaa gtatgtatta aatacatcca aaaaggcaaa aaagacaaaa     2220
   tag                                                                   2223
<210> 102
   <211> 1467
   <212> DNA
   <213> Unknown
<220>
   <223> Unknown gut bacteria
<400> 102
   atgaatatag tctttttacc ggatcataca gtaccggcgc tggtcggaat ctacacgatc      60
   aatattttga tgattatcgc tgtcattttt gcggagcgca aaagcccatc ggcaacgctc     120
   gcgtggatca tggtgctggc ctttgtccca attatcggct tcgtttttta tctggttttt     180
   aatcagaata tcgccagaat taaaatcaat cggctgacgg aaaacgaaaa atacgtggtg     240
   agtcacgcgt taaaaaaaca gatgcaggaa atggacaatc cgcgctacgc gtttcattgt     300
   ccgacgacca ggaactggcg gcatctgatc aagctgaatc aggtctacgg acacgctttt     360
   tacagcgaga acaatcaggt ggagctgatt acggacggaa tgacgctttt tgacaggatg     420
   atgcgagata ttcagggagc gaaggacacc atcaatatgg aatacttcat catcaaaaag     480
   gattttgtcg ggatgtcgct gatcgaactt ttgacacaaa aggcgaagga aggcgtgacg     540
   gtacgcctgc ttttggacag catgggctgc cgaaccatca atgaacatgt actcaaggac     600
   tatctggcgg caggcggcaa ggtcggcttt tttttcaaac cgaaattcaa gatcttcggt     660
   ctgaagctga actaccgcaa tcaccgcaaa attgtcgtca ttgaccacaa aatcgcttat     720
   acgggcggct acaatatcgc gaaggagtac atcggacaaa agaaaaaatt cagttattgg     780
   cgtgacaccc atgtcaggat taccggcgac gcggtggcgg atctcaacgg ccggtttatt     840
   ttagattggc ggttcaccac caaggaacag ctgcagcttg aggcggcacg tatggcagag     900
   ggggagacac cgggacatat cggcgtgcag atcgtttctt gcggaccgga atctccatct     960
   gaggaggtaa aacgtgccat gatgcgtatg attaccttcg ccgagaagcg tgtttactta    1020
   cagactccgt atttcgtacc ggatccgagc attgtcgaat ccctaaaaat ggcggtgcag    1080
   tcgggggtcg atgtcagaat tatgattccg cacatgcctg atcacgtttt tgtctactgg    1140
   gcgacgtatt cttatgtcgg agagctgctt cgatcaggcg ccagagccta tatctatgac    1200
   gacggcttct tgcatgccaa gacactggtc gtggacggag aagtcggcac catcggctcg    1260
   accaactttg acaaccgtag ttttcgtttg aattttgaaa ccaacgcgtt catttttgat    1320
   aaggacttcg cgcagcagat ggagcgcgcc tttatccacg atatggcaca tcacggacat    1380
   gaattgacct acgcggatta tctgaagcga ggaaaactca tcaaatttaa ggaagcaatc    1440
   tcaagactct tatcggatat attatga                                        1467
<210> 103
   <211> 2064
   <212> DNA
   <213> Unknown
<220>
   <223> Unknown gut bacteria
<400> 103
   gacaaatacg aaccgagaag aagaggctgg gtatggagag agaaagctga caatcagcac      60
   atgggaccgt ttgagtaccg tgagatttcc aagccgttga agcagagcgt tccgctgcct     120
   cctgcaaaat tcatggaaga tattgaccca cagccgatgc agaccatcac tacagagatc     180
   gcttccggaa gattcgagga cgatatccgc agaatgagaa tgggcgcttg gcacggcgct     240
   gaccatctga tggttatccg tcacatgggt cagtctcaca tcgacggcct gatggaaggt     300
   acaccgcagg gtatcggcgg tgttccgatc accagaaagc agatcagagc acagagaaaa     360
   gccatcgaca tgatcgagga tgaagttggt cgtccgatca actaccactc ctatgtatcc     420
   ggcgttgccg gtccggacgt tgctgtactg ttcgcggaag aaggcgtaaa cggcgcgcac     480
   caggacccac agtacaacgt tctttacaga gatatcaact gcgtcagatc ctttgtagat     540
   gcttgtgaat ccaaaaaaat catggcttgg gcagacatgc tgcagattga cggtgcgcac     600
   aatgcgaacg cgaccgcaag agaagcatgg aaggtacagc cggaactgat cgtacagcat     660
   gccatcaact ccagattctc cgagaaggta ggcatcaagc cggaactgat ctccttatcg     720
   accgtaccgc cgacagcgac accggcgcct tgtatgtata tggacctgcc ttacgcggta     780
   gcactcagag aaatctgcaa cagatataag atgagagcgc agatgaacac caagtatatc 840
   cagtcctccg ctcgtgaagc gacggttacg cacgttatga acatgttcat ctccaagctg 900
   accagcgcgg atattcagtc cacgattaca ccggacgaag gcagaaacgt gccttggcac 960
   atctacaaca tggaagcctg cgacaccgcg aaacagactc tgatcggctt agacggcatc 1020
   atggatatgg tagagctcaa aaaggacggt ccgctgagag atatggccag agacctgaaa 1080
   gaaagagctt gcttgttcat ggaagaaatc gtagagaacg gcggctactt ccaggcagtt 1140
   caggatggct tcttcgttga ctccgcgaaa tatccggaaa gaaacggcga cggcatcgcg 1200
   cgtaagatcg agggcggcgt aggctatggc tttatctttg agagagatga agattacatg 1260
   gcgccggtaa ccgcgcacta tggctataac aacgtagaac agtacggcgg agaccctgag 1320
   aacccatccg cgctgatcgg cggctgtacg tttgaagaca gaagcaagat cgtttacatt 1380
   gacgagcttg atgaagagga taacgtaaac gtaagactcg cgaaggtcgc gaaatacaga 1440
   accggcgaag ttatcacacc ggaagtggaa tggtgcggcg acagagtggt tatggttacc 1500
   atgatgcttc cgaccaacaa gcgtgttgca gaagcagcag cagttgaatt aggtaagaaa 1560
   ttaggtctgg tggatccgga agttatctct gcggaagtga tgcaggatgc ggaaggtacc 1620
   agaatcgaat tgaagggcag agtggacttc gatatcgact tgacaactct tgaaattcca 1680
   ccggagccac attatctgac agacaaagag ctttatgatg aattcgaagg acacgaacac 1740
   atgaaggttg tctgcggtac agtcggtgaa gacgaacact ccgtaggtat gagagaaatc 1800
   atcaacatca agcacggcgg aatcgaaaaa tggggtatcg aatgcgtata cctcggcact 1860
   tccgtaccgg tagaaaagat ggttgacgct tgtatcgaag aaaatgcaca ggcgatcctc 1920
   gcttccacaa tcatttccca cgacaatgtt cactacaaga acatgaagag actgcacgaa 1980
   ctctgcgtgg aaaaaggtat cagagacaag gttatcatct gcgcgggcgg cacacaggtt 2040
   attccggaag aagccgtcaa gacc 2064
<210> 104
   <211> 2655
   <212> DNA
   <213> Unknown
<220>
   <223> Unknown gut bacteria
<400> 104
   atgaaacaac tgatcgcgga cgcgctgagc tgggaggagc tcggtgagat acataaaaca      60
   gaatatcaat ttgaacttgg caagggcgtg ctgcataagg aggcaggcag actgaccctg     120
   ccgattcact gcaattttgt gatgccgttc gcggactgcg aaaaggtcaa agcggtgatt     180
   ttacacaagc tcgacatgct caaggaggtc gaactggatt tttcgtaccg cgattttgtg     240
   atgccgaagg aggacgctgc cagactctat gtcccgcata tggttcggat tctcaacggt     300
   tcgtatctgg cgatcaccaa gaccatcgtc gaaaaccgcg tcatggtcag agacggatgc 360
   ctttactgtc cggttctcgg taaagtcgcg gcagagcagc tgaatgagag agtggcgcaa 420
   aaattccgag aaatactcaa agaaactttc gattttgata tggagatcat ctttgagaac 480
   gatgaagaac tgctggcgga aactctgcgt tctttccatg agagtgaggc gaaggatatt 540
   gagcgctcca tgcgggagta ccgggaagtg ctcaagacgc gggcgaagga aagaccgcag 600
   gcggcggaca gcgtcacagg cggcggcttc ggcggcggaa acaacggcgg cggctttggc 660
   ggaagcgcgg caggtgccgg cagtaagaag cagggcggct ggaaacgcag ggaaaaggaa 720
   ctgcccgcgg agggcaaccg catcatggga cgcggcattc cgggcggtaa gaccgcggag 780
   ctgcgagaga tcgcaccgga gatgggggtc gttatcctgg aaggtgtgct gttccgcaag 840
   acggagcgtg tcatcaagag cggcaattat ctcgtttccc tgttgattac cgataataag 900
   acgacgatct gtgccaaggc gtttgtcagt gagaacaaat ggaaggaaat cgatgaactg 960
   ctcaaaccgg gcgatggtgt caagctgcgc ggtgaggtgc agtgggacac ctttgaaaac 1020
   atgaatacgg tcatgtatac ggacctcgaa aagaccgacc gcgaaatcgg ccgcgaggac 1080
   acctgggaga acggccggcg tgtggagctt catattcact ccaagatgag ccagatggac 1140
   ggcttcaacg aagccaaaga catcgtggcg atggcagcta agtgggggca gcccgcggtc 1200
   gcggtgaccg atcatggcgt ggtgcaggcg ttcccggact gcgcggcgga ggccaaaaag 1260
   caaggcgaaa aaggccgcga gatcaaggtc atttacgggc tggagggcta tgtgttcgac 1320
   gacgcggact gcgtgcagga ggacggcagc attgattaca agagcaaacc gacccatcac 1380
   atcatcattc tggcaaagac gcaggaggga ctgaagaaca tttacaagct ggtttcctat 1440
   tcccacctgg attactatta caaaaaaccg cgcttgccgt ggtcggtcat cgaagcgaac 1500
   cgcgaaggac tgatcatcgg ctcagcatgc agcgcaggcg aagtgttttc ttcgatggtc 1560
   aaacacgtga cctatcagaa gcggacgcgt accgaaaaga tcatggagca gaaatccgcg 1620
   gaggagcagg cgcgtatcgc ctcccgctat gactatctgg aaatccagcc tgtcatcaac 1680
   aaccgcttca tgttcgatga ggagttttat ctggacggca ggcgcattga gaatgaggac 1740
   gatatccgcg agatcaaccg gcggatcgtg gcgctggcgg acaagctcgg caagcgggtc 1800
   gtggcaacga cggattcaca ctatacggag ccggatgatg caatctaccg caatatcgtg 1860
   atggcgggca tgggcttcga agacgcagag agcggcgagg gtctgtatat gaaaacgacc 1920
   gatgagatga tggaagagtt ttcgtatctg ggcgcgcgcg cccaggagat cgtcgtggac 1980
   aatacgaatt acatcgcgag tttgatcgac ggtgatatcc gtccggtacc cgcgggcaag 2040
   ttcccgccga agatcgaggg cgcgaaggaa accctgcgca agacctgtat ggaacgcgca 2100
   cacgcgcagt acggagaccc actgccgaag gaaatcgagg atcggctgga tatcgagctg 2160
   aattcgatca tcaacaacgg ctacgcggtc atgtacgtca gcgcgcagat gctggtaaac    2220
   aagtcgctgg aggacgggta tctggtcggc tcgcgtggtt cagtcggctc atcgttcgca    2280
   gcgacgatgg cgggcattac ggaagtcaat ccgcttgacc cgcattatct ttgtccgaac    2340
   tgcaaaaaga ttatctgggg tgatatgcag aagcacgact gcggcgtagc tatgccgccg    2400
   ctggactgtc cggactgcgg cacgccgatg aaacgggacg gctatacgat cccgttcgcg    2460
   accttccttg ggtttaaggg caataaggag ccggatatcg acttgaactt cgccggggaa    2520
   tatcagccgg tggctcaccg ctatgtcggg actattttcg gtgaaaagaa tgtgtttaag    2580
   gcgggaacgg tcggtacggt cgcagaaaaa accgcttatg gttatgtcgc gaagttcttt    2640
   gaggagcgcg atatt                                                     2655
<210> 105
   <211> 2073
   <212> DNA
   <213> Unknown
<220>
   <223> Unknown gut bacteria
<400> 105
   atgagaagca gaatttacag caagctggca tggaccaata tctgcaagaa ccgcaagact      60
   tatctgccgt acatggtcag cgccatcggc atggtgatga tgttttttct tacgatcaat     120
   ctgtccatgg atccgcaggt ggcgaccttt gaggaaggaa actggatgca gcttatcctg     180
   agacttggct caattgtgat cggcctgttc tcggtaatct tcctgttcta tacgaacagc     240
   ttcctgatga agcgccgtaa gaccgagctg ggactgtatc atgtgctggg catgggcaaa     300
   acccacatcg cgagtgtgct gctctgggaa actctcttta cggctctgct cagcatcggt     360
   gccggcgcga tcggcggcat ggtgttcagc aaggcggcac aggtgctgct gctgcggatg     420
   atgggaagga gtatcgatta tgtgttcacc ctgcagccgt cggcgctcgt atacagcgtg     480
   attctgtttg cggcaatttt cgcgctcatc tacgtcaaga gcgtgatcga gatcagcagg     540
   caaagtaccg tcgatctgct ttacagcaag gcgcagggtg aaaaagagcc gcgggcaaat     600
   ttcgtgctgg ctttcatcgg catcttgtct ttgggggcag ggtacgtatt ggcgatgacc     660
   gtacaaaacc cgacagacgc gattctggta ttcttcatcg ctgtcatcgc tgtcatcatc     720
   ggcacctact gtactttcat cgcgggcagc gtggcattgc tcaaactgct gcgcaaaaat     780
   aagaactatt actatcagac cggtcatttc attgcgacct cgtcgatgat cttccggatg     840
   aaaaagcatg gggcggggct tgcgagcatc tgcatccttt cgaccatggt gctggtgatg     900
   atctcatcga cggcctgtct gtactttggc gccggcaacg cgatcaacgc agcctatccg     960
   cgcgatctgg aggcgacaat cggcatgagg gaaaccaatg acgcgacctt tgcggcagca    1020
   ggcgcagatc tggatcgtct gcttgctgcg tcgcttcaaa aacagggact gaaggcaaag    1080
   aacgagatcc gctatacgct ttgtgccttt tacgggcaaa tagaggacgg cgtattgacg     1140
   ccgcttgcgg aatatacagc ggacggttat acctgcaagg tcatgaccgc ggaggaatac     1200
   aacgagaatt ttggaaccca tgtaagcttg gaacgcagtc agctgctcta caagaaaaat     1260
   gagggaagct tgcgggaaac catgctgacg attgccgaca aacgttacgc tgtcattgga     1320
   gaagcgaaaa aaatgattct gcgtggtacc gacgcagtag atatggcaaa cgccatttat     1380
   ctggttgttt ctgaccggag tgaattcgat cgtgtggtgc aggctttgag gcaaagcgcc     1440
   gcagcggaaa gctttcgtct tgtcaactgt cgggcgttga atctgacaga ggcaaacggc     1500
   agtgcggcgt ccgaaacgcg gattttggca gcggaagagg agctgcaaag agaagtttta     1560
   aagatgcagg ataaaccaga caccgcaatc ggctcccaca agggcaatca gccggtctac     1620
   gcttcgatgg atatgaactt ccgcaccaaa ggcagcgcga tggaaacttt cacgaatctg     1680
   tacggcggtc tgtttttcct ggggattctt ttgagccttg cgtttctggg cgcaacggtc     1740
   atgatcatgt actataagca gatcaccgag ggctacgacg ataaggaacg cttccacgtc     1800
   ctgcgtgacg tggggatgag cgaccgcgag atccgaaaat cgattcggga tcaggttctc     1860
   aaggtctttt tcatcccact gcttggtgcc gggctgcata ttgcttttgc gttccacatc     1920
   atcaaattga tgatcaaggt attcggcatc agcaacacaa cactgctgat ctacaccacg     1980
   ctggcgtcct tcctgctgtt cgccgctttc tacattgtgg tatacctgat cacctccaga     2040
   gcgtattaca aaattgtcgc agtggagcag tag                                  2073
<210> 106
   <211> 1260
   <212> DNA
   <213> Unknown
<220>
   <223> Unknown gut bacteria
<400> 106
   gtcaatctcg cgctggaagt catcgaaaag tacggaaatc ttaagcagcc gctgttcgcg      60
   acacgcagga aggtttccat gcagggcctg cggtgtctga tggcggattc cggaaacacg     120
   attcgcgaac aggtggaaga tattctgaac ctgaatcatc ggattaacgc gatctgcgag     180
   cgcagaaata agctggaaca ggacgaaact tctctcaaag cctgggaggc ctacgacctt     240
   ccgctggagc tggccggcac caaaaagacc ttggcgcaga ccggtgtgct cccaatgggc     300
   acagataccg acgcgctggc aggcgcgctg gcgggacagg cggaaacgga cggcacagta     360
   ttcaaaatcg ttcatataga caaggatatg tactacgcgg cgattctgat gctgaccgag     420
   caggcagccg atgtgcagac aattttaaag caatacggtt tttcacaggt gaccttcggc     480
   acgatgaagg gcacagcaag cgagaacctt atacggattc aaggggaaaa agatgtatta     540
   agggaagagt atgaaaaggc agcgaaggag gctgccgcaa aatccgcgcg caaagcagat     600
   atcgaggctt atgccgatat gctgaccatc gcggcggata aggaaaaaat gaaaacaaaa     660
   ctgctgaaaa ccaaacgaac cttcttcctg gaaggatgga ttccacaaaa gtgcgcaaaa     720
   aaagcagcgg agcttttgga cgactacgaa tgctattacg cgttccgcga gccggaagca     780
   gaggaagaag tccctgtact cttggaaaac aggagctttt tcagacctac ggaggcgatt     840
   accgagatgt attcgctgcc gagctattgg ggctttgacc cgacctccat ctacgcgctg     900
   ttctatatcg tatttttcgg tatgatgttc agtgacgcgg gatacgggct gctgatggca     960
   atcgggtgct tcatcattct caaaaaattc gatctcgaag gcacaacatg gaagatggtg    1020
   aagctgttct tctactgcgg gatctccacc ttcgtgtggg gtgcgctgtt cggcggatgg    1080
   ttcggcgatg tggtgacggt gttctcaacc accttcctcg gcaaggccgc ggagctgaaa    1140
   ccgctttggt tcaatccgct tgacgacccg atgaagcttt tgatcctgtc gctgatccta    1200
   ggtgtgatcc acctgtttat cggcatgggg atccaggcct atatggagat aaaggatggc    1260
<210> 107
   <211> 897
   <212> DNA
   <213> Unknown
<220>
   <223> Unknown gut bacteria
<400> 107
   atgaaagaaa agacaagcta cgcaaagcag agagtcaagc gaatgatcat cctcggcatc      60
   tatcagtttc aggaccccta ttaccagggt gtggcggcac agctggcctt tttcctgttc     120
   ctctctattt tgcctactct gattcttttg tcccaggttc tgggtctgtt ctcattgtca     180
   ctggaggaac tgcagacttg ggccaatatc aatatcagcg gagagggtgt tgatttgatc     240
   gacgatatgc tcagctactc accatccggt gcgaacagcg tgtttctcgc ggttaccgcg     300
   gtgtgggcag cctcgcgcgt gcagttcgcg ttgattcgcg tgaccaacta cacgctgacc     360
   gacggagctt ccacagggga aggctatgtt cgagatcgca ttcgctccat caagaccatt     420
   ttgataacag tttttacgat cgcgttttct ctggtggtgc tggtatatgg accgatcctg     480
   ttaaagctgg tattcggcaa ggttttgggg acagatctcg cgaacgcggc atgggttgct     540
   tttcgctggc cggttacgat gggactgtat tttttgatga tttcgtacaa ctattacgtg     600
   ctgccgacag aaagagtgcc gtaccgggac attgtaccgg gcagtatctt cgcttccatc     660
   ggttttttgg tcgtgacgat tgtctataat atctacacaa aatacagcgt gaattatgat     720
   attctttacg gatccttttc gaatatcgtc gtgctgctga tgtggttttg gtttatttcg     780
   tgggtgatgt gcctgggaat cacgaccaat cgtgtctggt gggccacgcg aagaaaaaat     840
   ccgattccga ttgcggtcaa aaagcggccg cgcaggaaac cgatcaatat tatttag        897
<210> 108
   <211> 975
   <212> DNA
   <213> Unknown
<220>
   <223> Unknown gut bacteria
<400> 108
   catatcgata gaaatctggc atttgaaatc gagaatgccg gtgtagaata tatttacgta 60
   tacagcacgg ataaggaaaa ggaagggcag gtaaccaagg ttatcggcaa tcgctttgtc 120
   gatctgcagc aatacgtcaa ttttgacgtg tcggaactga aggtcgcgga caaggtcttt 180
   tatccggttc tcaaggaaat cctcgcggag gcagcctctg aggacgaatt aaaagaaaaa 240
   cttacggcga gaaagaacga cctttctccg aaacacatta tcatagaaga tatcatcgct 300
   tccatcagct atattctcaa cttgaattac ggcatcggaa ccgtagacga tatcgaccat 360
   ctgggcaacc gcagactgag aacagtcgga gaacttctcc aaaaccagtt ccgtatcggt 420
   ctcgccagaa tggaacgtgt cgtcaaggaa agaatgacca cgcaggatat cgaggtgacc 480
   acaccgcagg cgctgatgaa catcagaccg gtaaccgcgg caatcaagga gttcttcgga 540
   agctcacagc tttcgcagtt catggaccag aacaatccgc tggccgagct gacgcacaag 600
   agaagacttt cggcactcgg accgggcgga ctttcgagag aaagagccgg cttcgaggtg 660
   cgtgacgtgc accattccca ctacggcaga atgtgtccga tcgagacgcc ggaaggtccg 720
   aacatcggtc tgatcggctc gctgaccact tatggcgtca tcaaccagta cggcttcatc 780
   gaaacgccat acagagtggt agataaagaa acacacagag tcaccgacga gattcactat 840
   ctgacggcag acgaagaaga aatgatggtc gtcgcgcaag cgaacgaacc gctgaccgaa 900
   gacggacact ttgtcaacgc gaaggtcgcg tccagaggcg aacatggaga aatcgcactg 960
   atgccgagag aaaaa 975
<210> 109
   <211> 2001
   <212> DNA
   <213> Unknown
<220>
   <223> Unknown gut bacteria
<400> 109
   acggtttctc tgaccggatt gggactggat tttcgcccga tggctgtacg tgctctgggc 60
   gcgctgctga actatctttt tgaaacacaa aaacaaaatc tcaaacatct gacgcacatc 120
   aggcattatg aaatcggcac gcgcatggcg ctggacaagg cgacggtgcg caatctggaa 180
   atcaccgaga cgctgtacga aaaaaaagta caagggtcgc ttttgggtgt cctcgacaag 240
   acgcgtaccg ccatgggcag ccgcaaaatg cgccaatggc tgcgcgaacc gctcaatgac 300
   gcagcggaga tcaacgagcg gctcaatggt gtggaggaac tgtatcatga tctgctcaca 360
   cgcagcaata tcagagaaaa tctgcgacac gtctacgatt ttgaacgtct gatcggacgc 420
   attgcatgtg ggaacgccaa cggcagagac atgatcgcgc tgcgtaattc catcgcggtg 480
   ctgccggagc tcaagagtga tctcgacctc atgcaaagcg cggcacttcg aaaactttgt 540
   gacatgattg actgtctgac ggatgtttat gacgtgatcg accgcgcgat ctgtgaagac 600
   ccgccgttta ccatcaaaga aggcgggctg atcaaggagg gcttttcgga ggaactggat 660
   catctgaact tttcgattca agacggaaag gagtggattt ccaatctcga aaacaccgag 720
   cgcgagcgca ccggcatcaa gaacctgaag gtcggctaca atcgggtgtt tggctattat 780
   ttggaagtta ccaaatccta ttttgatttg attcccgaaa actacattcg caaacaaacg 840
   ctggccaacg cggaacgctt cattacgccg gagctgaagg agatggaaaa tctggttctg 900
   aatgcgcaga ccaagatcaa tcaactggaa tacgagcttt ttgtcggtgt gagagaggaa 960
   atccagcgct atatggaaac cattcaacag acctccgcgg cggtcgcgga gctggatgtt 1020
   ctgtctgcct tcgcgcaggt cagtgaaaag cttggctatg tgaaaccggt ggtggatact 1080
   tcgatggaac tggagattgt caagggcaga catccggtca ttgagcagat ggtcagtgac 1140
   ggtttgtttg tgtccaacga cacctatttg aatgatcgtg acgcgagcct gcttttgatc 1200
   acaggaccga atatggcagg taagtccacc tatatgcggc agaccgcgct gatcgtgctg 1260
   atggcgcagg cgggctgctt cgtgccatgc gcgcaggcgc atatcggggt gtgtgaccgc 1320
   atctttacgc gcatcggcgc ctcggacaat ctggcacagg ggcagtccac cttctttgtg 1380
   gagatgagcg agctgtccta tattctcaac aacgccaggg cgcgcagtct gatcattttg 1440
   gatgagatcg ggcgcggcac cagcacctat gatggtctgt cgattgcctg ggctgcctgt 1500
   gagtatctct gcaatgatca cacccatatc cggactttgt tcgcgacgca ttatcacgaa 1560
   ctgacggtgc tggaaggtca gcttccgggt gtacgcaacc tgaatgtcga tgtcacagaa 1620
   gtcggcggca atatcgtctt cctgcacaag atcgtggagg gctccgcgaa tcgcagctac 1680
   gggatccatg tggcaaagct ggcgggaatc ccgaagattt tgcttgaaaa cgcgcaggaa 1740
   aagctcgctg agctggaggg cagcaccacg agcgccgcag ggaccgcacg cgtaagccgc 1800
   agcgccgatc catcgggaac ggaaggacgc gaggacagag caaaaacgcc tgcctgtgag 1860
   gaggaaagcc agctttcaat gttttccttc gcgccgaacc cggtgattga aaaattacgc 1920
   gcgctgaatt tgatggaaat tacgccgtcc aaggcgtttg cgattttgga ggagctcaag 1980
   gcttctctgg gaaaagaata a 2001
<210> 110
   <211> 951
   <212> DNA
   <213> Unknown
<220>
   <223> Unknown gut bacteria
<400> 110
   ttcttcgcgg cggtgctgtt cttcgcgctg cgcccgaaca acattatgaa ctccatcggc       60
   aaatatctca atccgatatt tttaatttgt ctgggtatat tggttgtcgc ggcgctgctt      120
   gacccgatcc agagcgtagg ttctgtagcg gcaagcggag cttacgcgga taacgcgttc      180
   tccacaggct tcctgcaggg ctatgacacc ttagacgcgc tggcttcttt ggcattcggc      240
   attatcgttg tcaacgtgat caaagagatg ggtgtcgagg aaccgggtgc gatcgcgaaa      300
   aatacgatta aatccggcgt tttcagcatg gtgctgatga cggtcatcta cgctctgatc      360
   gcgattgtcg gcgcgcagag ctacggtttg tacgcggatc agctgggaga accctccttt      420
   aacggcggca acgccttcgc gattgtcgcg cagcattacc tcggcaaggc gggcatgatc      480
   gtgctggcgc tgactgttac cttctgctgt atgaaaactg ccatcgggct ggttacagcc      540
   tgcgcggata ccttcagcgt gatgtttccg cgctccctga gttatcgcag ctgggccatc      600
   gtgtttaccg cgttttcgct tctggtatcc aacatcggct tgtcgaagat tatttccttc      660
   tcggcaccgg tgctgtattt cctgtatcca ctggcaatca tgctgatcct gctcaatatt      720
   tttggtaagt actttgacta cgcgcgtccg gtattcactt gggccattgg ctttacgatg      780
   atcgcggcgg tctttgatct gctcaaggtc gcgggtctcg cgcagggtgt tgtggactgg      840
   gtcagcacat ggctgccgtt ctattcgctg ggtctgggct ggatcgtgcc gggtgctgtc      900
   ggtacgatga tcggacttat aatcgccaag cccggcaaga aaacagcgta a               951
<210> 111
   <211> 1116
   <212> DNA
   <213> Unknown
<220>
   <223> Unknown gut bacteria
<400> 111
   atgatcacaa aagctgaatt tttagaaaaa atcctaaata tcaatcctaa atacaacacc 60
   gagctgatcg gcaaagcatt cgatacagcc cagcgtctgc acgagggaca actgcgcaag 120
   agcggagaac cgtattttat tcatccgatc aatgtcgcgc tgatcctcgc ggagatgggt 180
   atggacgacg cgacgatcgt cggtgggctt ttgcatgacg tggtagaaga tacaccttac 240
   accagagagc agctcgtcgc cgacttcggg gaggaaatcg ctcttctggt ggacggtgtt 300
   acgaaattag gcacgattaa atttgaatcc aaagaggaaa tacaagcgga gaacttccgc 360
   aagatgtttt tggcgatgtc caaggacatc cgcgtcctga tcatcaagct ggcggacaga 420
   ctgcacaaca tgcggaccat ggagtacatg agccccgaga aaaaaatcga aaagtgcaac 480
   gaaactctcg aaatttacgc gccgcttgcc agcaggctcg gtatttccaa agtcaagttt 540
   gagcttgagg atctggcact caaatatctg cacccgcagg agttccagga cctcaaagaa 600
   aaggtcaata agcgtaagga agagcgcgaa gcgaccatca atctggtcat cagcgagatt 660
   cgagacgcgc tgaacgatat gcatctgcat tatgatattt acggcagagc gaaacattat 720
   tacagcatct acaaaaagat gaaatatcaa aacaagcaga tcgatgaaat cttcgatttg 780
   atcgcggtac gcatcatcgt cgataatgtc aaggactgct acgcggtact cggtatcgtg 840
   cataccatgt ggaaaccgat tccgggcaga tttaaggact atatcgcgat gccgaaaccg 900
   aatatgtacc aatccctgca caccacagtt atcgctgaca acggggagcc gtttgaaatc 960
   cagatccgca cctatgagat gcatcaggtc gcggaatatg gtatcgcggc acattggaaa 1020
   tacaaggagg gagacaccac cggcaaagct tccaatgatg acatgaagct ggcatggctc 1080
   agacaatctt tggagtggca gcaggatatc aatgat 1116
<210> 112
   <211> 1608
   <212> DNA
   <213> Unknown
<220>
   <223> Unknown gut bacteria
<400> 112
   caggtatcgg taccggaagg catgaccatc atggaggcag ccgagaaggt tggcgtacat      60
   atcccgcacc tctgttattt gaaggacatt aacgagattg gcgcctgtcg tgtctgcctt     120
   gtggaattaa aaggaatgga aaagctcgtg accgcgtgca acaccatctg cgaagacggt     180
   atggaaatcc tgacgaacag tccgcgtgtc agaagaacca gaaggatcaa tgtggaactg     240
   atgctttcgg accacaacgc gaactgcgcg agctgcatca agagcggaaa ctgcacgctg     300
   cagacgattg ccaatgacct tggcatcatc ctttccgatt atgaaaatac ggcagaaaaa     360
   ggccgctggg ataggaatct tccgctggtg cgtgatgctt ccaaatgtgt gaaatgcatg     420
   cgctgcattc aggtctgcga aaaaattcaa ggcatgggca tctggacgct gatcggttcc     480
   ggctaccgga cgaccatcgg gctcaaagat aatcagtcct tccgcgaaag cgactgcgcg     540
   ctttgcgggc agtgcatcac gcattgtccg gtcggcgcgc tgcgcgaaag agatgacaga     600
   ttggatctca acgacgcgct cgcggatccg gaaatcgtca ccatcgtaca ggtggctccg     660
   gcggtgcgta ccgcgtgggg agagcagatt ggtctcaagg ataaggacgc gaccatgggc     720
   aagctggtct gcgcgctcaa aaaaatcggt attgattatg tctttgatac caattacagt     780
   gcggatctga cgattatgga agaaggcagt gaattcctcg aaagactcgc caacaggaat     840
   cgtcataaat ggccgatgtt tacctcctgc tgcccgggct ggctgcgctt tgtcagaacg     900
   cagtatccgg atatggtaga ggacttgtcc accgcgaaat caccgcagca gatgttcggc     960
   gcgatttcca aaacctatat cgcgcaaaag cttggacttg acccaagtaa gattctttca    1020
   atttctatca tgccttgcgt ggcgaaaaag tttgagtgtg atgtcgagca ggtaaacagt    1080
   gccggtcatg gcaaggatgt cgatctggta ctgacgacca gagaacttga ccgtctgatt    1140
   cgctgcgata gcatcaagcc gcaggatttg atggatatgc cgtttgatga tatctttggc    1200
   gagggctccg gcgcggctgt gatttttggt acgaccggcg gcgtcatgga agcggcgctc    1260
   agaagcgcct attatctggt taccggaagc aatccggatc cggacgcgtt caaggaagtg    1320
   cgcggtatgg atggctggaa ggaagcaaaa tttgatctgg ccggcaccac gctgaaggtc    1380
   gcggcggcca gcggactcgg caatgcgagg aaactgatcg acgcagttaa ccgcggagac    1440
   gcagagtatg atttcgtcga aatcatggct tgtccgggcg gatgcgtggg cggcggcggt    1500
   cagccgtttg tggacggcaa ggaattcgcg ccgaagcgcg cgcttaaact ctacggtctg    1560
   gataaggtca ataacctcag attttcacac gagaatcaag cggtacag                 1608
<210> 113
   <211> 2055
   <212> DNA
   <213> Unknown
<220>
   <223> Unknown gut bacteria
<400> 113
   atggataaat ttgaaagatt tgataaattt gaaaagctgg accgaaaagt agaagaaacc      60
   attcgcgaag tggaaaccat ggacagacgg aagttctatc gcaacagttt tgtctgcgcc     120
   ggcgtctggg cgctgctctt gaatctggtg attgaaacgc tcggcaggtt tccgacagtg     180
   ggaatctttg gcggactgtc ctttatggtg cacagcccgc tggtcttttt atataacgcc     240
   ttgattctct acgcgacctt agtgctggca tcgttgttca aacggagagt tttttctttt     300
   accattgttt cgattttctg gctgggcatc ggcatcatca acggcgtgat cctgacacag     360
   cgcatgacgc cgttcaccgt aaaggatctg agcaatctca atgacggact gaccatcgtg     420
   accaattacc tcagcaagct tgaaatcgcg ggtgcgattg cagcggtgct gatcgctgtc     480
   ggcctgctgg tgctgctttt tatcaaggga ccgcaaaaaa gagacggcgt gaaacgcaag     540
   cgcaatctgg tcgcggtgat tttggtcatc gtcttcacct tcctgtcatc ctcctttgtg     600
   attaagacag gcattgtgca gactttcttc ggcaacctcg cctatgctta tcgagactac     660
   ggtgtggtct actgttttgt caatacctgg ctgaacaccg gaatcagcaa gccggacgga     720
   tacagcaggg aagaaattct ttccatcttc gacaaaaaag aactcggtga tgacaacacc     780
   atgctgctta cccaaaagga tgaggacgag cagcatccga acatcctgtt tttgcagctg     840
   gaatctttca tcgatccgat gacgattcag agcatcacac tttcgcagga cccatgcccg 900
   aacttccgca agctgatgca gacctattcc tcgggcgagc tgaccgtacc ggcctgcggg 960
   gcaggtaccg ccaacgtgga gtttgaagtg ctgaccggac tgtcagtaaa gttcttcggc 1020
   ccgggcgaat atccgtacaa gtcggtctta aaagaaaaaa ccggagaaag ccttgcctac 1080
   gacctcgaca gtctcggcta cacctcgcac gcgattcata atcacagggc agtcttttac 1140
   aaccgcaaca gcgttttttc gaacatgggc tttgacacct tcacgagtgt ggaatacatg 1200
   aaaaatgtcg aaaagacgcc gaagaactgg gcgaaggact atgtgctgac ggattgcatt 1260
   actgacgcgc tgaattctac aaaggggcgc gatatgatct atacgatatc ggtgcaggga 1320
   cacggcaagt atccaatcca gcagatcatg caaaatccgg cgattgaggt cccctcggcg 1380
   ccgacggagg cgctgaagtg gaaatatgaa tactatgtca atcaggttta cgagatggat 1440
   cagtttatca aggagctgac agatacgctc gccaagcgtg aggaaccgac cgtcctcgtg 1500
   atgtacggcg atcatattcc tgcgctggat atgaccgagg atgatctgga aagcggcaat 1560
   ctctacggca cgcagtatct gatctggaac aatataaatc tgccgaagga cgacgaaaat 1620
   ctccacgcct atcagctcgc ggcgcacgtg atggacatgc tggatatgcg ggtaggcacg 1680
   atctttacct atcagcagaa ccacaagaac agcgaaacct atctcgcgga tttgaaggcg 1740
   cttggctatg acatgcttta cggcaaatac tatatctacg gcggcatgaa tccgttcaag 1800
   ccgacgaatc tgaagatggg cgtcaacaat atttctatcg aagaaatcgt caaaatcgga 1860
   gacaaatatt acatcaaggg caggaatttc acgcagtaca gcaaggtcac gctgaacggc 1920
   aaggagctca agaccatata tctgggcgaa aacgtgctcg gacttttgga ggatgttgat 1980
   ccgaacgacg cggataagat gaaggtcagc cagattgaga acaagagcgg agaaattttg 2040
   agtaccaccg aatag 2055
<210> 114
   <211> 1488
   <212> DNA
   <213> Unknown
<220>
   <223> Unknown gut bacteria
<400> 114
   gagagcgtaa cgatctgtat gccgaacgcg ccgcaggcgg tcatcatgtt ctatgccgtc 60
   aacaaggccg gcgcgattgc caatatggta catccgctgt ccagtgaatc cgaaattgaa 120
   ttctgcttgc gccagtcggc cagtgtggtc tgcctcacgc tcgatcagtt ctacggcaag 180
   tttgctaata tccgcggcag ggtgcagctg cgaagtctga ttctgaccag catcaaggac 240
   gcgctcagcc cgatcaaacg caagggttat tatctggcgg agggccgcaa ggtgaagaag 300
   gttcccgtca atgccgaaat tgtctggtgg gaaaaatttc tgcgtgacgg cgcggactat 360
   cacggtccat atcatgtggt gcgcgaggcg ggcgatcctg cggcgatcct ttacagcggc 420
   ggcacgaccg gaacgatgaa agggatcctg ctttcaaatc tgaatttcaa cgcgctcgga 480
   cagcagatca tcgcgacgaa cccgatgttc aagccggggg acaagatgct ggctgtcatg 540
   ccgatcttcc atggcttcgg actgggcgta tgcatccact ccatgctttc gcaagggggg 600
   cgctgcctgc tcatcccgcg cttcaatcca cagagctacg cgaagctgct caaaaagcac 660
   aagccgaatt tcatcgcggg tgtaccgacg ctctacgagg cgctgctgcg tctcaaaacg 720
   ctcaaacatg tggatctttc ctgcttaaaa ggcgttttca gcggcggaga ttctttgtcg 780
   attgagctaa aaaaacgttt cgacaaattt ctcgcggagc acaacgcgac gattccggtc 840
   agagagggtt atggcacgac ggaatgcgtg accgcgagct gtctgacacc gtcgcactgg 900
   gcgaaagagg gcagcatcgg actgcctttc ccggatacct tctataagat cgtcaaacct 960
   ggtactgaag aggaagtgcc ttacggcgag gaaggtgaga tctgcctgac cggcccgacg 1020
   atgatgatgg aatacatcaa caatccggag gaaacggcga acactctgcg caggcatccg 1080
   gatgggctga aatggctgca taccggagac cttggcatga tggacgcgga cggtttcatc 1140
   tacttccgcc aaaggatcaa gcgcatgatc gtcaccagcg gctataatgt ctatccgtct 1200
   cagatcgaaa acgtactcga cgcgcacgag ctggtgcaca tgagctgtgt gatcggtgtg 1260
   ccggatccat acaagatgca gagggtcgtg gctttcatcg tgctgaaacc ggacgtcaag 1320
   attccggagg atgcggcgcg cgaaatcctg ctcgcttatt gtgggaagca tgtcgcgaaa 1380
   tacgcgatgc cgaaggagct ccacttccgc gacagtctgc ccaagacgct ggtcggcaag 1440
   gtggcctatc gggttttgga agaggaagaa gcaaagaatt acgcgtaa 1488
<210> 115
   <211> 1884
   <212> DNA
   <213> Unknown
<220>
   <223> Unknown gut bacteria
<400> 115
   atgaaaaaag atctgacaca atatcgtttc cccgaagacc tcaaaaacat gtccgacgag      60
   gaaatggagc tgttatctta cgcgattcgc gagtttttaa tcgagcatgt ttccaaaacc     120
   ggtggacatc ttgcttccaa cctcggtgtc gtggagctga cgatagcttt gcataagatt     180
   tttgacagtc cgcgggacaa aatcatttgg gatgtcgggc atcaatccta tgtccacaag     240
   attttgaccg ggcgcgcgga cgcgtttgat accctgcggc agttcggagg tctgagcggc     300
   ttcccaaagg gcaaggaaag cgcacacgac gcgtttgata ccggacacag cagcacctca     360
   atctcggcag cggcaggtta cgctgcggcg cgggatattc agggcgagga ttatgaagtt     420
   gtcgcggtca tcggagacgg ttcgctgacc ggaggcatgg cttatgaggc gttaaacaat 480
   atcggagctt ccaagtccaa gctcatcgtg attctcaacg acaacggcat gtcgatttcg 540
   ccgaatatcg gtgggatttc caggcatttg gggtcgctgc gcacctccaa aggttatctg 600
   agcgcaaaga gcttcgtcaa gaataagatc ggcgcgattc ccagtgtggg caaaggcatc 660
   gcgcagggat tggccgatat caaaaatgac attaaataca cgattttgga gcgcggcggc 720
   gtgctctttg aagaattggg atttacctat ttcggaccgg tggacggaca tcatctgccg 780
   gagcttttgg atgtattgtc gaaggcaaaa aacctgaatg aaccggttct gatccacgtc 840
   atcacaaaaa aaggcaaggg ataccgcaac gcggaacaaa atccgggcaa atttcacggg 900
   atcggcagct ttgaccccga aacgggcaaa gaggcggcga aatccgagaa accgacctat 960
   tcaaaggtca tgggcgagta tctgacagag cttgcaaaaa aagatccgcg cgttgtggca 1020
   atcaccgcgg caatgggaga ggcgacaggg cttggaggat ttgccgaaaa gtttccggag 1080
   cgcttttttg acgtcggcat tgccgagcag cacgctgtga catttgcggc agggcttgca 1140
   aaagccggta tgcggccgtt tgtctgtatt tattcttcct tcctgcagcg ggcctacgac 1200
   cagatgctgg aggacgtctg catgcaggaa cttccggtaa cctttatgat cgcccgcgcc 1260
   ggcatcgtag gcgcggacgg tgagacgcat cacggcatct tcgatctcag ctatcttgct 1320
   acgatgccgc agctgacggt gctggcaccg acttccgacg agatgctccg cgcgagtatg 1380
   gatttcgcga tggaacaagt cggaccgtgc gcgatccgct atccgcgcgg ggaggttatg 1440
   actgaagaag aaggcaggat tccgagcctg atgtattaca accggacaat ggaatacggg 1500
   aacacgctcc ttacggatcg tgtggagatt ctcggcgtcg gcaaaatggc cggcatcgga 1560
   cgggaagttt ttgtccggct gttagaaaaa ggcgtgcagg cacgttatca ggatgtgaat 1620
   atcgtgaaac cgatcgaaac ttccgtaaat gccatgaaca cgctgcgcgg cgatctgatc 1680
   gtgaccatcg aagataatat tgtatccggc ggtttcggcg aaagcctgcg aggcgcttta 1740
   gagccgaagc atcgtctttt gaacttcggc tggccggatc gctttatcgg ccaggggacg 1800
   tttgacgaac tgactgaaaa atacggcctg tctgcggcaa aaatcacaga aaggatttgt 1860
   gaagaacttg aaagaaaggc ttga 1884
<210> 116
   <211> 1578
   <212> DNA
   <213> Unknown
<220>
   <223> Unknown gut bacteria
<400> 116
   atgaaggtaa gcgttaagga ttgtctacag ctggaagctt tccgcaagtg cattgtcgtt        60
   gcaggagaaa agcacatgga tcgccgggta aaagcggttt cggtcatgga cgcggcaacg       120
   gcggaggaag cggtcaagtg cagcggcagc agcggcaata tggtgttgac ttcgttcgcg       180
   ggcatgaaac atgacgtgaa acagcagcag gaaacaattc gcgaactggc aaaggcaggt       240
   gtcgccgcgc tggtggtttt tcgcagggag cagaccacgc agcctgttga caaggcagtg       300
   acgcgcgcag ctgatgaggc cggtctgccg ttgatcatca tggtcgatgg agaaaacatg       360
   gactattcgc agatcatcgc ggaggtcatg gaacacgtgc tttatggcaa taattttaag       420
   aacagcctga tcaacaacac gatttttcat ctgttgaatt ttgaaaagca cgcgaatttc       480
   cagcaggcgc tgcgtgaggc ggcgatcaac aacgactttc aggtagtgct tttgagcgag       540
   gacttcaacc cgatcttcgc gatcgaaacg cggcatcaga ccacaatcaa cgaggcgatc       600
   cgccgcggga aggaagcggc gctgaacctc agcaacatct acagtctgat cgagatcgac       660
   ggggtgctga cctactgggg tctggtacac atcaataacg acaaatacta tctgttcatt       720
   gtcgataacg acgacaatta ttcgtccggc gaaatgacca agctggccga gatcatcgag       780
   ctggcgatgg gcatgtggaa gtacacgccg gagcgtgacg cgaaggcaga gcttgtcaag       840
   gcgctgatgc gcggcaacaa gagtctggcg tattcgatcc gcgaggaagc aggcgtgaaa       900
   ttaaccgaca ttctttcggt cttttatgcc aaaggtattg aggataacca tgcggaggaa       960
   gtgattcatc ggtttgtcag cgagggcctt ttggacatca tcaagatcag cgaggaaaac      1020
   gaaagctacg cgctgctgct caagggggcg aagctggctg atcaggagga aaatgaggaa      1080
   aaggctgttt gcctgagatt cttcgacgcg ctcaaggaaa acaagacggt ccgcattttc      1140
   catgtgaccg gcatcgacgg catcgagggc gcaggagacg gattccgtct gatcggcgaa      1200
   acctgggcgt ttgtaggaag cattttcccg tacaagcggg tgttcaccaa atatgaactg      1260
   acactggtca gcaactgcat caatattcaa gtgcagggcg gctatctgaa aaagaactat      1320
   atggatcttt tggagccgtt tcgcaaggag ggggacaaca agtccaggca gcttttggaa      1380
   accctggaaa ccttcgtgtt ggacgcgggc atgaacagcg gcaagacctc tgagtttatg      1440
   ggcatccaca caaacacagt gcagtatcgc ctgaagaaga ttaatgaaat cctcggcgcg      1500
   gagattaccg gaaaccgcgt gattccggga ttgacaatcg cgctcgcgct gcggagaatg      1560
   gaagcggcgt cgaaatag                                                    1578
<210> 117
   <211> 1389
   <212> DNA
   <213> Unknown
<220>
   <223> Unknown gut bacteria
<400> 117
   atgaggtatg aagatttaaa gtatttggag gtaccattac cggaagcaat cgcgaaggaa      60
   aaatggggcg gtttctttga gaatgcccgc agccgaatcg cggcatatct ggatgacgaa     120
   acaacagatt acgcgctgcg ctgccgattg gaaatggaac ttggaatact gcaaaacctg     180
   caggaaagat atactctgtg tccggaagag gctcttttga tcatgcagga aaaaattccg     240
   gttatgaccg cggaagaatt ggaaaaactg cgcatggaag ataaagcaga ttggatttac     300
   ataaacggac aggttcgtta cttggacagc tttgctgcta cgctttgcaa ggcttatccg     360
   gaagtttggt cgcgaaccga agccggtgat acgagcgatt acacgctcat agaggattac     420
   attcatggct gcgaagacaa tctcgaaaga accgcgcata tacacatccg acatgaagtg     480
   gaaatcgcgc cgcgggcgct gcgtgaagga gaaacgattc gagtacatat tccgattccg     540
   cgggaacggg ccggcatcac gaattttaaa atgctgcgga cttcgcatac gccttcgttt     600
   atttccgata gcaaggcggc acaaccgaca atctattttg aagaaatcgc ggcgaaaggg     660
   caggtgttct ccgtggagta tgagcttgat tacacaactg cgtatcataa gcttgaaaca     720
   aggtcggagg cggatatgcc gcaggaggcg agcactgctt gtgaagaggc tttttcgagt     780
   gaaaacctca tggaagaagc gccgcatatt cgcttcactc cgcatctcgt cacgctttgt     840
   gaaaaactta tcggaggcga agtcaatccg cttcgcaagg ccagagcaat ctataattat     900
   gtgacaacga aaatggacta tcgttttgtg cgtgattacg cgagcattga aaatctatca     960
   gaatactgtg ccttgaatcg taagggggat tgcgggattc attctttgct gttcattacg    1020
   ctttgccgta tcgcgggaat tccggcgcag tgggagtccg gattagaagc ctcaccgact    1080
   tatgttggcg aacatgattg ggcacgcttc ttcattcaag atttggggtg gctttacgcg    1140
   gaccctgccg gaggagtgct ggcttacgtg agaaaatcgt atgatcgctg gaatttcttc    1200
   tttggaaatg tggacccgta tcgaattccg atcaatgatc ggtttcagca tgatttttat    1260
   cctaaaaagc agcattggcg catagatcca tatgacaatc aatgcggtga gatggaatat    1320
   gaggacagcg gcgtatatgg agatgatctt atttaccgcc atatcccgat tgacatctat    1380
   ttgaaataa                                                            1389
<210> 118
   <211> 2325
   <212> DNA
   <213> Unknown
<220>
   <223> Unknown gut bacteria
<400> 118
   atggcagaaa gaatggtcat gccctgcgtg ccgctgcgtg gattgtcggt gtttccgcac        60
   acaatcctgc attttgacat tggcagggaa aaatccgtca aagcactgga agccgctatg       120
   acggcagata aacagttatt tctgacttct caaaaggatg aaaacatctt gattccgacg       180
   ccggaggatt attacgaggt cggaacggtt gtaaagatca agcaaatgct caaaatccaa       240
   ggtgacgcag tgcgggtgct ggtagaaggc cagtatcgcg cggtgatgag cggcgtcgcg 300
   acggaggagc catatatcat ggcagagctt acggaggcgg aaattacgcc ggcagatccg 360
   caggaggtag aagtgcaggc gatgatgcgc tctgtactct atgccttcga tgaatacatg 420
   gaattgaacc cgtcgatcaa ggatgaggtt tatgagctga tcagctcgat cgaggagccg 480
   gatatcttcg cggatacggt ggcgatgcag ctggacgtta aggttgctgc caagcaggag 540
   gtgctggagg ccttcgacgt gaaacagcgt ttgcaaatcg tcaaccgcca gattttggag 600
   gagaatcaga ttcttgccat ggagcgcgac atcagcgagc gtgtgcagga agcggtgaaa 660
   cagaatcaga aagaatatat cctgcgagag cagatgaagg tgattcagga cgaactcggc 720
   tacggagaag aagccgcaag cgaggtggag cgctggagtg agcagcttca aaagctgaag 780
   ctcgatccga agattgagga aaaggtaaaa cgtgaaatcg ctaaatttac caaaatgccg 840
   ccgagctccg cagacggcag cgtcatccgc aattacgtgg agacaatttt ggaattgcct 900
   tggcacaaat cctccaagac caatgctgac ctcaaaaagg cggaacgcat tctcaacgag 960
   gatcactatg gacttgaaaa ggtcaaggag cgtattctcg aatatctggc cgttgtgcac 1020
   ctcaataagg cgatcaaagg tccgattatt tgtctggtag gacctccggg tgtcggcaag 1080
   acctcgatcg cgaaatccat cgctcgcgcg acgggcagag agtttgtccg catgtcgctg 1140
   ggcggcgtgc gcgatgaagc agagatccgc ggacacagaa gaacctacat tggcgcgatt 1200
   ccgggacgaa tcattacctc tatcaaagac gcgggcaagg ataatccggt cttcctgttc 1260
   gacgaagttg ataagatcgg cgcggacttt aaaggggatc cggcttcggc actgctggag 1320
   gtgctggacc cggagcagaa caaggaattc accgatcatt tccttgacat tccgtttgac 1380
   ttatccaagg tcatgttcat tacgacggcg aactccaccg aaacgattcc gcgtccgctt 1440
   ttggacagaa tggaagtcat ttcggtaccg ggctacaccg aggaagaaaa agtaaaaatc 1500
   gcgcagcagt acttaatccc gaagcagatt cgggcacatg gcttgaagcc gaagaatttt 1560
   ttgatcagtg agaaggctat ccgcgatctg atcaattatt ataccagaga atccggcgtg 1620
   cgtaatttag agcgcgagat cggaagtctc tgccgtaagg tcgcgcgtaa gattgtttct 1680
   aaaaaaggga acgcttttcg cattacgccg gcgagtctgg aaagctatct gggcaaaaaa 1740
   agatatcatt acgatgtcgt ggaaggcgag agcgaggtcg gggtaacgac cggcatggca 1800
   tggacacagg tcggcggcga tacattgttc attgaaacag cagtcgtgcc gggaaccggc 1860
   aagattcagc tgaccggaca gcttggtgac gtgatgcagg aatctgcgaa ggcggccatc 1920
   acttatattc gttcaattgc gggcgagtac gggatcgatg aagaattcta taagaagaac 1980
   gatctgcacg ttcatgtgcc ggaaggcgcg gtgccaaagg acggcccgtc cgcgggcgta 2040
   acgatgttca cctcggtgat gtccgcgctg acaggcaatc caatccgtaa ggacgtggca 2100
   atgaccggag aaatcacgct gcgcggtaag gtgcttccgg tcggcggcat caaagaaaag 2160
   gtgctggcgg cacaccgcgc gggcattcgc acgattcttt tgccgcgtga aaacgaagcg 2220
   gatatcgaag agatcccgca aagcgtgcgc aagcagctca ctttccacct gctcgataaa 2280
   gccaaggagg ctttggaata tgcgatggta aaagattcag agtga 2325
<210> 119
   <211> 1125
   <212> DNA
   <213> Unknown
<220>
   <223> Unknown gut bacteria
<400> 119
   atgagaaaag agtgggaagg atttgtcgcg ggaaactgga cagagcacat tgatgtagag 60
   gaatttatta aattaaacta ccatccgtat gacggtgacg gcagtttcct cgcgggaccg 120
   acagagagaa ccaaggaatg cagtgacaag gtgcacgagc ttttggtcgc ggagcgcaaa 180
   gcaggcggta cgctgaaggt tgacgcgagc cgcatcatgc gcgtgaacgc attcggcccc 240
   ggctacatcg ttccgggcaa ggatatcatc gtcggactgc agacagacga accgcttaag 300
   agaggtattt gcccgttcgg cggcatcaag atggtcagag aagaagtgaa ggagtacggc 360
   gttacactgc cgccggaaat tgacaatatg ttcaactact tcacgacgca caacgacggc 420
   gtttttaagg catactccaa cgaaatgcgt caggtcagac atctcggctt cattacggga 480
   cttccggacg cttacggccg cggcagaatc atcggtgact acagaagagc agcgctctac 540
   ggcatcgact atctgatcga ggaaaagcag aaagaccatg aaatgatcgc ttccagacac 600
   gtgatgaacg aagaaaacat tcgcaccgcg gaggaagtca gcaatcagat ccgctccctc 660
   aaggaaatca agaaaatggc agaaagctac ggcttcgaca tctccggacc ggcgaaagac 720
   gtcaaggaag cgatccagtg gacctacttc gcgtatctgg caggcatcaa ggaagaaaac 780
   ggcgcggcga tgtccatcgg cagaacctcc acgttcatcg atatctacgc agagagagac 840
   cttgcgaacg gcacctacac cgaagaacag atccaggaat tcgtggatga ctatatcctg 900
   aagctgcgtg ttgccagaca tttgagaacc agtgaataca acgagctgtt cggcggagac 960
   ccgatgtgga ttaccgaagg tatcgcaggc gtcggcatgg acggcagaca taaagtaacc 1020
   aagaactctt acagagtgct gcagacctta tacaatttag gaccggcacc ggagccaaac 1080
   ctgaccgtat tatggtccga aaagctgccg gaaccgttca agaag 1125
<210> 120
   <211> 1803
   <212> DNA
   <213> Unknown
<220>
   <223> Unknown gut bacteria
<400> 120
   atggattatc agcagagaaa acaaatggag cgcaagagag ccgaggattt catcgcgagc      60
   attaacgcga tgcaggatcc ggatattatg tacaaagtca gccatccgat caccgatctc     120
   aaggatatga tgaaccagag cgtagcgcgc tacggggcgg atcatgtcgc cttcaaacag     180
   cgcttcgtga agggggagcc gtttcgcgag attacttacg gacagacact gaacgatatg     240
   aacgcgctgg ggacagcgat gctggcgcgc ggcctgcggg acaagcgcgt ggcgatcatc     300
   ggagataatt gctaccagtg ggcttcaacc tatctggcgg ttacaggcgg ggtaggcgtg     360
   gtcgtgccgc tggacaagga actgggcgcg tctgagctgg agcagttaat tatcgaagcg     420
   gaatgcgagg cgattttctt tacaaaaaaa tttctcagca tctttaccga tatgcgcgag     480
   cgcggtgaga caaagctgcg tatgcttgtg aatctcaacg cagaggcggc ggcagacggc     540
   gtcgaaagct ggtccgcgct gattgcggag gggcgggaaa agatcgcggc aggcgaccgt      600
   tcctatctgg acgcggaagt atatccggat gagatgggtg tgctgctgtt tacctccggc     660
   acgacgggca tcgccaaggg cgttatgctt tcgcagacaa atatctgctg cgacctgatg     720
   agcgcgccga ccctgctgga caccaacgag cgggatactt tcttcagcgt gctgccgatc     780
   caccatacct atgagtgtac ctgcggcttt ttgatgccga tgtataaggg tgcctgcatc     840
   ggatactgcg aggggctcaa atacattcag aagaatctgc aggagatcca gccgacgttc     900
   tttttgggtg tgccgctgat ctttgagagt ttgtacaaga cgatctggaa aaatatccgc     960
   aagcagggca aggaagccac ggttaagaaa gtgatgaagc tcaacaagac cacttctaaa    1020
   ttcggtctga atctcaataa attgctgctc aaggatatcc tcaaggtctt tggcggcagg    1080
   atggatatga tgattgcggg cggtgcggcg atcgacccgc agattctgca gtttttccgt    1140
   gatctgggca tcatggcgct gcagggttac ggtttgacgg agtgcgcgcc gatggcggcc    1200
   ttgaatcagg atttagagcc acgcaacgat tcagtcggtc atgtgctgcc gggcatggaa    1260
   gtcatgatcg cggacaagga tgaggacggt atcggagaaa tctgcctcaa gggaccgaac    1320
   gtcatgttag gctattatca catgccggag gaaaccgcga aggttatcgt cgatggctgg    1380
   tttcacaccg gtgacctcgg ttacaccgac ggtccgtata tctatattac cggacgtaaa    1440
   aagaatgtca tcattaccgc gaacggcaag aatgtattcc cggaagaact ggaatattat    1500
   ctcggcaaga gcacgctggt ttcggagtcc atggtctggg cttcacagga cgcggacgga    1560
   caggacgctg tgatcgtagc gaccatcaag ccggatatgg aggaagtgga agcagagctc    1620
   ggcaaggaag cggcgcagga tccgaagaag gtggaagcac tcttatggag catggtggat    1680
   aaggtcaacg caaacctgcc gctcttcaaa aagatccgcc ggatcaacgt tcgcaaggaa    1740
   gactttgaaa agaccaccgg caagaagatc aaacgcttcg tggaatccaa taaacagcag    1800
   taa 1803
<210> 121
   <211> 1617
   <212> DNA
   <213> Unknown
<220>
   <223> Unknown gut bacteria
<400> 121
   gatgcaccgg cagctccgct ggtaggcaga gacaaactgc acacggaatt cttaaacgat 60
   attctggtac cgactgcgaa tctgaccgct gacaacgttc cggtttccaa gtttatggat 120
   accgtaaccg gcatgatgcc tgcgggtacc gcggcttacg aaaagcgcgg tgtcgcggcg 180
   gatcttccgc attgggacgt gaagaactgt atgcagtgta actggtgcgc gagcgtttgt 240
   ccgcatgccg tcgttcgtcc gttcgtgatg accgcggagg aagcggaagg cgttgctgac 300
   gcagtaccga tgaagggcgc gaaggacaag aagttcacgc tcggctggtc tctgatggac 360
   tgcacaggct gcggttcctg cgcagaggta tgccctgcga gaaacaaggc catcaccatg 420
   gcaccggcag aagatcactt catgcacgcg gcacaggcaa aatacgatca tttatataag 480
   aatgttgacg aaaaggaaac gccgttcaag gaaagcaccg tcaaaggtgc acagctcaag 540
   acaccgctca tggaattctc cggcgcttgc cctggctgcg gcgaaactcc ttacgcgaag 600
   ctggtaaccc agttgttcgg tgaccgcatg ttcatcgcga acgcgacagg ctgctcctcg 660
   atttggggcg cttccgctcc ggcgacaccg tataccgtaa acaaagaggg cagaggtcct 720
   gcatgggcaa actccctgtt cgaggataac gctgaattcg gtttcggtat ggcgacctcc 780
   gtcaacgcga gaagaaacga attgaagtcc gcagtcgaaa gactgtatga cgctgtcggc 840
   gtgccggcaa aggcgtgggt ggcttccatg cacgacggca aaacctccaa gacaaccgga 900
   gaagttctgc tggaagcggc gaagaagctc gcggacacta acgaagacgc gaaatatgtc 960
   gttgagaacg ctgacctgct gaccaaacca tccatgtgga tcttcggcgg cgacggttgg 1020
   gcttacgata tcggttacgg cggtctggat cacgtcattg cgtccggcga agacgtcaac 1080
   atcatggtat tcgataccga ggtatattcc aatacaggcg gtcaggcatc caaatccaca 1140
   ccggtcggcg cggtcgcgaa attcgcggct tccggtaagg cagtcaagaa aaaagacctt 1200
   gcgcagatcg caatggctta cggctatgtc tatgtcgcgc agatcgcgat gggcgcgaac 1260
   ccggaacaga cactcagagc aattcgcgaa gcggaaagct atcacggtcc ttccctgatc 1320
   atcgcttacg cgccttgtat caaccacggt gtcaaagccg gcatgaacaa gtccatgctc 1380
   gaaatgaaga aggcagtcag agccggatac tggaacctgt taagatacaa tccggagctg 1440
   gaagcgaagg gcgaaaatcc gctgtccgtt gactccgctg caccgacaga atcctacaga 1500
   gatttcatca tgggcgaagt tcgttacaac tccctgaagc tcggattccc tgaaagagca 1560
   gaaaaactct tcgaaaaggg tgaagaatgc gcgatggaaa gatataagga actgaag 1617
<210> 122
   <211> 1218
   <212> DNA
   <213> Unknown
<220>
   <223> Unknown gut bacteria
<400> 122
   atgtataact ttgtacttgc atttgtaatc tgtgccgttg tttacatcat cggcgaggtg      60
   gtcgcgactg tgaccaaggc atgggttccg tccgtattcg tcaccgcggt ggtactgctg     120
   atcggatact ggactgttat cccaaaaaca gtagtcagtg attctgtact gattccgttc     180
   ggaagcagta tcggtattta tctgttgatt acccacatgg gtactgtcat cagtttgaaa     240
   cagctcaagg aacagtggag aaccattgtc gtttgtctgg caggacttgc cggcatgatg     300
   gtactcggct atttcgtcgg cagtctggtt atggacagaa acctcatcat cgcaggtctt     360
   ccgccgctga ccggtggtat tatcgccgcg acgatgatgc agaccgcagc gcaaaacgcg     420
   ggactggagc tggcagcggt attcgcaatc gcgatgtact gtattcaggg ctttatcggc     480
   tatccgctgt ccacgatctg cctgcagtcc tatggcaaga agcttgtcaa gggcctgcgt     540
   gacggctcca tcgtcattac cgagcaggac agaattgaaa tggcaaacgc gggcatgacc     600
   gcggtgccta ccgatgacgg caagaaaaaa ctgcttccgc cgattccggc aaaatacaat     660
   acccctgtga tgatgctctt gaagctcggt atcgtagcat ggctcgcgag cctgatgggc     720
   actgtgtcct tcccgatcat cggcaccatc agcggcgcga tttgggcatt gatattcggt     780
   gttatcttca ccaccatcgg tttcctggat tctaatttgc tgaacagagc gaactccttc     840
   cagatcacga tgttcgcact gatgatgtac gtattcgacg gactcaagga ctgcacaccg     900
   gagatgctga agcagatcat cggaccaatg ctgatcctca tcgtcatcgg cgttgccggc     960
   atggccttgt tctcattcat catcgcgaag atattaaaga tgtccttccc gctggcgttt    1020
   gcaaacggtc tgaccgcttt atacggcttc ccatgtgacg caatcatcac agagtccacc    1080
   tgcaacggcg tcggacagac ggaagaagag agaaaatacc tgctgagcaa gatgttcccg    1140
   tccatggttg tcggcggctt catcacagta acgattactt ctgtatttat cgcaggcgca    1200
   tttgcgaaat tactctaa                                                   1218
<210> 123
   <211> 1824
   <212> DNA
   <213> Unknown
<220>
   <223> Unknown gut bacteria
<400> 123
   atgggactaa aaaaactggt cgttgacgcc ttggcgctcg gcccgacatc gagaggtttt      60
   acaggaggag aagcgcgccg ctgggtgcgg gagtcttaca ggcacagaac ccgtaactac     120
   ggattttcca aaaaagatac gcgctgggca ttgcggcacg gcttcctgcc ggaacaggcc     180
   gccagacttg gcatcactgc cgagaacgtc ggcgactata tctctgcgaa ggactacgcg     240
   tttttaagac cgctgaacgg aacctacagc aagtggatca ccgataaggt aaccatgcac     300
   acgattttta agccgttccg cggttatatg ccgacactct actatcaaat cagcaagcgc     360
   tacgatgaaa cggtgctgat tcctctgtac gacgacaacg caggtgacac tttcgaggat     420
   cttttcgcgt tattgcagga aaagggcagc ctgacggtgt cctctgccaa tggtggttac     480
   gcgagtacgc tggagtatcg ggatggtgtg ttttatctgg agggcagaga acgcccgaag     540
   gagagaattc aggaaatcct cagcgattac cgcgtaacac tggtagtcaa agaacaggta     600
   gaactcagcg aagataccga ttacggtgta ctgaatctca tcgttttcaa tgagtttggt     660
   gacaatcccg tcatcggtga cggatacttc gtctttgacg aatacgaaaa gacttctctc     720
   aaggtgttgg cgatgaagca ctcggacagc ttggaagaag ccgatgtcga ggatgatatc     780
   tatcgctttg tcaaggcgga gccttgcgat gtgaccgaag gcagctggcg cggcaaaccg     840
   attccgcatt gggacgaaat tgcggatgtg attcgtcgcc tttgcgtatt tgtgccgcag     900
   ctggagtttt tctgcgcgga aatcgtgatt tccgcggatg gtttcaaaat tgttaatctg     960
   ctcaaccatc cggaatatcc gaccgcgaaa ccgttcagca aggaaacctc cgcgtatctg    1020
   aagcgtaaag tagaacaaaa aaaagaagcc tacgcgaaag cgggggtcag aatcagtcgc    1080
   gggctgcata agatgcatct gcgtattcgc gcgaaattcg cgcgcgcctt ctatccgaag    1140
   gggctggtgc cttaccagtc tacccgttgg atttctaatg tctggacaga tttttggacc    1200
   aataaggagg cgaccctccg agaaaagctg tgggcgtaca agcatggatt tttgtcttac    1260
   cgcatcccac aatacggcat caccgaggaa aatctcggag aatacatttc cgattttgaa    1320
   tacaagtggc tgcgtcacat taacccaaaa taccggaagt ggatggagga taagattacg    1380
   gtcaaatatg tctgctcgga ctacaacgac tgcttcccgg cttactatta ccatatcatc    1440
   tgcaagaacg gaaacaacaa ggtaatctcc atgatggatc tgccggaggg ttacaccaac    1500
   accttcgacg agatcttccg tctggtggag caaaaaggcg tgctcgcgct caagccggat    1560
   gaaggttcac atggagacgg cttctataaa ttcacctatg aagacggcaa ataccagctc    1620
   aattatcagg acgtaacgaa gcagcaggtg ttggatattc ttgaagatat caacaaccag    1680
   tatctggtaa ccgaatatat caatatgtgt gacgagctca aggctgtcta tgacggcgcg    1740
   gtcaatacgg tgcgcatgat cgttttcaaa aaggacggca agaatccgca gattggcaat    1800
   gcctatgtgc gtttcggctc caaa                                           1824
<210> 124
   <211> 1602
   <212> DNA
   <213> Unknown
<220>
   <223> Unknown gut bacteria
<400> 124
   atggcaaaag aaaaattaaa ctgggaagat gaacaggtta gacataccta cagacatacc      60
   gcgtctcaca tcatggcgca ggccatccag cacctgtggc cggacaccaa gttcgcgatt     120
   ggaccggcta tcgacaacgg attctattac gatatggagt gtgagcataa attcagcgaa     180
   gaagatttcc tcaagattca gaaggaaatg aaaaagattg tgcagcaaaa cctgccgctg     240
   gtcagaagcg aggtttccag agaggaagca cttcaattct ttaaagagag aaatcaggac     300
   tacaaagtgg aactgattga agacctgccg gaggatgcgg tcatcacgat gtatacacag     360
   ggagatttta cggacctgtg cgcggggccg cacatggaat ccaccggtca gattaaggcg     420
   ttcaagctga tgagcgtggc gggcgcttac tggagaggcg attccgacag acagatgctg     480
   cagagaattt acgcgactgc attcccgacc caggaagaac tggatgccta cgtcgaaaaa     540
   ttggaggaag cgaaaaagag agaccaccgc aagatcggca aggagatgga tctcttcatg     600
   ctgaccgagg aaggtccggg attcccgttt ttcctgccaa agggcatgat catccgcaat     660
   gagctggagg ctttttggag agcggagcat aagaagagag gctatgaaga gatcaagaca     720
   ccgctgattc tcaacgagca tctttggagg acctccggcc attgggatca ctataaggac     780
   aacatgtatt tcaccaagat cgatggcgag gattacgcga tcaagcctat gaactgcccg     840
   gggtcgatgc tggtatacaa gcgtaaaatg tggtcctacc gagattttcc gattcgctgc     900
   ggcgagctcg gacaggtgca cagacatgag ctttccggcg cgctgcacgg tttgatgaga     960
   gtccgtacct tcacgcagga cgacgcacat atcttcatgc tgcctgaaca ggttaacgat    1020
   gagatcaagg gcgttgcgaa attctgcgat gatgtctata aaatgttcgg tttcgagtat    1080
   catgtagagc tttccaccag accggaggac tccatgggaa ccgacgaaga atgggaaatg    1140
   gcagaaggcg ctctcagaaa cgccatcgag gaaatgggtg ttccgttcgt catcaacgag    1200
   ggcgacggcg cgttctatgg cccgaagctg gacttccatc tgcaggattc catcggcaga    1260
   acctggcagt gcggcacaat ccagcttgac atgcagatgc cgcagagatt tgatttaact    1320
   tatgtgggat cagacggtga aaaacacaga ccgatcatga tccaccgcgt catctttggt    1380
   tcgattgaac gttttatcgg tattctgatc gagcactgcg ccggcaaatt cccgctgtgg    1440
   ctggcacctg tacaggtaaa acttctgacg gtcaccgaaa aattcgcacc gtacgcacag    1500
   gaagtcgccg cgaagctgga agaagcgggt ctgcgtgtgg aaacggatat cagaaacgaa    1560
   aagatcggct ataagctcag agaggccaga aacgagaggg ac 1602
<210> 125
   <211> 1098
   <212> DNA
   <213> Unknown
<220>
   <223> Unknown gut bacteria
<400> 125
   atgttagaca tcaaacgaat cagagaagac tacgaaggcg tcaaggccgg cgtagagaga 60
   agaaccaagg gcagcttcgg catcgagaag attccggcac tggacgaaaa aagacgcgcg 120
   gttctggcgg aagtggaagc gatgaaaaac aagcagaaca ccgtatccaa ggaaattcca 180
   aagctgaaaa aggcggggga ggataccacc gcgatcatgg cggagatgcg ggaactgtcc 240
   gacgaaatca aggtgctcga tgcacaggtt tcgcagctgg acgcggagat taaggatacc 300
   ctcctgaaca tcccgaacgt gcctgcggac ttcgtgcagg tcggcgagga cgacagcgcg 360
   aacgttgaga ttcgcaaatt caaagaaccg accaagtttg acttcgaacc gaaggcgcac 420
   tgggacatcg gtacggatct ggatattctg gactttgaaa gagccgcgaa gatctccggc 480
   actagattta ccgtttataa gggactgggc gcaagactgg aacgtgcgat tatgtgcttc 540
   atgctcgacc tgcatacgct ggatcaggac tacaccgaga tcctgccgcc gttcatggtc 600
   aaccgtgcgg cgatgaccgg caccggtcag ctaccgaagt ttgaagagga catgttctat 660
   gtgccgcaga aggacttttt cctgatcccg accgctgagg ttccggtcac caatctgctg 720
   gcgcaggaga tcgtaccgga ggcagacctg ccgatccact atacggcata tacgccatgc 780
   ttccgcgcgg aggcgggctc cgcgggccgc gataccagag gcctgatccg ccagcatcag 840
   tttaacaagg tagagctcgt gaaattctgc aagccggagg attcctggga cgagctggaa 900
   gcgttgacca aggacgcgga agcggtgctg cagattctcg atatcccgta cagagtggtc 960
   agactgtcca ccggcgacct tggattctcc tcggcatgca cctacgacat cgaggtttgg 1020
   atgccgtcct acggcagata cgtggagatt tcctcctgct ccaacttcct ggattatcag 1080
   gcaagacgcg ccaacatc 1098
<210> 126
   <211> 1269
   <212> DNA
   <213> Unknown
<220>
   <223> Unknown gut bacteria
<400> 126
   gaaaacattc tgcttaaccg cgagccgaag aaatccaacg tggtctccga aatcttcggc 60
   gaccgcctca atacgcttga ctacaagcag atgaacgaac gctcggcaga agccatcaag 120
   aagatgggcg ttcatatcga ccagcatatg gtcatcagcg acatgccggt cggtcacaag 180
   cagtttacgg aaattgcgcg agagctcagc aaggacaacc tcaagctgct cattctcgac 240
   gagccgaccg cggttctgac cgagaaggaa gcggcggcgc tgctgaactc catccgctcg 300
   atggcagcgc gcggcatctc cgtgattttc atcacccacc gtctgcagga gatcctgtcc 360
   gtgtgcgaca aggtcgtcat catgcgcgac ggcgtggtgg tcaaagatac ccccgcaagt 420
   gaaattgatg ttcccgagat tacgcgctgc atggtcggca gatcggtgga tctgtcgtct 480
   gaaaagcgcg aaatcgtcga tcattcgaat gcaaaaacca ttctatccat ccgcaatctc 540
   tgggtcgata tgccgggcga gaccgtgcgc aacgtgaccc tggatgtgaa ggcgggcgag 600
   attctcggca tctgcggtct tgccggtcag ggaaagctcg gcatccccaa cggcgtaatg 660
   ggtctttatg aagcaggcgg caccgtcgag tttgacggca aaccgattcc gctcaacagc 720
   ccgagaaagt gcctcgacgc atcgcttgcc ttcgtctccg aggaccggcg cggcgtaggt 780
   ctgctgctcg atgaaacgct ggagtggaat gttgcgttcc ccgccatgca gattcagaac 840
   aaattcctca aaaaaatcgg accgatcacc tggcgtgatg aaaaggcaat ccatgatata 900
   acccagaagt acatcaacga gctgatgatc aagtgcacca gttccctgca gaaggcaaga 960
   gagctttccg gcggcaacca gcagaagatc tgcctggcaa aggcgttcgc gctggagccc 1020
   cggttcctgt tcgtttccga gccgacccgc ggcatcgacg tcggcgcgaa ggcgctggtg 1080
   ctcgacgcgc tgcggcgctt caaccgcgag tccggcgtca ccgttgtgat gatctcctcg 1140
   gagctggaag aactcagaca gacctgcgac aggatcgcca tcgtttcggg cggacgcatt 1200
   gccggcattt tgcccgcaac cgattcctcc gaggcctttg gtgctttgat ggtcagtcag 1260
   gtgaaataa 1269
<210> 127
   <211> 1206
   <212> DNA
   <213> Unknown
<220>
   <223> Unknown gut bacteria
<400> 127
   atggaaggat gtgtgagtgc tttggctgga ctgtccgcag taatctttat tcctgatgat 60
   acgcccaaaa ccggtttttc ccgcccaatg atgctgcaaa atatcatggg tacgcctttg 120
   ctatcctggc tggcgtcctc tttgattgcc ggcggcgttg ggcggttctt tttggtctgc 180
   cacgagcggt tcaagcgcga ggcgcgcgca tgcttcccgg acgatgtgga gttcagctgc 240
   ccgtcggtcg aggcaacgag cgaccagctg cacgtgtttt tatccacagc ggacgagtcg 300
   gaagaggaca tcattgtggt gacgggtcct gcggtgattc tgccgtttgc ggcggaagaa 360
   gagcagttcg attccgcgcc gattgtcagc cccgtgacct ccgtctccaa gcaggcgctg 420
   atggcggcgc tggacgaaaa attcatcttc accgcgtttt tgaaggacca cggcgtgccg 480
   tataccgacc gcgacggcgt gtacggtgtg gcagacctgc aggagatgac cagctggcag 540
   ccggttctct cgcgggcaaa gctgtatgag ctttcgaagc agggcgtcga gatctgggac 600
   tacaacacga cgtatgtgga cccggcggct tccgtcggac cggggacggc gctgctgccc 660
   ggaacgattc tgcgcgggca gacctcgatc ggcaaaaact gcacaatcgg accgaacagc 720
   tatctggaaa atgcacgcat cggcgacggc acgaaggtca acgcctcgca gatctataat 780
   tcctccgtcg gctacgacac gcacgtcggt ccctttgcgt atgtccgccc gggcagcacc 840
   gtcggaaacg cggtgcgcgt gggagacttc gtggagatca agaactctgc catcggcgac 900
   ggcacgaaaa tctcgcatct gacctatgtg ggagacagcg acgtcggcaa gaacatcaac 960
   ttcggctgcg gcacggtgac ggtcaactac gaccgctcaa aaaaataccg caccgtcatt 1020
   gaagacaacg ccttcatcgg ctgcaacacc aatctcatcg cgcccgtgcg cgtgggagag 1080
   ggcgcgtata ttgctgcggg gtctacgatc accgacgata ttcccgcgat ggcgctttcc 1140
   attgcgcgcg cccggcagca gaacaagaag gactgggcaa gcaaacacaa gctcaaggaa 1200
   aaataa 1206
<210> 128
   <211> 1083
   <212> DNA
   <213> Unknown
<220>
   <223> Unknown gut bacteria
<400> 128
   atgggaggac ggaacatgga actgacagaa cgaaaaaagc agattctgcg ggcaatcgta 60
   gacagctaca tccgcacggc agagccggtc ggctccaaaa cgatctccca gctgccgggc 120
   atggactttt cgccggcgac catccgcaac gagatggcag acctgaccag catggggctt 180
   cttgagcagc cgcacacgtc ggcggggcgc gtgcccagcg cggcgggcta ccggctgtat 240
   gttgacgagc tcatgcagaa ctaccgcctg tcgatggacg aaacgaagac catcaatcag 300
   gcgatggagg tcaagatgca ggaggtcgac aagatgatct cccaggtcgg aaagctggtc 360
   tccaaaatga ccgatcttcc cgcctacgcc gtcgcggcca gaagctcgca gcggaccgtc 420
   aagcgctttg atctcattct ggcagaagca ggaagtttta ttctggttgt catgttaagc 480
   gacaatcagg tgcagaataa gctcatccgc ctgccgctgg atgtgtcgca ggaggatttg 540
   cggcttctgt ccgcggttct caacgcgagc ctcaccgagc tgacggcgga cgaaatcacg 600
   ccggagcttc tggcaaaggt cacgcgcagc gcagccggtg cggcaagcct ggtgccggtg 660
   attgtggatt acacggtgga actcctgaaa aagacccaca gcgaggttta catgaccggt 720
   caggcaaagc tgctcggtca gccggagtat cacgacgtgg aaaaggcgca ggaggttttg 780
   acaagcctcg atgaagacgt catttccaat cttccggcaa cgctttcgtc cggcacgacg 840
   cagattctgg tcggaccgga gaatgtggca aaggaactga aagattccag cgtcgtcatt 900
   acaaagttcg atatcggcga cggcatgcag ggcatgatcg gcgtcgtagg tccgacgaga 960
   atggattacg cgaagatcac tgcgcgcctg agctattttg ccgagaatct cggcaggatg 1020
   ttcgccaagc cccagcagcc ggcactgaac gagcctgagc ttcccgaaga accgaaaata 1080
   taa 1083
<210> 129
   <211> 699
   <212> DNA
   <213> Unknown
<220>
   <223> Unknown gut bacteria
<400> 129
   atgtattgtg tcaattgcgg tgtggagctt gccgacagcg aaacggtctg tccgctttgc 60
   gggacgcggg tgttccaccc cgatcttcca agaaaacagg cgccttctcc ctatccggcg 120
   gacccgcacg gaaagccgga ggaggtcaac cggtcgggaa ttctgtttgt gatgacgatg 180
   ctggcgcttt tgccggcggt catctgcctg ctgtgcgact ggcgcatcaa cggcgggatt 240
   gtctggtccg gctacgccgc gggcgcaatt gggcttttat atgtgctgat tgtgctgccg 300
   atgtggttca gtcaccccaa tccggtcgtc tttgtgccgg tggattttgc ggcaatcggg 360
   ctgtatcttc tgtatatcga tctggtgacg aagggcggct ggtttttgtc gtttgcgttt 420
   ccggtcacgg gggcaatcgg gctgctgtta acggcggtgg tggcgctggt gcgctatgtc 480
   catgcggggt atctgtatat cttcggcggc gcgctgctgc tctcgggcgg tcttgcggtt 540
   ctcattgagt tcctgatcaa tgtgaccttc ggcatccata aggcgctttt gtggtcgatc 600
   tacccgctgg cagcgggcgt gattctgggc ggcatgctgc tcgtcatcgc gatctgcaag 660
   cctctgagaa aatcgctgca ccgaaaattc ttcttttaa 699
<210> 130
   <211> 846
   <212> DNA
   <213> Unknown
<220>
   <223> Unknown gut bacteria
<400> 130
   cgcaaggacg gtgtgctgct gcgcaacctg gacggcatgc acttcattac gccgatcatc 60
   tatcccaatc gctgcgacaa tgaagcctac atctccgaaa ccatcgatct gaccaacatg 120
   aatgcgtatc tgcagaagaa gaatgcgcag agcgccgact tcccctatac gatgttccac      180
   atcatcgtcg ccgcgctgat caaaaccatc acgctgcgtc cgaagatgaa ccgcttcatc      240
   gccaacaaga atttctatca gagaaacggc gtgtcggcgt cgttcgtcgt caagaagcag      300
   ttcgcagacg aagcggcgga ggcgcttgcc gtggtgcgtg gaaaagatga atcgacgctt      360
   gataccatcc acgaggacct gcgccaccag atcatggact gcaaggacat gacgaagacc      420
   gatacctcga cggacttcat ggacatcttc aaccgcatgc cgcgctggct gggtaaattc      480
   atcgtctgga ttctcacgcg gctcgacgtt cacggctgga ttcctgcgtc gatcattgag      540
   acagacccct actactgcac agtcgtcatt tcgaacctcg gctcgatcaa gctgcacagc      600
   ggctaccatc acctgacaaa ctggggcacc tgctcggtgt tctgcatcat cggtgaaaaa      660
   tcgctgcgcc cggttttcca gcccgacggc agctacgaga tgcacgagat gctggatctg      720
   ggactcacga tcgacgagcg ccttgccgac ggctactatt actccaagac aatcaagctg      780
   ctgagaaagc ttctggaaaa ccccgaactg ctggagacgc cggcaaatca ggaaatcgaa      840
   tactga                                                                 846
<210> 131
   <211> 1203
   <212> DNA
   <213> Unknown
<220>
   <223> Unknown gut bacteria
<400> 131
   atgaaaagaa gaattcttgc gcttctgctt gtgtttgcaa tgttcgccgc gctcatgacc 60
   ggctgcgcat ccggcaagca ggagacggct gcgaacgaca ccgcgcagac cgaacagact 120
   gcgcccgagc agacggcgga atctgccgca gaggaaaccg aagctgcgga agagactgcc 180
   gaaccggcgg aggaaaccgc cgcagaaccg gaaacgcttg ccttcaccga cagcctcgaa 240
   cgagaagtcg agctgcccag ggacatcacc cgcatcgcgc ccagcggcgc cgttgcaacg 300
   atgatcctcg cggcaattgc cccggagtgc atggtcaccg tcaacgcgac acccagcgag 360
   agccagatgg catttttgcc tgcgaacctc gcgtcgctgc ccgagaccgg tcagatgtac 420
   ggcagcaagg caaatctgaa cctcgagacg ctgctcgctg ccgacccgca ggtcgtcatt 480
   gacctcggcg acaagaaggg cgacatgacc gaggacctca acgcgctgca ggatcagatc 540
   ggcatccccg ttatcttcat cgaagcagat cttgcgcata tggcagaggc gttccgcatg 600
   ctcggaaacc ttctgtcggg caagactgac cgcgggcagg agcttgccga cctcgtcgac 660
   cgcacgacca caatggcagc tgagaattcc gcaaagatca ccgatgacat gcgtctgcgc 720
   gccatgtaca ccaccggcga ggacggactc ggcaccaacg ccgcaggctc cattcaggcg 780
   caggttctgg acatggtcgg cgtggagaac gcagttgttg ttgaggacgt cagcaataag 840
   ggcggcggca atgtcatcag cctcgaacag ctgtataact ttgatcccga cgtcattctc 900
   tttgccgacg gcagcatcta tgacaccgtg accgacgatt ccgcatggtc tcagctgacc 960
   gcgatttcga ccggaaaata ctatgaagtg cccggctccc cctacagctg gctgtccggt 1020
   cctccgtcga tgaacatgat cctcggcgtg tggtggctcg gcaacctgat ctatccggaa 1080
   atctatgact acgacatggc ggaaatgacg caggagtttt acaagactct ctggggctat 1140
   gagctctcga gcgacgaagt gactgcactt ctggcgaatt ccaccctcaa ggcagctcaa 1200
   taa 1203
<210> 132
   <211> 1833
   <212> DNA
   <213> Unknown
<220>
   <223> Unknown gut bacteria
<400> 132
   ttggcagaac tcaagcttat ctcgccgctg ctttccaata tggaagtagt cgagtgtgtg 60
   agcgttcgcg gcgcagccgc agtatatatc gtcagaagca ccaaaagcgg gcagacctac 120
   gttctcaagc acatctccgt gccggagtcg cagaagcagg tcgacgcgct gatgttcacc 180
   ggagccgccg ccacaaagga agacgcgcaa aaatattatg agcaggttgt ttccgattat 240
   cagacggagc tgacaacgct tgaaacactc tcggcaagcc cgaatctcga ttgcttccgc 300
   agctatcaga tcgcgcccaa ggaagacggc gtcggctacg acatgtatct gcttgccgag 360
   catcgcaaga cgctcgtcga ttacttatcc gacaacgcca tgacgaagct ctgcgcggtc 420
   aatctggcga tggacctttg cagcgccctc atcgatctgc gtgcagcggg gctcattcac 480
   cgcgacgtca agccgtccaa catctacttg aacgcacagg ggcatttcct gctcggagac 540
   ctcggcatcg cgaaaatcga cgatctcaaa tactgcacga tgcccgagag catgctcagc 600
   tcctactccg cgccggagct gttcagtctg ctcggcacga tcgagccaac gacggatatt 660
   tattccgtgg gcttgattct ataccggatt ttcaacggca accacgcgcc ctttgaggac 720
   gaaaacacgt ccgcgaaggc agcggatcgc cgcagagtca ccggcgaggc gctcccggtt 780
   ccgatgtacg cggactacga gatttccgaa atcattctca aggcgtgtgc cttccagcct 840
   gccgaccgct accagagccc gcaggaactg aaggacgcac tggtcgagta catgaagcgc 900
   aaccaggtcg acgacacgct gattgttccc ccgatcgcgg gtgagcacga gccggtcgat 960
   ctgacgcagg aggaagaaat tgagccggtg cagtttgcag acaccgattc gctgcccgag 1020
   gatttcaagg agaacttctc gccggacacg cagatgctca actcgctcat cgactcggtc 1080
   cataaagaag cgcagaagga cccggagggc tatggcatct ccgggtctct ggagccggat 1140
   gaagaagacg acgcaccgct ggatgattcc ggcgcgccca aaaagcgccg caaaaagagc 1200
   cgctggcttc ccatcgcgct gtgctgcctg ttgctgattg ccggcatcgg cgcggcggtc 1260
   tacttcttca tcattgcgcc cagcaatctg cacatcaacg agatcaccct tgtggacacc 1320
   ggcacggact atctgaccgt gcaggtcgat tccggcgaaa aggaaggctc gttccaggtt 1380
   gtctgctccg acacctacgg aaacaagttc caccagagct tccaggcaaa gacagacctt 1440
   accttctccg gtcttgtccc cggcacacag tacaccgtga ccgtcgagcc gcagaacgga 1500
   gaaaccccga ccggcaatta cacgctcatg gcaacgacgc tcgcgcagac ggagattctg 1560
   agcttcacgg caacgcctgt ctccgtgacg caggcggagc tgaacctcat cattttggat 1620
   ggacccgacc ccggcagctg gaccgtcagc tatgcggcag agggacagga agcgcagacc 1680
   gcgaccttct ccggtcacag caccgtcatt gcaggtctcg cctccggcac cgagtatacc 1740
   tttaccctgc aggagcctga gaacacgaag ctcatcgggc agacgagcgc aacgttctct 1800
   acggttccga ccgtcagcat cgtcccgccg ctg 1833
<210> 133
   <211> 1167
   <212> DNA
   <213> Unknown
<220>
   <223> Unknown gut bacteria
<400> 133
   ggtctgccga cggcaattac gtttacgctg gcgggcttcg aggtctgcgg gtttgatgtg      60
   aagcaggagg tcgtcgacca gctgagcagc ggaaaaatcc acattgtaga gccgggcttg     120
   caggatgcgc tgacgcaggc gcttgccacg ggtcggctgc actttacgac gaagccggag     180
   ccggcagatg tgtttatcat cgcggtgcag acgccgtata aggaaaccga ggatcacaag     240
   cgggtgtctg acatgcgctt tgtcgagtcg gcggcgcgcg aggtcggttc ggttgcgaag     300
   ccgggagatc tttgcgtgct ggagtcgacg tcgcctcctt attccacgcg gctggtggag     360
   tcgattgtgg cagaggtcag cggcatgaac aggagcgatt ttatgacggc gcactgccca     420
   gagcggatta tccccggcag aatgcttctg gagctgcgtg aaaatgaccg aattatcggc     480
   tcgaatcgcc cggaggctgc cgagtacgcg aagtcgatct atgagaaggt tgtgaccaag     540
   ggcacaatcc ggctgacgga cgacttaacg gcagagatgt gcaagctgac agaaaacacg     600
   ttccgcgaca tcaacatcgc ctacgccaac gagctgagca aggtctgcga ccggctgggc     660
   atcgatgtgt ttaatctgat tgagctggca aactgccatc cgagagtcaa cgtgcatacg     720
   ccgggcgtcg gcgtgggcgg gcactgtatc gcggtcgacc cgtggttcat ccatgaaaaa     780
   tttgaggaca ttacgccgct catttacgag gcgcggacag tcaacgacgg aaagccggcg     840
   tgggtcgccg agcgcatcgg gcgcgacgtc aagcccgggg caaaggttgc ggtgcttggc     900
   atgagcttca aggcgaacat cgacgacacg cgcgagagcc cgtcggtgct gtttgccaat     960
   atcctcaagg agaagggtta cgaggtgatt gcctgcgaac cgtttatccg gggtgatgtg     1020
   gcaggataca aaaatcatac gctggaagag gcaattgccg cgtgcgacta tgcggtcatt    1080
   acgctgatgc acacgccgtt tgtggaaaat aaggcgctga ttgcgacgaa gccgtattac    1140
   gactgcgtcg gtctgatgcg caaataa                                        1167
<210> 134
   <211> 1821
   <212> DNA
   <213> Unknown
<220>
   <223> Unknown gut bacteria
<400> 134
   atgaagaatt tcaccatgaa gcgggtactt gccggcgtcc tttgcgccgc gggcgtgatc 60
   gcctccgcgc ttaccacagc ctctgcctgc accacgatct atgcgggcgc aaatctcaca 120
   caagaaaaca cgcgctttat cgcccgcagt gaggactacg gctcggacat gaacaagctc 180
   tggttcgtca gcgaatccgg cgcttacagc ggcacttacc gcggctgccc ggaatacgga 240
   ccgtttacct atgagctgtc ccaccccagc taccgcttca cgtacttcaa aaacgacaac 300
   gtctacgacg gcgtctgccc cgagtgcggc gaggaaaatg cgcagcatgc ctcctatacc 360
   gagttcggta caaatgaaaa gggcgtgtcc gtctccgcca cggaaacgat ctccggaaac 420
   gacgccgttc tgtccgtcga cccctaccgc aacgccggtt gggcgcagga aaacggcaaa 480
   cccgccggca ttgaagaggg cgacattccc acggtcctgc tcagcgaggc agcgtcggca 540
   aaagacgcgc tcaagctgct tctgaacatc tacgacacct atggctgcgc cgacggtgcg 600
   gggcttttca tcaccgatca gaacgagagt tggtacattg aaaactgctc cggcacgcag 660
   tatgcggcaa tccggctgaa cgatgatctg ctgttcctgg agcccaacat ggcggtcatc 720
   ggaagcgtcg atctggacga taccgaaaac gtgattgcct cgccgaagct catcgagacg 780
   gcaaaggcag ccggaacgtt ccaaggcagc gagattgaca acgtgattga ctaccgcgcc 840
   agctacgccg gaagactcga ctctgccgac aagcgtctgg tcgaaggtct caactatctc 900
   aacagcgcct accgctacga cgcggacgcc ttagtcaccg acaacagccg ctttacgatt 960
   tccaatcttg acaagaccgg cgcgattgtc ccgctctaca cgaacatctc cgccgaccgg 1020
   acgctggatg tcgacgatat tctgaactac tacaagctca gcaccatcgc aaagtccggc 1080
   aatcaggaga ttgaaatctt ccagatgttc aaagaccgcc ctgccgaata cgcaacggtc 1140
   ggctgggtcg cagtcggcga tctggcgtgc aatgtgtttg tgccgtatta tccgatgctg 1200
   atcgattcca tgtatgaggg ctaccaggca ggcacgccga ccgtgcagtt taccaccgaa 1260
   cagccgaccg gcggtctgtt ctacccctat tccaagcgct cttacaaccg cgaaaccggc 1320
   gagatcacca cgaccgacgg ctaccggatt ctgcccgagg gctgggagaa atcctactac 1380
   tggagctttg aaattctcaa cgactacgtg cgctactttg aatctgccga cggctcgaag 1440
   ctcgtcagcg acgcggaccg gcagtatgtc aaggcgaagc tgaacgactt gcagcaggaa 1500
   ttctacagcg atttcgtttc catgcagacg ctgcagtcgt cttcgaacgc ccgcaagctt 1560
   gccaccgaga acggcgcggc aatggcaaag aaggcacagc agaccgcgca ggagctgatt 1620
   gcctatctgc agggctccga ggcgctcacg cgcgccgacg cagtctacag tctctggctg 1680
   cgctccggct ctccgaaggc gaagacctcc gcgtgcccgt ttgcggatgt ttcggaaagc 1740
   gcttactact atgacgccgt cctttgggca gccgaagccg gcatcgtccg cggtgtcagc 1800
   gacagcagct ttgccccgga c 1821
<210> 135
   <211> 738
   <212> DNA
   <213> Unknown
<220>
   <223> Unknown gut bacteria
<400> 135
   atggacgaga ttctcagact cgagcatctg gaaaagacgt tcggcacgca tcaggtgctg       60
   cgcgacatca gcttttcggt caataagggc gacgtcatca gcatcatcgg ctcgtccggc      120
   tccggcaaaa gcacaatgct ccgctgcgtg aacctgctcg aagagccaac cggcggcaag      180
   atcatctttg agggcaagga catttccggg aaagagctga acctgtctca ataccgcgcg      240
   cgcgtgggca tggtgttcca gcagttcaac ctcttcaaca acatgaacgc actcgagaac      300
   tgcgtctgcc cgcagctgac ggtgctgcac cgcaagcgcg aagaagccga gaaaatcgca      360
   agagactact tagagcgcgt cggcatgtcc gcctactgca atgcaagacc tgcccagctc      420
   tccggcggtc agaagcagcg cgtggcaatt gccaggacgc tctcgatgaa ccccgacatc      480
   atcctcttcg acgagcccac aagtgcgctc gaccccgaga tggtcggaga agtgcttgac      540
   gtcatgcagc gccttgccaa agagggcttt acgatgctgg ttgtgacgca cgagatggcg      600
   tttgcgcgcg atgtgtcgag ccgcgtgatc ttcatggacg gcggcgtcat tctcgaagac      660
   gccgcgcccg aagagatttt caataacccc aggcagccca gaaccagaga gtttttgagc      720
   cgctacctcg caagataa                                                    738
<210> 136
   <211> 891
   <212> DNA
   <213> Unknown
<220>
   <223> Unknown gut bacteria
<400> 136
   atgaatacgc agcatttgca atatctgatt gaaatagaac gtacccgctc cgtttcccag 60
   gcggcggaaa acctgttcat cggtcagccg aacctcagcc gcattctgca cgagatggag 120
   gcaaatctcg gtttcaagat ttttgagaga accagcaagg gcgtgcgttc caccgagcgc 180
   ggcgcaattt ttctgcagca cgccaagagc atcatccggg aactcgaacg cattgacgcg 240
   ctcggagcgc gccacccggt ggaaaaccgg ctgcgcatct gcattccgcg cgcggcgttc 300
   attctcgatc tgacggcgga ctatctcaat acgctgccgg tcgaccagaa tctggactcg 360
   gtggtgcgcg aatgtcacgc acggcaggca atcgaaatgc tggaaaacgg tgaggttgag 420
   gtcggcatca tccgtttccg cagcgaatac cgcgactatt ttgaagagca gagcgtctcg 480
   cgcggcttcg gcttccagat cctcagccgc taccgctatc agctgctgct gcccaagacg 540
   catgcgctgg cggagaagca gctcattacc gggcaggatc tggcagggct tcccgagatt 600
   gtccacggcg acacgttccg caaggggccc aaagtagacg agggcttgcg gcggaagatc 660
   tatacggtcg accggctggc gcagctgcgc cttttggaga cggtgccggg cgcctacatg 720
   tggtgcgcgc cgattcccga ccggtgcctg accagatgga gccttgtgca gaagccgagc 780
   aacatcaacc agactgtcta ccatgacgcg ctggtttatc agctgcagta cgacatgacg 840
   cagattgaac gcgggtttgt cgagtttgtg cagcagcgct accagaagta a 891
<210> 137
   <211> 1029
   <212> DNA
   <213> Unknown
<220>
   <223> Unknown gut bacteria
<400> 137
   atgggttttg atcatgtaaa gctggaaaag ggcatgtacc gcgagagcgg caagacgttt 60
   acgcaggttc tcgaatcgct cgacccgagc gaaaactaca agggcacggc gctcgagggg 120
   acggacgcct tccagcgcca gcttaagcga tttgatatcc acgcaaaggg tgcgtactcg 180
   gacccggtgg aaaaattctt ccgcacgatg gactcgagcg tcctgttccc ggagtatatt 240
   gcgcgcgcgg tcaagcaggg catggaggaa aacaatgtgc tgccgaagat tacggcaagc 300
   acgacgacca tcgatgcgat ggattaccga agcgtttatt cggttccctc cgagcaggac 360
   aagatgctca tgcaggtatc cgagggcgca agcattccgg caacccaggt caagacgaag 420
   gcgaaccttg tcaagctcaa caagcgcggc agaatgctcg tcgcgtccta tgaggcaatt 480
   cgcttccaga agctggacct gttttccgtc acgctgcgcc agatcggcgc gtacatccag 540
   acaatgcatc tgaaagacgc ggtggaggtg ctggtaaagg gcgacggcaa caacaacgcg 600
   gcggcaagct ttaccatcgg agatagtccg ctttccggta cggcaggcac gctgacctat 660
   cagcagctgg tggaattctg ggcgcagttt gaaccgtatg agctcaactg cttcctgatg 720
   aataccggca caatggtcaa gatgctgggg ctgagcgagt tccagaatcc gctgacgggt 780
   ctgaacttcc agggcacggg cgcactgtcg acgccgctgg gcgcggagct tctgaaggca 840
   aactgcgtgg acgccggcaa gattatcggt ctggacagac gatacgctct ggaaatggtg 900
   caggcgggcg acgtgtcggt cgagtacgac aagctcattg accgtcagct cgagcgcgca 960
   gccatcacgt cgatttccgg ctttggcaag atcatgccgg aggcttcgaa ggtgctggtg 1020
   atcggatga 1029
<210> 138
   <211> 852
   <212> DNA
   <213> Unknown
<220>
   <223> Unknown gut bacteria
<400> 138
   atgctcaccg tgcttgccgc cgtgtttgcc tttttgttcc tgatgccgac ggtgctgacg 60
   atcacgaact cgttcatgac gcagagcgaa atcaccgcca actacgggca ggtgtttcag 120
   aacgcaaccg acggcaagag ctacatcaaa gacaaaatca atctgaaatt catcccggac 180
   aaagtaagct tcacgcagta ttttacggtg ctgctgaaaa gcccggactt cctctataaa 240
   ttctggaact cggtgattct ggtcgcgccg atcgtgctgg cgcagctggc ggtcgcgtct 300
   gtcgccgcct acggcttcac gcgatggcgc ggcaaaatcc gggacaccat cttctttgcc 360
   tacgtcattc tgatgctcat gccctatcag gtgacgctgg tgccaaacta tctggtctcg 420
   gattttctgg gactgctcaa cacccgctgg gcaatcattc tgccgggcgc gtttgcgccg 480
   ttttcggtgt ttctgctcac caagtccatg cgccggattc cggcttcgct gattgaatcg 540
   gcgaagctgg atggcgcggg cgagtggcac attttctgga acatctgcct tccccagtgc 600
   cgcagcgcac tgtattccag cgccattctg gtcttcatcg actactggaa catggtcgag 660
   cagccgctga ttctgctgcc cgatgcctcg cagcagccgc tttccgtgta tctgtcgcag 720
   atcaacgcgg gcgagatcgg cattgcattt gccgcggcgg tcatttacat ggtgccggga 780
   cttctgatgt tcctgcatgg cgaggcatat ctcgtggaag gcattactta cgcaggctcc 840
   gtgaaaggat ag 852
<210> 139
   <211> 1107
   <212> DNA
   <213> Unknown
<220>
   <223> Unknown gut bacteria
<400> 139
   ctcaactggc gcgaggcgct gcatttttcc acgcagattg ccaaggcact cgagcatgcg      60
   cattcgcgcg gcatcatcca ccgcgacatc aagccccaca atatgatgat tttgaaggac     120
   ggcagcgtca aggttgcaga cttcggcatt gcccgcatct cctcggcgca gaataccctc     180
   acgcgcgagg cactcggctc ggtccactat atttctcccg agcaggcgaa gggcggcaag     240
   gtcgactacc ggtcggacct ctattcgctc ggcgttgtga tgtatgaaat gctcacggga     300
   agaccgccct tcgacggcga tacgcccgtt tccgttgcca ttcagcacat caacgccaag     360
   cccgtcatgc cgcgcgaatt gaaccctgat attcccgagg gcttcgagca gatcaccatg     420
   cacgcgatgg cagcggatat ttccaagcgc tacccctcgg cgacgcggat gctggcggat     480
   ctggaaattt tccgcaaaaa cccgaatgtg attttcgatt ttaccgacga tcaggacgcc     540
   ttcgatgtgg agcgcctgct caacgacccc gactatcttc cagaccggct tccccggcgc     600
   gagcgcaagc agccgtcccg ggtgtctatt ccacagcgca agcagccgcc tgcgccgccc     660
   gcgcagagcc agagcaccaa aaagggcgca aacgttgcgc tgacggcagg catcgtgtgc     720
   atcgtgctgg cggtcctcgg catcggctac ttcctgtatt cgtatttctt ctcggacctg     780
   ttcagccgga ccgaggaggt cgaggttccg cagatggtcg gacagtatta cgagaacttc     840
   gaccagagcc agtaccccga tctgaccttc gaagtcacgg actggcgcgc gagcgacgct     900
   tacgagagcg gctatgtgat cgatcagagc gcgaacgcag gcacgatggt caaggttggc     960
   tccaaaatcg agctgacgct ttcgaccggc attgaaagca atgtcatgcc gaacgttgtg    1020
   aacctgccgc tggaaaacgc gcaggactat ctgaattccc ttcccatcac gctttccatc    1080
   tcggtggatt atcaggaaag cgatgtg                                        1107
<210> 140
   <211> 1056
   <212> DNA
   <213> Unknown
<220>
   <223> Unknown gut bacteria
<400> 140
   tcgcaattca aaacgccgtt cggaacgctc ctgcccggac agggctgtcg gctgcgggtg      60
   gacattccgg tctcgtgccg gaccgtgaag gcggaggtta ttttcctgca ggacgacgct     120
   ttgaccgagg cgttccgcgc gccgctggca aaggcagagg cgaatgcgct ctacgaggcg     180
   tggagcgggg gattttcgtt tgaaactgcg ggcctttatt tctactattt tttcatcaca     240
   acgcagaacg aggcgttccg gctttataaa cagggaaacg atacgaatat ggaggcaggc     300
   gacctctggc aggtcagctg cgtgcaagac cgcttccccg cgccggagta tgcaaagggc     360
   gcggtcatgt atcagatttt cccggaccgg ttttacgccg cggggcgctg cgactgcacg     420
   gagaaattac agccgtactg ggtgcatgag gacaagcagg acgtgccggt gtatctgccg     480
   gacgagaacg gaaaggtctg gaacaacgac ttttacggcg gaaatctcgc cggaatccgg     540
   gaaaagcttt cgtatctgca ggagctgggc gtggagcttt tgtatctgaa cccgattttc     600
   ttcgcgtttt ccacacaccg gtacgacacc tgcgactacg gacgcatcga cccgatgctg     660
   gggaccgagg aagatttccg ggctctgtgc gaggacgcgc atgcgcgggg gatgaaggtg     720
   attctcgacg gcgtgttttc ccatgtggga agcaggagcc ggtatttcca ggacgcggtt     780
   cccaaccctg accccaaata ccgcagctgg ttcgacttca agcactatcc ggacacctac     840
   accagttggt gggggattac ggatttgccg tgcatccgga aggacaacga ggactttttg     900
   cgctatatca tagacgacga ggattccatc gccgtgcact ggctgcgcgc gggcgcggac     960
   ggctggcggc tggacgtggt cgacgagctg ccggaccgtt ttgtgctgcg gctgcgcgag    1020
   cggattcgaa aggaaaagcc ggacgcgctg ctgatc                              1056
<210> 141
   <211> 753
   <212> DNA
   <213> Unknown
<220>
   <223> Unknown gut bacteria
<400> 141
   atgaaacaga caatcaagcg ggtgctggcg gctttgctgg cagcgggact gctggttctg      60
   tgcgcgtgca gcgcgaagac ggcgcctgcg caggagcagg agccggaaca gacggctgcg     120
   gtggagactg ctccggcaga gaagccgcag gaagctgcgt cggtggagga aacgccggag     180
   aaagcggaag gcgaagcgca aacgcccgaa gagcagccgg aggcggaaaa acctgcgccg     240
   gaggaacctg cggagcagcc gaccgagccg gaggtgtgcg cgccgatgga cgagcagccg     300
   atgcaggagc agcctgatgc acaggacacg ccgatgacgt gtacgatcag catcagctgc     360
   gcgacgatcc ttagcaacac ggacgagctg gacccggaga agctggagct ggtgccggag     420
   gacgggtgga ttctcgcgcc ggtgacggtg gagtttaccg cggggcagag cgtgtttgac     480
   gtgctgctgg cgacggtgaa agcgcaggcg attcatatgg agtatcagaa cacgccgatg     540
   tatgagacgg cgtatatcga gggcatcgga aatctgtatg agtttgactg cggcgcgctc     600
   tcgggctgga tgtaccgggt gaacggatgg ttccccaatt acgggtgcag ccggtatgcc     660
   gtgcaggcag gcgacgtgat cgagtggatg tatacctgcg atctgggaag cgacgtcggc      720
   ggcagcggcg tgagccaggg gtatgtcgag taa                                  753
<210> 142
   <211> 1218
   <212> DNA
   <213> Unknown
<220>
   <223> Unknown gut bacteria
<400> 142
   tacggcgaca tgatgagcta tatcatcgac tccaacacgc cgctcaatga gcagtactat        60
   gacgaagaaa acggcattac ctgggcagac accctgattg agcagggtct gaccagcgcg       120
   gcgcgcatct acgccatcta cgacgaagca gttgcaaacg gcgagacgct ttccgacgac       180
   gatcagtcca gcatcgacgc cactgtctcc aacctcagcg tctacgccac ctactacggc       240
   tactccaacg tcaacggcta ccttgccgcc atgtacggcg caggctgcaa cgagaagaac       300
   ttccgcgagt tcctctccat tgaaaccctt gccaacaact actcgcagaa ggtctacgac       360
   acgcccgtct acaccgaaga cgagcttgca gcggactatg ccgccgaccc caacgcctat       420
   gacaccgttg actaccgcgt gttcgccgtg accgacgcgc tgtttgacga ggaagacgag       480
   gaaaagctgg ctgatctcaa gaagaccgct gccgacgagc tgcttgccgc tgccgaggaa       540
   ggcgacgagg cgttcatcca ggctgcttac gaccacgcct ccgaagacac caaggaaaac       600
   tacgaagacg acagctacac cctgcgcacc gacgtcggtc attccgactc cgatctttcc       660
   aactggctgt ttgaatccga ccgcaaaacc ggcgacgcca cctgcgtcga gaccagcggc       720
   acctactatg tcgcgatttt tgagagccgc gacacccacg actacgcgct tccgaatgcc       780
   cgccacatcc tgatctccgt ctcggatacc actgacgaag aggcaatggc gactgcgaag       840
   gaaaccgcag agaagctgct tgccgagtat gaggcgggcg aacacaccga agacgccttc       900
   gctgccatcg gcgacgcccc ctacgcagac ggcactgccg cagagtctgc ccgctatgag       960
   gacatcacgc cgggtcagat ggtcgaagac tttgagaact ggtgcttcga cgccagccgc      1020
   caggtcggcg acaccggcat tgtggaaagc acctacggct accacgtcat gtacttctcc      1080
   ggcttcggca cgaactaccg catgaagctg gtggaagacg agcagcgcgg caacgtctac      1140
   accgactggc tcaccgccac taccgagaac gcaagctatg agaccgttgc ctccggcatg      1200
   cgctttgtga ccaagtaa                                                    1218
<210> 143
   <211> 819
   <212> DNA
   <213> Unknown
<220>
   <223> Unknown gut bacteria
<400> 143
   atgctacagg atattgcgcc gcatatttat cacaatcaaa tgtcttggaa ggagccggag        60
   gcggacgatt ttgtgctttg ctaccgcggg cggacgctct attgtaaggt ggaagacgga       120
   agcctcgtgc tgccgcgcgt gaaggacgtg gagccttccg cgctgcaata tgcgttttcg 180
   attgacgagc gggcggacta cctgctctcg gacgcggaac tgaaagaggc aaatggcttt 240
   tcgtacttcg acaccggcaa gctccgcacg ctcgtgccag gacctgcgct catggctgcg 300
   gcagcagggg aaagcttata tcgctggtac agtgggcagc ggttctgcgg caggtgcgga 360
   aagccgatgg aaaagagcaa gatcgagcgg gcaatggtct gccctgcgtg cggcaacacc 420
   gtctacccca aaatctgccc ggcggtcatc gttgcgattc acgacggcga ccggctggtg 480
   ctgacaaaat acaaggaccg cccggtcaag cactacgcgc tcgttgcggg cttcaatgaa 540
   atcggagagt ccatcgagga aacggtccac cgcgaggtgc tggaggagac ggggctgcgg 600
   gtcaggaacc tgcgctttta taaatcgcag ccttgggtct ttaccgatac gctgctcttt 660
   ggcttctttg ccgagttgga cggcagcgac aagattaccg tccaggagga cgagctgtcg 720
   gaagccggct ggttttaccg cagcgaaatt ccggaggacc ggacgcattt gagtctgacc 780
   ggtgagatga tggaacagtt caggaaagga cttgcttaa 819
<210> 144
   <211> 927
   <212> DNA
   <213> Unknown
<220>
   <223> Unknown gut bacteria
<400> 144
   cccgctgcgg gctggcagca aaaaacctgt tttcccctct tgacactccc actgtgtcag        60
   gctttacaat acctgtggat ccaaaaaatg aactgcatgg agggtaccgc cttgctgaaa       120
   atcggagatt tttccaagct gtccagaatc agcgtccgca tgctccggca ttacgacgag       180
   attgggctgc tgcgcccggt ctacgtcgac cggttcaccg actaccgcta ctatgacgag       240
   caccagctgc caaccgtcgg gcgcatcaca gcgctcaagg acatgggctt tcatctggcg       300
   gaaatcggac aattgctgga gctgtatgac gacaaggacg cactcgacgc gcatcttgcc       360
   gcgcagcagc agcagttgga gctgcttgcc gcagacaccg cgcagcgcct tcgtctgatt       420
   gcggcagcga ggaaccgctt acgaaaggac gaacctatga agtacaatgt cacactgaaa       480
   acgcttcccg aacgctactg cgccacggtg cgcatgacca ttccctgtta tgaggacgag       540
   ggcatggtat ggagcgttct gtgcagcgaa accgctgcga tggcgctggt ccctagcgac       600
   ccctgctact gcgcggtcga atttctcgac aacgaattca aggagcagaa cgtcgagatt       660
   atggcgtcca aatccgtaaa gggcacatat ccggataccg agcacgtgaa gttccggacg       720
   cttcccgccg tgacctacgc cggctgcacg ttcaagggca gctacgcgca gatcaacgac       780
   gttgtcgcca ctgtcagcgc gtgggtggag gacaacggct acgactacgc cggtcccatg       840
   ttcgatatct accacgtcag cccgcacgag acgcagaacc ccgacgagtt tgtcaccgag       900
   gtctgcttcc cggttaaaaa gaagtaa                                           927
<210> 145
   <211> 861
   <212> DNA
   <213> Unknown
<220>
   <223> Unknown gut bacteria
<400> 145
   aaggggcaca tcgactacat ctgccgcgag gcgatcaaca agtatggggc cgacccgatt 60
   ctgaccgtca aggtcgcctg ccaccacgcc gcgcgctact tcctgctcaa caaccgcggc 120
   gccatcgccc ccggctatct ggcggatttc gcgatcatcg acaatttccg gaattttaac 180
   gtcgaaatgg ttttcaaaaa gggcgagctg tattataatg acggtaagct caaggatttc 240
   cccgcccccg caatcgagga atatctcgac gagcgggcgc acgacacgtt ccatgtccgc 300
   catctgacga agtccgactt tgaagacgtg cgtcagcgcg gcgtcatcgg catgattccg 360
   ggagagattg tctccaccga caacggctat gcggaccatg tggacctgca gaaggacatt 420
   ctgaaaattg ccgtcgtcga gcgccacaaa aacaccgggc acatcggact cggctatatt 480
   cagggctatg ggctcaagtc cggcgcggtt gcaacgtcca tctcccacga ctctcataac 540
   atcatcgtcg tcggcacgaa cgcagaggat atggcatttg cggcaaacta catcgtcgaa 600
   aaccacggcg gcatcgtcgt cgtagacggc gggaaggtgc ttgccagcgt cgttctggaa 660
   atcgcgggca tcatgtccga cgaaccgctc accaagatga acgaggagct ggaggcggca 720
   aaggacgcgg cgttccgtct cggcgtcagc cgcgggattg acccgtttat gacgctcagc 780
   ttcatggcgc tgccggtcat tccgacgctc cgcatcacca cgcgtggcgt catcgacgtc 840
   aacacccagc agtacatctg a 861
<210> 146
   <211> 783
   <212> DNA
   <213> Unknown
<220>
   <223> Unknown gut bacteria
<400> 146
   ggcccctgcc gcatcacccc gaaggcgccg cgcggcatct gcggctgcga cgttcacggt 60
   atcgttgcgc gtaactacct gcgcttcacc gcgggcggct ctgcaacgca ctccgaccat 120
   ggccgtcaga tctgccacac cctgtatcag gcaaaagagg gcggtgcata tcaggtcaag 180
   gaccctgaaa agctgaagag aatcgcacac gaatgggaca tcgagaccga aggcaaggat 240
   atctacgatc tggcacatga agtcgctgag gtcggtctgc tggaatacgg caagcccttc 300
   ggcacacagc gctatctgaa gcgtgcgccg gagcatacgc aggagctgtg gcacaaggca 360
   ggcatcgagc cgcgcgccat cgaccgcgag gtttctcagt cgctgcatat gacccacatg 420
   ggctgctcgt ctcttcctga ggctctgatc aagcagtctc tgcgcgccgg tctgtccgat 480
   ggctggggcg gctccatgat gggcaccgag ttctccgaca tcctgttcgg cacaccgaag 540
   cccgttgaca ccaccgccaa catcggcgtt atggacaagg acatggtcaa catcatcgtc 600
   cacggtcatg atccctcttt gtccgagatg atcgttttct atgcgcagga tcccgagatg 660
   atcgcgcttg ccaaggaaaa cggcgcaaag ggcatcaaca tcgcgggcgt ctgctgcacc 720
   ggcaacgaag ttgccatgcg tcagggcatt ccgatggcgg gcaacttcct gcagcaggaa 780
   aac 783
<210> 147
   <211> 747
   <212> DNA
   <213> Unknown
<220>
   <223> Unknown gut bacteria
<400> 147
   acctcttctt ccatgagctc ggattccagc tggcgcttca ccggtcatct ggcaaacctc 60
   gagtactact acgagtccgt cgatgatccc accgcatggg agacctgccc ggctaccatc 120
   accggcaact acatgcagaa ctacaagaac ctgtgggatc tgtatatcaa caactccgca 180
   acgaaccccg cggacctggc tgccggcggc cttgacgcac aggctgagtt cgcagagggc 240
   aaggcagtgt tctattctca gggcaactgg gaatggtccg cactggctga gaagggcatg 300
   agcgctgacg acctgaccat gattccgtac tactgcggcg tggaaggcga agaaaaagcc 360
   ggtctgaact gcggcaccga gaactgctgg gctgtcaacg cggaagcttc cgaggaagac 420
   atccaggcaa ccctcgactt catgtactgg atggtcaccg atgctgatgc ttcgcgcatg 480
   ctcgtcgatt ccttcggcgt tatgccttat aagcaggcag ccgagtccac caacccgttc 540
   ctgaagaacg ccaatgagta cactgccgaa ggcaactatg tcatgccttg ggtcaccaac 600
   ttccagccga acgttgacgc ttaccgcgaa gcgctcgtcg ctgccatgaa ccagtacgac 660
   gccgatcaga ccgacgccaa ctgggagctg gtcaagaccg cgttcgtcga cggctgggca 720
   gtacagtatc aggcagccaa cggctga 747
<210> 148
   <211> 1056
   <212> DNA
   <213> Unknown
<220>
   <223> Unknown gut bacteria
<400> 148
   tcgctggaca atctggagca taattatcgg acattgcgcg agcatgtcgg aaagggaccg      60
   agattcctcg gcgttgtgaa ggcggactcc tacggtcacg gcgccgtgca ggtcagccgg     120
   acgctggaag agctgggcgc ggagtatctg gcggtctcca acctcgaaga ggcaattcag     180
   ctgcgccgcg gcggcgtggg acttccggtt ctgattctcg gctatacgcc ccctgagtac     240
   gcgcctgatg aagcagagat gtccatcaca caggaggtcc acggactgca gtatgcaaag     300
   gacctcaaca aggcgctcga aggtacggga cgcaagctga cggttcatct gaaaatcgac     360
   accggcatga cccgcctggg cttctttgcc gatcagccgg agcagacgct tgccgcgctg     420
   gtagaggttg cacagctgcc gaacctttac attgagggcg ttttcatgca cttctgcgtg     480
   tccgactcca aaaagccgga ggacgtggca tttacgaaac tgcagcatga gcgctttgtg     540
   aacatgctgg accttatgaa gtcgaagggc attgagccgg aaatccgcca ctgctgcaac     600
   tccggcgcaa cgattctgcg ccccgagtac gcgatggata tgatccgccc gggcgttgca     660
   acctacggct tccatccgtc gcccgacact gtgggcgaaa tcgacctgcg gccgatgatg     720
   agcctgcata cctccatcgc acagatccgc accatcgcgg caggaaaatc catcagctac     780
   ggcagaacct acacgacgcc ggaagacaag ctcgtcgcgg tcctcccgat cggctacgca     840
   gacggtctga gccgtcttct ttccaaccgc gtcaccttcc gcatctgcgg caaggaggcg     900
   cccgtcgtcg gcagaatctg catggatatg tgcatggtcg atatctctga cattcccgag     960
   gcaaaggtcg gcacaacggc gacgctcgtc ggctacgatg aggatggaac gcttctgccg    1020
   tgcgagcgca ttgcggacat gctcggcacg atcaac                              1056
<210> 149
   <211> 1173
   <212> DNA
   <213> Unknown
<220>
   <223> Unknown gut bacteria
<400> 149
<210> 150
   <211> 1350
   <212> DNA
   <213> Unknown
<220>
   <223> Unknown gut bacteria
<400> 150
   gtggcagaaa aaattccttc gcgccaggag cttgcgcggg aagatacatg ggcaattgag      60
   gatttatatg agacggacga ggcgtggctt tccgatctgg aaaagctcaa ggcgctcggc     120
   gcgcgcctgc cgggctatgc cgggaagatt tccgcagacg caaagacgct gctggaatac     180
   cagagactcg gagaagagat ctcgctgctt ctcgacagtc ttgcaaacta tgcgcagaga     240
   aagagcgacg aggacacgag aaaccccgtc tatcaggacc tgaccggcaa gctgaccaag     300
   gtctgggtac agctgggcgc ggacacggcg tttgaggtgc cggagctttt gtccatttcg     360
   gacgagcgga tggaggaatt ttaccgggaa gaaccggagc ttcagctcta ccggcggcat     420
   tttgacgtca ttcgccgtaa aaaagagcac attctctccg agagtgagga aaagcttctg     480
   gcgatggcag gcgagatggc aggcacgccg gaggagatat attccctgtt tgcagacgcc     540
   gatctgacct tccgcccggc gctcgaccgg gacggaaacg aacaccccat cacgcagggc     600
   agctatattg ccgcgatgga atcacccgac cggacgctgc ggaaaaatgc ctttgagagc     660
   ctgtatgcgg gcttcggagc gtataaaaac tcggttgccg cgatgctctg cgggcaggtc     720
   aagcagctgg aatttttctc gaaggcgcgc cggtatgcct cgcctctgga agcgtctttg     780
   gatgcaacgg aggttcccgt gggcgtgtat catgcgctga ttgaggcggt gcatcagaat     840
   cttgacaagc tgcgcaggta tatgcgtctg cgcaagaagc tgctcggtgt cgacgagctt     900
   cacatgtatg atctttatac gccgctgatt gaaacgtcgc agaagagaat tccttttgac     960
   gaggcgaaaa agaccgtagc gcaggcactc ggtgtgatgg gcgagcgcta cggcgcgatt    1020
   ctgcaggagg gattccagaa ccgctggatt gacgtctatg agaacaccgg caagcgctcg    1080
   ggcgcgtatt ctgcgggcgc gcgcgtgcat ccgtatgtgc tgctcaacta cacgggaacg    1140
   ctggacagcg agtttacact ggcacatgag atggggcacg cgattcattc gtacctgtcc    1200
   aacaagaccc agccggtcgt ttacgcggac tatgtgattt tcgtggcaga ggtcgcgtct    1260
   acctgcaacg aggcgcttct gatgcagcat ctgctctcga ccacaagcga gaaaaaagag    1320
   cgcgcctatc tcatcaatca cttcctcgag                                     1350
<210> 151
   <211> 795
   <212> DNA
   <213> Unknown
<220>
   <223> Unknown gut bacteria
<400> 151
   atgcgccgaa aagcacgcga tatcctgcgg gaccagcagg gcctttcgat gattctggtg      60
   ctctgcatcg gtgccttgtt tgtggccctg tctgcggcac tggtgtacgc ggcgtccgtg     120
   ttgacggcca acgccaaccg ccagcttttg gagcaggagg cctaccagct ggccacgagt     180
   ttttccgacg tgctggaagg ggaactgaac acccccacaa gctccttcgc cgcgttcgtg     240
   aatgacagct tcatgacctc cacggcctac ggcggcgata tctacggcag cccctcgcag     300
   ccctactcct acacatggaa agcggccgat gccgacacga gcggtgcgga ggaaatcacc     360
   gtcacgctgc gccgccgccc gggcgaggac gctgaaaagc tgaacctgag cggcgtgggc     420
   cagtccgtca gcaccaaaag cggctggcgc gaccaactga ccagctggca gagcaacgac     480
   tacctgctga ccgatctgca gctggacgtg accgtcaccg ccaccaaggc gggggagagc     540
   ttcgcctata ccgtgaccta tgaccggcag gtacactacc cggtgtctgg ctacacattt     600
   gacgatagta cgacggttta cacatggagc aagggcacaa acacctttgc cagccccggt     660
   ttgtcgagta tcaatattaa accggaggac gcagagcgaa tcattgtgcc ccactttgac     720
   accaccaacc cggataagtc caccatccac tacacccgcg gtgcccgcca gagcaccagc     780
   acagcacagg agtga                                                      795
<210> 152
   <211> 1689
   <212> DNA
   <213> Unknown
<220>
   <223> Unknown gut bacteria
<400> 152
   tcagtgcggc gcaggcggct gtggggcaaa aaagattcca ccgtttacac tgcgaatgtg        60
   gaaaacttcc tcacgaccca gaaaaccacc gccgccggga tgatgctggt ctgcgcggtg       120
   ttgttcctgc ctgcgctggc actgcgcccg gtgctggctc tgccgctgga tgctctgcag       180
   ccggtgacgg agcgcacccg ggcggcggcg ctgggggcgg ccatcgagtg gctgccaaaa       240
   atcagcggcg gggcgttgaa ctttcacgtc agcgcggcgg cgggcggcgt cgaggacggc       300
   agcctgaccc agagcggcgg cctggccctg caagggttgg aggatttact cgtcacgacc       360
   gacgagaagc cgacggagac ggtctacctg cgcggtttta tcggcgcgga atacgatggg       420
   catagctggc agcctgcgga cgccgagcag ttcgacaatg ccgccgccag ctggaaaacc       480
   gacggtgaca gccgtttggc aatcgcgaac ctgccgtttc tgcgggctgc ctacgatggt       540
   gccgcaccgc agcagatgac cgtgcagcgg ctgcacgccg gcgatgccta cacttttgca       600
   ccctataacg cctattttaa cgactactac cgcctggacg gcgacggcgc ggcagcaggc       660
   cagaccgcgc aggacgacac gttctactac ttcccgcgcg gggaggcaaa aacgctgctg       720
   accgcccgtg ccgacgcgga tcccagtgtg ctggaccggc tggaaagcgc ctacgcggcc       780
   tacgccgaga gccgctacct ggcgacgccc gacggcgcag actacggcac attgcaagaa       840
   ttttgcgatg cagcggaaaa agaacacaaa ctaaccggcg acgccgacgc gatacgaacc       900
   tttgtgcgca gctacctgaa cgagcactgc acctacacca gtgacccgcc ccagccagcc       960
   gaaggggccg acccggtgtt gtattttctg agcgagagcc atgcgggcag cagcacgcag      1020
   tttgcgtcgg cggcggtggt gctctgccgg atgctgggcc tgcccgcccg ctatgtggtg      1080
   ggctatgccg ccccccaaag cctgtttgcc gagagtgacg gcggctggca ggccgttttg      1140
   caggacgata acgcccacgc ctgggccgag gtgtaccttg ccgggcaggg ctggaccccg      1200
   ctggaaacga cccccggcat ggtcagcgaa ctgaccgagg accagctggc cgccaacgcg      1260
   aacgctgtgc cgactgccga tgatgccgag accccgctgc ccgccgtgac acccgcgccg      1320
   gaggtggacc cgaccgcccc gcaggcccga gctttttggt ggctgctgcc gctggcggcg      1380
   gtgctggccg cgctggccgt gtggggcatt gcccgtgcac ggcgcacccc ggccgatact      1440
   gtgcgggcgg agttccgcgc gctgtacaaa aagatgtgcc gctgcggcct gggcgaaggg      1500
   gtatcctctg atcaggaaga gtttaaacaa tttttatggg aacacaccaa aacggcggcc      1560
   ccgcaggatg tggaaacgct gctggcgctt gtgcagacgg cgcagtttgc gccggggccg      1620
   gtcagcgcgc aaaccgcgca acaggtacgc ggcttgtgcc gcatactgcg caaaaattta      1680
   cacaggtaa                                                               1689
<210> 153
   <211> 1629
   <212> DNA
   <213> Unknown
<220>
   <223> Unknown gut bacteria
<400> 153
   atgaaacaac aaacaaaggg cggggcgctg cacaaggtct ggcaggcgct gccgtggctg 60
   tggatggcgg cagcgtacct gtttgacctg tggtatcagc tggtgcccgg caaatggatc 120
   gtcgattccg accttgcctc ggaaatgatc ctctccgacc tgctcaacaa agagggaacc 180
   atcatcagcc ataactggtt ctactccacc gagctgaaag tcgtcaacct gcaatggttc 240
   taccgcctgg ggctgcggat tttccctaat gactggcacc tggcccgcac gtttggcatg 300
   gccatcacgc tggcgctgtt cgcggcggcc atgctctttt tcgtcaaatg cgcggggctg 360
   gggcgcgcgg gcctgtggat ggtcggtacg ctgctgtggc ccttcggaca gcattacctg 420
   gtctacgcga tctacggcgg ctattatctc gtttatacgt tcttttatat gctggtactg 480
   gcgctggtgc tgcgcagcct gaacgccgat aaaaagcact gcgccctgca atgggtgctg 540
   gcttgcgtca tcacggcggt ggccggcatg aacggcgtca agcagctcat ggtgttccac 600
   gcgccgctct gtctggcagc ggccattctg ctggtgttgg cactgcacag ctgcggcaag 660
   accaactgga aggccgccct ggacgccagc cgcaaagaag ttcgtttgtt tgcggcgtcg 720
   ctggtcacgg cggtggccgc tgcggcggga tacttcgtca gcaacgcggt tttgtcccgg 780
   atgtatgact tcaaatccta taacttcatc gtctggaacc gcgacgagga ttggttcacc 840
   ctcgaccgca tcctgatgga cttcttccat gagtttggct acgagaacgg ctccggtgtg 900
   ttccacttcg gcggcattgc ggcggccgtg ggcctgctgc tgggctgctg gatgttcttc 960
   tgcatcgtgc ggctgctgct gcggctggac aagctggaac gcaacgacaa gctgctggtg 1020
   ctgctgctcg tcgccatgct ggcggtctgc ggcgtggcct acacctactt ccacgagtat 1080
   tacctctact tctggctcat gaatatgccg gtggccatcg ccgtgatggc ggtggagatc 1140
   aagaccgagg acttccacat cctgggtgcc cggcagctgc tgggcgtggg gctggcggcc 1200
   tgctttacgc tctgcgccgt cagcacggta cgccaggagc aggaacaccc ctacctggcc 1260
   cacaaggggc tgaacaccgc cgccgaatgg ctggtggaca acggctatac ccagggctat 1320
   tccacattct ggaacggcaa cgccatgacc gagttgacca gcggcaagct ggaagtctgg 1380
   acgctgcaat cgctggaccg cgacgatgtg cccaactggc tgcagcccaa gagccatctg 1440
   accaccgacc cggagcaccc cttcctgctc atcgacaccg agacggacgg cccggcagag 1500
   agcgccaagc taatccagta cggtgactgc accgaggtgt acaacgatgg ccgctatgtc 1560
   atctacgatt tcgccgatgc ggacgccttg cacgcagcgg cccaggcggc agcggatgca 1620
   aaacaataa 1629
<210> 154
   <211> 1164
   <212> DNA
   <213> Unknown
<220>
   <223> Unknown gut bacteria
<400> 154
   atgctcgtca tcgccctgtc cctgcacttc tacccggaaa gcattttctt ctatccacag 60
   atactatacc gcgaaaacac ttttgtggca agtgtaaact tttttgggga atcattgcgg 120
   ttttgtggca catttgctat aataaacaca aattcccgcc aaagtgaggt gtcctgcatg 180
   aaaaaacatc cgttgcttac tgcggccgcc gcagccggta ctgccgtgct cggcgcagcc 240
   tacggcatct accgcgtcgc cttttaccaa aagcccacca ccgccgtgcc ctcccccgcc 300
   gatatgccca cgcgggaggt ctaccgcaag gccatgtgcc ccgacgccga tgcgctggcg 360
   cgggacacct tcacgccggt acatatcacc gcgcaggacg gcacaccgct ggccgcgcga 420
   tactaccacc acaacgacgg tgccccgctg gctatcatct tccacggcta ccggggctat 480
   gccgtgcggg acggcctggg cggctacacc ctctgcacca agctgggcta taatgttctg 540
   ctgcccgacc agcgcgccca cgggtacagc ggcggccaca ccattaccat gggcgtgaag 600
   gagcgctacg acgcccggga ctgggctgtc tgggcacgca gccacttcgg cccggaagtt 660
   cccatctttt tgatgggcgt ctcgatgggc gcagccaccg tgctgctggc cgccgggctg 720
   aatctgccgg acaacgtctg cggcatcgta gccgactgcg gctacaccag cccgcgcgag 780
   atcacccgca aatgtctgcc ggagtacctc cccggcttcc ctctcggccc cacctacgcc 840
   atcgggcggc tgggggccat cctgttcggc cattttgacc ctgaggatgc cgactgcacc 900
   gatgccgcgg cccacagcaa agtgccgatc ttcttcgtcc acggcgatgc cgacggcttt 960
   gttccctacg agatgggccg ccgcaactac gaggtctgcc gcgccaaggg caagcgcttt 1020
   ttgacggtgc acggtgccga gcacgccgtc agctactacc acgacaacac cgcctacacg 1080
   aaagaatgca ccgacttcct gcaagactgc ctcacccgcc gccacttcgc ctggccgaag 1140
   gaagcctccg acgacgcgcg ataa 1164
<210> 155
   <211> 1449
   <212> DNA
   <213> Unknown
<220>
   <223> Unknown gut bacteria
<400> 155
   atgactgaac tgatccaaag cctgaaggcg gcggtggcgc ggtatccggg cacgatggcg 60
   gaactggcgc gccgggcggc catcgaccgc tctactttat ataagatact cggcgggcag 120
   cgcacgcctc ggcgcgaaca gctggagcgc ctgaccgacg cactggaatt gaccgaggaa 180
   gaaaaaacca ccctgctggt gcagtacaag cagcgtggcc gcggcacgga ccccaagatg 240
   cgcgctgcgt tgaaccaact gctggaagcg gcgttccggg tcgaggatta cacccggcgc 300
   gagggaaaca tcaccaacga ggccgcgcgg cagatgagcg ttgcaggaca gtatgtggag 360
   ggtacccgcg cggtgacggc tgccgcagcg gcgctggtgg ccgagcatat gctatccggt 420
   gatacgcggc cgctgctgct ctcgccattc acgaatccgg tgctggaccg ggtgctggtg 480
   aaccgctttt ctgcgccaga cgggcagacg gcgtcgctgt cccagctgct gatttttgtc 540
   gaggaaagcg atatcccccg ggagaaagcc gggaatatcg ccacgctgac ccgcgcgttg 600
   ccgctcttgt ttctgcccaa gctcaactac gatgcccgcg ttgtacggtg tgtgacgagc 660
   gaacccagcc ccggtacgct gatgccggtg tatctgctgc tgcccacggc cgccttgatg 720
   atggaccgct tggggcagaa aggatatatc atcaccgacc gggaggtcgt ggaaaatctg 780
   cgcctgtcgt tcagccgaaa gtacatcgac gcagtgccgg tgctgaaact ggcgaccggg 840
   acccacggtt ttatggagtc catgcagttg tacagcaagc tgtttgccga gacgcgcaag 900
   tgctgcatgg tgcgctacca gccgccgttc agcctgtttg ccgatgaaaa gatcgcgctg 960
   cgggtcgtgc ggcaggacgc agccgtgcag ccctacctgc ccgcgctgct ggattacctg 1020
   aacacctgga accagcagac gccggatctg tatttctgcg aggaaggact tttgcagttt 1080
   gtgcgcaccg ggcagatgtt tgacctgccc tcggctctgt acgaaccgcc ggacgtcgag 1140
   acgcgccggg agctgctgct gcggctgcgc cgggcggcgg cctccgacaa gcagacgctg 1200
   cgcattgtgg aatcggccca gctgccgctg acaccgtcga tgagcgtgaa cgctttccgc 1260
   gggcgcgggg tggtgttctg ccaggctgtg ctggaaccgg gcaaaatgta ctgccgcgaa 1320
   tatctgatgg aggatcccca gatgaccgag gcagtgctga attatctgga ggagggcaaa 1380
   accggcgacc gggtgcgcag ccaaaaatat acggtggatt ttattgatta ttgcctgcgg 1440
   ttgttgtaa 1449
<210> 156
   <211> 1503
   <212> DNA
   <213> Unknown
<220>
   <223> Unknown gut bacteria
<400> 156
   atgtttaata aaaagaatcc catgcggtat ttctccggca gcacgtttgc gcggctgcca      60
   ctgtataagc tggcggcggg cggcacggct gtcgcgctga cggccagcat cggcctgggc     120
   gcgttcgcgg cgggcgttac gccgttcaac ctgcccacgg ccaccccggc cccggtcgtg     180
   accgccacac cggaaacggc agccacgccc acgccgtcgc cgtcccccac gcccagccct     240
   acgccggtgc cggaccgcac cctgaccatg gaggccaccg tcgtgcagca ggacgtcggc     300
   gtgacgctct acgtggaccc gctgcccgaa gccacagcaa cgccggaacc cacggctctg     360
   ccgtctgcgt cgcccaaggc tggcgcgtcg gcggcgtccg gggctaaacc cacggccacg      420
   ccaaaggcga ccgccacccc gaccccgacg cctaccgctg ctccgaccaa ggacgacgag     480
   caggaaaaga cacctctgct gggcgttgag ggcaccatca ccctgaccgc cgccaagggc     540
   aacgccaccg actacgcgct tgaccccgag accggcaccg ccctggccga ggacgtggaa     600
   cccggcgact acacgctggc cctgcagccc atcgaaggct acaccacccc cgaagcccag     660
   accgtaactg tgaaggaaaa ggtcgtctac aaggcggatg tacaggctgg caaggaaaag     720
   atcgttcagg caagccaggt caacgagagt gccgaggact ccggcgtcag caacgcaggt     780
   tccgcgccca tcgtcgagga agtgactgac acggttacct atgccgagag cgacaagacc     840
   gagaccggca ccaaaactac ttacacggcc aagttgagca gcagcggcca cctgctgctg     900
   gccgacggca ccgagacccc ctatctgccc gtctaccagg acggcacgcg ggatctgatc     960
   ggcgcgaccc gcgacccgaa ctacgcggcc aacacgaaca cttctgcctg gctgcccggc    1020
   gcagcggatt ccattcagct gctgggcgcg gccaacaccc gcatggcgtg ggcacaccgc    1080
   gccgatgaac ccgccgtgca ggagggggat gaggcggcct ccatcgccga ggagacgccc    1140
   gctcccaccg aggctcccac tccggaaccg acggcagagc ctactgccga gcccaccgca    1200
   gagccgacgg cagaaccaac cgccgagcct acaccggaag tgacggcgaa gcccgaaccg    1260
   acggtcgccc ccacgaacac cccggccccg accgaggccc ccaccgccac gccggatccc    1320
   acggcgggct ggcctgacaa tatcgaggcc aaggatctgg cgagctacgg ttttgacgtg    1380
   accagttccg acgacaccgt gtaccagtac accggatggc aggaaattga cggcgtgacg    1440
   tactattatg acccgacgac ccaccagccc gtcaccggac agcaggtcat ccagggcgac    1500
   gtt                                                                  1503
<210> 157
   <211> 783
   <212> DNA
   <213> Unknown
<220>
   <223> Unknown gut bacteria
<400> 157
   gtgccgaaat tgatatttga cccagaaatt tactgcaatg ctgccgagaa cgccgagctg      60
   gacggcagta cccccctgac cctgaccggg aacggcctgt gggtgggggt actctcccgc      120
   ggggaatgcc tgctggacgg ctttgaccgc ccaggccaga gcggcgatat cctgctgggg      180
   cctgctcccc tgacgctgac gccccaaacg gactgccacc tgctggctgt gcggctgacc      240
   ggccactgca cggacgccta cctattacag ttgggtgcgg cccgctgggc tgacggcgcg      300
   gcctgccccg gcgcggccga gttgattgcc cagctgcacg gcatccacac ggcggcgatg      360
   gcctatgcgc tgctctgcgc cgtgggcaag gctgacgaga cggcccgtcc cctgcccccg     420
   ctggtggccg aggctgtggc ggccatccgg cagaactaca tggccctcta cggcgtggag      480
   gaactgagcg agcagctcgg cgtgacgaag agccatctgg tgcgggtgtt caagcaggaa      540
   atcggtatcc cacccggaaa gtacatgacg aatgtgcgca tcgaggccgt gaaggcgctg      600
   ctgctcagcg atgagtataa cctggatatg attgcgggcc tgaccgggtt ttccggcgca      660
   aactacctct gccgcgtgtt caagcgggag gtgggcctct cccctgccgc ctggcgtgcc      720
   gccgctgcgc cgctgcccgc cgtgcccaaa ctgccgccgc gcagtcagga aatgtatgtg      780
   taa                                                                    783
<210> 158
   <211> 1224
   <212> DNA
   <213> Unknown
<220>
   <223> Unknown gut bacteria
<400> 158
   ttggcacaaa acaagaaaca gcggctgctg tggggcctgg ggctgttggg cacagcggtc 60
   tttaccctgc tgatgggctg gctgcactac caacagctgc tctccaccgg gtactattac 120
   tctgatatcc cggcccacat ggcgggccat gccatgggca ataccggctt ttcgctggtc 180
   acggtcctgc tgcagggcgt gttcgccatc ggcggcaacc tgggcacggc ggtgtttttg 240
   gctgtcgtca acctgctgac gctgctgata tttgcctggg ccatccgtaa ggctgtgccg 300
   caggtctgcc ctggcgcagc gctgctctgg tcgctggctg caaacctctg tcaggcggtc 360
   tggatgccaa acggcggcta ctggtacttt ggtgccatca ccggcacgat ctaccacaac 420
   actacctaca ttatgctgca accgttggcg cttatcgcgt ttttcctctt cctctcgctg 480
   gcagagcacc atggtcagct gcaatggcgc agttgggccg cgttggcggt gctgcttacg 540
   gtggcaacgg ctatcaagcc cagctacctt ttcgcctttg ccccagccct gcttgtggcg 600
   ctggtcgttg acctgatccg cacccacggc aaggccctcc aatgggaagt ctggctcggc 660
   tgtacggtgc tgccgggcat tctgctctgc atcattcaag ctaaggtgct gttcaccggc 720
   ggggatgatt ccggcatggc actgatcttc accaccgact tcgaccccga aaaagtgctg 780
   tggggtgtct ttaattacag cgccaagcac ggcctggtgc gctcactggt gttcgtgggt 840
   gcggtggttt tgctgctgtt ccggcagtgg cgcggcgctt acgcctataa gttcagcctg 900
   cttctgtttg ccatctcgct ggccgagggc atctgcctga ccgagaccgg cagccgtatg 960
   tacgacggca acctgtggtg gggcgcgttc atctgctatg acatcctgct gctggaatcg 1020
   ctgattcagc tgctgcgccg cctgcataaa aagccgcgca ccccggtgga gaccggcacg 1080
   tcggttttgt gctgcggggc gctggcctgg catattgcca gcggcattgt gttcctgggg 1140
   ctgatgctgg ccggtatctc ctatgcggtc atgatcggca ccgaaagcta cctgctgcaa 1200
   gaaatgtggg gcattgtact gtga 1224
<210> 159
   <211> 1602
   <212> DNA
   <213> Unknown
<220>
   <223> Unknown gut bacteria
<400> 159
   atgtctctgc ggcggtttta tgggctttgc ggggcgttgg tttttctggc gggggtcgtg      60
   ctctgccgca cctactgggt ggggcagcag gccgcctatg ccgccagcgc aggcggccag     120
   accgtccaga cactggactt gccccgggcc aggggcgatt tctacgaccg caacggccgc     180
   aaactgactg gcacgcaaac gcagtattac gcgctctgca tccccggcga cgatggctac     240
   acggcgctgt ttccctatgt ttcctacgaa aagcagggcg tgctttacga ccgccgtaac     300
   ctggccgcgc cgttcctgat tgaagtcagc cgcgacctga ccgggcgggg catcgccacc     360
   tgcgcggtgc ctgcgcatac ggtggatatt gcgccgcatc tgctgggcta cctcgacggc     420
   gaaggccgcg gcgtcgccgg gctggaacgc gcctacgacg cgctgctgac ggcctccggc     480
   gataagcgca ccgtcagctg cgtgctggcg gcgcacagcg gcctgatggc ggggacaagc     540
   cctgtatggg ctacgaggaa agaaggcact gggcagggcg tgcagctgac actggacgcc     600
   gacttgcagc gcgcctgtga ggcgctggcg gcgcagctta tgccgcgggg gtgcatcctc     660
   attatggacg ctgctacagg cgaggtgctt gccagcgtca gtgcgccgac gtttgacgca     720
   caggatgtgg ccgccagcat ccgcgccgac gatacttcgc tcatcaaccg cccatttgct     780
   gcgttcagcg ctggatcggt gtttaaagtg gtgctggccg cagctgccta tgaacagggc     840
   ttggactggt tcacccacga ctgcaccggt gaaatcgtgg tggcggggca gacctaccgc     900
   tgcgcccaag gccgcgccca cggcgaagtc aacctgcgcg gtgcgctgga agaaagctgc     960
   aacacctatt ttatcaaact gggccagctg ttgggggcag aagccatccg ggaagaggca    1020
   gaacacttcg gctttggcac agcggtacaa attgccccgg gcctggccgc agctgccggt    1080
   acgctgccgg acgcggagac gctggaaaac cccggccagt tggcaacgtt cagctttggg    1140
   caggggtcgc tgaccgtgac gccgctgcaa atcaccgcca tgatgaacac tgttgccaac    1200
   ggcggggtgt ataggacacc tggctttgtg cagggcatca ccggggccga tggtatgttg    1260
   accgacccgg cggcagcgga gagcgccccg cgtaccgtct gtgacgccgc cacggcccgg    1320
   gtgctgcgca gtatgctggc ctcggtggtg acggatggca ttggccatga cgcccagcca    1380
   cgccatggta cagcgggcgg caagactggc acggcccaga ccggccagta caacgccgac    1440
   ggtgaggagc tgttgaacta ttggttcacc ggcttttacc cggctgatgc gccccaatat    1500
   accatcacgg tcatgcagga cggtatcttg aagcccgaaa cctccagcgc cgccctgttt    1560
   gagcgaataa cggaaattct ggatgtgtgg gcggggtact aa                       1602
<210> 160
   <211> 822
   <212> DNA
   <213> Unknown
<220>
   <223> Unknown gut bacteria
<400> 160
   atggcggtac tgttgcatga acggccgcag acggccggcg gaaaaacgcg caagaccccg 60
   aacctgccca aaacctctgc tgtggtggcg ctgttcgcct ttagctcccg gctggaccgg 120
   gatacgatga gcctgtttgc caagacccac agcgatgaag tccgcgcggc ggccattgcg 180
   gccaccgggc aggaggtgct ggaactgctg cacggcgggc tgcgccccaa cgtattggtg 240
   ctggacgcgc tgctgaccaa gcccggcgtg accgaggtct tgcaggaaat cggcacgatg 300
   cagcttgacc cggaaccagc ggttctggtg ctggttcctg tgccggagga aaccgccgcc 360
   cgcaaggcat tgcggctgtt ctcccagtac cagatcatgc tgcgccctta cggcatgaaa 420
   aatctctttg atgaggtcta ccgcatgggc acgaacgagg atgagcacca gctgtactac 480
   ctgcgccagt gctgcgacga tgtgctggat gcctacggcg cgcccgaaac gctgagcggc 540
   taccgctatg ccgagcggat gctgctgtac gccctgtacg cggacaaacc gcagcagatc 600
   ggcagcctgc agcaatacgt ggctgcggag gaaggcatcg agatcggcac cgtgacttcg 660
   gcgctgaatc gtatgtcggc agccatggca aagcggggcg aagaacctta ccggacgtta 720
   tgcctgcgca gcggccgccc ggcggacggt gtactctcca atgggcagct gttcaaaggg 780
   ctgctgaaag atttgcggga taagacggaa cagtacttat aa 822
<210> 161
   <211> 846
   <212> DNA
   <213> Unknown
<220>
   <223> Unknown gut bacteria
<400> 161
   atgtcagttt tgctgattcc gaaccccacc aaggacgcag ggcttgccgt tacgcggcag 60
   gctgctgctg tgctggccgg gttgggcgta caggtgttga tgccgcagac cttcgcgggt 120
   gagggcaggc ttgccggggt acagtttttg ccggaggctg acgcctatcg cgcggcggac 180
   caggtcgtga ccatcggcgg cgacggaacg ttgctgcggg ccggactctc ctgtatgcgg 240
   cacggcaagc cggttctggg tgtgaacctg ggccgcatgg gctttctggc cacctgcgag 300
   gtcgccgaac tgcccacaaa attgcagcgc ttggcagcgg aggagttcac cctcgtgaat 360
   cgcgggctgc tccacgccga ggtccccgcc cacggctggc gggccgatgc catcaatgat 420
   atcgttctgt tcggccagac gcggctgcac ccgatggatt ataaagtgta ctgcgacggc 480
   gcgtttgtgg gcagctaccg cagcgacggc atgatactgg caacgccaac cggctccacc 540
   gcgtattcgt tttccgcagg cgggccgatc ttggatgttt ccgcagatgt gatggtctta 600
   acaccggtct gcgcccacaa tgtttacgtg gcaccgcttg tttttgccgc cagccgtcgg 660
   cttgaaatca ttgccgacgc cgagaaccgc gacggcagct ttgcctgcgc ggacagcagc 720
   gaccaatgtg acctgctccc cggtgagcgc ctgtgcataa cgggcggcgg gcagggctta 780
   cagctgattg cctttgacga cgcggagcag ttccacgcca tagagaacaa attgatgagg 840
   agatga 846
<210> 162
   <211> 1368
   <212> DNA
   <213> Unknown
<220>
   <223> Unknown gut bacteria
<400> 162
   atgaaactgc aaaaactgac ggaaaaacga tggttctggc cgctggtctg cgctgtgtcg 60
   gtcgtgttcg gctggtggta tctcagcatc gtgacctgtg cgcccctggg cggcgacgat 120
   gaactcatca acctgcaaaa ctactattat attacccaca catcctttgg gcaaagcgtg 180
   ctggattata tcggcgacct gtggacccag ttcagcttgc agcaggggcg gttccggccc 240
   ttctcctcgc cgccggtgcg cgggctgacg agctggtttt tgggtgatct ggtgggctat 300
   cggctctaca tcctggcctg gacctacacg gatatcctgc tgaccggctg gctcgtgggc 360
   aaggccagcc acaacaaaaa gctgggcatc gcctgtatct gcctgctgcc catgatgttc 420
   agtgtctggc aggactccac cggcaacagc ctgtattcct acggcgcgct ggtgcaaagt 480
   acgctgctgc ccgcactggt ggcagggctg gctgtgctgc gtttgcagga taccgggcac 540
   aagcggtggg ccgtgttggc ggggtactgc gcgttccagt gctgcgccac gtttgaaatt 600
   ggttttacct atattgtgcc gattttcggc ctggcctggc tctacaccga caaggcccgc 660
   gacgccctgc ggctgagcat tccggtgttt gtgggcgagt gcgtgacgct ggccttcaac 720
   ctgggggcgc ggctcgtcaa caccttgcag gagatgggca ttctggccgg ggatgcccag 780
   ccaatcggcg gtgtatcgcc gaactttgac atcccggcgg tgctcaagac ctgggccatg 840
   cagatgtcgg cgggcttccc gctcaatgca ctgttcgcgg gcaaggtccg ccccggcacg 900
   atctacccgg tggatatcct ctgcggcgtc atggtggccg gggctgcggt ggcggcgctg 960
   actgccctgc acgagctgcc aaaccgcaag caaaacctgc tgctgttctt gaccggcctt 1020
   gccatgctgt ccgcaccgtc gctgctcatc gggctttcgc ctaaatatca gcaggcgggg 1080
   cagatcgatt ggcggcacgg ctacatcccg cagacggtgg aaagctacgg cgttggcctg 1140
   atggccctgg cgctgctggt gctgctgctg cgctgggcac ggagcaaaac ctggtggccc 1200
   aagaaccgcg cggtgctcta ctgcctgctg gctgtcggca tggcgggcac ggtggtctgg 1260
   cagcgcgccg cgacccgctc agcctacgcc gagggtggcc gcgcctacac ggtctttggc 1320
   gagacggtag ccgccggggg ggccggggcc gccgggggcg cgggggtg 1368
<210> 163
   <211> 1305
   <212> DNA
   <213> Unknown
<220>
   <223> Unknown gut bacteria
<400> 163
   ttgaagaaac atatcatcaa aatcgccgct tttctcgtcg gctttttgct tatcaatctg 60
   cttttgtgcg gctttatgcc gccggataac ggttccagcc ttgccatgtg gcgcaactat 120
   cacagcaaag attccggcga tatcgccgtc attggctcgt cgctggcctc ctgcgctttg 180
   ccggaggaag aactggccgc cgccactggc aaatccgtgg cgctgatggc aaccaactcc 240
   caaagctggg atatgtccaa aattgcactg gaatccgtgc tgcgggaaca tacaccgcag 300
   tatgtcatcc tggtcatgga cctgtacaat atgacggggg agccataccc caaggcgcaa 360
   aaggcttttc tctctgccga gttgcagact tcccgctggc aggaccgccc cgccgaactg 420
   ctgcgttaca tgaccagcgc cgagcgcttt accgacgcgg attcgctgaa cgcgctgttc 480
   ccctggcagg gcggcaactg gcccaccgat tggcccgcca ccatccggca aaagtggaac 540
   gccctgactg cccgcattgc tgcgcgcggc gcggcacacg gccagccgga aggggatacc 600
   gtcatcgatt ttgacacgat cggcaatgtg aatacctgga accagacgaa ccacccattc 660
   acccagcagc acattgacga gcttgtctcg atgatgcagc tctgccaaga gaaaggcgtg 720
   ccgctgctcg tcatcagcgc cccaaaaacg acgctggacg tcatcagcta cgatacttat 780
   tttgacgatt acgccttttt caaaaagctg gcagggcttt acggcgcatc ttactatgac 840
   ttcaactttg ccaaacagga gctgttcgcg aacgttgaac cggattacta caaggatttc 900
   acccacatga acactaccgg cggccacggt ttcagccagt gcatggcaaa gttcctggcc 960
   ctgctggacg agggagctga cgtggatgcc ctgtttgaga cgccggaaga atacctggcc 1020
   gccgtcaact atatcacaaa tgtgtacctg acgccggaga ttcaaacgga caaaattctg 1080
   ctgctggcgg gttcctacca cggaccctca gtcgaggctg agtatgagtt ctgggtcaaa 1140
   gcccccggtg agacggaata cgcaaaaatt tccgattacg acacccgtac ctgggtcgaa 1200
   taccccacaa cagtacacgg tagctatcag ttccgcgtca atgcgcgggt agtcgggtct 1260
   gatgcagatt ttgagcgtta ttacgaatgt tatcttgatt tttaa 1305
<210> 164
   <211> 1776
   <212> DNA
   <213> Unknown
<220>
   <223> Unknown gut bacteria
<400> 164
   atgacacaca gaaaacgtac ttctatctta ttggtgattt cattcgtatt gattttggtt 60
   ggcagtttct tttctaacgc aatcaacacg gattttggaa aaattaaaac agatcgcctc 120
   tatctgatga atgacaatgg ttatacggta agtgcaagaa tttatatccc caaaacggca 180
   acgcaggagg cacctgcccc ggccatgatt atctgccccg gtggtgacag cccgtccgat 240
   ttgcttactc cctgggccag tgagattgca cgccgtggct ttgtggtagc actggtggac 300
   tataccggct gtggcgatac ggaagttgat aatgcttcgc agtattggac gaaccacggt 360
   gctatggagc tggaaacagt ctacgactat ctggcaaatc ggccattcgt agacgcaacc 420
   cagattggcg tgggcggaca ttccatgggt tcgctttaca gctattgcct gtcaaccaaa 480
   cgtcaggttt ccctggtcat ttctgatgtt atttacagcg aggctatgcc cacctacaac 540
   ctggactttg tacagatctc cgggcagcat gatgaaggtc tgttggcgcg cgtcaataag 600
   attgatgaat tgtttaaaga tccgttcctg acagaactgt tcggcacgga tgagatcgaa 660
   cccaacaaac tgtatggctc gtgggaagac cacaatgcac gcatcttcta tgtggtcaac 720
   cagacgcatg cagatgatat gtactggggg cagtttgtcc gcctggtagt ggactccgcc 780
   atgaacagca tggaagcacc taatccgctt cctgtaagca atatgatcta cggctggaac 840
   tttgtggcgc tgtttgttgt gatcattggc atcgttatga tgttgttctg cgtggcagat 900
   ctgttgctgg attctgacct gttctcgtcc ctgaagctgc ctgccccgca ggttacagca 960
   ggatttgcct ttaagtcaaa gggctggtgg atctgcgctg ctattctggc actgattccg 1020
   gtactgttct tcttccccgg tacagcggtc ggcaaccaga tggcttctaa taaactgttc 1080
   cagctgggta ctacccccaa tggctttctg atctggagcc tgttcgcggc ttgcggcatg 1140
   ctggtattct tccttgcgta ccactttatg tatggcaaaa agaaaggctg caatgtttcc 1200
   agctacggcc ttgccacagg tagtgatacc aaagtacaca ttggttatat tgtaaaatct 1260
   gccgtctttg cactgatcct gtttatgctg ggatactttg tcctgatgtt gatctatcgc 1320
   tatgcggata cagatgttca cggcctcagt atcagcttcc gtctgctgaa tgacgccaaa 1380
   tgtgcaacga tgccttggta ttgccttggt atgctgccat acttcctgct ggttacgctt 1440
   gcaggcaata ctttacagtt ccgcggagat gtcagcaaag gtaaggatat gaccaagtcg 1500
   gttattttgg gcgcctgcat tggcatggtg ggtatgctgc tgctcttcat cttctatgaa 1560
   gtaacgctgc gtctgaaccg cccgttctac acaggtcatt ttgcacactt ctacatgctg 1620
   ctcctttcta atgtgctgct gcagttctgc gtggcttccg gtctggcaat ttttatccgc 1680
   agaaagacca acagcatctg gcctggtgta tttattggta cggcactggt tgcatttggc 1740
   atggttagct ccaacagtat tgccatgatt atctga 1776
<210> 165
   <211> 1179
   <212> DNA
   <213> Unknown
<220>
   <223> Unknown gut bacteria
<400> 165
   ttgaaagacg aacatatctg gtccaaaaag ccagagacga tattggattg gttctgcgtg      60
   gtcggggcct gtatcgcatt ttatctgatt ttaaacaacc tcgggtattt cctgggcaaa     120
   atcggcttct ttatcgggat tctgtcgccg tttgcgggcg gcgtggtcat tgcctacatc     180
   ctggatccga tggtaaaatt cttttaccac aagctgttca aggaaaaaaa caagacacgc     240
   ggcattgcta ttctgatggc ctatgccgtg gcgatcctgt tgatcgtgct gctggcatgg     300
   ctggtcatcc cgcagatcgt gaacagtatc gtcatgctgt ttacgaactt ccccagctac     360
   atacagggcg tgcaggatct actggtcatg atgcaggatc gctacggcat cgacttgcag     420
   caggctatca aaatgctgga cgattctgag accatggtca aggaagtgta cagcctggcg     480
   accgccgcca tgccgcagat cgtggacagc atcggcagcg tggcgtccaa ctttgtgggc     540
   atctttactt cggtggcggc cagtatctat atgttggcgg ataaagagca tctgctccac     600
   cagctgcgca cgctgaccca cgcctttttg ccggagaaag cggctgacaa cacgctgcgc      660
   atctgccatt acgccaacgt gaactttacc gggttcttcg tcggcaagat catcgactcg     720
   gccatcatcg gcgtcatcac ctttgtggca atgacgatcc tgcggctgga ctttgcactg     780
   ctcatcagtg tgtttatcgg catcacgaac atcatcccgg tgttcggccc cttcatcggc     840
   gcaattccca gcatctttat cctgctgctg gtcgacccca tccaggcggt tattttcggt     900
   gtgctcatcc ttgtcatcca gcagctggac ggcaacttca tcggcccgaa gatcttaggc     960
   tcgtccatcg gtatttctgc cctgtggatt ttgttctcca tcgtggtggg cggcgatttg    1020
   ttcggcatcg tcggtatggt cgtcggtgtg ccggtgttcg ccacgctcta cggcctggcc    1080
   caggagttcg tgcattacac gctggataag cgaggcatcg acgctgaggg caacccaagg    1140
   gaacaaacgg ccgaggataa ggaaaacgtg tttgagtaa                           1179
<210> 166
   <211> 2811
   <212> DNA
   <213> Unknown
<220>
   <223> Unknown gut bacteria
<400> 166
   atggcggcaa gcacggccag cacagtgttc gcggcggacg atctgcccgc cctgaccgag 60
   ccgcccgccg tggctgcccc ggctgaccct gacccgcctg cggcggaaga agtcgtggtc 120
   gagaccgaag tgcccgaccc cacggcggaa ccggctccgg aaacaaccga aaccccgccg 180
   gactctgctg ccgagccgac cccggaaccc atgcccaccg aggcccctgc cccggagccg 240
   acagccgaga caacacccga gccgacggcg gaacctgccc ccgaaccgac ggaaccgccc 300
   atcccggacc ccactgccgc gccaacccag gccccggctg gcccggagcc gacggcggcc 360
   ccaacgccgc agcctaccgc tgcgccgctg cctaccgtgc cccccgcgcc cacggccagc 420
   ccggtttcac agcagactgc ggaggagacg aaatatgccg ccgtgctggc agcgcctata 480
   ccagagccga cggccgtgcc ccttgtagtg gacacgctgg aagtagagga caccatcagc 540
   acagacggct gctttaccgc tgtcgtcaac ggcagcgcgg agcgccagga cgtgacctac 600
   acctggtacc gcagccacga tggccagaac tgggagcagg tcacgccgcg catctgcacc 660
   ggcagcgagt ggaacatcac cgagggcagc gagcacaagc tgaacgccgc catcgattcc 720
   tgcatcgcca atgtcggccc taccgagcgg ctgtattaca aagtggaagt caccggcttc 780
   aacggtacta aggcggccga agcccgtgtg ccctaccatt tgcagctgca aaacggaagc 840
   tttgaaagcc cgagcctgca cagcatggcg gtggagcata cccggaccgg cgtgctttcc 900
   tatgcaccgg aggacaaaga gcagaccata aaaagccatt ttgcccagct ggcggacacc 960
   acacaggggc ttgtttggca cacgacaggc ctgtactacc actggacgga cgaggatgaa 1020
   acacgcgccg ataaaaccca gtgggccaac tacatagaaa ttgtagacgg aaccaaccac 1080
   agctataatg acgacttaga aaacaacgac cccagggtca gctacaacac ccgcagcgcc 1140
   caggacggca atcagttcgc cgagctgaac tgcgaagcct acggtgcgct ctatcaggat 1200
   gtgctgaccg tgcccggctc cacgctgaac tggtcgctgg cccacaaggg ccgcaacggc 1260
   gcggacacga tggtgctgct gatcgcgccg gtcacggtgg ccgaagcaat caccaaaacg 1320
   ctgactgccg ccgcccagaa cgaggctaaa gacagtgtgc gcaatgccct gaacgatctg 1380
   gtcgagtaca acggtcagaa ggtgcccatc agcactttta tggtcggcgg cgaaatgacc 1440
   gatggcgtcg ccaactgggc aacacacaag ggcacatacc aggtccctgg cgggcagtat 1500
   gtgagccgct tctttttcct ggcggtgcaa acccaggacg acaagcgcac cgagggcaat 1560
   ctgctggaca acgtctggtt cagcaccgac cctgccccgg cggtggcggg ccgcgcaaat 1620
   ctgaccatcc gcaaaaccat aacgggcggc ctgactgccg aggagctgac cgccgcgcgg 1680
   gcaaacctga cgttcaccgt ggcgaaccag aacggtacgg tcgcgacgat ccacggtgcg 1740
   gatatgaccg tagacagtgc tgaccccacc cgcagccgct ataccctgac cgacctgccg 1800
   ctgaccagcg acgacggcag cgtaaactac acctacaccg tggaagagac tggccacgat 1860
   gccccggcgg gcctgctcta cctgggcacc caggcggcgg tgaacggtga caatgagcag 1920
   acaggcacaa gcctggcgga cattacgctg accacaagcg ccgacacgac cgcagagttc 1980
   cgcaacatct acgcccgccc caccggcagc ctgcacctga ccaaggctgt ggccgacgag 2040
   gcgcagcaag cggaagccga cgcagtgacg aacaccttca cggtcagcag cctgcccatc 2100
   ggcagctata cactgcaatt tgatgatggt acgaccgaaa cgcgggaact aacggcgggc 2160
   ggcgcgctga ccattgcgct gaagggcctc accggcgtga cgctgaaaga catcccggtc 2220
   aaccgctata ccgtgaccga gacggcccac cccgacctgg caaactacta ctgcacgacc 2280
   accgccgacg atgccgcggc cgaagtgcag gtcatgtccg gccagacggc agaagtgacc 2340
   atcacgaaca gctatgcgcc gttcctgacg ctgaccatca ccaagcaggt gacaggcggt 2400
   ctgggcgacg cccgccgtga gtttcccttc acggccacgg tcgccggcca ggccatcacc 2460
   gccgccagcg cctatgtgac gaaaggggaa ggcgcggccc tgaccgggag cggcttcacg 2520
   ctgcgccacc ggggcagcat caccatcggc cacctgcgcc cgggggatgc cttcaccgtg 2580
   accgaaaccg acaacaccgg ttatgagacg agcttcgacc ttgatggcag cgtcgaggtc 2640
   ggcaccagcg gcgggggaac cctgggcgca cagagcgcca cgcttacctg tgtcaacaac 2700
   aaggacggcg caccgcccac gggatttggc ccggacacgg ccccggcagt ttggatgctg 2760
   ctctgcgccc tgctgggctt ggtctgcctg cgccgccgca agggggcgtg a 2811
<210> 167
   <211> 1704
   <212> DNA
   <213> Unknown
<220>
   <223> Unknown gut bacteria
<400> 167
   atgaagaatt tacacaaaaa gcaacttcgc ctgtcccgcc ggggcttctg gctggtctgc      60
   gccctgctgg cctgcctgcg cctggcgctg acgagctttc agcaggcgta cacctgggtg     120
   ggcggcgccc cgctggacga tgaactgatg ttccacgcgg cgcagaatat cacggcgggg     180
   gagtggctgg gcgcgtatga ctacctgacg ctgtccaagg ccatgttctt cccggtgtgg     240
   ctggccctgc tgcacgcact gcacctgccg tatctggtcg cgggcgcggc gctctggtgc     300
   ggcgcggcgc tgctggcagc gtttgcattt tcgccgctgt ggcgaaaaaa acggcccgag     360
   cagggccgtg tgctgacgct ggcgctgttt gcggcgctgg cgtttctgcc gtcgtcctgg     420
   gcggcttaca cgctgcgcgt ttaccgcgac aacatcttcc cggcactctg cctgtacttt     480
   tttgcgggca tggccgggat ggcactgcgg gccgtctttg aaaaagacaa gccgctctgg     540
   ccctggctgg cagcggcggg cgcgggccgg ccctgcggct acctcgaccg cgaggatgcc     600
   gggctgttcc tgttgccctt tgccgcggcg gccaccgtca ttgtggcgtt ggtgctggtg     660
   ggcaagcgcc gctggaaagc ctttgccgcg caggtcatcc cctacgccat gctcggcgtc     720
   ggtgtgctga ccttctgcac cctcaactac acccactacg gcgtgtttgc cctgtcggat     780
   ttctccgagg gctcgtttgc cgccgccatg ggcgcgatga tgcgcgttga cacggacagc     840
   gacaaaccgt acctgtctgt gcccgctgac gcacgcgaaa aaatctacga agctgtgccg     900
   gaactgaaac ccgtcgccta ctggttggaa gaggacgccc agatggagaa cgacttccgc     960
   gacccaggcc ttgacgacta ccgcgcgggc agcttctatt gggccatccg ccgcgccgcc    1020
   cagtatgagg gcatctacgc agacgcccag accgcagcca actattggca gaccgtggcg    1080
   gacaagatca acgccgcctg cgacgcgggc accctgccca gccgcaccgg caaacgggtc    1140
   gccaccagcc aacccatcac ggcggcctat gtgcccgcca cgctggccga gacctggaac    1200
   ggcttccggc acgtgctggg cttccgcgac tgtgcgccct acgaggcgct gcggtcgatt    1260
   ggaaccgacg acgacattgc ggcatggtcc ggctacctgc actgcggctt caacgctgcc    1320
   gccaacgcgg gggaggacac gccctattac agcccctacc agcgtgcgat tttcgccgtt    1380
   atgcaggggt gggcttgggt gtacggcgtg gtgctgacgg tctgcgtgtt ctgcgcggtg    1440
   gtgttccagc ttatgaagct tttatcgtgt ctgcgcaaaa aatgcacggc ggagaccgtt    1500
   gtgccctggc tgctgctgtt cggcatcttc ggtattgcgc tgctgcgctg cgccatgatc    1560
   gcctttgtcg aggtgtccag cttcggcatc ggcaccagca ccatgtacct ggctccggta    1620
   cacccgctgc tggttctcta cgtatttgcc gggctgtttt tgtatggacg gcctatatct    1680
   gttaaaagaa aggacaacgc atga                                           1704
<210> 168
   <211> 1140
   <212> DNA
   <213> Unknown
<220>
   <223> Unknown gut bacteria
<400> 168
   atgcgttcca ttaaaaccaa gctgttcagc cttgccatgg ctgtttccat ggtgctggcg      60
   ctggctggct gcgccatgtc cacgccgtcc accgtcggct ccatcggcgg gatcgagatt     120
   ccggccggta tctacctgct ggcccagtac aactcctaca acacctccgc caacgcggca     180
   gagcttgcca ccggcgagac tgcctccgac gtcaaggccg tgctgaaggc cacctgcacc     240
   ggcaccatcg gtgatgagga agtcactgcc accggcagtg agtatgttgc caagctgacc     300
   gaccgcgcca tttcctacta cgccgaggtc gagcgccgct ttaccgagat gaacggcgtg     360
   ctggatgacg ccgctaccgc cgaggccgcc gacactgccg acaacctgtg gtccaccaac     420
   ggcgacctgt ataccgccaa tggcatttcc aagacgagtc tgcagaccta cctgctcaac     480
   gcccagaagg ccaaggctct gctgaagatg acctacggcc ccgacggtac gacgcctgtg     540
   gccgaggacg agtacaccga cttcgtcaac aacgactgct actacatcga ggctgtgcag     600
   ttcccgctgg tggattactc cacctactcg atggccgatg acgaccagaa ggccgccatc     660
   atggccaccg ccgaaagctg catggccgaa ttgaacacga ccgccaccgc cgagaccgcc     720
   agcaacagcg ccctgtacac cgccgccatg acctatgtgc cgcaggcgat ggccgccatg     780
   ggcagcagtc tggacgccag ccaggccgtt tactacgcag cttcccagct gtacacgccg     840
   gacgacctga gcagctacgg cagcgacgag tacaacaacc tgaccgaccc gctggacgag     900
   gccgggctga accactggac caccatcaac ctgggcacga cgattttggt tgcccgtaag     960
   attgatccgt tcaagaccta caccgtggac gagctgaaca gtatgtacga cctgttgagc    1020
   agcatgaagt ccaccgacgt ccagagcgag ctgtacgccg ccggtgccgc actggagcat    1080
   aacctgaaca gttctgccct gaacacatac agcgcatcca agattaaaaa gaacgtttga    1140
<210> 169
   <211> 2463
   <212> DNA
   <213> Unknown
<220>
   <223> Unknown gut bacteria
<400> 169
   atgaaaaccc gcacgctctt ctgcgtgacg ctctttatcg tcggcgcatt cggtctgctg      60
   atttatcagc tctacgccct gcagctgcgt gatgccgaac tctaccgcac cgaggccgtg     120
   acccagcagc tgaaggatac gacgctgcct gcgctgcgcg gctcgattta cagcgtcaac     180
   ggcaagctgc ttgccaagag cagcacggta tggaatatcg ttgcagaccc ttcctctatc     240
   gccaagagcg gcgcgtcgga gagccagctg cgcacggcgg cggaaaacat tgccgcactg     300
   ctgggcgggg acaccactgc ggataccgtc tatgaggttc tgaccgctaa aaacagcagc     360
   ggcgagccgt atcagtaccg tatgctcgcc aagggcgtgg ataagccggt ggtggactcc     420
   atcctgaact atgccgatga gtaccgcatg gatcccgagg acggcgccac gaccggcaaa     480
   cgtattttgt acttttcgac cgaacaggcc acgacccgca gctaccccta cggcgagttc     540
   ctctcctcgg tgctgggctt ctgcaacagc gatggcgagg gcgcttacgg cctggaaaaa 600
   tactacgatg aaaccctggc aggcacgccg ggtcgcagtg tggccgagac ggatgtctac 660
   ggcaacgccc tggccgccgg gcaggccgat gtgcacgagg ctatcgacgg caacaacctg 720
   ttcctgacca tcgacgaaaa tgtgcagtcc atcgtcgaag aatacctgac cgaggcgatg 780
   aacaccttca cggtgcatgg ccgcggcagc gcaatcgtca tgaacgtcaa gaccggcgcg 840
   attttggcta tggcgtccat cgagcagttt gaccccaacg atccgtataa aatcaccgac 900
   accaaaatga ccgacatttt gggaaaagag gaaatcgacg ccaacgatat tgattggctg 960
   gaaggccgcc tgggcgaaaa agccgtggcc gatatcgtgg ccgacggcaa gatcagccgc 1020
   gaaaaaacca tcggcgagga cggcaaggag gtcgccagcg agtacaccca attacagggt 1080
   atgatgcgcg aggcccagtg gaagaataaa aacatcaccg agctgtatat gcccggctct 1140
   gtctttaagc tcatcaccgc atcggcggga ctggactccg gcgtcatgag cacgagccag 1200
   accttctact gcggcggcag cctgaccgtc aatgagggca gcgaactgtg ggagcacacc 1260
   taccgctgcg ccaacggcga ggttcaccgt atgcaggaca tggcgggtgc gctgaacaac 1320
   agctgcaacc tctggttcat ccaggctgcc gagacactga aaccgcagat tttctacgac 1380
   tatatccagg cgtttggctt cacgcagccc accggtatcg acctgcccaa cgagacccgc 1440
   tggaccagcg tctacaatgc cgagcagatg gccgaggtcg atacgaacct ctacaccgcc 1500
   gctttcggtc agaacgaatc catcaccccc atgcagatgg caaccgctgt ggcggccatc 1560
   gccaacggcg gctaccttgt cacgccctat gtggtggact ccatcacaga caacgacggc 1620
   aacgttgtct cccagaccga gacaaacatc cgccgccagg tcatttcaga ggacgtcagc 1680
   cgccagctgc ttgccatgat ggaaaataac gtctacgggg aaggggacta ccattcctgc 1740
   atcaacgcct atgtggcggg ctaccgcatc ggcggcaagt ccggcacggc agagcgcacc 1800
   gaccgccacc tgcgcggcga cggcgactac tacaaggcca tgagctttgc cgctgtgctg 1860
   cccatcgacg accccgaaat cgaggtgttc gtcctgctgg acgacccgcg ctgggtcaag 1920
   gactacgcca gccaggtcgt ggccccggtc gtgggcaaca tcatcagcga ggtcgcgccc 1980
   tacctgggca ttgagcagga ccccggctac aaccccagcg gcaccgtcaa ggtgcaaacc 2040
   tgcctggatt atacctggac caacgcccag gttacactga accgccaggg cctcaagcac 2100
   aagctcatcg gccccagcag cggcaatatc gtctaccagt acccggtggg cggcagcgat 2160
   gtccccgcag gctccaccgt ctacctgtac accgccaccg accagaactc gatgaccacc 2220
   gtgcccgatg tggcgggcaa gaccggcacc tttgccgagc agatgctgaa agccgccaac 2280
   ctgaacgtgc agttcagcgg cgacagcggc ggtaaggttg tcagccagag cgtggaggcc 2340
   ggcaccagcg ccgcctacgg cacaatcatc accctgacca tggacagcgg ggaagacacc 2400
   cccgccgaaa ctacccccgc cgccgaggaa gaaaccatcg acccggcgaa tgtggaaggg 2460
   tga 2463
<210> 170
   <211> 927
   <212> DNA
   <213> Unknown
<220>
   <223> Unknown gut bacteria
<400> 170
   atgcacgtta cacatcgcaa acgctttgtc agccgtgacc ttgttgcgct ggcggcccgc      60
   catccgcagg agctgactgc tttgagcgaa cagcactacc atgaccagat cgaggcaatc     120
   gccggggaag tcctggccgc aggacagcgt atcgtcatgc tgaccgggcc gtccgccgcg     180
   ggcaaaacga cttccgccca caaaattgcc gacgccattg cagcccgggg ccaccacagc     240
   caggtcgtca gccttgatga cttctacatc ggcgaggggc gttaccccaa gaaccccgac     300
   ggcagcgacg actatgaaag cttgcaggcg ctggatttag aaaagctcca cacctgcctg     360
   aaagacctgt acaagaccgg cgtatgtgac gcacctgtgt tcgattttac tgtgcagcag     420
   cccaccggca cccggcgcat cgacgcgcgg gacggcgtgg tcatcatcga gggcctgcat     480
   gccttgaacc ccgtgctgac cgagcagctg cccgatgacg cagcactctg cctgtacgcg     540
   ggtctgcggg aagaatacgc cgacgagcgc gacgcccgct gcctggccac ccgggatatc     600
   cggctggccc gccgcctggt gcgggacttc cttttccgcg gccacggcgc agcgttcacg     660
   ctggggctgt ggggccatgt ctgtgcaggc gaggatcgct acatcaagcc ctacaagccc     720
   cgcgcgaatc ttttactgga cacgacccac acctacgagg tctgcctgtg gcgcactgtg     780
   ctggacacga tgcccgccga cccggcgctg accgccatcc aggcccgcca actggcctcc     840
   ctgcgggaaa aattcgctgc attcccggcg ctttccaccg cgcttgtgcc gcaaaacagt     900
   atgctgcggg agtttattgg cgaataa                                         927
<210> 171
   <211> 1485
   <212> DNA
   <213> Unknown
<220>
   <223> Unknown gut bacteria
<400> 171
   atgaagcaaa ataaattaaa actgcgctgc accgaagatt acgcagtgct ctattgggat     60
   aaacccgccg ctgcgcccaa aggggcggag tataccgtct cgctgaacgg cgaggtcatc    120
   ggccgcacgg ataaaacaca tttcaccatc gagggcctgc cttacggcag cacttaccgg    180
   ctggaagttg ccagcggcag ctattgcgtc ggggtcgaga cgctgcaatt cggcaaagaa    240
   aagcgccgcg tcaacgtaac ggctgcaccc tatttctgca agggcgacgg caaaacgctg    300
   aacaccgaca acttgcagcg ggccatcaac gactgccgcg ccgatgacat cctgtatttt    360
   ccggcgggtg actacctgac cggggcgctg cagctgcaca gcgatttgga gatttatctt    420
   tccaaggggg ctgttttgca gggcacggcc aacatcaacg actattcgcc ccgcatcccc    480
   agccgctttg aagggatgga gatgcgctgc tattccagtc tgctcaacgc cgggcggctt    540
   gaccacacga aaggctcagg ctgccataac atcatcgtgc gcggcaaggg caccatctgc    600
   ggcggcgggc aggtgctggc aaagaaaatt atcgaggacg agcgcgcccg catcaagagc    660
   tttttggacg ataacgagga acttgtcaaa agctgcgaga acgacgacac gatccccggc    720
   cgtgtgcgcc cgcgcttagt aaacctgagc aactgcgaga atgtctggtt cagcggtatt    780
   acgttcaaaa gcagtccggc gtggactttc cacatggtct actgcgataa tatccagacc    840
   gaccactgcg tttttgaatc cgagggcgtc tggaacggtg acggctggga cccggattcc    900
   tcgacaaact gcacgctgtt cgccagcgag ttccataccg gcgacgacgc agttgccatc    960
   aagagcggca aaaaccccga gggaaacgaa atcggccgtc cctgcgccca catcaaggtg   1020
   tttgactgcc gcatcgggta cggccacggc atctgcatcg gcagcgagat gagcggcggt   1080
   gtggaggacg tgcagatttg ggactgcgac tttgccaact cgctgtacgg cattgagatc   1140
   aaggccacac ccaagcgcgg cggctatgtg cgcggcgtga ccgtgcgcga ttgcaccgcc   1200
   ccgcgggtaa ttttgcacag cgtatcctac aatgacgacg gcgaacccgc ccccgagccg   1260
   ccgaagttca gccattgctg gtttgagggg ctgaccctga ccggccgcac gctggatcac   1320
   gaccacatct ggcaggacgc cgcgcccatc gagattgtcg ggttcgactg ccatggcggg   1380
   gagatcgagg atattacgtt taaggatatc acaatcacga gggagagcgc agaactgaag   1440
   ctggggctgt gcagcggggt gaatctctac aacgtcacac tctcc                   1485
<210> 172
   <211> 1053
   <212> DNA
   <213> Unknown
<220>
   <223> Unknown gut bacteria
<400> 172
   atgtttgatt tttcgtttga tttgggcacg ctggttcctt ctctgctgct ggcgcttctg      60
   gccgttgtgc tgccgatgct cttccgccgc attgccgatg gctttctaag gcgtaccaag     120
   tcccacatgg catcgtggct gcacattttg ctggaaagtt acgtttttcc gcttgctatg     180
   atactgcgtg tttttctttt cttgctggct atttccatgc tgccacttgt atttgtcaca     240
   gatgccaaat tccagaatgt attggatgtc ggctccgatc tgttggtcat cttttttctt    300
   gggcttggca gctggcgcgc tgcgccggtt tcccgtttgc tgatgaaaag tgcggagaat     360
   cgtcttgact tgcagaccca ccaaacaatg ggccactttt ttgagaatat ctttcacgcg     420
   ctggtcgtgg tgttcaccgg cattgcggcg ctggaccgcc tggttgtgcc ggtcagcggt     480
   ctgctgaccg gtgccggggt tgccggcctc gctgtttcac tggctgcgca gtccacactg     540
   tccaacctga ttgccggcat cacgcttgtg ctggagcatc cttttggtat tggggattat     600
   gtggttttcg gagactatga aggaaccgtc gaggatatct ccttccgctc tacacggata     660
   cgtaccccgg ataatgttgt tattacgata gaaaactcga aggttgccgc cgagtatatt     720
   caaaactgtg attgccgcac aagccgcctg tggagtttta ccatcggcct tacctatgat     780
   accccccgcg aaaagctgga acgtctgaca acagacttga caggtctgct gcttgcccag    840
   cctgatgtga aggaaaacgg cgtaagtgtc acgcttgata aattcaatga ttacagtatt     900
   gacctgctgt gccgcgtgta cataactaaa acttctctgc gcgattattt gcaggtcaag     960
   aaccgcctga acctgctgat tcttgataaa gtccgcgcgg acgactgcga gtttgctttc    1020
   cccaccacga cgctggatat gccaaaatct taa                                 1053
<210> 173
   <211> 729
   <212> DNA
   <213> Unknown
<220>
   <223> Unknown gut bacteria
<400> 173
   atgatgactg taaaagaggt cagccgcctg accggtgtga gcgtgcgcac cctgcaatat 60
   tacgaccaaa tcggcttgct gcaccccggc acccgcaccg aggcgggcta ccgcctctac 120
   gatgccacca ccctggagac cttgcagcag attttgttgt tccgtgagtt gaaattctcg 180
   ttaaaagaga tcgccgtgat cctgcaaaac ccgcacttcg accgggaaaa ggcgctggaa 240
   cagcaaatcg agctgctgac cctgcaaaaa cggcatctgg aaagcctcat cactctcgcg 300
   gaaaacatcc gacaaacagg aggaaatgcc atggatttta ccgcatttga cacgaagaag 360
   ctcgacgagt acgccgcaaa ggccaaacag cagtggggcg caacggcggc ctaccaggag 420
   tacgagacaa agaacgccca tgagacaaca caggcccagc aggaaaacgc ccgtgcgttg 480
   atggccctgg tcgcggcgtt cggccacatg cagacgaaag acccggctga cccggccgtg 540
   caggcacagg tgcagaaatt gcaggcgttc atcacggaac attactacac ctgcaccaaa 600
   gaaattttgc acagcctggg ccagatgtac ggtgcgggcg gcgaatttac cgcgaacatc 660
   aacgcggcgg gcggccccgg cgcggcggag ttcgcccgga aagccatcga gcgatactgc 720
   tgcgggtga 729
<210> 174
   <211> 1521
   <212> DNA
   <213> Unknown
<220>
   <223> Unknown gut bacteria
<400> 174
   atgcactaca acaaatcgcc catgctgcgt tttttcaacg cgctgtttga atttatactg        60
   ctggtcgtcg tttttatcat tgcgggcgcg ctgcgctccg tcagcccgat cggcagccgc       120
   ttctttatgt gggacgtctg ggcgtttttg cccatcgcgg ggctgtatgc gctggtgaac       180
   gtggcctgct atgtggtgga aggctgctac cgctcgctgc acagccagaa caaaatgcgc       240
   cagctgttcc agatcggcgt aacgaacctg gtgggcctgg cgatgatggc gacggtgttc       300
   tacatcttcc gcgtcagtca gctttcgcgt ttgctgctgg cctactatta tgtcttgtcg       360
   gtggtgggca tcctggccaa gcggatgctg ttcagcgccc tggcgaccgc ctatgtgcgg       420
   cggcatgaca ttgtgccccg cacgctgatc atcggcagcg gcgagctggc tcaccggttc       480
   tacaacgaag ttttccggga aaagaaaacg atggccatgc ggtacagcgg ctacctggcg       540
   gcggccccct gcgccgacct gcccgactac cgcggcacgg tggccgacct gtttgaggtc       600
   gtgcgcaccc agaaaattga aatgctggtg ctggcccagg atgtgcagga cgccgaggaa       660
   gtgcgccgta tcatgctgct ggccgataac ttccacctgc gggtggcggc ggtgccggtg       720
   tacaacgact ttgtgaccgc tcgcagcgaa ctggacagca tcggcggtat gcggtacatg       780
   gacctgcggt tgatgaacac ctgcgacatc atgggcgtga acatcgtcgt gaccgatatg       840
   gacaagaccc tgcgcctcat cgaggaaaaa ctggaagatt ggcgcgggaa atacatctgc       900
   gtggcaaatg tccacaccac tgtgacagcc cacgaggacc ccgactacca gaaggtgcag       960
   aacggcgcag tcatggccct gccggacggc ggcccgctct ccaaatgcag ccgccagcag      1020
   gggtatgggg acgcggcccg cgtgaccggc cccgacctga tgaaggagtt gctgaaggag      1080
   agcaagacca agcattaccg ccattatttc tacggctcca cgcagaaaac gctggatgtg      1140
   ctgcgggaaa aagtcacgga gaattatccc ggggctgtca ttgcgggaat gtactccccg      1200
   ccattccgcc cgctgacgcc cgaggaggac gaggacgtgg tgcggatgat caatgaggct      1260
   aagcccgatt ttgtctgggt gggcctgggc gcgccgaagc aggagcgctg gatggccgta      1320
   cacgaggacc gtgtgcaggc gctgatggtg ggcgtgggcg ccgccttcga ctacgaggcg      1380
   ggcaatatcc gccgtgcccc gcagtggatg cagaagcaca acctggaatg gctctaccgc      1440
   ctgatgcagg accccaagcg gctgttcaag cggtattttg tcacgaacac caagtatttg      1500
   tggtggacgt ggcggcagtg a                                                 1521
<210> 175
   <211> 1377
   <212> DNA
   <213> Unknown
<220>
   <223> Unknown gut bacteria
<400> 175
   atgaaggaga tgtacacccc catgaccacc gcacacaaaa gccgcgtgct ggatatgacc      60
   cagggcgacc cgttccgcct ggtgctgcaa ttttcgatgc cgctgttctg ctcgaacctt     120
   ctgcagcagc tttacaattt gactgacacc gcccttgccg ggcacctgct gggcgcggcg     180
   gcactggcgc agatcggcgc gaccgccgca ctgtacggtc ttatcatgaa ctttgccttc     240
   ggcatgaaca acggcctggc actgacggtc agcaggtatt tcggcgccgg ggatgaaaat     300
   ggtatgcgcc gcgccgtggg ctggatggtc acgctttcgg tgggcacctc gctggtgctg     360
   accgccactt ccctgctggg ccgcggtgcg ctgttgactc tgctgcaggt ccccgccgaa     420
   acgtgggacg gtgccgccgc ctacctgacc gtaattttgc tgggcatccc gctgacgatg     480
   ctctacaata tggaggccgc gctgctgcgc gccgtgggca acagcatcac gcctcttctg     540
   tttttactct tcagcaccgt gctgaatatc gggctggacg cggcctttat gggcccgctg     600
   ggccttggcg tgcgcggcgc agccatcgcc accgtgctgg cacagggtat cagcgccctg     660
   ctgggcgtgt ggtacatcct gcgcagctac ccggaactgc ggttccgccc ggcccagctg     720
   aaagagacga cccgccatgc cgtgacccat atgttctggg cgggcctgag tatgggcctg     780
   atgagcgcca tctacaacct gggcagcgtg gccctgcaaa gcagcatcaa cgcgctgggc     840
   agcgcctaca ttgcggccca gaccgccgcc cgccgcctgg ctgagctgta ctttatcccc     900
   ggcggtgcgc ttggcatcgg tgtggcgact ttcagcagcc agaacctcgg cgcgggcaag     960
   cgcagccgca tctggcagag cgtaaaggcc gcgctggtca tctattttgt gtggtgggta    1020
   ttcatcatgg cgtttacctt cctctgcggc gaggcggcca tccgcggcgt gaccggcagc    1080
   gacgacgaaa tcattattgc caatgccctg ctgtacttga aaatcagcgt gcccatcatc    1140
   ccgcccatgg ccgtgctggt cattttacgg aatatgctgc aaggcatcca gcacaccatt    1200
   gagccgctgc tggccagcgc cctggagctg atcggcaaag tgatttttgc tgtctggctc    1260
   gtgccggtct ggggctaccg cgccgtctgc ttctgcgaac cgacgacctg ggtcatctgc    1320
   tttgtgttca tcctctttgc cgcctggcgc tgccgtggtg atttgaggga tgcgtaa       1377
<210> 176
   <211> 813
   <212> DNA
   <213> Unknown
<220>
   <223> Unknown gut bacteria
<400> 176
   atgttaaaga tcggagaatt ttccaagctg tcccgcgtca gcgtccggat gctgcgccat      60
   tatgacgcca tcggcctgct gaccccggcc tacatcgacc acttcaccga ctaccgctat     120
   tacagcgagt cccagctgcc caccgtctgc cgcatcaccg ccctgaagga catgggcttc     180
   cgtctggcgg atatctgccg gatgctgtcc ttctacgatg accggcagcg gctggacgaa     240
   tgccttgccg cccagcaggc ggcgctggag gcccaggcgg aggacgtggc ccggaagctg     300
   cggctgttgg acagcgcccg gaaacgacta agaaaggaag ataccatggc ttacaatgtt     360
   accgtcaaga ccctgcccca gcgttacgcc gcctgtgtgc ggatgaccat tccctgctac     420
   gagcaggagg ggacggtgtg gggtgtcctc tgcgccgaga ctgaccatat gaacctgatc     480
   cccgacgatc cctgctattg cagcgtgacc tttttggacg ccgagtataa ggaaaaggac     540
   gtggaggtag aagctcagaa aaccgtcaag ggtcagtatc cggacaccga gcatgtgaag     600
   ttccgcactc tgcccgccgt cacctacgcc ggctgcacct atcagggcgg ctacgagcat     660
   atcggtgagg tcaacgccgc cgtggccgcg tggatcgagg ccaacggcta ccgctatgcc     720
   ggccccatgt tcaacatcta ccacgtcagc ccccacgaga cccggaaccc cgacgaattc     780
   gtcaccgagg tatgctaccc catcacgaaa taa                                  813
<210> 177
   <211> 1185
   <212> DNA
   <213> Unknown
<220>
   <223> Unknown gut bacteria
<400> 177
   agggaaaagg agcatgaagc cgtggagcgg atctatctgg acaacgccag caccagcttt 60
   cccaaggcgg aaggcgtagc ggaggcggtc tatcagtata taaaggagtg cggctgtaac 120
   atcaaccgcg gcggctatga cgaggcgtat caggcggagg agctggtgct ggagacccgg 180
   cagaggctga cagcgctgtt ccacggcccg gactgccgga acgtggcatt tacccgcaat 240
   attacggaga gcctgaacgt gctgctgaag ggctttttgc gccccggaga ccatgtgctg 300
   gtgtccgcca tggagcacaa cgccgtcatg cggccgctga cccaactgca aagccggggc 360
   gtgtcctttg accgtatccc ttgcgctggg gacggtacgc tggatacgtc tgccatggcg 420
   gctctgctgc gggataacac gcgggctgtt gtgatgctgc atgccagcaa cgtctgcggc 480
   acggtgctgc ctgcggcgga ggttggggct ttctgtcatg cccacggcct gaaattcata 540
   ttggataccg cccagacggc gggcgtgctg cccatcgaca tggcggccat gcatattgac 600
   gcgctggcct ttaccggcca caagggcctt ctgggcccgc aaggggtggg gggattcctt 660
   ctccagccgg acatggtgcc actggtcacg ccgctgatcg ccggcggaac cggcagcgtc 720
   agccatgagg agcagatgcc gtcattccta ccggataagt tcgaggcagg taccctgaac 780
   ctgccgggaa tcatgggcct gcatgccgcg ctatgctggc tggagcggga gaccatcgac 840
   aaggtgcgca gccacgaatt ggcgctgaca gagcgctttc ttataggggc ggaggagatc 900
   ccgaacctgc gcgtgatcgg acgcaagggg acggaggggc gcgtcggcgt ggtgtccgtc 960
   ctgccggaga acgtggatcc tgccctgatg gccgacgcgc tggggcagga atacggtatc 1020
   atggtgcggg tggggctgca ctgcgccccc aacgcccaca agacgctggg cacatttcct 1080
   acaggcacta tccgcttttc cttcgggcat cagaacacga cccggcaggt ggacgcggcg 1140
   ctgctggcgc tgcggaaatt atgcgggagg accacatgga actga 1185
<210> 178
   <211> 1566
   <212> DNA
   <213> Unknown
<220>
   <223> Unknown gut bacteria
<400> 178
   atgaagctga actacaaacg caccattctg gtgggctttg cctttttcct gatctgcgcc        60
   ttctggcagg cctatgacaa caccattccc ctgatcctga ccaacaagtt cggcatgagc       120
   cagacctggt cgggcatcat catggcggcg gacaatgtgc tggcgttgtt tatgctgccg       180
   ctgttcggcg ccatctccga taaatgcagc cacaaaaagg gacggcgtac gccctttatc       240
   gtgatgggta cgctgatcgc cgccgtggcg ctgatctgtc tcagctttat cgacaacgcc       300
   cagctgaagc atctgggcga cgccgcgcgg atcgacgatc ccgccgccct gtccgccatc       360
   tatgaaagtc aggcggacga gcagctgatc acgccccacg gcgagaagtt cgtgctgtcg       420
   gagcggttca cggaggcgga attcgccgcc atccgcagcc agctcaccga cgagagcggc       480
   aagaccgtca ccaatcccga ctacaccaac tacgtgaccc ccgcccggca ggcctacgcc       540
   tggcatgtga cggcggcgca tcccgccacg ctggtgttct tcatcgctgt gctgctggtg       600
   attctggtgg ccatggccac cttccgctct ccggcggtgg cgctgatgcc ggacgtgacc       660
   atgaagccgc tgcgctccaa ggccaacgcc gtcatcaacc tgatgggcag cgccggcggt       720
   attctggtgc tggcgctggg catggcgttc gccaccgcgt cggtacacaa cgcactgatg       780
   agctacaccg cctatttcgc ggtgatcgcc gccattatgc tggcggcgct ggtgatcttc       840
   attctcaccg tgcgcgaggt ggagtgggcc cagcagatgc agtgcgacag cgtggaatac       900
   ggtgtggagg acacggcgga ggacggcgcg tccccgcagg aggagcggaa gctgacccgc       960
   ggcgaggtga agtccctgat tttcattctg ctgagtatcg tgctgtggtt cttcggctat      1020
   aatgccgtca ccagcaagta ctccgtgtac gccagcaaca tcctgcataa agactataac      1080
   ctgaccctca tcatcgctca ggcggcggcc atcgtggcct atctgccggt gggtgtgatc      1140
   tccagcaggg tgggccgcaa gcgggccatt caggcgggcg ttatcctact gaccgtggcc      1200
   ttcggcgtgg cgggcttcct ccacgcggag agccccacgc tgctgatgaa cgccatgttc      1260
   gccctggcgg gcatcgcgtg ggccaccatc aacgtgaact ccttccccat ggtggtggag      1320
   ctgtccagcg gcggcagcgt gggcaagtac accggctttt actacaccgc ctccatggcg      1380
   gcgcaggtgg ccacccccat gctgtccggc cttttgatgg acaagtgggg tatgcgggtg      1440
   ctgttcccct atgccacggt gttcgtggcg ctggcctttg ccaccatgct gatggtgcac      1500
   cacggcgaca gcaagcccca gatgaagctg ggactggaag cattacaaga ttcggagatg      1560
   gattga                                                                  1566
<210> 179
   <211> 1773
   <212> DNA
   <213> Unknown
<220>
   <223> Unknown gut bacteria
<400> 179
   atgttttcac agagcttttt ggacgagctg atcgcccgca acgacattgt ggacgtggtg      60
   ggctcctacg tggcgctgac gccgaagggc ggcagctatt ggggctgctg tcccttccac     120
   aatgagaaaa cgccctcctt ccacgttttg caggacaagc agttctatca ctgcttcggc     180
   tgcaaaaagg gcggcggcgt catcaatttc atcatggaga tcgagaacct ctcctatccc     240
   gacgccatcc gctttctggc caagcgggtc aacatggagg tgccggagga tcgggaatcc     300
   gccgacgccg accggctgcg taagcgactg ctggcgctga accgtgacgc ggcccggtgg     360
   tactacgacg tgctgcaaag ccccgacggc gcggcggtgc gggcatacct cgacaagcgc     420
   gccatctccc ggaagattgc tgtgcggttc ggtatgggcg cgtcgccgga cagctgggac     480
   gcgctgctgc gcaccatgac cggcaggggc tacaccaagc aggagttgct gaccgccgga     540
   ctgatcgtgg ccaacaagag cggcggcttt tacgataagt tccgcaaccg gctgatgctg     600
   ccggtcatcg atgtgcgggg cgatgtggtg ggcttcggca gccgcgtcat cgacaagtcg     660
   gagccgaaat acatgaacag cacggagacg ccggtataca gcaagcgccg ggtgctgtac     720
   ggcctgaatc tggcgaaaaa gaccaagcgt cccaacatga tcctgtgcga aggcaacctt     780
   gatgtggtga cgctgcatca ggcgggcttc gacaacgcgg tggcctccat gggcacggcg     840
   ctgacgacgg agcagattcg attgctggga cggtacacca aggagctggt gctgtgctac     900
   gacaacgaca acgccggtca gctggccacc cagcgggcgc tggagatgct gaacaacacc     960
   gagttcaccg tccgggtact gaagctgccc cggcggctgg tggacggcga atatgtgaag    1020
   caggatgccg acgatttcat caagtttcag ggcgctcccg ccttcgagcg gctgctgtcc    1080
   ggcagcgaga acggcgtgga gttccgtatg tcgcaggtgg ccgccaagta cgacctgcgc    1140
   agcgatcagg gccgcatcga ctacaccgcc gagatcagtc aggtgcttgc cgagctggac 1200
   aacgccgtgg agcgggaggt atacgccaat cgtgccgccg agacggcagg actttcgccc 1260
   gacgccatgc ggcaggaggt ggagcgggcc acccgcaagc gccgctatag cgaccgcaaa 1320
   aagcgggagc ggcaggagct gaaccccgcc ctgaccgtac agcccgccca gcggggcagc 1380
   cgctatacca atctccgcag cgccatggcg gaggagggcg tgatccggct gctgcacctt 1440
   gaccccacgc tgttcgggga cacgccgccc ctgacgccgg aggagttctc ctcgccgctg 1500
   ttgggaaaaa tttacggcgc catgtggcac cagcgcggca accgcagcgg cctgtcggcg 1560
   ctgacggggg agctgtccgg cgacgagatg agccacctga ccacgctgct gcaaaaaccg 1620
   gaatccaccg ccaacgcgtc tcaggcgctg gcggactaca tacgggtgat ccgcgaagag 1680
   cagaccaaac gggacgactc cggggtggat cccctgctgt tggcaatgga aacatttaaa 1740
   gataagaaac gatatggagg aaagcgcaca tga 1773
<210> 180
   <211> 1332
   <212> DNA
   <213> Unknown
<220>
   <223> Unknown gut bacteria
<400> 180
   atgttaaaag tagcaaaatt cggcggctct tccatggccg acgccgtgca atttgccaaa 60
   gtcaaggcca ttgtagaggc cgacgacgcc cgccgcgtca tcgtcgtgtc cgcccccggc 120
   aaacggtaca aggacgatca caaggtgacg gatctcttat atttgtgcag cgcccacctg 180
   aaatacggcg tcagctgcga gaaaattttc gccctgatcc gccagcggta tcagcagatc 240
   gccgacgact gcggcctgtc cctcgatctg gcggcggagt tcgacgctct gtggcagcgg 300
   atgtgcagcg gtgaagcgga cgaggagtat ctggcctctc gcggcgaata tttcgccgcc 360
   cggctgatgg cggagtatct gggctatacc tttttggacg cggcgctgtg gctgcggttc 420
   aagtttgacg gcacggtgga tcaggaagcc agctatgagg cgctgcggaa ggcggcggac 480
   ggtcagcggg tggtgatccc cggcttctac ggggctatgc ccgacggccg ggtggtgacc 540
   ctgacccgcg gcggcagcga catcaccggc gcggtggcag cggcggcgct ggacgccgcc 600
   gtatatgaga actggacgga cgtgtccggt atcctgatgg ccgatccccg catcgtggag 660
   aaccccgaac ccatccgccg cgtcacctac agcgagctgc gtgagctgag ttacctgggc 720
   gcgcaggtgc tgcacgaggg cacggtgttc cccttccggg agaaaaatat ccccctgaac 780
   atccgcaaca ccaacgatcc cgcccacgac ggcaccatga tcctcagcag catcgacgac 840
   gaggagcagg acgaaagctt catcaccggc atcgccggca aaaagggctt ctccatcatc 900
   accatcgcca agagcggcat gtcctcggag cccggctctc tgctgaagct gctatccatt 960
   ctggccaagc atcaggtaaa cgtggagtac gcgccctccg gtatcgacac cgtgtcgctg 1020
   gtggtggagg ccgccaaggc cgccccgtgc ctgtacagta tgctgggcga gatccagcag 1080
   gaggtgcagc ccgacaccat caaagtgacg gagcacatcg ccgtggtggc ggcggtaggc 1140
   cggaagatgg cgtaccgtct gggcgtgtcc ggtaagatct tcagcaagct gggcgagcac 1200
   ggcgtcaaca ttcgtatgat aacgcagggc ccggaggaat tgaacattat cgtgggcgta 1260
   gaggaaaggg attttgagca ggccattcgt gtcctgtatg acagctttgt aaaggaggaa 1320
   gttgcaaaat ga 1332
<210> 181
   <211> 1317
   <212> DNA
   <213> Unknown
<220>
   <223> Unknown gut bacteria
<400> 181
   atgggcggcg tggttgtctg cttcgccctg tttctggcgg cgatggcggt ctgccttgtc      60
   atgggctggt cgttcctgtg ggcgctgctg ctgggtctgg cgctgttcac gctccacggc     120
   cgcaagcagg gccacagcct gcaaaccatg tggggcatgg cctggtcaga gggccgcaag     180
   gtgctgatcg tactgcgaat ctttgtgttc atcggagcca tcaccgccct gtggcgcagc     240
   ggcggcacca tcgtgttttt catctactac ggcgtacagg ccatctctcc caagctgttc     300
   gtactggtgg cctttctgct gacggcgctg atcgcctacg cactgggcac gtccttcggc     360
   gttatcggca cggcgggtat cgtgctgatg gctctggccc gatccggcgg cgtcagcgag     420
   gcggtggcgg caggcgcgat cctgtccggc gcgtatttcg gcgaccggtg ctctcccgcg     480
   tcctcctctg ccgcgctggt ggcggcggag acggggacga agctctatga caacctgcgg     540
   aacatgctgc gcaccggcgc gctgccggtg gtgctgacgg tgatcgtcta cgctgtgctg     600
   tccgtccgcc accccatcac ggcggtggac gagagcctgc tgcaagccct gtgggacagc     660
   gctgtcatct ccctgtgggc gctgctgccg gcggcggtga tgctggtgct gccgctggtg     720
   aaggtgccgg tgctgatcag cattgccctc agtgtggcgg cggcgggact ggtcagcgtg     780
   ttcgtgcagg gcatgacggt gtgggacatg ctgaaaacgg cggtggtagg ctactatccc     840
   gccacgggac agctgaagga tatcctctcc ggcggcggta tcgtctccat ggtgaagccg     900
   gcggcgctgg tgtttaccac ggggctgctg gcaggtttgc tggacggtat cggcgtgctg     960
   aactgcgcgg tgggactggt ggaaaagctg gcccggcgct ggggccgctt tgccgcttcg    1020
   gcggcggtga gtctgggcgc gggtatgatc ttctgcaacc agtcggtggt gatcgtcatg    1080
   ggcaatcagc tgctgcaccg ctgctatacc gaccacaatg agcaggcggt ggacatggaa    1140
   aacagcggca tcgtcctgtc ggcgctgatc ccgtggaaca tcgcgtgcag catcccattg    1200
   gtgatgctgg gggtggatca gcgggccatt ccttacgccg tgctgctgta catgatcccg    1260
   ctgatctacg gcctgacaaa acgatatgtg tatccccata aggagaaatg tgtatga       1317
<210> 182
   <211> 981
   <212> DNA
   <213> Unknown
<220>
   <223> Unknown gut bacteria
<400> 182
   atgataccgt attttgacgc tcactgcgat accatttacc gctgtctgga gaccggcgag 60
   acttccgcac tggagtatgg cgaggatcgg gaggagcagc gccgttatta cgccgcgtcc 120
   acccatctgc gaaaaaacgg cggccatatc gatctggaac ggggactgaa gttccggcgc 180
   tgtgcccagt ttttcgccct gttccacgac gcggcggagg ctccggctga cggcatgtgg 240
   gcacaatgtc aacggatgca cgatttcttc ctgcgggaga tggcggacaa cgccgatatc 300
   gtccgccatt gccgcaccgg cggggaagtg gatgtcacgg tggtggaggg taaggtggcc 360
   gcgttgctga gcatcgaggg cgccgacctg atcgactgcg acgtacataa gatcgaaacg 420
   gtgtccggtt ggggcgtgtg gctgctgaac ccggtatgga accgtgccaa tgtgctgtcc 480
   ggcaccaatg ccgaggaacc ggagcgggga ctgtccgccg aaggccggga ctttattcac 540
   gtgctggagg agcacgagat ctatcccgac gtgtcccacc tgtccgacgc tggcttctgg 600
   gatcttgtcc gcatggcgaa gcggcccatc gtggcgtccc actctaacgc acgggccgtc 660
   tgtccccacc gccgcaacct gacggacgac cagttccggg ccatccgcga tctgggcggc 720
   gtggtgggcc tgaacctgta tcttgatttt gtgggccagc ccacgatgga tgcgctggcg 780
   gcccatgtgg agcacttcct gaacctgaac ggcgaaaaaa cggtgtgtct gggcggcgat 840
   ctggacggct gcgaagcgct ggccgccggt atgaccggca tgcaggatgc gccgaagctg 900
   tacgacgcgc tgaaagcccg cggctacagc gaggcgctgc tggaggatat cttctggaac 960
   aacctccgcc gcctgatctg a 981
<210> 183
   <211> 624
   <212> DNA
   <213> Unknown
<220>
   <223> Unknown gut bacteria
<400> 183
   atggtaaata gcaaagaggc tagttttgcc tatttggaga cattgagcga cgagcagcta 60
   tgcgccgcct gccaattggg gaaccgcaac gccgaggagg ttctggcggc gcggtatcac       120
   cggctggtgc gtagcatcgc ccgaccctac tttcttgccg gcggagacaa cgaggatctg       180
   ctgcaggagg ggatggtggg gctgatcaag gccatgcggg aatacgaccc ccatcaggcc       240
   gcgtccttct gcacctttgc cgaggtatgt atccggcggc ggctgtattc ggtgctcaaa       300
   tccgcttcct caggcaagca tcagcctctc aatcaagcga tccccctgaa tccctcattc       360
   tttgacgtca gtctcgcttt tgcacaggtc gaccccgagg tcctgctgat tgacagggaa       420
   aaaaccgccg gtctgctgga gtacacatgc aaacagttat ccgccttcga agctgagatt       480
   ttagggtact atttggacgg ttttacatgt cgtgagatcg cagagactgt gggcaagccg       540
   ccaaaatcgg tggaaaatgc tgtgcagcgc atacgccgca agatcgggcg gcaacttcaa       600
   tctggcgata tcagcaaagg ctga                                              624
<210> 184
   <211> 915
   <212> DNA
   <213> Unknown
<220>
   <223> Unknown gut bacteria
<400> 184
   atgaccttgc agcagctgaa atacgtatcc atggtggaaa aatgtggctc cttcagccgg       60
   gccgcccaaa agctgtacgt ctcccagccc agcatcagca atctggtgaa cgcgctggag      120
   gaggagctgg gcatcaccat tttcacccgc tcctccaccg gcattgccat caccaacgag      180
   gggcgggagc tgctgaagct cagcaaccgc ctgctgcggg acgccgacta catcagcgag      240
   tatttccaca gcgacacgga gcaagaaaag ccctccttct tcctcagcag ccagcactac      300
   gacttcgtgg tcagcgcctt tgaggaattt gtgggcggca tcgacgccga ccgctatacg      360
   ctggggctga accagaccca gaccgccgcc gtcatcgagg acgtgcgcaa gcagtacagc      420
   gacatgggcg tgatcttcct gtcggacgtg aaccgccgcc acatgctgaa ggtgctggag      480
   gacaacaacc tggaattcca cagcgtatcc cgcgccgcgc cccacgcctt tctgtcggtg      540
   gagcatccgc tggccaagcg ggagagcgtg acgccggagg agctgatgga ctacccctgc      600
   attgtctatg agcaggacgg cgactccccc ggcttcttct cggaggagat gatgctgccc      660
   aatttctacc cccggaaggt ggtctacatc agcgacctgt acgtgagtac ggcgctgatg      720
   cggggctgca acgcctacga catcggcacc ggcgtgatct caaagcggct gtcccgcgag      780
   gtggcctgcg tgcctatcgc caccgacgac cgggtggata tcggccggat cggcattcgg      840
   gacagggcgc tgcggcccat tgagcgctca tttctggact gcatgacccg gcagctccac      900
   gccgcacagg ggtaa                                                       915
<210> 185
   <211> 1350
   <212> DNA
   <213> Unknown
<220>
   <223> Unknown gut bacteria
<400> 185
   atggcatacg aagaatttct gggtatgtat caatccaagc tgaccacggc agaggaagcc      60
   gtaaaagtga tcaaatccgg cgactgggtg gattatggct tctgcaacac ccacccccat      120
   gttctggacg aagcgctggc tcgccgcgct cccgagctgg aggacgtgaa ggttcgtggc     180
   ggtatcgccc tgtggaagcc cgcgatcttc gatatcgagg agcctgccaa gcatatcatc     240
   tacaattccc accacaccgg tggctttgag cgtaagcaca tcgataccgg ggcctgcttc     300
   tatgagccta tgcgctattc cgagctgccc cgttactatt atgaccatat caatccgccg     360
   gatgttgccc tgatccaggt tggccccatg gataagcacg gctacttcaa cttcggcgtt     420
   tccgcttccc acatgaaggc catctgcgac acggccaaga tcctgatcgt ggaggtcaac     480
   cagaacatgc cccgctgtct gggcggcttt ggcgagagcg tacatatcac cgacgtggat     540
   atgatcgtgg aaggccgcaa cgaccccatg ggtcagatgc tgtccgcacc tcccacggag     600
   atcgacaagg ccgtggccaa gcaggtggtg gagcgcattc ccaacggcgc ctgcctgcag     660
   ctgggtatcg gcggtatgcc taacgctgtg ggtatgctgc tgtgcgagtc tgatctgaag     720
   gacatgagcg tccataccga gatgtacgtg gaggccttcg tagatctgtc cctgtgcggc     780
   aagatcagcg gcgccaacaa gagcatcgac catggccgcc agacctattc cttcgctgcc     840
   ggcagccagc gggtctatga cttcctggat gacaaccgca tgtgcatggc tgctcccgtc     900
   agctatgtca acgatatccg caccatcgct tccatcgata acttcatgtc cattaacaac     960
   gcggtggata tcgacctgta cggtcaggtc agcagcgaga cctccggcat ccgccacatt    1020
   tccggcgccg gcggccagca ggacttcggg ctgggcgcat acctgtccaa cggcggcaag    1080
   agctttatct gcctgtcctc caccttcaag aagaaggacg gcacgctggc ttcccgcatt    1140
   cgtcccacgc tggcagaggg cagcatcgtc accgataccc gcgtcaacac catgtatgtg    1200
   gtcacggagt atggctgcgt caacctgaag ggcctgtcca gctgggagcg ggccgaggcg    1260
   ctgatctcca tcgctcaccc tgatttccgc gaggagctga tccaggctgc ccagaagcag    1320
   aagatctggt atcacagcaa caagcgctga                                    1350
<210> 186
   <211> 627
   <212> DNA
   <213> Unknown
<220>
   <223> Unknown gut bacteria
<400> 186
   gtgatttttt ataccgccga tcttcacttt ctctatgagg cgctgctgtc ctcccggccc 60
   tttgccacag tggaggagat ggacgagacg ctgatccgca attggaacga aacggtatcg 120
   ccggaggaca ccgtgtatct ggtgggtgat atcgggtata acgaggggca ggttccccac 180
   cagctgctct cccgcctgac gggacacaag cacctgatcc ggggcaacca cgacaccggg 240
   tacgaggacg ccgcctccct ctaccgctat ttcgagacca tcaccgactt caatgagatc 300
   gacgacggtg agacccatat tctgctgtgc cactacccca tcatttaccg caagcggggc 360
   tatatgatcc acggccacat tcatcagagc cggggacagg agtaccaact gctgaaggag 420
   ctgccccgtg tgctgaacgc cggggtggat atcaatttct accgccccgt gacgctggag 480
   gagctgatcg aaaacaaccg cgccttctac agcggtgagt gggatgacct gatccctccg 540
   ccgccccgca cgccccgggg cgataagggc tggcttccgg ccacgccgga cttccgcccc 600
   atcccggagc ggccaaacgg ccaatga 627
<210> 187
   <211> 828
   <212> DNA
   <213> Unknown
<220>
   <223> Unknown gut bacteria
<400> 187
   atgtcctttg gagagcagct acaggaggtg cggcggagca acggcatgac gcaggagcag 60
   tttgccgagg cgttacaggt gtcccgtcag gcggtgagcc ggtgggagtc gggccgcagc 120
   tacccggagg tggataagat cctctatatc tgcaaccgct acggcgtgtc catcggcacg 180
   ctgtttgccg aggagaatcc cactcccgac agcggcagac cccagccgcc tcagacggcg 240
   gcccagccgc ccaaggacag cctgttcacc tcgctggatt ctttcatcac caacttatcc 300
   cctaagagca agtggatcgg catgggtatt ctggtgggcg tggcgctgct ggcgctgctg 360
   attggcctgt gcctgaaagg aggaagcgga gacatgtcaa cgatcatatg gatcgccgcc 420
   atcgtcatct tcggcgtggt ggaggcggcc acggtaggac tggtgtccat ctggttcgtc 480
   atcggcagtg cggcaggcct gatcgccgcc gtgctggacg cgcccatctg gcttcaggtg 540
   gtggcgttct tcatcgtgtc cattgcggcg ctgatcgcca cgcggccgct ggtgaggaag 600
   ctgggcagca agggcggcac cgctaccaac gccgaccggg tgctgggcgg caccgcccgc 660
   gttaccgaga ccatcgacaa caccatcccc tccggcgagg tgtacatcga cggcaaaaca 720
   tggacggccc gcagcgaatc cggtgcggtc atcgccccgg agacgctggt aaaggtcatc 780
   cgcctggagg gcgtgaagct gtatgtggac gttccccata acgtgtaa 828
<210> 188
   <211> 786
   <212> DNA
   <213> Unknown
<220>
   <223> Unknown gut bacteria
<400> 188
   atggacaaac agcgctgcat cgccgcggcg gcggaaacgg aggaggaccg gctgctgctg      60
   gcccggctct acgataagct gaaccacggg gcggagcggg agatcttcag ctgcacggcg     120
   tttctctcgc cccgtgagca ggccatggtg aagaagctgc tgccccatgt gccgctggtg     180
   tttttcggcg gctgcgacgg ggcggagcgc tgcatggcct gctacgtgcc ggagtatctg     240
   ccggaggact atctgacctc ggaggacggc ccggcggctg ccgtgcgggc cgtgtactat     300
   gagaaggacg cactgaccca ccgggatttt ctgggcagcc ttatgggtgc gggcatcaag     360
   cgggaaacgg tgggggatat ctatgtctct gccggacagt gcgacattct ggtgacgcgg     420
   cagatcttgc cctacctgct ggagcagtgg gacagcgcgg gccggacgaa gctgcacctg     480
   tcggtgatcc cgctggcgga ggtacagcgc ccggcggtgg aaaagcggga gatcagggac     540
   acggtgccct cgctgcggtt ggactgtctg gtggggtcgg ccttttcgct ggcgcgaggg     600
   cctgccggac agctgatcgc cgccggaagg gtggccgtca acgatctggt gtgtctcaag     660
   ggcgaccggc aattacagca gggcgaccgc atcgccgtgc ggggactggg caaggcagcg     720
   ctggcacagg tgggcggcac cacccgaaag ggacggctgt ccgtcacgct ggagcgctgg     780
   atataa                                                                786
<210> 189
   <211> 1527
   <212> DNA
   <213> Unknown
<220>
   <223> Unknown gut bacteria
<400> 189
   ttgaccagtc gtaaaatcat caacagtaaa ctgaaggaag cggtggattc cgtattgccc 60
   atcaccctga tcgtggcggt gctgtgtttc ttcttcgttc ccgtgggcag cggactgatg 120
   ctggcctttc tggtgggcgc ggtcatgctg attttgggca tgggcctgtt cacgctgggc 180
   gcggagctgt ccatgaccca gatgggcaat ctggtgggcg cgaagatgac caaatcccgc 240
   cgcctgtggc tcattctggt gctggccttc gttctgggcg tggtcattac cattgccgag 300
   cctgacctgc aagtcctggc ccagaacgtg ccgggcatcg acaagaccat gctgatcctc 360
   acggtgtccg tgggcgtggg catcttcctg accatctgca tgctgcgtat cctgctgggt 420
   atctccttgc ggacgctgct gctgatcttc tatgccattg tgtttattct ggccgccctg 480
   tccgacccgg acttcctgtc cgtggccttt gactccggcg gcgtgaccac cggccccatg 540
   acggtgccct tcatcatggc gctgggcgtg ggcgttgcct ccatccgaag cgacgaaaag 600
   gccaagtccg acagcttcgg tctggtggcg ctgtgctcca tcggccccat tctggcggtg 660
   ctgctgctgg gcttcctgta cccggaaaac accgcctctg cctcggaaac ggtggtcaac 720
   agctttgaaa ataccctcac cgtgggctac agctatttgg aggagctgcc cgccatgatg 780
   ggcgaggtgg ccatcgccct gctgcccatc tgcgtgttct tcctgctgtt ccagatcttc 840
   tcgctgcatc tgcggaagct gcccctgctg cgcatcctca tcggcgtggg ctatacatat 900
   gtaggtctgg tgctgttcct gacgggcgtc aacgtgggct tctcgtcgct gggctacgtg 960
   ctgggcggcg tcatcgtgga ggagggtctt ggccttctga tgatcccgct ggccaccctg 1020
   atgggctggt tcatcatcaa cgccgagcct gccgtccacg tgctgaacaa gcaggtggag 1080
   gagctgagcg ccggcgctat ttcctccaag gccatgggca tgagcctgtc catcgccgtg 1140
   gccgccgcca tgggacttgc catggtgcgc gtcctgaccg gcatctccat tctgtggttc 1200
   gtgctgccgg gctatatcat cgcgctgggt ctcagctttt tcgtccccag catcttcacc 1260
   gccatcgcct tcgactccgg cggcgtggct tccggcccgc tgaccgccac cttcatgctg 1320
   ccctttgcca tgggcgctac cacggcgctg ggcggcaatg tgatgaccga cgccttcggc 1380
   cttgtggcgc tggtggccat gatgcccctg atcgtggtgc aggtcatggg cgccatctac 1440
   gtggtcaaga cccgccgcaa cagtgcgcag gaatccgcgc ccgtattcag caacgagggc 1500
   gttatcgaac tgtgggaggt ggcctga 1527
<210> 190
   <211> 1332
   <212> DNA
   <213> Unknown
<220>
   <223> Unknown gut bacteria
<400> 190
   atgaagatac agttatccga tcactttacc tataaaatgc tgctgcagtt cgtgctgccg 60
   tccatcatca tgatggtgtt cacctccatc tacggcgtgg tggacgggct gtttgtgtcc 120
   aatttcgtgg gcaagacccc cttcgccgcc gtgaatctgg tgatgccctt catcatggtc 180
   tttggcggca tcggcttcat gttcggcacc ggcggcagcg cactggtggc caaaaccatg 240
   ggcgagggcg acaagcaccg cgccaatcag tacttcacca tgataatcgc cgccaccctc 300
   atctgcggcg tcatcatctc cacgctgggc gtgatcttta tggagcctat ctccgtcttt 360
   ctgggtgcgg acgcggaaat gctgccggat tgcgtgctgt atggccgcat cgtgctggcc 420
   ttcaacacgg cgtatatgct gcaaaacgtg ttccagagct tcctgatcgc ggcggaaaag 480
   cccaagctgg gcctgtgggt cacggtggcg gcgggcgtta ccaatatggc gctggacgcc 540
   ctgttcatgg cggtgttcca ctggggcgtg gcgggcgctg ccatcgccac tggcatcagc 600
   cagatcgtgg gcggcgtgct gccgctggtg tacttcctgc ggcccaacag cagcctgctg 660
   cagctgacaa agacaaagct ggagctgcgg cctatcttgc aggccagcgg caacggcgcg 720
   tcggagctgg tcagcaacgt gaccgcctcg gtggtgggca tgctgtataa cttccagctg 780
   ctgcggctgg cggggcagaa cggcgtggcg gctttcggcg tgctgatgta cgtggagttc 840
   atcttcgccg ccgtgttcat cggctatgcc atcggctgcg cgcccatcgt cagctatcat 900
   ttcggcgcgg gcaaccgggg cgagctgcag aacctgctgc ggaaaagtat tctgctgatg 960
   ggcgtgggcg gccttgccat ggtgggcgtg gcccagggac tggccggacc tatggcacgg 1020
   ctgttcgtgg ggtatgacaa taccctgtgc cggatgacgg tgcatgcctt ccgcatcgcc 1080
   acattgtcct tcgtgctggt ggggttcaac atcttcgcct cgtccttctt cacggcgctg 1140
   aacgacggcg tggtgtcggc catcatttcc ttcctgcgca ccttcatgtt caagctgtcg 1200
   gcggtactgc tgctgcccct gatctgggag ctggacggca tctggtgggc cgaggtggcg 1260
   gcggagatct gcgctttcgg tcttggcatg gcgttcctgc tgggcaaacg gaaaaagtac 1320
   gggtatatgt ga 1332
<210> 191
   <211> 1134
   <212> DNA
   <213> Unknown
<220>
   <223> Unknown gut bacteria
<400> 191
   atgatttatc tggatcacgc cgccaccacc cccattcctc aggctgttgc cgacgagatg 60
   taccgcgtgc tgacgcagca gttcggcaat ccctcctccc agtacccgct gggacaggat 120
   gccaagaaga tggtggaggg ctatcgggcc gctatcgccg gggcggtggg ctgccagccc 180
   aaggaactgc atttcacctc ctgcggcacg gagagcgaca actgggccat tcaggctgcc 240
   ctgtggcaga accgccatgt gggcaagcac atcatcacca ccaccgtgga gcacagcgcc 300
   gtgctggaga cctgccgtgc gctggagcag caggggtacg aggtgacgta tctcaagccc 360
   gacaagaccg gccacatcac cgcccaacag gtgggcgacg cattgcggga ggatacggcg 420
   gtggtcagcg tgatgctggt gaataacgag accggctgcg tgttccggct ggcggagatc 480
   gccgccattt tgaaggcacg caaaagccgc gccctgctac ataccgacgc ggtgcagggt 540
   tttatgaagg tgccctgcga ccccaaaacg ctgggggtgg atatgatgag cctttccgcc 600
   cataagatcg gcggccccaa gggtatcggt gcgctgtatg tgggccccaa cgtccgcaac 660
   atccggccgc tgctccacgg cggcggacag gaggagggca cccgctccgg ccccgaggcc 720
   accgcccaga tcgcgggctt tgccaaggcg gcggagctgc gcgctgacgg tctggcggac 780
   aagctggccc acatggcgga ggtcaaggcc tgctgtaaag agaagctcct ttccatcgac 840
   ggcgtagtgg ccgtgggtca gggagaagcg ccccatatcc tgatggtgtc gctggtgggc 900
   taccccagcg ccaacgtggt caccgatctg gggagccagg gcatctgtct gtcggcgggc 960
   agcgcttgcc accggggcaa gctgagccac gtggtgacgg cgctggggct ggacaaacgc 1020
   accgccaacg gcgtgctgcg gatcagcttc ggcccggaaa ccacctttgc cgagatcaac 1080
   gccctgtacg aggcgctttt gcggcacaaa aacacccgat ttcctatgct atga 1134
<210> 192
   <211> 1632
   <212> DNA
   <213> Unknown
<220>
   <223> Unknown gut bacteria
<400> 192
   atgtacgaaa acggcaagat ctatatgggt ctggcggacg gacagcgggt ggagctggct 60
   ctgaacatgt gcaaccgtca cggcctgatc gccggcgcca gcggcaccgg caagaccatc 120
   accatgaagg tgatggccga gtccttttcc gatgcgggcg tgcccgtgtt cctgtgcgac 180
   gtaaagggcg atgtggcggg tatctgctcg cccggcgtca gcaacgaggg catggaaaag 240
   cgcatcgaca aattcggtgt ccgcgatacc tttacctata aagcataccc caccaccttc 300
   tgggatatct atcaggaggg cggacatgcc gtccgcgcca ccgtcagcga catgggcccg 360
   gagcttttga gccgcattct ggggctgact ccggcgcagg aggggatcct gcacatcgtg 420
   ttccgcattg ccgacgacaa gggcctgctg ctgatcgacc tgaaggatct gcgggccatg 480
   ctgacctacg tgaacgagca ccgctcggag tacatgatga cctacggcaa catcacctcc 540
   cagtccgtgg cggcgatcct ccgggcgctg ctgccgctgg agcagcaggg cggcgacctg 600
   ttcttcggcg aaccggcgct ggacatccgg gactggatgc gcaccgacgc cgatggacac 660
   ggcatgatca acgtgctgga ctgcgtcaag ctggtgcaga accccaccct gtacgccggt 720
   ttcctgctgt ggatgctgtc ggagctgttc gagaatctgc cggaggccgg tgatctggac 780
   aagcccaagc tggtgttctt cttcgatgag gctcatatgc tgttccggga cgcgcctgcc 840
   gtgctgctgc agaagatcga gcagacggtg aagctgatcc gctctcgcgg cgtgggtgtg 900
   ttcttcgtca cccagagtcc cagcgatatt cccaacgccg tgctggccca gctctccaat 960
   cgggtgcagc atgctctgcg ggcctatacc cccgccgagc tgaaggccgt ccgggtggcg 1020
   gcgcaggcct tccgggccaa cccggccttc caggcggagg acgccatcat ggagctgggc 1080
   gtgggcgagg cgctgccctc cttcctggac gagggcggcg tacccgccat ggtgcagcgt 1140
   accaagatca tctgtcccca gagcctgatg gccgcaccgg agcccatggt ccgcgccaag 1200
   gtgctgatgc gggacggcat ggagaaatat gacgactacg tggacaatct gtccgcctat 1260
   gaggtgctga cggaggaggc caaaaaggct gagaccgcca agcaggagga ggccgaccgc 1320
   aaggcccgtg agaaggcgga ggccgaggcg gaaaaaaagc gtctgaagga ggaagaagcc 1380
   gacaagaagc ggcagcagaa gttggaggac gaggcgcgca agcgcccgca gaagctggac 1440
   gatgaagcac gccgccgcca gcacaagctg gaggatgagg agcgccgcaa ggccgagcgc 1500
   cagcaggaaa aggcggaagc cgaggccaag cgtaaggcgg agcgccgtgc agccaagatt 1560
   gaaacgcagc tgatctccgc cggagggcag atcctcaagc ggggtctgct gggcgtgttg 1620
   aaaaagagat aa 1632
<210> 193
   <211> 1347
   <212> DNA
   <213> Unknown
<220>
   <223> Unknown gut bacteria
<400> 193
   atgaaaaagc tactggctct gctactggct ctgctgatgc ttctgtccct tgccgggtgc      60
   ggcaagcagg cgcaggagga cgaggagccc gccgtgatcg agctgacgct gtggacgttc     120
   cccgcaggcg actggggcga tccggccgcc gtggagacgc tgctgaccca gttcaacagc     180
   gtataccccg acattcacgt gacggtacag tgtctggacg aggccaccgg cgacgacaag     240
   ctcagcaccg ccgtggacgg cggcgtgtcc cccgacattc tgctggccca gccggatcgt     300
   ctcatcaaag catggggcgc agagggcatg ctggccgatc tgtccgacat gtgggacgac     360
   gccgacaagc aggaggtcaa cgccgcctgc cagagcgtct gcttcacccg ggacggcaag     420
   tgctacgcct atccctttgc catgaacgtg cagtgcatgg ccatcaacta tgacgccttc     480
   gtcaccgcag gcgccgacca gtacatcgat ctggagacac gcacctggac caccgagcag     540
   ttcacccagg ccgtcaaggc gctgcaaagc aagtacggcg aggcggtggc cgccgtgtac     600
   tgctccggcc agaacggcga tcagggtacc cgcgcgctgg tgacgaacct gtacggcggc     660
   cagttcgcca acgactccta caccggctac gtcgccaact ccgaggccaa ccagcaggcg     720
   ctgaccctgc tgcgggacac caagggcatt gaatttgacg acgccatcaa tgggcaggag     780
   gaggccgcgc tgttctatca cgaatccctg aaaatgtcct tctgctggag tcttgcccag     840
   cagctggccg agacggtgga cgagcccacc gaggagggcg gcgagccggt ggtgcatccg     900
   gcgggcaaga ccgtcaacgg ccagaccatc gagttcatgg ccttccccac cgatcccgag     960
   atcagccagc cccggcttca gggcgatatc tggggcttcg gcgtgtttga caacggcgat    1020
   caagagcgga tcgaggccgc caagctgctg atccgtttct tcgccgacgg gcagggcgcc    1080
   agcgccgccg tgaaggccgc cgggtgcttc cccgtccgcg acacggtgga cggcgccgac    1140
   ctgcggaaca tctgggcggg cagcgacgtg atgacggaat acgccaagct gacggacttc    1200
   ctgtccggct atgacgcgct gacgcccggc tgggccaccg cccgtactga gtggtggaac    1260
   atgctccagc gggtgggcaa cggcggcgac gcggtgctgg aaaccgacac cctcgtggaa    1320
   aacgccaacg ccgcagcgaa ggagtaa                                        1347
<210> 194
   <211> 822
   <212> DNA
   <213> Unknown
<220>
   <223> Unknown gut bacteria
<400> 194
   atgcgtcctg acggcaagcg ggtcaaagac cttccgccca ttgtggcggc tattccctat        60
   atcatgccca agcggtatga cgcccagaac accattaccg agtatattga cgaggagctg       120
   atcaagtcct acatccggga aaagcgccgt accggcgtcc ggatcaacca tatggccgtc       180
   atcatcgccg cctactaccg ggccgtgatg tataacccca agctgaacta cttcatcatg       240
   aacagcaagg tatacaagcg gaaccacttc tgcgtgtcct tcgtgatcct gaagaagctg       300
   gcggacggac ggcctgacga aacgtccctg aaaattttcc tgacgccgga ggacaccgtg       360
   ttctccatcc agcagaagat caccgacgcc gtgtccgcca acgccgccac cgagcagaag       420
   aacaataccg acaagttcgc ccggatgatg ttctccatcc cgttgctgcc caagatcgtc       480
   atctggctgg cctaccatct ggataagctg ggtctgctgc cccggaagat catcgacctc       540
   agccccttcc ataccagcat gttcatcacc aatctggcct ccatcaagac cagctatatc       600
   caccaccatt gctacgagtt cggcaccacc agcgtgtttg tctgcatggg caagcctgtg       660
   ccggattaca tgagcggcaa tctcaacaag aagatcatgc cgctgggtgt ggtgatggac       720
   gagcggatct gcaccggcta tgaatacgcc gccttctgca acgatttccg caagttcctc       780
   cgcaagccgg aggagctgga aacgccctat caggcggaat aa                          822
<210> 195
   <211> 921
   <212> DNA
   <213> Unknown
<220>
   <223> Unknown gut bacteria
<400> 195
   atgcggatct tatttatggg cacgccggaa tttgcggtgg cctccctaaa agcactggtg        60
   gaggccggtc acgagatctg cggcgtcttt acccagccgg ataagccgaa aaaccgcggc       120
   cacaagctga cattctcacc tgtgaaggaa tatgccttgt cacagaatct gccggtgtat       180
   cagccgctga agctgcggga cggcacggcg ttggcactgg tgcaggagct gcacccggag      240
   ctgatcgtgg tggctgccta tggccgcatt ctgccggagg atattctgaa catgccgccc      300
   tacggctcta tcaatgtgca ttcctccatc ctgcccaaat accgcggctc ggccccgatc      360
   aactgggcgg ttctcaacgg cgaaaaggtc accggcgtga ccatcatgta tatggccgcc      420
   gagctggacg cgggcgatat catccgcgtt gcagagactc ccattgatcc agacgaggat      480
   gcccagaccc tgacggctcg tctggctctg ctgggtgctg acgcgctggt gcagacggtg      540
   cacgacctga cggccggtac ggccacccgt acaccgcagg agcacgacaa gcacacctat      600
   gcccctatgc tggataagag catgagcccc atggacttta cgaagcccgc ccagcagctg      660
   cacaatcagg tgcgtgggtt gattccctgg ccctgcgcga caatggagtt gaacggcgct      720
   agcgtgaagg tctaccgtac cgcagtgggc gaggagaccg ccgccgctcc cggcgcagtc      780
   gtggaagcca acaagaaagg cattgctgtt gcctgcggcg acggtaagct gctgcggatt      840
   ttggagctgc aagcacaggg cggcaagcgc atgtccgccg ccgcctatct ggcaggccat      900
   ccgatcccgg tctgcctatg a                                                921
<210> 196
   <211> 1269
   <212> DNA
   <213> Unknown
<220>
   <223> Unknown gut bacteria
<400> 196
   atggagcttc tggagaagct gacgatcctc tcggacgccg ccaagtatga cgccgcctgc      60
   acatccagcg gcgtcaagcg cagctttcag ccggggaaaa tcggcaacac gtcctcctcc     120
   atcgccgggt gctgtcacag cttttccgcc gacgggcggt gcattacgct gctgaaggtg     180
   ctgatgacaa actgttgcgt ctatgactgc aagtactgcg tcaaccggcg ctccaacgac     240
   gtgcagcgca ccgcattcac gccccgggag ttggctgagc tgaccatcgg cttttatcgg     300
   cggaattata ttgagggact gtttctctcc tccggtgtgc tgcgcaatgc cgactatacc     360
   accgaacaga tgatccgcgc cctgcgcatt ctgcggcagg actatggctt caacggctat     420
   attcacgcca aggccatccc cggcacgtcg ccggagctgg tgcagcagct gggactgttg     480
   gccgaccgca tgagcgtcaa tatcgagctg ccctctcagc agggattgca aacgctggcg     540
   ccggataaat cgcgggaagc gatcctgcgg cccatggggt tgatccggga caaggcgttc     600
   gagagccgtc aggagctggt caagtacaag cacgcgcccc gctttgcccc ggcgggacag     660
   agcacccagc tgatcgtagg cgctacgcag gacaacgacc ggcatatcct gcgcttgacg     720
   gaggcgctct atcagaaata ccggctcaag cgggtgttct attccgccta tgttccggtg     780
   gtggaaaaca gccttcttcc cgctctggat accaagccgc cgctgctgcg ggagcatcgg     840
   ctttatcagg cggactggct gctgcggttc tatgggtttc aggcctccga gctgctggac     900
   gaggcgcacc cgaattttga tccgcagctg gatcccaagt gcagctgggc ggtaacgcat     960
   ctggagcagt ttccggtgga ggtcatgcgg gcggattatg aaacgcttct gcgtgtgccg    1020
   ggcgtcggcc ccaccagcgc caagcgcatc gtctccgccc gccgcacggc gcaccttcga    1080
   tttgaagatc tgaaaaagct gggggtggtg ctcaagcggg cgcagttttt cctgctttgc    1140
   gatggccgcg ccgcgccggg actccatttt tcacccgcta ccctcgtgca gcagctgaag    1200
   gccgtggagc gcggcgcgct cccctccggg gaaatggaac agctatcgct gttcgacgca    1260
   gttggataa                                                            1269
<210> 197
   <211> 480
   <212> DNA
   <213> Unknown
<220>
   <223> Unknown gut bacteria
<400> 197
   ttggaaccca tcattcgtgt cgatcatctg acacatacct atagcgccgg aacgcccttt        60
   cagcgcagcg ccgtcaagga tatgtcgctg gacattctgc caggcgagtt tctgggcgtc       120
   atcggccata ccggcagcgg caaatccacc ctcatccagc acctcaacgg ccttttgaag       180
   cctacggacg gccacatcta cctgaacggg caggatatct gggccgaccc caagcagatc       240
   cgtcaggtgc gcttccatgt ggggctggtg ttccagtatc cggagtatca gcttttcgag       300
   gagacggtct ataaggacgt ggccttcggc cccaaaaaca tggggcttga cgacggggag       360
   atcgaccgcc gtgtccgcca gtcggtgcag gccgtaggtc tcagcgccga cgtgctggag       420
   aagtccccct tcgccctgtc cggcggccag aagcgccgcg tggccatcgc cggcgtcatg       480
<210> 198
   <211> 867
   <212> DNA
   <213> Unknown
<220>
   <223> Unknown gut bacteria
<400> 198
   atgaaacaga acgtattaca tacgatacgt gccagccgag acagcttctc caagggacag        60
   aagcggatcg ctgactatat cctgaacaac tatgacaagg ccgcgttcat gcccgccgcc       120
   cgtctgggac agacggcgca ggtcagcgag tccacggtgg tacgctttgt cgccgagctg       180
   ggctacagcg gctatcccgc catgcaaaag gccttgcagg agatcatccg gggcaagctg       240
   acctcggtgc agcggatcca ggcgtctcag gatcagatga ccggcaacga cattctggcc       300
   acagtgatgc agcgggatat ggacagcatc cacaacgcca tcgagcaggt ggatcgcggc       360
   gagttccagc atgtggtgga actgttgctg catgcccagc acatctatat tctgggggta       420
   cgctccagct catttctggc gggctatctg aatttctatt tccacctgct gttcaaaaac       480
   gtgatctatg tgcagaacac ggcggcgggc gagatctatg agcagatgat ccacatcggc       540
   cccggcgacg tgatggtggg catcagcttc ccacggtact ccaatatggg cattcacgcc       600
   attcagctgg ctcatgaccg tggcgcggat gtggtggcca ttaccgacag tcagctgtcg       660
   cccctgtatc cattggcgac ggcggcgctg ctggtgcggt gcgaggcgct gtcctatgtg       720
   gattcgctgg cggcgccgct gagcctcatc aacgcgctga tcctggcggt aggccagcgg       780
   aagcgggacg aggtggcgca ggtgttcgcc gacatggagc aggtgtggtc aaaatacagt       840
   atttttggaa aagcggaaga tgaataa                                           867
<210> 199
   <211> 1632
   <212> DNA
   <213> Unknown
<220>
   <223> Unknown gut bacteria
<400> 199
   atgagcctgt acgcacgtct ggcgtgggac ggcatccgga aaaataagcg actgtacctg        60
   ccctatatcc tcacctgcgt gggcatggtg atgacgcatt atatgatgag cgagctgtgc       120
   gccagcgacc tgctgcggta catgaagggc ggcggcgcgt cccatattat tctggggctg       180
   ggcagctggg tcattgcggt gttctcggcg ctgtttctgt tctacaccta ttcctttttg       240
   atccggcggc ggcagcggga gttcggcctg taccacgtgc tgggtctggg caagcggcat       300
   ctcgcgctgc tgtgcgccat tgagacgctg ataacggcgg ccgtcgcgct ggtggcggga       360
   ttgattctgg gcgtggcgtt gtcctttgcg gtggagatgg gcctgcgctc caccgttcag       420
   gcggagatgg acttcacctt tgcggtgagg ggcagcgttg tggcgcagac ggcgctggtc       480
   tatctggcca tctttctgct gacgctgatc agcacactga taaaggtggg gcgggggagc       540
   gccgtctccc tgctgcacag cgagaattgc ggcgaaaagc cgcctaaagg caacctgttt       600
   gtggcgctgc tgggtgcggt gctgctggga atcgcctact ttatggcggt gtccattcag       660
   gagccgatga aggcgctggc gctgtttttt attgcggtga tcctggtgat tttggccacc       720
   tatctgctgt tcatcgccgg aagcgtggcg ctgtgccggg tactgcaaag gaaccggaat       780
   tactactatc agcctcggca tttcgtgtcc gtgtcctcta tggcctaccg catgaagcgc       840
   aacggcgcgg gactgccctc caccagcatt ctggccacca tggtgctggt gatgctgtcc       900
   tcctctgcct gtctgtactt cggcgcggag gacagtctaa aggccacgta cccccacgag       960
   tttgcactgg aggtgtggct ggagaacagc gctgacatgc cgaaattgca ggaggctctg 1020
   accaaagcgg gcgcggccta tggcgtgaac gtctgggaga agggcactgt ggaaccgggg 1080
   agcggcaata cgtggagcgc cgcctttgac accggccttt cccaagagca gcagctggcg 1140
   ttgagccgcg ccattgacga ggcgctgggt gacacggaca ttacgtggaa ctacttccgc 1200
   tcctaccggg cggaagctgc tgatgatttt tacggcacct acggcagcct gttctttatc 1260
   ggcatcgtgc tgagcgtgct gttcatctgc gcggcggtgc ttatcatcta ctacaagcag 1320
   ctatgcgagg gatacgagga tcaaccccgt ttcgccatca tgcagaaggt gggcatgacg 1380
   gcggcggata tccgcaggag cgtcaattcc cagctgctga cggtgttctt cctgccgctg 1440
   gcgggcgcgg cgctgcacat ggcgttcgca ttccccatga tccggcggat gctgctgctc 1500
   tttgagctac acaacacggg actgttcatc gccacggcgc tggtcagctt cgcggtactg 1560
   gcggtggtgt acacgctggt gtacaaggcc acgtccaacg cgtattaccg gatcgtgaag 1620
   ggtagcgtgt ga 1632
<210> 200
   <211> 1332
   <212> DNA
   <213> Unknown
<220>
   <223> Unknown gut bacteria
<400> 200
   atgcgtattt gcaaggagag cgtcactatg agcaccgtca tcacgtctat cgacccccat        60
   tccccggcgg aaaaggccgg aatcgttgtg ggggagcagc tgctggagat caacggccac       120
   cacatcgtgg acgtgctgga ctaccggttt tatggctatg accctgtggc ggtgttgcgt       180
   ctcagcaagg acggccacac ccgcaccgtc accatccgca aggaggaggg ccgtgacctg       240
   ggcctgaact tcaacaccta tctgatggac gagatgcacc agtgcgccaa ccactgcctg       300
   ttctgcttcg tggatcagat gccgcccaac atgcggccgt ccctgtacat caaggacgac       360
   gacgagcggc tcagcttcct gctgggcaac tacaccaccc tgaccaacct ctcggagcgg       420
   gaggcacagc ggatcatcga cctgcacatc agccccatca acgtgtccgt ccacgccacg       480
   gagccgcagc tccactgtac gctgctgggc aacaagggag cggagcggtc gctggaatac       540
   atccggcgat tctgcaaggc gggcatcgtc atgaacggcc agatcgtggt gtgccccggc       600
   tggaacgacg gcgacgctct gcgtcgcacg ctgcgtgacc tgacggactg gcagttttcc       660
   agctgctcgc tggtgccggt aggcatcacc aagtaccgta agggccttgc caagctgcgg       720
   cctgtggaca gcgaatgcgc gcgggagatc atcgccattg ccgaggagta cgggcaggag       780
   aacctgcggc gctacggcac ccggcggttc ttctgcgccg acgagctgtt cctgcgggcg       840
   ggactgcccc tgccggagga ggactactac gagggctacc gccaaatcga aaacggcgtg       900
   ggcatgctgc ggtcgctgga gcaggagttc ctgtccgcca tgaagctgga ggagccggac       960
   gccgcgccca gccccttcac catcgccacc ggcgtggccg ccgcgccctt tatgcagacg      1020
   ctggtggacg ccgccaaagc gtacttcccc aagcttgacg cggaggtggt ggccgtggag      1080
   aacgacttct tcggccgcac catcgacgtg gcggggctgc tgacggggca ggacatcgcc     1140
   aagcagctgc aaggccgggt cactggccgc cgggtgctgc tgacggtgca tatgctgcgc      1200
   cacggcgaaa cggtattttt agatgactat acggtggaca ggctttctca ggaactgggc      1260
   agccccgtcc agatcgtgga cgacgacggc ggctcgctgc tggacgccat gctggaagcc      1320
   accgtgggat aa                                                          1332
<210> 201
   <211> 414
   <212> DNA
   <213> Unknown
<220>
   <223> Unknown gut bacteria
<400> 201
   atgcttgatt acgatctgac ccgcgcagag caggtcagaa gaacggatct tgcgcaaaaa      60
   gcccgcagga tgatcctgtc ccacatgaag gtggaggcgg aggacgaaac ttcgctgacg     120
   ctggatcacc gcggctacta tatgcagatc tcgttctcgc cgctgcaccc gctgctggtc     180
   gtctgcctgg ctaaggcgat ccgaaacccg gatggcgcac gaaaacagca gcttgccaac     240
   gagctgaacc tgcacagcgt gttgggcagc cacgccatca acgaggacgt cggctgttat     300
   tcctatcgcg ctacccactg gctggatacg gagctcacgc cggaacggtt ctttgaaata     360
   ctcagccgct gcgttgacga agcggatcgt ggctatttca atctggcagg atga           414
<210> 202
   <211> 1131
   <212> DNA
   <213> Unknown
<220>
   <223> Unknown gut bacteria
<400> 202
   atgattaccg ttcggccggg aagtcatgtg catcggctta tcacagtgct cgggctcgcc      60
   ggcgaatacc ctgtccgttc gcttggcgtg ctgggaaacg agcgtactct cagagcgctg     120
   gtgagtaaac tttccacaac acaggagctt cgtaatccgg ataccggcga gcggatgagg     180
   gtaaagctgc tgcagctgac cggtactggg aatgccaaag cgattcgttt ctgtaagggc     240
   gctctcccca tactggaatg gatacatccg gacgcatacg gctattatat ggccgcgttt     300
   tacaatcata gattccccgg cggtatggcg catcgggacc gtaaccttcg ggttgccgag     360
   acgattggga tgcacctgac cgccggtatc gagacaaggg cctatctact ccccgccttg 420
   caaaaccggg ctatccttcg gataacaccg gacgcgcctg ccttctatct ggcgcgggat 480
   ttcaaaaaga tcaccccagc ggaacagaac aaaaccatgt tcacacgcat cgtcggcgca 540
   atcttctatc ccggcggctg ttacgccgtt tacaacacac ggaacgccgc gatgaaatgg 600
   aacggcatgg gagagttcaa ggcgctccac agcctgacag agcttgcccg catgaatgcc 660
   ggggttcaga gcatcgactc cgcaatcctg ctgggcgagt cctatgatac ggcgctgacc 720
   acactgctgg agtcggataa aaaccggcga ctggaacttc gctttgacgg catctatcgg 780
   catatctact ttgtgcccat gaatgccggc ggcatccggc agttcagatt gatcaccgtc 840
   ccagactgga aagaaaagct gcttgagctg ctgttcgacc cagacgtccg gtcatataac 900
   cggggattta tggaatacga cgccagtatc ggcggcacct gtgttttctc gcatcttgac 960
   ggcgacatcg cacgcctaat ccgttttcgt gaggcaatgc agaccggcgc agggagcttc 1020
   gaggtactct gcttcgatga tcaagcgccc tttctaagag agtaccttgg tccgcatatc 1080
   atactcaaaa ccatcgacgc tgccacagta gaagcagaac tgggactata a 1131
<210> 203
   <211> 615
   <212> DNA
   <213> Unknown
<220>
   <223> Unknown gut bacteria
<400> 203
   atggcgcagt atctgaccac acattttcgc tatccaacat taaactcctg gaacagatcc 60
   acatcgtatg cttgtaacct aaaaatcaca caccttgggc tctcgcccga ggtggtggac 120
   aagctgttcg atatgatcca gacccaggag ttttttgatg ccatggacga gtgtaaatgg 180
   gaatttgctg cgaaacacaa ctatctctgg caggcagtga tgaacggacg aagcggcggg 240
   tatctggttc tctatcaggg agaaaagcac ccgagcggct acaagtccta ctgcactaac 300
   tgcggacaga ggaattacca gcttgctgca gatggcaact gcacctgcgg tgtctgcgga 360
   aggcccactc gggtcaattt ctcgcagacc cacatgcagg tggtaactta tccggggcgt 420
   ggcactgacg acggcgagga tttcgaggac tggagcatgc acgcgcttcg tgaacgtgta 480
   aaactcgtgc aggagctgga tcaactggca gaccgtatgg ttgagctggc gctccggctt 540
   acaagagaag cacaggtcat tgaagaggag tatttccttc cgcagacccg caaggtgctg 600
   gtcacgaccc tataa 615
<210> 204
   <211> 753
   <212> DNA
   <213> Unknown
<220>
   <223> Unknown gut bacteria
<400> 204
   atgcaaaaca aaaaattcaa gcgccacatc gcctatgagg ttttgaccat actggggaca      60
   ttggctctgc tgctttttgt ttgcaggctt tggccgattc tcttattggt gatactcggt     120
   atttttatcg ccgccatgcg actgctcttt ctctctgcca gaaaagtgga gaatatcccg     180
   cctctgcttc cggctgctcg cccagaacct acggatcagg atatccggga catggcgttc     240
   ggttcaatgc agcggcggat tacagagctg gtcacggccg atttccccaa cgccagatgg     300
   gtatggaaga cacctaatgc catacgcaat attgcgacag gcaacgaggt gcgaatcctg     360
   ctcaaccatg ccggcggata ccgtgaagcg atcgttgtga ttcgcggcct tcaagtgtgc     420
   agcctgacct acccgcacgc ggaagaagtc aagatgccgg aaacggaaaa tgatgagacg     480
   ggcacagagg aaactgagaa tgacactccg cagaaggtcg atcccccgga gaattatgag     540
   tatctggctt ttgaatgggt ggacgcccat gttatggaac tcaatgagcg gtgcaacgag     600
   agcatcgcgc aagggctatc catgctggaa ataacggcgg cagaactgcc tgcacgagaa     660
   agctggccgg acatctgccg ggagctggaa cgaaacggta tggagaactg ctgctgtaca     720
   gataccggta ttaccgttga tttcacgcag tga                                   753
<210> 205
   <211> 1134
   <212> DNA
   <213> Unknown
<220>
   <223> Unknown gut bacteria
<400> 205
   aaaatgagtc attttgtaac actggtcaat ttttatatgc ccgagctgga ggaaaactgc      60
   gaggaaaata tgcgttatgc ggaacagatc gcagaagtca aagaaaagct ggcgcaggac     120
   ccggagagtt ttgctttgcg gtttcttctg aaacgacttc aaagcaaggc cagcactctg     180
   gaacgttcgg ctgagtgcga gattgatgag ctgatggccc cattctgtga aggaacggat     240
   gacccggcct atctggagtt tgaggatcga accgatgacc tgcggcggga ttatgagacc     300
   gacaaaatca actgtgtccg ttttccggac ggaactgtcg ttccggagta caaccggctg     360
   ttatacgaaa aatatctcat caaagatggc aaggtgttcc agaaaaaggc cgggcacctc     420
   ggccatgaga agcgcacgaa gaaagcgaaa aagacgcgcg catttatggg atatccggtg     480
   aagaagctct atcccagtct gaagcagtat gcggaggact actgtggtta tacctatgac     540
   agcaagaaca atacgtatgg gtattactgc aatccgaatg ccttctggga ttggtattcc     600
   attggtggcc gctggccgtt ccaattcctt gtccgtgaca cagcggaacg aattaacggt     660
   gagcgcacat ggggcaacga agatgctgta tgtaaagcgc ccgaaggcta tatatgggtc     720
   tgcggcgcaa gaaaaaggga tattgcatgg gatctcatga aggaatggga gctacagcac     780
   gccaaaaagc gctttaaact tcttgcagag acttttcgtt ccggcaaggc tccggaaggc     840
   agcttctgga agatcacgga ggatggagtt ttctcttttc tgacgcagat ctatttcaag     900
   aatgaaagcg aggatgccta tctccgccgc aacggactcg caccggatca gcgcatggtg     960
   cccggcgcct attccttcct tcaggacgga gactggcact ccaagggaga tatgggctgg    1020
   tggggtatca gcagcaacga taagaaaccg gacgcatgga ggcagatgtt ggcagactac    1080
   atagacagca ttcctgacga tcatttcatt gtcgggattg actgccatat ctaa          1134
<210> 206
   <211> 372
   <212> DNA
   <213> Unknown
<220>
   <223> Unknown gut bacteria
<400> 206
   ggttccgttg tgaagcggga tgggaaatac cgtttttcgc tgcagttcgg ttcggaaacg 60
   cgagaacagg tgcaggcagg agagctttta gagcgtcttg gaaaccgaaa aagcgcagtt 120
   gtcatagccg cactgaatgc atatattgct gcacatcccg agcttgctga acctgcgccg 180
   gtaagtgtta aaattgacgg cgggataagg cgtgaggcgc ttgagcaaat gattcgatcc 240
   attattgacg agcgaatcgc aaacggtcag ttgcttgccg gaggcgttga aacaccatct 300
   atacaaatgc aggaagattt gaacgtggac attgcggcaa tgatcagtaa tctggagttg 360
   tttgatggtt ga 372
<210> 207
   <211> 516
   <212> DNA
   <213> Unknown
<220>
   <223> Unknown gut bacteria
<400> 207
   atgacgctta cacaacccca gcagagaaaa gtggagcaaa acctcgggct ggtcgggaag 60
   gtgatcaagg acaaggtaca taatcccggt caaaacagta tctacagtta cgatgacctc 120
   tatcagatcg gctgcattgg cctctgcaag gcggcataca gcgataaagg cggatgcttc 180
   tctacgtatg catacaggct aatctggaac gagatatgca cggcgttgat ctatgccaac 240
   cgccgggcgg caaaggagtg cgaactgatc ccggaggtgc tcgggaaaga agatagttta 300
   gacgagcatc acgaactgag tatgatactt gaccgactgg aggccaacgc ttccgaggcg 360
   atggcgaaag gcatccgggc gatccgtctc agcgccgaag gctattccca cgcggaaatc 420
   ggcagcatac tcggggaaag cgacaaatgt gtcgccgccc gaatctcccg aaccaggaaa 480
   tatctcaggt cgcatccgga ccttgcggca ttctga 516
<210> 208
   <211> 1806
   <212> DNA
   <213> Unknown
<220>
   <223> Unknown gut bacteria
<400> 208
   gtgcttggcc aattgctgat aaactacgag ctttggatga attccggcgg tcttacaggg 60
   caaacgacca tcggcgccgt agactggaat ccgatcgtgt gtctgggcag cgccttcagc 120
   atcccggggc taaaaagcat cggtatactg ctgctgaccg gggtcggtat cacggcctat 180
   gtgcgtttcc acgataaatt cagctccaag gactacgatg agcgcggatt tacccgcagc 240
   aagtacggaa cctacggcac cgcatcatgg atgaaggaca aggaactgaa agaaattctg 300
   gagatcaagc ctccgtcgca ggccgatggc atcattttgg gcgaaaaaaa cggcagcgtc 360
   gtctgcttac ctaaagacac cagactgaac cgccacatcg ccgtattcgg tgcatcaggt 420
   acccggaaat cccgtggcgt gatccgtccg gcgctgttca ccatactcaa gcgcggagag 480
   tcggcagtca ttacggatcc gaaggcggag ctctataacg ataccgccga gttgttccgc 540
   aagaacgggt atgaggtaaa agtattcaac ctggtagacc cccggcacgg cgactcatgg 600
   aactgcatgt cagacctgaa cggcgatact ctgctggcgc aggtgttgac caatgtcatc 660
   atctccaaca ccagtgaggg caagggcgac catttctggg acaacggcga ggccaatttg 720
   ctcaaagcgc tggtgctcta tatcgatctg gacaggagcc gcagcccgga gacgaagaac 780
   ctggcggccg cctaccagct cctgacccaa aacagcgaac ggcagctgac ggcacttttt 840
   gaaaagctgc cgctggatca ccccgcccgt gcgccgttca atcttttctc gcaggccagc 900
   gacacggtgc gttccggcat cgtccttgga ctcggcaccc ggttacaggt cttacagaac 960
   gaggctgtgc gggacatcat cagccgcagc gatatcgatc tcacggcgcc gggcaagcgc 1020
   aaatgcgcct acttcgtcat actcagcgat caggacgcaa cgatggcgtt tctgtcctcg 1080
   ctgttctttt cgtttctatt catcaagttg gtgcgctatg ccgacagcac gccggagctg 1140
   cgctgcaaag tcccggtcaa cctcatcttc gatgagttca acaatgtggg caagctcggc 1200
   ggcgcagcgg acggttcgga tttcgcccgt acgctcagcg ttatccgcag ccgtgcgatc 1260
   tatgtgatgc tggccgtgca gagcctcggt cagctccaga accggtatcc caacaatctc 1320
   tgggcagaga tcgtcggcaa cctggacgtg cagcttatgc tgggctgtac cgatgaggtc 1380
   agcgccgaat acttctccgc gcggagcggt gatatgtcgg tggagatcaa ctccaccatg 1440
   accgtgcgca agaccattgc cgtagcgcag gtcatccccc aataccgtca gacggagggc 1500
   caggggcgca gacggctgct gactccggac gaggtgctgc gcatcccaaa cgaggaactg 1560
   ctggtggtca tccgaggaca caatgtcctg aaactaaaaa agtttgatta tgctgaccac 1620
   ccgctggcca aggaattgac tcccgtcagc attcttgact acacaccccc gcacgcggca 1680
   gcgccttttc tccagaccga aacgacttcg ccacacgcag cgcctgagga gcggccgcac 1740
   acatcgtcca taccctcaaa acgcagaact ctgtacagct ccgcaaaacc gccgagcgag 1800
   ttttga 1806
<210> 209
   <211> 1056
   <212> DNA
   <213> Unknown
<220>
   <223> Unknown gut bacteria
<400> 209
   atggacatct ttcatatgtc agatatcatt ccgctgattg ggcttccata cccgccatac      60
   ggaagaagca actacaatgt gccctgcccg tgttgtgatg acgagccgca cgcaaaacat     120
   ctcaacatca atctgcagaa ggatgtattt cgctgtccgc gctgtgggtt ctccggcggc     180
   attttcgacc tgtatgcata ctatacgggg attgcccgcg aggaggtccg tgatgctctg     240
   attgcacgct tggatgtgca gggcagtata ccgaagccgg agcacattcc tgtgcccgtg     300
   gttgcagaag tcccaccgac agatatttta tcacgaagcg acacctatac cgggcttctg     360
   tccaagctga cgctggcctc cgaccatcgg cagaacctgc gaaaccgggg cctgtcagat     420
   gaggaaatcg acaggcttgg gtaccgtacc actccggtgg tggggatgca aaccattgca     480
   aggcagcttc aatccagcgg atattatctg tccggtgttc ccggctttta ccgaaaagac     540
   gggaaatggt cctttgcctg tgagagccgc ggtattctga ttccggtgcg agacagtaaa     600
   ggccgtattc aaggcatgca gattcgccgg gataatgcga cgcggcgcaa atttcgctgg     660
   gtatccagca ccggccgcac agacggctgt aaggcagagg gttgggtaca tattgcgggg     720
   gaaccgcgcg ctatggtgct gctcacagag ggccccatga aagcggatgt catccattac     780
   ctgacaggac agacagtact cgcagttgcg ggggcaaatg cattgaccca attggagctt     840
   atcctgccgc agctgcatga gcaaggtgta gaacgtatca tgactgcatt cgacatggat     900
   ttcatggaga acccccatgt gcaaggcgga tacaggacgc tggtctcact gctttcggat     960
   gcaggctttc gctatggcac ctatctgtgg gatccccgct acaaaggtct tgatgattat    1020
   gtttgggagc actgcttcca gcggcaatta tcttag                              1056
<210> 210
   <211> 609
   <212> DNA
   <213> Unknown
<220>
   <223> Unknown gut bacteria
<400> 210
   ttacaggagg tcattatgaa aaagatgaaa acaggcatcg gcgtaatgct ggcgctattg        60
   tgctgcatcc tcatgtgtac aggtgttgtc tatgctactg gcgatgacac ggacggcacg       120
   gagcttcaag tcgcgcagcc cgtcatcctt gaggtacagc ttggccccca atgggcgggt       180
   gttgaattcc agctcaagac ggatgcaggc ctttatccgg gcacgattgc cgtggggcag       240
   gacgggattt tacgaaccga gctgggcaat agcggcagct acgtcctttc ttgcctgact       300
   ctaaatgccg ccgtgccggc gccgggcgat atcccaagtc ctgatgtgac gcaacaagaa       360
   caggagccgc tgacaacaga cagtgcagag aacgcggacg ccgcttcgca gaatgtgcaa       420
   gaacaggatt cgggccgtcc taccgtcggc gggattcccg tgatgcacat tatcctgttt       480
   ggcggagggc tccttcttgc ggcaggttcg ctggtcgcca tgcgctttat tcggaaccgg       540
   cgcccatcag aacgctcgcc gtatgatccg gacgatgagg acgatgacga ggatgaatac       600
   gaatattga                                                               609
<210> 211
   <211> 2115
   <212> DNA
   <213> Unknown
<220>
   <223> Unknown gut bacteria
<400> 211
   atggacgcgg cggggaatgt ggtttctgag atcaaggcgg tgggttattg cctgccgacg        60
   gcagataatc tgcgctatga tatgcgcgga cgttggagcc ggagcgccaa gcatggcatc       120
   caatttgaag tcgagggctt cgatgaagtg attgtaccca gcaaggaagg cgtcatcgct       180
   tacctgtgct ccgggcagat caaggggatc ggccctaaaa ccgcggaaaa gatttatgcc       240
   atgtttgggc agaacacgct gaccgtgctg gatcatgagc cggaacaact gcttgccgtg       300
   tcgggcatca gcaaaacaaa gctcaaaaag atttgcgact cctaccttgc ctcccgcggc       360
   gcacgcgatg tggttgcctt tctggcgcca catggtgtaa cgcctaacag ggcggtcaag       420
   ctgtacaagc agtacggaga gcataccatg gacatcgtca gaaatcaccc ttaccgtctg       480
   tgtgagatgg tgggcatcgg ctttaagacg gctgaccgta ttgctgtcag catgggcttt       540
   gaccctcttt cgccggaacg tgtggatgag gggctgatat tcacgctgac cgatgcagag       600
   ctgaaaggac atctgtgtat ggaaaagcac agctttctca agcaatgtct gaagctgctg       660
   gataccgaag ggctgacgga agagatgctg gcggtacggg cttcaagaat gcttgagtcc       720
   ggccgcttgg cctcctacga caggcaggta taccgcgcag tcacagcgcg aacggaacag 780
   agccttgcgt gggccgttta cagaatgctt acttatgaga aagcggggtc tcacgtcaac 840
   attgatacgg agatctccgc agaagaaaaa aagcagggta tacagttggc ttccgaacag 900
   cggcacgcag ttacgacagc cctgacatca cagctcagtg tgattaccgg cggacccggc 960
   accggcaaga cgctgatcca gcgattcctg ctggatatct atcgcaggaa aaaccgcgga 1020
   gcaaaaatta tctgctgtgc gcctacagga cgcgcagccc gcaggatgga gcaatcgacc 1080
   gggttcccgg ccactaccat ccacaaagcg cttgggcttc tggccggtga ggacggcgac 1140
   tactgtgaac cggagatgct ggatgcggat ctgattgtag tggatgaggt gtccatgatg 1200
   gatatctatc ttgccaacca tctgttccgc tccgtaccgc acggcagtca gcttgtgctg 1260
   attggcgatg cggatcagct tccctctgtc gggcccggcg ccgtgctgag cgagatcatc 1320
   gcctgcggcg ctgtaccggt agtgcggctg gatcgcatat tccggcagag ctatggtagc 1380
   cgtatcgccg ctaatgcaaa gctcatccga cacggaaatc ttagcttgga atacggcgag 1440
   gatttccagt tctatgactc ctgtgacatg agtgcttccg cacagcagat cgagacactg 1500
   tacctacagg aaaccgccag atacggtgtt gacaatgtgg cgcttctaac cccataccgc 1560
   caaaaaacgg agacgggcgt gaatgcgctg aacgagcggc ttcgggaaaa gctcaatccg 1620
   caggacggga aaaagccgga ggttgcatac agaaagcgga tataccgttt gggagacaag 1680
   gtcatgcaga taaaaaactg cgaggacatc agcaatggtg atatcggtta tatcacatcc 1740
   gtcacccgcg atgacaccga cagcgtgctg actgttgact tcggcgatgg ccgaattgcc 1800
   gagtatgacg gcagcgacct tgaaatgctg gatctggcct acgcctccac catccacaag 1860
   tcccagggca gcgaatataa gtctgtcatc atcaacctgc agtgcgccca tgcggtcatg 1920
   ctggtgcgtc cgcttgttta tacggcaatt acacgcgcaa aggagcgcgt gctgatcgta 1980
   ggagagcgga gagcattatg cattgccatc aaacgtacag atacggaaca gcgcgggacg 2040
   atgctctcgg cgcgaatcaa agagctaatc tcaaatgtga aaggagatca ttacaatggc 2100
   aacttggctc agtga 2115
<210> 212
   <211> 3114
   <212> DNA
   <213> Unknown
<220>
   <223> Unknown gut bacteria
<400> 212
   atggccaaag caaaaggaaa aagaaaccgc agaaaagaaa agcttgagtt tgtacaggat        60
   tatttgccca tccgcgacct gaaaaacggg atcattgaaa cgacggacaa ccgctacata       120
   aaaatccttg agatcgagcc gatcaacttc acgctgcgat ccgatgagga gcagttcaac       180
   atcatctcca gctttgccag ctggctgaag atatccccca tgcggctgca gttcaagtcc 240
   attacgcgcc gcgcggactc ggataagcac atatccatga tctgcgagga attggaacac 300
   gaggagaatc cgcaatgtaa ggagcttggc gaagattata tcggactcat caaggatgtg 360
   ggaaatcggg aagcgctgac gaggcgcttc tttctgattt tccagtatga ggcggtaggc 420
   cgcaacgaaa gcgacgacta tgcaaaaatc tatggcatgc tccagaccgc cgagcagaca 480
   gcacgggcgt atttcatgca atgcggcaac agcatcgtcc agtccaaaga cccggatgaa 540
   gcgactgccg aaatcctata catgttttac aaccggcgct cctgcacgga cgagccgttt 600
   tcctcccgtg tagatcgcat cgtggtggac accatggcgg caaagaagaa aatcatcggc 660
   atggacccgg tgccgcctat ccgtatggcc aactttctgg cgccccgcgg catcgacctg 720
   agccattata actatgtcgt tatggacggg ctgtactatt ccttcctcta catcaaggcg 780
   ggcgggtatc caagccgcgt ccgggccggc tggatgtcct cgctcatcaa cgccggagaa 840
   ggagtggata tcgacgtcca tctccgccgt gagaaccgca gccgcaccat agataaggtc 900
   gcccagcgga tcaggctcaa ccgcaccaag ctcaagagca tgcaggatac cagcaccgat 960
   tatgaggagc tgaccaattc catacaggcg ggctatttta tcaagaatgg aatcgccaac 1020
   tacaacgagg acctctttta catgagcgtt ttcatcaccg tatcggccaa aacctacgag 1080
   gaattgcttt ggcgcaagca gcagatggtg gatatgctca aatccatgga tatgtacacc 1140
   agcgagtgca gctttcagca ggaggccgca ctgcagtcgg tgatgccgtt cctgaaaatc 1200
   tcgcccaagc tggagaaaaa ggcgcagcgc aatgtgctga ccagcggcgc cgcatccagc 1260
   tatatcttca ccagctttga gctgtcggat gacaatggcg tgctgttggg catcaatcga 1320
   tacaacaact ccctgtgcat cgtggatctg ttcaacacaa agatcaataa aaatgccaac 1380
   ctgaacctac tgggcacatc gggcgcaggc aagaccttca ccatgcagct gctggcgctt 1440
   cggatgcgga tgcgcggcat tcagtgctac attctggctc ccatcaaggg acacgaattc 1500
   cgtagagcct gcaataagat cggcggcgag tttatcaaga tcgcaccggg gtccccccac 1560
   tgcatcaatg taatggagat acggcatacc atgtcgccgg agatggagct gattgacgag 1620
   atcgattatg tggagatggg ctccatgctg gctcgaaaaa ttcagcagct catgaccttc 1680
   ttcggcctgc tgatccccga tatgagcaac gaagaggaac agatgctgga tgaagcgttg 1740
   atccgaacct atgcggactt cggcatcacc catgataacg actccatcta tacggatatg 1800
   gcctccgctc cgcccaaaat gaagcagatg cctatcctgg gcgacctgca taagcacctg 1860
   caggagaatc ccatgaccca gcggctggcg gccatcatca gccgttttgt caccggcagc 1920
   gcccaaagct tcaacagaca gaccaatgta gacctctcta atatatacat cgtacttgac 1980
   ctgtcggagc tgaagggcaa gctgcttccg gtgggcatgt tcatcgcact ggattatgtg 2040
   tgggatcaga taaagtccga ccgcaccaaa cgcaaagcca tctttatcga cgagatctgg    2100
   cagctgatcg gcgccggcgc caaccgcatg gccgcagagt tttgccttga gatattcaag    2160
   gttatccgcg gcttcggcgg agcggccatt tccgcaacgc aggatctgtc ggatttcttc    2220
   ggtctggagg acggcaagta cggcagggcg atcatcaaca actccaagaa caagatcatt    2280
   ctgaacctgg agccggacga ggcccgctat gtacaggaaa cgctgaagct cacccgcacg    2340
   gagatccgcg ccatcacccg cttcgagcgc ggcgaggcgc tcatcagctc caacaacaac    2400
   aaagtgcccg tggtggtgaa ggcctccaag ctggaaaagg aaatgatcac cacggatcgt    2460
   gccgaactgg aggcaatcct caaagaacgg cagagggaga atacgcattt cgcaaagaat    2520
   acgcacacac agaaggaggt gaacaatatg ctgcttgatg atgagcgcca tgtgatcaaa    2580
   tggttgtcgc agtacggcgc actgaccaaa acacaggtga tccgactgct gcgggacaaa    2640
   accccggaga cggcggagaa gattcttcgc aatctgaaaa gggagcatca aatctcggat    2700
   gtggccggtg gctactatct ggctttggat gggctatgca agccggatca gcggacgatc    2760
   cttgccgtat gggtgctgct gcgctttatc gactatgtgg atcccatggc gcattatcct    2820
   gcggtctacc cgtcccaaat cttttttctg aaggaaaaca ccggctatga gatcgttgtg    2880
   ctgtatgacg gcgagcagca ccttagcaag ctgctgcagc cgcaggatga cgagatgaaa    2940
   tatatctttg tcctgcccaa tatcgccatg gcgtcccaga tgatgctccc caaggcgccc    3000
   tgcctgtttg caaccgtcaa ctttgccggg caggaagaac cggacattac attttacaca    3060
   gaggaggctg caaccgatgg actcgataaa ctcgttcatt catctgctgg ataa          3114
<210> 213
   <211> 936
   <212> DNA
   <213> Unknown
<220>
   <223> Unknown gut bacteria
<400> 213
   atgggaggta ctcttatgtt gatcgccatt gatcatggaa attatgccgt taaaaccccg 60
   catttctctt tcatttccgg gctgacagaa cacaccgtaa aaccgcctat ggcagaggag 120
   gttttggagt acaacgggaa atactggtcg ctcaccgggc agcgcttgcc ctatatgaga 180
   gacaagacca gagatgatcg atactttatc ctgtcgctct ttgccattgc taaggagctt 240
   tccgctgccg gtgccaatac ggcgtttgag cagatcgacc ttgcggttgg cttgccgccg 300
   gaacactacg ggatgctgag agaacgcttc gcgcagtatt tcaggcggtt tggaacagtg 360
   aattttgctt acaacaaccg tcctatgagc attgtgatcc gaaatgtctt tgtgtatccg 420
   caggcgtatg ccgctgttgt tcctcaaagc ggacagctat tgaaaacgct gcggatgttt 480
   atcgtggata tcggcggata tactaccgat gtgcttctcc ttcgcaacgg aaagccggat 540
   ctgcaattct gccgcagttt agagagcgga gtcatcacca tgaacaatga tatcattggc 600
   aaaatcagcg cgcagcacga catgcgcatt gaagacgagc atatctgtgc agtgctgcag 660
   gggcaggaaa cgatactgcc agatgaggta aaaaaggcga ttcgtgatgc ggctcagctg 720
   cacgcaaaag atattttggg caagctgcgt gagcttcagg tggatttgcg taccaatccc 780
   gccatcttta ttggaggcgg aagcgtcctg ttcagaccgt tgattgagca taccccgctt 840
   gtggcgaagg ctaccttcat cgaggatcca aaggcaaacg cgatcggtta tgagatgctt 900
   gcgcgaggac agatggccat gcgcacggta caataa 936
<210> 214
   <211> 1881
   <212> DNA
   <213> Unknown
<220>
   <223> Unknown gut bacteria
<400> 214
   atgagcgtat caatcaacaa tctattcaac tactcccgtt cgctgcccgt cccctttgat      60
   accatgacaa ataagaaggt caaggtggca tccatgtacg gggcaaagac ggaatcaacg     120
   ctatgtggtt ctgtgatcaa ggcagtccac gccatgtgcc ggtgcatgaa cggcaccggg     180
   gaaggtgcgg tcggccagat cgatacaaac aaaagcgtgg cggagtataa atcctccgtc     240
   ggtccggatg cctatcatct tgtggtgttc gatgcagcct ccggcagcgc tttggcaagc     300
   gtttatgaca aaaacaccga gctcatcgag cagtacgtgg cgcatccgag ccagcgggac     360
   ggcgcagcaa tcttctttgc cctaatgccg ttcctgatgt cagatgcgga gttcgatgaa     420
   acctttcagg agtactacga ccagttcatt gccggatacc cggatatggc aaaggcgacc     480
   gaaagcatgg caatcctctg cgacaatgcc taccgcagaa tcaaggacga tacctgcccg     540
   gcgcatatca atatcacagt ggacaagtcc ggaaacctga tgcgggtctc tcaaggtcag     600
   ctggattccg gatcctttgt gcccaccagc gtcacagccg gggaattcac gatctttgcc     660
   aagaccggac cggccgttat caaaaaagcc ggcgtcgtgg tggagcatac ggatttcgtc     720
   ggcaagtatc ccctgacgcc gggacgcacc ttgagcgcgt tggagctgag cctgattccg     780
   aaactcccgg aatggtatat cattccgccg gaagtggtgg atatttgcaa gcatgcgcag     840
   aagaccaccg ggcggcccat gcagatgcga aacttcctgt tgcgtggacc tgccggtact     900
   ggcaagacca tgggcgcaaa agccattgcc gccggactgg ggctgccata catgaagtat     960
   acctgctctg ccaacacaga aatatttgat ttcaccggca tgatcttccc tgaaacggac    1020
   gcagtgtcca ccggtagctc ggagcttgac cgtgagcgcg agatcctcaa gtccatggga    1080
   ggtatcagct atgccaatgt agcaaagctt atgcgccttc cggacttgga tgatatggac    1140
   tacgatcctg ccggcgtcta tcaggctctg acaggcgtgg aaaatctggc ggctaccgtg     1200
   caggactgca tgagcgttgt gctggaaaag gtcacggaaa aggtacaggc tcttagcaag     1260
   cgtgctgaaa accgtcaaag ctccggccag aactatactt atgtggagac ggattttgtg     1320
   aaagcgttaa aacatgggta tttagtcgag gttcaggagc cgtccacgat cattcagccg     1380
   ggtgtgctgg tgggccttaa ctctcttctg gagcaggagg ggtctatcac gcttccgacc     1440
   ggcgagatca ttcgcagaca ccctgatacg gtcgtcattg tgacgaccaa tgtcagttac     1500
   gaaggttgca ggtcgatgaa ccagtcggtg gtggacagaa tgagtctggt caaggacatt     1560
   gaactgccgg agcccgaggt tatggtgcag cgagccatgg ccgtgaccgg atgtgccgac     1620
   gaatacctgg tatcccagat ggtgcaggtg gtcaacgaca tggccgatta ctgccgcaaa     1680
   aacagtatca ccgacggtgc ctgcggaatg cgcagcctga tcgattgggt catcagtgca     1740
   gagatctccg gcgaccctta tctgtcggca aagtatacgg tcatcagcaa agccactgcc     1800
   gacgaggaag accgggaagc actgattaca accattctgg accctatgtt tgccccaaag     1860
   cggaagcgaa catcggcata a                                               1881
<210> 215
   <211> 1101
   <212> DNA
   <213> Unknown
<220>
   <223> Unknown gut bacteria
<400> 215
   catggcgcgg gcgcttacgc tgaggtgggg cgattacgac cttgcgaagg cggtgagcta 60
   ctgatgtccg ctccgattat tctctgcgac actgcaggca tgaccaacga acgatggttg 120
   gagtgccgcg cccacgggcc taagggcacc attccttata cggtaggagg cagcgatgtg 180
   gctgctatct tcggcgtatc gccgtggaca acgccgctgg agctatggat gatcaaaaag 240
   ggccggatga agacacctgt gaaatccaat caggagcagc tgataatggg gcatcttctg 300
   gaacctatcg cggcctattg gtatcagcaa aaaaccggca atacggttta tgacgatacc 360
   tatatgtatc agcacgcaga tcacccgtgg gctcttgcga actttgaccg ccgctttacc 420
   cgtaaggagg acaacgctcc cggcattttg gagtgcaaaa gctgcaccta tcacaaagcc 480
   ggagattggg ctgacgatgc ctatcccatc tactatgagc tgcagcttcg cttctatctg 540
   gctgtggcgg atgtggagat tggcgcgttt tctactattt ggggaaacaa tccggaacat 600
   gatctggcaa tgccggcgct tacccgtgac cgagccaagg aggatatgat cttccagcgg 660
   cttgaggaat ggatttggag cttggagcat gacaagccac ctacgatggc ggatgtcaag 720
   ccagcactgg cactggaatc cctggcgcgc atttacggcg ccagccagcc cggactcccc 780
   acgattgaat ttcctgcaaa gtatgagaag gcgctgcgcc agatcgcgct gctgcagggg 840
   aaaatctccg attgcaatgc ggagatcaaa acctatgaaa gagagatcgc tgcacacagc 900
   gtgcgaatcg cagaattgat gcaggcgcat gagcatggcg tactgagcac gaccggcgac 960
   aagctcctca tcgattttgt caccaaaaca accaaacgtc ccgactcgaa acttctcaaa 1020
   gagaagtatc ccaccgtgta tgcggatgtg ctcaatacca gtgagagccg gaaactaaaa 1080
   gtatcggtta caccgacata a 1101
<210> 216
   <211> 360
   <212> DNA
   <213> Unknown
<220>
   <223> Unknown gut bacteria
<400> 216
   atgtcaaagc aacttgtatc aggtaagcat cggctcagaa atacctactg gtcgttagtg 60
   ggcgccgcgt cggcaacgat cctctccgtc cagcccgtaa tggctgaaac aatctgggat 120
   cgcttttcca cgatcatgcg ggatatctat ggacagctgg tcgggatcag taccattgtc 180
   gccgtgaccg tggcagccat tgcgttgttg gtgcggatgg tcagccggaa ccagcgtgcg 240
   gtggacgagg ctaccagctg gctcaaacga gtcgttgtga cctggattat tcttaacaca 300
   ctcggcttta tcgtagcata cctgcagccg ttgatcgccg gcgggcagta tacttggtga 360
<210> 217
   <211> 693
   <212> DNA
   <213> Unknown
<220>
   <223> Unknown gut bacteria
<400> 217
   atgtgcttac gcggattttt tcttgcactc acgaaaccgg aaaggagaat cgacatgaga      60
   aaaatagaaa gcagtgccgg aaaggcgctt tatgaacagc aggaaaccaa gctttcgcag     120
   caatctgccg catttattga gcttctaaca aagcgcggca ttctgggaga tttgtctatc     180
   gatgatgaca gagtccgcca agccaaaaag gagaaaaaga aaagcatgta tcataacacg     240
   cttctcctgc tgcagcacta ccgaaccatt ctctggacgc tggaatgctt cccgtccagc     300
   gtagcagagg agctggatcg cccgctgaag gatctggata cgctgctgga tcggatcgac     360
   ctggaacttg gaatgaacaa tcgaaagctg gaaggccgga tggagagtgt acgccgctcc     420
   cgcctgctgg ttgaccggct caacgatgcg gtcagtatat tgaaagccaa accggtcaac     480
   gggcacaaga tgtatgacat catctacatg acctttctcg ggccggacaa gctgatgcac     540
   gaagagcttc tgtaccgtct ggacatctct gcccggcact attaccggct ccgcacgcag     600
   gcaatcaata tcatcgccct gcgcctatgg tctgcccctg ccggcgaggt ggattcctgg     660
   ctggaggttc tttctatttt ggaaaactgg taa                                  693
<210> 218
   <211> 2271
   <212> DNA
   <213> Unknown
<220>
   <223> Unknown gut bacteria
<400> 218
   atggcaagag tgaatcataa gcgggttaaa cagctgctca atgaaaaacg cagcagaatc      60
   acagaccggc aattcttcac gtcccgcatc cttgcgggac attttgaaga tatggccatg     120
   gcacagacgc ggcggtataa atacaaccgc cgcatccatg tggccatcag ctggagtccg     180
   aagtccggtg aagtggcatg caccaataac ctgtcggtgc tgatcaatgc ggggcatagg     240
   ctggtgaccc aaaacagggg gcgggagaac cgctatgaga tcgtttgcgg actgtttgcc     300
   cacgaacttg ggcattgtct atacacggac tttcttgcgg gccagaccta caacaactac     360
   ctcagccggg agaaatggta cccggagccg cctgtttaca agctgcccaa agacactgta     420
   agcgaacgag cgctgtggga gtatgtaagg ctggagccgc ggaacaatga gatgcttcgc     480
   tatgtcgcgc accatatttc caatgtcatt gaggacgcct atatcgaaaa tcggatactc     540
   gctcaattcc gcggcacgct gggcaactgc ctggaagcgt tacgggagca gcagtatgag     600
   agcattccga cggtaacgga gcttatcgag aaagaggatg acgggaactg ccatatcttt     660
   gagagcatcc tgcagatcat gctgtcgtat gtgaagttcg gtgaaatcaa gtacggagat     720
   gctcccctca gcgatgagcg cattcaggtc gtgttcaaac tgattcacga tctggacgcc     780
   gccattgtca atccctccgg aaaagaacgg ctcaaggtcg tcaatctcat actggttcgc     840
   tgctgggact atatcgagga tttcctggag atctgcaaaa agcgccagga ggaggccaag     900
   gcgtccggtt ccacagagtc gctggccgag accatcgcgc agattctgca ggcaatggcc     960
   ggaagctctg tgtccggcga gggcagcagc acaccgatcc ccgaagtggg tgcggtctgt    1020
   gtggcagccg gagctgggaa acgggcgcag acggcagctc aggcagaaaa aagcgagtct    1080
   gaaggaagca gcggctctga agagcccgag ccccagactg accccgaatc cgagcgttct    1140
   tctgccggga tctcccagac cgaaaatccg gcggaagaag gcgcaattcc cggagatatg    1200
   gaaggcgctt ccggtgggaa gcaggcagta tcagcggaag aagacggacg catcccctat    1260
   cagcagacag atcgtgtttc cgaaggcagc ggaggggcga ccaagcacaa cgatgcatat    1320
   gagcgtgaac gctatgacca tgcagcggag gacattgagc gcctgctgga caagatggcg    1380
   gagaaggccg cctgcgagca gctggagaat gaacggacac gggaactcaa cgatgtggcc    1440
   cagagcatct cctatggaga tgtccatgcc ggtgttgata tctgtgtgaa ccggatatcc    1500
   accgttgatg aggagcttgt agagcagtat aacgccattt ccgggccatt gctggatatc 1560
   tcacggcagc tgcagaaaag cctcttgcag cagctgaagg ataaacagcg cggcggcaag 1620
   cagaccgggc tcatgatggg gcgacggctg gatgcccacg ccctctgccg gaacgatggg 1680
   aaggtttttt ataaaaatgc cctgcccaat gaaattccac agatgtccgt agggcttttg 1740
   ctggacgaat caggcagcat gtgctcctgc gaccgctgca cctacgccag gtcttcggct 1800
   atcattctat atgacttctg caaggcgctg catatccctg tcaccgttta cggtcattcc 1860
   accagcggca gcagtgtgga gctgtattcc tatgcagagt tcgaaagcat cgataacgat 1920
   gataaatacc gcctgatgga cattgccgcc cgtggcagca atcgcgacgg cgccgcgctt 1980
   cgttttgtgg cggagcagct ggttaaacgg ccggaggagg taaaaatcct gattcttgta 2040
   tccgatggtc aaccggcagc gcccggttac tacggcagtg ccgccgagga agatctccgc 2100
   ggcatcaagc aggagtaccg gcgcaaggga gtattgttca ttgcggccgc tatcggggag 2160
   gacaaacaga acatcgagcg aatctatggt gattccttta tggacatcac agatctgaat 2220
   caactgcccg taaagctgac cgcagcggtc aagcgccata tgcgggtcta a 2271
<210> 219
   <211> 705
   <212> DNA
   <213> Unknown
<220>
   <223> Unknown gut bacteria
<400> 219
   atggactcga taaactcgtt cattcatctg ctggataaaa tagagacgca ggagcgacgg 60
   ctcggcatga ccataaaaaa tctccgctgt ctgaccggag ggaaaagcat ggagctggcg 120
   tatgccatgg cgctgaagct ggctgaggaa agtgaaaaat tgacgctgct gaccagagca 180
   cttcccgcct acacgggaaa tccgcgggca ctgcaggatg tggatgcggt tgcagcagag 240
   tcggttccaa tagaagcggg ctttaccatc gaaggctggt tctgcatccg gctgcctctg 300
   ctgctcccca aaaaggagag gagctccgcc tcctatattc ggggcatcct gtaccccgcg 360
   ctcaggaact tctttcaggg caaagcgccg gttcgatacc ccgaaagcat tatgatattc 420
   cgccatgtat atgaaaaagg caggccggag cggcggtacc gggatcatga caatattgag 480
   gtcaatatgg tctccgatac cgtcgccatg tatgtgctta cggatgacgc gccgccgtgc 540
   tgcgatcatt tctactgcag cgcggcctcc acgcaggagc gtacggaggt gtatgtggtc 600
   ctggcacggg attttcccca gtggctggtg ctggagcccc tgatcccgga cgagggggtg 660
   atgctccatg aaaatgtgcc ggagcggcgt caaaaacatg tgtaa 705
<210> 220
   <211> 1236
   <212> DNA
   <213> Unknown
<220>
   <223> Unknown gut bacteria
<400> 220
   atggcaagaa agaaaaaaga gatacccaag tatggcactg tggtgctaaa gggggtcgaa      60
   tattacagaa cacgaattga agatgccgat ggcagacgag tcgcccttta cgcaaggact     120
   ccggaagaac ttcttgaaaa agtggaagaa gcacagcgcc aaattgagga tgcggttttc     180
   cgtagaacaa caccgactgt agccgaatat tgtgagcgtt ggctcaccat gcaatctgca     240
   agaatacggc caacaaccct catagactat acttcaaagg tcaaaaacta tatcattgaa     300
   ccgctgggcc ataaatatat ggcagaagtc tcttcagatg atatcaagct tgcgatggtg     360
   aatgtctcct caaaatcatg ctcagtatat cgctcggttc agatgctgta taagtgcatt     420
   ttctattctg cagtcgaaag caaaatcatc gattcctgcc cctgtgaaac tctttcttcc     480
   aaaggaggaa agccgcagaa tgaacgagag gcacttacag acgaacaggt tgataaactt     540
   ctcgttgcca tcaaggggct tcctccgtat gtgtttgtaa tgattggtct gtatgctggc     600
   ttgcgacggg aagaaatctt ggcgttaaaa tgggactgtg tgtttcttga tgccgaagca     660
   ccataccttt ccgtgcaaag agcatggcat tcggaacata atcgacccgt tatctcgtcc     720
   gatctgaaaa caaaatctgc aaggcgggac attccgctac cttcagttct aacggagtgc     780
   ctacgggaag caaaagaggt tgcaacttca gaatatgttg ttgccaatcg tgatggaggt     840
   ccgctgtcat atacgcagtt caagcgagta tggcagtata tcgtcacaag atcaacaaag     900
   gagcgtacct atgtccggta tatcaatggg cagaaaattc agcgcacggt aacgccgaag     960
   ttgggagaaa aagcagcgca taatggaaat gtggtgtaca cccttgattt tcaggtaact    1020
   ccacaccagt tgcgccacac atacatcacg aatttgattt actccggcgc agatccgaaa    1080
   acggttcagt atctggcggg gcataagcat agcaaaatta cgatggacat ttatgctaag    1140
   gtgaagtata ataaacctaa agaattgtcc tcggttgtga acaaagcatt tgcgcccaaa    1200
   aaaggtgcta caccgaataa gacgcggaat gggtga                              1236
<210> 221
   <211> 516
   <212> DNA
   <213> Unknown
<220>
   <223> Unknown gut bacteria
<400> 221
   cgtttccgca ttgctcaggc tactgaagaa aaagagaatc cttctgctgt gaggtgcgta 60
   atgagagaaa aattcgaaaa aatcccaccg ccgctgcaaa aacagattat aatgagatgt 120
   ggagccgcgc tggtaagcct tttattgatg gtggtgtctc tgttgttgga gggcaacctt 180
   tatctgtctc tttccgtcct gctctttttc gccttttatg ggttcagtgc attacaactg 240
   ttgtatagag cagtcaccga tcaaatagtg gtaattcgtg gtcactgtac cgggctggaa 300
   aaaactccga tgcgccgccg catcaaaacg ctctatctgt ggtcagaccc acatacagtg 360
   aaaatacaga tcgccggaaa acttcgaaat gttgatgagg gcgacatcat cacggtatat 420
   acggcaaccg gcacaccggt atatgaaaac gaaggctgcc aacagcttag cacatatttg 480
   gcaatcgata ttttgaaagg aagaggatca aaatga 516
<210> 222
   <211> 243
   <212> DNA
   <213> Unknown
<220>
   <223> Unknown gut bacteria
<400> 222
   aatgcgcata tggaaaaact gattacacga aaagaagctg ccaagatttt ggggattagt 60
   attgccacgc tggatgcagc cagacaaaac ggcttgattt cctacgtcca gtatgttcca 120
   aatgggtgcg tgtattttac agatgcagcg cttcaggaat atgttgccaa atgcacaaag 180
   agggcaaaac ctgccgataa caacttgaca taccgcaagc agcaccgtcc ccaaaagaaa 240
   tag 243
<210> 223
   <211> 1209
   <212> DNA
   <213> Unknown
<220>
   <223> Unknown gut bacteria
<400> 223
   atgggttgct ggtttttgtt atatcacggc gaataccgcc taaaaaaacg aaaggagaac      60
   acaaaaatgt tacaaccatg ccaggacaac tactccacga cctttgcttc ctacgagggg     120
   atgcgccggt accatgagaa gaaatcgctg gaaagccgat ggcacagatg cagggtcaac     180
   gaactgcata ttgagccgct ggataaggcg tcgcctcttt atggaactcc gtccgcattc     240
   gctgcgggaa tcagcgccga gtccgttgag gacacggcgg aaaacctcgg tcttgccatg     300
   cgggtagatg gttcttacta tcccgtccgc agcacggcct acaaaagcct gttggacagg     360
   gcgaaaatca gcggatcggc attgacgaag ctgagccgcc aaagattggc gtcggtgctt     420
   aacgattgcc tggagctgta ttcttcggag acgctcctgc ttatccgaga tgaaaaaatc     480
   tcggcagcac actccggcga ttccatggat tattccgtgc tgccgattga tgagctgctg     540
   aaggttctga ccaaaaagtt ggacgaccgt ttccccggca gcatgtttca gggcggttat     600
   cgcgatcatt cgctctccag tgcctcatgg actatgcccg ggcaaaagga agacctgctc     660
   ggtgcctatg ccaagctgct ggcgtcgcag ggaaaaccca cacttgctgc caaacttgtt     720
   cccgccatcc gctttgttac ctcggatacc ggcattgcct cggctaaggt ttccgccatg     780
   ctggagggta cgcagcaccc gatccatatt gggagctgta tcgccgtgga ccacaggcac     840
   cagagtaaag tggaggactt tgaagcagct ttggaccagc tgtttgcgca gttcggtgat     900
   tcagtcgcca gattacaaaa gctgttggat atccatctga gctatccggt caatgcgatg     960
   accagaatct gcaaaaagtt gtctctgccg aagaaagctg ctttggaagc aatcggtatg    1020
   tttgagatgg cctatggagg aggaactgca accgcccatg atgtgttcat ggctatgcag    1080
   gagatcccct atattctcaa atccgaccat gcaccggaga gcaaaatgct cgttgtggag    1140
   gagaacatgg cgcgggcgct tacgctgagg tggggcgatt acgaccttgc gaaggcggtg    1200
   agctactga                                                            1209
<210> 224
   <211> 423
   <212> DNA
   <213> Unknown
<220>
   <223> Unknown gut bacteria
<400> 224
   atgctgaaag ttggagataa actgatctgc aaaacagaag gacagccgga aacggaattc      60
   tgcattcgcg gcgaggtcaa aaaggtcgaa ggagacattg tgaccatcag ccgcgcgctt     120
   aaattcggga cactgcccat gcacaaggat ctgacgctgc cggagagcga gctgctgaaa     180
   tactacacgc cattggacaa tcaagcgacc gatgactgcc ccgttagcgg gtctatctat     240
   gaggttacga tcgtccgcac aggtgtcgtg ttcgtaagag cagctacctc ggctgcggcc     300
   atggatcttg cagaccatct cacaaccgat catgtcaact ggtcagatga ctggagcccc     360
   accgacgctg aagaacgcag tgactacgac ggcatggtct ttgaggagcc tgactttgac     420
   tga                                                                   423
<210> 225
   <211> 996
   <212> DNA
   <213> Unknown
<220>
   <223> Unknown gut bacteria
<400> 225
   atgggttatt gggcgaagct ggcctcgcgg gcggcatttc gcaagggccg ggcaaaatac      60
   tgcggataca caaacgatgt aatgccggga atgtggtccg gtggcgtggt cggcttgaag     120
   agcattctgg gcgtcaaaag ccgcacggag gctctggaaa tcatgaatac gctcagccgg 180
   ttcgggtata tccgttacac gcttgacgag aaaacaaaga agctggaata tgccgtcacc 240
   gattgggtcg taaaatgctc cggcaccgaa tgcatgagcg gaacagtcta tgcaacagac 300
   ggctatggct ttctctgcct tccccgcaac atcacgcagc gccttgcgga tcagcgttat 360
   acattcggcg aatcagatgt atggctggac ctctggtgcc acacggtttg gcaggagacc 420
   ggcaatgcgt tttcctgtct ggcgcctgcc gttcaattcg ggagacttgg cgccgctttg 480
   acattggaag ccttgggacg gcgttggggc tgggaaaaga caaaggtgtg gagatttttt 540
   cagaagaatg gggatgcctt cgctctgcac cgtctgccgg gctcctacgg ctgcctcatt 600
   ttcaatagac tgtatccgct ggatacagag gcttcgctgc ccaccggcgc agaaattgaa 660
   cgcattattg agaaaatgcg tatttgcgca ggaaatgcgc atattggagg ctcggacaat 720
   gcacgcctga acaagatgat cctttggtat agccgcagga tcattatcat gagaaccact 780
   gattatgccg aacaggtatc aaaaaatcgc gttgcacttt cgtcccctat aatacgcgcg 840
   tatatttctc tgtgttggaa ttgtcagaac tgtatctatg actgcagagg tgtaagtagt 900
   agactacgcc gcatagatgc gaaccacatc cgcgggccat gtgcttacgc ggatcttttc 960
   ttacactcac gaaaccggaa aggagaatcg acatga 996
<210> 226
   <211> 789
   <212> DNA
   <213> Unknown
<220>
   <223> Unknown gut bacteria
<400> 226
   gtgggcgtgt gcgccctgac ccgtgaacct gatacggcta ataccgtcgg agggaaagat      60
   gcatatatgg gatacaattc agtatcgcgt tgcacctgga cggtttgcaa tgcgatactt     120
   atttttttgg aggttcccaa aatgaaaacc aaaagactga cggaaagcgc catgctgctg     180
   gcgctggcca ttgtgctgga gctgatctcc aaggccatca tccccgaaat gcccttcggc     240
   ggtcaggtga cgctggtggc catgctcccg gtggtgctga tctcctaccg ctacggcgtg     300
   aagtggggct tcgtttccgg cttctgctac gcgctgctgg aaatggcact gggcgcgaag     360
   accgtgagcg ccgcctttca gcccggctat ttcggcgacg acaccatgat cttcaaggcc     420
   atcctcatgt gcctgctgga ctatctgctc gcctacacca tgatgggtct gggcggcatt     480
   ttccgggata agatcaagaa tcccggcacc agcctgatgt gcggttctct ggtggcgctg     540
   ggtgcgcggt atctgtctca cattctgtcc ggatacctgc tgttctccgg ctgggcggag     600
   tggttcttca cgcaggacgg cttccccgca tggggcgcga agctggtgga gagcctcagc     660
   cccgtcgcgc tgggctgggt ctattccatt gtgtacaacg gcttctacat ggttcccgag     720
   ctgatcctca ccgccatcgc ggcgcagctg atcgccagaa ttccgaagat cgtggtcaag     780
   gttgactga                                                              789
<210> 227
   <211> 1077
   <212> DNA
   <213> Unknown
<220>
   <223> Unknown gut bacteria
<400> 227
   gatccgctgc tcagccgaag acgttttgtt cagagattcc gccaaaagaa aggggcggcg        60
   gatgccatga caatcaaaga tctggctgca aagaccggct atgccgtcgg caccgtttcc       120
   cgggcgctga acaatcatcc caacgtcagc gagcgggcaa ggcaggaaat cctggaggcc       180
   gccgccgaaa gcggctttca gctgaacgtc aacgccaagc agctcaagca gcaccactcc       240
   accgccattc tggtggtggt aaagggtatc ggcaacgagc tgttcgggga gatggtggag       300
   tccatccaga ccctgatcgc ccagacccac taccagctgg tggtggacta catggacgag       360
   gacgaaaacg aagtgcgccg agccgcgcag ctgtgccggg aaaagaagcc tctgggcatc       420
   ctgttcctcg gcggcaacaa cgagaatttc atccgggatt tcgggggcat tgacgttccc       480
   tgcgtcgtgg tcaccaacga cgcctccatg ctgtcctttg acaacctgtc cagcgtctcc       540
   accgacgacc gggaggccgc gtgcagcgcc atggatacgt tgattcgtct gggacatagc       600
   cgtatcgcca tcatcggcgg cgaccgggcc atttccgaca ccagccgtct gcgctacgag       660
   ggctgtatgc agtccttcac tgcccacggc atccccttcg accccaagcg ggactatcag       720
   ggcgtgcgct attcctatca ggcggggtat caggcgacca aggcgctgct ggataagggc       780
   tgccggttta ccgcgatttt cgccaccgcc gacgtgatgg ccatcggcgc catccgcgcc       840
   ttgcaggaca acggcctgca ggtgccggag gacgtgtcgg tcatgggcgt ggacggcctg       900
   ccgctgagca gcttcctggt gccgagactg gccaccgtcc gtcagaccgt ttccctgctg       960
   gcaaagcgca gcgtggaaat tctgctgggg aatatcgagc ggaacgcgcc cgcctgtcac      1020
   gagacggtac cctttgagat cctctcccgg gagagcatcc gggaaatttc acactga         1077
<210> 228
   <211> 1941
   <212> DNA
   <213> Unknown
<220>
   <223> Unknown gut bacteria
<400> 228
   atgggtgcat actttttcgt gatggctgct ttctgcgccg cggcgctgct ggtggggcat        60
   tactggctgg ctgccggaga aatggcggtg acggtggtca cgttcctatt gtacgttctg 120
   gacaggacac gccgggagcg taaaattcag cagtatatcc agtcggcctc caacacgctg 180
   gaagcttcca gcggcgggga agcgcccctg cctgcggtga tggtccgtct gggggacggg 240
   gacatcgtct ggggcaacca tcgtttcagc gagctgacgg ggtacgccga caccatgacc 300
   cagctgcggc tggaagaggt gctgccggac ttcaccctgg actggctcac cgccggaaaa 360
   acggaaagcc cccatgacgc ggttctgggc aaccgccgct accgggtata cggcaccacc 420
   atccgggcgg aggacaaccg gggcaccatg ctgggcgtgc tgtacttttc cgacctgacg 480
   gagctgtatc aggtgcggga cgagtatatc cgctcccggc ctgtggttgc catcatcctt 540
   attgataatt acgaggaact gaccaaaaac ctgacggaaa gcgccatttc ctccatgaac 600
   gccaaactga acgaaaccat cacccggtgg acggagggct acagcggatt gcttcgccgg 660
   ttggagcgca accgcttcct gttcattttc gagaagcggg acttagaacg ggcgaaggaa 720
   aataagttct ccctgttgga agacattcac gagattgtca attcctccgg cctgcccgcc 780
   tccatttcca tcggtctggg cgtggacggc gagagcttcg aggaaagcta tgattttgcc 840
   gcgctgggca tcgagatggc actgagccgc ggcggcgatc aggcggtcat caaggatcgg 900
   gtcaacttca atttctacgg cgggcggaac cgggaagcgg attaccgcag caaggtacgt 960
   tcccgtgtga cggccaattc cctgatggag ctgatcggcc agagcggacg ggtgttcatc 1020
   atgggacaca agaacgccga cttggacgcc gtgggcgcgg ccatgggcat cagctgcctg 1080
   tgccgcaagc gggggaagaa ggccaatatt gtcattgact tggaaaacaa tgccgccgga 1140
   aagctgatcg aggaaattca ggcggttccc gaataccggg acgtgttcgt gtccgggcag 1200
   gatgccctgc tgatggcgga caaccggagc atcctcgtgg tggtggacac caaccggccg 1260
   gatcaggtgg agtgcagacc gctgcttgaa gccatttcca aggtctgcgt ggtagaccat 1320
   caccgccggg cggcggatta tatcaacccc gtggtggtca accttcacga gccttacgcc 1380
   tcctcggcgg cggagctggt gacggaggtg ctcatgtacg ccgtggagaa gaaggacgtg 1440
   ctgcctattg aggcggaaag cctgatggcg ggcatctgtc tggacaccaa gttcttcaac 1500
   gtccgtaccg gcgagcgtac ctttgaggcc gcagccgtcc tgcgccgtct gggcgcggac 1560
   accacggagg tcaagaagct gatgcagaac gacttccagg acaccatggc gaagtatcag 1620
   atcatcaaat cctcccgcct gtaccggcag gagatcgcca ttgcggcgct gaacaccccc 1680
   acgacccggg tgctggcggc gcaggcggcg gacgagctat taaatatttc cggcattacc 1740
   gcgtccttcg tcctctatcc ggacggggag caggtcatca tttccgcccg aagtattggc 1800
   agcgccaacg tccagatgat tctggagccg ctgggcggcg gcggcaacac cgctacagcc 1860
   ggggcgcaga tgaaggacac caccgtcaag gaagcgctgg acgagctggt ggcatccatt 1920
   gataagttct acgaagaata a 1941
<210> 229
   <211> 909
   <212> DNA
   <213> Unknown
<220>
   <223> Unknown gut bacteria
<400> 229
   atgagttggt tttggttttc cattattgct ttgctgtgct ggtccggttc cgatttcttt 60
   tccaagatcg gctgccggga cgcatccgac aaatacagtc agtataaaat ggtcacagcc 120
   gtcggcgtgg tcatgggcat tcatgccgcc attgagattt tcgtcggcgg cgtagagatc 180
   tcatggcagg tcatctggac gtacctgcct gtctccctgc tgtacatcgg ctccatgaca 240
   ttgggctatg tgggacttcg ctacattgag ctgtccatct cctcccccat ctgcaacgcc 300
   agcggtgcgc tggtcgccat cattgccatt gtcagcggca ccgccggaaa aatggcgatt 360
   gctcagtaca tcgccatttt cctcgcctgt gccggtgtca tcggtcttgg ctttgtggag 420
   gccaacgagg acgatgacct tcgagccgcc cggcaggagg cttccaatta caagtacgca 480
   aaatcatggt tagctctggc gctgcccatt gcctactgca ttctggacgc ggcgggaacc 540
   ttcgccgacg atcttgtgct ggaaaccctg aacgaggatt ctgccaacgt cgcctacgag 600
   ctgacctttc tggtcgcggg catcgtcagc ttcgtctaca ccgtcatcat caagaagcag 660
   aagctgctgc ccaaggcgga aggccccaag tacatcggcg cggcctttga gaccgccggt 720
   cagctggcct acatctacgc cctcgccagc ggcgagtccg cgctggccgc ccccatcatc 780
   gcctcctact gcatggcttc cgtcctgtgg agccgcctgt tcctgaagga aaagctgagc 840
   tggaagcact acacctgcat tctggtcacc tttgccggta tcctgatcat gggaatttac 900
   gacatgtaa 909
<210> 230
   <211> 1356
   <212> DNA
   <213> Unknown
<220>
   <223> Unknown gut bacteria
<400> 230
   atgaatttct ccaacgcttg caggcttaaa aaaggtttga gcctgattct gatcgtactt      60
   atggtagtcg gtatgttctc cggctgtaag aagaacaact ccgagaccga gccgaacacg     120
   actccctccc tcaatctgaa tatgtcggac acctccgctc ccacgcagga gacggtcccc     180
   cccaccacga cggaacccat cgtgtataac gagaatatgg gtaccgtcac caaccagatc     240
   agtgtccgcg tcatccccag caaggacgcc aacgtcaccg gcacactgga cgccggcacc     300
   gtggtggaga tcctgcgtca ggaaaccatc gccgacatca catggggcat gatcagcacc     360
   cctgtcagcg gctggatctg catggactat gtgaagatgt attcttctca gaacggctcc     420
   gccagcagcg agaccgatcc caatgcttct cagcccacca cccccgcacc gtccgacaat     480
   acgggcaata ccaccaacaa tagcggtact cacaacacca agggcgtcat cagtgcccgc     540
   agtctgaacg tccgtgacga ggccagcgcc tccggcaaga tcgtcggctc ctacttcaag     600
   ggcgacgttg taactatcct cgagactaaa gacggctggg gccgcaccga caagggctgg     660
   atcaagatgg agtacgtcaa caccaccggc actaccaccg gcggcaacac cacgacgacc     720
   accaccggca acaataacaa cactgccggc aacggcagca ccactaccgt cgccaagggc     780
   atcgtcaaca cccaggagct gaatatccgc agctctgccg gtactgacgg cgaccgggtc     840
   ggcagcctga aatacggcga tcgggtggag attctggaaa agagcggcga ttggggccgt     900
   atctccaagg gctggatctc cctgaactac gtctatcagg acggcaccac cggcaccaag     960
   tccgcaaagg gcatcgtcac caccgacggt ctgcgcatcc gcagcggccc cggcaccacc    1020
   tatgaatccg tcggctccta caacagcggc gaccgggtca aggttctgga gcagttcacc    1080
   tacggcgaca ccacctgggg ctgcaccgac aagggctgga tctccatggg ctacgtctat    1140
   caggacggca ccgatgtggg caagaccgtg gaaggcatcg tcaccaccga cgagctgaac    1200
   atccgcagcg gccccggcac gggctacgat tccgtgggca agctgtatac ggacgaggtc    1260
   atcaagatct tctatcaggt cactgtaggc gaaaccacct ggggctgcac caaggacggc    1320
   tgggtctctt tgaagtacgt cagtctggaa ggataa                               1356
<210> 231
   <211> 2052
   <212> DNA
   <213> Unknown
<220>
   <223> Unknown gut bacteria
<400> 231
   atggaggctc tgcgcgcggc cgttcagaac ggcgctgacg ccgtctacct cggctgcggc      60
   gcgttcaacg cgcggcaaag cgcgaaaaac tttactcccc agacgctggt ggaggccatt     120
   aaatactgcc atgtccgggg cgtgcaggtg cacctgaccc tcaacaccct cgtctccgac     180
   cgggagatga ccgaggcggc ggagctgatc cgctatgccg cgcagaataa tattgacgcc     240
   tttatcgtgc aggacttagg cgtggtgcag ctgtgccgtc agatcgcccc cggcgttccc     300
   atccacggca gcacccagat gaccgtccat tctctgcccg gcgtgctgct gtgcgccgcc     360
   atgggcatca agcgggtcgt cctcagccgg gagctgagcc gggaggaaat tcgctacatc     420
   tgcgccaact ctcccattga aattgaagtc ttcgtccatg gcgcgctgtg catgtgctat     480
   tccggccagt gctacatgtc cgccatgatc ggcggccgct ccggcaaccg gggccgctgc 540
   gcccaaccct gccgccagag ctacggctac acccactggc aggaaaaata ccccctgagt 600
   ctgaaagaca actgcctgat tccctatctg cgggaattgc aggaaatggg cgtggtctct 660
   ttgaagctgg aaggccggat gaagcgggcg gaatacgtgg ccaccgtcac cgccgtctac 720
   cgcaaggcgc tggacgagat gaccgtcacc aagcccatga tggacgccct gtacgccgcc 780
   ttcaaccgtc agggcttcac cgacggctac tatacaagta gggtggatac caaaatgttc 840
   ggcgtgcggc aggagaatga cgacgacggc aaatggcttc aggccgcacg gcaatcctac 900
   gaagcagggg agacccccct tgtggatatc cgctttcagg ccatggtcac cgtggacggc 960
   agctgcgtca tcgccaccga cccggagggt cggacatgcc gctccgacgg ccccgtgccg 1020
   gaacgcgccc agaacatttc cctcaccggg gcgatgctcg cacagcgggt ctccaagacc 1080
   ggcggcacgc cctaccggtg cgtggaggtg cgcacccggg tggatcccgg tctcattatc 1140
   tccgcttccg ccatcaatgc catgcgccgg gacgtgatga accagctcac cgccatccgc 1200
   gcccggcgag aggagcagac cctgcgcaag cccaagcccg tgccggaata caaaggcccc 1260
   tcgggtctgc ccggcctgac cattcaggtc acctcccggg agcagctgac ccccatgctg 1320
   ttagatctgg aaaccacact gctgtacgtt cccatccaca tcttgctcgc cgacggggag 1380
   ctttgcgccc ggctggtgca gcggggacga gtggcggcgg tgctgcccag aatcgtccac 1440
   gacggggagc tgccccggat caaaaagagc ctggaagtcc tccgcgccgc cggtgtccgg 1500
   gacgcgctgg tgggcaatct cggcctgatc gcccccgtgc gggaggccgg aatgcgggtt 1560
   cacggggatt tcggcctgaa catcttcaac tccgcctcca tgaacgtcat gcgttctctg 1620
   gaaatggctt ccgccacggt gagctttgaa atgaccctgc cccagatccg ggacatgagc 1680
   aaggcggtga acgccgagct gatcgcctac gggcggctgc ccctgatggt gacggagcac 1740
   tgcctcatcc acaacaagac caacgaatgc acctgccatc tgaaagccac ccgcctgatc 1800
   gacaagaccg gcgcggagtt cccggtcatc cgggacggcg acagctgccg cagcgtgctg 1860
   ctcaacggca agaagctgag ctggctcgac cggcaggacg atcttgccaa gctgggctta 1920
   tgggcgacgc ggctgtactt caccacggaa aattccaagg aagttgaccg cgtgctgaac 1980
   gattacatga accccgaacc cctcgacccc ggcgcgtgta cccggggact gtacttacgg 2040
   ggtgtggaat aa 2052
<210> 232
   <211> 1392
   <212> DNA
   <213> Unknown
<220>
   <223> Unknown gut bacteria
<400> 232
   atgctaaact atcatgtcaa ggtcggcctg gttcccattc gcagagacgt tacgccccgc 60
   cccggtatct ttaactggga aaaggcggag gagcgctgcg ccgcagcggt agcctatatc 120
   gaggagcatt tcacttccga gaacgtttcc ttcgtggatc tgaagggaat caacgccgtg 180
   gatgttctct acagcgacaa ggacgtggac gccgttatcg agcgctttca ggcggagaag 240
   gtggacgcgg tattcctgat caacggcaac ttcggcaatg aggaagtggc cggcgcggtg 300
   gccaaggcca tcggcaagcc cgtgctcctg tggggccctc aggacaccgt gtttgaggcg 360
   gacggcaccc ggtataccga ttcccagtgc ggcctgttcg gcatgagccg tcagctgcag 420
   agaatgaacg ttccctttac ttacatcgaa aactgccgca ttgaagaccc gattttcacc 480
   gagggcttcc tgcggttcgt gggcgtggcc tgcgcggtga agaactttaa gggtatgcgt 540
   gttgcgcagg tgggtatgcg tcccaagccc ttctgctccg tgatcttcaa cgagagcgac 600
   ctgatggaca agttcgacat ccatgtcatc cccgtcaatc tggccgtgat tcaggacaag 660
   tataaccgga ttctggagac ccggaaggac gagctggctg aaggtgttgc caagctgcgc 720
   cagatgtacg agatcgacgc cctgtccgag ccggtgctgg acaaggtgta tgctttcgtg 780
   ctgctgtatg aggaaatttt tgaagagtac aacgtgaccg ccgtttccgc ggagtgctgg 840
   actgccatgc agctgatggt gggtgctatg ccctgcgctg cctatccctt gctcgcggac 900
   atgggctata tcatcggctg cgagagcgac atgcacgcga ccctgacgca ggttgtgctg 960
   aagagcctga ctctgggaca gaagaagccc ttccttggcg aattcaccac ccgtcacccc 1020
   agcgatcgga atgtggagct gctgtggcac tgcggcccct ttgcctattc cctgaaaaag 1080
   catggtaccg gcgcgaagat ggtcaacatg cgtcagtgga tgcaggtgga ggacggccat 1140
   ttcaccgtgg cgcggatcga tcaggatcac ggcaactatt ccattgccgt ctgcgagtgc 1200
   gacagcgcgg aaggccccta taccttcggc tcctatatgt ggggcaagtt caaggatctg 1260
   cccggggtgg agcgcaagct catcgaaggc ccctacatcc atcacatgtc cgagatcgag 1320
   ggcagcctga cggattccat ccgggaattc tgcaagtacg tacccggtct gacctttgac 1380
   ggcctcgact aa 1392
<210> 233
   <211> 909
   <212> DNA
   <213> Unknown
<220>
   <223> Unknown gut bacteria
<400> 233
   atgaacaaac aggaactggc cgccatggtg gcggagatat tgagcggcat ggagcatgag 60
   ccgccggtga agggcggtga ctatcgcccg accgcgcccc agccggagaa agcgcccacc 120
   ccgtaccggg acggcgactt tgtcccggat gtgacggcgc tggacttacg gaagctctat 180
   ctggtggaac acgccgaaaa cgaggaaaaa ttccggaaaa tgaaagaaaa gaccccggcg 240
   cgtctgggca gcggccgggc gggggcgcgg tacaagaccc tgaccatgct ccgtttccgc 300
   gccgaccacg cggcggcgca ggacgcggta ttttctcagg tcagcgagga cttcgctgcc 360
   aaaaacggca tggtggaggt cagaacgaaa tgccgggaca aggacgaata cctgacccgc 420
   ccggacttgg ggcggtgctt tgacgaggaa aaccagcaga aaatccgtgc cgccgtcccc 480
   ggaacgcccc gggtgcagat cgtggtgggg gacgggcttt cctctgccgc catcaccgcc 540
   aacgccatgg attgcctcgc ggccatccgg gacggcctga agctcagggg tattgacccg 600
   ggaacgccga ttttcgtccg ctactgccgc gtgggcgcgg gggacgccat cggcgacgtg 660
   acggggtgcg agctggtgtg tatgctggtg ggcgaacggc cgggtctggt gacggacaag 720
   agcatgtccg cctatatcac ctaccagcca aggactggcg tttccgaatc cgcccggacg 780
   gtggtgtcca acattcacgc acagggcacc ccggcggtgg aagccggggc gcacattgcg 840
   gggctgatcg aaacgattct tacaaaaaag gtctccggcg tgggactgca tctggaggcg 900
   ggggcatga 909
<210> 234
   <211> 1392
   <212> DNA
   <213> Unknown
<220>
   <223> Unknown gut bacteria
<400> 234
   atgaatcgac gaaaccgggt tgcctttttt aacattctga gcaccgtttt gctcagaggc      60
   atttccatat tcacagcgcc cctgtttacc cgtctgctgg gaaccagcgg ttacggcgtt     120
   acccaaattt acaacacatg ggtggctgtg atcgccattg tgttcacgct gcaaacccag     180
   gggacgctgg tcaacgcccg ggtggagtac ccggagggag accagctgcg ctaccagtcc     240
   tcggccatga gcctgtccac gctggtgtat ctgattttca gcgcactggt gctgtgcttt     300
   atcggcccca tttccggggc gctgaagctg agctggtttt tgatcgtgct gatgctcatt     360
   cagggcttcg gcaccttttg cgtcaacttt ctgaacacca aattcgtcta cgaattcaag     420
   gcagggcgga acatggtcat gtcccttgtg gtgacgctga ccaccctgat cctctccatt     480
   gtgctgattc tgctgctgcc caagcagatc aactacttag gccgcatttc cgccattgcc     540
   gtgacctacg gcgtgctggg cattccggtt tgcatctgga ttctggcgaa ggggaagacc     600
   ttttacaaca aggaatattg gaaattctgc gtcgttcttg ccattccagc ggtattttat     660
   aatctttccg acctgattct ggggcagagc gacaaggtga tgctccagca gatgatggac     720
   gaggccacgg tgggctgcta cggcgcggcg ctgaatttcg gcggcatcat gttcactctg     780
   ttcaccgccc tgaacaattc atggtgcccc tttttcttcg aggacatgaa gcagggcaac     840
   cgggagaatc tgcgggaaaa ggcggggaat tttctggagc ttttcacggt gctttccgtg     900
   ggcttcgtgc tgctggccag cgaagtgtac cgctatgtct atgtttcccg ggatttctgg     960
   ggaagcacga tgctgattcc catttttgtg tccagctact acaccaattt cctgtgtacc    1020
   ttcccggtga atttcgagta ttaccacaaa aagaccaagg tcgtctcgat catcactatt    1080
   gtgtcgtcgc tgataaatgt ggggctgaat tacctgctga tcctgcgcat cggcatggcc    1140
   ggcgcggcgg tggcgacgct gatttcccac agtttgcagc tgacgctgca ctatctgtac    1200
   gtccgtttcg ctctgggcgg gaaagaatac cccttcccgc tgaaaatgtg ggtcgggtac    1260
   gcggcggtgt tcgccgctgt ggccgtggcg tccgtggcgg cggatggact gtggctggcg    1320
   cgctggggac tgggcgcagc cattggccta tgggagcttt tgcggatcag aaagcggaaa    1380
   gtattgatct ga                                                        1392
<210> 235
   <211> 864
   <212> DNA
   <213> Unknown
<220>
   <223> Unknown gut bacteria
<400> 235
   atgcgcaaga tttgggaaga ttaccgctgg attccggcga cggctgtggg aagcgcggtt       60
   ttcgcgctgg gcttttccct gttcctacag cccaatgaga tcagccccgg cggcatttcc      120
   ggccttgcgc tggtggccgt ggagctgctg ggctacggca gcgtgggcgt gctgacgatc      180
   cttattaacc tgccgctgtt catcctcggc ggtgtgaaga tcggcagacg gttcttttgc      240
   ggcagtctgc tgggaatggt tctcagctcg atcctgatcg acgcttttgc caaaatcacg      300
   atgccgccca tggattcgct gctctgcgcc ctttacggcg gcatgatctg cgggctgggg      360
   ctgggcatgg tgttcatctg cggcacctcc accggcggct cggacatcgt ggtgcggctg      420
   ctgaagctca aataccggga cgttcccatc ggacagattt ccatgtgctt tgacgccggg      480
   gtggccgtcc tgacgggcat cgtgtttcag gacatcacca aggcgctgta taccggcgtg      540
   gcggtgtttg ttacgggcaa ggtgctggat gccgtggtgt accggttcga ctattccaag      600
   gtggcgctga tcattaccaa ggagtacgac gcggtggcgg aggccatcgg caccaagctg      660
   gacagaggcg caaccttcct caatggggaa ggctcctaca gccatgagcc gaccaaggtg      720
   gtgctggccg ctgtgaaaaa gcagcaggtc acggagctga aggagctggt catggccatc      780
   gaccccaacg ccttcgtcat cgtgcaggag gcgcatcagg tgctgggaga cggattctcc      840
   cattattcca aacaatcgct gtag                                             864
<210> 236
   <211> 1071
   <212> DNA
   <213> Unknown
<220>
   <223> Unknown gut bacteria
<400> 236
   atggaactga cagagcggaa aaagaaggtt ctgcggtcgg tggtggattt gtatatccgc 60
   acggcggagc cggttggttc caaggccatt accgagctgc cggacatgaa gtattcctcc 120
   gcgaccatcc gcaacgaaat ggcggaactg accgccatgg gttatctgga gcagccccac 180
   accggcgcgg ggcgcatccc ctccgccgcc ggttaccggc tgtacgttga cgagctgatg 240
   atggattacc gcttgtccat tgacgaaacg aagtccatca gcagcgccat cgaggaaaag 300
   atgcagcggg tggataaaat ggtggaaaag gtggcaaagc tggtatctca ggccaccgat 360
   cttcccgcca tctccgccgc gtcccgacag ggcagcgcct gcgtcaagcg gttcgacctg 420
   atcctggccg gacagggaag cttcattctg gtgctgatgc tgggcggcga gcaggtggta 480
   aacaagctca tcaagctgcc ggtaaacgtc agcgaaacgg atctgaagct cctgagcgcg 540
   gtgctgaacg ccagcatgac ggacactccc cttgaggact tcaccgccga gctgatggaa 600
   aaggtcatgc gttcggcggg tgcggcggca agtctggtgc cggtgattct ggacttcacc 660
   caggagacgc tgaaaaacag cgactccacc agagtggccg tcaccggtca ggcaagactg 720
   ttgagtttgc cggaataccg ggacgtggac aaagcccagc gggtaatgga ttccattgac 780
   gaagaggcac tggcaaacct tcccgccgtc atggagggcg ccaacggtac gaaagtgctg 840
   gtaggccccg aaaacgtggc ggatgagctc aaggacacct ccgtcgtgat gaccaagttt 900
   gacatcggcg acggaatgca gggcatgatc ggcgtggtcg gccccacccg gatggattac 960
   gcaaaggtaa cggcgcgcct gagctacttc gccgagagcc tgagcaagat gttcggcaag 1020
   cccgagcagc cccagcagcc aaagctgccg gagccgccga aggaaggata g 1071
<210> 237
   <211> 999
   <212> DNA
   <213> Unknown
<220>
   <223> Unknown gut bacteria
<400> 237
   ttcatggcgg tgacgatcta tgatgtggca aaggaggcgg gggtatccat tgccacggtt 60
   tccaaggcgc tgaacgacag ctacaccatc cccgaccaga ccaaggcggc gatacgggaa 120
   atcgcggacc gtctcggcta ccgccccaac gcgcgagcca agaattttgc ccggcaggca 180
   agcggcacgg tgctgttcat cacggatttc taccagaaca ttgcctttga aaatccccat 240
   atgttcgaaa tcatctccgg cattgccacg tatctggacg gcaaggacta ttccctgacc 300
   ctgaagccgc tgacgaaaca ggcggctccc ctttacatcc gggacatcat ccagcagcgg 360
   caggcggacg cgctcatcat tcatgctttc gttttaagcc cagaactggc cgcgatcctt 420
   tcccgggtgg atttccccta tctggtcatc gggaagccgg gattccgggg caatatctgc 480
   tggatcgacg tcaatcacga gcaggcgggg cagattgccg cgaattatct gctggacaag 540
   ggctaccgga gaatggcctt tatcacgggg caggaaaacg acgccctgtc ccagagccgc 600
   ctgcgaggaa tcaatcaggt catgtctgag gaagagctga gcgttgaggt cgtctgcaat 660
   gaatcgccct acgcactgga cgacgaagcg ctgtacggtc tgctgatgcg ccgccccggt 720
   ccggaggggc tgctttgcag caataaccat cttgctctgc actgcctgca atacatccgc 780
   cggataggtc tgaaggtacc gcaggatatc gcgctgctca cctttgacaa ctaccccttt 840
   tccatgctga cagacccggc catcacggcc gtggaggtgg acatgcacga catgggcgtc 900
   aacgccgccc ggtttgtgct gcagcggata cgaaagccca atttacaaac ccaatcctac 960
   tgcacctccc cgtgcatcct ggaacgggag tctacataa 999
<210> 238
   <211> 876
   <212> DNA
   <213> Unknown
<220>
   <223> Unknown gut bacteria
<400> 238
<210> 239
   <211> 1527
   <212> DNA
   <213> Unknown
<220>
   <223> Unknown gut bacteria
<400> 239
   atgaagaaac tgacggatct ggaatacctc aggctgaaca cgtttcagcg tctgtggtac 60
   aacattgtcc tgttcgttct cggcatcccc ggctggctgg cctctttggt caaagccgtc 120
   gggaacggga tcgcaggcgc tttcaggggc atccgaaacg agctgacgga ttgcgccacc 180
   acctttacaa agggcagctg gaaaacgaag gtatcctacc ttgtgatggg cttcggcaat 240
   attgcccggg gtcaggtgct gcggggactg ctgttccttg tctttgaatc tgtgttcatc 300
   gtttatatgg tcacaaccgg cagctactgg ctgggcaagt tccgcacgct gggcgatgtg 360
   ccccccggag aggtatacaa cgaggtgctg gacacctatg ttcgggtcaa cggcgacgac 420
   tcttttaaaa ttctgctgta cggcctcctg accattctct tcatcatcgc ttttgtctac 480
   acatggcggc tgaacgtgaa gcagaaccgg atctcggagg agatccttgc cacgggcaag 540
   aagctcaaaa gcggaaagga cgaccttcgt tccgtcctgg acgatcagtt ccacaagacg 600
   ctgctggcac tgcccatcac gggcgtcctt gtcttcacga taatcccgat tctgttcatg 660
   atcctcgttg ccttcaccaa ctatgacggc gctcacgacg gctacagcaa cctctttaca 720
   tgggtggggc tgagcaattt caacgagctg atcagctcca gcagtgcgga cgcgaacctc 780
   agctacacct tcggcgaaat cctttcgtgg acgctgatgt gggcgttctt tgccacattc 840
   accaattatt ttctgggaat gctggtggcg atcctcatca acaaaaaggg aatccatttc 900
   aaaaagctct ggcgcggcat tctggttctg accatcgcgg tgcctcagtt catttccctg 960
   ctgtatgtct ccaagctctt cgataccaac ggcgttgtga atgccatgct cctgaagctg 1020
   ggctttatcg atcaggccat ccgcttctgg gagaatacca cctccgcccg aattctcgtc 1080
   atcgtaatca acatctgggt cggtatcccc tatctgatgc tggtgagcac cggcattctg 1140
   atgaatattc cgcaggatct gtacgagtcg tccaaaattg acggtgccag tgtctggcag 1200
   cagtatatga agatcacgct tccgtacatg ctcttcgtca cgggtcccta cctgctgacc 1260
   cagttcatcg gcaatatcaa caacttcaac gtaatctatc tgctgaccaa cggccgcccg 1320
   gcaagtccgg atcttgtaac cagcggcggc agtgccacaa ctacggatct tctgatcacc 1380
   tggctgttca acattaccac cggtgccagc agcaactata aactggccgc cgtgattgcc 1440
   atcatgatct tcgttgttgt cgcggttctt tccctgatcg tctacaacgt gatcccgtct 1500
   accagaaatg aggaggatta ttcgtaa 1527
<210> 240
   <211> 1113
   <212> DNA
   <213> Unknown
<220>
   <223> Unknown gut bacteria
<400> 240
   atgaacgtaa ccggtattgt atttaccaac gacgcaagcc ttggtgagct gaccaccaag      60
   cgcaccatgg cctccctgcc cttcggcggc cgctaccgtc aggtagactg ggcgctgtcc     120
   aatctggcct gcgccggtgt ccgccacatc ggcatcatct cccgccacaa cttccagtcc     180
   ctgatgaacc acatcggcga cggtgaggag tggggcctgg agctggagga gggcggtctg     240
   gaattcctga ctccctatgc ccagtccgaa gtccaccgct accggggcaa gctggagtct     300
   ctcgccagcg ccatggactt cctcgcctac ggctctgatg acgagctggt catcatgacc     360
   gactccgcca ttctctccaa catcgacctg accaaggtca tcgccgccca cgtaaagagc     420
   ggccggaaca tcaccgttgt caccaaggcc ggtatctgca acggggaaaa gatgatcgat     480
   ctggccatga agctggacgg gaaaggcaac gtgaccgaca tgctggtgga ctttgccgcc     540
   cccgccgact acgttgcctc catggacatc ttcgtggtgg acaagaagtg gctcatgcag     600
   cagaccagag aaatgattgc ccgggacaag ttccacatgg atcgcgacct cgttctgggc     660
   ggctggcagc ggggaaaggt ctccgtcaat gtctatccct tcgacggtgc cgccctgtac     720
   aacgagtccg tggaggaata tttcaaaaac tccatggcgc ttatcgaccg ggacatccgc     780
   cacgacatgt tcggcggcaa tcatcccatc tacaccaagg ttcgtgaccg tgtccccacc     840
   tactacggcg aggaatgcca gatcgccaac tgtctggcgg ccgacggctg catgctggag     900
   ggcaacgtca aggaaagcat tctgttccgg caggtcacca tcgccccggg cgcggacgtg     960
   gaaaactgcg tcatcatgaa cgactccgtg gtaggcgagg gcgcggaact gaaatacgtc    1020
   attctggaca aggacgtcac cgtcaccccg ggggcaaagc tcatcgggac aaagaagagt     1080
   cctgtcatcg tcaagagggg ggaaaccgta tga                                  1113
<210> 241
   <211> 801
   <212> DNA
   <213> Unknown
<220>
   <223> Unknown gut bacteria
<400> 241
   atgaacattg ccattgtcac gggagccagc tccggcatgg ggcgggaatt cgtccgccag      60
   ctgggcggat acgtttccgt ggatgaaatt tgggcggtcg cccgccgggc gtcggctctg 120
   gaaaccctga aagccgaagc aagcgtcccg gttcgcccca tcgtgctgga cttgctggaa 180
   gaatccagct ttacagaact ggaagccctg ctggaatccg aaaagcccaa tgtcaggctg 240
   cttgtcaacg ccgcgggctt cggcaaattc ggcgcttacc agaaggtccc cgcagaggac 300
   gactgccgca tgatcgacct gaactgcaag gcgctgctgc tcaggacccg gctgtgtgtc 360
   ccctacaggc agcccggcag ccacattctg gagctggaca gcctgtctgc cttccagcca 420
   gtgccgtaca tcaccaccta cgccgccacc aaggctttcg tcctcagcta cagccgctcc 480
   atgaaccggg agctgaaagc caagggcatc cgggtcatgg ccatgaaccc cggctgggtg 540
   aaaacggaat tcttcaatca tgcgttccag accaacgccg ataacgaggt gcgctacttt 600
   aaccgcctgt acgaggccaa ggacgtggtc aaaaccggcc ttaacgacct gtaccactcg 660
   aaaaaggact gctccatcca cggcttcccc gtcaaattcc aggtgttcct ggtgaagctg 720
   ctgccccaca gcctggtgat gaacacctgg cttaaccagc agaaaaaagc aaagaacaac 780
   aaggggctta cgacgaaatg a 801
<210> 242
   <211> 660
   <212> DNA
   <213> Unknown
<220>
   <223> Unknown gut bacteria
<400> 242
   atgctggata aagcgatgat cgaaatgatg gcgcagggca tctgggatac ctgctacatg 60
   acccttgtct ccaccttttt cggctatgtg atcggtctgc cgctgggcat tgccctgacg 120
   ctgacggacg aaaacggcat taccccaaac cgggcggtat accggattct ggatatcatc 180
   atcaatgtca cccggagcgt tcccttcctg atccttctga tcgcggtcat gcccctgacc 240
   aagctgcttg tgggcaagag ctatggctcc accgccacca tcgttcctct gacgctggcc 300
   gcggcgcctc tggttggcag aatggtggaa tcctccttga aggaagttcc cgccggggtc 360
   attgaagcgg cgctgagcat gggcgcgaaa accggcacca tcgtccggaa ggtgctcatc 420
   ggcgaagcca gaacttccct gcttgtgggc ggcaccatcg ttctcggcac ggtgctgggc 480
   tactccgcca tggcgggcgt cattggcggc ggcggactgg gcgacattgc catccgctac 540
   ggctactacc gctacgacac cgggggcatg ctggtgacgc tggtattcat cgtcgccatt 600
   gtgcagctgc tgcagggcat gggcaacctg gcggcgcgga agatcgatca cagaaagtaa 660
<210> 243
   <211> 1170
   <212> DNA
   <213> Unknown
<220>
   <223> Unknown gut bacteria
<400> 243
   ttgaaatatt ggttcggcta cctcaccgcc gccatcttcg gcgccatcac atgggtgctg      60
   atgcggttcg gtgaacggtt ttctactctg gtggatatgg tgtaccccta cgtcatccgc     120
   acctcggagt ccattcttgc ccagtgggca agcggcgtgg acttccccgt ctggcagctg     180
   ctggccgtgg ctctgtgcgc gctgatcctt gccagcattg tgctgatgat cgtgctgaaa     240
   tggaacccca ttcagtgggg cggctgggtg ctcgcgtttt ttgccggtat ttacatgctc     300
   cacaccatgc tctggggtct gaattactac tccggccccc tgtccgacga catgcggctg     360
   gacgtgggca gctacaatct ggaggaactg accgaagcca ccgagttcta ccgggacaaa     420
   gccaatgccc ttgccctgca agtcaaccgg gacggaagcg gtaacgtggc ctttgcggac     480
   tttgatgcgc tggcgggcaa ggccggcgag ggcttccaga cgctgaccta cgactacttc     540
   tatcccattt tcgccggttc cacccttccg gtgaagaagc tgggctgggc agacatgtat     600
   tcctccatgg gcatcaccgg cgtcaccttc ggcctgaccg gcgaggcctg cgtcaatccc     660
   cagatccccg gcgtgtccct gccctttacc atgtgccacg aaatggccca ccgcatgtgc     720
   atcgccccgg aacgggacgc caattttgcc gcgttcctcg ccgcctccgt ccattccgac     780
   ccggaattcc agtacagcgc ctattttatg gccttccgct actgctacag cgccctgagc     840
   agcgtcaata accagtccgc cgcggcggct gccgcacggg tgagcgccgg ggtcaacgac     900
   aatctgaaat tcgacatggc tgcctacaac agcttcttca attcccggaa aagcaccgcc     960
   gccacgaacc tcgccgatgc cgccaacgac acctatctca aggtcagcgg cgacgaaagc    1020
   ggcgtcgctt cctacggcga ggtctgcgac ctgctggtga actggcacat ccagaccgtg    1080
   gtgctgccct ccatcaccgt ggaggaatcc cccttcgacc cctacgacga aacgcaggtg    1140
   gatctgagcg gcatcgtcaa cgcaaggtga                                     1170
<210> 244
   <211> 840
   <212> DNA
   <213> Unknown
<220>
   <223> Unknown gut bacteria
<400> 244
   atggttgcga ccgacttttc ccgggagagg gggtatactt ttgccatacc ggctggaggt 60
   gatgcgttga aaatctatct ggacttggtg gtaattctga actttctggt ggattttttg 120
   ctgcttttgg ggacaaaccg tttatcgggc tttccggcgt cgcccctccg ggcggcggca 180
   gcggcggcgc tgggggcggt ctacagcggg gtgtgcatga tgccccggat gcggttcctc 240
   gggggactgc tgtggcggat cgtcagcctt gccgggatgg cggtcatcgc cttcggctgg 300
   gacaaaagcg catggaagcg cagcggtgtg ttcgtgctgc tcagtatggc gctgggcggc 360
   gcgtcattga gcatcggccg gggaaatttc ctgacgctgg tgctgaccgc cggcggcgtg 420
   tgggcgctgt gcatcgtggc cttcggcgga cgggtgggtt gcagagagta cgtcccggtg 480
   gagctgacct atggcgagaa aactgtcagc atcgtcgccc tgcgggattc cgggaacact 540
   ctgcgcgacc ctgtttccgg ggagcaggtg cttgtggttt ctggggaggt ggcggaaaga 600
   ctgacgggtc tgacgcagga gcaactccgt gcgcccctga aaacgctgat gtgccgcccg 660
   gttccggggc ttcgcctgat cccctaccgg gcggtggggc aggcggggga tatgctgctg 720
   gccatgcgtt ttgacggggt gaaaatcggc agccggatgc agagcgccgt ggtggccttt 780
   gcggcggagg gacttggcag gggagacgta tatcaggcac tgacaggagg ggtcatatga 840
<210> 245
   <211> 849
   <212> DNA
   <213> Unknown
<220>
   <223> Unknown gut bacteria
<400> 245
   atgggaatcc ggacgaaaag taccgtttcc aatactgtgc tctatgtgat ccttgtcctc      60
   atgtccatta tctggctaac gccctttgcg tgcatcttgc tggagtcctt ccggactgaa     120
   tccaccggac gtgtgggcta catcatcccc aagcagtggg gctttgataa ctacatcaat     180
   ctgtggacca aaacgagctt ccccatttgg ttccgcaata cgctgatcac cgccctggct     240
   acggcggtgc tgcaaaccct tatggtgctg agcgtcagct atgttctctc ccggcttcgc     300
   ttcaagggca gaaagctgct gatgaacacc tgcctggttc tgggcatgtt cccgggcttc     360
   gtcaccatga tcgtgctgta taaggttctc agcggcatgg ggctgacggg agccaactcc     420
   gtgccgggac tgattctcgt ctactgcgcc agctccggca tgggatacta tgtgtccaag     480
   ggcttcttcg acaccatccc caagagcttg gacgaagcgg ctcgggtgga cggcgcttcc     540
   cgctttcagg tgttctacaa ggtgatcatg cctctgtcca agcccattgt catttatacc     600
   gttctcactg cgttcatggc tccgtggggc gacttcatgt ttgccaagta tatctcccac     660
   aatacggagg tgggcatgaa tgtggcggtg ggtctgcaat actggatctc ctcgaagacc     720
   ctgattgcca ccaactatac catgttctgc gcgggcggcg tggtggtggc gcttcccgtc     780
   accatcctgt tcatgctcct gcagaggtac tatgtggaag gcgtcaccgg cggcgccgtg     840
   aaaggataa                                                             849
<210> 246
   <211> 2931
   <212> DNA
   <213> Unknown
<220>
   <223> Unknown gut bacteria
<400> 246
   atgaacacat tgttccagaa catcctgact gccagcttcc acggcagcat cgtcattctg 60
   gccgtcatgc tgctgcggct ggtgctgaaa aaaacgcccc gaaagtatat ctgcctgctc 120
   tggctgctgg cgggactgcg gctgctgatg cccttccaga tccaaagcga cctgagcttg 180
   cagcccagtg ccgacccggt ggcacaggtg cagcagcaga tcagccgttc ccagacgccc 240
   gccgcgccgg aagatttctc cccggccgat ctggacgtcc cggcgccggt gcagacgccc 300
   gaagctcttc cggacatcca gactcccgtc accaccgtta tcagcagcac cccctccccc 360
   gccccggaaa ccgcgccgga accggataca ggctttgatt tcctgtcggt tctgccatgg 420
   ttctggcttg gcattgcctg ctgcttcggc atttacagtc tgtacaccta cctgaccctg 480
   aagctgaagg ttcgggaggc cgtcaggatc cccggcggct gggaatgcga ccgcatcgag 540
   accgctttta ttctgggctt catccgcccg aatatctaca tccccatggg catgaccccc 600
   gaggaacagc gttacattct cgcccacgaa cgcacccatc tggacaaggg cgaccactgg 660
   ttcaagatgg tgggctttct ggcgctggcg ctgcactggt tcaatccgct ggtgtgggca 720
   gcctacatcc tgctgtgcaa ggacatcgag atcgcctgcg acgagcgggt ggtgcagttc 780
   atggagctgg aggagcggaa agcctactcc gccgccctgc tgacctgctc caccaatcgg 840
   gcgcacttcg cggcctgtcc cgtggccttc ggcgaggtga gcgtcaagga gcgcatcaag 900
   tcggtgcttt cctataagaa gcccggcttc tggatcagtc tggtgggcgt gatcgccatc 960
   gtcttcgtgg cggtgtgcct cgtgaccagc cccgcgagaa aagatgcggc agccgccggg 1020
   gataccgaac caacctccgt ctccgacgcc gtttccgtac ataatgtgga cgagctgctg 1080
   gccgccatcg cgccggacac cgtcatccag ctggagccgg gaacctacaa tctgtccggc 1140
   gccaaaggct acggcctgcc ctcggaaagc ccctactacg catggacgga aaagtacgac 1200
   ggctttgagc tgatgctgca aaacgtaaag aacctgacca tccgcggcag cggcaaggtg 1260
   aacacgactc tggaatgcga cccccggtct gccagcgtca tcaatctgaa gaattgcgaa 1320
   aatgtcacgc tggaggattt caccgccggt cacactctgg aactgggtca gtgcagcagc 1380
   ggtgtcatct atctgcaaaa ctgcaccggc gtgggcatga accgcctggg catttacggc 1440
   tgcggcatga tcggattgca ggcggcggat tccaaggaca tttctcttgc ggacagcgac 1500
   gtgtacgact gctccagcag cgccgccgag atcatcagct gtgaagacgt caccatctcc 1560
   ggaacccgca tttaccgcat cggctccgag gaatacggcg gatacaccta ttttgatatt 1620
   ggttccagcc ggaatgtgac ggtggaaaac tgcgaaatca gcgacagcag ccttatgtat 1680
   ctggcaatcg tttccggaag caccgttcag atgaagggca atctgttctc caacaaccgc 1740
   agccagctgg aagcattcaa cttccagtat gagtaccacg aagataccca gacgccggca 1800
   gacgaatcga gtatggttct ggacggcaac cggtttgaaa attgcagcgt ccgccgctgg 1860
   tttggagccg acggtaccgt cacggacggc gcaggcaatc ctcttaccga tgagcagatg 1920
   aacaagctgt actccacagg cgcgacggag ccgacccaag cgcagctgga aatccacgtt 1980
   tccacggtgg atgagctgat cgccgccatc ggccccaatg tggacatcgt gctggacggg 2040
   gagctttacg acctcagcac cgccaccggc tacggcacct ccaagggcga ttattactac 2100
   tggctggacg aatttgacgg cccccagctg gtcatcgacg gggtggacaa tctgaccatc 2160
   cgcagcaatg acggcagcgt aacgggacac accatcgctg ccatcccccg ctacgccaac 2220
   gtcctgacct tccagtcgtg cagctacatc accctctccg gcttcaccgc cggtcacaca 2280
   agagagcccg gttactgcag cggcggcgtt ctgcggttcc aggacagcga ccacatcacc 2340
   gtggacaaat gcggtctcta cggctgcggc attctgggcg tagacgcgga atactgcacc 2400
   gatatacggg tcgccaactg cgacatctac gagtgcagcc agggcggcgt ccagttctgg 2460
   agctccgagg gcattgtaat ggaaaacaac accctgcggg atctgggcgg cagcgacatg 2520
   agcttccacg actgcaagga tgtgacggtg aacggtgaaa ccgtgattcc cgccagtatg 2580
   ttcttcagtc ggatcctcca gtcggaggaa cagcagggca cagaggcact caacgccttc 2640
   gttacggagt ttgcgtgtgc ctatttttac ggagatatgg atacgctgcc ccggctgctc 2700
   tccaaagact acgcaggcgg agtggaagcc tattccggca atacaaacga cgtgattgtg 2760
   tgctggcatg aggtcacggt ggacatgtgg aaagaggcag aggccaacgg cacctatgaa 2820
   tttgcctacc cctaccgtga gaacgttgac agcgaaattg cctatctgac catcgtcgtc 2880
   gtcagagaaa acgacgcatg gaaggtcagc agctactctc tggccgaata a 2931
<210> 247
   <211> 1287
   <212> DNA
   <213> Unknown
<220>
   <223> Unknown gut bacteria
<400> 247
   atggagattt cctacaccat tttcgacctg cttttctatc tgctgctcta cgccttcctc      60
   ggctggtgcg tcgaagtctt ttgcgccgcc gtgaaaagcg ggcggtttgt gaaccgtggt     120
   tttttgaacc tgcccctgaa tatgccctgc ggcattgccg ccgcgctgct gctgccggtg     180
   ctgggcacca tgaaccacaa gaccatctta caatatgtca tgacgctggt gatctacatt     240
   ctggtccggt gcgtgaccga ccagttcgtc cggggcgtca gccggggaac ctttgccgcc     300
   gcgccgaagg gaaggccgtt tttcctcaac gcggccgccg cggcggccgt catgctgctg     360
   gtgtatctcg tggtgcatcc cggcatgatg ggacttttgt acctgatgcc aaagtggctg     420
   gaacggacgc tggtcatcgc tgggatcgtc ctgattctgg cggacttcat cggcgtgatg     480
   cgcgccctgc gcaccagcag cgccacccgg ctgaataagg tcgggcagga gaccacccag     540
   cgcatggccg accggatcag caataccatc tggcggcgtc tgcaaaaagc ctaccccggc     600
   cttgcggcag cggatgtgcc ggggccggaa cggtatgtct ttgcccgggg catctgcttt     660
   gacaagctgg tgtgggtgtt tctcgtgacc tctttcttag gggcgctgat cgaaatggtc      720
   ttctgccggg tcacgtcggg gcagtggatg agccgctccg gcgtgctgta cgggagcttc     780
   agcttcgtgt ggggtcttgg cgcggtggtg ctgaccatca ccttgcagcg gattgcggac     840
   aagcccgacc ggcggatctt tctcgcgggc ttcgtcatcg gcggcgctta tgagtacctt     900
   tgcagcgtgt tcaccgagct ggtattcggc acggtgttct gggactacag caaaatgccc     960
   ttgaacatcg gcggccgcac caacgtgctg tactgcattt tctggggact gctggctgtg    1020
   gcatggatca aggtgctgta tccgcccatg agcaagggca ttgaaaagat ttcgccgctg    1080
   ctggggaagg tggttacatg ggttatcatc tttttcctgg cctgcgacgg tctgctcact    1140
   ggtgccgcca tgctgcgcta caccgaccgt cagaacaacc cggaacccaa aaatgtggcc    1200
   gaagcgtttc tggacgacac ctatgacgac gactggatgg aacaccactg gccgaacatg    1260
   gtggtcacaa aggacgtgga atcataa                                        1287
<210> 248
   <211> 1284
   <212> DNA
   <213> Unknown
<220>
   <223> Unknown gut bacteria
<400> 248
   ctgacccggt tcaagcccgc caagcgggac atgtacgccc gggaaggatt cgtctttacg 60
   gcgctggcat gggtggtcat ttccctgatc ggcgcggcgc cctttaccct gtccgggcag 120
   atccccagct acttagacgc cgtgtttgag acggtttccg gctttaccac caccggcgcc 180
   agcatcctga ccgatgtgga ggcgctggac aacgggatgc tcttctggcg cagcttcacc 240
   cactggttag gggcatgggg cattctggtg ttcatgctgt cgctggtgaa tatgggcgga 300
   caggccaacc acctgctccg ggcggaaagc cccggcccca ccgtgagcaa gatggtgccc 360
   aatatgcgca aatccgccgc cattctttac ggcatttaca tcgtcatgac ggcggtggag 420
   gtggtgctgc tgcttatcgg gggaatgccc ctgtttgaca gcctgtgtac cgccttcggc 480
   accgccggca ccggcggctt cggcatcaag aacagctcca tgggctacta taacagctac 540
   tatcttcagg gcgtggtctc cgtgttcatg gcgctgttcg gcgtgaactt caacgtttac 600
   tttttcctgc tgatgaaaaa atatgccatg gcctggcgga ataccgaggt gcgggcgtat 660
   tttgccatca tcgccgggag cacgctgatt attaccctta acattctggg gatgttcccc 720
   acggcgtttg acgcattcca ccacgctttt ttcgccgtct cgtccgtcat taccaccacg 780
   ggcttcagca caacggattt tgacctgtgg ccccagctca gccggtgtat tctggtgctg 840
   ctgatgatcg tgggtgcctg tgcggggtct accggcggcg gcgtgaaggt ttcccggctg 900
   gtgattctgt gcaaagccct tggctccgag ctgcgcaagc tgcttcatcc ccgctccgtg 960
   cgggtgctga cggtggacgg aaagcccgtc agccgggaga cggtgcaggg cgttcagagc 1020
   tacatgaccc tgtattttct ggtgacgctg gtgtccgtgc tgctggtggc gctggacaat 1080
   ctggattttg tcaccacgtt taccgccgtg ttggcgacgc tgaataacat cggccccggc 1140
   ctggggctgg tcggccccac gggctgcttc gcggcctttt cgccgctggc gaagatcgtc 1200
   ctgacggcgg atatgctgtt cgggcggctg gagctgttcc ccatgctgat cctgctcatg 1260
   ccctcgacat ggcggaaata ttga 1284
<210> 249
   <211> 1347
   <212> DNA
   <213> Unknown
<220>
   <223> Unknown gut bacteria
<400> 249
   atgaagctga tactttcctt tatgctgccg ctggttttcg gactgctgtt tcagcaggtt 60
   tacagtatgg tggataccat tgtggtgggg aaatttctgg gtgtggacgc gctggcgggg 120
   gtcggctcta ccggttctat tacgtttctg gtgctgggac tgtgcaacgg cgtgtgcgcc 180
   gggtttgcca ttcccgtggc gcagaaattc ggacagcggg attttgacgg gctgcgccgg 240
   ttcgtcggca atatgatctg gctcggctgc gtcatcgccg cggcggtgac gctggtcacg 300
   acgctgctgt gccgccagat tctcctctgg atggacaccc cggcggatac gttttcctat 360
   gcttacgact atattttcgt gatttttctg ggcatccccg cgaccatgct gtacaatctc 420
   ctgtcgggca ttctccggtc gctgggagat tccaaaacgc cgctggtgtt cctgattatg 480
   tgcagcctgc tgaacgtggc gctggacatc gtgtttattc tgacgctgaa tatgggtgtc 540
   gcgggggcgg gctgggcgac gctgctgagt cagctgatct ccggcgtgct gtgcctgtat 600
   ttcatcgtga agaaattccc gattctccat ttgaagcagg acgatctgcg gctgcggaaa 660
   atctatgtcc ggaagctgct gaatatgggt ctgcccatgg gcttgcagta ttccatcacc 720
   gccatcggca gcattctgct gcaaaccgcg gtcaacggcc tgggcgcgac cgccatggcg 780
   gccatcgcgg cgggcagccg ggtgagcatg ctccttgtct gcccctttga cgccatggga 840
   accaccgccg ctaccttcgc cggacagaat ctgggcgcgg ggaagctgga tcggattcac 900
   cagggggtca aggactgcac ggtactgggc attatcatct ccgctgcgat ttttgtgatc 960
   acctggtttg gcggcagtgc catgaccaca ctgttcctgg atacggcgga cggggcgagc 1020
   gtcgacatgg tggtgccgat gggacagcag ttcctgatca tcaacgcggc gtttgccatt 1080
   ccgctgctgt tcgtcaatct gctccgcttc accattcagg ggttgggcta ctcggagttt 1140
   gcggtgttcg cgggagtgtt cgagatggtt gccagaggtg ccttcgggct ttgtctggtg 1200
   ccggtgcttg gctatacagc cgcatgcttt gccagccccg cggcatgggt gatggctgat 1260
   ctgtttctgt tccccgctta tttccactgc atgaagaagc aggggtattc cttcaaaccg 1320
   acgaaggcca gtgcagcgac agaataa 1347
<210> 250
   <211> 1038
   <212> DNA
   <213> Unknown
<220>
   <223> Unknown gut bacteria
<400> 250
   atgagcaaag aattccttta caccctgctt gacagcatgt ccgtttccgg ccatgagatc 60
   ccgctgcaaa agaaggtcat cgccgagatg accccccact gtgacaagat catcacagat 120
   tataccggca acgtcgtctg cgtgctgaac cccgacgcgc ccttcaaggt gatgctggcc 180
   gctcacattg acgagatcgg tctgatcgtc acccacatcc agcccgacgg cctgcttcgt 240
   gtgtccaagt ccggcggcat tttccccggc gtttatcccg gccatcaggt ggccgtccac 300
   ggctatgccc agaccgtcta cggcgcggtc gtccgcagca aggacatgga caaggccgac 360
   ctgaaagcgg acgagctgta catcgacatc ggcgcgaagg acgccgctga cgcccggaaa 420
   tacgtgcagg agggcgaccc cgtccacatg aacacctacc atcaggaaat gctcaacggc 480
   tttctctccg cccggggcat tgacgaccgg ggctgcgcct acatcattct ggaagccctg 540
   aaacgggcaa aggccatggg ctgctccgtg ggcgtttacg ccgccaccac cgtgggcgaa 600
   gaaaccaccg gccggggttc ccactgggtc ggctctgcca tccgtcccga cgttgccatc 660
   gtcaccgacg tcacttttgc ctccgattat cccggcgtgg atgccaagca gtccggcgag 720
   gtgaagctgg gctgcggcgg cgtgctctgc aacagctcca tcggcagccg gaaggtcaac 780
   gacctgctga aagccgttgc gaaggagcat aacattccct atcagatcga gtcctacatg 840
   ggcagaacct acaccgacgc ggacaacctc cacgtcaccg gcaccggcgt ggtcacagcg 900
   ctgctgtccc tgcccctgcg gtacatgcac tgcccctgcg aggtgtgcca catggacgac 960
   atcgaaaact gtatcgagct gctggcgcag ttcctgtgcc gcatcgatga gcatattgat 1020
   ctcgacccct ttcactga 1038
<210> 251
   <211> 3165
   <212> DNA
   <213> Unknown
<220>
   <223> Unknown gut bacteria
<400> 251
   gagagctgct acacgcaggt cacaacaact gaaaaggaaa ttcttgcctg ccccgtgtca 60
   gcggagagcg ggcttgttgt cgtgcaccgg cacgattcgc gctgctatga cgaagacggc 120
   atgcttgcat gtcctttgcc ggagcttgcg gcgcatgtac ataccgaggc ctgctacgac 180
   taccccgaag cgcatgtcca cacagatgcg tgctattcgg aggaaagagg cgagctgatc 240
   tgcggtttgg aagaaggaga ggagcatacc cacacggacg attgctacgc gtgggagacg 300
   gttttgtcct gcggccagac ggaggaggag cctgccgagc cggtgctcgt ctgcggcaaa 360
   aaagagatcg ttctgcaccg gcacgacgag gattgctacg ccgcggacgg aacgcttacc 420
   tgcgcgcttc cggaagtgat cgaacaccag cacacgggaa aatgtttcaa gaccgttcag 480
   gtccctgtgg acacgcaggc gctcacctgc acgaatacgg accctgacca tgtgcacacc 540
   gcgctgtgct acggcacgtg ggtcctgacc tgcgatcagg gaaccgacga cacgaccccg 600
   gacacacctg cggacgaaga cccggcggac gatcccggct ccgaagaccc ggcagaggat 660
   gaagatccgg acgatgcgga ttctgaagac cccggcgcgg aagactccga aaatcccgaa 720
   gacccggaga accccgaaga cccggaaaat ccggaggacg agcagccgga agacggcgcg 780
   gacacctacg cgtccgagca gatgacgctg cggtactacg tttatctcga cggctcgctg 840
   gatcctgttt tggaggagtc cagaacgctc gagcgggaga acggacgcta ttacatcacg 900
   gcgcagcagc tgcagaccgt gtacggcgat tttggtttcg aggcggacag ctatcaagga 960
   gaacgcttct tcccgcatac tgatctgcac gacacatcgc ttatctgggc ggacgcgccg 1020
   gcgtatccgg ccgaaaacgc ggacggaacg caggaatggc gcattccgct cagccgcacc 1080
   acaacaagct atatctacta tctgccccac aacacgcagg actacggcag ctactttgaa 1140
   ggcagtgcct ccaacaccaa tgcggcgctc atcagcgaca acacgttcta caccgtgaac 1200
   atttccgcgc cgcccgatgc cgcgtcggag agcaagatct atcacgtact gaacggagcg 1260
   acgttcagcg tcgagctgtc tgtgttcgac gactacacct ggagctacac caacctcgcc 1320
   gatggcagcg agatcacgcc gagcgatgtg caggagctgg aaggcggcaa gcttttcatc 1380
   aagttccgga acgtctcgca tcccatcaag gtcactgcgc gcaaaacgat cgccgacgaa 1440
   cagatctata ccatccgcta ccatgcagat acgctcgccg actgtctgga acgaatcagc 1500
   agtgcggtta gcgccaacgc gcagagcgtt atcaccgacg gcaaggtcgg cggcatggcg 1560
   acgcttaccg aacaggtaac ggtcacggcg gatacgatct atccgctccg ttcgccggat 1620
   tcgctgcgcg tggtgtcgca ggcagacccg accagacagg ccaagcacta tttctacacc 1680
   ttccgcggct ggcgcgtcaa ggcgaccaac accgttctcg accccgcgca gccgctgacg 1740
   gcggtgcagc tggcgggcta tgaaaacggc ggcgtgatcg agctggaggc ggtctggagt 1800
   gtaaaagacg caaatggcag agccacatcc gtcaacttct ttattcacct tttctgtgag 1860
   atcaaggact tccacagcaa cggcttcacc aacaacccgg tcggtgacta taccaagtcc 1920
   ctgttcacca cgacgctgct cggaactgat gcactctcct cgtccgataa ccttctggtt 1980
   cttgcgcctc cgacgactga cgcaacggcc tacgaaacgg atgacatcct gcgcaccatg 2040
   acggatacgc cctacgatgg cctgacgatg gaagcatttc cctctgaccg cgagatgttc 2100
   gccctgctcc gcgagaacaa ctacaccatc aaaatcggca atagcacgat cccgcaaagc 2160
   aagctgactt cagactattt tcaggttcgc tggaccaacc tcaaatacga tatcagcgac 2220
   ggctggcatg tcgacggtgt gcttgtagcg aaggaagcaa agctccgcgt aacgaagaca 2280
   ttcctcggaa gcgccgcagc cattgcaagc gttaaggcgc agaccggcgg gatggaatac 2340
   ggaatccgga ttcaaaacac gacgacgaat gccgaagagc tcctcctcac gctcaaccct 2400
   gccgggcagg agcagcgcac ggattgcgtg ggatatgacg actacgatgc cgccagcaat 2460
   acctatacct ggatcgtcag cgggcgcgtc gacagtatct atgcgctttc cgagcagaac 2520
   tatcttttgc aggatgtgcc gtcgacctcc tattaccgca tatctgacat ggcaaaaccg 2580
   ggtgactggc agaactatga cgctgacagt cagcccacca cggctatggt ctcctacgcg 2640
   acggataccc cgaaaaacag gtaccgcacg gtatccttcc gcaacaagta tgtcgatgcc 2700
   ggtacgttga cgctgcgaaa gttcgactcc ttcacacaca atggcatcgc aaatgtggaa 2760
   tttctgctta ccgcagatga cggcgttagc ggccagcatc tgcaccgcaa gccgggaacg 2820
   agcatgtata ccaacgccag cacctccggt tcggaatata ccgaagcgac ggggaacagg 2880
   atcgtgacgg acgtgaacgg tgacgcatac cttcgcctga cacccggcgc atacacactg 2940
   caagagacat tgccgaccgg atacagcgga gctgcggaga tccggctcac tgtcgacgag 3000
   gagggcagcc tgaccgcgct gacctttgac ggcgcgccgg ccgcctcttc gcccggcggc 3060
   gtggcatccg gtgtgggaac cacgcttctg gccattatga atgattcgca agttctgacg 3120
   acagttaccg ctgtgaagga ttggggcgag gtgccggacg agtgg 3165
<210> 252
   <211> 1602
   <212> DNA
   <213> Unknown
<220>
   <223> Unknown gut bacteria
<400> 252
   ctcaacaggc ttgcgggtga cctgattttc agggacgaga gcgggaacga tgtgacgcag      60
   gacgtccagg gcaagttcac agtcacggaa gcaagcttca acgcacccgg cttctttcag     120
   gcctattgca tcaccggcaa gatcgcgatc aaggacgagt acgcgtcgga acttcagctg     180
   aacaaggaga gcttcacgat caatggcaaa atcgagaagg ccaagcctgt cttgacgggt     240
   ctggagttca agcccaaaaa acagctttac gccggcgact cgcttggctg gaactttgac     300
   aacaacaacc tttcgggcgg cacggccacg atcaccgctt acaacggaag caccgtaacg     360
   gttcccggca cgttctcctt tgatgagggc gagcttcatc ggggaagccc gctgaagggc     420
   ggggagaacc gtcttccggt gcgatttgac ccaaaggacg ttgcaaacgt cgaagaggca     480
   gcctgcaaag tggttgtcaa tgtgccctac cgcaattatg actgcgtcag cgcgtcgtat     540
   ctctatcccg catatggcac ggcctttaaa gatctgaacc tgccggattc cgtgaccgtc     600
   aacgcccggt atgacgcaag cgacggcggc gtgggcttca actattacgc agacatcacg     660
   tgggatgctt ccaaatacga cccgacaaat ctcgacgaac agatcatgta cggcacattt     720
   gataggtcgt attgggagga ctatatcgcg ttcccggagg gcgcggatgt tcgggtcaag     780
   cttgtgatca aggtgcagcc tcccgtacag gctgagacgg aaatcacaaa gcttcctacc     840
   ttccagtgga caaaaagact ggacaatggc ggcacgttca agcttccggt aaacaccatt     900
   ttcctgttta ccaagtttaa agttggaacc tactccgggc aaaccggcgc gctgaccggc     960
   ggcaaggcca gaattaaggg cacggatacc gagctttccg gtacattcgc cctcgagttc    1020
   ccctacgggc agacggaaac gttctacagc gaagcgggca cgaaggaagt aaccgtggtc    1080
   ttcaagccgg aaaatagcct gttctatgcc gaagcaagag cgacgctttc cgttgaggca    1140
   atcaagctca cgctggcgaa gatgtacaac acagtctcca tcacggacaa ggacgtggga    1200
   acgcagttcg cagatctggg gcttccgaca tctgtaggct tcgagacgga ggacggcacg    1260
   gtgaagttca tctctgtgga atggaatgcg gagggatatg acaggaattc ttctgcggag    1320
   cagaccatta cgggaacgct gaaatacgac tcgtttgacg aacgcgaact caatcccact    1380
   acgctcaccg cgacggcaac ggtcaagctc attccgccgg cgcatgtgca cgcctacacg    1440
   cagaaggttc agaaggcgga gaccctgaaa acgcccgcaa gctgtacgga agacgaggtg    1500
   tactacctgt cgtgcgaatg cggcgagatc agcccgaacg aggccgatgt gttcatcgcc    1560
   gaaaataccg ccctagacca tgactttggc gactggacgt cg                       1602
<210> 253
   <211> 708
   <212> DNA
   <213> Unknown
<220>
   <223> Unknown gut bacteria
<400> 253
   acatgccaag cacgaagaaa tccggggaag gggagtgatc ctatcgcaat cacaagcgcg       60
   agcctgccat cgaaaaagcg catcctgacg gtctgcgtca agctcttttt ggagcagggg      120
   tacaagaaga cgacggtcgc acagatcgtc cacgaggcgg gcgtttccaa cagcacgttt      180
   cagaatattt tccgcgccaa ggacggcgtt ctgacagagc tcgtcgagtt catgttcggc      240
   aaccagtttg ccatggcgcg ccagacggcg gggcagacgc tgccgcccat ctatgtctac      300
   gcggtcgaga cagccattca gatgacgctc acagagctca acgagaattt gcgggaaatt      360
   tatcttgagg cctatacgca gaaggaagcg tccgagtata tcttccgcga gacggccaag      420
   gagatccagc agattttcgg cgcgtaccag ccggggttca cggcggccga tttttacgcg      480
   atggagctcg gctcggcggg catcatgcgc gcgtatatgg cgcagccgtg cgatgaagcg      540
   ctgacgctcg agcggaagat ccagctgttt ctgacgatga gcctgcgcgc ctaccgcgtg      600
   ccggaggagg agattgagaa gacgctggcg tatatcgcgg ggcgggatat ccgcgcggtc      660
   tcggagcggg tcatgcacat cctgtttcag acgctcgcca tgcgctac                   708
<210> 254
   <211> 810
   <212> DNA
   <213> Unknown
<220>
   <223> Unknown gut bacteria
<400> 254
   aagctttcgg gttacgtgca gttttatgca ttctgtacgg tgtggctcga acaggacggg 60
   agctttgaag cgcactacgc gggcgctttg gagcatttta accgccagct ggacgagaac 120
   aaagaccaga tcgtccgctg caaaaacgaa gcggatgcgc gcgcggccat cgggagcgga 180
   aaatgcggtg cgttcttgtc gatcgagggc gcggaggcca tttcctgtga ccccggaaaa 240
   ttagagcttg cctgcgcgca gggcgtgcgc atgatcgcac cggcgtggaa cgcgcagaat 300
   gcgctgaccg gaagctgcat gacaggcgga ggtctgacgg cacagggcaa agaattcgtg 360
   cgccgggcgc agaagcttgg catgatccta gacgtcagcc atatctcgga gaaagcgttc 420
   tgggatcttt gcgacatcac agaaaagccg tttgtggcga gccattccaa ttcgcgcgcc 480
   atctgcaagc atgtccgcaa tctcaccgac gagcagttta aagcgttgtg cgaggcgggc 540
   ggtacggcgg ggctgaatct ctacggcgca tttttacgcg atgagggcag gccgacactg 600
   gaagacgtct ggcgtcatat cgagcatttt ttggagctcg ggggagacgg acatatcgcg 660
   ctcggcggcg atctggacgg atgcagcgtg ctgccggagg gctttgccgg tgtggacagc 720
   tatgcaatgc ttgcgcagtg gctggaagac aggaagctgc caagcagtac aatagaaaat 780
   atattcagtg agtctttatt aaaggttgtg 810
<210> 255
   <211> 2169
   <212> DNA
   <213> Unknown
<220>
   <223> Unknown gut bacteria
<400> 255
   atggtttccg gtcttgcggg cagcaagggt ctgttcgact tcgtcggagc ctgtgtgatc 60
   aaaaacctta ccgtttccgg tgcgattaaa gaaggcacaa acatgggtct gcttgcagac 120
   gtcagcgcag gtacggtaga aaactgcttc accacgggtt ctttgcacag aatcaactcc 180
   tacggcacaa caggcggcct catcggacgc gcagacgcag gtacggtaat ccgcaactgc 240
   ggttctgctg caaacgtcag ctgctcgatg aagagcctga aagcagagct caacatgggc 300
   ggtctggttg gcaacctgta cggcacggtg gaaaacagct acgcgaccgg taccgtcaag 360
   gtggaagcgg gcagcggcta tacggcggtc ggcggcttca tcggccagac caaaaacact 420
   gcggccatta cgaacagcta cgcggcaggt acagttaccg gttctgcggg cggtgcgctc 480
   ggcgcgttcg tcggtgtgaa cagcggctcg atctccggca gctactaccg cgaggatgcg 540
   gcagaagctg cggtcgcgac cggaagtgca gacggcctca cggcgatgac gaaggctgtc 600
   atgcagagcg atggcttcat caaggatcat ctcggtctgg aacgctatca tgtcgacacc 660
   gacggcatca acggcggcta cccgatccac gcatggcagg gcggcaaaga agcggaactg 720
   accgaggacg aaaaggcagt cacgctcgac gttcgcgcgc ttgtcctcaa agatgcaaaa 780
   caggcggcgc ttgtacagga gctttgcgcg caggtgcagg aggagacgga cgcgtttgtc 840
   gcggaaactt ccctgagtat gctccttacg tacatccgcg acgactgggg ctatgactgc 900
   ggtgtattga cctcgccgga ggacgcaaag ccctacgtct atcagcagtt ctgcaaggaa 960
   aaagaagccg agtatctgga agaatacggc gagcagatcg acctgagcaa caccgagggc 1020
   attttgacgc ccgataacaa cggcatatac catatcaaaa cgcaggcgca gctggtgctt 1080
   gcagcgacgg gtgaaaacgg cagccggatc tcctggcatt cggacagcga cgcactgaac 1140
   gcggaaaccg gcgcagtcac gctccccgag gcagacacga tcaccgtcag gctcaccgcg 1200
   accgcggcaa agggtacggc agaaaagagc cgcgagatca ctgttattct ctactctgtg 1260
   aaagctgagg ccggaaacgt gctcggcagc atcgaaaaag cgctgtcggc aaaggatgcg 1320
   tttgtccagc ccgtgcagat ccgcggccac gagacggcaa tggacgccgt ttcctactgg 1380
   ttgtataaca acggctatga cgacagcatc gaggtcgaat ttgtcagtgc aggtacgctt 1440
   gcgacaacct ctgttaccgg ccattcgtac ctcgcagaag acggaaccgt tacctattat 1500
   cagggcgagg gcggcacgga caccaagtat gtgacctaca ccggcgtaca gttcaagctt 1560
   tccaaactcg gcgcagagaa gacggttacg gcgaacgtgc gcatcggctg ggatgtggaa 1620
   aaagcgcagg aactcatgca gagggcgctc gacgagcagc tcacatggga agccatccgc 1680
   ggcgaaaaca ccaatacggc aaaggccagc acggataaga gctttaccgg catggtcgtg 1740
   gacggagaag tttccgaaat gctcgtcgca ccgcgcacgg tcacagtgga cggcgttgca 1800
   gtccgcgtcg gcttcagtgt gaacccgtct gacgcggtga cctatgaatt tacggacagc 1860
   cagatcaaca tttcgccgcg ccgtccgtca cagggcaaac ctgccacaac gttcgagctg 1920
   cggatggtca cgctgttcga cgaaaacctt gacccgtaca cgatctctgc catgatgagc 1980
   agcagcggcg acaatgtcag cacgctccag ctccaaaacg cgttccgtat tacggtaaag 2040
   cccgagacag aagatgcgca gaatgtgctg tcgcagaatc tcaaggataa actccccggt 2100
   cttgtgacca actactatga ctacggcgcg aaggtcgacc tgacgaagcc tgtcaaggac 2160
   gatctgctg 2169
<210> 256
   <211> 669
   <212> DNA
   <213> Unknown
<220>
   <223> Unknown gut bacteria
<400> 256
   atgagtttgt ttgtttccca gctgtttaac ggattgcaga caggttctgt gtatgcgctg 60
   gtggcccttg gctacagcat ggtctatggt atcatcctgc ttctgaactt tgcccacggc 120
   gacatcatca tggtcggcgc gtataccgcg ttctacgcca tgactgcgtt ccacctgcac 180
   ccgattctct ccgttgttct ggccgttgtc acaagtacgc tgcttggcgt tgtgattgag 240
   aaggttggtt atacgcctct tcgctcggcg ccccgtcttt cgcttctgat cacagcgatc 300
   ggcatttcgt tcctgcttga aaacggtgcg cagcttctgt tcggcgctga caccaagagc 360
   atggacacgc ttgttcccgg caatgtgtcg ttcggctccg tcacgatcag ctatactgcg 420
   atcttgacca tcatcgtcgc tgtcatcgcg atggtcgcat tgacgctcct tgtccagaaa 480
   acgaagctcg gaaaggcgat gcgcgcggtc tctgaggata tgggcgcggc gcagctgatg 540
   ggcatcagcc tcaacaaaac gatctcattt acatttgcgg tcggctcagc gcttgccggt 600
   atcggctcgg ttctgtatct ttgcgcgtat tcccaggcat cgcccacgat gggctcgatg 660
   ctcggcctg 669
<210> 257
   <211> 681
   <212> DNA
   <213> Unknown
<220>
   <223> Unknown gut bacteria
<400> 257
   atgaacaagt acgaagaaat tgcgcgctgc ctcaaagctc gtattcggtc cggcgcatat      60
   atccccggcc agaagctgcc ctcggaggcg gagctctgcc gcgaattttc tgcgagccgc     120
   ctttccgtgc gcagcgcgat cggccagctt gcggcgcagg ggctcatccg gacgcatcag     180
   ggcaagggct cctttgtctg tccgggcgcg ccgtcagggg atacagagct ttcctttcag     240
   ggcgcttcca tcagccggac ggattttttt gagctgcgcc ggatcctgga gacagagatc     300
   gcgggtctgg ccgcccaaag agccgggcag gagacgatcg accgactgcg cgagctctcg     360
   tttcagatgc agcgcgccga gcgggaatcg gagatggcgg aggccgacga ggcgtttcat     420
   ctgcttctgg ccgaggcctc acagaatgcc gccatccggg ccgtttttca gatgctccgg     480
   ccgtatatcc acacgatgat ggtgcagaac gtctccgtat tgggcgcaga cggctgtacg     540
   gcgcatctta aaatcgtcgc ggcgatcgag gcgcgaaact ccgaacttgc caagacccac     600
   atggcagagc acctcaaccg ctccatggag cagacgaata tgctcaacta tgtgaaaaag     660
   ctggacgcac cggccgactg a                                               681
<210> 258
   <211> 1095
   <212> DNA
   <213> Unknown
<220>
   <223> Unknown gut bacteria
<400> 258
   atgctggttc tgctcggcgc agtggtgctt ctggccgttg tgctgatcgt catcatttcc 60
   agctgctcga agaaaaatca gggtgcaggc atcaccgcgc aggccttttc caccggctcg 120
   gagctgacgc ttccggtcaa caccgagctc aacagcggag actatgtctc ctacggcggg 180
   taccagtttg aaacagggac gaagctttcc aagatggcaa agctcatcac gaaaaataac 240
   gagaacgtca cggccacaag ctacgataac gcctacggct cggcctacct cttcacgcgc 300
   gacacaggct caggtgtgga aagctggtgt ctgtaccaga aggatccggc gcacatctcc 360
   aagtggtaca tcttcatggg caaccaccgc gaggtcgagc tggcggatgg aacgctggat 420
   cttctcttgc cgctccacct gatttccgac agctatctgc gcgacaacat gggaagcaag 480
   ctttctctcg gcacagctta cgcctgcggc acaaagagcg ccgaccagac gatgctcgag 540
   cagttcaagg aattctacgc gaattccggc ctctacaata tgatcacgct cgaaacaggc 600
   ttcattctga cggataaagc caccggccgc gaggtcgagt tcagctttga agagaacgac 660
   gagtcctcgt ggttcacgct caagagcacc atccccgaag agcccgagcc ctcgagccgc 720
   gtccagctca gcagcaccga cgcagcctcc acggacgagc ccaccgagct ttcggaggcc 780
   gacgcgctca cgctcagcaa gatcctgatc gatgcaaact acaagaccgg cacgaccgcg 840
   tccgagtatc cctataaggc aagcctcaat ggtacggatt acatgctgga gctcacatgg 900
   aaggacgaca cctggtccgc ctccgtccaa agcggcgacc agaccgcgcg ccttaccacc 960
   aagcaggcct gcaccgtcgc ggccattctt gcctccaatg gcgtgatgaa ggttgaaagc 1020
   gacgagagct cgtggccgga tgaggccggt accgtcagcc cgatgggcgc gtgcatggtc 1080
   acgacaaacg acgtg 1095
<210> 259
   <211> 819
   <212> DNA
   <213> Unknown
<220>
   <223> Unknown gut bacteria
<400> 259
   ttgccaaagc tgctgttcca gcgggtcgtt gtggtgtcgc tggctatttt gctgcaaatt        60
   ttattcgtga ttgcgggcat ggattggctg cgcgaatact ggcactggat gaagaccgca       120
   atggacgtgg tcaagtgggt cgcggtgctg acgatcatga ccggaagaag caatccgtcg       180
   tataaaatgg cgtggatcat cctgattctc gccttccctg tcgcgggcat tacgatctat       240
   ctgctcttcg gcggcaaccg tatctcaaac cgcgagaacc gccgcatgaa ccgcgtggag       300
   gtcatggttg cgcagcacct gcggcaggat cagaccgtca tgcaggcgct cgagcgggag       360
   aacattccgg catggaacca cgcgcgatac cttctttcga gctcgcgcta cccggtctat       420
   gataactctg cggccgtcta tttcccgagc ggcgagagct gctacgcgcg gatgctcgaa       480
   gagctcgaaa aggccgagca ctatattttc ctcgaatttt ttatcatcaa taacggcaag       540
   atgtgggact ccattctgga ggttctgcac cgcaaggcga tgcagggtgt agacgttcgc       600
   gttctgtacg acgatttcgg ctgcatcacg cgccttccga tgcgctactg caggaagctg       660
   cgcgagatgg gcattgaggc gcacgtcttt aacccgttca tccccgtcct ttccggccgg       720
   ctcaataacc gcgaccaccg caagctcatg ctcatcgacg gaaaggtggg atttacgggc       780
   ggcgtgaacc tcgcggatga atacatcaac gaaatcgag                              819
<210> 260
   <211> 858
   <212> DNA
   <213> Unknown
<220>
   <223> Unknown gut bacteria
<400> 260
   atgatagcac tgaaaattct gcctgcgggc gagaaagcat acgaaaggaa cggggctatg        60
   aagatcacat ttctcggaac gggcaacgcg caggcgacgg cctgctacaa tacctgcttt       120
   gtcatggagg aaggcggcaa gtgcctgctg gtcgacggcg gcggcgggaa cctcctgtta 180
   agaagattac aggacacggg ctataaatgg caggatatgc gagacaacat cgtcacccac 240
   aagcacatcg accacctgct cggcatcatc tggatgcagc gcatgatctg ccagaacatg 300
   gcgcgcggaa aatatgaggg tgaggcgcgg ctgtatgcgc acgaggaggt catccggatc 360
   cttcatcaga tggcggagat gctgctgacc gagaaggagc ggaaattcat tgacgagcgc 420
   tttttcctca tcgaggtcaa agacggcgag acgcgcgaga tcatcgggca gaccgtgcag 480
   ttttttgaca ttcactctac gaaggccaag cagttcggct tcgcgatgac gctcaaagac 540
   ggtcgtaaaa ttgcctgctg cggcgacgag ccgtattgcg aagcggagta cgaatacgtc 600
   aagggcagca cactcatgct ccacgaggcg ttctgcctct attcccaacg cgagattttt 660
   gagccctaca agaagaacca ctccacggtc aaagatgcat gcgagttggc ggagaaactc 720
   ggcgtgccga acctcgtgct ctaccacacg gaagacaaga gcatcgaaaa ccgcaaggcg 780
   ctctataccg aagagggcag gcaatattac catggcagcc tgatcgtccc ggacgatctg 840
   gaaacactgg aactttaa 858
<210> 261
   <211> 615
   <212> DNA
   <213> Unknown
<220>
   <223> Unknown gut bacteria
<400> 261
   atgcaaaccg gcatgtgggc ggtgttcgcc gcttcgttcc ggaaaaacct gaccgccatt 60
   ttcggtctgg acggcaaaac cgcgcgtacg gtaactgcga acgcaaagca aacgtatgcg 120
   cagattttaa aggagcttcc ggactttgag aagggcgacc ggttcctcat gaatattgtc 180
   agctgcgcga tgctggccgc gttcgttctg agcatgccag agcgcccgga tgtaacgcgt 240
   ttgacctcgt attacgagca gtccatgatg acccccgcca tgcggtttta ctgccgcaag 300
   agcggaagca ggcgcttttc tcccgagagc ctttcagcca tggaaaaaac tgcgcagttt 360
   cgcgccgcgg accggaaccc gtattcctgg aacatggact atctccccta tgccgacggc 420
   tccggctttg aagcgcgctt cacggcctgc ggcatctgtc agctgatgaa aaagcttggg 480
   ctctacgatc tcacgcccgc tctgtgccac ctcgactacc cgatgaccga gaccggcggc 540
   acaacgaaat ttgtccggga ctacacgctt gcctccggcg gcccctgctg cgactgcggc 600
   taccgcaagg tctga 615
<210> 262
   <211> 990
   <212> DNA
   <213> Unknown
<220>
   <223> Unknown gut bacteria
<400> 262
   gaaacagctg cgcccgcagc cgaagaaacg gccgccgagc ccgctgctgc tgaaacggca      60
   gaagaaacct ctgaagctga ggaagcccct gcaactgaag aagcaaagac gagccttgac     120
   atcgtgttca tccccaaggg cgttgcagac tactggagca tcgttcaggc gggctttgaa     180
   aacgcggcga ccgagcttgg catgacgccg agagttgtct atccgtccaa ggaagaagcc     240
   ggcgttcaga tcgaaaccgt gatggacgtc atcaactcca agccggacgc gattgtcctt     300
   gcccctgtga gcggcgacgc gcttgtctcc gcctgccagg aaatccagaa ggccggtatt     360
   ccgctgatca tcgccgatac cgccatcccc acggaggact atctccagtc gttcatgacc     420
   gataacgtcg ccgccggcgc gatggcagct gacgagctgg ctgcgctgat gggcgaagag     480
   ggccaggttt acatcttctc cgattctccc gcgaaggctt ccgttgccga ccgtgtgacg     540
   ggctttgagg cgagaatgaa ggaagcttat ccgaacatcg aggtcctcgg caccctctac     600
   agcgaagcgg atatcaacct ctgcacgacc cagtgcgtcg atgccatcac ggcaaacccc     660
   gacatcaagg gcatcttcgc tgttgacgag aaccgcaccg cgggcgttgg caccgcgctc     720
   atccagctcg gcagagaaga catctttgtc ggcggcttcg acgcgaacaa gaacaccgtt     780
   tctctgatgg aagacggcgt gatcaacttc ctgactgtcc agcagccgta ccagatgggc     840
   tacatggcgt acaaggcagc ggctgacgcg gccaacggcg tggagctcga gcacggcatg     900
   gtcgacaccg gctgcaccat cgtcccgaga gacaagctgg acgacgagga catgcaggcc     960
   atcctcttcc cgctggagca cctgaactga                                      990
<210> 263
   <211> 1203
   <212> DNA
   <213> Unknown
<220>
   <223> Unknown gut bacteria
<400> 263
   atggaacctg aaatcagtct ttcgtgcaaa gacttagtca agacctatcc cggcgttatc      60
   gccatggatc acatcaactt tgaactgcgc aagggcgagg tccacgcgct gctcggagaa     120
   aacggcgcag gcaaatccac gctgatcaag tccattgcgg gcgcgcacca gtgtgactcc     180
   ggcacgatca ccattgacgg caaggaattt cacagtctga cgccgcttca ggcgaaggaa     240
   atgggcgttg aggtcgttta tcaggaattt aaccagatgc cgtctttatc cgtcgcggag     300
   aatatttatc tgaccgagct gcgcggcaag cgcattgtcg ccgatcagag agagtttgag     360
   cgcaaggcag cggagctttt caaggagatg aatctcgacc tgaacccgaa ggtcaaggtt     420
   gcatcgctga caaacggcta caagcagatg gtcgaaattg cgaaggccat ttccaagccg     480
   actaccaaaa ttctgattct cgacgagccg acggcgcctt tgaccgtcga ccaggtcgac     540
   attctgttcc ggctgatcca caatctgaag gagaagggaa tctcgatgat cttcatctcc     600
   caccgtatgc cggaaatttt tgagatctgc gaccgcgtga ccgtcatgcg tgacggcaag     660
   tcgatccaga cgctgaacgt gaaggacacg aaccgtgagg agctcgtctc tttgatggtc     720
   ggccggaagc tgaacgaaaa cttcccgccg cgcgagacag aaatcggcga tctcgtgctc     780
   gaggcaaagg acctgtgcgg caacggcgtg gagaacatct ccttccagat ccacgcgggc     840
   gagatcctcg gctttgcggg tctggtcggc acgggccgta ccgaaacgct gcgccttgtc     900
   tacggcgcgg acaagatgga ctccggcgag gtttacatgt atggcaagaa gcttcatatc     960
   cgctcgccga agcaggcgct ggcgcatggt attgcgcttg tccccgagga cagaaagctt    1020
   cagggcgtgt tcctgcacat gagcatcaag gcaaacacga atatttcgat tcttccgaag    1080
   atctcgaggg gcattttcgt ccaggcgcat aaggaaacgg aaaacgcgga tcagtacatc    1140
   aagagcttgc gcatccgtac gcccggcatg atgcagctgg ccggcaacct ctccggcggc    1200
   aac                                                                  1203
<210> 264
   <211> 786
   <212> DNA
   <213> Unknown
<220>
   <223> Unknown gut bacteria
<400> 264
   ccctttgcct ttacgggtaa taaatttgag ttccggatgc ttggctcggc caactccatt        60
   gcctgcacga acattatgct caacggcgct gtcgcgcagt cgctcaaagg ttttgccgat       120
   gcgctagaga aggcagatga ctttgaaggc gcgctgcacg atctcatccg ccgcacgatt       180
   cacgcgcaca agcgcatcat cttcaacggc aacggctacg acgacgcatg gatcaaggaa       240
   gcaaccgaac agcgcggcct tctgaactta agaactacgc ccgacgcgat gccgacgctg       300
   ctggaaaaga aaaacgtcga catgctgacc ggcctcaaga ttttcacgaa ggcggagctc       360
   gagtcgcgct acgagatcat gatggaaaac tacgtcaaga ctgtcaccat cgaggcgcag       420
   acgatgctgg acatgacgcg caaggagatc ctgcccgccg ttgccggcta cacgaaggag       480
   ctcaccgaga cactggctgc caagcgcgcg atcttaccgt cgctgccggc aaaatacgaa       540
   accggcacaa tcacgaagct ctcttcgctt ctcgaccaga tcgacgcggc ggcagaggag       600
   cttgaaaaaa ccacgctcca gatcaaagcc attgacagcg tcaccgagtc cgcgttccag       660
   attcgcgacg cgatgctccc gagaatggcg gcgctgcgcg tggtgtgcga tgaggcagag       720
   cctctgaccg ctgaacgctt ctggccgttc ccgacgtacg acaagcttct gtttggtgtg       780
   agataa                                                                  786
<210> 265
   <211> 864
   <212> DNA
   <213> Unknown
<220>
   <223> Unknown gut bacteria
<400> 265
   atgaaaaagc cgtccaagcc cctgcacgca cgcggcgagc tcaggtccgc catccggcgt 60
   cagcccgtcg tgtttgctgt ctatctcgtt ctgcggctca tcgttcttgc gtcgctcgtg 120
   tcggccatcc tccggcagga atatgaaagc gcgtttgtgt gcgtgctggt gctgtttttg 180
   ttcatgcttc cgtttttcat ccagaaaaac ttcggcatct gccttccgag cacgctcgaa 240
   atcatcatcc tgcttttcat cttcgcagcg gaaatcttag gcgagctcca gagctacttc 300
   atccagtacc cctactggga cacgatgctt cacacgacga acggctttct ctgcgcggcg 360
   gtcggctttt cgctgatcga catcctgaac cgcgacgcaa agatcaagtt tacgctctcg 420
   cccgtgtatg tggcgctggc cgcgttctgt ttttcgatga cgatcggtgt tctgtgggag 480
   ttctttgagt tcgggatgga taggcttttc cacatggaca tgcagaagga tacgatcgtt 540
   cacaccattt cgtccgtgat gctggacccc acgaacagca acattcccat caccattgac 600
   ggcattacct ccgtcgcggt caatggccgg gatctaggct ttcacgggta tctcgatatt 660
   ggcttttacg atacgatgga ggacctgttt gtcaacttca tcggcgcgac cgtgttcagc 720
   gtgatcggtt acttctatct caagcaccgg ggcgagggca aatttgcaaa ggccttcatc 780
   ccgaccattg aagaaacgaa caaggagcct gattctgacc atgtcaaaga gccctcgcag 840
   gaagctgaaa atcgggacaa ttga 864
<210> 266
   <211> 867
   <212> DNA
   <213> Unknown
<220>
   <223> Unknown gut bacteria
<400> 266
   atggaactgc gcgtcttacg ctattttctc gcggtggcgc gggaggaaaa tatcacaaaa 60
   gcggcggcgc tcctgcatgt gacgcagccg accctttccc gccagctcat gcagctggag 120
   gaggaactcg gcgtcaagct ctttcgccgc agtcagtacc gtgttgtcct gacggatgac 180
   gggatgctgc tgcgccgtcg ggcgcaggag atcgtggagc tggcagacaa ggccgaacgg 240
   gagctgcagc atctcgatgc ggaactgacg ggcgagatcg ctatcggctg tggcgaatcc 300
   gtgggcatga cctttctctc ggaacacatt cgtgcgttcc gcaggctgca ccgtcaagtg 360
   cagttccgta tttacagtgc caacgccgac gatgtgaagg agcgcattga aaaggggctg 420
   ctggacatgg ggcttctgac cgagccggtg gacattggcc gatatgcttt tctgcgaacg 480
   ccgcaaaagg accgttgggg cgtactgctg ccaaaggagc atccgctggc acagaaaagc 540
   acggtcacgc cgaacgattt gttgggtgtt ccgcttcttc ttagcagccg cgaaagcgtc 600
   cgcaacgagc ttgcggggtg gttcggcgat acatacgaca aaatcgaagt tgcagccaca 660
   tttagcctga ttttgaacgc ggccaacatg gtaaaaaacg gtgtgggcgc ggcgctgtgc 720
   tatgattttg gaaatctctc tgatgcgctg gaatttgtgc cgctttctcc aaagctggaa 780
   accggaacgg tgttggcgtg gaagaaggat cagacctgct caccggcggc ggatcaattt 840
   ctcgactttc tcaaaaatgc gatttaa 867
<210> 267
   <211> 879
   <212> DNA
   <213> Unknown
<220>
   <223> Unknown gut bacteria
<400> 267
   atgcgcgcat tgaaaaccgt cggatatgtc atcagccata agaatgggga gaaccgaaga 60
   gcactttttc cggcccagtg cacgcagctg aaatttccgg agcaggcgat ttttgagacg 120
   ggctacggtc ttagcctcgg cttttctgac gcggactatg aacgcgcggg cgtgaagatt 180
   gcctcccggg aggaggtttt gcgggcggat gtgatcgtag acgtgaagct cggagatgcg 240
   gagtatctgg acgcgcttgt gcccggaaag atcctgtgcg gctgggcgca cacggtgcag 300
   aagacggact ttgccggaaa agccatccgc gcggggcata cggtcatcgc gcttgagcac 360
   ctgcgggaaa acgggcggta ccttttttac cgcacccgcg agattgcggg cgaggctgca 420
   attctgcatg cgttccgcta ttgcggcaaa atgccctacg actgcaaggt ctgcatcgtc 480
   ggcaacggcc agacggccaa gggtgcgatg cggatgctga cggggctcgg tgcggaggtc 540
   gatatcttcg ggcggaagat ggaaaagacg ttccgcaagc tcatgttcca gtacgatgtg 600
   atcgtcaact gtgtctattg ggacacctca cgctccgacc ggctcatcta ccgcgaggat 660
   ttaaagagaa tgcgccccgg gacgatgatc atcgatgtga gctgcgaccc gtatctggag 720
   atcgaaacgt cccacccaac gacgatcgac catccggtct acaccgtgga cggcgtgatc 780
   cactacgcgg tcgacaacac ccctgcgatg tacccctaca caacttctaa aattttcagc 840
   cggaactttt taaaatatgc cgatgcgctg gcaagcggc 879
<210> 268
   <211> 915
   <212> DNA
   <213> Unknown
<220>
   <223> Unknown gut bacteria
<400> 268
   gtcagcatcg tcccgccgct gtcggttgca atttcgagct cgtccgcaat tctctcgtgg      60
   aacattgagg gcgacgcgcc ggagagctgg actgtcacga tcacaggcgc agacgattac     120
   tccgacacgc agatcgtttc cacgccgtct gtcacctttg agggtctgac cccgggcgag     180
   acgtatgagg tcatcatctc cacgcccacg atgctgcaaa gcgtctcgac gtcgatcacg     240
   ccgaacatca cgaagctctc ctcgttcaca gcgaacgtcg acggcgaaga tttggtcctt     300
   gactggacgt gcgaggtcga cccggcagag cccggctgga cgctcgaggc aaagatcgcg     360
   ggaatcgata cgcccgtcac acttcccgag acagaggaag cgcctgttcg cattccgctt     420
   tccgatcttg taccggacgc ggactatacg ttcaccgtag cgctcaagag cggcgaaaag     480
   ctcgagggtg agacgaaaac gacgtatcac tcccccgccg tcacggacaa atacaccgac     540
   cacggcatgg cgggcacata tcttggcttc ttcctgaagc ccgcgaatgt ggaaaccgtc     600
   aacagtacga atatttccac cacgcgcaac aactattaca ccatcaacga gcagatggcc     660
   tttgcgctgc agacgctcgt gacgctcgac acaaaggcgg aggaagatat cgccgtcatg     720
   cttgttgtcc gcgacgaggc gggcaagctt gtaagcttcg aggagtcgca tcggcaatgg     780
   agcacgatgt gggagaaaaa tctcttcgtc ggtacgctct cgagcatgcc gcagaaagcc     840
   ggaacctaca cgcttgaaat ctacttcaac cataagctcg tcaccaccaa ggacttcaac     900
   gtccgcgaat cgtaa                                                      915
<210> 269
   <211> 900
   <212> DNA
   <213> Unknown
<220>
   <223> Unknown gut bacteria
<400> 269
   ctcctgctgc ttctttgcgc ctgtacatct gaaaaacaag cagatttaca gccggagacg       60
   ccggatattt cggaaagcgc cgtctcctgg aacgacctga cgtttgaccg gacgcttccg      120
   ctctcttacg ccacgcagtt ttccgtcagc tacgcgggag acgattatac ccgcatcacg      180
   atcggaagcg atcaggagtt tcttctggtg gcagaaaacg ccgagacccc ggagcatatt      240
   ccggaacgcg tcaccgttct gcaccggccg ctttctcata tctatctggt tgcgtccgcc      300
   gcaatggact attttgacca tctggacgcg gtcgaccgga tttccctcag cggcctcaaa      360
   gcagacgact ggtacatcga aagcgcgaag gccgcaatgg aagagggcag catgcgctac      420
   gccgggaagt attccgcgcc ggactacgag gcgattttgg agagcggctg cgacctcgcc      480
   attgaaaaca cgatgatcta ccacacgccg gaggtcatcg agcagctgca aacgaccggc      540
   gttcccgttc tggtcgagcg ctcgagctat gaatcgagcc cgctcggaag aatcgaatgg      600
   ctcaagctct acggcgcgct ggtcggaaaa gaggacttgg cctgctcgca gtatgacgag      660
   ctccttgccg cgctgtctcc cattctcgat caggaggcaa gcggtcaaac cgtcgcattt      720
   ttctacatca attcctccgg cgcggtcaat gtccgcaaat cgggggacta catcgcaaag      780
   gccatcgcga tggcgggcgg gcaatatgtt tcgttcgacg aaagcggcga ggaaaacgcg      840
   ctgtccacga tgacgattca gatggaatcg ttctacgcct ccgctgtcga cgcggatatc      900
<210> 270
   <211> 1041
   <212> DNA
   <213> Unknown
<220>
   <223> Unknown gut bacteria
<400> 270
   tacagctatg atcccagcct cccggctgag gttcaggcaa cgattcctaa ggacccggac      60
   acctacactg tgggccagaa ggtcacaacg gcagctaagc ccaattctgt gagagttggc     120
   gaaaatatct ggacgttcaa gacgtggaag cttgatggcg tcgaggttgc accgaacacg     180
   caggttgaga tggtttccgg cggtctgcac tttgaaggca tctgggaggc gaaactgatc     240
   cactacagcc tgacaatcca gtacgtcgat aagaagggga aggaactggc cgagagccat     300
   atggatctcc tgtccaaggg tgaagcgtac aaagtagact ctccgaagat caagggctac     360
   aagctcaaga acaaggatga cgcggtgatc gaaggtacaa tgcccggtcg taatctgacc     420
   atcgaggtcg tctacatcaa gaagagcacc ggaggcagtt caggtacgat cgactccccg     480
   aacaagccta cgacgaaggc accggcgctg acgctcaaca ccgatgacca cttcgcgttt     540
   atcaacggtt atcccgacgg ctccgtgcag ccggaaggca atgtcacccg tgcggagacc     600
   gcagcgatcc tgtaccgcat catgggcgac gagtgccagt cctactacaa gaccaacagc     660
   agctcctatt cggatgttgc ccgcggcgac tggtacaaca cctacgttgc tacactcgag     720
   aatgccggcg tgatcgtcga caccagaacg aacggcaagt tccgtccgaa tgacgcgatc     780
   acccgcgccg agctcgcttc catgatcgcg cagttcgccg gtctggagag cgcttccgct     840
   gcgaagttca acgatgttgg ctcgcgctac tgggcagccg aggaaatcgc gattgccgcg     900
   aagatgggct ggatcaacgg ctatcccgac ggcagcttcc gcccggaccg caacgtcacc     960
   cgtgccgaac tcatggcaat ggtgaaccgc gcgctcggcc gcacgccgaa gtctgcaggc    1020
   gatctgttgt ccggcatgaa g                                              1041
<210> 271
   <211> 903
   <212> DNA
   <213> Unknown
<220>
   <223> Unknown gut bacteria
<400> 271
   atggaacagc tctatcactt ttccgcgccc ggccgcacgg agatcagcgg caaccatacc 60
   gaccatcagc acggctgcgt tctggccgcc gcggtcgata tgcagacgga cgctgaggtt 120
   cgcctcaacg gtacacacct gattttaatc gactctgagg gcttcgcgcc ggtcacggtt 180
   gacctcgacg atctgtccgt ccacgaaacc gagaagaaca ccacagcctc gctcgtgcgc 240
   ggcgttgcgg cggcatttca ggcgcgcggc tgcaagcttc agggcttcga cgcgaaaatt 300
   tcgtccaccg tgctccccgg ctccggttta tcgagctcgg cggcctttga ggtcctcatc 360
   ggcagaattt taaacggtct gttctttgag aacaagctct cggccatcga gattgcacag 420
   atcggccagt acgccgagaa cgtctatttt ggaaagccct cggggctcat ggaccagatg 480
   gcgtcctctg tcggcgggct cgtgttcatc gactttgcgg accccaaaaa gcccgtcgtc 540
   gaaaaggtcg actttgactt tgcccactgc ggccatacgc tctgcatcat cgactcccac 600
   gcaagccacg cggatctgac ggatgagtac gcggccatcc cggtggagat gaaaaaggtc 660
   gcggccttct tcggaaagga agtcctcaac gatgtggacg agagcgcgtt ctacgcttcc 720
   cttcctgccc ttcgcgcaag ctgcggtgac cgcgccgttc tgcgcgcgat gcactatttt 780
   gaagagaatc agcgcgtccg gctccaggtg cgcgcgctgc gaaacgacaa ttttggagcg 840
   ttcctgacct acgtcaacga ctccggccgc tcgtcgtggc tcaagctcca gaacgtcacg 900
   ccg 903
<210> 272
   <211> 717
   <212> DNA
   <213> Unknown
<220>
   <223> Unknown gut bacteria
<400> 272
   tatgccacgc agatggaggc gggcgtggat tctgccgacc ttctcatcgc ggtgacgatg 60
   tcggacgagc tgaaccttct ttgctgcctc gtcgcaaaga aaatcggcgc gcggcacaca 120
   atcgcccgcg tccggaaccc ggagtatagc ggcgaactct cgctcatctc ggacgatctg 180
   gggctgagcc tttctgtgaa ccccgagctt ctgtgcgcaa cagagatcgc tcggacgctt 240
   cgtatcccgt cggctatcaa aacagatacg tttgccaacg ggcatgtgga acttttaaag 300
   gtgcagatcc cgcaggactc ccttatcgcg ggcaagacgc tcatggagct tccgagcctc 360
   gtgaaggcca agatcctgat ctgcgcggtc gagcgcgggg agcaagaggt ctatattccc      420
   tcgggctcgt tccggctgga ggcgggcgac cgcatctcgt tcgttgcgtc catccgcgac      480
   gcgcaggcgt ttttccacca gatcaagctc gcctcgtcgc gcgtgcggac ggtcctgatc      540
   gtcggcggcg ggcacattgc gtattatctt gccaagcagc tgacggacct cggcgttggc      600
   gtcaaaatca cggaggtcaa ctatgagcgg tgcttgcagc tctcgtcgct tcttcccaag      660
   gccgagattc tgcatggcga cggcacaaac gaaggcttcc tgcgcgagtg cggcgtt         717
<210> 273
   <211> 2010
   <212> DNA
   <213> Unknown
<220>
   <223> Unknown gut bacteria
<400> 273
   atggaggaag gtatgaagaa gagaattgtc tcgctggtgc tggcgctggt tctggtgctc      60
   gggctgctgc cggggcctgt gatggccgcg tggcccagct tccggggaaa cggaagcaac     120
   atgggcatta cagacgtgca gacacccgcg cctgactatg caaagctcca atgggcggct     180
   aaaatcggca cgtcggcgga gagcatgacg acaagcaccg tctatgccgg gcagccggtc     240
   gtggagggtg agtttgtcta tgcgacggcg gggcggacgc tttataagat caaggcgctg     300
   gacggaaacg tggccgtgca gcaggtgatg gacgggaagg accgtttcgg cacgatcccg     360
   ccgacgatcg ccggggacac gatctatgtg gcgctggacg gcggcatggt cgaggcattc     420
   aacaaggata cgctcgcgaa gaaatggacg cttggcgggc tttccgccgg aggacagaac     480
   agctgcccgg ttctggtgtc cgaaggccgg gtctacacgg gattttatga ttcggagacc     540
   ggcacacaga actatgtctg cctgacgacg gcgggaaagc gcgtctggag ctatgcaaac     600
   gcgggcggct tttactgggc cggcgcggtc gcggtcgggg actatatttt tgtcggcacg     660
   gacgatggcg cgaagagcgg tgtgaacgga acctcgcgca ttttgagctt ccgcaaggac     720
   gagacgcgcc aaaatcccgt tccgcaggtg ctcgagctgc aagaccttgg cgaccagaga     780
   agctcgctcg cgtattccga acagaccggg cggatttatt ttacgacgaa gggcggctat     840
   ctctgctcgg cgaaggtgga cggtacgacc ggtacgctca cggagctcag gacctgcctt     900
   ctcggcatgg gaagtacgag cacgccagtc tattataagg gctatgtcta tgtcggaacc     960
   ggcacaggtc cggggccgag cggtgggttt gctattgtga aggagaacgc gcagacgggt    1020
   gatctgacgc tccaaaggga gctcacgatg cccggcagca cgcagggctc gccccttctg    1080
   agcacagcgc gggaagcgga ggggtatctc tatttctatg aatgcgcaaa ctataagccg    1140
   ggcgggctta tctgcatccg ggtgaacgcg aatcgcccgg agagcacgac cgcgtcggac    1200
   gtctttacga tctttgacgc ggcggattat ccggagtact gcgcggcaag cccggtctgc    1260
   ggagacgacg gcaccattta ttacaaaaac gactccggct atctgttcgc ggtcgcgaaa 1320
   acgcaggctc cggcggtcaa gtttacccgg catccgcaga gcgcggcata tacgcccgga 1380
   aagacggcac aggcccttac ggtggaggca gtggcggcag acaatgcggc gctcagccat 1440
   cagtgggaga agtcttcaga tgaaaaaagc tggacggcca tcgaccgtgc gacgacgagc 1500
   agctatgtcc cggaaattcc ggcgaaaatc gagggcgagc aggtaaccta ttaccgctgc 1560
   aaggtgacgt ctaccctgaa cgggaagacg gcgtgggatg tgtcgcacac ggcggccatc 1620
   acggtcaagg cgctcagcgc ggacacgacg atttcctata ttgtgaccga cggcaacgag 1680
   gaaccgacag gcgcaccgac ggcggcttcg gtcagtgaaa caacagttgt gctcaactat 1740
   gcggagctgg gggcgaagaa accgcgcgtc tggttccagc cgccgcaagg cggcacgctg 1800
   cgcgtaacgc ttggtacgtg cgacgacccg gcggcgcgct atgacggttc gggtgattat 1860
   accgaccgcg tctacttccg aagtggtata acgggcacga atatcatccg tgtcaccgcg 1920
   acggcggaag atggcgtgac gcagacgacg tataccttcg ttcttacgcc ggacggaaac 1980
   tatgacggcc tgcggagcgt ctatgtgacg 2010
<210> 274
   <211> 600
   <212> DNA
   <213> Unknown
<220>
   <223> Unknown gut bacteria
<400> 274
   ttgtgttttc aacagctttt atggtatact ataagcatat tagaaatcct tgccaaagag 60
   gtgactgaaa tggaagaacg cggattcatc cgggacatgc tggatgtgaa ggttttgatc 120
   ctgtttgtgg cggcgacggc gaagtatccg ctgacgctgc agaagatcta tgagctgtgc 180
   tatcaggacg atcggctgag ctattttgat gtgagtattg cagtgccgca gatggttgag 240
   agcggacatc ttgagcagct ggcggacgag acgtatgtga ttaccgaaaa gggacgcgag 300
   caggaggaag tgacgcgcga ctcgatcgcg tttcccgtga tgcagcgggc aaaagcggcc 360
   gtggaccaat ataacgcgaa gacccgtcgc agcgagttag tgaaaagcga ggtcgttgag 420
   caggaggacg ggagctgcgc ggtgcggatg tacttaagag acgacgtggg gagccttatg 480
   aagctggagc tgaccgcgcc gagcaagacg cagggaaggg ttttgcagcg ggcgtttgag 540
   cagaaggcgg agatcatcta taacagcgtt atgaagcagc ttctgcagca ggagaaataa 600
<210> 275
   <211> 528
   <212> DNA
   <213> Unknown
<220>
   <223> Unknown gut bacteria
<400> 275
   tacgagaagt tctatgcggc gtttggcagg catatcaaat acggcgtgct gaacgattac      60
   ggcgcgaaaa aggagctttt gcaggacctg cttctttact ggtcccacaa gcagaacaag     120
   ctcgtctcct tcaaggaata tgtggacgcc atgcccgagg agcagaagaa tatttacttt     180
   gcaagcggcg aaaatcggca gagacttgcg cagctgccgc agcttgagaa gctgggggag     240
   aagggcttcg acgtgctgct gatgaccgac gaggtcgaca gcttcgtacc gcagacgctc     300
   ggaaagtatg ccgaaaagga cttccgctct gtcacgcagg aggacctcgg cctttccacc     360
   gacgaagaaa agaaggaagc tgccgagcag gccgaaaagt cgaaggcaac gctggagttt     420
   gtgaagcaga cgcttggcga cgatgtgaag gaagtgcgcc tttctgaaaa cctcggtacg     480
   catcctgttt gcatggtacc ggaagagggt atgacgtttg agatggag                  528
<210> 276
   <211> 2718
   <212> DNA
   <213> Unknown
<220>
   <223> Unknown gut bacteria
<400> 276
   atggcttcat tgtccgtatt ggtgattgcg ctggtttttg attcggtcga tggtattgtc      60
   gatctggaaa tttatatcca atatgttttt gcatcggcag tcgtgttgtt gttttctttt     120
   gtcattcttt acctgtttcg atttttcccg ctggcaagat ccggactgaa ctggaataat     180
   cgttcctcga tcatcattat taaaaagcat ttcatggctt tttttgccgt ttccctgatt     240
   gtttcgataa tcgcttatct ggttttttta tatcttgcca gaaaccagag tgaaaaggtt     300
   gatctgaatc tggcgcggat tgccaaaatg caagtaaaac aggtgaacga ctggcggaac     360
   aggcaggctt acctgtcgga aagatatgca ggtgatgtca ggattattca tgaggcgaga     420
   ggtttcgaag aaaaaatgaa ccggcttggg aaccgaatga aaaacggtat cgctgacgag     480
   atcgggggag atcctgaggt tcagagtatt cacaagcttt tttcgttgac aggacgttat     540
   gatcttgtca atgtgctcaa atacaggcgg gtctggatgg gaccacccaa aaagataaaa     600
   gacataaacg gaaaatggct caatgtgctg acgatgtatg tcccttttct gaaaatgaaa     660
   cctgaaggca aggaagcggg tatcaaaacg cattatgtcg atccttccga ttcgctgatt     720
   cccaggcttt cggttttgcc ggcgatcggt atgtcatcca gagtttttct tttcagccgt     780
   tatggtgaag agattgttgt tttgcgcaat tcattgaatg accatgttgt ttatccattg     840
   aaactgagtg agtcgagtcg ggaacaatgg ttaaagaaaa tgagtgatgg tgtacagggt     900
   attttccatg aactggatta tcgtggaaaa cgtgtcatcg gttatgtaga gaaaattccc     960
   gattcagact ggtcgttgat gatcaagatg gatgaacggg aagcgtacag tcagatccgg 1020
   cagatggcgt ggtacattct ttcgatcgct tttttgctga cggctatggc cgggctggca 1080
   ctttttttcg tattgcgtca tcggcatatg caacatcaaa cggagcgatt gaaaatggga 1140
   ttgaaacaga aagcgatacg tcagcactat gattatctga gcaagtttgc caatgatgtc 1200
   attattctga tggatggaga cggaacagtt atcgaggcaa acgaccgttc ggaaagaatg 1260
   ttcggcaagg tccgttccga gatgataggt gaaaatatca gaacggtgtt cgatctgaaa 1320
   gatgatttcg ctttcgagga aaaatggaat gatctggagg aagaaaaaag cctgattttc 1380
   gaagcgtatg gacagcatac gaatggcagt atttttcccg tcgaaatcag ttcgagggtt 1440
   atcgaagctg acggccgtct gtttatccag ttcattatac gtgacatatc cgaaaagaaa 1500
   aaagcggaag aaatggtctg gagacatgcg aattttgatc agattaccgg tttgcccaac 1560
   aggcggatgt tttcggaaag gctgtttttt gaaacacgca aggcgcagag agcagggtct 1620
   tctgtcgctc tcatgtttct tgatctcgac catttcaaag acgtcaatga tacgatggga 1680
   catggtgtcg gcgatctttt gctgaaagag gcaagtgacc ggctgagatt gtgtgtccgc 1740
   gagaccgata ccgtgtcccg gttgggtgga gatgaattca ctgtaatcct ggccgatctg 1800
   ggagatttca aggatgtgga gagagtggca cagaatattc ttgatgaact ggcggaaccc 1860
   tttgctctgg aaggacaaac ggttcatatt tctgccagta tcggtattac gttttttccg 1920
   gaagatgcca ccgataccca tgatctgatg aaaaacgccg atcaggcgat gtatgccgca 1980
   aaaagtctgg gaggaaacca gtatcattat ttcacacagt ccatgcagga agcggtgata 2040
   cagagaatgc acatgatcaa tgacttgcat gtcgctctgg aaaacaatca gttcgagaca 2100
   gtgttccagc cgattgtcga tctgaaaaca ggcgggatct gccgtgccga agcattgatc 2160
   cgctggcatc atcctgttcg cggtgttatc aatccggccg catttgtacc actggcggaa 2220
   gatacaggtc tgattcaggc ttttggcgac tgggtttttc ttgaggcggc cagacaggct 2280
   gcaaagtgca gacgatatat ccccgatttt caagtaagtg tcaatatttc tccggtccag 2340
   ttcaggaagg gcggtattga taccgatgtg tggtttgccc atctgaagag tctggatctt 2400
   ccgcctgaag cgatcgtgat cgagattacc gagggattgt tgcttgagat tgacgaaagt 2460
   attaccgatc agcttctggc tttacgtgat gccggtgttg aagtcgcact ggatgatttc 2520
   gggacggggt attcttcact ggcctatctg aaaaagctcg atatcgatta tgtgaaaatc 2580
   gaccagatgt ttgtcaagaa tctgtctgtc agcgaggagg acagggtatt gtgtgaagcg 2640
   atgacactga tggcgcataa actggatatc gccgtgattg ctgaaggtgt cgagacacag 2700
   gcgcagttga agatgatt 2718
<210> 277
   <211> 996
   <212> DNA
   <213> Unknown
<220>
   <223> Unknown gut bacteria
<400> 277
   atgaataagc ctaaagttgt attgactcat tgggttcacc cggaaattat cgaattgctg      60
   cgggaaaagg ctgaagtcgt cgccaatccg ggccggaaaa cacttccgag agaggtattg     120
   ctggaaaggg cgaaagatgc cgatgccttg atggctttca tgccggactg tattgacgag     180
   gcctttctga aggcctgtcc cagattgaaa attatcgctg cggccctgaa aggattcgac     240
   aattttgatg tggatgcttg tacccgtcat ggtgtatggc tgactatcgc accggatctc     300
   ttgacgattc ctacagcaga actgacagta ggcctggttc tgggtatcac aaggaatatg     360
   cttgagggtg acaggcatat acggagtgga caatttgccg ggtggcgccc ggaactttat     420
   gggctcggat tgtacgggcg gacagccggt attgtcggtt tggggcttgt gggcagagct     480
   gttgctgaac gcttgaaagg gtttggcatg aatctggttt attctgatga aatttcactg     540
   ccgcctgatg aagaagaaca gcttgggctt acgaaagtga ccttttcagg attgctggat     600
   tcaagcgata tcgttatccc tttgctgccg ttgacggacc ggacgtttca tttgtttgac     660
   agaaatgcat tggcaaggat gaagaaaggc agttatctgg tcaatgcctg ccgtggctcg     720
   gttgttgatg aaatggccgt tgtcgaatct cttgaaaagc ggcatcttgc agggtatgcg     780
   gccgatgttt ttgaaatgga agactggatt cgtccggacc gtccccgttc gatcccgaag     840
   gcattgctgg agaatacggc gcaaacattt ttcaccccac atctggggtc ggccgtagat     900
   gatgtcagga tagaaattga acgttattgc acccggagta tttttcaggc actggatggc     960
   aaaattcccg aagggaaggt aaatgatatc tgttga                               996
<210> 278
   <211> 1599
   <212> DNA
   <213> Unknown
<220>
   <223> Unknown gut bacteria
<400> 278
   ctttatcaaa tattgagtgc agacattgca gaacagcgtg gtcttgatgt gtatgcttat 60
   gagaccatgt tggatgtcgc aaaagaaact gcagatcctc gtcttgccag aagagcggct 120
   gagatcgctg tcaaaaatag aaacataaaa gatgctttgc aagcagtacg cctttggcac 180
   aaattggccc cgcaatctga agaagctcag aagtatttgc tgggatttct ggtgctggac 240
   aacagactgg atgaggtcaa agattatttt tcttccagac tggctgcggc aacaccagag 300
   aaacgggtag cgctgtttta tcagcttcag cagcttctcg caggaaccaa gaacaaatcg 360
   gatgcttttg cgataatgga agaagtcgcg gcaccgtatc aggatatacc tgaagcacat 420
   ctatcgctgg cagtatccgc cttgatgaaa aacgatcgaa gcagagccag agatgaagtt 480
   caaaaagcac ttgttttaaa accggattcc gagatggctg tgttgacata tgcacaagtt 540
   tcttccaata ccagtgaggc cattgatatc ctttctgcat ttttgctgaa atatccggag 600
   tcacgcgaag taagaatttc acttgcccgt ttgttgctgg gacaaaagaa gtatgaaaac 660
   gcaagacagg aattcgagat attgctgaat tccgatcctc aggatttgat ggcgctctat 720
   tcgcttggat tattgtccat tcaggaaaat gactatattt cggcagaaaa atatctggaa 780
   aggtatcttg gaacatcttc tgctcagtcc acggagaatc gtcaggaatt aatacaggtt 840
   ctgtttttgc tgtcccagat agcggaagaa cagcatcgat atgatgaatc acttcagtgg 900
   ctttcccgaa taaacccgga tgaggatgat gaggttgcat tgggtgtgga gatcaggaaa 960
   gctcagattt atgcaaaaaa aggaaatgtc aacagggcaa ggaaaatcat tacggatttg 1020
   gcgaatgaaa atccttatga acgggaaaaa ttgttgctga ctgaggcaca aattctgaaa 1080
   gaagtcaaaa gaacaaaaga tgctttcaat gttctcaagt cgggagttgc gcaatttccg 1140
   cagaatacca atattctgta cgactatgct ttggccgcag agaatctggg gaaatacggg 1200
   atcatggaag aatcattgaa acggatcatt gaaattgatc caaattatca gcaagcttat 1260
   aacgctttgg ggtattcatt ggcagatcgt aatcttcgac tggatgaagc atttatactg 1320
   atcgataagg caataaaact ggcacccgat gacccttata ttatagatag tcttggttgg 1380
   gtttttttca gacagggtga tcttgattta gctgaacaga atttgcgtca tgcctacaaa 1440
   ttgcgatcgg acaatgagat agcaattcat cttgccgagg tgttgtggac aaaaggaaat 1500
   cggcaagagg ctttgtcgct tttcagtaaa gtcaaaaaga aagatcctga taatgatctg 1560
   ttgaattcaa ctttgacacg tctgaaaatc aaactttag 1599
<210> 279
   <211> 1347
   <212> DNA
   <213> Unknown
<220>
   <223> Unknown gut bacteria
<400> 279
   atgaaaattc gaaaattttt ctatggtctg atgtttgtcc ttttctgtgt acaaaacggg      60
   gcatatgccg aaccggcaaa agagttttat cttgaaaacg gtatgaaaat cattgtcaag     120
   gaagaccatc gggctccggt agctgtgcat atggtctggt atcgtaccgg atcgatggat     180
   gaaacgaatg gaacaacagg cgtcgcgcat gttcttgagc atatgatgtt caaggggaca     240
   ccgaagtatc ccgaaggtag cctgagcaaa acggtcgcaa aactcggagg taaagacaat     300
   gcttttacca atactgatta cacggcttat tttcagcaga tccccaaaaa cagtcttgag     360
   aaaatgatgg aaatggaagc ggatcgcatg agtaatctgc agttcgcgaa ttcggatttc     420
   gagaaggaaa tccgcgtcgt catggaagaa cgacgctgga gaacggatga ccagccggaa     480
   ggacgtgtcg atgaagcatt aagagcggca gcctttgtcg cccatccgta tcattggccg     540
   gttatcggct ggatgaacga tttgcagaat atgactgttg aggatgccag aaactggtat     600
   gaaagatggt atgcaccgaa taatgcgatc atggttgtcg tcggtgatgt cgatgccttt     660
   gcagtcagga atatggcgaa taagtatttt ggcaagatcc ggcctaaaaa aatcgttccg     720
   gtcaaacccc agctggaacc ggagcagctt ggtcctaagc gggttgctgt ttccgcacct     780
   gcagaaaatc cgatggtcgt actggcttat aaggtcccgg ctttgagaga tgtcgaaaaa     840
   gatgacgatg tttatgcact cgatgtgctg gcttctgttc tggatggcta cgataatgca     900
   cggcttccag cttctttggt gcgcaaggaa cggatagcgc tgtcggtcgg gacagattat     960
   tccgccctgt cgcgtggtcc ggcattgttt gtgttacaag ggattcctgc aaaaggtgtg    1020
   acggttgacg agctggaaaa gcgtttgcgt catgaagttg ccagtattgc gaagcatggt    1080
   ataaccatgg aggaattgca acgggtgaag atgcagttga tttcgtcgca gatttacaaa    1140
   cgcgattcaa tgttcggaca ggcaatggaa atcggtgttt ttgaaatgac cggcatcggt    1200
   cagaaacgga ttgaccatgt tatcgggaag ctcaaggagg tgactcctca acaggtacag    1260
   cgagtggcgc aaaaatattt cacggacaat gcattgacag tcgcaacact tgttcctgag    1320
   gttttgcctg atacaaaggt taaatga                                        1347
<210> 280
   <211> 1488
   <212> DNA
   <213> Unknown
<220>
   <223> Unknown gut bacteria
<400> 280
   atgaatgcga tgaacctgat tcccgccata cccgaactga tactggttgt atcggtgccg 60
   cttgttttgc tgatcgatct gtttttgccg caaaacagaa gggatatcac gtttttcctg 120
   tcgattctgg cgatttgtgc ctgtgcagtg gccagtttcg tccttttgcg tgaaaaggcg 180
   gtcgtgtaca cctttgccgg ggcgttcgtg tcggatactg tggcgaacct gctgaaattg 240
   tgtacctgcc ttgccatgct gttcacgctg gtttattcac ggcgttatat caatgaaagg 300
   cgaatgacgg tcgggcatct gggaggcgag ttttacgtgc tggcgctgtt ttcgatgctc 360
   gggcagatgg tggtcatttc cgccgccagc ttcctgaccc tgtttctcgg aatcgaactg 420
   atgtcttttc ccctttatgc cctgatcgcc ctgaagcgcg atgacgggag ggcggtcgag 480
   gcagccacga aatttttcat tctgggagcg cttggttccg ggcttctgct gtatggcatt 540
   tcgatgttgt acggggctac cggatcgctg gatttcatgg aagtggcgag gatctcggct 600
   tatgccggta cgaactggct gattatggtt ttcggagtgg tgttcgtcgt ggcgggaatc 660
   gctttcaaac tgggcgctgt ccccttccac ctgtgggtgc ccgacgtttt tgaaggctct 720
   ccgtcagctg tgacgcttct ggtcgcaggt gcgccaaaac tggccggttt cgcactttgc 780
   atgcgcgttc tggtggatgc gttgctgcct ctggcatacg actggcagca aatgttgatc 840
   gtgatttctg tcctgtcgat ggctctgggc aatattaccg ccatattgca gaccaatatc 900
   aagcgtatgc tggcctattc gacgatagcc cagatgggat acatgttgct ggggatgctt 960
   gccggtgtga aagacggccg tttcgggatg gagtccgttt tcgcctacag tgcatccctg 1020
   tattacacta tcgtgtacgt tctggcgacg cttggcgcgt tcggtgtcat tctggtgatg 1080
   tccgccaatg gcgccgaggc cgaaaaactg gccgatttcc ggggactgaa ccgtcgtaat 1140
   ccctggtatg ccctgatcat gttgttgtgc atgttttctt tcgccggcgt tccgccactg 1200
   gtcggttttt acggcaagtt gtccgttttt caggccgtcg tcaatgtcga tctgttctgg 1260
   ctggctgtcg tcggcatttt ctttgcggtc gtcggcgcgt tctactatct gcgggtcgtc 1320
   aaggtcatgt atttcgatga accgccgggt accgggaaaa ttcatgtacc gaaggacatg 1380
   gcggttgtgc ttggcatcaa tggtgttctg gtactgttgc tcggaatctt gcccggaccg 1440
   ctgatggact ggtgcatgag tgcggtccag cgcgcgtttc tgatgtag 1488
<210> 281
   <211> 1224
   <212> DNA
   <213> Unknown
<220>
   <223> Unknown gut bacteria
<400> 281
   caggaaaaaa acgacgacaa gaaccagttt ggattattca atgaacctgc tttccagcaa 60
   ggtgaatttg tcggaaaaca aaaatatgaa acggtactga ccgaaaaaca gcttgataac 120
   tggctttgta aaatcgatcg ggccgaactg acttcgttgg acacggaaac gacttccctg 180
   gattccatga aagccagatt ggtcggaatt tccctttccg tcgaaccagg tcttgcggcc 240
   tatattccgc tcgcccaccg tacgattgaa cctgtcgaac aattgcccgt tgattatgtt 300
   ctgggaaaac tgaaaaactg gcttgaaaat ccctgcaaaa agaaagttgg ccagaatctg 360
   aaatatgact gtcatatttt cgccaattac gacattcacc tccaggggat cgagcatgat 420
   actttgcttg aatcctatgt actggaatcg cacaggcgtc atgatatgga cagcctgtct 480
   gagcggttac tcgaacataa accgatcagc tatgaagaaa tttgtggcaa aggcgcttca 540
   caaatcggtt tcgatcagat tgaactggat tgcgcaacag aatatgcggc ggaggatgct 600
   gacgttacac tacagctcca caaaaaaatg tggccacaaa tcgaaaaaaa cgagggatta 660
   cgtttcattt acgaaaaaat cgaaataccg actctcgttg tcttgcaaaa aatggagcgt 720
   aatggtgtcc ttattgatcc agaacgtctt ttaggacaat cttcggaact cggaaccaaa 780
   atggacgaac tggaaaaaag cgcatatcgg gccgcagaac aggcattcaa cctgaattca 840
   ccgaaacagc tggctgaaat tctttttgac aaactgggac ttccaatgat caaaaagaca 900
   ccgtccggtg cgccttccac cagtgaagaa gttcttgaaa aactgtcgct tgattatccc 960
   ttgccaaaaa ttattctgga ttatcgtggc atgtcgaaac tgaaatcgac ttatacagac 1020
   aagctgccca gaatgatcaa tgccgaaaca ggaagagttc ataccaacta tgcgcaagca 1080
   gttgccgtca ccggacgtct ggcatccagc gatcccaatc tccagaacat tccggtaaaa 1140
   acagaagagg gaagaagaat cagggaagct ttcatcgccc cggcgggaag ttatattgtc 1200
   tcagccgatt attcccagat cgaa 1224
<210> 282
   <211> 1047
   <212> DNA
   <213> Unknown
<220>
   <223> Unknown gut bacteria
<400> 282
   atgacacaag acaaactcat cggtcccgga aaaacatttc ttatcaatac cttatccaac      60
   cagagcatcc tggctatcct cgctttgctg gctgtattgg ccattgtctt catcgttttg     120
   cgactgaaac gattccgcaa caatatgcca ttgcacatcg tgcgtgtctc tatcgctttt     180
   ttttcacttt atctaacagg caaactgctc tcacagttca ttgaattgca tgacatttca     240
   atagtcacac caaaagaaat caatttcctg tttctggtcg ccatcgtcgc acttgccatc     300
   agggaatgct tcctttttgc agatcacttc agaagacatc tggaaaatac cggaaaaaat     360
   gccacttcgg cacaaatcat tacccgggtt ataaaaatcg tgtgcgccat ttccattctg     420
   ctcatgttcg gtgaacaatt cggcctgagc tttgccggat tattgacatt cggaggaata     480
   ggcgggattg ccatcggtct ggcgggaaga gatattctga gcaatctctt ttcgggaatc     540
   atgatctatt tcgatcgtcc cttcaatatc ggtgactgga tccggtcacc ggatcgcaat     600
   attgaaggaa cggttcttga aatcggatgg agaaccacaa aaatcatgac gttccagaac     660
   cgcccgcttt atgtcccgaa ctatatattt tccacaatat gtgtggaaaa tccgggaaga     720
   acacgaaact ggcgcatcaa aacttccata ggcattcttt gtgaagatgt cgacaaaata     780
   aaagttatcg tcggccaaat ccgggatatg ttgaaaaaga acgataatat tgaccagagc     840
   cagaccttgc tggtgaattt caatcagatc ggagactttt ccctcaacat tctcgtttat     900
   tgtttcacga aaacgactgt ctggagtgaa tggctgcaaa tacaggaaga cgtttatctc     960
   gaaatcatca acattgtcca gaaaaacggt tctgacctgg catatcccac ccgcaaggtt    1020
   tttctggaac agatggcgaa gcaataa                                        1047
<210> 283
   <211> 1008
   <212> DNA
   <213> Unknown
<220>
   <223> Unknown gut bacteria
<400> 283
   cccgccacat tgccggagag tgtgttaatg caggctgccg ctgagatgtg cgactggcat 60
   ggatgtcgta tgtctgccat ggagctgagt catcgtggtg cggaatttgg caggatcatt 120
   gagcgggcaa ggaatgatct tcgcgatctt ctggaaattc cggaatccca tcgcatactt 180
   ttcatgcagg gcggcgcaac ggccatgaat gcgatcattc ctctgaatct cgtcaatcgt 240
   cccggctcaa atgccactat ggatttcgtg cacacaggga tatggtccgg aaaatcgctg 300
   aatgaagcga ggaaatatgg gcatgtcaat attgtcgctt catcggagaa gagcggattc 360
   ctgtctgtcc cggaaagaag tgtctggcat ctgtcggacg gtgccgccta tattcatgtc 420
   tgcacgaatg aaacgatcaa tggctgtcag tttcatttta ctccggatac cggtagcgct 480
   ccgctcgtgg ccgatatgtc ttccgagatt ttgtcccgtc ctgtcgattt ctcgatatat 540
   gatgtggttt ttgccagtgc ccagaaaaat ctggggccgg cgggggtgac tcttcttatc 600
   gttcgggaag atctgatcgg atatgcttct ccgttttgcc cgtccgtttt tgactggaag 660
   aatgtcgtgg aaagcgattc catgttcaat acgccaccga cttatgccat ttatatctgt 720
   ggtcttgttt tcaggtggct gaaaaaacag ggaggggttt ccagaatgga acgtgaaagc 780
   atcgaaaagt cgtctttgct ttatgacttt atcgatcaca gtgattttta tcataataat 840
   gtcgaaacag gcagtcgatc aaggatgaat gttccttttt ttcttgagga tgaatccttg 900
   aatgacaggt ttgtcatgga agcttctgca catggtctgt tccaattgaa aggacatcgc 960
   ttgcagggag ggatgcgtgc atctctatac aatgccatgc cgatagag 1008
<210> 284
   <211> 726
   <212> DNA
   <213> Unknown
<220>
   <223> Unknown gut bacteria
<400> 284
   accgaagtca ttcacgattt cagaaaagaa gacttccttg cggccgtccg gaaaacaaag 60
   gaatacatcg ccaatggcga ttgcatgcag gtcgttttga gccaacgcct gagaaaaccc 120
   tatacggact ctccattatc gctttaccgc tccttaagag ccctcaatcc ttcgccttat       180
   ctttattact accatttcga tgaccttcac atcatcggtt cctcgccgga aattctggtt       240
   cgccaggaaa atacccctga ggggcgtaaa gttattgtcc gtcctcttgc agggacacgc      300
   ccgcggggag ctacaccgga aaaagaccgt cagctcgcat cagaactgct ctcggatacc       360
   aaggaaatcg ccgaacatgt catgctgatc gatctggccc gtaacgatat cggacgaatt       420
   gccaaaatcg gcagtgtcaa agtcacggaa aaaatggcca tcgaaaaata ttctcacgtc       480
   cagcatatcg tttccaatgt cgaagggctt ctgaaacctg gcctgtccaa tctggatgta       540
   ctgaaagcga cttttcctgc cggtacgctt tccggcgctc ccaaagtccg cgccatggaa       600
   atcattgacg aaatggagcc ggtaaaacgc ggtatttacg gcggtgcctg cggttatctt       660
   tctttcggcg gagaaatgga tctggccatc gctatccgca caggtgtcat ctataaaaac       720
   atgctt                                                                  726
<210> 285
   <211> 645
   <212> DNA
   <213> Unknown
<220>
   <223> Unknown gut bacteria
<400> 285
   atgaatgctg ataccgaaat tttcttcgta tctggcaatg ccggaaatct tgaatgtgcg        60
   cttgatttac ccaaaaaaaa gccggttggt attgcccttc ttgctcatcc tcatcccctt       120
   tatggcggta ccatgaataa caaggtagtc cagatgatgg cacgatcttt tattggcctg       180
   gggtttctgg cggtcagaat gaatttccgt ggagtcggga agtctgaagg tgcacatgat       240
   tttgggaacg gcgaaacgga agatatggaa aatgtgctgg aacatatgca aagaaaatat       300
   ccgaacttgc cggttgtgct gggaggcttt tcatttggca cttatgttca gtcacggttg       360
   cagcaacaga tggttttaaa gggcaatccg cctgaaggga tggtttttgt cagtacgaca       420
   gcggggaaat gggccgtgga gaaagtgccg gaaaataccc ttctgattca tggcgaaatg       480
   gatgaggttg tgccgctgtc cgatgtcttc aattgggcac gtcctcagga tctgtccgtt       540
   gtcgttgtcg cgggtgccga tcatttgttc aatcataaat tgcatcatat ccgtcgtatc       600
   atcacgaccg ctttcaagaa tcggcaagag gccggaaatt tctga                       645
<210> 286
   <211> 1737
   <212> DNA
   <213> Unknown
<220>
   <223> Unknown gut bacteria
<400> 286
   atgagtgcga caacagacga ttcactgaaa ttagggagtg cgaatacagg taccatttca 60
   ggtggtcatc ttgtcgcgaa agcgcttaga aatgaaggaa tcgacacgat attcaccctc 120
   tccggaggca atatcgtgga tatatatgac ggttgtgtca atgaaggcat ccgggttatc 180
   gattttcgcc atgaacaggt agcagcacac gccgctgatg gatatgcacg ccagacaggg 240
   cagacaggat gtctggtcac gacagcaggc ccggggtgct gtaatgccat aaccggaatg 300
   gcgacagcat tgcgatcgga aacgccactt ctgcatattg gtggccaagg tgccacttcc 360
   cagtatttgc agggttcctt gcaggaattc gatcatgtca gactgatgtc tcccgttacc 420
   aaatgggcgg caagtgttcg ctcgactgaa cggattgccg atatggtttc catggggtgt 480
   cgggaggcat ggggcgaagt tcccggtccg gtctatcttg aaataccccg ggatcttctt 540
   gatcgcagta ttgaactgca agattgcgtc attcccgcag ctggtcatta ccgtttctcc 600
   tgtaaagtga tgtgttcacc tcccgagata gaaaaactgg cggatctgtt ggtccatgct 660
   gaaaaaccgg ctgccctgtt cggaagtcag gtatggaacg gaagaagtca tcgggaagcg 720
   cttgaggtga tcaggacttt tgatattcct gcctacacga atggttctgc ccgcggcatt 780
   ttcagaaaca atgacccgct ttcctttgac aggacacgtg acaaggcatt ggccgaagcc 840
   gatgttattc tcgttgtcgg cacacctttc gatttcaggc tgggttatgg caagtcgctg 900
   aatccggcag ccaaagtcat tcagatagac caaaattatt cggtaatcgg caaaaatcgt 960
   gatattgaac tgggtctgct gggacatgcc ggtaccattt tcagagcggt tgtggatgct 1020
   gtttccggaa gtgaaaaacg gaaaatggca acgaagcgaa aagaatggat tgcaacgctt 1080
   cgggccgcgg aagcagaagc acttgaaaaa ctgatgccat tgttcaaatc ggagaacagt 1140
   ctgatcaatc cgtaccgggt ggcatatgaa ctgaacgagt ttcttgagga aaacacgata 1200
   tatgtcggag atggcgggga tgtcgtgacg atctctgcac aggccgtacg tccccgcaga 1260
   ccgggacaat ggatggatcc ggggcctttg ggaagtctgg gggtcggtac aggtttcgct 1320
   attggagcca aactggccaa tccggacaag gaggtcgtgt gctactatgg tgatggcgct 1380
   ttctccatga cggcgttcga tatggaaacg gccaaccgct tcaaggcccc ttacctggca 1440
   gtggtcggta acaactcggc catgaaccag atccgatatg cacaactggc caaatatggt 1500
   gatcgtggta acgttggcac tttgttaagt gacttgccgt atggtaaatt tgcggaaatg 1560
   atgggcggct acggtgagga agtctacaat cccggagata ttgcgcctgc cttgcggcgt 1620
   ggacgtgagg ccatagccag aacgggcaag tgtgctgtta tcaatatctg ggttgacccg 1680
   caagaatggt cgccgggaac caaggcacgt ttgaaggcac gtatggccgg caaatag 1737
<210> 287
   <211> 1464
   <212> DNA
   <213> Unknown
<220>
   <223> Unknown gut bacteria
<400> 287
   atgaaatatc gtgacttaag agactttgtc gaccagcttg cccaggaaaa ggatgtgaag      60
   gaaatagaga ttcctgtatc cccttgtctg gagatgacgg aagtttcccg tcgcgtactc     120
   gtgcaggatg ggccggcact gtttttcaaa aatccggaag ggtgttccat gccggttttg     180
   accaatcttt ttggcagtac gcgacgcatt gccagagcaa tggggaaaga gacaatcggc     240
   gatttaagaa gcatcggaca gttcctcgct tccctgaagg aacctgaaca gcccaaaaat     300
   ttttctgaaa tagtcggcat gggcaccatg ctgaaatctg ttctggatat gcggccaaag     360
   gaacgcaagg acgctcgctg ccatgaaatc gttctggaag gacgtaatgt cgatcttgat     420
   gcgttacctg ttcagacatg ttggccggga gatgctggcc ccctgattac atggggactg     480
   gtcgtgacaa aggggccatg ccggaaaagg cagaatctcg gggtttaccg acagcaggtc     540
   attggcccga acaaggtcat tatgcgatgg ctgccgcaca ggggaggggc acaggatttc     600
   agggatcaca cgatggtgaa tcgtggaaaa ccctttcctg tcgccgttgt gatcggtgcg     660
   gatccagcaa ccctgctgag tgcggtcatg ccgatcccgg atgaattgtc tgaatatcag     720
   ttcgccgggt tgttgagagg ggggcgtacg gaactggtca aaacgattgg aagcgatttg     780
   cttgttccat caaatgccga gatcgttctg gaaggataca ttttgccgga tgaaaatcat     840
   ggatcgggat atgagcatgc acttgaaggt ccattcggcg atcatacagg ttattacaat     900
   gaaaaggact ggtttcccgt cctgacggtt gaccggatta ccatgcgcag aaatccggtc     960
   tatctttcga catatacggg gcggcctccg gatgaaccgt ccatgctggg actggctttg    1020
   aacgaagttt ttattccgct gttgcagaaa cagttttctg aaataatcga tttttatctg    1080
   ccggccgaaa cctgcagtta ccggtttgcc gttgtcaaga tcagaaaggc ttatccggga    1140
   catgcacgga gaatcatgtt tggtgtctgg agttatttgc ggcagtttct ctataccaaa    1200
   tttattatcg tcgttgatga agacattgac atccgtcggt gggaagatgt cctctgggcg    1260
   atttcaaccc gtgtcgatcc ggcaagagat acggttctgg tagaaaacac accgattgat    1320
   tatctggatt ttgcatcacc tgtcagtggt ctgggggcca agatgggaat tgatgccaca    1380
   gacaaatggg caggcgagac atgcaggacg tgggggaaaa taatccggat gtcagatgat    1440
   gtgcagaaga aagtggatat tatt                                           1464
<210> 288
   <211> 1080
   <212> DNA
   <213> Unknown
<220>
   <223> Unknown gut bacteria
<400> 288
   atggcactat ttacaccaca gcaaagacaa accgcactat ggaccggtat cggcatattg     60
   ctcattgcca ttctcctgct gcttggcccg atcctgaccc ccttcattgc cggttccata    120
   ctggcatatg ccttgaaccc gatcgtcgaa tggctgcgtt caaaacgcct gggaaatctc    180
   agaatgcctc gcatcatcgc cgtcatcatc gtcatgatgc tgctattttc cgtcgtgacc    240
   gctttcgcac tgatcatgat tcccatgttg atccgcgaat ggcccatgct tcacgaacag    300
   attcccgctt tcatcgccaa agtcaacaat tacctgaatc ccatactcaa cgaactcggc    360
   atccaggtcc cggcagacaa tgccagcatc aaacggatac tgaccgaaca atttgtcccg    420
   aaaggagacg gtgtctggaa atcacttatt gcctctgcaa aattcggcgg cactgcggta    480
   ctgacatggg catggaatct gattcttatt cccgttgtac ttttttacct tttaaaagac    540
   tggcacatca tgatgcgcaa gctgagagac atgataccca gacgatgggt ttccaaggcc    600
   gtcagtttct cacgggaagt cagcagcatg ctggcccaat acctgcgtgg acaattacta    660
   gtcatgatga tactttccac ttactattcg attgctctgg cactggccgg gtacaacgtt    720
   gcattgcctg tcggcatcat gaccggcatg ctggcattca ttccttatat cggtatctgt    780
   ttcggactgg gtctggccgc cacggcaacc cttttgcaat ttaccggcat ggaagcctgg    840
   atcacactcg gtatcattta tggtttcggc tactctttcg acggtctgct catgacaccc    900
   aagctggtcg gggaccggat tcagcttcac cccctgattg tcattttcgc cctgctcgca    960
   ttcggccagc tcttcggctt tgtcggcatt ttgcttgccc tgccttcttc cgctatcctg   1020
   tcggtcgctt acagacacct tcgcagttat tatttcgaca gtaattttta cagaaactga   1080
<210> 289
   <211> 1536
   <212> DNA
   <213> Unknown
<220>
   <223> Unknown gut bacteria
<400> 289
   gactatatcg gcattttgcg tgcagccctg cacaatctgg tcagcaacaa caggcaaggc 60
   gcttcgacaa tcacccaaca ggtcgccaga aattttttcc tgacaagtga gcaaaccttc 120
   aaacgcaaat tgtacgaagt tctgctggcc tggaaaatcg aaagaacgct gaccaaagac 180
   cagattctcg aaatttatct gaaccagatc tatctgggac agcgtgcttt cggtttcgct 240
   tccgccgcgc aggtctattt cggaaaaccc ctgcaggaca ttaccctggc tgaagcagcc 300
   atgctcgcag ggcttcccaa agcaccttcg gccaataatc cgatcaccaa tccgactcgc 360
   gccaaacagc gtcaacaata tattcttctc agaatgaaac aactcggtta cgtcaccgat 420
   caggaatacg agaaggcaaa aaacgaagag atcaaagtaa aaacaagccg caacgaattc 480
   agcgtacatg cacaatatgt ggcagaaatg gtccgtatga tggtatacga tcaatacaag 540
   gacgaaacct acacacgcgg gttgaatgtc tatacaacca ttacacaaaa agaccaggaa 600
   gccgcttatc aggctgtccg gcgcggagta atggactacg acaaacgtca tggataccgc 660
   ggtccggaag gatatatgga aatcccggca gccagggacg ccatcgctga cgctattgag 720
   gatgagcttg cgaaataccc ggacagtgac ggcatcctgc gtgcggttgt acttgaggca 780
   tccccgggaa gagtccgggc cgttttatca tcaggcaacg aaatttccgt cactggaaac 840
   ggactgaact tcgccgcttc cggtttgact tccaatgccg gcagcaacaa acagatccgg 900
   cggggctcaa tcattcggct cgcccaggac atacagggaa actggaatat cgttcagatg 960
   ccacagatcg aagccgcctt cgtttctctg aacccgaatg acggcgccat aaaatccttg 1020
   ataggcggat ttgatttcac gcaaaagaaa ttcaatcatg tcacacaagc atggagacag 1080
   cccggttcgt ctttcaaacc gttcatctac tctgcctcac tcgataaaaa cctggctcca 1140
   gccagtatta tcaatgacgc ccccgtatcg ttcagtgggc agacaaacgg gcaaaactgg 1200
   actccgaaaa atttcgattc aaaatatgag ggtccaataa ccatgcgtcg cggattgatg 1260
   aaatccaaaa acatgatttc aattcagata ctgaatcaga tcggcgcaca atatggccag 1320
   gaattcatca cccgatttgg ctttgatccc gacaaaaatc cgccttatct gacattggca 1380
   ctgggggccg ggtccgtcac accgctacag atggctacgg cttattctgt ctttgccaat 1440
   ggcggctata aaatcagacc gtatatcatt tcccatatca ccgattccga cggaaaaata 1500
   ctctcccagg cacgtccgga aaaagccgga gatgaa 1536
<210> 290
   <211> 1257
   <212> DNA
   <213> Unknown
<220>
   <223> Unknown gut bacteria
<400> 290
   cgctccgaac tggaaaaaaa agtcgaaatg aaaaagaccc gtctggaaaa atggacgaaa 60
   tccggtttct tttctccttt cgcactttat ttgggatcca tttcaattcc cgcattgatt 120
   ctttcaggtc tcatggtctt cggcaccgga atcgaaaatc tccctgtctg gcttggattg 180
   atgaccggtc tgtgtgccct gatcgtttcc agtcagcttg ctgtagccgt cgccaactgg 240
   ttcagtgcct ttccctgcgc gacaaatccg cttcccaaga tggatttttc aaaaggcatt 300
   cccgaaggat acgacacgct tgtcgtcgta ccttccatgt tgatcagtgt agaacaagtc 360
   gaaaacctgt gcggtacact ggaagtccat tatctcgcca accgggataa acgactgaga 420
   ttctgcctgc tcaccgatct gactgacgcg gagacggaaa cgacacctga cgatatcgtt 480
   ctgatcaacc atctgaccca tctgatcaat gaactgaacg gcaaacacgc ttctgataac 540
   acgggtccgt ttctggcgtt gagccgccca cgacactgga atccgggaat cgggaaatgg 600
   atcggatacg aacgaaagcg cggaaagctg gaagaactga accgttatct gaaaggctgc 660
   gacgaaaacg acttcaccac cagaatcggc aacatcgaaa atctcgaaaa aacccgatat 720
   gtcatcactt tggacagcga tacccggtta ttgcgcgatt ccgcaagaca attcgtcgga 780
   accatggcac atcccttgaa cgcaccgaaa tttgatgcca tgaaagaccg cattaccgaa 840
   ggttacggta tcctgcagcc ccggatcacc aacagtctgc ctgccggcaa tgcatcctgg 900
   tatgaaaaac tgtgcagcgg tgatgcaggg atcgatcctt acacccgcgc agtttccgat 960
   atttaccagg acattttcgg tgaaggttcg tttatcggaa aaggaattta tgatgtcgat 1020
   gccgtctatg caaccttgaa tggaagactt cccgaaaaca gtatattgag ccatgatctg 1080
   ctggaaggtt gttatgcccg ttcgggtctg ctaagcgatg gacaactgta cgaacaatat 1140
   ccctcgagtt acctggctga tgtaaaacgc cgtcatcgct ggatcagagg agactggcag 1200
   ctgctgaaat ggttgttcgc tttcgttccg gacgtccgac cggaaaacag aacggga 1257
<210> 291
   <211> 1296
   <212> DNA
   <213> Unknown
<220>
   <223> Unknown gut bacteria
<400> 291
   atgattcccc atttagttac cgcactgaac ggacctttgc atgaactgga aaaaaccatt 60
   atcgactcga caccctccat tgaaagatgg ttcaggctgg aatggcagga tcatactcct 120
   ccattctact gttcagtcga cttgaggaat gccggtttca agctggcgcc agtcgatacc 180
   aatctttttc ctggcgggtt taacaacctg tctgttgaaa tgctgccgct ggctgtacag 240
   gccaccatgg ccgccattga aaaatactgt ccggatgcca aaaatctgct tctgattccg 300
   gaaagtcata cgcggaatat ctattacttg caaaatattg caagactgat ccggattttg 360
   cgtcagacag gccttaatgt ccggctgggt tccttatcgg aagatatcaa ggtaccgact 420
   ccgtttactc tgccggacgg aacgacgctg atgctggaac ctttggcgag aatcaataac 480
   ggacgccgcg tcggcgtggg ggatttcaat ccgtgtacga ttcttttgaa caacgacttg 540
   agttccggga ctcccgaaat tctcaaaaat gtttatgaac agagtctttt ccctcctttg 600
   catgcaggct ggactgtccg tcgcaaaagc aatcatttcg ctgcatatga cgatgtcgcc 660
   aaacggttct ccaagatgct gggaatcgat ccctggctga tcaatccgta cttttcaaaa 720
   tgcagtcatg tcaatttcgg cgagcatgag ggtgaggagt gtcttgcggc gagtgtccat 780
   tccgtactga tcaaaatccg aaggaagtac aaggaatatg gtatcaagga aaaaccgttt 840
   gtcattgtga aagcggatgc aggaacatac ggtatgggta tcatgaccgt tacggatgca 900
   agcgaagtca gggaactgaa tcgcaagcag cgcaacaaga tgtcggtcgt caaggaaggt 960
   cttcccgttc gtgatgtcat tattcaggaa ggcgttcatt ctttcgaaag agtggatgac 1020
   gctgtcgccg aaccggtcgt ttacatgatt gaccggtatg tggtcggtgg tttttaccgg 1080
   gttaatgaaa agcgcgatat caatgaaaat ctgaatgcgc caggcgcgca tttcgttccg 1140
   ttggccttcg cacagcaaca tacgttaccg gacagaagtg cggaaccggg gacgacggca 1200
   cccaaccggt tttacatgta tggggtgatt gcacgtctgg cattgctggc ggcttcggtc 1260
   gaactggaaa aaaccgatcc cgacatgcaa tcgtga 1296
<210> 292
   <211> 1290
   <212> DNA
   <213> Unknown
<220>
   <223> Unknown gut bacteria
<400> 292
   atccccgtta ctgacattac gaccgaagtg gaaaaacgtt tgaaagaacg tgcacgtact      60
   gccaaagctc caggtttcag gcctggcaag gttccgatga agatggtcgc gcagatgtac     120
   ggcgcgcaga tccagaatga agtcatcaat cacaaagtgg gagcggcttt cagcaaggct     180
   gttcaggaca atggactcca gattgcgggt gctccgcgtt ttgaggaaaa aacaggtgat     240
   gatgttgcgc aggacgaagt ggcgttttat gccattttcg aagtttatcc tgaagtcaag     300
   attggcgatc tgggaacgat tgagctggaa aaagccgttg ccggtgtcac ggatgccgaa     360
   gtggacagaa ccattgatat cctgagaaaa cgtcaggcac actttcatgt caagggagaa     420
   gacagtgaac acggaaaagg taacggcagc gttgtggcgg aagatggcga ccgtgtgacg     480
   atcgatttta ccggtcgtat cgacggtgtt gaattcgaag gcggcaaagc ggaaaacttc     540
   ccctttgtcc tcggcgaaaa acagatgctg ccggaattcg aggatgctat ccgggggatg     600
   aagacaggtg aaacaaaagt ctttccactg acttttccgg aaaactatca tggcaaggat     660
   gtggccggta aaacatcgga attcactgtt gaggtcaaga aaatcgaatg ggcacatttg     720
   cctgaagtca atgaggaatt tgccaaggcg cttggcgtca aggatggcag tgttgaaaaa     780
   ttgcgtgaag acatcaagac caatctgcag cgtgaagtca agaatcgcct gatcgccatc     840
   aacaagaatc gtgtcatgga tgccttgatc aaggtgtccg aattcgatat tccgcaatcg     900
   ctggtcaagc aggaaatcgg cgaattgacc gagttgacac gacgggatct ggctgtccgc     960
   aatccgtccc acaaggatgt tgccttgcct ccggaactgt ttacggcaca ggctgaaaaa    1020
   cgggttcgtc tgggcatgat tctgggcgaa ttgatgaaag acaatgttct ggcggtctct    1080
   gctgataaac tgaaagaaag agccaccgaa attgcttcca gttacgaaaa tccgcagatg    1140
   gtcatcgatt attatctgaa tgaacccggc cgccgcaagg agctggaagc gatgctgatg    1200
   gaagaaaacg cgatggatta tgttttcggc aaagccaagg ttgttgagaa agaagtgccg    1260
   ttcgatgaac tcatggctca gcaaatgtaa                                    1290
<210> 293
   <211> 753
   <212> DNA
   <213> Unknown
<220>
   <223> Unknown gut bacteria
<400> 293
   cgcgaacctt tgcggcggtc atccgtctcg aaacaggggg cggacacgcg gattaccgaa      60
   cagcccggtt ttgtcctgca cagttatgtc taccgtgaaa gcagcctggt tgtcgatgtc     120
   tttacccgtg attatggccg agtggcgctg gtggcgaaag gggcaaagcg tcccgcttcc      180
   cgtctgagag gtgttttgca gacgtttcag ccgctttcag tcggctggtc cggcaaatcc     240
   gagttgagaa ccctgacatc ggccgaatgg gtcggaggca tgattccgct ggacagttcc     300
   gccttgctgt acgggtttta tctgaatgag ctgctgatca ggctgctggc gcgggaagac     360
   ccacatcagg gactgtttga gcagtatgtc cgggcactga cacgtctggc gacttccgaa     420
   tccgcacaga cagtcctcag gattttcgaa ctggcgctcc tgaaggaaac gggagtggcg     480
   gccgatttgt cgatcaatgc aaaagaccgg cgtccggttg agccggaagg tatttatgtc     540
   gtcgatccgg aaaacggtcc gcgtttcgcg acgctggaag acacggcgcc ccgtgtttcg     600
   ggcaaaacgc tgcttgacat ggtcagcgag gattattccg atcctcagac ccagttgcaa     660
   agcaagcttc tgatgcgttt tctgctggcc catcacttga acggtgttcc cttgcagaca     720
   cggaaaatac tgatagacct gcgtcagctg tga                                  753
<210> 294
   <211> 894
   <212> DNA
   <213> Unknown
<220>
   <223> Unknown gut bacteria
<400> 294
   gatgatttca tcgcttttgc cgccgagccg aaagaagacg attctcccgc acggttttcc      60
   atcaataaca ttcaggtcga aaacgcggtt atcgagttcg acgattttcc gcaaaataaa     120
   catcatgtcg tcgatgaact gaatgtttcc attccattcg tttcgaccat tccttcacaa     180
   gtcgatattt ttgtcgattt ggcgatcaat gggcgattca atcaggaaaa aatcgagctg     240
   acggggaaag caagaccctt tttcaaagac aaggatggaa ccgtcaatat ccggctggac     300
   aagatcaatt tgccggctta tgcaggttat ctgccggtca agccggattt cgacctgaaa     360
   gacggaaagt tgtccatcga catggaagtc gggttcggtc agcgtgcaga aggtcagcaa     420
   ggacttactc ttgagggaaa tgttgtcctg gaatcgttat tgttggccga accggatggc     480
   aaaccggttc tgaaaatccc ggaaatgact gtccggattg cccatagcga tgtgctgtca     540
   gggaaattcg acatcggcca ggttctgctg aaaggacctg aagtgtatct tgaccgtaac     600
   agggcaggac aatggaatgt cgaacgggtc gcgaggttca gtcctgcaga caataccgga     660
   aaaaatcatg tcggagcggc agcagatgcc agtgcgggaa aaaaggatag ccggccattt     720
   tccgtcagtc tcggaaaact gtctgtcgaa aacggagagc ttcgggtcag tgatcagatc     780
   catgtcgttc ccgctgattt ttcggtcaga aatttcgcgc tggatgtaga tggcattgta     840
   ctggatgttg cggagaaaaa cgtgacggtt gacaggatgg tgtctgccgg aact           894
<210> 295
   <211> 1245
   <212> DNA
   <213> Unknown
<220>
   <223> Unknown gut bacteria
<400> 295
   atgacgtata aaacccgatt caagtttctc gttctggctc tggccggctg cttcaccctt      60
   tcggccccgg catccgtttt cgccagcgac atttcccttc cgaaatacag caaagacgcc     120
   cagctcgccg cctttcattc cgcagccgac ctcgatctca tcggtgacga cgcatacgac     180
   gacgatcccc tgacagaaat tctttccatc aacgaagtcg atgtctgggg aagaatccgc     240
   tcgggtttcg ccatcgccga tctggacaat ccgctcgtcg aaaaccacat gaattatttc     300
   gccagacgtc cggactattt ccagagaacg acgcaacgcg gatcgaaata cctcttccat     360
   atcgtgcagg aacttgagaa acggaacatg ccgacggaac tggctttgct tccgtttatc     420
   gaatccgcct acaatcccca tgcccgatcc attgccaagg ccgaaggtct ctggcagttc     480
   atccccagca cgggacgcca ttacgatctg gcacaaaacg ctttcaagga cgaaagaagg     540
   gatatcattt cctcgaccga ggcggcgctc acctatctgc aaaggctcca tgacatgttc     600
   ggagactggc agcttgcgct ggcgtcctac aactggggcg agggatcggt catgcgggcc     660
   atcaaaaagg ccgaacgtgc gggaaaaccg atcaccttca acagcatttc cgcctacatg     720
   ccggctgaaa cgcgcaatta cgttccgaaa cttcaggccg tcaaaaacct gatcaccaat     780
   ccacgccgat acggcatcga tcttcctccc gtcgaaaacc agccttactt cgtcacgatc     840
   ggcaagaccc gtgatatcga cgtcaaggta gctgccagac tggccgaaat gtccctggat     900
   gattttcggg cactgaatcc ccagttcggt ccctatgtca tcgccggcgg caacgaggtc     960
   aaaatactcc tgccgaagga aaacgccgaa aagttccgga aaaatctttc tctctggaca    1020
   cgaccgctgt cttcatggac ggcctatcag gtaacgagcc cgcgggaaaa agtcgaagac    1080
   atcgccgccc gtctcaatat ctctcccgac gtaatccgtc aggccaacaa catcccatcc    1140
   agaagcgtcc tgaaattcgg atcggccatt ctggttccga aaacggccgg ttacgatcgt    1200
   gacatcgctc ccgaacttgt tgaaaacgcc aagctggcct gggaa                   1245
<210> 296
   <211> 849
   <212> DNA
   <213> Unknown
<220>
   <223> Unknown gut bacteria
<400> 296
   ccccccccag ccgataacgt tgtcctggtc aagataccgg aaatacccgt tgacgatctg       60
   atcgaattgc cgatcatgct caatcttgaa gaaagtcttc gtgcggaaaa aatcctgcct      120
   catcttcagt ctttgcgata taccggcagc aagcctgtcc gctttctggg aatgccggac      180
   aataacgatg tgcccgaaaa gaactggcag gcgattgtgc ccggaaacag ttatcgccac      240
   ctcaaggcat gtgtgcaact ggccaatcgt aatggtgcct tgaacgaaat agagtattcg      300
   gaactgctga tgcgtttgcg ccagatcgcc gatgctattg gcgccgaacc tgaagtgccg      360
   gaaatgtcgg atgttattga aaaggcagga caactctatc agttcgtcgc cgatcacgat      420
   gccaagctgg gtatcagtat acggagtaac ggagcaccgt ggcagatcgt aaccctgaaa      480
   accgcgcttg aaaaacaggg ttttgaatca aggccagatg gccagttcgt gatgaaagac      540
   cgtgaaaacg gcggaattct gttttctctt tcaaccaatg agcttcctgc gtctgaaacg      600
   gccacacggc tgacattgct gatggatgtg ccctgtattg cccaggaaaa ggatggtttc      660
   gggcaaatgg ttcaatgcgg gaaagcgctc tgtatccgtc tggatggcgc tctggttgat      720
   gatggcaacc agatgttgac ggacaaattt ctggatgaga tttcagggca ggtcaacgat      780
   ttctacaagg agatgcagga atcatctgtt cctgccggat caatccgtgc cttgaagctg      840
   tttaactga                                                              849
<210> 297
   <211> 966
   <212> DNA
   <213> Unknown
<220>
   <223> Unknown gut bacteria
<400> 297
   gcgtatgtcg atagccagac gccggaagaa attgcgaaaa accggggcag tttctccgag       60
   ccgggaaaaa attcccctta tcgtgaacgt ccggcatccg aatcgctcga tcttttccgg 120
   aggatgaaag cgggtgaatt tcccgatgga gcgcatgtcc tgcgggccaa aatcgatatg 180
   gcgtcaccca atatgaacat gcgcgatccg gttatttacc ggattcgtca tgcccatcat 240
   cacagaacgg gtgacaaatg gaatatttat ccgatgtacg attatacgca tccgatttcc 300
   gatgcactgg aaaacatcac tcattcgatc tgtacgctgg aattccagga tcatcgtcct 360
   ttctatgact ggattcttga acggctcacg gaaacgacga cgttaccgga cggtcgtgta 420
   atcggagggt ttttgcagaa accgttacct caccagtatg aatttgcgcg gctgaacctg 480
   acctatacca taaccagcaa gcggaaactg ttgcaactgg tggaagaaaa aatcgtggat 540
   ggctgggacg atccgcggat gccgacgatc gtcggtatcc gtcgccgggg ctacacgcct 600
   gaatccatcc agcttttctg tgaacggatc ggagtggcca aagccgatgg ctggatcgac 660
   atgggtacgc tggaaggcag tttgagggat gatctcgacc ccaaggcgcc cagggccgct 720
   gctgttttgc atccggtaaa gctgattatc gataatttcc cggaacatct gcaggaagaa 780
   tgtacatctc cggttcatcc tcattatcct gaacggggag tgcgccgttt cccgatcacg 840
   aaggagttgt ggatagaacg cgaggatttc atgattgatc ccgtgaaagg gtatttccgg 900
   ctattccctg gcaatcgtgt ccggcttcgt tacggttatg tcgttgaatg taccggttac 960
   gatatg 966
<210> 298
   <211> 681
   <212> DNA
   <213> Unknown
<220>
   <223> Unknown gut bacteria
<400> 298
   atgacgcagg ctatcgattc ccaaaccgtt gcacaatatc ttatcgacca ccccgaattc 60
   ttcgagaatc atgccgacat cctttccggt ttgcgcctga ttagcccggt catcggcaag 120
   gccgtctccc tgcatgagcg ccagatcgaa gtattgcgaa gcaaatacaa aaccctcgaa 180
   ttgcaactgt ccaggctgat gcacacggca cagaacaaca agagcctgat ggaaaaaatg 240
   gtagtctggg cctcgagact tctcgaaaaa cgccatgaaa ccgatgatat gccgcaaacg 300
   gtcgtcaaca gtctcgaatc cgttttcaat gtgcccgaaa cgacattgcg gctctggaat 360
   atccgggaag aatacgcgga cagctggttt acccggcatg tcgatgaaaa catccgcaat 420
   gccgccaacc gcatggaaca tccgtattgc ggaaaagtgc cggaaaatca ggatgctgtc 480
   aaatggcttg atcttttctc ctcgatccgt tctgtcgccc tggtaccgct gaaaaaagac 540
   gcgcaatccc ttccgttcgg tctcattgta atgggatcgc ccgatgccac ccgtttccag 600
   aacgatatgg ccaccgattt tttgtctgct atcggagaac tggccagtgt cacactccgt 660
   catctgctgg tgaccggctg a 681
<210> 299
   <211> 921
   <212> DNA
   <213> Unknown
<220>
   <223> Unknown gut bacteria
<400> 299
   atgaaagtta ttctggccca accccgagga ttctgcgccg gtgtcaaacg tgcgatcgag 60
   accgtcgagc gtgctctgtc tcttttcggc aaaccggtat atgtccgtca cgaaatcgtc 120
   cacaacactt acgtcgtgaa ccgcctgaaa gaaaaaggcg ctgttttcat cgaagaactg 180
   gatgacgtcc ctgccggcag cgttctgatc ttttccgcgc atggggtttc gcaggcgata 240
   cggcgcgaag cggaaaaacg ccgtttcaga cttttcgacg cgacttgccc tctggtgacg 300
   aaagtccatg ccgaagtggc caaaagacgg aaagacggcc atgaaatcgt cgtgatcggg 360
   cacaaggggc atcctgaggt ggaagggacg ctcggacagg cggattcagg catgtatctg 420
   ataaacgacc tgaacgatat cgcgacacta caggtcgaaa atccggaaaa gctggcctat 480
   gtctcccaga ccacgctatc tgtcgatgat accgccgaaa tcattcattc cctgaaaacc 540
   cggttccctt tcattgtcga gccgaaaaaa gcggacatct gttatgccac aaccaaccgg 600
   caggaagcgg tcaagttgct ggccaaaaaa gccagcctga tcattgtcgt cggcagcccg 660
   aacagctcca attccaaccg gttaagggaa atggccgaga aaaacggggc aacggcccat 720
   atggtggaca atgcatcgca aatcagcatg gactggcttg ccggtcacga atatatcggc 780
   gtcacggcgg gagcttccgc tccggaaatt ctcgtgacgg aagtcgtcga tctgctcaga 840
   cgggctaaag cggaatcggt gacggaaatg cacggcatgg aagaatcgct ggtttttccc 900
   ttgcccaagg gactcaaata g 921
<210> 300
   <211> 1608
   <212> DNA
   <213> Unknown
<220>
   <223> Unknown gut bacteria
<400> 300
   atgtcgaatg atgcctttgc caaactgaat gcccagcaat atgaagcggt ccactatatg 60
   gatggcccct gtctggttct ggccggggcc ggttccggaa aaacgcgtgt cattacgcag 120
   aaaatcgctc atatgatagg cagtggccag tatgaagcca gaaacattgc ggcattgacc 180
   tttaccaaca aggcagcggc agaaatgcag gaacggattt cccgcttgct gaaagagccc 240
   tctgacgctt cacagttgac gatttcgact ttccattctc ttggtgtgaa gattctgcgg 300
   caggaagcga aagaactgaa tctcaaggac cgtttttcca ttcttgacag tgatgactgt 360
   ttttctctgg ttcaggatct tgccgttacc actgacaaac aggttatccg ccggattcag 420
   ggtacgatat ccttgtggaa aaacggaatg gtcgatccgg aaaccgcttt gaaaagtgct 480
   gtggatgatg aagaagcaca ggctgcacgc atataccgga attatgtggc gacgctttcc 540
   gcttatcagg cggtggattt cgatgatctc atccggttgc cggtcgagct gttccgttcc 600
   agtgacaggg tacgtgaaaa atggcaaaga cgattgcgtt atttgctggt ggacgaatat 660
   caggatacga atacctgcca gtatgagttg gtcaaattgc tggtatcggg aatcggaaaa 720
   aaaccgatgt tcactgccgt gggagatgac gatcaggcca tttatgcatg gcgtggtgcg 780
   acgatcgaga acctgaaaac cctgcagaaa gatttcagcg atcttaaagt gatcagactg 840
   gaacagaatt atcgttcttc catgaggatt cttcaggcgg ccaatgccgt gatcggtaat 900
   aatccgaaat tgttcgaaaa aaaactgtgg tccgatcatg gtctgggcga accggttgaa 960
   acactggcca tgtcggatga ggaacaggaa gcggaacagg ttgtcatgct tttatcggct 1020
   cacaagttcg aacggcgggc cagattttcg gactatgcca ttctttaccg ggggaattat 1080
   caggcccgta tattcgaaac ggcattgagg cgggaaaata ttccttacac catttcgggg 1140
   gggcaaagct ttttcgcccg tgccgaaatc cgggatatca tcagttattt acggctgatt 1200
   gccaatctgg atgacgatcc ggcttttatc cgggctgtca ccacacctcg tcgcggtgtc 1260
   ggacaatcca ctctcgagac gctgggggct tttgccggcc aatggcagtg ttcattgttt 1320
   gaagcggtat acaagggggg gctggaagcc aagttgtctc ctcgccagct gggaccactg 1380
   cgtgggtttt gtgattttat taacgggatt gaggaaaggg caagtcgtcc cgggttatcg 1440
   gccaaaggcg aaaatgctgc ggcactgctg gatgagctga tgcacgaaat agcctatgaa 1500
   acctatcttt atgataattt cgacgaaaga acggccaaaa acaaatggca aaatgttctt 1560
   gaatttacaa actggctgaa agaaaaaagc aaaggcggca aactggaa 1608
<210> 301
   <211> 1302
   <212> DNA
   <213> Unknown
<220>
   <223> Unknown gut bacteria
<400> 301
   atgaaaaaga tactaagtat attttgttta gcactactac tcggagcctg ctcagacttc      60
   ctgaaagagt actcacaaga cctttccaag gtggagagct acacggatct ggatgaactt     120
   ctgttgggag acgcatacct gccagtaggt cgaattacga tcgaaaacag tatgtttaaa     180
   agagaaaact catatttcca aaccgtacat tacatgtccg acgaactact caatttccag     240
   ttaaccgata atggcggata tcatggaatt caagaagaaa tgttcggctg gcacacgtgg     300
   caacaagatg tcggtttaaa tgccgaagga aattctcggg gtgccgaaga tgcggattgg     360
   aatatcgctt accatagcat caacacatgc aatatgatcc tggatgccat tgatgaacaa     420
   cacgccgaaa acgaggaaca ggaactggaa aaaaatcgaa ttaaaggaga aacccatttc     480
   ttgagagcgt tatattattt cacgttagtc aacctgtacg ggcaacccta ctccgaaacc     540
   aaccgcacat ctcccggtgt acccctaaaa acaacatcct acgtggaaga taaagaatac     600
   accatgaatt cggttgatga agtgtacacg caagtactga aagatcttga cttggcggac     660
   acctgtctgg taaacacaac aattaaaaac cacccttacc gcgcagacat caccgccgtg     720
   tacttactga aaagccgcat ttacctgtac atgcaaaact gggaaaaagc ccttgaatac     780
   gcgcaaaaag tgatgacaaa gaataaaagc ttactggatt taaacacatt gacaacggaa     840
   acgggagacg tgttaaccaa atcatcaccg gaaaccatct tctccatggg aggacattta     900
   ttagcctctt ccctcatcat gaaatatgga gaaaaccggt ggggagactg ggaagcctta     960
   ccttcctaca cgatctcgga cgatttagtg gcagcttttg acgaaggaga gaatgatttg    1020
   agaacccaat actacatttt caaaacgaca ctgggagacg actattatgc gccctatgcc    1080
   gatggctggc tctttcgtaa agttcgcgga tgggaatacg ggtacaaaga agtctcagat    1140
   aatttcctgt tccgtacagc tgaagcctat ctgaatgcgg ctgaagctgc ggcttatacg    1200
   ggagatgaag gaacagcccg taacttgtta aaagaattac gggattaccg catgattgac    1260
   agtagatcca tcacggaaag cggtgaatcc ttagttacct ta                      1302
<210> 302
   <211> 1734
   <212> DNA
   <213> Unknown
<220>
   <223> Unknown gut bacteria
<400> 302
   atgattttcc aaatagcaaa aaatgaattg aggaatatgt tctgttcccc gatagcttgg 60
   cttgtattgt ttatatttgc agtacagacc ggaattgaat ttacgggaag tatggctatg 120
   gaagttcggg acagtgttat gggtgttggt ttatatcgag tgacggcaaa ttgttattcc 180
   ggtataaggg gtttattttc ttcgttgcaa ggttctttgt tcctgtatgt tccgttattg 240
   acaatggggt taatgagccg ggaaaggagt agtggttcga ttaaattgtt gtattcttct 300
   ccggttagta atagttcgat tattttaggg aaatatattt ctctgttgat ttataatcta 360
   atactgattt gtattttatt gatttatgta tttctcggat tatgtattat tgaaaatgta 420
   gatttttggt taccgttttc gggacttgtc ggtttatact tgatagtttg tacttatgcg 480
   gctattggac tgtacatgtc atctattact tcttatcaag ttgttgcggc attggggact 540
   ttagccgtgt tggcgattct gaattatttg ccgtctttag gtggaaatat cgaggttgtg 600
   caagagatca cgtattggtt ttcaatttca ggaaaagcta atcaaatgat cgatgggttg 660
   atctgtagtg aagatttatt ttatttcttt attgttattg ggttgtttct gtctttatct 720
   gtattgaaat tgcattctga acgaaaagtg atttcaaagt ctcgttgtat tgtaacgtgt 780
   attggtattc tgacgtttgt tcttttattg gaatatgttt gtactcgtcc tgcttgcatg 840
   tggtattgtg atgtcactca agataagtcg aggacgctta gtaagggaag tcaagaggtt 900
   atgaaagcat tagacggagg tttaacaatt acaacctatg taaacttgtt ggatgatcat 960
   cgttggcaag ggttacccgc taatcgtgtg tatgatgttc gtggttttcg gcagtttact 1020
   cgttttaagc cagaaattca gatgcgttat gtctattatt atgatcaaac agataatccg 1080
   atactggata agttgtatcc gaatttgagc atggaggaac gggcaagaga attggcacga 1140
   gtagaagatt tggattttga taaattcctt tctcctgaag agattcggga gaaaattgat 1200
   ttgacttcgg aaggtaatcg ttttgtacgt ttgttggaac gggaaaatgg tcgtaaaaca 1260
   tttttgcgaa tttataatga tggacaagct aatcctggcg agagggaaat aattgcagct 1320
   ttgaagagat taacgattgt tcctcctaaa gttggatttt tgacaggtca tgatgaacgt 1380
   agtattcggc aggttggcga atgggattat tcggcttttg ccgcaaatat tggtttccgg 1440
   tattctttgg taaatatagg gtttgatgtc gaggaggttg atttaagaga tacgataccc 1500
   ggagatattc atgttttggt aattgctgac ttgaaaaagc cgttgacaga tgacgaacaa 1560
   gagtatttgc aacgatacgt tgataaagga gggaatttat ttattttggc aacagccact 1620
   aggcaggaat tgttgaatcc tgttacacgt tcgttaggtg tttattttga gcctggaatt 1680
   ttggtgcaaa atcataaaca gcatcagcaa gatttgattt taggagaaat tact 1734
<210> 303
   <211> 2361
   <212> DNA
   <213> Unknown
<220>
   <223> Unknown gut bacteria
<400> 303
   ttgacttatt atatccgcca tacggcagcc caaccgggat atgctgatta ttatcttgtg      60
   cagaatgtcg gttctttgat ggaagacgag gaccaaaatg ggttagctca tgtgttggaa     120
   cacatggcat ttcatgccac ggaaagtttt ccggaaggag tgccggcttt tttgcagcga     180
   catgggattg aaacatttaa tgccgttacg aggtatgatg aaacgatata ccatgttgat     240
   catgtaccga cgatatcctc ggaattggta gattcttgcg tgttagtttt gcgggattgg     300
   tccggttttc tgatgttgaa gccggaagat atggatcggg agcgtaaagt gatccgggag     360
   gaacgtcgga tcagaatgaa tgtatctaag cgagtacaga aaatggcaga gccttacctg 420
   tacaacagga gtaagtatgc ccttcatgat attatcggtt ccgtagaggt agtgcaaaat 480
   tttactccag agcaacttcg cggttattat aacgatttct atcgtccgga tcaacaggcc 540
   gtgattatta tgggggatat tgatgtcgca cgggtagagg ctacggtgaa acgtttgttt 600
   gaggtcattc ctaaaagaga aaatccgaaa cccaggcttg tttatagaat tgaggataat 660
   gaagagcctt tatatgctaa gttaattgat aatgaaattc ctaataattc aatccagtta 720
   gtgaaacgta tccctcgtcc tcgtgtgaat tctttggaag agatgttgcg agagatgttg 780
   ctgcgagatt tttacaattc gattatgagg ggatatataa cggaatatgt agaagcggga 840
   gagtcttatc ttttgggggc tggtgcatgg atttctagtt tggtgagaaa ttatgatagt 900
   tttaatttgg tattgacctc attaccggga aaggaaaggg aggctttgca acaagtattg 960
   gagcaggtcg aatatgtaca tcgttttgga tttgcggatg aggttttgca acctttaatt 1020
   gagaattatc ggcaaggggt gaaatcgaat atggggcaag aaaagagtat gcctaatgat 1080
   gtgtttctcc agatttatca agataacttt ttgttaggca ggcctgtatg tacggttgat 1140
   gagaaacttg cggccacttt gtcggtgctt gactcgttgt ctgctaaaag ttttcaggat 1200
   tggatcactg gttggtacaa aggacgggat aattgggtgt tcctcatgca aggtaatgat 1260
   tctacttatc ttttcccgga tgcacaagag attgaaaaga tgattcggga ttcccattcg 1320
   gttgatatgg aaaaggagcg tcctcaagaa cagatggtag aggaaaatgc tgaattaatt 1380
   gatttcgaaa ttaagccggg gaggatcgtg aaagagagga agataaaagc gttggatgcg 1440
   gaagagtgga tattggataa cggggctaga gtcttttata aatatacgga tggtgataaa 1500
   ggcatgttta atatgttggc gggaagtccc ggtggacgtt ctgttctgaa agccgaggat 1560
   ttgccatccg ctgatgcgtt atcagccttg tttttgcaag gtggtttgta taagtatgat 1620
   atgcgtacgt taggattttt tctgaaggat catacggttg atgtgaatct gtctttagag 1680
   gaacgttcgg agagtattag tgttgtcggg gcatcggttg atgcagaact tgcttttcag 1740
   ttgttctacc tgacagtgga gcgtcctcgt tttgatcgtg tgctttataa tcgttttgtg 1800
   gcaattaatc ggatgaatcg ggcgaattta aaggcgacgc taaacgatac gattagtgag 1860
   atgttatctt cgattagaag aatggaatca cctcgtcttt ggagaaagga tactacttac 1920
   tatgcggcaa tgaattataa tcgaatgatt caaatctata aggaacgttt tcaagatgct 1980
   tctgatttta cattttattt ggtgggggat attgaaagag agaaagctcg tgagttgacg 2040
   attaaatatg tgggagccgt ttcttccgtg tttagaaagg agaaggcagt ggagcatgtc 2100
   tacgagcgaa gagagaatat aacgaaagat gttgaagtga atatgccgga tcgcaaatat 2160
   ctagtcagta tagagtttta taacaaattg aaaaccaatc cgacagaaga gttgtgtatg 2220
   cgtattttga aaatgtattt tcagtatcat tttcaagaaa aaattcgagg agatcaagga 2280
   gctgcatata gcgtccaagt attagggggt acttcttctt cacccgatta tcagcaggag 2340
   cttttcattc gctttgcgac t 2361
<210> 304
   <211> 1863
   <212> DNA
   <213> Unknown
<220>
   <223> Unknown gut bacteria
<400> 304
<210> 305
   <211> 1161
   <212> DNA
   <213> Unknown
<220>
   <223> Unknown gut bacteria
<400> 305
   atgaaaataa atatgtttgt tgtgcttgca agtttgttta tggcttgcgg cacacctaag 60
   gacgggtacg tggtaaaagg aagcctgaaa aatgcgggag acgggtatgc ggtgatctcc 120
   gtggccgacc atacgggcgc atcggtgata gcagatacag ccgagatgag cgggggaaag 180
   tttcgtttcg agggtaagac gcctttcgtg gcggcgtgtt ctttgcgagt ggtaccggaa 240
   gggaaagaag tgttggatat gcttatcgtg ttggagaata gtccgatcaa gctgcaaggt 300
   gattgggcaa atctggaacg gaataccggg ggaagtttct ttatccggga tatgaaagtc 360
   acgggcggca aaaacacgga ggtgtgtaat cttctggatg aagtgtattt ttccacccgg 420
   agattgccgg agttcaagga ttatgaacga ttggaaaagt ggttcgcttc gctggatgaa 480
   aatgcggagt tgacggacga ggtgatgcgg aaggcggata cgttgcagga gatagaggat 540
   tgttttcggg agttggtatt cggaaaacaa ctggaaatca tggcggcaca tccgtcatcg 600
   gaagcggcag cgctgtactt gacccccatg atagacggga tgaagttgga cgagttcgag 660
   cgggtattcg ggctgtttga tgaggcagtg aggaatagcg agatgttgac cgacatacgt 720
   gaggaattgg aaactcgcca gcgtctggct ccgggacgga tagctcccgt gttctcgttg 780
   gcacagcgtg acgggcagat gctgtcgctt gccgatttac gcggtaaggt ggtgttggtg 840
   gacttttggg caagctggtg tggtccgtgt cgtgcgtctt tcccttggat gagggagttt 900
   tacaagaagt atcacgacaa aggggtggaa attctcgggg tcggcgtgga taaggatgtg 960
   aaagcatggg aaaaagcatt ggatgcggaa aaattaccat ggttgcatgt acgggatgcg 1020
   aaagttccgg gtggaaagca tatggtaagt gacttgtatg acgtgacggg tattccgcac 1080
   ttcgtgttga tcgaccgtga ggggaagtta gttagatgtg gctattttga gtacgaactg 1140
   gaggagttgg tggataaatt a 1161
<210> 306
   <211> 696
   <212> DNA
   <213> Unknown
<220>
   <223> Unknown gut bacteria
<400> 306
   atgaaaaata gaatatttgt catattagct tttgtattcg tgtggatggg atgtgatgaa      60
   agagagatcc ctgtatatga tactggaaag ttttatatcg agtttgaaaa tgagacggtt     120
   gattcgacaa ttttcacgtt tatatattat ccgcgtaacg aatattatga tttgcccgta     180
   gcaatgaaaa tagctggaga aactgcaaaa cgggatttaa cgtataagat ttccgtggat     240
   gaagaacttt ctacggtgga ggacaaacat tattctttgc cggaaacaac ggtgtttagt     300
   aaaggtgtgt atcatgatac ttgttacgtt cgtttgaaca aaacagccga tttgtctaat     360
   cgggcggtga gacttgtact tcgattggaa gcgacttcgg atatggaggt tggtaagttg     420
   gacaatgcgg tggctattat tcaaatttcc aatacggtag cccgaccatc atggtgggat     480
   tctaacatag aacaatatta tctgggagct tattctcaaa agaagtttgt tctctttctt     540
   gaagtaacag gggcggattt gacggatgcg aatgatagtg aaaaacgggc ttatgctttg     600
   gaattcaaac aatatttgat tgatcatcgc ggagagcaag aaacgataga tgaaaacgga     660
   cagcctatga cggttcctgt gttaggattg gaataa                               696
<210> 307
   <211> 1929
   <212> DNA
   <213> Unknown
<220>
   <223> Unknown gut bacteria
<400> 307
   atgcaacata gttattcaaa gaataagtcc tctcaaataa acgggaattt gaatttttca      60
   tgggatatta caccttggtt aaggtatgaa tttgtgggtg gtataaataa caatgtcgga     120
   gtatcagagt cttatgcggg agaacaaacc cattacgtgg ccaagaatta tagagggtat     180
   gatttcggct cggaaatgtt tggttcggat aagtataaag cagcgatgtt gccatccgga     240
   ggggaattat ataatggaaa ttcggatgta ctggggtgga atgttcagaa taagattact     300
   tttcagaaaa cgttgaagga agatcatcgg atcaacttat tgataggtac ggaagtgtcg     360
   tcaactaaaa cagagaatag gggacaaaaa atttatggat acgtggcaga aagaggcgag     420
   agtgttataa gaccgacacc aataaaggag ttggtgccta ttggtaatgc tcctgtaaca     480
   ggatggggaa ttttagagga tttgtatgat ggagcaggat ggagtcgtag cacgttgacg 540
   gataataaat tttccttgtt tgccacgttg gcttactctt ataaaaatag atacgtgttg 600
   aatgccagta tacgtaacga tgcttccaat cgcttcgggc aggatcagaa taaacgtttc 660
   gacccgactt attctttcgg gctttcttgg aatgttgctc aagaaccttg gttgaatagt 720
   atttctaata tattgaacca gttcaatatg agagtgtcct atggtattca gggaaatgcg 780
   gtgaattcaa taagtcctga attaatattg agcatgggga ccacgaaact ttattatggt 840
   gattatatgt cgactatctt tcgtattcct aatccccatt tgagttggga acgtacgaag 900
   atttggaact ttggtttaga tgttcaattt attcaatgga tcacgatgaa cttggaatat 960
   tacacgagaa cgtccaataa tattgtgaat caacgaatag ccttagagta tggtagggaa 1020
   ggaacagagg tgaacggtgg gcgtattgtg aattccggag tagagtacac gttgaatatt 1080
   acaccaatac gtacgaagaa ttgggcttgg acgatcgggt taaattcatc taaaaattgg 1140
   aataaggcaa aaacccaatc gattagtgaa attacgctga gagattattt gtcgggatct 1200
   tctgataagg tattgaaaga gggatatgcg gtatctagtt tttggtcata taattttaaa 1260
   ggtatcaatc cgaatgacgg aagtccggaa tataatttac tttttgagca ggacgagcag 1320
   ggggattatg tgagaaatga agataataat cttgttttaa gggaaatttc cgattacacg 1380
   gatttacttg tttattctgg taaaaccgag ccagatttca ctggaggttt gacaactcgt 1440
   ttgaggtgga aaggtttgac gttcggggcg aatttttctt tgttgttggg agccaagaaa 1500
   aggttgccga atccatatcc cgcgagcggc aatattccat tatctaacgt aaatttgagt 1560
   aaggatctga agaaacgttg gaaagtgccc ggagacgaga aaaatacaaa tattccggga 1620
   gtttatgccg gtcgtattcc taatatgatt aatcttcaag atggaaaatc gtataacgtg 1680
   tatgatatgt ggggacagtc ggacgtgatg gttgtaaatg ccgatttttt gcgttgtcaa 1740
   caaatgtcgt tgacttggaa tatgaatgag gaatggtgcg cgaaaatagg agtgaaaagt 1800
   ttatcgctta acgcaattat gaataacgtg tttgtgattg ccgataaaaa gtttcatggt 1860
   tttgatccgg aattgggaga tagcgtgcag cccaagacat actcttttgg tgtaactgtt 1920
   ggtttttaa 1929
<210> 308
   <211> 1065
   <212> DNA
   <213> Unknown
<220>
   <223> Unknown gut bacteria
<400> 308
   atggaaggtg gagcgatatt ggccatggat acgattcaag acggcaaatt tgaactacac      60
   ggcgtaacag cttcaccgca gtactgtaat ttgataatca gctctaacac ctcttcatcc     120
   gacatgagca aaatggttaa tatttatatg tttctcgata actccgaact gacagtgaac     180
   gcagcccatt tagacagtct acaatttgta catcctctca tatcggaaac cgccataccg     240
   aaagcccggg tggaaggcgg tccgttacag cgagagtttt atgagtatcg cgatacctta     300
   acgcctttgc tgttgacggc ctcggaggta aataatgctt tagccatgct gaatatgcaa     360
   aacgaccaat acacgacaga ggaatacaac aaccgatttg acgaactata tcccaagaaa     420
   ctgacaacgg aagcagctat cgatgctgct aaaatggaat ttatccgcca gcatccacaa     480
   tctccggtat cgcttttcat cgcagaaggc ttgttgaaca ccactttcat tcgcactgcc     540
   gaagaaataa aggaactgac gcgcattgcc gaacaaatcg aggataccgt gcgtcgtccc     600
   cgtgtcttaa aaatggctga aatagcaaag acattgtaca agggagcatc atacaaggat     660
   atagaactaa ccgacccgac aggcaaaacc gtgaaactat cacagtatat acttcccgac     720
   cactacacgc tggtagactt ctgggcttcg tggtgcggtc cctgccgctg ggccatcccc     780
   gacgtgaaac aaatttataa acgctatccc caagatcgac tatccgtgat cagcatatcg     840
   ttcgaccaaa agaaaacaga ctgggaaacg gcaatgaaag aggaagccat gccttggacc     900
   caattatgga caggcaatcg cgaccaagtt accgcagctc agcgtgccta caacatcagt     960
   ggcattccac gcttgatgct cattgcccct gatggcaaaa tcgtttttag cggtaacgat    1020
   gctaacgcat tacgggttgc cgtggaagaa tgtttaggga aataa                    1065
<210> 309
   <211> 2475
   <212> DNA
   <213> Unknown
<220>
   <223> Unknown gut bacteria
<400> 309
   atgtggtccg gattattaat tggaaacctg agggcacaat ccgtgatagc agacaatcct      60
   aacgtgacgt tgcagctgag taacgtgaca ttgattgaat tttttaataa attgacagag     120
   gttacaggaa aaaatttcgt gtacagtgcc gatattttgg aacagaaggg acgggttagt     180
   gtgacggcag aaaacgagcg gttggacaag gttttggata gatgcttgaa agaaaaagga     240
   ttagggtaca cgtttcaaga tgatgttatc ttgatttatc ttcagaacga gaaggtttcc     300
   ccaggagaga aggtgaagaa acgacttgta cgaggagtcg taagggatca acagggtgac     360
   cccttggtgg gtgcgactgt agttttgaaa aatacccaca tgggggtggc tactgatgtg     420
   gatgggcgtt ttgagatgga aataatgccg gagaccgaat atttagtatt ttcttttatc     480
   ggtaagaagg aaaaagaggt aagtgtaaaa aagcacaagg aattagaggt ggttttagag     540
   gatttgatgg agaatttgga agatgttgta attaccgggt atggtaatat cagcaaatca     600
   agttttacgg gtaattcggt gagcattaaa cgtgaagatt tgttgaaagt gtcgaaaagt 660
   aacgtgattg cggctttaca aactttcgat ccttcttttc ggattcaaga gaataatcaa 720
   tggggatcag accccaacgc aattccggag atgtatatcc ggggacgttc gggtgtggga 780
   ataaaagaat tggataagga tcaattgagt aaatcacaat tagagaataa tcctaatctt 840
   ccgcttttta ttatggatgg ttttgagatt agcgcccgga aattgtatga ttttgacccg 900
   aacaggatag agaatgttac tattttgaaa gatgccgcgg caacggccat gtacggttcg 960
   cgggctgcta atggcgtggt ggttatcacg acgattgccc cgaagcctaa taaaatcatg 1020
   attacctata atatgacggg aactgttatg atgcctgatc ttagtgatta caacttgatg 1080
   aatgcccggg aaaaattgga agcagaggtg gcaagtggtg tgtatcaggg atcgaatgct 1140
   ttcggacagg atttgttgaa ccgggagtat catgctaagt tgtcaaatgt attgcgtggt 1200
   gtggacactg attggttgtc tcaaccgtta cgaacggctt ttaatcatca acacagtctt 1260
   tatttggaaa gtggcgcgga gaatttgcgt ttcggggtcg aattgaatta caataataat 1320
   gacggtgtta tgaaggggtc gtttcgtgat cgggctggtg taggcctttc tgtagattat 1380
   cgaatcggag attttcaaat ccgtaattat ttttcttata catcggtaaa atcgaaagaa 1440
   tcaccttatg gaattttttc tgattatact aaaaagttac cttatgatga gatgaaagat 1500
   gaatatggtc aatggttatg ggaaacaact acttggcacg acaataccgg gaatacgaca 1560
   gttaatccgt tgtatgaagc tggattgaaa agttttgaca agacggagca agaggagttc 1620
   gttgataatt tgaatgtgaa ctggtatgta acgcctcatt tacaattgaa aggacaggtt 1680
   agtgttatta aaaccggttc ggaacatgag aagttcgtgg atccgaaatc cagacataat 1740
   actcgaccat tgtctaatag taatgtttct tccggggagt tacacacgac atcaggagat 1800
   gaattatcat ggaatgctaa tgtatttgca gcatataatt attttttgaa tgagcataat 1860
   attaatttga atgtaggttt taatatccgc acttccaaca cggagtcttt gaatgctttt 1920
   taccggggat ttccttcggg tattttgcat tcaccgaatt atgcgcaaga aattgttact 1980
   aaacctactg ctaatcagaa taaaacccgt ttgttcgggg tggttggtac gctgaattat 2040
   tcttatagga atatttggtt attagatgtt gctttccgtt cggacggttc gtctgagttc 2100
   ggtagtgatc ggaagtttgc tcctttttgg tctggaggag tgggaatcaa ttttcacaat 2160
   tttgctttta tgcagcaaac gggatgggtg aatacgttta gactgaaggg gtcttacggg 2220
   gtaaccggaa aagtgaattt tgcaccttat tccgctcaaa caatttatca gatacaaaat 2280
   gatgagtggt ataaaactgg ttacggggct actttgatgg ccttgggaaa tatgtccttg 2340
   ggatgggaga aaactcgtaa gttgaatatt ggggcggatt tggaattatg gaaaggggct 2400
   ttacagcttt ctgcttcgtg gtataaggat aaaacggtgg atttaattaa tgatgtaacg 2460
   attcccgcat cttcc 2475
<210> 310
   <211> 1281
   <212> DNA
   <213> Unknown
<220>
   <223> Unknown gut bacteria
<400> 310
   atgaaaaaga tattttacat gattgtgttc ctaatgctag gaacattggg aaacttatca 60
   gctcaaataa ccttatttaa aggtacattt gatgaggctc taaaaaaagc acaacaagag 120
   aaaaaggatt tgttcgtgga ctttttcgca gaatggtgtg gaccgtgcaa aatgatggca 180
   agcgaagtat tcacgcaaaa agaagtcgga gaatttttca acaatcgttt catatgcgta 240
   caagtagacg tggatacaca agaaaacaaa gacatcgcaa aaagatacaa cgtaacagcg 300
   ttaccgacta tggtgtttat cagtcgggta ggtaaagaat tgcgccgagt acagggaagc 360
   gttcctgcag aatcgcttat taaagaggca aaaattgcca cgggagaaga attgagtttc 420
   gagcaattgt acgaaaaata caaaaagaag aaaaatgacc tcgatgtaca acaacaattg 480
   ttgatcgaag cccccgcatt tatagctaca caacagggat acaatcaaca gaaatgggga 540
   acccgtattg aaagtctttt cccagaatac ctaaaaaaca aaaaaatcga gaacatggca 600
   aacgaagcgg actttctagt gctaacgatg tatcaccgtg cggcttcaaa agaagaccct 660
   atctttgatt acctggcaca gaactatcaa aaatttgcca aagaaatcgg gaaagacaac 720
   gtggctcgct acttggtttc tttgaataac acatacatta tccaactctg taaaaaagga 780
   gacttaaatt acaaaaaacg gctggaacgg gtaaacgggg atttaaaaga tgtttactcg 840
   gaattttctt tcgggaatct ttcggttcag aatgccctca cgttacttgc cgatgccacg 900
   tacagcctgc atcgccatga cgagaacacc ttcttcaaga aaatggatgc ctattttgcc 960
   gggaaaggcg acaaaacgga attgaacgac tatttccaac ccttacaaga catcgctgtc 1020
   gtgtacgagg gtaaattgtc ggaaaacgct taccggagat gcatcaactg gatcgccaaa 1080
   gccttgacat tcgataacgt gggagcgtca aatcgtaccc gcttgttgat gatgatgggg 1140
   gactgcttga aaaacacagg cgataacgcc aaagcaaaac aatcctataa ccaagcattc 1200
   ctagcgagcg cacaaatcga gaacaaaatg gagatgcaac aactccaaca aggtatccaa 1260
   caatcgttac aaggcttatg a 1281
<210> 311
   <211> 1539
   <212> DNA
   <213> Unknown
<220>
   <223> Unknown gut bacteria
<400> 311
   atgaatagaa tgataaaata cgggttattg gtgaccatgt tttttatggc atcatgtggt      60
   gatttcttgg agcctaaatc ccagagtgaa tatgttccca aattagttaa atcattagac     120
   gaattgttgc tgggagaagt atatatggga ccgggatata gtgatgggca gttttacggg     180
   gtgttggggt tattcgatga cgatgtggct attcggccgg attggaaggc gaatgcttct     240
   gaggaagaga agataaataa aatccggatg gcttacacgt gggctaaaaa catgaaagat     300
   gagtttagtg gatataatac ctacggggaa gtgtacaaaa aaattcttgg atgtaactct     360
   gttttggatt atattgatga ggtgcaggga tctgaagagg aaaggaatag ggtcatggct     420
   caagctttgg ctttccgggg atatttttat tttcacttga taaatttgta tggaaaacct     480
   tactccgtgg gtaaagattc gccgggagta cctttgaagt tgacggccga gatggggaca     540
   agcggaatga aaaggaacac ggttgaggaa gtttatacgc ggataataca agatttattg     600
   gaagcggaac gtttgtttaa gacattgccg gaaggagagt ggagtttaca aaataaccgg     660
   atcaatttac cttgtgtgca attacttatt tcccgcgtgt atttgtacat ggagaattgg     720
   gcgcaggcgt tggcttacgg ggaaaaagtt attaccgatt atggttacga agtattggat     780
   ttaaatggag agcctctacc caatgagaaa atgaatccta cttataggag ttattatact     840
   tgggaaaatc cggagattat atttttgttt gggaatcaag gagatgtatt ggaactgcca     900
   ctaacaaggg tttattatag tggagataaa ggtttcacaa gttatatccc tgccgtggct     960
   tcagataatt taataagaac ttatgatctt acggatttac ggcgagatca ttatttaatg    1020
   tgggaagtaa tagattatgg atatactcca aggtataaaa aggccgtgag taaatttgag    1080
   gctgttagta attacaatat agaggtttct tggaatggtg gaaagtgggg cgttgctttt    1140
   aaggtcacag aagcttattt gaatgcggcg gagggagctg caatgcttta taaagaaacg    1200
   ggaaatggag aatttcaaat gaaggctcag gctttgcttg ataaattacg agtgaaaagg    1260
   tttagtgccg cggcatttgt gagtacggat attagtgccg cggatgagtt gatcgctttt    1320
   gtccgggatg agcgtcgaag agaattgtgt ttcgagaatc atcgttggtt tgatttgcgt    1380
   cgctatggaa tggaagagat aaaacatgtt tggtatgatg cttccggcaa tgcttccgag    1440
   tatgtgttgg agaaaaatga tcctggtttc acgcttcagc ttccgaatga agcttttgaa    1500
   tataattctg gtttggagca aaatgaaccg agaaaatga                           1539
<210> 312
   <211> 852
   <212> DNA
   <213> Unknown
<220>
   <223> Unknown gut bacteria
<400> 312
   gggctgtttc aaatcgggat gataggagga agattgcgtg gatccacgga agttgccgcg        60
   attactccgg gaagtaacaa ggctcaattg attttatctg atggagaggt cgtggatttg       120
   catgcagcgg taagacaatt gcccttgtct gttaccggga ttattcgaaa tgattcgttg       180
   gaaggtttga aatactcggg aaatgtcggg atgaaagaag agtataatgt gttgcaaaca       240
   ccggttgggg gtttttatca tttagaattg tccgatggta cccgggtctg gttgaatgct       300
   tgttcagaat tgcgttaccc ggtaagtttt gtgagaaaag aacgtcgggt gatgctgaaa       360
   ggagaggctt attttcaagt gagccgggat tcatcgcgtc ctttttatgt gcaaacttcc       420
   cgtcaaaata ttcgtgtgtt gggaacttca tttaatgtgc gttcttattc tgcaaatcaa       480
   gagtatacaa cgttggagtc ggggcgtgtt agcattcatt gtttggggca agattgtctt       540
   ttaagacctg gagaacaatt gcgtttgtcg gatacatctg ttgttattgt tcctgtacgt       600
   tcggaaagtt atatcggatg gaaaaatcaa tgttgggtgt atgacaattg tatgttgaag       660
   gaggttttca aggatttgga acgatggtat aatgtccaga ttgaattgga ggatgaatct       720
   attggtttac gacatttcac ggggaatttc agacggtatg ataatataaa tacagtatta       780
   gaaatgatag gattagttgc acatgtaaaa tacgaggtgg aaggacggga gatcgtgttt       840
   tcatggaaat ag                                                           852
<210> 313
   <211> 2400
   <212> DNA
   <213> Unknown
<220>
   <223> Unknown gut bacteria
<400> 313
   cggtacgaaa gattgttttt tttaaatgct ttgcgtaata cggttgctcc ttacaaaacc      60
   gcgtccggtg agtggaaaat tcctacatct tatgaagagg tctacgctaa taaaagcact     120
   aacggtccca tgtataactg gttttgggga aaaacgggac ctcaagatgc agtgatgtta     180
   caagatagtt tgaacccttt ttacaataat tcaacgcatt ggtatcgcca aaattacagg     240
   accgcaggag tgattaacgc aaacttgcaa gcatccgggg gatcggagaa cgtgcgttac     300
   atggttgggg ccggttatta tgacgagaaa ggaatcgcaa tcaatactga ttttaagcgt     360
   tttaacgtat tgtcaaattt aactgcccat ccggctaaac gtttgcgtat ggatacgcag     420
   ttgggactta cttacagtga tcgtagtagg ggaacttctg ctggagccgg gggaagtaag     480
   tttgaaggaa ttagttctga ggccaaaact acttctactc tgttgccggg aggaggagct     540
   atcggagatt taatcattca aaagtataac gaaatttcgg agaaaaatca aagctattct     600
   ttacgttata atatggcatt ggattacgaa ttgatgcgta atttaaattt gagagtatct     660
   gctggaatcg attataatca acagaatcag aataccttta aaccacgagc tttagatgcc      720
   tatacacatt tttccagttc ggaggggacg attacgaaag atttgtcgat tttgaatgaa      780
   aatttgctga cgtacacgta taattatttg gataagcata atttgaagat tatgctagga      840
   ttgtcgttcc aaaaagacca gtcctttaat aataaaggga aagcgatgaa cgggccgaat      900
   gataagattc attatgtcgg tgccggttgg ggaggagcta acgggttgaa taatacgggt      960
   acggcggctg atccttttta tgtgtctgct tttgaatact cttcaggttt ttcagaagaa     1020
   cggatgaaca gttatttcgg gcgtttgact tataattata aagaaaagta tttatttgaa     1080
   gcaacgcttc ggcgcgatgg ttcttctgtg ttcggggaag atgtgcgttg ggctactttc     1140
   ccttctatag ccgtggggtg gactttttcg gatgaaaagt ttatgaagcc gttctattgg     1200
   ttgagttttg gaaaaatacg ggcgagctgg ggtgtttccg gtcaaaaatt cagtcaacgt     1260
   tatttgtccc agggattatt gtctgctaat ggaaccacct ttcatgggga tatagccatg     1320
   ttcccggata taagaggagg agttattaac aagaaattaa gttgggaaga gactaatcaa     1380
   tatgatttgg gatttgactt gagttttctg gattatcgat tgcggcttgt catagattat     1440
   tattatcgtt acacgaaggg acagttgaat caaatttctt tgccgggaga tatttattac     1500
   catcaattcc aatggcagaa cgggatggcg atttccaatc aaggtttaga gcttgaattg     1560
   gaggcagata ttttgagaca cacggcagta aaatggaaaa tgaaattcaa tgcttcacga     1620
   aattggaatc gttttgaaaa aagtgctgac ggttttgatt ttatggaaca ggttatcgga     1680
   aaacctttgc atcagataaa ggtttttaaa acagacgggt tctataattc attggatgaa     1740
   gttccggttt attatttggc taataaggag gtacaacctc tttattccgg aattacggca     1800
   gggatttatc ttccggggag tcgtcggctt gttgatttga acggggacgg taggattacc     1860
   gaggatgctg atgtttattt tgcagagagt cctctacctt tagctcacgg tggttttatt     1920
   aaccaacttc gctggaaaaa ttttgacctg aatattttct tcaactattc gctggggcgt     1980
   catatcgtga aagtttacga tgatgtgagc atgacggcaa atgggagcgc aaaacctttg     2040
   ttcgtggatg tcaatgatgt gaaaatttgg acggaagcag gttctaaggc cgattacccg     2100
   aaatctcaag tatatgctta tttgatgaat caaacaagtg gacgttatga ttgtgatatc     2160
   gaaagtgtta gtttcttgcg gttaaagcaa ctgacgttgg ggtataatgt cggggaacgg     2220
   gttcttaata aattgaagat ttcaggggct agaatatttg ttacgggaga gaatttgttc     2280
   ctgttgacga attattccgg tttagatccg gaactggtaa atcctaccca aggtgtggat     2340
   aatatggggt cttatccctt acctcgtaaa tttaccgtgg gtttgtctgt aaacttttaa     2400
<210> 314
   <211> 1389
   <212> DNA
   <213> Unknown
<220>
   <223> Unknown gut bacteria
<400> 314
   gagagatctt atgtacgtaa atctcatcaa gatattttgt ctattacacc gacggtaacg      60
   acggatgctg atgtggaata tgcttgggaa ctttggactt cagtccggac agatggaatt     120
   atttacaagc cggatacgat tgggcgggag aaaaatttga actatgaagt gatggaagat     180
   cctggtacct attatttggt atttaatgtg aaagataaga aaacaggagt gacaaccgtg     240
   tgtcgtagta ctttgaatgt agagactctt aattcaaccg gttggtatct gctgaaggac     300
   gaagaggtgg ctggagagat gtacacggac tatgacttta tttatgacgg gggacgaatt     360
   gataattgga ttgcttttta caataacggg aaaaaattga aaggtgaagc agtaaaaagt     420
   atattttctt ctaattattc agatcaactt tccggtggag ggtataatta cataaacgtt     480
   ttgatgccgg tgtccaaaga ggaagtttct gttttggaag ttagtaatgg catagtccgt     540
   ttgccttttg aatctttgtt ctttgaatct cttgaagtga aaaattttca agatatttct     600
   attcaaaatg atggaacgaa tgttggtttg gtaaatgatc atcggtttta taatatgtct     660
   acacagaatg caacctcttt ttatgcttca tacggagatt acaacgtggc tagtggtatt     720
   atgagaggat taaacggacc aacttttttt gatagtgtaa cagaatcatt tgtttacgtg     780
   tcttacggta atataaccag atttccagca gtttcaggtg atttgacttg taataacatg     840
   aatgccgatt tattatggag tagtgttatt aattttagag tacagggtac aaagtggtat     900
   gcttacgctt tgatgaagaa acatacaggg gaaaatcctt atatgttgat aaaatttaga     960
   ctaaattggg gtgatacaga aaatcgtgta gagcaagtta acattcttcc gtctacgctt    1020
   aatttggtaa ccgcagagag gtgggctgta aatgcaaact atggaattat ttatttcgtg    1080
   aaagatcaaa aattgtatag atatagtata gatagtggta atgaagaggt aattttagat    1140
   attcccgcag gagaagaggt aacttgtatg cagcatatta tttatcctaa tctgatgtct    1200
   gctgatgatt atggctctgt catagatcaa tttgcagtag caacttatgc taacggaaaa    1260
   tacaaagtgt ggttgtatga atttgatgcc ggaaatttga aaccgttaag taaacccacg    1320
   tttgaaggga acgggcgtgt tgcttgtata aattatgttt ccggtaatag ttctaatttc    1380
   ttattctaa                                                            1389
<210> 315
   <211> 2733
   <212> DNA
   <213> Unknown
<220>
   <223> Unknown gut bacteria
<400> 315
   caggcccggg agacggagag aaagccggta accgtgaagg ggaaagtggt tgatgagaag 60
   aaacagcctt tacccggggt tacagttttg ttaaagggta cgactttggg agtaacaacc 120
   gggggagagg gtgatttttc gattgtgatt actgatacga ccaaggctga actcgtattt 180
   tcatttatcg gtatggtgtc tcaaacaatc ccttataaaa gtattcctaa aggggagtgg 240
   acgatcgttt tgaaggatga tgtacaggag atggatgaag ttgttgttac gggtatttat 300
   tctcgtaaaa aggagagttt taccggatca tcaacaacct atacggcaaa ggaattgaaa 360
   actatcggga accagaatgt gcttcagagt ttgaaaacga tggatccctc gttcgcgatc 420
   gtggagaaca atcagttcgg atccgatccg aaccgtctgc cggatattaa cgtgaggggt 480
   aaaaccagcg tgatcggttt gacacaggag tacgaggtcg atccgaatca gcccttgttt 540
   attttggatg ggttcgagac aactttaaag acgatcagtg atttgagtat ggatcgcgtg 600
   caaagtatta cggtgctgaa agatgcggca gccacggcaa tttatggttc gaaagcggcg 660
   aatggtgttg tggtggtgga gacgaaggct cccgctcccg gaacattaag gttaacgtat 720
   aacggaaatt taaatctttc ttttgctgat ttatcggatt ataatttgat gaattctaag 780
   gaaaaattgg aatttgaacg tttggccggg tattacggag atttggatgc taacggggaa 840
   atcgtgtcgg aatatgacca gcgtttgtat taccagcgtt tggccgaggt gaaacgaggg 900
   gtggatacct actggatgag tgaaccgtta cggttcgcaa cttctcatag tcacaacctt 960
   ttcgccgaag gaggggatga caggatgcgt tacggcttgg gattcagtta taacaagacg 1020
   caaggtgtga tgaaaggatc tgatcgggat gtgattaatg gcaacgtgca attgttgtat 1080
   cgctacaagt cattgtcatt caagaattac ttgaatttgg attactcgat gtcttcacga 1140
   gagaaggtca cgttttcaaa attttcacag gcgaatccgt accacaggaa aaagaatgag 1200
   gatggtgtgg tggagcaagt gttagagatt tacactgatt cggatgcaaa ttctcctact 1260
   tatcttcagg aattaaagaa ttataatcct ctatatgata tgactttagg ctcttccgat 1320
   gaaacctcct cttttggttt tacgaataat ttcgagatcg attggcaggt gtttgatggt 1380
   ttgcgggcga aggtcgtttt tagtatttca aagtcaacgg accgggggga agtttttaaa 1440
   tctcccaatg cttccgatta tgctgaaacc gcttcaacgg aacgcgggtc ttaccgggag 1500
   acaagagacg agaatttgag ttatgatgga gatcttaacg tgacctacgc taaattgttt 1560
   aacgaggtac acatggtaaa tgccgtgggt ggaattcgtt tggcttcgag caaatcacaa 1620
   tccagtggtt acgagtctat cgggtttatc gatgaccgtt attcaaatcc ttctttttct 1680
   tcagggtatc ctacgggagg taagccatcg tatttcacga gcgagaagcg ttcggcgagt 1740
   tattatctga atgccggtta cgcttatgat gatcgttatc tgctggacgt gaatcttcgt 1800
   tcggatggtt cctccgtgtt cggcttgtct caacaattca ccacaacttg ggctatcggg 1860
   ttggcgtgga acgtgcacaa ggaagacttt tttcagaatc agaactggct gaattttttg     1920
   aaattgcgtt tttctatcgg taatcccgga aatcaaaact ttgatgctta tatttccatg     1980
   aacgtgtaca agtacgcaac tggttacccg aatcctttcg gggtaagtgc tattgtttcg     2040
   acttggggaa ataatgattt ggattggcag aaaacaatcg accagaatta tggtattgat     2100
   ttggcttttt tgaataatcg gttgaaagtt acggttgatt atttctataa aaataccgat     2160
   cctctattgg tttatgttca gacgccgacc tctaccggaa cctctactgt tccgatgaac     2220
   ctgggtaagc aagtttctca gggattgaca gcaacattga attatatgat cctgcagcga     2280
   gagaatatga gttggaattt taattttaat gctcgtcata ttacttacga gtaccggaat     2340
   atcgggaccg ccttggataa gtttaatcag gagaacagga gttctaattt ggtgagatat     2400
   tacgatgggg gaagtccttc cgatttgtgg gccgtgcgtt ccgcgggaat tgatccggct     2460
   tccgggcgag agatattcat taaaaaagac gggacgcaga cattcgagca tgattacgcg     2520
   gacgaggttg ttgtcgggaa ttcggatccg aaggtggaag gggtaatcgg tacttctttc      2580
   tattacaagg gattttctgc ttctgtcaat ttgcggtatc gcttgggagg acaaatattt     2640
   atgtcgactt tatacgacaa ggtggagaat atttcggaaa atgccttgca atataatcaa     2700
   gataaacggg cattgtatga tcgttggcaa aaa                                   2733
<210> 316
   <211> 1131
   <212> DNA
   <213> Unknown
<220>
   <223> Unknown gut bacteria
<400> 316
   atgaaaaagc tctgtttttt attactgttc ggcgctgctg cttgtacgca gcagcctacc      60
   gatcatttta tcctgcgggg tacagtcccc ggagtcatgg acagcacgaa agttacccta     120
   cgaacagtca cccgctggga caaagattta gctagcgctt acgtcattga cgggaagttt     180
   gagttgcgcg ggcaactgga tgctcccacc ctatgcaaac ttagcctaaa caacgcacgt     240
   tcttacgagc ttgatttttt tgtcgaaaac ggcaaactta cctttaccac accacatatc     300
   gacagtctgc cacaagcgta cggactttac gacatccgaa aagaaaagaa ctatcgggtg     360
   gaaggttcca ccacgcaaga tgcgtactac cgctaccaac aacaaaccct tccgttgcgt     420
   tacggcatac aggaactccg caagcaggca aatcaaatcc cggactataa cagtcgattg     480
   gaagccatga atgcggaact agacaaaaca agccgggcat ttatccgtaa caatgacaat     540
   ctggctacca atctctacat tgccgggaca ttgaagaaac ccgcattcac ctacgatcaa     600
   acttatcttg acgaactgga acaactcttt gcttcctgtc aagatacctg tgccgcactg     660
   aaagatttcc gcagttatct ccgtgatgcc tcccgactag tccaaggctc cccgctacaa     720
   gaggctgaag tggtcaatga caaaggagaa agtgtatcac tgatcgccca gttgaacaga     780
   gaagggtaca cgctgatcga tttttgggca tcgtggtgta tcccctgccg agtgagcata     840
   atattcctac gcgaaatata caaaactcac ggggattcaa ttcgcttcat cagcatatcc     900
   ctcgatcaac aagaggccga ctggcaaaaa gccctgaaag aagaaaaact gccatgggca     960
   caattccgta gcctacccga acagactaaa acattcacag aacaatataa tctcattgga    1020
   attccagcct tctttatcat cgatagcgaa ggacgaatcg tcttttcagg cagttccatg    1080
   aacgaactga cggaacaact acctaaaatg attacaaact ctacacactg a             1131
<210> 317
   <211> 1434
   <212> DNA
   <213> Unknown
<220>
   <223> Unknown gut bacteria
<400> 317
   atgagaaaag tgattattat attgacagct ctggcgggaa gcttattctt caatagctgt 60
   aatagttggc tggatgtaaa gccttatgat tctatggcag aagaacagct ttattctacg 120
   gagaatggga ttcagcgggc tttgaacggt ttgtacctga gtcttgcatc gaatgacttg 180
   tatgcagaga atttaggagg gggtgctatt gatgtattgg gacagcggta taaattgtcc 240
   agcgaacatt cttattatga tttaagcgaa tataaataca cgaatgacgg tccgaaagag 300
   acttttgagg caatttggaa ggctgcttac aagttgatcg cagattgtaa tgaatttttg 360
   gaacaagttc ctaggcatca ggatttattg tcccagggaa attataaaat gatgatgggg 420
   gaggctctgg ctatccgaac attattgcat tttgatatgt ttcggatttt cggacccgtg 480
   tacacggaag cgaataagca agaagcttct atcccttatt atagtatggt gacagatgtt 540
   acacaaccta tcctgtcaac ggaagaacta gttgaacatt tatttacgga tattgattcg 600
   gcaatcgtgt tgttgtcaaa agatgtgatt ttagcggacg gggttgttaa aggtgaagga 660
   ttttgggatt atcgtaattt acgtttgaat tactatgccg tatgggcatt gaaggcacgt 720
   atgtgttggt atttggggga tgcgtatgct gatgaggctt accggattgc aactgctttg 780
   ttggagggga aagatcctaa aacaaatgag gtaaataatt ttaatacggt ttttgaaccg 840
   gtttctcaaa atgtggcggt gaccgatcga gttttcttta gtgaagagtt gtttgccttg 900
   cataatatga agcgtactac ggtttataaa gagttattta gttctgattt ggaagataat 960
   aatatattgt tgtctacaga ggattatatg aataagcttt ttgatgaacc caacgattat 1020
   cgttggcatt cttgggaggt cgctaccggg cgtcaaactt ccggattgat ggtatttgtc 1080
   aaatatgtag aacagacgga aaatagaaat gatccttact tgtatgaaat ccaatccatg 1140
   ttgcggttga gtgaactcta tttgattgct gcggcaacag ctcccgatga tgcaacagtt 1200
   actgcttggc ttgaaaaatt gcgaatgatg agaggttatc agtctggaat ggtaggttcg 1260
   gatgctcaga aagtattgcg gcaagaatgg gaaaaagaat tttatggtga aggacagtat 1320
   tattattttt tgaaacgcaa cggtaagact tctgtttctg gaccgaatgg atctaagaaa 1380
   ccgaattatg ttattccatt gcctgagagt gaaacaaata atagacgcga ttaa 1434
<210> 318
   <211> 1920
   <212> DNA
   <213> Unknown
<220>
   <223> Unknown gut bacteria
<400> 318
   atggaagaat acaacagtct gttcaaaaat atgcccatta tatatataaa agaagaattg      60
   atatataaca aggaaggacg agtaatagac tttatattca aggaagtcaa ccccactttt     120
   gaaaaatata ttactgcaaa aagcaatatt ctaggaaaga aatacagcga aacaagcgga     180
   caacacacca gatgtataga tctttacaat tcattgcaga acaaaaagga actatccttt     240
   caatactatt gggaagctaa acatatttac ctaacggtta tcattacctg ttcccccaaa     300
   aaaggcttct tagatgtttt ctgcgtggac aacacggaac tggcacaagt tcaacaaata     360
   ttacgctcaa ccaaccacaa gttatcgacg gcactcgatg tcgccaatat cactccatgg     420
   aaatgggatt tggaaaaaca tacgattcta tgtgatgtaa accgtcccat tgaaatcagt     480
   ggcaataact cccctgccga agaacaacaa ttatccgttc cggattcatc ctatttttcc     540
   aatatttgca aacaagatat tgatcgggta agaaatgctt accaaagttt gatagagggg     600
   aaagttccga gaataaaaga agagttccgg gttatttcca acaaaggcta ttccgtccat     660
   tacgaatggg tggaagttca agccaccatc gatgaataca accaagcggg aaaagcgaaa     720
   acgctagtcg gttcttcttt gattatcact caaagaaaag ccatggaaaa tgacttgata     780
   aaagccaaag agaaagccga agagtcaaac aaattgaaat ccgccttcct agcaaacatg     840
   agccacgaaa ttcgaactcc cctgaacgcc attgtcggtt tctctggtat tctggcatcg     900
   atgaactcgg atattgatga agaaaaacag gaatacatac aaatcatcga aaacaacaac     960
   aacctactcc tccaattaat caacgatatt ctagaccttt caaaaataga agcaggcgta    1020
   ctggaattct cgtacaacaa cattaatgtc gacgaattgt tcatggacat agaagagtcc    1080
   actaaaatga ggaaccaaaa caaaaacgtc tgtattctat acaaacggac tttaccgtca    1140
   tgttacatca gtaccgataa aaaccgcttg actcaagtca tcacgaacat gattaacaat    1200
   gcgatgaaat tcacgcaatg cggcagcatc gagttcggct acaaccttca aaacgaagat    1260
   tcattatatt tctacgtgag tgacacggga tgcggtatcc ccgaaagtca agtaaaccgt    1320
   gtttttgaac gttttgttaa actcaacaat tttgcacaag gtacaggcct ggggttaccc 1380
   atttgccaaa ccatcatcac aagcatgggg ggcaaaatcg gagtaaaatc aaaagaaggt 1440
   gaaggaagta cattttggtt tactcttccc tatcatcaag cagatcaagc aggagataat 1500
   atcgccaaag cacccgaaac ccctcgtctc gtggacaaaa aagataaact cgtcatctta 1560
   atagccgagg acaatgaaag caattacaag ttatttgaaa ccatactgaa aaaaaattac 1620
   acattaattc atgcctggga tggagaggaa gccgtacaat tattcaaaca acacaaccca 1680
   caccttgtat taatggatat aaatatgccg aagatggacg ggtatgaagc tactcaaaaa 1740
   atacgggaaa tatcccccga tgtacccgtt atggcaataa cagcctttgc ttatgccgaa 1800
   gatgaacaac acattctgaa tagtgggttc aacgcttaca cgtccaaacc gattcaggca 1860
   gaacaattac aaaaacaaat cgggaatttg ctaaaaaaac attttttatt cctgtactaa 1920
<210> 319
   <211> 1854
   <212> DNA
   <213> Unknown
<220>
   <223> Unknown gut bacteria
<400> 319
   gatggtatcg tgatgcagga tcccgttgat gtaccggcat cagacttgaa tgatcctgat      60
   tacattaatc gtatcggtaa cgccattgcc ggaatcaacc cgcaagacat cgagcgtatt     120
   gacgtgctga aagatgccgc cgcaaccgct ctttacggta ctcgtgccgc caacggggtt     180
   atcgtggtaa ccaccaaaaa aggacgcatc ggacgcccca ttgttaatta caatttaact     240
   accacctttc gtcaacgccc ttcttataac gaccgggcca ttaacgtgat gaattccaga     300
   gaacgtatgc aattttcccg tgaattggtc gcacaacact atccatttcc ggatgacata     360
   aacatggtag gttacgaggg gctattaaat caattatata atcacgaact aaccgatgaa     420
   caattcgaga aagaagtgga acgtctcagt gttatcaaca cggattggtt cgatttatta     480
   actaaaaact ctgtatcaca ttctcacacg atcagtattt ccggaggttc gtctgaagca     540
   acttattatg cctcgatcgg ctacactcgt gataatgatg tggtttggga cagtaacaac     600
   gaacgctaca cagccacgat gaaaatgaac acgaacctca cttcctggtt caacgcttcg     660
   ctgaacgtga acgggaatgt ctctacccgt cagtattatc aacaggaatt atctccgatg     720
   gattatgcct ataaaacaac tcgggcgtta cccgcatatg acgagaatgg ggaatatttt     780
   tattttcaga aaagaaaatg gggaaccatt agtccggaat attatcgata caatattctc     840
   aatgaattgg aaaacagttc ctacaaccag cgtggttctg cgttatctgt tacggcaaac     900
   ttacaattca gtctgataaa ctggctgaaa gctaatgtta ttgcatctta ccagacatcg     960
   aacacgattc aggagagctg gtggggagag caaaccaact atgccgcaaa attacgaggc 1020
   acagaatatg gtgtattgcc agcacccaga accgaagggg aaccggatta tctaggtaat 1080
   cctacctata ccgggggaac aagcctcttg ccctatggag gagaactttc acacaatcaa 1140
   tcggacaatc actcttatac cgtccgttta cagttggacg ggaactactc tttcggcaaa 1200
   tcagatcagc ataacattaa cggttccatc ggttttgaaa tgagttctac ccgcaaccga 1260
   ggttattcac gcaccgaacg tggttattac aaagaccggg gcatgagttt tgtgaatgac 1320
   attgatcatg agaaatttcc cgattatgcc cagtgggtgc tacaaaatcc cgctcgaatg 1380
   actgatggta aaagtaatac catatcaagc tatgcttcca tatcttatag ttactacaac 1440
   tattttaccg tgaataccaa tgctcgcgtg gatggctcca acgcattcgg agaccaaagt 1500
   aacgacaaac tgttgcctat ctggtcggct tctgccaact ggaacatttc ggagttaccc 1560
   ggcttgaaag agacaaagtg gttggacttt ctccgtctga aagcatcgtt tggttaccaa 1620
   ggaaatatgc tcagtgatca aagtccggtg atgattatct ccaaagaacc gctaaattct 1680
   catttcggag agaacgtggc ttccgtttca cgctatccca acccgaactt gaaatgggaa 1740
   accaccactt cttacaattt aggcatggag cttggactgt ttcaaagaaa agtgatgata 1800
   gaggctgaat actggtggaa acacaccgag gatgccttta tggataaatc catc 1854
<210> 320
   <211> 1128
   <212> DNA
   <213> Unknown
<220>
   <223> Unknown gut bacteria
<400> 320
   atgaaataca ttttatttat cgcattcatt ctatccgtcg tcagttgtgc aaaacacgac 60
   ggatataccc tcacgggact cgtccccgaa gcatgggaag ggaaacctgt ttttttggta 120
   ctggatgata cgaatcaacc tcattttatt gacagcacga aaatatcggg aggtaaattc 180
   aaattccaag gtaaattcga ggttccgcgt tattgcacga ttaacattta ccttgatccc 240
   aatgaccgac agacccgtag taaaattata aacttctcgt tatttatcga cagcacagcg 300
   gtagaagcca cttgcaacta ttccggcaaa caccccgtgt tccaaatctc cggaagcggc 360
   acacaaactg aataccagaa ctacctgagc gagatacaac ccatggaaga tgaccggagt 420
   aaaacgttcc acgcttacgg ggaagcctac tacagtacga aggatttagc gaaagcgatt 480
   gaccttgcca aacttgtcac ggaaaaaaga ctcgcgatca gagaggctaa aatgaaatac 540
   ctgaaagcac accccgaatc cgccatcagc gttaagatag cgcaagaact ctccgacaga 600
   aattccgacc tttccctgaa agaaattgaa ggattgttca ctagcctatc gcccgaaatg 660
   caaaattcgg aaatggggca ggctcttcgg gcaacaattc aaggtagaca ggtatttata 720
   ggaagtccct ttatagaccg ggaactctcg gcaccggacg gaagtaaaaa gaaaatatcg 780
   gatttcgtaa aacccggatg tactacatta atcgaattct gggcatcttg gtgcaacccc 840
   tgccgtgtgg aaattcctca catgcgtaac acgtacaata aataccatcc caaagggtta 900
   aatatcgtga gtatctccat tgacagtgat ccgaagaatt ggcatcaagc catggaagag 960
   gaaaaaatgc cttggggaca gcttattgat aataccaaag caacctttcg agcttataac 1020
   ctgactggag tcccttcttc aatccttgta gacgacaaag gaaagatcat taacgtaaac 1080
   gcaagaggcg gatggctgga tgccgccatg caggaaatct atgattaa 1128
<210> 321
   <211> 1782
   <212> DNA
   <213> Unknown
<220>
   <223> Unknown gut bacteria
<400> 321
   atgttagcta tcggtatacc aaatccgctc tatgaagcga cattaggtaa ttttagccgg      60
   ggagagtctt cgaacttgac gaataacttg agcttttatt ggtttatcaa tgatcatttg     120
   caattacaaa gtcaattttc tgttacgaag aatgattctg aagacaaaga ttttacagat     180
   ccgttgtcaa cgaagtacgg ttcttctgac aatcctttta ctcggggaag tttgagcgtg     240
   tcaagtacga ataatttcaa ttggaacttg aatgcatttt tggcttataa caattcgatc     300
   gggaaaaatt atttgaattt atcttttggt atcaatgctc aagaatcaca aacaagtaat     360
   tcttctgctc aatacaaagg attcccgtct gcggctctgc acacggttgg gcatgcaaaa     420
   gaaatcgtga gtaaaccttc cggagcagac aataaaaccc gattgatggg aatttttctt     480
   agtggaaact attcttggga taacatcttt ttgggggatg tgtctattcg gtttgacggt     540
   tcttctgaat ttggttccga atcacgatgg gggtctttct ggtctttagg tgccggggtg     600
   aacgtgcata attttgaatt catgcaatcg cttccgtgga ttaaccagtt taagattcgt     660
   ggaacttacg gtgccacagg taaggtgaat tatcctcctt atgcggctcg ggatatgtat     720
   aatattttgt ttgacgattg gtattccacg ggtattgggg ccacgttaca gggagtcggg     780
   aatgaaaatt tggtgtggga aaaaacaaat acgacgaatc ttggtttcga tctcagtttt     840
   ttcaaaagca agtataacct tactttctcg tggtataatc gtcaaacggt agatatgatc     900
   acggatgtga cgattccctc ttctgctgga tttacatctt ataaagataa tatgggagaa     960
   actcggaatc gcggatatga aatcagcctg aatgctatga ttgtaaatca caaagatttt    1020
   ggggtgaatg tgtttgcgaa ttttgctcat aacgagggaa aattaatgaa aatatccgaa    1080
   tcgttgaaag cgtataatga gcgggtagat gcttatctga ctcctactta tgcttggtat    1140
   cgaccgacgg cagaacaatc tttgcctttc ttgaaatatg aggaaggtgg ttcgttaaca    1200
   gctatttacg gtatgaaatc tttaggaata aatcctcaag atggaaatga attatttgtt    1260
   gatcgttcgg ggaatgtcgt gggagaatgg atggcttctc aacaacaaat tataggaaac    1320
   acggaaccta agggacaagg ttcgtttggt attaatatcc gttggaaaag attaaccttg    1380
   tatacgagtt ttatgtatga attcggagga cagcaatata attccacgtt gttgaataag    1440
   gtggaatgtg ttgatttgtc ttcgtccaat gcagataaac gagtattgac tcaacgttgg    1500
   caaaaacctg gggacgttac gccgttgaaa aatatcgctg atcggaatcg aacgactcag    1560
   tctacttccc gtttcgtgca ggataacaat gaattttcca tcaattcgct ttcgttgtct    1620
   tatgatttca acccggaatg ggtaaggcgt atcggattgg atgttgttcg gattcaagca    1680
   agtacgaacg acttggcaac attttcatcc attaaacaag aacgaggttt ggactatccg    1740
   tttgcccgga cgtttaattt tggtttaagt gttagtttct aa                       1782
<210> 322
   <211> 1524
   <212> DNA
   <213> Unknown
<220>
   <223> Unknown gut bacteria
<400> 322
   atgagtatga aaacgataaa ctatatttta gggtgttgta tagcgatgac attgctatac      60
   gcctgtattg atgacgaggg aaattacgat tactcggaat tgaaaaccgt gaatattacg     120
   aatgtatata gcaactggtc cgtgcctttg ggggaggaat atgttataac cccgacaata     180
   gattacgggg atacggaccc gtctgaattt gtttatacat gggtgagtga aattcaagag     240
   gaatggagtg atacgatttc acgtgataag gagttgcgtt acacatttaa ggaaatagct     300
   aggtatatga cgactttcgt ggtggagcat gtgcctacgg gggcattaac atccaaacaa     360
   atagatttaa atacggtatc ccgttattcc acaggatgga ccattttgtc ggagcaaaac     420
   aacaaatctg tattgagtta tatccgggcc gagcagtcgg gagagaatag tataatctac     480
   aattttttta aggatattta cactacactt cgtggagatg atttgggaac aggacctatc     540
   cgaatgggaa gacatttttc cagttcgagt gatgaaatcc tagttattca ggagagtggt     600
   gcggtagaaa ttgacggtca agatttttcg aaagcaattg ccactcatga ggaatttgtg     660
   ggaggggcat atcctgccaa tttcgttcca aaacaagctg aatatggagc taggttggag     720
   gctattttag gtacagatgg aaacgtgtac acgcgtgtaa atccgaatgg aagttttcaa     780
   atttgtcaat acaatacgca tccagccttt agcaatgccc gaattgagaa gatgtattat     840
   tgtgcttatt taggctatat ttatatgtat gatgaattga atcatcgaat gattgctttg     900
   tatgatgaac ctcaggctta tacgggtatg gtgatttatc cgtccatgca tccggatagt     960
   acgcacgtgg ataactttac tccggttgat gatatgggag agggaacaca attggtattt    1020
   attgggtcgt acaatgtgga aggagctaca ggacgggatt ttgtacaaat tattaagaaa    1080
   ggaacgaatt attatttcca aacttacaaa gcctatttgc atagtggttc atcaaacttg    1140
   tatatttttg acgagaagga agaattgttt gtgggaaatg attatgtgaa tgagaattca    1200
   aaatattgta tagacggatc ttcatatctt tattttacag cagataatgt tctctattgt    1260
   tggaaccgaa tcaatcaggt tgaagtgttt tatacgttct cgagcaagat tactgatatt    1320
   gaacgttatg ctaatggtag tgatcgagaa gagattgctg tcgggcttga aaatggggag    1380
   ttttatatta ttgatgcttc ttacgagtct ataagtggtc agaaagagaa agttttgttc    1440
   aagactgaag gtttggggcg tgttgtggat gtgcaataca agtacgggaa ttcttcgaat    1500
   tttaataaag aatcgagatt ataa                                          1524
<210> 323
   <211> 1137
   <212> DNA
   <213> Unknown
<220>
   <223> Unknown gut bacteria
<400> 323
   atggcgcatt ggaacgaaat agataatata tccgagaagt tactcaagaa aattttattg      60
   ggagagaata aggcagactc catcccttca caagaggacc acggacgctt gacagacaaa     120
   tataaaaatt ggaatgcact tgccgatcaa tccaaaaaag ttttaaaata tgacgccaat     180
   aaggctttcc aaaaattaca attccagaaa tatcgtcgtc gcaaaataat aactggctgg     240
   agtattgcag cgagtattgt actactgctt tctatctcca ttttcttcat tcaggaagca     300
   cccgaagtaa cacccgtttc gaaaatcgaa ccgggttaca agaaagccac tctaacttta     360
   tccgatggaa caagctggga catccgggaa gcagtacagg ttatccagac aaaaaacagc     420
   gaactcaaga tagattcgtc aggattaagc gtacacagca acacgattgc tcctccacaa     480
   gaagaaacac aataccacac gctgaacgtt cccagagggg gtgaatataa tttaacccta     540
   tcggacggga cgcaggtatg gttaaattca gaatcagaat tgcgatttcc ctcccaattc     600
   gacacgaaca atcgaacggt atattgtaaa ggagaagtct atttcaatgt tacccggaac     660
   ccagaatctc cttttatcgt gaaattagat aaaggtgaag taacggtact gggcacagaa     720
   ttctgcacaa gcgattacaa aagttcggtt gtagaagtca ccctggtgac ggggaaagtt     780
   caattcaagg cagaaaacgg ggattccgta tccttgaccc cttcccaaca tcttgtttac     840
   aacacgacaa acgacagtat ttcagtcacc actgtcgaca ctcgtgtata tacagcatgg     900
   aaagataatt tattctgctt cgaggatgaa actctggaaa atatcatgca cacgttgtct     960
   cgatggtacg atgtagatat atattttgaa tcggaagatt taaaaagacg acatttctcc    1020
   ggaactattg aaaaatattc aaatataaaa tcattcttag aagtttttga aacaggaaca    1080
   ggtataaaat tcgacattta tggacacaag ataagcataa gacaattaaa caaataa       1137
<210> 324
   <211> 2655
   <212> DNA
   <213> Unknown
<220>
   <223> Unknown gut bacteria
<400> 324
   gtgctggttg gcgccgatca cgagactcta gttgattatt caaccataaa attaatattc 60
   aaagttatga aaaaaaatcc acgtagcatg cggattcctc cgcatggggt gaaaaaattt 120
   ttacatcaaa tgaaatttac attattattt gtcatgctat tctgctttta tgcagaagct 180
   tcttcacaga aagtcaccct aaaagtgaag aacatggatc tttactcggt tctcttacaa 240
   ttaaagaatc aaacgggtgt ccgcatatta tatgatgcag actacacaaa acaaattaaa 300
   tgtacagatg ccacattttt tgaaaaagac attaaagatg tgctatcaca attactgaac 360
   aacacgaaac tcgtttacaa agaggtcaac ggtactttcg ttatcagtaa agcatccgaa 420
   caagaggata aaacagtaaa actggccggt acggtaaaag atatacaagg aactccccta 480
   ccgggtgtta ccgttgtgat taaaggaacc acaatcggag tttccactga cgcacaaggt 540
   aattttacct tgaacgtgaa ggaacaagag aaaataacac ttgtattctc tttcgtggga 600
   atgaaaacca aagaagtcgt tgccactcct aacaaagcga tgatgatcac ccttgaggag 660
   gatacccaaa ccatcgagga agttgtgatc acgggttacc aagttgtaga caaacgaaaa 720
   aacacaagtg ccatcacttc agtaaaagcg gcagacatca tgcgccccgg tattaccacg 780
   attgaccaga tgctggaagg tcaaattccg gacttgattt ccatgaccaa ctctggagaa 840
   atcggtgtag ctccgaaatt acgtattcga ggtacctcta ctttgattgg aaaccgggaa 900
   ccgctttggg taattgacgg aattgtacag caagacccgg taaatatatc ccccgaggag 960
   ttgaacaacc ccgactatat taatcgtatc ggtaatgcta tttccgggtt aaacccacaa 1020
   gacatcgaac gtattgatgt tctaaaagat gcctcagcaa cagccctata cggaacgaaa 1080
   gcagccaatg gagtgatcgt gatcacaacc aagcgagggc atatcggggc tccgatggtg 1140
   agctacaata tgacaggtac attcaaattc cgcccccgtt acagcgaccg acatatcgac 1200
   gtgatgacct caaaagagcg agtgcaattt tcccgggatt tattgaatag taattataat 1260
   ttctctggca acgtgagtct ggtaggctac gagtatctat atgaacaatt gtacgcccgc 1320
   caaatttcat atcaagagtt cgaagagcaa gtcgcaaaaa tggaatcttt gaacacagac 1380
   tggttctctg ctctaggtaa cgatgttttt tcctcacagc acacgataag cctaacggga 1440
   ggatcggata tgattcgcta ttacgcatct atcggttata cagacgataa cgatgtggtg 1500
   aaatcgaatc ggaacgaccg ttacacggca aaattaaatt tggatattca tttctccgag 1560
   aaattctcga cttcattggg catcgacggt agcaaaagtt cgcagaagta ttaccaaagt 1620
   gaattggctc ccatcgatta cgcctataac acaagccgca caattcctct ctacaacgag 1680
   gacggaacat tacactatta ccaaaaaccc caagctggca gatactacaa cttcaatatc 1740
   ctaaacgaat tggcaaacag cgggtgcgag caagaaggtt caagcctggc gtttcaagcc 1800
   aatttactat acaaaccact agcttggttg agggtaaacg gaattttagc ttattcggtt 1860
   tccaacacgg agatcgactc gtactggggt gctgaaacac accatgtagc aagtatgcgc 1920
   cgtggggagt tcggaggaga gattgacgca gaagaaagcg aattacccgc cgggggagaa 1980
   ttaaaacaac agagttcacg caacgaatcg gtaaccgctc gtttgcaatt ggatgccaac 2040
   aaatatttcg ggttagccga ggaacacaac atcaatgcct cgttaggttt cgaggccagc 2100
   tccacaaaat acgtgggcac agagatcacg aaccggggat attatcccga tcggggatta 2160
   cagttcgcta cattcgaaca gggagtatac ccggtcttcg acgcttggct gttggcaaat 2220
   cgtcctgtat taacagataa tctaacaaac ttaatagcgg tttacgccag tatctcctat 2280
   tcttatcgca actggatcac gctgaacgca aatgcccgtt atgacggatc gaaccgattc 2340
   ggttcgcaga gtaatgataa gttgctgccc gtgtggtcag cctccttgtc atacaatccg 2400
   ctggaacaat tccaagcaac aggattcttt gattttctcc agctgaaatt ctcttacggt 2460
   taccaaggga atatgctgga aggacagtct ccggaaatga ttatcaaaaa gttgccgttg 2520
   gatgattatt accatgagct ggtgtcggag gtttctattt acccgaaccc ggatttacaa 2580
   tgggagcgta ccagttcgtt gaatgcggga atcgagttct ctatcctaga tcgtcgctta 2640
   atggtaagct cggat 2655
<210> 325
   <211> 1311
   <212> DNA
   <213> Unknown
<220>
   <223> Unknown gut bacteria
<400> 2
<210> 326
   <211> 729
   <212> DNA
   <213> Faecalibacterium prausnitzii
<400> 326
   atgattttat cccagcgcca acttgaagaa attgcagcct caacaacgaa ggacttcaac      60
   cggttctttt tcggggatga ggcggacaag cccgaccgat cagctttgcc aacacccatt     120
   gatcagtttg caaagaacta tctcggtctt cgcgtatcat tcgcccgtct ctcgccggac     180
   ggaagcatct gcggtgtcac tgcctatgcc gacactgagt acaagatcac ggaacttggt     240
   attacgcgca cactggcttt gaagcgtaat caggtcatct tggacgagag cttcattcga     300
   tccggcaacg tgcagcggct ctgcgccaag cgcagattta cccttgccca cgagtgcgcc     360
   catcagattc tcttccaact ggaatcggaa gaggtaaagg cttcctgcga aatgaaatat     420
   tccgcacgga cagcctatac gccgcgagag ctgaaaaccc gcgaggactg gaacgagtgg     480
   caagcaaatg tcttgggcgc ggcgatcctg cttcctcaaa aagaggttga cctggcaatg     540
   cgtcggtttg cagaaacgcc gctgatcaat tacgagggga ggtattcgta tggtgatcac     600
   ttaacgctgc gccttttctg ccggttgttc ggtgtctcca agacaacggc gtctatccgc     660
   cttcgtcagc tcggctacat ggtagatcgt ccattcagtg agtatgttga cccattggag     720
   gtgtggtaa                                                             729
<210> 327
   <211> 1890
   <212> DNA
   <213> Faecalibacterium prausnitzii
<400> 327
   atggctgaaa acaaaaatgc acagcaagtc cgcgaaatca cggacaagct ggaacagggc 60
   atcaaggagc tttttgaatc cgagcggttc aaggaatatc tccgcacgat gtccaagttc 120
   tacaactatt ccttcaacaa cacgctgctc attgcgatgc agaagccgga ggcaacctat 180
   gttgccggtt atacctcgtg gcagcgcaac tttgaccgtc aggtcatgaa gggcgaaaag 240
   ggcatcaaga ttcttgcacc cgcgccgtac aaggcgcagg aagagcgtga gaagattgat 300
   cctttgacgc agaagccggt gatcggcgca gatgggaagg ctgtcacgga aacggttgag 360
   gtcctgcgtc ctgcctttaa ggtggtgagt gtctttgatg tttcccagac ggacggcaag 420
   gagcttccgg acatcatcgt cgatgagctg aaaggcaccg tcgaaaacta cgaggcgttc 480
   ttcgatgcgc tcaagcagga atctcccgtc cctatttcct ttgaggacat tccgggcggt 540
   gcaaagggat tcttctcgcc ggttgaaagc cgcattgcca ttcaggaggg catgagcgaa 600
   gtccagacgg tcaaaaccgc cattcacgag atcgcccacg caaagctcca cgctgtcaag 660
   ccggatgaga aagccgcgcc agaagataag aaggatcggc acaccaagga ggttgaagcg 720
   gaaagcgttg cctacaccgt ctgccaacgg tacggcattg aaacctcgga ctactccttc 780
   ggttacatcg ccggttggtc atccggcaag gaaaccaagg aactgaaaag ctctctggac 840
   accatccgca agacggcggc tgagatgatc gagggcattg acgccaagct caaggtgctg 900
   ctggcagaga aagcacagtc cgcagagatg gaagtcgaag ctcccgtaaa ggaagctgtt 960
   tcggaggaaa agccggaagt ccccatttac cgcgagacgg cgaattacgc ctatgaagcc 1020
   ggtgagatgg acgcatatcg tgcttctctc gccgcaaacg cggaatgccg ccgtgcgatt 1080
   gaagcggcaa tcagttctaa ctacggagac aaccggctgg atgcggatgc tgccgtgaaa 1140
   agcgtccttg agcagttctc tccggagcgc gtccggtatg tcctcgcaaa caccattcag 1200
   cagaaagact ttgacgggcg cattccgcag cctctcaagg agtgggcgaa gagcgttgat 1260
   gtctgcccgg agaatgcctc ccgcttcctt gtggataaac ccaatcccgg actgacggca 1320
   cttttcgtgg atgcgttccg tcagcagacc gaaccccaga aggaagtcac ttctgagaaa 1380
   acagaggaaa gagacccgga ggttgttgca tgggagaacg atgagattgc ctctattgag 1440
   gtaaaaaccg tggaggtcaa gtctcccttt gcgcccttgc cggaggaagc agcaaaagca 1500
   ccgaagacac accgcctgac tgccgaagaa aaggagatca aagccgccgt catggacacg 1560
   ctcaaggggc agatcgccta taacaacgac ggtatgcggg cgtcctatcg cgcctctaac 1620
   cactccttca atctgctggc acggaacggc gtcaggatcg agggcaacac ggtcacgcag 1680
   aacggtgagc cgctgttcaa aattcatcgc cgtcatgcgg cgcggaaaac acagggctgt 1740
   taccgtgagc tgatgccgac gctggaatac gtcaagcagg agcagaagca ggaaaagccc 1800
   tctatccgcg atcagctcag gactgccgca aaacagcagc cggagaagaa gtctcaggtc 1860
   aaatccaaaa cgcacgacat ggagttgtga 1890
<210> 328
   <211> 1203
   <212> DNA
   <213> Faecalibacterium prausnitzii
<400> 328
   atgcccgtat tagatggtga ttttgaagcc ttcgtcacga accttggcaa gtacaacgag 60
   ggtatgctgg tcggtgagtg ggtgaagctg cccaccaccg aggaagagat gcagaaggtc 120
   tttgagcgca ttgggatcgg caagcaggat gagttcggtc agccctatga agagtggttt 180
   atcaccgact acgaatgtcc gatctacggc gttcagaaga tgcttggcga gtacgagagc 240
   cttgataagc tcaactacct tgccgctttg attgacgagc tttccctgag cgatcaggaa 300
   aagctcgttg ccattatgga ggctggctgc gatgaggtca gcgacatcga cgatctcatc 360
   aacctgacgt tcaatctgga ctgctacgac atcatgcccg gtatcaacga cgaatccgat 420
   ctcggctatt attacgcaca cgaagccggt atctactccg aaaaggatct cggtcctctg 480
   gcaaattaca tcgactatga acgctatggg cgcgacattg cgatggatga gcagggacgc 540
   ttcaccgatg agggctatgt ccgtgtcgca agcgaacgct gggacaggca gtttaacggg 600
   gagcttgatg atattcccga tgaataccgg attaccggct caggggaagc cgccgagcgt 660
   gacagcacca tcgccgttct cgtcgttgag ccgggaaagg agccttatgt gaaggagatt 720
   gactccggtc tggagtcctt gcagcatgag gtcggcggct atatcgaggc aatttatccc 780
   tacgaagacc ctgttgcctt agtctgtaat gaggaaggca agctggaagg tctgcccctg 840
   aaccgcgctc tgcgtgatga ggatggtgac atctacgacg ttgttgccgg aacattcatg 900
   gtagttggct tgacggatga cagcttcggc tctctgaccg tagagcagat gcagaagttc 960
   tctgaccact tcaaggtgcc ggagcagttt gttaagttgg gcgataagat tgtggcaatc 1020
   cccatgatct cgaaggagca gcagaagcag gaaagcatcg agcagaagga ctttgagatg 1080
   aatgccgaca cctccggtct gacggttgcc ggtcacatcg ggacgtggca caccattgac 1140
   cagcacgaag tcagcggtca cagcttttac ctgatggagc atgacaccta cggcgatgaa 1200
   gct 1203
<210> 329
   <211> 1176
   <212> DNA
   <213> Faecalibacterium prausnitzii
<400> 329
   ggggagcttg atgatattcc cgacgaatac cggagcaccg gctcagggga agccgtcgag      60
   cgtgacaaca ctattgccgt tctcatcgtt gagccgggaa agaagcctta tgtgaaggag     120
   attgactccg gtctggagtc cttgcagcat gaggtcggcg gctatatcga ggcaatttat     180
   ccctacgaag acccggttgc cttagtctgc aacgaggaag gcaagctgga aggtttgcct     240
   ctgaaccgcg ctctgcgtga tgaggacggt gacatctacg acgttgttgc cggaacattc     300
   atggtagttg gcttgacgga tgacagcttc ggttctctga ccgtagagca gatgcagaag     360
   ttctctgacc acttcaaagt gccggagcag tttgttaagt tgggcgataa gattgttgcg     420
   atccccatga tctcgaagga gcagcagaag caggagagcg ttgagcagaa ggactttgag     480
   atgaacgccg atacctccgg tctggcggtt gccggtcaca tcgggacgtg gcacaccatt     540
   gaccaacacg aagtcggcgg tcacagcttt tatctgatgg agcatgacac ctacggcgat     600
   gaggcggctt gcatcatcgt cgatgagcgc ggtaagctcg tccttgatga tgtctacaac     660
   ggctttgacg atgacacgct ccgccttctc gaccttgagg tcaaggaagt gccggaaatg     720
   cccgatcccg cgctctccgt tcaggatatg aaggactacg gctacgcatg ggctggcgtt     780
   cttcccgccg gtcaggaggc ggctgagacg gctttggaga agggctgcga ggtttaccgt     840
   ctttattccg ataacaccga gggcttgtgc gtagatgcca aagagattgc cgatcatgcg     900
   gcaaagggcg gaatgctcgg tatcagcaag gaaagctgga tggcggctct tgagaaggaa     960
   aactacctca aggcggcgga gatgtcgatg gaggatgact acggcatgat tgatgggatc    1020
   atcaacaacg gtccgaagga ggacaagacc gcagaggtca aagcccccga aagggtcgaa    1080
   aagtcctcca tcatggacag gctcaagtcc gcaaaggctg aaaagcagaa ggaatgctgc    1140
   cctccaaaaa agcacaaagg agagattgag ctgtga                              1176
<210> 330
   <211> 498
   <212> DNA
   <213> Faecalibacterium prausnitzii
<400> 330
   accgcaatgc aggatgaagt caacaccaaa gttgttgcaa tcatgatcaa gggcggcaaa 60
   atctcagccg aggtgctgaa aaaggcactg gacaagttcg tgcaggagat tgagaaggca 120
   cagaagcaga tgcagcagcc caaaacctat cgcggcaagc agtccatcaa gcatctgatg 180
   agccagaacg ccgccatctc caacatcgaa gtgacggacg gcaacatcaa gtcctttgag 240
   cggactgcca gcaaatacgg tctggacttt gcgctcaaga aggacgtttc cgtagagccg 300
   cccagctatc ttgtcttctt caagggtcgg gacgttgatg tcatgaccgc cgccttcaag 360
   gagttctccg ccaaaaccgt caagcagaag gaacagccgt ccatccggca caagctggat 420
   caggagaaag ctcagagcaa ggcgcagcac aaggagaagg tcaaggtcaa aaccaaggat 480
   cggggtgtgg agctgtga 498
<210> 331
   <211> 399
   <212> DNA
   <213> Faecalibacterium prausnitzii
<400> 331
   gaaaggagga aacagtcaat ggcatttgtt ccggtcccga aggatcttaa ccgcgtcaaa 60
   acgaaggtca tgttcaacct gaccaagcgg cagctcattt gtttttccat cgctgcggcg 120
   gtcggcgtcc cgatcttctt tctggcgaag gtgcatctcg acttgtctac ggcggcaatg 180
   ctgatggtgg tcatcatgct cccgttcatc ttcttcgcgc tttacgagaa ggacggtcag 240
   cccgccgaaa agtatctgta ccacatcgta cagtccatgt tcatccggga caaggtgcgt 300
   ccctaccgca caaacaatct ctacgctgag attcagcaga aaatcaaaga acaggaggaa 360
   ttgcagcttg aacaacagca cagcaaaggc aaagcctaa 399
<210> 332
   <211> 2016
   <212> DNA
   <213> Faecalibacterium prausnitzii
<400> 332
   atgaccgtca agaacggcgt cgtttatggc gatgcccttt ccgctcagga aaagaagcgg      60
   atcgtcatgc agaagaaaaa ggacaggaag gcaaagaaag tccgcaagtc cgcccagcag     120
   accattccct atgtggagat gtgccgcgac ggtatctgca aggtgaacag ccgcctctac     180
   acgaagtcca tcgcctttga ggacattaac taccagcttg cgcagaacga ggacaaaact     240
   gccatctttg agaactggtg cgactttctg aactacttcg acagctcgat tttcgtccag     300
   ctctccttca tcaatcagaa ggcaagtctc aatgagttcc gcaagcgcat caacattccg     360
   gcacaggagg acgccttcaa cgacatccgc tccgagtatt ccggaatgct gcaaagccag     420
   ctcaccaagg gcaacaacgg actggtcaag aagaagtaca tcaccttcgg cattgaggca     480
   gactccctcc gcacggcaaa gccaaagctc gaacgcattg aaaccgacat tctcaacaac     540
   ttcaaaaccc tcggtgtgag aaccgagccg ctgtccggct atgaacggct gaaagtgctt     600
   catgatgtgt ttaacatgga caccaatgag ccgttccgct tttcctttga catggtagcc     660
   cggacgggac tcagtacgaa ggatttcatc gcgcccactt cctttgactt ccgtgaaggc     720
   aagtgcttca agatgggcag aaccatcggc gcgatgagct tcctgcaaat cctcgcgccg     780
   gaactcaatg accgtatgct tgccgacttc cttgagatgg acagcaacat cacggtcaat     840
   tttcatatcc ggacgattga ccaggcaaag gcaatcaaga gcatcaagat gaagatcacc 900
   gacctcgaca agatgaagat cgaagagcag aaaaaggcgg tccgctccgg ctacgatatg 960
   gacatcatcc cgtccgatct tgccaccttc ggcggtgagg caaagcgtct gttgcaggat 1020
   ctccagaccc gcaatgagag actgttcctt gtgaccatcc tcatcatgaa tacggcaacc 1080
   aaccgccaga agctcgaaaa cgcggtgttc cagaccgccg ccattgccca aaagtataac 1140
   tgtgcgctca agcgtcttga cttccagcag gaggaagggc tgatgtcctc tttgcctatc 1200
   ggcgtcaatc aggtggaaat cgaacgagga ctgaccactt ccagcacagc ggttttcgtg 1260
   ccgttcacca cgcaggagct ttttcagggc ggcgaagctc tctactacgg gctgaatgcg 1320
   ctgtccaaca acatgatcat ggttgaccgc aagcagctca aaaaccccaa cggactgatc 1380
   ttgggtacac ccggttccgg taaatccttc tccgccaaac gcgaaatgac gaacgccttc 1440
   ctcatcacgg aggatgacat catcgtctgc gaccccgaag ccgagtattt tccccttgtg 1500
   cagaagctcg gtggtcaggt catccgcatc tcaccggtca gcacggatta catcaatccg 1560
   ctggacatca acacgaacta ctccgaagag gaaaacccgc tgacgctgaa atccgacttc 1620
   atcctctcta tgtgtgagct gattgtcggc ggcaaggacg gcttgcagcc ggttgagaag 1680
   accatcattg accgcagtgt ccgcatggtc tatcaggatt ttcttgcaga ccccaaaccg 1740
   gagaaaatgc cgatcctcga agacctctac aacattctga gaaatcagaa ggagccggag 1800
   gcacagcgca tcgcaactgc ccttgaaatc tacgttcacg gctctctgaa cgtcttcaat 1860
   cacagaacga atgtggatgt caacaaccgc ttcgtctgct atgacatccg ggaactcggc 1920
   aagcagctca agaaactcgg tatgctgatt gtgcaggatc aggtgtggaa cagagtcacc 1980
   attaaccgcg cccagcacaa ggcaacgcgc tactac 2016
<210> 333
   <211> 870
   <212> DNA
   <213> Faecalibacterium prausnitzii
<400> 333
   ttgggcagca ttttagaaaa gatcgaacaa gctctcaagg atatgctgat cggatggatc 60
   gagagcaacc tgaccaatat gttcaccgat gtcaacgaga aggtaggaac gattgccgcc 120
   gaagttgggc aaacaccgtc cggctggaac ggcggcgtgt accagatgat ccggggacta 180
   tctgaaaacg tgatagtccc catcgctggt atcatcatca ccttcgtttt gtgctacgag 240
   ctgatttcta tgatcaccga gaaaaacaac cttcacgaca tggacacatg gatgttcttc 300
   aagtggtttt tcaaggcggc tgtggcgatc tatctcgtaa catacacgtt tgacatcgtg 360
   atggcagtat tcgacatcgg gcagaacgtg gtttccggtg cagcaggagt aatccacggc 420
   aaccccagca ttgaccttga cgcgacgatt gcgcagatgc gtaccggcat ggagaacatg 480
   ggcgtcggag aactgctcgg actgtcgata gaaacgcttc tgatcagcct gtgccttaaa 540
   atcatggcga tcctcattac ggtcattctc tacgggcgca tgattgagat ttactgcacc 600
   gtgagcattg cgcctattcc catcgcaacc atgagcaacc gcgaatgggg cagcatcggc 660
   acgaactatc tgaaaggctt gttcgctctg gcatttcagg gctttctcat catggtctgc 720
   gtcggcatct atgcggtgct gatcaacggc atgatcatcg cagacaacat tcattcggct 780
   ctgttctctg tggcagcgta cacggttatt ctgtgcttct cgctgttcaa gaccggaagc 840
   ctcgcaaaat ccattttcca tgcgcactaa 870
<210> 334
   <211> 2070
   <212> DNA
   <213> Faecalibacterium prausnitzii
<400> 334
   ttgatcttag tcattgctga aaagcccagt gttgcccagt ccatcgcaaa ggtgttgggc      60
   gcgacgtccc gcaaggacgg ctacatggag ggcggcaatt acatcgtttc gtggtgtttc     120
   ggtcatctgg tggagctggc agacgccagc tcctacgatg agcggtatgc caagtggcgg     180
   tatgacgatc tgcccattgt tccggaaagc tggatgtttg aggtcacgaa ggacaaagca     240
   caacagttca aggtgctgtc ctctcttatg aaggacaagc gcgtcaccga gctggtctgc     300
   gcaaccgatg cagggcgcga gggtgagctg atcttccggc tggtctacaa caaagctgga     360
   tgcaccaagc ccttcaagcg tctgtggatc agctcgttgg aggactccgc catccgcgaa     420
   ggcttcaatc atctccgaga cggcaaggaa tatgaccgtc tctatgaagc agcactcagc     480
   cgctcgaagg cggactggat cgtcggcatc aacggcaccc gccttttcac cacgctctat     540
   cacaagaagc tggtagttgg gcgtgtccag acgccgactc ttgcaatgct ggtagagcgc     600
   gacggaaaaa tctccacgtt ccagaaggag aagtatttca acgtccatgt cggcaagggc     660
   gatctgactg ccgatctgga aaaggtcaaa accgaagagg aagcaaagaa gattgcggcg     720
   gcttgcgaga aaaagcaagc cgtcgtttct tctctcaagc aggaaacgaa gacggtcaat     780
   cccccaaagc tctatgatct gaccaccttg cagcgcgagg caaaccgata ctacggcttc     840
   actgcccagc agacgctcga tctcgtacaa acactctacg aaaagaagct cctgacttat     900
   ccgcgcacgg acagtcagtt catcacgggc gatatggagg acactgcccg tcaggtcatt     960
   tctatcgtct gccgccagct tccgcttttc tccggcgttt cggttactcc ggacattgcc    1020
   cgcgtaaccg acaacagcaa ggtcacggat caccatgcca ttctcccgac cgttcagctc    1080
   gaaaagcagg atgtttccgc gctccctcag tcggaacaga aaatcctcaa tcttgtcggg    1140
   atgcgccttc tgtgtgcgac cggtgagaag cacacctacg cagaaacgca gatcacgctc    1200
   tcctgcgagg gctacgagtt caaagccaag ggcaagaccg tcgctcaaaa cggctggaaa    1260
   gccgttgaag agctgttcaa ggcatctctc aaggcgaagg aaaaggatga tcccatgaag 1320
   tccctgcccg aagtccatga gggcgatatg ctggaaggtg tgtctgccag tgtcaccgaa 1380
   cacttcacaa cgcctccgaa gcagtacacg gaagataccc tcctgtctgc aatggagact 1440
   gccggaaacg atcagtttga cgatgacacc gagaagaaag gtctcggcac tcccgcgact 1500
   cgcgccggta tcattgaaaa gctggtgaag tccggctttg cagagcgtaa aggcaaatcc 1560
   ctcattccca caaaggacgg ctgcaacctt gtctgcgtcc tgccggaaca gatcacctct 1620
   cccgcaatga cggcggaatg ggaaaacacg ctcatggaga ttgagcgcgg caaggcagat 1680
   gcagacgcct tcctcagcgg cattgtccag atgaccggcg atctcgtaaa agcctatccg 1740
   tttctctccg atgccgaagc ccagcgtttc ggcacgggca aggaggaaat cggcaaatgt 1800
   ccccgctgcg gctctccggt ctatgtcggc aagggcaact tctactgctc gaacaaggac 1860
   tgctccttct gcctgtggga agacaacaag ttcttttcca gcaagaaaaa gaagctgacc 1920
   aagaggattg caaaggagct gctggacaag ggctggtgcc gagtgaccgg gctttacacg 1980
   ccgaagaagc ctcagctcta cgatgcggtg attcgtctgg atgacagcgg cggcaaatac 2040
   gtcagcttca agatggagtt cgaccgatga 2070
<210> 335
   <211> 2205
   <212> DNA
   <213> Faecalibacterium prausnitzii
<400> 335
   atgtccaatg aaccggaact taaagcccgc gacaaggtag tcgtgcggat gacgcgggag        60
   ggcgcggtcg aggaaaacct gacggctggc accgaacagc gtgtgtcaaa gaggctggaa       120
   gatgcggagc tggtgaagcc cggtgagaca gtcgagcctt ccgaagctct ttctgcggag       180
   gaacagaaaa aggtgcagat gcgccgtcag cagcgtcagt ttcagacgga acacgccgaa       240
   gataacgaca cacagccgcc ctcggaaacg tccgtcacag aagagaaaag ggcagaaaat       300
   ccaccccaga atgtacctga accgctgccc tcggaaacgc cgttcaagca tccaacttta       360
   gagcagcacg gcgtttcttc tcataccggc acggtgattg ctgaaacggt tgtcacacac       420
   aagctgcgca agacctcggc agtggaggca gtggatgcgg atgccgttct ctcccaagcg       480
   gcggagactt cctccgcaaa gccggtctcg gacgatgcag tcccgcccac gaagcggatg       540
   cagaagctcg aaaggaagtc cgagaaggcg catgagcgtc tggacgctgc ccgtgagaac       600
   ctgcccacgc acaaggttct caagaaagag cgtgtcttcg atgaagagac cggcaagggc       660
   aaaacccgcc ttcattttga ggatgagctg aaaaagccaa agtgcaaagg caagctgcaa       720
   tttgagacgg ataaaaccgt ccgcaaggtc ggtgacactc tcgcctccgg cattcacggc       780
   aaaatccatg aggtcgagca ggaaaactcg gcggttgagg cggcgcataa aacagagatc       840
   gccgctgaga ctgccgctcg tcatttcagt catcatcggg aaagcagcgt caacaagcct 900
   tatgagaagg tctccaagct ggaacacaag gcggatgctg ctgatgcgaa gctccaatat 960
   gaaaaaaatc agcaggagca tcctgagatg aagaagcaga acatgaacaa gcactaccag 1020
   aagcagagca tcaagaagga atatgccgct gctcgaaatg ccggttctca gaccgccggg 1080
   actgcaacaa agagcaccgg caagaagctc ggtgagaagg cgtccgacaa gatcaaggag 1140
   ttctttgaga agaacaagaa ggtcttcatc tggatcggcg tcggaattgc ccttctcgtt 1200
   ttgctcggtg ccggaatcag ctcgtgttcg atgctcacct ctaccggctc gtcggttatc 1260
   gcttcctctt atctcagcga ggatgacgcg atgctgggcg cggaggcgca gtattgccgg 1320
   atggagcaag agctgcaacg ctatctcgac acctacgaaa gcactcatga ctatgacgaa 1380
   tatcacttcg atctggatga tattgagcat gacccctatg tgctgatctc cattctctcg 1440
   gctctccatg agggcgagtt cacgctggat gaggtgcagg gtacgcttca aatgctgttt 1500
   gagaagcagt acatcctcac cgaagaggtc atcgtcgaaa ccagataccg cacggagacc 1560
   gacacatgga cggacgcaga cggcaacacg cacacggaaa cctatcgcgt cccgtatgac 1620
   tactacatct gcaacgtgaa gctcgaaaac ttcaatctct cccatgtccc ggtctacatc 1680
   atgtctcagg aacagctttc catgtacgca acgtatatgt cggtgctggg caaccgtgaa 1740
   gatctgtttg gtgactctcc ctatgtggac aagtacatca caaatccgcc cgctgactac 1800
   gatgtcaacc cggaatacct gaacgacgag aagtttgcag cgctgattac cgaggcggaa 1860
   aagtatctcg gctatccgta tgtgtggggc ggctccaatc ccgacacgtc cttcgactgc 1920
   tccggctttg tcagctatgt tctcacgaac agcggtcttg tgaacaccgg acggcttggc 1980
   gcacaggggc tttacaatgt ctgtgcgccg gtctcaaagg cgaatgcaca gcccggtgat 2040
   cttatctttt tcgtcgggac gtatgacacc cccggcgtgt ctcacgtcgg catctacgtc 2100
   ggtgatgggg tcatgatcca ctgcggcgac cccattcagt acacatccat caactcttcc 2160
   tattggcagc agcatttcta cgccttcgga agacccgcct attaa 2205
<210> 336
   <211> 1029
   <212> DNA
   <213> Faecalibacterium prausnitzii
<400> 336
   atgcccattg aaatgttgtc gggtttcaca gccaacattt caatggaaac acaaatccaa 60
   cagaacagga ggtacaccac catacaactt gacatcattc ataccggcga ttgccttgaa 120
   atcctgaaaa ccctgcccga tgacagcgtt cattgctgtg tgacgtcccc tccgtattac 180
   gcgctccgcg attacggtat ggaggctcag atcggcagag agacaacgcc gaaggaatat 240
   atctcgcgcc tgacggaagt gtttaccgaa gtcaggcgcg ttttgcgtcc ggatggaacg 300
   ctctggctga acatttcgga cacctacgct gggaaaggca atcagggtga ttttattgac 360
   ccgaagaacc ccaacggcag aaacggtcag gctgtggctc tcaacaacaa ggttgagggc 420
   tgcaagccga aggacatgat cggtattccg tggatgctgg cgtttgccct ccgtgatacc 480
   ggctggtatc tgcgcaacga catcatctgg atgaaggata accccatgcc ggagagcgtg 540
   aaagaccgct gcgcccgctg ctatgagcat attttcctgt tctccaagtc caaaaagtat 600
   ttcttcgact acaaggcaat ctccgagccg attgcccccg caacggcaga acgcctcaag 660
   cgcggcatga agggcggcaa caaatacggc aagcccgttc ccggtcagcc tcagccgcag 720
   tccatcaacc gcccccgtga gcatggcgag atcaaggacg cagacatcaa tccgctccgc 780
   aataagcgcg atgtctggaa gatcaacacc gtccccttca agggcggtca ttatgccgcc 840
   taccctccga agctggttga aacctgtctt ctcgccggtt gtcccgaagg cggcattgtg 900
   cttgacccct ttatggggag cggcacaacc ggcatggttg ccgcgcagat ggggtgtcat 960
   ttcgtcggca tcgagctgaa ctctgaatac accgagcttg cctacaagcg gattggaggt 1020
   gaaatctga 1029
<210> 337
   <211> 261
   <212> DNA
   <213> Faecalibacterium prausnitzii
<400> 337
   atgccgcttc aaattgtcag aaatgacatc acaaaaatga aggtggacgc catcgtcaac 60
   gctgccaacg agtcgctgct gggtggcggc ggtgtggacg gctgcattca tcgtgccgca 120
   ggacaggaac tgctggcaga atgtgaaacg ctccacggct gcaagaccgg gagcgcaaaa 180
   atcacgaagg gctacaagct gccctgcaaa tatgtcattc acgcagtcgg tccgcgctgg 240
   tatgacggac ggcatgggga g 261
<210> 338
   <211> 225
   <212> DNA
   <213> Faecalibacterium prausnitzii
<400> 338
   atgaagagag catctattcg ggttcaggaa ccgacgccgg agctgatcga aaaaatccgc        60
   agggcaagag ttgccatttc ccagcagaag ccccgatacc tgaaatgtcc ctattgtcag       120
   cataatgcca ttgctgtcta cgaggacacg aggggtcatg tagaatccaa gtgcaagaaa       180
   tgcgggcgga tcacagtctt tgatgtgctg aacatgagaa gactg                       225
<210> 339
   <211> 555
   <212> DNA
   <213> Faecalibacterium prausnitzii
<400> 339
   caacatgaaa ggggtgaagg tcccgtgata attcgctccg aggaaatata caaaaaggcg        60
   aacagcattg tcaaaagctg tggaacaaga gataccttga agattgcccg tgagctgggc       120
   attcatctcc attttcttga caatctgaac gatctgctcg gaatgtacac ctaccgccat       180
   aaagagcggc atattcttct gaactccaac atggagtatc tgatcatgca aatggtttgc       240
   ggtcacgaga tcgggcatga tacctttcac cgtgatcttg ccaaaggaaa cgaaccgctc       300
   ccggagttcg tgctgttcga tatgcgcaca aaacacgaat atgaggcgaa tgcgtttgcc       360
   tcacacctga tcattgacga tgatgagctg attgacctga tgaagcagga ctacgatgtg       420
   gtgcagctct cggctgcaat gggaacaaac atcaacctga tgctgatcaa gctcaacgag       480
   ctgaaccgca tgggctggca gctcaacttg ccttatgtac cgcactctga cttcctgaaa       540
   aatgtcagac cggag                                                        555
<210> 340
   <211> 240
   <212> DNA
   <213> Faecalibacterium prausnitzii
<400> 340
   aaggaaagga tatggaactc tgatatgaaa aactttaccg tggaagaaat caacctgatg        60
   tgttgcttca atacgtccag ccggaagcgg ctgatcgacg atatgaagcg cgtcactctg       120
   aacgacatgg acggcgaaat cgcagagctg atgtataaga ccatccggaa gctcgaatcc       180
   atgagcgacg cggagtttga ggaactgtat atcatgccgg acggcatggt agatgactga       240
<210> 341
   <211> 1608
   <212> DNA
   <213> Faecalibacterium prausnitzii
<400> 341
   atgagcaagg aatacaacat cggcatctac atccgcctct caatggctga tgaagatacc 60
   ggctatggca gcaaggcgga aagtgacagc atcggcaacc agcgtatgct catcaatcgc 120
   tttctcgaca atcatccgga gttgtctcac tgtcagcgat ctgagtttgc ggatgacggt 180
   tataccggca cgaactttca ccgtcctcag ttcacgcaga tgatggagaa ggtcaagcgc 240
   ggggagatta acctgatctg cgtcaaagac ttttcccgct tttctcgtga ctacattgaa 300
   acgggaaact atctggaatg cacctttccg ttcatgggcg tccgctttat ttccatcaac 360
   gacggctatg acagcgacga ttacaaaggc acaacgggcg gtctggaagt ggttatgcgc 420
   agcatcatct atgcggcata cagcaaagac ctctccgtaa agaccacatc ggcaaaaatc 480
   cagatgatga agcaaggcaa gtatgtcggt ggctacgctc catacggcta cgtcctgcat 540
   ccaaccattc ggaacaaact tgccgtagac ccggaggcgg ctgatgtgat ccgtcgtatt 600
   ttccgcgagg cgctggaagg cagcaacacc tctcagatcg cccgcagcct gaatgatgac 660
   ggcatcccga caccggggca atacttcaag agcaagcatc ccgacaagaa gaagttcagt 720
   aacatgagcg agaaaatcag ttgggaaacc gtgatggtct ataacatcct caaaaacctt 780
   gtttacaccg gaacgctggt cagccgcaaa atgaagtcct gtggtgtcgg ctcaaaaaag 840
   cgtgttgtca atgagccgat catcgtagaa ggaacccatg aagctattat cagcaaggaa 900
   gactttgagc ttgctcagaa ggtcattcga ggcggaggac ggaatcccac gcgcaagcag 960
   catgactatc cgctcaaggg actcgtccgc tgcggaaact gtaaacgtgc tatgacgcgc 1020
   cggaagaaca aggctggcat tcggtacttc cagtgcatcc actcggtcaa caacggaaac 1080
   acagactgtc cggttggcag gagctttccg gaaatggata ttgagaaggt tgtcttccat 1140
   gccattgctc agtttctcgc tttggcacag aaggaagcag tccaaaaccg agaagtcggt 1200
   gatcttcgga aatctgccat caaggaatgc gctgataaaa tccggattct tcaaaagcag 1260
   aacgagcagc acaaggcgtc caagctgagg ctctacgaaa agtacgcagc cggaagtatc 1320
   acgaaggaag cgtacattca gcaaaaggcg gcaacggaca cgaaaattgc tgaaaacgat 1380
   gaagcaatcc agtgcagtca caaacggatg aaggagcttg actccgagac ctcctgttcg 1440
   gatgaaaagc tggatgctgt ctgcgatcag tacgctgact gcaaagctct gacctatgag 1500
   ctgacccacg cattcatttc tgcggtctac atttacgatt tcgacaacat agaaatcgtc 1560
   tggaagttca aggacttcct caatacatca gaaggagaag ccaaatga 1608
<210> 342
   <211> 1857
   <212> DNA
   <213> Faecalibacterium prausnitzii
<400> 342
   atgccaggca aggtttaccg cacggcgatc tactgccgtc tgtcccgtga ggatggagac      60
   aaagtagaaa gcaactccat cgccagccag agagccatct gcgaggacta tatcgcaaga     120
   catgaggatt tggagcttgt ctgtgagccg tttgtggatg acggttacag cggcgtttcc     180
   ttcaatcgtc ctcagttcaa aaagatggaa gaggcaatcc gcaagggtgc gcttgactgc     240
   attgtagtca aggatctcag ccgcttctca agaaactaca tcgacggcgg acgttacatt     300
   gagaagatat tcccgcagct cggcatacgc ttcatcgcaa tcaatgatgc gtatgacagt     360
   ctgaccggtg atccgcagtc cgactccttt gttatcccgt tcaaaaactt gattaacgat     420
   tcttactgca aggacatctc catgaaaatc cgaagcagtc tggaagtcaa gcagaagagc     480
   ggtgagttcg tcggttcgtt ccccccttac ggctacatga aatcgccgga gaacaaaaac      540
   cagctcatcg tggatgaagc ggtcagcgaa tatgtgcaga tgatcttttc catgtacaag     600
   gacggcttct ccattgggcg cattgcaaag cgtctgaacc agatgggcgt cctgtcccca     660
   atggaataca agcattccgc cggtgtaaag ttcgataccg tcttcaaaac cggcgatacc     720
   gcaaaatgga catacaaagc cgtccagcgt attctcacca acgaggttta tatcggcgtt     780
   ctggcgcaag gcaagcgcgg cactcccaac tacaaagtcc gcgttgtgaa aagcaaggat     840
   gaatccgagt gggtcaaggt agaaaatgcg catgaagctc ttgtgtccta cgaggacttc     900
   atggcagtca aggtcatgat gcagcgggat atgcgctgtt cgcccgatca ggacgaagcg     960
   catctgtttt ccggcttcct gttctgcgga gactgtcagc agccaatgat ccgcaagacc    1020
   gtcccgtcga agacgaaaaa gtatatctac tacgtctgct ccaccaataa gcacagccgg    1080
   acgtgcagcc cgcacagcat cgccgcaaaa gaggttgagg aaaaggtctt ccgtgccatt    1140
   catgaccaga tcgagcttgt catcaatctg gaacacgcgc ttgcgatgat agaacggctt    1200
   ccgtctcaga gccgtaaggc tttcaattac gaagctcaga tcgccaaaat cgaggaagag    1260
   attgagcggt accaaaagct caagctgggg ctttacgaga acttcatcgg cggcgtcatt    1320
   gataagtcgg aatactttga gttccgcaac agctacacca aaatcattga gaacaagcag    1380
   gacgcgcttc tgcgggtcaa aaaagaaatg aagcagacgg tgacaaccgg cacaaccgaa    1440
   cgaaactggg taacgctttt caagcagtat gaaaacgtcg aagagctgaa ccgccgtgtg    1500
   ctgatgtccc ttgttgaccg cattctgatt cacgaaaacc atgcaatcga aatcgtcttc    1560
   aaatacaggg acgaatacca gcagacgctt gaatacgttc tcggctatgc cgatgaactg    1620
   gatattggcg tagaagggag gggtgagaaa atggcaagaa aaagcagaaa gcaaattgca    1680
   gtcgaagagc cggttatcga atctgtctct tccgaggtct tctcaacagc catctatgcc    1740
   cgtctttccg ttgaaaacag cggcaagtct gaaaaggtgg atgtcatcgc aaatcagatt    1800
   gagatttgca agtcgtacat tgcagagcgt ccctacctga atctgataga tacctat       1857
<210> 343
   <211> 243
   <212> DNA
   <213> Faecalibacterium prausnitzii
<400> 343
   atgatgggca ttgaaacaat gaaaaacgtc agcccgaaaa cggttgaccg tagcacactc      60
   gttcagagaa gcagcatccg gcttgatcct gcggcaccgc gagaggacag gctgagggag     120
   ttcatcaagc agatcagaaa tccctactgt tatctggacg ggaagacagt ggtgaagatc     180
   agcttcgccg agacggacac gacaatggaa gattgtctgg aacactatct gagaggtctt     240
   tga                                                                   243
<210> 344
   <211> 1137
   <212> DNA
   <213> Faecalibacterium prausnitzii
<400> 344
   cagttcatca tggatgatat ggaggacact gcccgtcagg tcatttccat cgtctgccgc 60
   cagcttccgc ttttttccgg cgtttcaatc actccggaca ttgcccgcgt aaccgacaac 120
   agcaaggtca acgatcacca cgccattctc ccgaccgttc agcttgaaaa gcaggatgtt 180
   tccgcgctgc cgcagtcgga acagaaaatc ctcaatcttg tcgggatgcg ccttctgtgt 240
   gcgaccggcg agaagcacac ctacgcagaa acgcagatca cgctttcctg cgagggctat 300
   gcgttcaaaa ccaagggcaa aagcgtcgtt caaaacggat ggaaagccat cgaggagctg 360
   ttcaaggctt ccctcaagac gaaggaaaag gacgatccca tgaagtccct gcccgaagtc 420
   catgagggcg atatgctgga aggtgtgtct gccagcgtca cggaacactt cacaacgcct 480
   ccgaagcagt acacggaaga cacgctcctg tctgcaatgg agactgccgg aaacgatcag 540
   ttcgacgatg acaccgagaa gaaaggtctt ggaacacccg cgacccgcga cggtatcatt 600
   gagaagctgg tgaaatccgg ctttgcagag cgtaaaggca aatccctcat tcccacgaag 660
   gacggctgca accttgtctg tgttctgccg gaacagatca cctctcccgc aatgacagca 720
   gaatgggaaa acacgctcat ggagattgag cgcggcaatg cggatgcaga cgccttcctc 780
   agcggcattg tccggatgac cggcgacctc gtgaaagcct acccgtttct ctccgatgcc 840
   gaagcccagc gtttcggcac gggcaaggag gaaatcggca aatgtccccg ctgcggctct 900
   ccggtctacg tcggcaaggg caatttctac tgctcgaaca aggaatgctc cttctgcctg 960
   tgggaagaca acaagttctt ttccagcaag aaaaagaagc tgaccaagaa gattgcaaag 1020
   gagctcctgg acaagggctg gtgccgcgtg accgggcttt acacgccgaa gaagcctcag 1080
   ctctatgatg cggtgattcg tctggatgac agcggcggca aatacgtcag cttcaag 1137
<210> 345
   <211> 351
   <212> DNA
   <213> Faecalibacterium prausnitzii
<400> 345
   ccggagggtg ctgctgcaca cttccggcag aaggaggata tagtgggtgc tatcaaagtc 60
   ttcttgaagg aggttgtgct tccaatcgcg cttgcgttct gcctcgcgtc atttctcaag 120
   ccgatctata tgcctgacgg cgtatgtgac tacttcctta tgtggatctg cgtcggcttg 180
   ccattcggca tccggaggat gtgcctctgg ctcgttccca gcggctacgg tatctccggc 240
   tcagtcggca tcttcgcatt gaacttaatc ataggcggtc ttatcggagg acttgccttc 300
   ttcatcgggc tgctgctcgg tgtcattcat accatccgag aaatcatctg a 351
<210> 346
   <211> 978
   <212> DNA
   <213> Faecalibacterium prausnitzii
<400> 346
   gcacaggagg atgatttcaa cgacatccgc tccgagtatt ccggtatgct gcaaagccag      60
   ctcaccaagg gcaacaacgg actggtcaag aggaagtaca tcaccttcgg cattgaggca     120
   gactccctcc gcacggcaaa gccgaagctc gaacgcattg aaaccgacat cctcaacaac     180
   ttcaaaactc tcggcgtgaa aaccgagccg ctgtccggct acgaacggct gaaagtgctt     240
   catgatgtgt tcaatatgga cagcaatgag ccgttccgct tttccttcga tatggttgcc     300
   cggacggggc tttccagcaa ggacttcatt gctcccactt cctttgactt ccgtgaaggc     360
   aagtgcttca agatgggcaa aaccatcggc gcggtgagct tcctgcaaat cctcgcgccg     420
   gaactcaatg accgtatgct tgccgacttc cttgagatgg acagcaacat tacggtcaat     480
   tttcatatcc ggacgattga ccaggcgaag gcaatcaaga gcatcaagat gaagatcacc     540
   gacctcgaca agatgaagat cgaggagcag aaaaaagccg tccgctccgg ctacgacatg     600
   gacatcatcc cgtccgatct cgctaccttc ggcggcgagg cgaagcgtct gttgcaggat     660
   ctccagaccc gcaatgagag actgttcctc gtgaccatcc tcatcatgaa cacagcaacc     720
   aaccgccaga agctcgaaaa cgcggtgttc cagaccgccg ccattgccca aaagtataac     780
   tgtgcgctca agcgtcttga cttccagcag gaagaggggc tgatgtcctc tctgcctatc     840
   ggcgtcaatc aggtggagat cgaacgcgga ctgaccactt ccagcacagc ggtttttgtg     900
   ccgttcacca cgcaggagct ttttcagggc ggcgaagctc tctactacgg gctgaacgcg     960
   ctgtccaaca acatgatc                                                   978
<210> 347
   <211> 594
   <212> DNA
   <213> Faecalibacterium prausnitzii
<400> 347
   atgcgcacta aggaggtcgg cagcatgaag aagtacagca tcatctacgc cgatccccct 60
   tgggcgtatc ggacttactc caagaaggga cagggacggt cggcggaaag ccactacccg 120
   acaatgtgca ttgaagacat caaggcactt ccggtcggtg agcttgccgc taaggactgc 180
   gctctgtttc tctggatcac gttcccgtgc ctctgtgaag cactcgaagt gctgacggca 240
   tggggctttt cttataagac cgtggctttt gtatgggtga agcaaaaccg aaggaacgac 300
   gatctcttta ccggcatggg ctactggaca agggcgaatg ccgaaatctg catccttgca 360
   acaaagggac atccgaagcg agttgacgcc ggtgtgcgtc aggtcatcct cagccacatc 420
   gaagagcatt ccaaaaagcc ggatgaggcg cgggaacgca ttgttcggct catgggagac 480
   cttccccgcg tggagctttt tgcccgtcag tctcccgaag gctgggacgt ttggggcaac 540
   gaggtcgaat gcacggctca tcttcctatg gaggaaacac catgctgcgg ctaa 594
<210> 348
   <211> 612
   <212> DNA
   <213> Faecalibacterium prausnitzii
<400> 348
   atgatgtaca atgaaaatct gcacgaagaa gaacaacatc tgatccagca gattgccgag 60
   caaacagaga gaggaaaaat cgactgggaa ctgacggagt ataatccgct gtccattctg 120
   aatgaggaca agatagacaa aaacccagca gtaatctgtc agtccttctc ctttgaagcg 180
   atcatcggcg gatctcgtta cgagcttgat gtcatggaga atattgatgt gccgtccggt 240
   atgggagact acaccataac cttgacacgg gatgaaaccg aaaattatct taaaattgag 300
   gatgcgcttt cctttgactg tgaccgctat gaatgcacac cggaggaagt cgcagagcga 360
   tttgcggaca gccccattgt ccgtctctgc aacgccatca ttcctgccac gcttgggcag 420
   gaggatttag aagaggtctt cacatgggca aggtttttca acgagactgg catttctgca 480
   aagctgatga atcatccgct gaccaagctc tgtgaaaagc tgttcgacga acaccgtctg 540
   atggacttcc atcgctgcgt cttggatgtg gactatcgaa agctgctcct aaatgaactg 600
   gctcataact aa 612
<210> 349
   <211> 825
   <212> DNA
   <213> Faecalibacterium prausnitzii
<400> 349
   atggaataca acgactataa agaagccctt ttctatgccg cttccatctt taacgaacgc        60
   ttgggggcag agttcagcga ggacaatctt gtactgcgct gctttcagac ggaaaaccag       120
   caggaagtct ttgagcagtt ctgcaagcag tatttccctg accggctgga agaccgatat       180
   acagaggacg gctattttga ctttcacgcc tctgcattcg tcggcacagg agacggcgcg       240
   gacggaatcc ttctgcgcac agacatagcg cgtcatccgg cagagttgaa gcacattctt       300
   ctgcatgagc tggcgcatat cttctgcacc cgcaacgaga ttggcggaga taatttcttt       360
   gagcgatact gcatggacga taccatcagc cgcgaagagg acggaaccat taacgccggt       420
   tatgcggtct ggcgggaact gattgcggag ctgattgcat ttgagctgga tgacaactgc       480
   gatgtagttc cgcttcgacg caagaaagac ctactcagct attacgaagg agaactcctg       540
   accggcaacg gaaaaatggg cgtcagcatg attctctgtg aggcaatgac cagtgctgag       600
   ggcgaagcgt ctatgacatg ggacgctgcc aaaagcaagt ttacgcggtt caagcccttt       660
   gatgatccac tgtacaggga cttgatggaa ctggttttta cacacgttag agaatacttt       720
   atcgtgatcg accgcgactt tatctatgaa attggagttt tgtatctgag cattgccgca       780
   caagcgatga ttgtgtccct aaagaacaga tttcaggaag aatag                       825
<210> 350
   <211> 1707
   <212> DNA
   <213> Faecalibacterium prausnitzii
<400> 350
   gtggcaagaa aaagcagaaa gcaaattgca gtcgaagatc cggttatcga atctgcctct 60
   tccgaggtct tcgcaacagc catctatgcc cgtctttccg ttgaaaacag cggcaagtct 120
   gaaaaggtgg atgtcatcgc aaatcagata gagatttgca agtcctacat tgcagagcgt 180
   ccctacctga atctgataga tacctatgtg gacaacggac gaacgggtac ggtttttgat 240
   agaccggagt tcaaccgtct gatgaacgac atccgcaccg gcaggatcaa gtgccttgtg 300
   gttcgtgatc tcagccggtt cgggcgagac tacatcgagg caggaaccta tctggaacgg 360
   gtctttccgc agatcgggct tcggtttatc gccatcaagg aaaactacga caactttgat 420
   acggacagct ccggcgaaag cctcatcatc cctctgcaaa acatgatcaa caccctctac 480
   tcgaaggaca tctcccgcaa ggtttctact gcgctcaagg cgcagatgga aagcggagag 540
   ttcaagaagc gcaatctccc gtatggctac cgctgggatg aagaacactg caatatggtt 600
   ttcgatgagg aaaccgcgcc gattgtccag aagattttcc aatggaagat tgaagggctg 660
   tcccttccgg cgattgcaga ccggcttgat gccatgaacg cgcccaatcc ggagtttcag 720
   aagtatcagg tcggcgtccg cacaggcaat gctacggcaa agaagatttg gaacaaatct 780
   tcactcacta ccattctgga taatccccat tacgtcggag ataccgttct cggacggacg 840
   ctgaacgcca tctataaggg tgtcaagaat cagcatatcg accgcgagga atggattgtt 900
   tttcccaata ctcacgaggc gattatctcc cgtgaggact tccagaaggt acgagaaatg 960
   cggaacgctg ctgcaaggac aagggttgag aagatggagc gcacggagga aattcgcgct 1020
   acactggtca atctctttga agacagaatc gtctgcgcag actgtggcag gaagctctat 1080
   ttccatcgca agcgcgttga caagcgcaag gacggcgcat ggtacgcctt ctatgagtgc 1140
   agctcatccg tcaagcgcgg caatctctgt acgccacact atacgcggca ggacacactc 1200
   gaagccgatg tgcttgcggc gatccagctt caagtcaagg cagctctcaa ttacgacaag 1260
   ctgcttgcca agctgagaaa cagcgaaggt gaacgcagca tccgcgatca acaaaatgcg 1320
   ctcattacga gcctgaatct gaaactcagc ggcgtctcca agaaacgcac ccggctctac 1380
   gaggacttca cggaaggcat tctcgatgaa gaggaatacg cctttgccaa aaaagcctac 1440
   gatgagcagt atgccgacct ttcccggcgg ctggatgaag cagttcagcg gaaggtcaag 1500
   tttgccgaag caatgtccga ggacaataag tggctcacac tgatgaaatc cgtcagcggt 1560
   gcagaaaagc tctctcagga gttagttgac gagtccgtag agcttgtgaa agtccatgag 1620
   ggcggttcaa tcgagctggt catgaaatac ggcgatattt acgctctgac cgttcagagt 1680
   atcaaggaag tacaggaggc gatgtaa 1707
<210> 351
   <211> 822
   <212> DNA
   <213> Unknown
<220>
   <223> Unknown gut bacteria
<400> 351
   gtggtatact ataggcaacg cggagaaacc gaatcattcg cggaggaggg aagaaacagg      60
   atgggaaaga tcatatcctt cgtaaaccag aagggcgggg tagggaagac gacgtcggcg     120
   gtcaacgttg cggcgtgcct gggcgtactg ggatgccgca cgctgctgat cgatctggat     180
   ccgcagggga atgcgacgac gggcgtaaag atcgccaaga agaatctgaa atacacggta     240
   tgcgacctgc tgacgggcaa catcaatgcg gcggatgccg taatacagac agaatacgac     300
   aaattatggg taatgccggc aaacgggcgg ctggcaaaca cggaatttga gctgtacaac     360
   ggggaggagc cggttttcgt catgcgcaag gcgctggaat cgctcaagac cgtgtttgac     420
   tacattatag tggactgccc gccctcgctg ggaatgctga cggtcaactc gctgacggcg     480
   agtgacggcg tggtggtgcc gatgcaatgc gaattttacg cgctggaggg gttgtcccag     540
   ctgagcaaga ccatccggac ggtgcacgac aactacaaca gcaccctgaa catcaccggc     600
   attctgctga ccatgtacac aaagcgtctg ctcctgacgc agcaggtgga gcgacagatc     660
   gaaaaacact acggcggcaa gctgttttcc acgcccatca cgcggagcgt caagctatcc     720
   gaggcgcccg gatacggcgc accgttctat tattacgaca aatccggcaa gggaacaaag     780
   gaatacctga gcgttgcgaa cgagctggca tcgcgcatct ga                        822
<210> 352
   <211> 1026
   <212> DNA
   <213> Unknown
<220>
   <223> Unknown gut bacteria
<400> 352
   atgatcagca tcgtgattcc tgcgtacaat gccgcggcaa cagtcggacg ctgtcttgac      60
   tctctgctgg cacagacata ccgcgacttt gaattgattg tcgtggacga cggttcaacg     120
   gacagtacgg cacagattat ttccgcctat gcggagcgtg acagccggat ccgcttgatc     180
   aggcaggaga atgccggagt cagtgcggca cgcaatgtgg ggcttgacgc cgcacgcggc     240
   gacctcatcg gctttaccga cagcgacgat gccgtatcgc cggaatttct tgaagcactg     300
   ctgtcactgt atgcgcccgg cgtcctgccg gttgccgata tagagcggag cgacggcgac     360
   ggttcggtgc tggcttccct tccgcccgcg atcacgctgg atcccacgcg attgccggag    420
   gattacttct gcggcacgct cggacagggg attgcctttt cctcctgtaa caagctgttt    480
   tcggcgacac tcctgcggga tgcctgcctc cgttttgcgg aggagctgac tgtcggggag    540
   gatatgctgt tcgtattccg ctacctgcgc cgttgccggg aggtacgcct tacgcgccgc    600
   gccgtgtatc ggtacaacat tgcggaaaac tccgcgatgc agtcccgccg cgactatacc    660
   ggagcgtatg agactgtcct gcgcacactg cgccgaccgg acggagactt tccgccggtt    720
   gcggaggaag tgctggcaat gtgggctttg gagtcctgcg ttttcattct aaccaatcct    780
   gcggtttcgg aacagcccta cgccgcattt tccgcatggt gggaaggctt cacgcgcacc    840
   ggactgtacc gggctgccgc gcaggcgacc gacctgccgg acatccgtat cagccgtgcg    900
   cgacgcgttc tgttccggct gatgcgaggc aatcgcacgc gatccttgca caccctgctc    960
   cgtgctttcc gaaaaagccg gacgttcaaa catacacgcc ccccgcgcaa aggagatgcc   1020
   ccatga                                                              1026
<210> 353
   <211> 1449
   <212> DNA
   <213> Unknown
<220>
   <223> Unknown gut bacteria
<400> 353
   atgaaagata tttcccgcaa cctgaccatg ctctgtgact tttacgagct gaccatggca      60
   gggggctact tccgttccgg cattggcgac cggatcgcct actttgacct gttctaccgg     120
   gacaacccgg acggcggcgg atatgccgtg gtcgcaggac tggagcagat cgtggactac     180
   atccggaacc tgcatttcac cgaggaggac atcgcctttc tgcgcggcaa gggctgcttc     240
   gacgagccgt tcctcgatta cctgcgcagc tttcgtttta ccggcgatat gtgggcggtg     300
   ccggagggaa ccttcgtatt ccccggtgaa ccgctgatta cggttcgcgc tcctgcgata     360
   caggcgcagt tcattgagac ctaccttttg atgatcgtca accatgagtc gctgatcgct     420
   accaaggcgg cacgcatcac ccgcgccgca aaaggacggg cggtcagtga attcggttcc     480
   cgccgtgcgc agggggcgga cgccgccatt ctgggtgccc gcgccgcctt catcggcggg     540
   tgccttccca ccgcctgcac tctgtcggac gaggtgttcg gcgtcccggc gggcggcacc     600
   atggcgcatt cgtgggtgca gatgttcgac agcgagctgg acgccttccg cacctactgc     660
   gaaatatacc cgcacaatgc gaccctgctc atcgacacct acaacgtgct ggactccggt     720
   ctgccccatg ccatcgcggc gttcgacgaa gtactgcgcc cgcgcggcat cacgtcctgt     780
   gcaatccgca tcgactccgg cgacatcacc tacctgactc agaagatccg cgcccggctg     840
   gacgaggctg gctggcagac ctgcaaaatt gtggtttcca actccatgga tgagtacatc     900
   atccgcgagc tgatccggca gggtgcctgc atcgacgcct tcggcgtggg tgaacggctg     960
   atcactgcca agagcgatcc ggtgttcggc ggcgtctaca agctgtgcgc cgtggaagcg    1020
   ccggacggga cgattattcc caagatcaag atcagcgaaa acgtttccaa gattaccacg    1080
   ccgcacttca agaaagtcta tcgcctgcgc ggacgggata ccggcaaggc ggaggcggat    1140
   ctcatctgcc tgcatgacga gaccgtggac gattcccagc cgctggaaat attcgacccg    1200
   cagaacacgt ggaagcgcaa ggttttgaca gactttacgg cggaggaact gcttgtgccg    1260
   gtcttccggg gcggcgagct ggtatacagt ctgcccacac tgcccgagat ccgcgcccgc    1320
   tttgaggcgg agtgcgacgg gatgtgggag gaggtacgcc gcttcaccaa cccgcacaac    1380
   tactacgtgg atctgtccca gaagctctgg gatatcaagc aggtcatgct caacagccgg    1440
   acacactga                                                            1449
<210> 354
   <211> 588
   <212> DNA
   <213> Unknown
<220>
   <223> Unknown gut bacteria
<400> 354
   atgaatctta aagagctgta cctggagatc taccggagcc gcccggaaag cattctgcgg 60
   ttcatgctgg tcagtctgtt tgtctggaca ctcctcactc tgctgttcgg gagcctgcgc 120
   gcgacgcgac gcggctggat gtggttcaac cgcatccttg ccggatgcgc ggtgctgggt 180
   atcctgggta taacggtgtt ttaccggttt atcggcggcg gcggcgaggt cggcatcaat 240
   ctggtgccgt tccgcgtgcc ggaggaggcg tggcgggcgg aggaatacta ccggatgctg 300
   ctgatgaatg cattcctctt ctttccgctg gggctgtcgc tgggtaacgc gtggcgcccg 360
   gagcgccgca tcctgcgccg ctttggactt acggttctgt gcggactggc gctgagcctc 420
   ttctgcgaga cgatgcaagg tcttttcatg ctgggaacgg cggagaccga cgacctgctg 480
   atgaatacgg caggcgttgc gctgggcgcg gtcagtgcgc ttgcggcggg ggtactgtca 540
   ctgccgatcc ggtggttgcg caagagcggg gagactttcc gaaaatga 588
<210> 355
   <211> 1023
   <212> DNA
   <213> Unknown
<220>
   <223> Unknown gut bacteria
<400> 355
   atgtataatt attcacgcag aaaggaatat ttcatgattg aactgcggaa tctgaataag 60
   accttccaca cgcaggacgg ggcgctgaca gcactggatg acatcaacct gacggtgcag       120
   gacggcgaga tattcggcat catcgggttg tccggcgcag gaaagtccac gctggtgcga       180
   tgtatgaacc tgctggaacg accgacgcac ggcgaggtgc tggtggacgg gcaggatctg       240
   atgaagctct cccccgccgg cttgcgggag gtgcgccgca acatcggcat gattttccag       300
   agctacaacc tgctggagca acgcacggta ctgcgcaacg tgctgtaccc gctggagctg       360
   acgcacgagg acaggcggac ggcgcgcacc cgcgccatgc agctgctgga gctggtggga       420
   cttgccgaca gggcgtccgc ctacccggca cagctgtacg gcgggcagaa gcagcgggtg       480
   gcaatcgcgc gggcacttgc caccaacccg cactacctgc tgtgcgacga ggcaacctcc       540
   gcgctggatc cgaccacgac ggactccatt ctggcgcttc tgcaaaccat caaccgcacg       600
   ctgggcgtga ccgttgtggt catcacgcac gagatggcgg tggtggatcg catctgccac       660
   cgggtggcgg tgatagacaa cagccgcatc gtggaaatgg gcgcggtgga tgcgatattc       720
   gccaacccgc gttccgccat tgcgcgggag ctgatcctgc cgcacgtcaa ggtggcggcg       780
   accgtccggg gcggggcgca cgtccggctc gccttcaacg gggagtctac ctacaagccg       840
   ctggtgtcgg cgctgatcct ggaaaccggc gtcccggtca gcatcctgtt tgcggacacg       900
   cgtgacatcg gcggcaagac ctacgggcag atcctgctgg agcttcccgg cgaggatcgc       960
   cttgcccagc gcgtgaccgc gtggctggac gccaatttcc cgtcttacag tctggaggtg      1020
   tga                                                                    1023
<210> 356
   <211> 1200
   <212> DNA
   <213> Unknown
<220>
   <223> Unknown gut bacteria
<400> 356
   atgatattca cgaccgacag gaccggacgg cacgccttcc cggtctatct tgataacgcc 60
   gccaccagct ttcccaagcc tccctccgtc cttcgggcgg tggaggcttg cctggaacgt 120
   tacggtggca atccaggcag aggctcgcac agacttgcgc tgcgcgccgc cgaggaggta 180
   tatgcctgcc gtgtcgcggc ggcggatatg ttcgggctga gcgatccggc acgtgttatt 240
   ttcacgcaaa ataccaccgc cgcactcaat cttgccatca agggcatttt gcgacgcggc 300
   gagcgggctg tctgctcgga catggaacac aacgctgtct ttcgtcccct gttccggctg 360
   gcaggtgagg gaggaatttc ctttgacaga tttcagacct tttctgctgt cccggagcgg 420
   acagacgcga tgctcctgtc atcactggag catgcccttt cccgtccaac gcggctcgtc 480
   gtgtgcgcgc atgcctccaa catctgcccc gcaacgcttc cgcttgccgc catcggcagg 540
   ctgtgccatg cgcacggtgc gctgtttgtg gtagatgcgg cacagtcagc cggcgtactg 600
   gatatcaaca tggaggagat gcagatcgac gcgcttgctg ttcccggtca caaaggattg 660
   tacggtccca tgggggtcgg tatgctcctg ctcggcaagg gaatccgacc ggacaccttg 720
   cttgagggcg ggaatggatt ggactccctg tccgggggca tgggagacga tctgccggag 780
   cggtacgagc ctggcaccct gcctctgcct gccattgccg ggttgcgcgc gggcattgaa 840
   ttcgtgcgca gggtgacacc ggaagcaatc cgcgaaaagg aaacggcact gggcgttttt 900
   ctacgggatg cgttaacgga agtgccgggt gtacacgtgg cggtgcctca tctggccggg 960
   ggaaccgtgc tgtttcgggc agacggcgta tcctccgagg acatcgccgc ccgattggat 1020
   gctgctggct ccggcatctg tgtgcgcccc gggtttcatt gtgccgcgct ggcacaccgg 1080
   acgttgggaa ctccggaggg tggtgctgtc cgtgcttctt tcggctggtt caacacccgc 1140
   cgcgatgcgg agcaactgat ccagtcgctg cgcggtggct tacccgggcg aaacgggtaa 1200
<210> 357
   <211> 789
   <212> DNA
   <213> Unknown
<220>
   <223> Unknown gut bacteria
<400> 357
   atgaaaaagc tgtttgcggt tctgcttgca gccctggcgg cgctgacgct gttcggttgc 60
   cagaacacgg agaaggatcc cacggggagt gattctgcga ctgtcggtga gacgactgcc 120
   gataccgtga aggtgatgac ctacgctgag ttcctcgctg ccgaggcgga tagcaaggta 180
   acggttgagg cgtatgtgca ggcaaagcag tcctggtgga aggacgacaa gggcgttggc 240
   aaggctaccc tgtacacgca ggatcaggat ggcgcgtatt tcatctacga actgcctatg 300
   gaggaggatc agtacaacaa gctgaccacc ggcaccaaga ttcgcgttac cggcagcaag 360
   gcggtgtggg aaggcgaaat cgaaatcatg aacggtacgc tggataaggt gctggagggc 420
   aattatgttg ctcccgctgt cgatgtgacc gctaagctgg gcaccgatga tctggctact 480
   tacatgaacc gttttgtttc cttcaagggc atgactgtgg ctgccaagaa ggacgccaac 540
   ggcaacgatg ttgcgttcct gtacaagtgg aacggcgccg gtcaggaggg cgatgacctg 600
   tacttcgacg tgacgctgga cggcaagacc tataccttta cggttgagtc ctacctgtgc 660
   ggcaaggaca gcgaggtcta caaggcggtc aaggcgctga aggtcggtga caagatcgac 720
   atggagggct tcctgtactg gtacaacgga gccaatccgc acatcacatc cgtgaaggct 780
   gcgaagtaa 789
<210> 358
   <211> 513
   <212> DNA
   <213> Unknown
<220>
   <223> Unknown gut bacteria
<400> 358
   atgaagaagc tgattctcat ggggcgcgtg gcggctggca agactaccct gacgcaggca 60
   ctcaagggcg agcagctgca ctactgcaag acccagtata tcaattacta cgacaccatc 120
   attgacaccc ccggtgagta taccgaggtt catgcgctgg gggcggcgct tgccgtctac 180
   tcctatgagg cggacgtggt cggcatcgtc atcagcgcaa acgagccgtt ctgcgttttt 240
   cctcccggct gtgccgccat ggtcaaccgg gatgtgatcg gcattgtcac cggaatcgac 300
   aagccggatg ccaacgtgcc gcaggcaacc cgctggctgg agctgtgcgg gtgccaccgg 360
   atattctaca tcagctccta caccggtcag ggctgtcggg acatcatgga ttatctccgg 420
   gagccgggag acgcgccggc acagctgcag acggcatccc cggaggacag tgcatcccgt 480
   aatataccga aaggacagga acgccatgcg tga 513
<210> 359
   <211> 687
   <212> DNA
   <213> Unknown
<220>
   <223> Unknown gut bacteria
<400> 359
   atgacatacg tagtatccga tctgaacgga catcttgatg tattccgcga cctgctgaag      60
   aagatcagct tcagcgaaaa gcgggatgtg atgtatgtgc tgggggacat cgtggactat     120
   ggtccgcaac ccatggaact ggtgcaggat ctgagcaccc gcatcaacgt ctacccgatt     180
   gcaggcgacc gcgactacac tgccgcccgc ctgctgacgg catacgagaa aatgctgaaa     240
   accggggaga gaccggcgga ttttgcggag gagatggcag actggatccg cgacggcggt     300
   cgcaccacta tggaggagta ccgccagctg gatgatgacg accgcgaggg cgtgctggat     360
   tatctgacgg atatgccgct gtatgaagaa gtgacggttg gcgggcggga ctacctgctg     420
   ctgcatcgcg gcatatacga ttttacgccc aatctggatc tggacgaact ggcaccggag     480
   gacttcttct ccgagtcgcc ggatcctgcc gacacgtatt ttacggacag aatcacggtc     540
   gtggggcata tgcccaccgc cgagacgcaa aataccgagc cgcgcattta ccgcgggaac     600
   ggcattattt tcattaactg cggactggcg cagggcggca gactcggctg cctgcggttg     660
   gaggacggag cagaattcta tgtctga                                         687
<210> 360
   <211> 1821
   <212> DNA
   <213> Unknown
<220>
   <223> Unknown gut bacteria
<400> 360
   atgatccggc attccacaga cagacagttt tctctcggcg ccggctgcgt ccggctggac 60
   ggtccggcgg acagcaagat ccgtcttgtg tgcgagggca cgctcaaaag gattgatatg 120
   cgggcgttgg cggattattt ccgcaacacc gccgaccagt tcgccaccgg cgagttctgg 180
   ggcaagctga tgcgtgccgc gtgcatgctg tgcgcctaca ccggcgatgc tgacctgcgg 240
   cgcatcgtgg acgattctgc cgctgacatg atgggcattc agcgaccgga cggatgtctc 300
   tccaccgttc cggatgcact ccagccgcag ggaacgcacg gctccgacct gtgggagcgc 360
   aagtatgcac tgatggggtt gctctcctac tatgagctgt ccggctctgc ggcggcgctg 420
   gatgcctgcg tccggctggt gcgctacacc aatacgcagg tcggtgaccc gccccggaca 480
   cccatcaccg aaacgggatg ggcattctgc ggcattgaat cctcctccat cctggagcct 540
   gtcatgcggg tataccacgc gaccggggaa ccggcggcgc tggagctggc tacgcacatc 600
   gtccggagcg gctgctgcgg gcgcgagaat atttttgagg caatccgggc aggaaaatcg 660
   ccctaccgga tcggggacaa cgggaatccg cgcgagagta tcgcaaaggc atacgagatg 720
   atgtcctgct ttgaaggtct gtgcgaattt taccgggcga cgcgtgatgc gcgcgacctg 780
   gaaacggtca ggctgttctg ggacaaggtc agggaggagg agatcacact gctcggctcc 840
   ggcggggcgg atgcgccctt caatctcggt cccggaaccg gggaacagtg gaatcggacc 900
   cgtctggagc agaccaaccc tgccattgat ttgatgatgg aaacctgcgt gacggtgtac 960
   tggatgcggc tgtgccacca gctcctgcgg ctgacggggg aagtccggta cgcggacgcc 1020
   atggaggtca gtctgtataa cgccattttc ggtgcgctcc gcccggacgg cgccttcttt 1080
   gaatacttcc cgcgcttcaa cggagcacgg aacccgcgcg tcaattattc cttcaacatc 1140
   aaaggctttg acctgtcctg ctgtaccgcc aacggtccga ccgggctggc gttggcacct 1200
   gatatcgcct ttgcgggcac gccggacggc gttgccgtca acctgtacat gacgggatgc 1260
   gcccgcgttc cggttccggg cgggcaggtg gtgctgtcca tgcagtccga gtttccgcgc 1320
   gtcgggtacg cccggctgac aatcgaagca ggcgacctgc ccgccgggac gccgcttgcg 1380
   ctggatctgc gcgtgcccgg ctatgcggag catttcgccg tcctgccggg cggcaatccc 1440
   acacggatgc tgaccggcgt gccgggcaca tacctgcgca tagccaatac cgtgtccgac 1500
   ggggatgagt tcattatttc gctggactac tccccgatcc ggcacgccgc cccgcacggc 1560
   agtgaccgcg cgggtgacgg attcgttgcc tttacctacg gtcccattct gctggcgcgc 1620
   gacagccgcg attgtgccga tccgcttgcg ccggttccgg atgccgggat cggggactgg 1680
   cgcgtggagc tgccggagcg tccggggctt ttgaccgcgc acctgcaggt cggcggcgaa 1740
   acgctcacgt tggtggacta tatgtccgcc ggctccgctt gggacgacgg tagttttgca 1800
   agctggctgc cgatacggta a 1821
<210> 361
   <211> 1413
   <212> DNA
   <213> Unknown
<220>
   <223> Unknown gut bacteria
<400> 361
   ttgtatgata tactatatgt gatcgtgagc ccctaccgcc gcaacagtct tgtcagaaag 60
   agagaggagg cactcatgtc ccggtttatc agaaagcacc cctatcagaa ggttcctgca 120
   atcccgccat tcttgcgcaa tcacctctgg caaccgctca aggcattgct tgcctcctta 180
   gagctgtggc ttctgatccc cattacgctg cacagcccgg acggtgtccg ctccatgttc 240
   acagtggcgt acggcacctg ggcagacgga atgatccttg ccgtccagct ggtttttctg 300
   tatattctga ctcacttttt tgattcggtt gacgacatca ccttcgtgga ctactgcaag 360
   tcgccggttc ccctcctgcg gcgctactcg tggtggctgt cggtgggact gtgcacggtc 420
   ggatgcgccc cgtcgctggc ggtggggatc aatctggttc tgcggaacac ggcggcggcg 480
   aaggcggcgg caccgctcta cacgctgttt gtatacctgc ttgccgggct ggtcgtatgc 540
   ggcatccggc tgttccagat atggctgctg catcggaagt ggagccgtca gcggctggtg 600
   gaggacggct ccacctattg gccgagactg caaacccgca tctttcaggc aatcgtcctg 660
   cctctttcgc tgttcctgtt cgtgcggctg cttctgaaga caggctacct gtacattatc 720
   ccgaatctca tctgggcagt gctcgtgctg gtcaggcggg agcgtgccgc actgccgatc 780
   ctgctggcgg cagtcagctt gtgcctgctg attgtaggca tacgtctgct ctgtgcgtgg 840
   cgcaagcgga aacagctcct cgatcagttg gaagagctgg cacacagcgg acttctgagc 900
   tatcgcctcg tggggcaccc cttcctgtcg cttttgtcgc agcggttcct gttcgggctg 960
   ttcgtcgtag acacacgcgg aaaccgctgg cgcgtcggca ttgcctcctg ccggcacagg 1020
   tcgcggccgg cggtcgcctg tgaggggcat ctgctccggt tccgacacac gttccggttc 1080
   cggatggcgc ccatcatgaa cgcctacgga gcctatatgc atcagggcga gcagatgggc 1140
   gtccggtaca ccaactaccc cgtgcttttc cctgtcggca ctttcccgga tgacctgccc 1200
   gctccagcca aacagcgccg gggcaccggc agacggcagt atgagccgac cgaatccgaa 1260
   atcatgctgc tgtcaccgcc gcccgcacag ctatatgtca gggcgccgca aggcttggca 1320
   gagctggaca acggcagcga ggtatacggc tatcaggtct ggagcggcgg tgcgttcttc 1380
   cgctttctgg agcgtatgta caccggggac tga 1413
<210> 362
   <211> 720
   <212> DNA
   <213> Unknown
<220>
   <223> Unknown gut bacteria
<400> 362
   atgggcttcg gattgttggt ggcggggtac tttaccctga ccttcctgtc gttcgcggtg 60
   ggggattacg ctttcgcttt ctatgcagtc ggcggcgctg tgagctttat agccgccgga 120
   aagctgtacg agtacaagca ccgtttcggt ataaccgtgg caacctcgct cgggtggatc 180
   gcactgggcg tgtacggtgc tgttgcgttt ctcggcgagg tgttcctgtg ggagctgcct 240
   ttgccgggga tggacgcgac cgttccgaac cttgtgctgt tcctccttca gctggtgcat 300
   acggcgggac tgctgtccgc gacggactgc cttgcgcgcc aggttggcgc ggagaaagtg 360
   aagagtgcca tcccgtggga cgccgggata ctggggacgt gggcggtcgg acaggtcatc 420
   ctgcttctgt ttccggcggt cgccgccttt gagggtcaga ccgtgaccaa aatcgtcctg 480
   ctgatccagc ttatcggcta tctcatggtc tgcctgacgt tgctgcgtgc ctaccgcctg 540
   atctgtccgg caggtgagga ggaaaacgcg ccgcgcaagc gctcccgctt tgctctgatc 600
   aaccgcatca acgacaagtt ggaggagcgt tccaagcggg cattggagga aagcatggag 660
   taccgccgtc agcgggagga atcccgccgc acgcgccgga acggaaaaag aaaaaagtag 720
<210> 363
   <211> 1839
   <212> DNA
   <213> Unknown
<220>
   <223> Unknown gut bacteria
<400> 363
   ttggagtata atagtgaact gcttaacgat tttgagaatg gcaccgccta ccgtgcgcgg 60
   tgcggaaagg aatcactcat gttttttcgg cttctgagaa gcgtccggga atacaagcgt 120
   cctgccatcc tgacactgat cctgatggtg ggcgaggcgg tcattgaaac ggctctccct 180
   tacattaccg ccacttacct cataaaccgg attcaggaaa agggtgcgga gctgagcctg 240
   acaggagaca ttcttcccgt cgggcttctc atggtggggc ttgcactggt ttcgcttatc 300
   tgtgcggggc tgggcggctt tacctgcgcg cgtgcctcct ccggctttgc caagaatctg 360
   cggcacgaca tttttcaaaa ggtgcagacc tttacgtttg ccaacattga caaattctcc 420
   tcctcctcgc tggtcacgcg gatgaccacc gatgtcgcca atgtgcagat ggctttcatg 480
   atgctgatcc gcacggcggt gcgcagtccg ctgatgctga ttttttcggt tgtcatggcg 540
   actgtcatgg gcggttggct ggctttgaca tttgctgttg tcattccgtt gctggcgctg 600
   ggattcctgc tcatcagccg caaggcgatg cccgccttcc accgtgtgtt ccggaagtac        660
   gacgccctga acgaatccat tgaggaaaat gtccgcggga tgcgcgtggt caagggcttc        720
   tcccgcgaag agtacgagaa gaaaaagttc gcgggcgcgg cggatgccat taccgaggac        780
   tttaccaaag cggagcgtat cgtggcgttc aataccccgc tgatgaactt ctgcatgtac        840
   ttcaacatgg tgttcatcct gctgatcggt tcgcttctgg caatcaagca tcccgaaaca        900
   ggcatcaaca tcgggcagat atcggcactc ctgaactacg ggatggcagt gctgatgtcc        960
   ctgatgatgc tctccatgat ttacgtcatg ctcaccatat ccgccgagtc catgcgccgc       1020
   atcgtggagg tgctggatga ggtgccggat atgaaagcgg cggctgaccc ggttgataaa       1080
   atcccggacg gatcggtcga tttcgagaac gtcagcttca agtattccgc gtcggctgaa       1140
   gcatatgccc tggcggacgt gaacctgcac attccgtccg gcgcgacagt cggaattatc       1200
   ggcggcaccg gatcctccaa gtccacgctg gttcagctta ttccgcgtct gtatgatgtg       1260
   agcgaaggga caatccgcgt aggcggcgtg gatgtgcggg actacgatct gaacgtcctg       1320
   cgcgacggcg tagccatggt tctgcagaag aacctgctgt ttgcgggaac catccgggaa       1380
   aacctgcgct ggggtaatcc ggatgcgacc gatgcagagc ttgaggaagc gtgccgcctg       1440
   gcgcaggcgg atgagtttgt ccgagccttc ccggacgggt atgacacgca tatcgagcag       1500
   ggcggcacca acgtgtccgg tggtcagaag caacgactgt gcatcgcgcg ggcgttgctc       1560
   cgcaagccca aaatactcat tctggacgac tccacctcgg ctgtggatac ccggacggac       1620
   gccatgatac gggcgggttt tgcctcctac attccggcga cgaccaagat catcattgcc       1680
   cagcggatcg catcggtcga gaatgccgac atgatccttg tcatggacaa cgggcgcgtg       1740
   gtggatatcg gcaggcacgc cgaactgctg gagcgcagtc cgatatacag ggaagtttac       1800
   gagcagcagt cgagaggagg tgagcaggat gaagcataa                              1839
<210> 364
   <211> 1986
   <212> DNA
   <213> Unknown
<220>
   <223> Unknown gut bacteria
<400> 364
   atgacacgcg ccgcgcgaag caacgggcgg tcccccgcgc cgtactcgct cgccgccaaa      60
   acagccgccg ttctcctgct gaccgtgacg gctttcctta ccgccgtctc cggcgtgggc    120
   atcgggctgt cggctgtgta tggcatttac gacggcgtat cgccggagca gctgctcgcc    180
   gaccttaccg aaagctacct tgaccgcgcc atgcatgacg gaacgaacta ctacctgtcc    240
   gatccggacg gaatggacag ctggatacgc gcatggagcg gactgtacta ccaggtcaag    300
   gacggggacg gaaacgtgct ttcgggcaac tatcagggcg agacgggact gtcccatctg 360
   gaatggagac ttacggtgcc ggattcgaac aaccctgccg tgaccaaaga cgttaccctg 420
   cacctctggg cagattccgg cgccatgcac acatggtatc gcggaatgta ccggagcctg 480
   ctcttcctga gccgctaccg cgttacgaac gtagcggtgt gccttgccgg ccttgccgcc 540
   tccatatgcc tcctggtctt tctgctgagc gcggcaggca ggcatccggg cgaggacggc 600
   gtgcgcgagg gtgcgataga tcgcgtcccc tttgatctgt tcaccttttt cgtcggtctg 660
   ctggaggcgg tgatgctgct gctgttgctg accgcactgc cgacggtcgg cggcagtatg 720
   ttctcggcat ccgcagtgct gattattgcc gtttacatcc ctgccatgct gctactcatc 780
   gggtatgcca tgagtctggc ggtacgcatc aaagcgggaa cggtattccg gcatacgctt 840
   gtctggatat gcctgcgggc tgtcgggcaa tcgcttcgct ggctgttccg ccgcacgcgc 900
   gccgccttgc aggcaatccc ccctgtccgg cgggcgttcg tactcctgct cgtcctgctg 960
   gcactcaatc tgtcggtggg ctatgcgctg atggtaggcg ggtacgccgg gatctttctt 1020
   ctgctggttc ttctggagct ggtggtgctg ggcgcgtgcg cgctccgcta cgcgctggga 1080
   ctgcgccgtc tgcaggacgg tgccgcccat ctggcaaagg gagagtttga gtaccgcgtg 1140
   agcaccgccg gactgccgga tgactgccgg gtctcggcag agaacctcaa ccgcatcggg 1200
   caggggcttg ccgacgcggt cgagcagcgc atgaaaagcg agcatttccg caccgagctt 1260
   atcaccaacg tgtcccacga tatcaaaacc ccgctgacct ccatcgtgaa ctacgtggag 1320
   cttatccggc aactctcccc cacggacgaa accctgtgta cctacattga tgtgctggac 1380
   aggcagagca cccggctcaa aaagctgatt gatgacctgc tggaggcatc caaggcgtcc 1440
   tccggcgtgc tgaatgtgat tcctgccccg tgtgaggtgg gcgtattgct ggagcaggtg 1500
   cagggggagt acgccgaacg tctgcacgcg cacgacctga atctcatcgt gcagacaccg 1560
   gacaagacgt tgaccgtgct ggcagacggc aggcacctcg ggcgcatttt tgacaacctg 1620
   atgaacaaca tcatcaagta tgcccagccg ggcacccgcg tgtacctcga cgcccgggca 1680
   gggctggatc ctgccgctcc gggcgggagg cgaccggtcg cggtcattac cttccgcaac 1740
   gtatccgccg agccgctcaa catccccgcc gaggaactgc tggagcggtt cgtccggggg 1800
   gacgcctccc gacacacgga aggctccggg ctggggcttg ccattgtgca aagcctgacc 1860
   gagctgcagg gcgggagcct gcagcttgcc atcgacggcg acctgttcaa ggtgcgcctt 1920
   accttccccc tgtacacgcc ggcggatgcc gcccaggcgg cttcatccgg gaaggaaacc 1980
   ccgtaa 1986
<210> 365
   <211> 924
   <212> DNA
   <213> Unknown
<220>
   <223> Unknown gut bacteria
<400> 365
   atggaaaacc ggaaacggga aaaaggcgcg ccttttattg agatcaagga tctttcctat      60
   gcctaccccg gtgaggaggg acgcggacag caggtactgt cacacatcaa tctgacgatc     120
   cgggaaggcg agtacaccgt gattctcggg cataacggat cgggtaaatc cacgcttgcc     180
   aagctcatca acctggtcat cgacgattac acgtatgcgg aagggcagat actggtcggc     240
   gggtgggacg ttatggcgga cgacctgacc gaggaagacc tgctggagct gcgcagacag     300
   gttggcatgg tgttccagaa cccggacaat cagctggtcg cgaccattgt cgaggaggac     360
   gttgctttcg gaccggagaa tctcggcata cccaatccgg agttgcggga gcgggtggat     420
   gccgcgctgg cgacggtcgg tatgagcgag tatgcgcacc atgagccgca ccggctgtcc     480
   ggcgggcaga aacagcgcgt tgccatcgcg ggcattatcg ccatgaaacc gcgctgtatg     540
   atcttcgatg agtcaaccgc catgctggat ccgttggggc ggcgcgaagt gctcgccacg     600
   atggaaaagc tcaaccgtga ggaggggata actgtgctgt ccatcacgca ctatatggat     660
   gaggcggcgc gggcggatcg cgttatcgtg ctgtcggacg ggcagattct gctggatggc     720
   acgccgggcg aggtgtttgc acatgcggac acgttgcgga cggcaggatt ggatgtgccg     780
   caatgtaccg ccgccgtgca gtatctgcag gcgcgcggcg tgcgcatccg gggggatctg     840
   aacacaccgg agcagtgcgc cgccgcactg ctggatgcat ggcgtacgcg cggaggcgca     900
   ggcaggacac aggcaggaaa gtaa                                            924
<210> 366
   <211> 1284
   <212> DNA
   <213> Unknown
<220>
   <223> Unknown gut bacteria
<400> 366
   atgaagatca tggatattca aaaactgctt gggcagaatc gggaaatcag cggattccgg      60
   atcgtatcga ccaagaccga ctcctacgag ctgttctttg tgcaccggag gctggaaacg     120
   gttcgtgcta ccgacacgga caccgtcagc gtaaccgttt atgtcaatca cgacggcaag     180
   gtcggcgatt cgaccttctc ggtttacggt tccatgggtg aagcggaagt cgcggatcga     240
   atcgccgctg ccgccgagcg tgcccgtttg gtgttcaacg agccttacga gctgccggag     300
   ggcggcgtgc ttgaggcgga gttgccctcc aacctccggg acgccgagcc gcgtgcgctt     360
   gctgccggga ttgcggacgc tgtgttcgcc gccgactgcg tggagggtgg ttccatcaat     420
   gcaaccgaaa tctttctgta ccgcgacacg gtacgggtcg tcaacagtcg cggcgttgac     480
   aagacgcagg tcaaataccg cgccatgatc gaagcaatcc cgactttcac agagggggag     540
   gattccgtcg agctgtacga agcgtaccgc tttaccgatt ttgacccggc tcgcgtcacg     600
   tcggagattg ccgaaaaaat gcgggaggtt tccctgcgtc gccgggcgca aaagcctgcc     660
   acgcccatga ccgcccgcgt cgtactacgc agcaaggaaa tcggcgagct gatgtccgag     720
   ctggcaggcg acctgaatta tgccaacgtg tacgcgcaag gtaatcttca caaaaaaggg     780
   gaagccttac agggggatac gccctccggc gacccactca cgctgacgct ttgcgggcag      840
   gtacgcggta gcggaaactc tgccttcttt gacggagacg gcgtcgcgct gaccgataca     900
   acggttattc ggaacggaat cgtttctgcg tactacggca cgcaccggtt tgcctcctac     960
   ctcggcgaac cggaaaccgg cgacctgccc tgcctgcggc tggcgcccgg cagtctgacc    1020
   gcggacgagc tgaacgcaga gccatggatc gagtgcgttt ccctgtccgg attgcaggtc    1080
   gatctgtaca acgattacat cggcggcgag atacgactgg catatctcca ccgggacggc    1140
   gaatccctcc cgctgaccgg catcaccatg agtgccaaac tgtctgacgt gctgggtagc    1200
   ctgcgcctgt ccgatacgac atctgtcaac gacaactacg aaggtcccga cagaatgctc    1260
   ctgcgcggca tggcaattct gtaa                                           1284
<210> 367
   <211> 663
   <212> DNA
   <213> Unknown
<220>
   <223> Unknown gut bacteria
<400> 367
   gtgcagctgt ttccccaaag ccccatactg aaaggagtct atcccatgcc cgagatcaat      60
   ccgcaggaac tgaaggtggc agagaaccgc cgccgcttca ccgagattta ccggagcacc     120
   gtcacgcgcg agggcgccga ccggctgttc gcctacctgg agagcacgga tttcttttcc     180
   gcgcccgcct caacccgcta ccacggcaac tacgagggcg gactgtgcca gcacagcctg     240
   aacgtgtatg actgcctctg cgacattctg gcgcgtcccc gtatgcagga ggtgtacggc     300
   atccgctact ccgacgagag catcgccatt gccgcgctcc tgcacgacct gtgcaaggtc     360
   aacttttaca agaccagctt ccgcaacgcc aagaacgagc agggcaagtg ggagcaggtg     420
   ccgtactaca ccattgagga caacctgccc tacgggcacg gggagaaatc ggtgtacatc     480
   atttccgcct atatgcgcct gacgcgcgac gaggcgttcg ccatccgcta ccacatgggc     540
   ttctccgcca acgatgaccc cggcaacgtg ggcaaggcgc tggagatgtt cccgctggcg     600
   tatgcgctgt gcgccgcgga tatggaggcg gcgtacttct gcgagggatc gccgaaaaag     660
   taa                                                                   663
<210> 368
   <211> 1053
   <212> DNA
   <213> Unknown
<220>
   <223> Unknown gut bacteria
<400> 368
   atggcgatgg acggaaccgc aggaacagac ggcgggattg ccggagttgc ggagtggctg 60
   acgcagcggg aggaacagct gctcggtccc tacgccgcgc gcagcgcgga ttcacgcgga 120
   cgggcgcgtc cgcaggcacc gtccccgatg aggacggagt atcagcgcga ccgggaccgt 180
   atcattcact gtcaggcgtt ccggcgtctt atgaacaaaa cgcaggtgtt ccttgctccg 240
   gcaggcgacc attaccgtac ccggctgacg catacgctgg aggtttcgca gattgcgcgt 300
   accatggcac gtgccctgcg gctcaatgag gatctgaccg aggcggcggc gctggggcac 360
   gatctgggac atactccctt cgggcacgcg ggcgaggcgg cactgcgtgc gtgctacgac 420
   agccgctttg cccattacga gcagtcgctg cgcgtggtgg acagactgga aaacgacgga 480
   cgcgggctga atctgacctt tgaagtgcgg gatgccattg tcaaccacac cggtgagcac 540
   atggcagcca cgctggaggg cgttctcatc aagtatgcgg accgcatcgc atacatcaac 600
   cacgacatag acgatgcctg ccgcgccgga ttacttgacc ccggcgatat acccgccgat     660
   atctgccggg tgctggggga cagccacagc agccgcatca ataccatggt gtcctcggtc     720
   gtgcgggcaa gtatggatcg ggacagcatt tccatggagc cggaggtgca ggaggctact 780
   atgcagctcc gggaattcct gtttgagcag gtctatctca acccgagggc aaagggacag 840
   gaaagcaagg cgcaggagct ggtcaagacg ctgttcggct actatgcgac gcatcccgac 900
   cggatgccgg aactgtaccg ccgccttgct gccgaggggg aaccggtgga gcgatgcgtg 960
   tgcgatttca tttccggcat gaccgatcgg tacgcgattg agacatacga ggagctgttt 1020
   gttccccgcg tctggagagg actgcacgaa tga 1053
<210> 369
   <211> 1224
   <212> DNA
   <213> Unknown
<220>
   <223> Unknown gut bacteria
<400> 369
   atgcagaatc gggaggaatc tggcatgaaa aaggttagac tcggtatcat aggcatgggt 60
   aacatgggaa caggacatct ggatcacacg ctgacgggac gtacgcccga aattgaggtt 120
   acggctgtgg cggataccaa tcctgcccgt ctggaggcaa cgagggcgtt gctgaacaag 180
   cggcgggcgg attatcccga cctgcagcag ccggatatcg cgctgttcgg aacggcggag 240
   gagatgctga actccgggct gattgatgcg gtggagattg ccgttccgca ctacgatcac     300
   ccccggtacg cgattgaggc gatgcgcgcg ggcattcacg tcatgtgcga gaagcctgcg     360
   ggtgtataca ccctgcaggt gcgggagatg atggaggaag cggacaggca tcccgacatt     420
   gtgttcggta tgatgttcaa tcagcggaca aatcacgtct accgcaaaat gaaggagatc     480
   atggattccg gcgcgctggg gccgatccgg cgcaccagct ggattattac caactggtac     540
   cggaaccagg cgtactatga ttcgggtgcg tggcgtgcca cgtggtcggg cgagggtggc     600
   ggcgttctgc tcaatcagtg cccgcataac ctggacctgt ggcagtggat ttgcggtatg     660
   ccgcgccttg tcgatgtccg gatgcacttt ggcaaatggc acgacatcga ggtggaggat     720
   gacgtgacag cgtacgtgga gtacccgaac ggcgcgaccg gaacctttat tacctcgacc     780
   ggggatacgc ccggcacgaa ccgttttgaa atcgtgtgcg atggcggcac actgaccacg     840
   gacggcggca cactgcattt gaccaagttg gaggtgccgg agtcggtgtt cacgcgggag     900
   aataaaacgc cctttgccag cccgaaagcg gcttcctgca acgtggaaac ggacggcaaa     960
   aacgagcaac acgtcggcgt tatgaacgcc tttgccgggg ctattcttcg cggagagcct    1020
   ctggtggcag acggcaggga aggcatccgg ggactgacta tttccaatgc catgcatctg    1080
   tccgcgtgga cgggcaagcc ggttgaactg ccgctggatg agcatctgtt cctcaatctc    1140
   cttctggagc acgtcaagac gtcccgccgt aaagagggca ttgagtcggt cttcgcgtct    1200
   accgagggaa cctacaacgc gtaa                                            1224
<210> 370
   <211> 789
   <212> DNA
   <213> Unknown
<220>
   <223> Unknown gut bacteria
<400> 370
   atgaaacgca ttctttctgt tgccctgatt ctgctgcttt ccgcctcaac gctggcatcc        60
   tgtaacccgg ataccggctc ggaatccggc acgactgccg taaccgatgc gaccggcgga       120
   tccgtgacgg agaccgcgac cgaaacgcag accgaaatgc ccaccgagac cgtcacggag       180
   cccgggacgg acgcgcccac ggagcctccg accgaggctt ccgccgagcc gtccgatacc       240
   gaggcgcccg acccgctcga gacgcccgtg cgcgtcggaa ccggctatga cggcaagaca       300
   gcctgctatg acggcagact gcgccatatg tatgagtcct tctcgaccag ctacggcgga       360
   acctttgatt cggtgcagga agcgatggac agccttgccg gaatgggcgg cgctatcagt       420
   gcggctttta acggtgacat cgggctttgc ctgtctgtca aggttccgga tgacggcaag       480
   aagtacatcc tgtactacaa cttcaacaac tgcgatttcg tgttcaactt cggctgtacg       540
   tttgacgacg ggaacggcgg ctatgcgtac tactcctcac tcaatattct ggacgatatg       600
   gcagaggatc tggacggctg tcatgtgtac gtctggagcg aggaggaagg tctggagccc       660
   ggttactacc tcatgacatg ggtcggaaac gggaagggaa ccgtcggtcg caactaccgc       720
   tacgataagg agggcgacct tgccgagacg tgggagggtc tgcccaaagg cgagtcatac       780
   tttgactga                                                               789
<210> 371
   <211> 801
   <212> DNA
   <213> Unknown
<220>
   <223> Unknown gut bacteria
<400> 371
   atgacgcaac gtaatacgac tacagaaacc tccgatctgt cggttcatgc cggacaccgc 60
   cagcgcctgc gggagcggtt cctgcgcggc ggacccgatg acctcgccgc acacgaattg 120
   ctggagcttc tgctggggta cgccatgccg cgcgtggatg tcaacccatt ggcacaccgc 180
   ctgatcgagc ggttcggctc actgtccggc gttacggaag catccgtgga agagctgatg 240
   tcggtttccg gcgtcggcga atataccgcg tgcctgatac gcctgctgcc tgccgttgtg 300
   cgccggtgtg ccatcgaccg ggtggagtcg ccccgcgtat acgacacggt gggaaagctg 360
   gtcgaatttc tgcgcccccg ctttaccgga ctgacgaccg agcgcgtcta tctcatcctg 420
   ctggacaacg ggatgcacat gatcgactgc cgcgtcatct ccgagggctc cgtcaacagt 480
   tccgccgtca ccgtccgccg gattgccgag ttgtgcctgt atatgcacgc cgcctgcgcc 540
   gtgctggcgc acaatcatcc caaagggatc gcggttccgt cgagtgcaga cctggacgtg 600
   acgcgccaga ttgaaagcgc gctggagctt atcggcgttc cgttgctgga gcatctcatc 660
   atcacggagt tttcggaagc gccgatcctc cgtccccggc ggggcatgac acgcctctcg 720
   ccgacatccg gcaaggttga cgccgccttt taccgctttt tctacggcgt cccggaagag 780
   gaaggagagc agaagccgtg a 801
<210> 372
   <211> 579
   <212> DNA
   <213> Unknown
<220>
   <223> Unknown gut bacteria
<400> 372
   atgaatcggg aggtattctt catgaaaaaa gctgtcctgc ttggtgactc catacgcctg 60
   ctggggtacg gtacgcgcgt gcctgctctg cttgctccgg atattgccgt ttggcagtcc 120
   gaggataacg ggcgtttcac atcctataca ctgcgtacct gctttgatta taaggcggag 180
   cttgaaacgg cagatgttgt ccattggaac aatggactgt gggatatgtg tgacctgttc 240
   ggagacggac cgtttacgcc gctccccctt tataccgatg cattggtgcg cattgcccgc 300
   atcctgcaag gctactgccc gcgcgtcatt tttgccacca ccacgccccc gcgacctgaa 360
   atgtgggggc atgatataga acgtgtccgg gcgtacaacg ccgccgccgt cgcagtcctg 420
   cacccgctgg gggtacagat caatgacctg tacagtctca tagaacctga tatgcagcac 480
   ctgatccgcg acgatgatct catccatctg acggatgcgg gcattgaact gaccgccgca 540
   caggttgccc gttccgtgcg ggacgccttg gcggagtag 579
<210> 373
   <211> 1698
   <212> DNA
   <213> Unknown
<220>
   <223> Unknown gut bacteria
<400> 373
   atgagcaact cctggactct gcaaagagac accgccgtcc gcgttctgcg cccggaaggt      60
   gccgtgtatg agactgccgc cgcacagctt ctgcactatc tgaacgcact ctgcggcatc     120
   accgaggcgc ggagccagtc cgcacccgcg atcatcctgc ggactgaccc ctctatgccg     180
   ccggatgcct tccggctggt tgtgcggggc aacgacctgc accttgtcgg gcagaacgga     240
   cgcggggtgc tgtacgccgt gttcgccctt gcagagcgtt tcggctgccg cttcttcgcc     300
   catgacaccg aggtgatccc ccacccggcg gagctgtctc tgcctgacgg gttggatgtg     360
   accgaggcat ccccgtttgc ctaccgcgac ctgtactgga tcggcaccac cgatccgctg     420
   accgccgtca agctgcgcat caacggaacg ggactgaaat caggtcagca catccgccat     480
   ctgacagccg aagtcggcgg cggcatcggc tacgccggac cgcacttcgt gcataccttc     540
   gacctgctgc tcccgcctga aaagtatttc gccgaacacc ccgaatacta cgcgctgatc     600
   gacggcaaac ggaccgcaaa acacctgtac agtcagccct gcctgtccaa ccccgatgtc     660
   ctgcgcatta tgatcgaggg catccgctcg tggctccgtg acaatccgga ggcgcggctg     720
   gtgtcggtct cgcagaatga cagcttcgtc atcggctcct actgcacctg ccctgcctgc     780
   agggcggtca acgaggagga aggcgcgcct tccggctcgc tgatccgctt cgtcaacgct     840
   gttgcggagg cactggagcc ggaattcccg gaagtcacct tcgacacgct ggcgtatcag     900
   tactcgactc agccgcccaa gctgacgcat ccccggcaca atgtcgccat ccggatgtgt     960
   accggcggtt gctcctcgca cccgatctcc gactgcgcca acaacgccgg ttcggcggat    1020
   gccatccgcg gctggtcgca gatatgccgt cagctgtata tctgggatta taccacggac    1080
   tttgcccaat acctcaaccc gtacccgaac ttccagacgt tgcaggacaa catccgcttc    1140
   ttccgcgacc acaacgtctg cggcgtgttc gagcagggca actataacga aggtctgtcc    1200
   ggcgagttcg gcgagttgcg tgcctacctg cttgccaatc tgctctggaa tcccgaattt      1260
   gacgtagagt ccggcacaga gtccttcctg aacgcctact acggcggcgg tgcgccatat      1320
   gtacggcagt atctgacctt catccgggac aaggtacggg atgttcattt caatctggtc      1380
   atcagcgccg ccgctctctg gaacgatctc atttcggacg aagagcttac catgctggat      1440
   cgcctgtggg cagatgccta ccgtgccgcg cgcgaaggcg gcgatgtccc ggacggcatt      1500
   tcctccgccc tgtgcgcctc ccatgtggag cgcagcgggc tgtgccaccg ctggttcaag      1560
   ctggacgcca aacggggtga attctcctat cctgcccgct ttgatgccct ctgcgacctc      1620
   ttttaccgtg actgccaccg cctcggcgtc acccgcctga ccgaaggcgc gaacgtgccg      1680
   tgggttgagg cgcagtaa                                                    1698
<210> 374
   <211> 1185
   <212> DNA
   <213> Unknown
<220>
   <223> Unknown gut bacteria
<400> 374
   aactggggcg acgagggcaa ggggcgcgtg gcggatctgc tctcccgcga catggatatc 60
   gtgtgccgtt gtcagggcac aagcgaggcg cgccacacgg tagtgaacga gcgcggcagc 120
   tttatgctga atcagctccc ttccggcatt ctgtacggcg gtgtatgttg catcatggga 180
   gacggaatgt atatccagcc agaccgcctg gacgatgaaa taacacgcct gcgcgatgcc 240
   ggggtagaca tctcgccgga aaacctgaaa atcagccgcc gtgccgcgct ggttctgccc 300
   ggtctgactg cgacgcagat gcgtcttctg ctggaaacgc gcgaccgtct gtgcatgggc 360
   gacctgacgg atgtcgaaat gctggctgag catctgaatg cattggcgga tgcgttccgc 420
   cgttacggcg gaaaaccgaa tatgtcggct tggtctgcgt gctgtcgccg ctatacggca 480
   ctgttcggcg actatattgc cgacacgggc gcctatctgt acgacgcgga gcacgccgga 540
   aagcgtattc tgctggaatc ccagtcgggc gctctgcgtg acaaggattt cggcgtgttc 600
   ccttacggct cgccgacaaa ctgtctggca gcctacacgc ccatcggtgc cggtatgccc 660
   ggcgtgcggc tctcccgcgt ggttggcgtt atgaaagcgt actccgcctg ccatggggac 720
   ggtccgttcc cggctgaaat cacgggtgag gaggcaacct acttccgtga actgggacag 780
   gaggacgagg acggcgccac gccccgccgt gtcggcggtt ttgacgcggt cgcgtcccgc 840
   tacggcgtcc gccttcaggg cgttgatgcc attgcactga ccaagctgga tgcggtatcc 900
   ttcctgccgc agatccccat ctgtgtcgcc tacgaagtgg acgggaagcg tgtcacggat 960
   ttcccgacac cgtccgccct gcggcgcgca aagccgatat atgaatacgt ggacggctgg 1020
   aactgtgaca tctccggttg ccgggcattc gcagatctgc ctgttcaagc catcatctac 1080
   atcaggtacc tggaagagtt ggtcggcgcg cccatcacct atgtgtcggt tggcgccgaa 1140
   cgggatgcct acatccggga cagtgtaaaa gcggaaattc agtaa 1185
<210> 375
   <211> 906
   <212> DNA
   <213> Unknown
<220>
   <223> Unknown gut bacteria
<400> 375
   atgacggatt tcatgaaagc catggcggat gaggtcgccg tccagggcgg cgagggatgc 60
   tattacacgg ttgtaacgct ggagccgccg caggagggtg acaccctgta ccatatggaa 120
   aaggtggatt tccgccccgg caacccggca catgactgct actcggtagc caagagtgtg 180
   gcggtcacgg cggcaggtct gctgtatgac cggggactgc tccgtccgga ggacacgctg 240
   gacagattcc tgtccgccta cctgaccggg aaaactgacc cgcgctgggg caggctgacg 300
   gtggatcagc tgctgcgcca ccggacaggc gccgcctccg gcgtagattt tgacctgacc 360
   aatgcgcatc tgtgggagga tccggagtgg ctgcataccc tgttcgcggc tcccatcacg 420
   cagacgccgg gcgaggaatt tgtatactcg gacgggaact attatatttt ggggcgcatc 480
   gtggaggagc tgctcggcga ggacatggag aagctgttgc tgcgcgaggt ctttgtgccg 540
   ctgggattcc atgccaatgc gtggagccgg gacgtgaacg ggcacaccgt cggcggcacg 600
   gggctgtacc tgcgcacgga ggacatggcg aagatcggct ggctgtacct gaaccggggc 660
   gcgtggggcg accgccgcat cttctcggag gcgtgggcgg atatcttcct gcatccgccg 720
   gtagacaggg tgcggtacgg ctacgccctg acggagacga ccgccgacct gtatctggcg 780
   ggcgggatgt acaaccaggg tttggacttt gacctgcgca cgcggcgtgt ggtggcgtgg 840
   cacgcctttg acgaggcggg cagcttcccg gggctttcgc aggcgttcgt gcggtactgc 900
   cgctga 906
<210> 376
   <211> 870
   <212> DNA
   <213> Roseburia inulinivorans
<400> 376
   atggatttct tatgggataa gatcacggaa tggctgaaag aaatgctcat cggcggtatc 60
   gtgagcaatc tgtccgggat gttcgatgcg accaatcaaa aggttgccga gatctccggt 120
   caggtgggct tatcgccgca ggcatggaac ggcagcattt tcagcatgat acaaaacctg 180
   tcggaaaccg tgatcgtccc catcgcaggc gcaattctcg cctttgtgat gacgctggaa 240
   ctgatccagc tcattaccga caaaaataac ctcaacgaca tggacacatg gatattcttc 300
   aagtgggcgt tcaagtccgc tgccgctgtc ctgatcgtga caaacacatg gacgattgta 360
   atgggcgtgt tcgatgccgc ccaaagcgtc gttgccagcg cctccgggct gattatcggc 420
   gacaccagca tcaatatttc aagcgtcatg gtgggcattg aggatcggct gatggaaatg 480
   gagctggggc cgctgttcgg cctgtggttc cagtcgctgt tcgtgggtat taccatgtgg 540
   gcgctgacca tctgcatttt cattatcaca ttcggaagaa tgattgaaat atatctcgtc 600
   acctctgttg cccccattcc gatggcgacc atgatgggca aggaatgggg ccgcatgggg 660
   caggactgca tcagctccct gctggcactg gtcttccaag cgtttttgat tatcgtctgc 720
   gttgccattt acgcggtttt ggtgcagaac atcgcgcttg tggatgacat catctacgcc 780
   atctggactt gcatgggcta cacggtgctg ctgtgcttct gcctgttcaa aacgagcagc 840
   cttgccaagt ccgtgttcaa tgcacattaa 870
<210> 377
   <211> 441
   <212> DNA
   <213> Roseburia inulinivorans
<400> 377
   gtgcagatca tggcctatgt caacgtccca aaggacttaa ccaaagtaaa aaccaaggtg 60
   ctgttcaacc tcacgaaacg acagctcatt tgcttcggaa gcggcgcgct catcggcgta 120
   ccgctttttt tcttgctcaa gggcagcatc ggcaccagcc ccgccgccat ggtgatgatg 180
   gtggtgatga tcccggctat gctgttcgcc atgtatgaaa agaacggtca gccgcttgag 240
   gtcgtcatcc gcaacatcta ccgtgtgtgc ttccagaggc cgaagcagcg tccgtataag 300
   accaacaatt tttatgccgt tctggagcgg caaaaccagt tagaccggga ggtgtatcaa 360
   attgtccgaa aagaaaaagc tgacccgcgc cgaacgaaaa cagatcgaag ccgccatcgc 420
   aagagcgaac cgaacggata a 441
<210> 378
   <211> 1851
   <212> DNA
   <213> Roseburia inulinivorans
<400> 378
   ttgtccgaaa agaaaaagct gacccgcgcc gaacgcaaac agatcgaaac cgccatcgca 60
   agagcgaacc gaacggataa gaaagaaaaa tcggcgcagg acagcatccc gtatcagcgt 120
   atgtggccgg acggcatttg ccgtgtgacc gacacacgct acaccaaaac gatccagtat 180
   caggacatca actaccagct ctcgcagaat gaggacaaga ccgccatctt tgaggcgtgg 240
   tgcgatttcc tgaactactt tgacagctcc gtgcaattcc agctttcctt tgtgaacctc 300
   tcggcttcgc aggaaacctt tgcacggagc atttccattc cgccctgcgg ggacgagttt 360
   gacggcatcc gcgccgagta cgccggtatg ctgcaaaatc agcttgcccg cggcaacaac 420
   ggcctgatta agaccaagta tctcacattc ggcatcgaag ccgacaacct ccgcgccgcc 480
   aagccccgtt tggagcgcat cgaaaccgac cttttgaaca acttcaagcg gctgggcgtt 540
   gtggctgcgc cgctgaacgg ctttgagcgc ctgcacgtca tgcacgacat tctgcggatg 600
   gacgagcagg agccgttccg cttttcatgg gactggctgg ctccctccgg gctgtccacc 660
   aaggacttta tcgcgcccag ctcctttgag ttcaagaccg gcaggcagtt tcgtatgggc 720
   aaaaagctgg gcgcggtcag cttcgtgcag attctggcac cggagctgaa cgaccggatg 780
   ctggcggatt ttctggatat ggagagcagc gtcctcgtca atctccatgt gcagtctgtg 840
   gatcaggtca acgccatcaa gaccgtcaag cgcaagatca ccgatctcga caagtccaaa 900
   atcgaggaac agaagaaggc ggtccgcgcc gggtacgaca tggacattat cccgtccgac 960
   ctcgccacct acggcgcaga ggctaaaaag ctgttgcagg atttgcagtc ccgtaatgag 1020
   cgaatgttgc tgctgacctt cctcattctc aacacggcgg acacacctcg gcaacttgac 1080
   aacaacatct tccagacctc cagtatcgcg cagaaatata actgtgcgct gacccggctg 1140
   gacttccagc aggaagaagg actgatgagc agcctccctc tggggctgaa tcagatcgag 1200
   attcaacggg gcttgaccac ctccagcgtg gcgatcttcg tcccctttac cacgcaggag 1260
   ctatttcaga acggttcaga ggcgctgtac tacggcatca atgccctgtc caacaatctc 1320
   atcatggtag accggaagct gctgaaaaat cccaacggtc tgattttagg cactcccggc 1380
   agcggcaagt cgttttcggc aaaacgcgag atcaccaatg cgtttctcat ctgccccaag 1440
   gatgacatca tcatctgtga cccggagggc gaatatacac cgctggtgga gcgtttgcac 1500
   ggtcaggtca ttaagctgtc gcccaccggc aagggctacg acggttcgcc ctgctacatc 1560
   aatccgatgg acttgaatct ggattacagc gacgatgata acccgctgag cctgaaatcc 1620
   gacttcattc tgtcgctgtg tgagttgatc gtcggcggca aggacgggct tgcccctgtg 1680
   gaaaagacca tcatcgaccg gtgcgtgcgc atcgtctacc gggactacct caatgacccg 1740
   aaaccggaga atatgccgct gctggaggac ttgtataacg ccctccgtgc gcaggatgaa 1800
   aaggaagcgc agtacatcgc cactgctttg gaaatctacg tcaccggctc t 1851
<210> 379
   <211> 249
   <212> DNA
   <213> Roseburia inulinivorans
<400> 379
   gtgaacgata tgaacccgaa aatcatgaag ctccgtggag agctggagaa gaacaaatgc       60
   aaaatctccg atctgcaagg ccgcaaccgc gagctggaaa agcagattcg tgagctggag      120
   gacaccgaca tcatcggcat ggtgcgcgag aacggcatga cgatggaaca gtttgccgag      180
   ctgttccgcc gtatgcaggc cgcgcctgca ccggccactt cggaaaagga ggcgttgaac      240
   catgactaa                                                              249
<210> 380
   <211> 1023
   <212> DNA
   <213> Roseburia inulinivorans
<400> 380
   atgaaaagag aaccgcgttt gcagttttcc gacgccgatc ttgccgagcc gaagctggaa       60
   aagcccatca agcgggtgaa aaaagcggag gccaaggcgg ataaagcgca ggccaaaatc      120
   cccaagaaaa ccgtggtcaa aaaggaacgc ggctttgatc ctgccaccgg caaggtcaaa      180
   acgcagctcc gttttgagga agtggataag aaaaagccgc cctcaaagct gacccatgcc      240
   gtgcaggatg cccctgccaa ctttgttctc tctcaggttc accgagaggt gcggcagagc      300
   gaggatgaca atgtaggcgt agaggccgcg cacaaggtag agcaagccgt ggagagcggc      360
   gggcggctgg tgcagtctgc ccaccgcgcc catcagctca agccttatcg tgctgccatc      420
   cgtgcagaga aaaagctgga acgagccaat cttgacgcgc tccagaagaa agcagagatc      480
   gacaacccca ccagcaatcc cgtttccaaa tggcagcaga agcaggccat taagaagcag      540
   tatgccgctg ccaagcacaa tcaggcggcg cagactacgg caaaggcggc agagaatacc      600
   gccaaggccg cgaaaaaagc cgctgaaaag gctgagaaag cgggcaaata tgtgtgggaa      660
   caccggcgcg gctttgccat tgccgccgca attctgctga tgctggcatt tctgctcaac      720
   ggcctgtcct cctgctcggt gataatggat ggcgtcggct ccgggatcgc ggccagtacc      780
   tatccgtcgc aggacgccga tatgctgggc gcggaagcgc agtattgcga aatggaggca      840
   gagcttcagc gctacctcga cacctacgaa agcacccatg actacgatga gtaccacttt      900
   gatctggaca ccatcgagca tgacccctat gtactcattt ccatgattac ggcactgcat      960
   caaggggaat ggacgctgga tgaggtacag ggtacgctcc agatgctctt tgaccgccag     1020
   tac                                                                   1023
<210> 381
   <211> 1659
   <212> DNA
   <213> Roseburia inulinivorans
<400> 381
   atggcgagaa aaagcaggaa agaaacggct gcggtggccg tgcaggaggc cgacgccgct      60
   tgccgcgccg cgatctacgt ccgcctttcg gtggaggata cccacacgca cagcgtatcc     120
   attgaaaccc agcagatgat tattgcccgc tatctggagc agtatccgga gatcagcgtg     180
   tacgatacct atatcgacaa cggcgcgacc gggacaaact tccaccgtcc gggctttcag     240
   cagatgctct cggatattga ggccggtcac gtcaactgcg tcattgtgaa agacctctcc 300
   cgtttgggcc gaaacaccat tgacaccggc tattacatcg agcagtattt ccgcatccgc 360
   agcatccgct tcattgcggt caatgaaaac ttcgacaccg ccaacccgga ggacgcccat 420
   tccggtatca tcatcccgct gcggaacatg ataaacgaag catacgcttt ggacatcggg 480
   cgcaagatca gggcgcagca gcggcaggcc atgaaggacg gcaagttcat cggtgcgcgt 540
   actccctacg gctatctgaa agccgaggac gattgccacc agcttatcat cgaccccgtt 600
   gccgccgttg tggtgcagcg gatgttccgt tgggcgtccg atggtgctgg cctcaatact 660
   atcgccgtgc ggctgaatga agcgggcatc ctcactccca gccactataa gaagatgcag 720
   ggcaagatca cccacgcaaa tttgctcggc aacggcaagt ggcagacccg aacggtcggc 780
   gttattctcc gctccgaggt ctacaccgga gatctcgttc aggggcagac caaaaccgtg 840
   gatcaccggc aggtcaaggc cgatgccgag gaatggacgg tggtgcggga cacccatgag 900
   gccataatca gccgggaaca gttcgcggcg gtgcaggaaa ttctcaatca gaccgccagc 960
   cgcgccaagg cacgggaggt caaagcctac acgccgaatt tgctcaaagg caaggtgttc 1020
   tgcgcccatt gcggcggcag cctgcaccgg cagagaaaca tccgcaagaa gtccgacgat 1080
   gtgtacttct accattgtct gagccagagc cgaatcagca aggatgcctg ccccggtgtg 1140
   accatccgcg aggatatgct gctggatatg ctggcagata tgcttcagga cgcgcttgat 1200
   acggcgctgg ggcaatacac cctctccctt gcagagctac cccggcaggc cgctgaacgt 1260
   gccgagctgc gggagaagat caccagccgc aagcaggaaa tccagcggct tcgcggtatc 1320
   gtgcggagct tatatgaaag cctcgtccaa ggcgttctta ccaaggatga atatttcgac 1380
   tacaaggaaa agtacgaaag ccgcattgcc gacctcgccg tggaaatgga gcagttggag 1440
   gacggtctgc ggacgatgga tacgcagatt gagcagcacc gggcgctgga acaggacgcc 1500
   gcgcagatca agaccgaccg tgcgctgacc ggcgcactca tcgagcggct gatcgaccgc 1560
   atcgaggtat cccacgacaa gcagatcacg gtgcgctatc gcttccagag cgagtttgaa 1620
   acctatgcgg aggtgctgga acaatgcaga aatatgtga 1659
<210> 382
   <211> 1035
   <212> DNA
   <213> Roseburia inulinivorans
<400> 382
   atgaagcaag tcaacgtcaa aaagctcatc cttcccaaca tcccgtatgt gttcatcgca     60
   ctgctggcca ccaaggtcag cgaggcggtg cggctggctc ccggttcaga cgcttcggcg    120
   aagctgctga acattatgac cggcctgaac acagcgtttc actcccttgt ccccagcttt    180
   catcccattg acctgtgcgt gggcgttgcc gccgccatcg ccattcggct ggcggtctat    240
   atcaagggca aaaacgcaaa gaaattccgc aaaaatctgg agtacggcag cgcccgttgg    300
   ggaaccgccg aggacatcaa gccttacgtc gatcccgcgt ttgaaaacaa catcattctc    360
   acgcagacgg agcggctgac gatgaataac cgccccaaag acccaaaaac ggcgcggaac    420
   aaaaatgtcc tgatcgtcgg cggcagcggt tcgggcaaga cccgcttttg gttgaaaccc    480
   aatctgctcc aatgcacatc aaaaacctat cccaccagct tcgtcgtcac cgatcccaaa    540
   ggggacatcg tggtttcctg cggccatgcg ctgcaaaaga acgggtatca gatcaagatt    600
   ctcaattccc tgaacttcaa gaagtccatg cactacaacc cgttcgcata catccactcg    660
   gaaaaggaca ttttgaagct ggtcacgacg ctgatcgcca acaccaaggg cgagggcaaa    720
   gccggtgacg atttctgggt gaaagcggaa acgctgctct acaccgccct catcggctac    780
   atccactatg aagcgccgat ggaggaacag aacttctcca cgctcattga atttatcaat    840
   gcgatggagg tgcgcgagga cgatgagaac ttcaaaaatc cggtggactt gatgttcgac    900
   gagctgaaaa agcgcaagcc agaccacttc gccgtccgtc aatatgccaa attcaagcta    960
   agtgccggca agaccgcgaa aagcatactt atttcctgcg gcgcacggct tgcgcccttt   1020
   gacattcagg agctg                                                    1035
<210> 383
   <211> 1635
   <212> DNA
   <213> Roseburia inulinivorans
<400> 383
   atgccaaaaa acaaagcaac cgcttatatc cgcctgtcct acaccgacga tcattccagc      60
   gaaagcgaca gcgtttccaa tcagcgcaag ctcattgaaa actttgtaga gcgcaacccg     120
   gatattgagg tcgtttccga aaagatcgac gatggataca gcggcatcat ctttgaccgc     180
   cctgcgttca aggaaatgat gcaggatgtc accgatggca atatcaactg cgtcatcgta     240
   aaagacctct cccgcttagg gcgcgagtac atcgaaactg gccggtatct gcgccgggta     300
   ttcccggcct acggggtgcg cttcatcgct atcaccgaca gcatcgacac cgcccacgac     360
   agcggcgatg atctgaccgt atcggtcaag aacattagga acgaacccta ctgccgggac     420
   atttccatca agacccacac ctctctggac gtgaagcggc gcaacggcga tttcgtcggt     480
   gcgttcccgg tgtacggcta catgaaagcc gaggacaaca agaatttgct cgtccctgac     540
   ccctacgccg cccgcgttgt ctgcgacatc ttccgtatgc ggctggaggg cgcaagcgcc     600
   tccaagattg catcagagct gaaccggctg ggtatcctct ccccgctggc ttacaagaag     660
   aacaacggtc tgccctacgc gaaaaagggc tatgcggaca aggctgactg caagtggtcg     720
   gccaccacca tcatccgcat cttgcaggac gaaacctaca ccggaacgct ggtgcagggc     780
   aaacagggta cgccgcatta caagatcaag cagatggagc agcgccccgc ctccgagtgg     840
   gtgcgtgtcc cggatgccca cgaagcattg atcgcccgtc aggattttga tctggtgcag     900
   cgcatcaagg ggctggatac ccgcacttct cccaacgagg acacggtgta cctgttctcc     960
   ggtattctga tctgcgggtg ctgcggaagc cgcatgaccc gcaagaccaa ccgtgcgaac    1020
   ggcaaggagt accactacta ttattgtcct accggcaaga aaaagggctg cgcccatccg    1080
   gtcatgctga aagaaagcag cctgatcgac tgtgtgcggg acagcctgaa agcctatatc    1140
   ggcaatattg cttctctgga ggcgctgctg accggcattg accagtccag catcaatcag    1200
   gcgcttgcca aggaatacag cgaccacatt accgacaacg agcgccggtt ggagcaggtg    1260
   ctggagttca aggcacggct ttatgagagc cttgtgggag gtatgcttac caaggaagaa    1320
   tacgcttcct ataaggcaaa gtacaccaag caggccgagg acatccgcga gagcatccgc    1380
   gttctcaagg aaaaactcac ggaggttttg gaaaaccgga gtgagcgcaa ccgctggatt    1440
   tcgcagttta cgcagttctc cacgctggaa accttagacc gcagggcgct cattcacatg    1500
   gtacagagca tccgcgtccg tggcaaaaag gagctggata ttacctttac ccatgaggac    1560
   gaatacaaaa aggcgttgca gcttttggcg ctggcagcgc agcagaaaga ttacgaacag    1620
   agaaaggtgg gctga                                                      1635
<210> 384
   <211> 561
   <212> DNA
   <213> Roseburia inulinivorans
<400> 384
   acgctggaaa acttcaatct ctctcatgtt cccgtttaca ttatgagcga agaacagctc 60
   ggaatgtacg ccacctatat ggccaccctc ggcaaccgtc ccgacctgtt ccccggttca 120
   ggctacatcg gcaagtatgt ggagggcagc tataccgact acgacattcc cccggaggcg 180
   ctggacgatg aggtatttgc cgccatcatc caggaagcgg aaaagtatct cggctacgcc 240
   tacgtttggg gcggcagcag tccctccacc tcgtttgact gttcgggctt cgtcagttgg 300
   gtcatcaatc attccggttg ggatgtgggc agactgggcg cgcagggact ttgcaacatc 360
   tgtacgcctg tatcctctgc taacgtcaag ccgggcgatc tggtgttttt caccggaacg 420
   tatgataccc ccggtgtcag ccatgtgggt atctatgttc gcaaccatat gatgatccac 480
   tgcggagatc cgatctctta tgcgaactta aattcaagct attggcagtc ccatttctac 540
   cgctatgggc gcttgccgta a 561
<210> 385
   <211> 810
   <212> DNA
   <213> Roseburia inulinivorans
<400> 385
   accgatgcta tggcgatcta ccagctacgc cgggatgcgc cggttgagct gcgttttgcc 60
   agtctcggca gactggccgc gtcgcctgac cccgccaact atgaagccat ctatacccgt 120
   gaggtgtacc ctgacgatga taccggcaga atccttgaaa acttctacta cattttcaac 180
   gacgagcgcc ccggcgattt tgtgggacat agcctctccg tcagcgacat cgttgcgctg 240
   aaacaggacg gcaaggtatc ctatcactac tgtgacagca tgggatttca ggagctgccc 300
   gcctttcaga agccggagta ttacctaaaa gcctccgaga tgtcgatgga ggatggttac 360
   ggcatgatcg acggcattat caacaacggc cccaagcagc ccaccgttgc cgaccttgaa 420
   gcacaggtca aagccggtat gtccatctcc ctgatggatt tggcagaggc ggcacaccgg 480
   gaaaagaaaa agtctgtgct ggagcagctc aaaagccaac cggcccaaga acgtccacac 540
   aaaaagcgcc caaaaagagc gcggaaaagg agctttgata tgaacatcac ctttgaagaa 600
   cgccagctta tgagcctcta caacaccggc acccgcacgg gcttgattgc ggcgctggag 660
   gaaatgcgcg gctacctcgc tgcggatgaa acggagctgc gggagctgac ggacagcgcc 720
   atcgcaaagc tgcgcaggat gagcgacgcg gaatatgacg agcttgacct gttccccgat 780
   tttgaaaagg aggatatgga tgccgagtaa 810
<210> 386
   <211> 1011
   <212> DNA
   <213> Roseburia inulinivorans
<400> 386
   atgccgagta aagtacagct ctatgcgcag atggcagacc gcacggcgga gcagatcacg      60
   ggaagctacc agaagtggac ggcatttctg accaccgccg caaggctgta taagtacccc     120
   tacaatgaac agctcatgat cttcgcccag cgcccggaag ccacggcctg cgcggaatac     180
   gacctctgga ataagcagat gcgccgctat gtgcggcgag gcagcaaggg catcgccctt     240
   gtggatacca gcagcgacca acccaaactc cgatatgtat ttgatgtttc cgacaccagc     300
   ggcggcgaga actctcgccg cccttatctt tgggaatacc ggcaggagca tcgtgaggtg     360
   gtatctgcgg cgctggagca gcgctttgac gtttccggtg aaaacggact tgccgatcag     420
   atggagcgtg tggcggcgca gcttgtagat gaatactggc acgacaactg gcgcgatatt     480
   gtcggcatcg ttgacggttc ctttttggaa ggttatgatg atttcaacat cggagccgct     540
   tttcggaacg ccgccgttgt cagcaccacc tatgcgctgc tgtcccgctg cgggatgcag     600
   ccgggagatt acttcgagca tgaggatttt ctgaacgtct ttgatttcaa cacccctcag     660
   accgtcgccg ctcttggaac ggccatcagc cagtccagcg agctggtgct gcggcagatc     720
   gaaatcacca tcaagaatta cgagcgcgag aaaatcgcgg aaaggagcga aagccatgag     780
   cgaactgacc tacacccgca gcggggatta tctgattccc gacctgagcc tgacagagca     840
   gccgcaagcc ccgctgggca agtacggcag gatgcggaag gattacttga aggagcatcg     900
   tccggtgctg tggaacagcc tgctgctgtc cgagaggctg tacccccatc tgcgggagat     960
   cgacgaggca gcgaacaacc gactggaaca gatgatgccg gagctgatga a             1011
<210> 387
   <211> 1875
   <212> DNA
   <213> Roseburia inulinivorans
<400> 387
   gagctttaca ccatccagcg ctatttgcag catgaccggc tgcgagagat gggtatgggc 60
   cacttcgatt gctgggcaag ccgctttggt gaaaccacca ccgcgctgga gcttgccccg 120
   gagggaaccg gctaccgggc aaggacgcgc tttgcaaagt ttttcaactt gcccgaattg 180
   atgaatctgt tcaaagaggt tgccgacatc aaaacggcgg atcagctcca cttgcccacg 240
   ccggaggttg cgtatcacac catcgccacc aaacccacgc agatccagca ggacatggtg 300
   aaagccctct cggaacgagc gtccaaggta cacagcggtg cggtcagccc ggatgtggac 360
   aatatgctca aaattacctc ggacggacgc aagctggggc ttgaccagcg catcatcaat 420
   cccatgctcc ccgacgagga aaccaccaag gtcaatcaat gtgtggcgaa cattctccaa 480
   tactggcgtg acggcgagga ggaaaaactg acacagcttg tgttttgcga catttctacg 540
   ccaaagacca cgccctcgca gcgggcggca aaggcttccc ccggtacgct ggacggcccg 600
   gaaattcatg cgctggagag cgcgatttca ttggaggaaa gctctgacac gccgttcacg 660
   gtctatgagg atgtgcgcca aaagctcatt gacgccggta tgcccccgga acaaatcgcg 720
   ttcatacacg acgccaacac ggaggtcaaa aagcgggaac tgttcgcaaa agtcaggagt 780
   ggtcaggtgc gcgtcctgat gggcagcacg gccaaaatgg gcgcaggaac caacgtgcag 840
   gatcgtttgg ccgcacttca cgatttggat tgcccgtggc gtcctcgtga tctcacacag 900
   cgaaaaggcc gcatcgagcg tcagggcaat cagaacaagc tcgttcatgt ctgccgctat 960
   gtgaccgagg ggacgtttga cgcctacctc tggcaaacgg tggaaaataa gcagaaattc 1020
   atctctcaga tcatgacctc aaaatccccg gttcgctcct gtgaggacgt ggacgctaca 1080
   gcgctgtcct ttgcggagat caaagcgcta tgcgccggtg atccccgcat caaagagcgc 1140
   atggatttgg acatcgaggt gtccaagctc aaaattatga aagcagacca caacagcaag 1200
   caattccgct tggaggacag tctgctgaaa tacttcccgg aaaagataga ggaacacaag 1260
   ggctttgttc gtgggctgga tgcggatatg cagaccctcg cggcgcatcc gctcccggca 1320
   gagggctttg tcggcatgga gattcgcggc gaccggctca ccgataagga aaacgccggt 1380
   gccgcgctgc tggacacctg caaggaggtc aagggcaaag acccggtaca gatcggcagc 1440
   tatcgcggct ttaccatgtc cgttgctttt gattccatgt ggaaaacgta cacgctgacg 1500
   ctgaaaggcc ggatgacaca tcgcgttgaa ctcggttcgg atgccagagg caaccttgtc 1560
   cgcatcgaga acgcgctgga taaaatgccg gagcgtctgc gcagcgttca ggagcagctt 1620
   gaaaacctct ataaccagca ggcggcggca aaggccgagg tcggtaagcc gttcccgcag 1680
   gagcaggagc tggccgcaaa aacggcgcgg ctgattgagt tggatatgga gctgaatctg 1740
   gacggcaagg gacagccgca gcctgagcag gcgattgcaa aatcggcgcg gccttccgtt 1800
   ctggataggc tgaaagcgcc gcctgttcat ggcgcaccgg aaaagcccca caaaaaagaa 1860
   atggaggcac gataa 1875
<210> 388
   <211> 591
   <212> DNA
   <213> Roseburia inulinivorans
<400> 388
   atcgccaccg ctttggaaat ctacgtcacc ggctcgctga atgtgttcaa ccatcacaca 60
   aacgtggatg tgaacagccg cattgtctgc tacgacatca aggagctggg caagcagctt 120
   aagaaaattg ggatgctcgt tgtccaagac caagtgtgga accgcgtgac catcaaccgc 180
   gccgcccata aaacgacccg ctactatctt gacgagttcc atcttctttt gaaggaggaa 240
   cagacggctt cgtacagtgt cgaaatctgg aaacgctatc gcaaatgggg cggcatcccc 300
   accgggatca cgcaaaatgt caaagacctg ttatccagcc gcgaggttga gaacattttt 360
   gagaacagcg atttcatcta tatgctcaat caggccgctg gcgaccggca gattctcgca 420
   aagcagctca acatttcgcc gcatcagctt tcttatgtga cccactccgg cgagggcgag 480
   gggctgctgt tctacggcaa cacgatcctg cccttcattg accacttccc caaggacacc 540
   gagctgtacc gtgtcatgac caccaaaccg caagaggtgg cgtcggcatg a 591
<210> 389
   <211> 846
   <212> DNA
   <213> Roseburia inulinivorans
<400> 389
   atgattaaga ctgcacggca gctcaaagac ctgatacgca atctctccaa ggacaaatcc 60
   gcagatgcgc aaatcctgat gcgcaattac atgatggagc gctttctgga gcgcatttct 120
   ctttcggaat atcgggacaa gtttatcctt aaaggcggga tgctggtggc tgccatggtc 180
   ggccttgacg cccgctcaac gatggacatt gacgcgaccg taaagggtgc gacggtcggc 240
   atcgaagaag tagagaatat gattgcctcc atcatctccg taccggtgga cgatggcgtg 300
   gagttccggt tgaagcgcat ttccgagatc atggacgagg ccgagtatcc gggcatccgt 360
   gtcagcatgg aaacggagtt tgacggtgtg ataacgccgc tcaagatcga catttccacc 420
   ggcgacgcca tcacgcccca cgaggtgcgt tacagcttta agctgatgtt ggaggatcgc 480
   tccatcgaaa tttgggcgta caatctggaa accgttctgg ccgaaaagct ggaaaccgtc 540
   gtgtcccgcg ccaccaccaa cacccgcatg agagatttct atgatctgca cattctgagc 600
   cagctccacg gccagagcat catcccagcc gatctccgtg cggcgctcat tgcaacggcc 660
   aggaaacgcg gtactgagaa gtaccttgcc gacgcgcctg cggcctttga tgaagtcgag 720
   gccgacccga acatggaaaa gctgtggcgg gcttatcaga agaagttctc ctatgctgcc 780
   gacctgtcgt ggcatacggt cgtggaatcc atccgcagtc tatatagatt agcgcgggct 840
   gaataa 846
<210> 390
   <211> 603
   <212> DNA
   <213> Roseburia inulinivorans
<400> 390
   gcggagaaat caaaatccgc aaacacgccg tcgtcattga ggggcatatc ctccctgacc 60
   ggaaaaagcg gcgtcactaa agtgattgga ggaattacta ttagcagagt aactgagctg 120
   gaaaaagcgc tgaccacgct gttccgcaag ggtacggagg tcggctacac ggaaacggag 180
   atcgacgagt tcctggaaaa catcgactat cacgccctgc tgcaagccgt ttggaacaag 240
   gccgaaacgg cctatgccta ccgtgcagat ggcaagaatg cgctgagctt ggactatcga 300
   agcgcggagc tgttcaagca gcgagcgacg cttctctatg aggacgtggg cgacagctat 360
   atcggcgcgg tgtttgctgc ccggtcgatg gagctgtggc tgctggagga tatgggcttt 420
   gccgtcgttg ccaaattcag cgtgaacgcc ggagaaggcg aatacgtcac cgagtatcgc 480
   gtctacaagg gcagcgattg ggcagacagc gagatttctc tggatttgga ggacttggtg 540
   gccgagcttg ccgccctgtg cgatccctac tacgaacacg agcagcccat ctatgagctg 600
   tga 603
<210> 391
   <211> 339
   <212> DNA
   <213> Roseburia inulinivorans
<400> 391
   atggcaaaac gcaagcggga tattcagctc aaattccgcg tcacgccgca ggagcgggaa 60
   atgattgaaa cgaagatggc acagttcggc accaccaaca tggcggcgta tctccgcaag 120
   atggcgattg acggatatgt ggtgaagctc gaccttccgg agctgcggga gctggtgtcc 180
   ctgcttcggt attccagcaa caacctcaac cagctcaccc gccgcgccca cgaaacaggc 240
   cgcatctatg aggccgatct ggaggacatt cagcaaagtc aggagcggat atggacggcg 300
   gcagagaaaa ttgtttcctc gctggcggcg ctgaaataa 339
<210> 392
   <211> 216
   <212> DNA
   <213> Roseburia inulinivorans
<400> 392
   acaggatact acggaggata cggacacatg agatacttca ccaataatcc gctggaacgc 60
   ctgatgatgc agaagccgaa agcagcgcgg gaggacagcc ctcccgacgc tgccaaggaa 120
   cacttctgct acggctgcaa gcgcttcgga cagagctgcg ttcgcccctg ctaccgtgac 180
   gtaaagagaa tggaggctgc accatgcgcc ttgtga 216
<210> 393
   <211> 537
   <212> DNA
   <213> Roseburia inulinivorans
<400> 393
   ccctcccaca gacttgaggg ggaaccctgc ggtatgcgcc agctctttga ggcagcggta 60
   tcgtggagtt atgacagccc tgccaagggc ggcctgcatc atgcccacta tccggggcgc 120
   gtggcaaata ggatggatct tctcaaatgg acaagcagag cggctgcgcc ggtattcgcc 180
   attatgttat gtggctccaa ttctgcccgc gcagccgcct ctcaaagtca gataccatgc 240
   gctgacgggc tttcccatca gcgcattcat gtgaccttga ggaaacccac caaaccgaga 300
   aaggagatcg ccatgatgca gccaacgcca aacgaatccc gaatccacac ggatgagctg 360
   gtggacatcc gggaagtatc tgttgacaaa aatcttccca aggaagaacg cattgccgcc 420
   tttatccgcc agatcaaaaa tccctaccgc ttccgctgcg gcgatttcgt ggtaaacgcc 480
   tgctttgccg ggaacggtgt tacgttggag gaatgtctgc aaggcatttt gcgctga 537
<210> 394
   <211> 174
   <212> **DNA**
   <213> Roseburia inulinivorans
<400> 394
   atgcttctgg cggtcgtttt tcagctgctc cagcagcttg gcaacggctt tgcgaaattc       60
   ctgttcgccc agctttccgc aggagatcat cagggatacg attctctgtt caacttcttc      120
   ctgcatgact tatcacgctc ctttccttgg atttgcaggg agaatgccgg gaca            174
<210> 395
   <211> 240
   <212> DNA
   <213> Roseburia inulinivorans
<400> 395
   atgatcgtcg gtgtaggaca ggcggatata agcggtggct ttgaatttag gcatgaaaaa       60
   gcactccttt ctcccggact gctcccgcat gaaaagagtg gttatctggc tatttggttt      120
   tcatcctttt ccacaccgat catagcactc tctgcggaaa aaagcgagga tgtcgctcag      180
   cgcaaaatgc cttgcagaca ttcctctaac gtaacaccgt tcccggcaaa gcaggcgttt      240
<210> 396
   <211> 186
   <212> DNA
   <213> Roseburia inulinivorans
<400> 396
   gtggaagaaa tgtaccctca aaaatctaaa acatattttt cggaggtact atttatggct       60
   ttctttaatt ctgctgttgg tgttcttcag accctcgttg tggcgctggg cgctggcctc      120
   ggcatttggg gtgcgatcaa tctgctggag ggctacggca acgacaaccg tgcgatgcgt      180
   tcctga                                                                 186
<210> 397
   <211> 915
   <212> DNA
   <213> Roseburia inulinivorans
<400> 397
   atgcctgaaa aaaccgtgca gaaggaaccg ggcaaaaagc ccgcgccatc caaaaagccg 60
   aaaccacagc aggaagtggt cgttcagatt ccactctccg agctgcatcc gttcccggat 120
   catccctttc aggtgcggga ggatgcgtcc atgcaggaaa ccgccgaaag cgtcaaggaa 180
   tacggcgtcc tcgtccccgc gctggcgcgg ccacgggagg acggcggcta tgagctgatc 240
   gcaggccacc gccgcaaaca cgcctgtgag ttggcggggc tgaccaccat gccggtcatt 300
   gtccgcgaca ttgaccggga cgctgccacc attatcatgg tggacagcaa cctccagcga 360
   gagaacattc tcccctccga acgcgccaaa gcctacaaga tgaagatgga ggccatcaag 420
   cggcagggcg cacggacgga tctgacttcg ccgaaaattt cggcgaagtt ccgcagcgat 480
   gatgaagttg gtcaggatgc cggtgtgagc ggcgatacca tccgcaacta catcgccctg 540
   acgcagttag tcccggagct tcagcagatg gtggacgaaa agaaaatcgc cctcagtccg 600
   gcgtatcaga tcgcggcgct aacacccaag gagcagggct tgcttctgga aaccatcgac 660
   agcgagcagg ccaccccctc actctcgcag gcgcagcgga tgaaaaagct cagtcaaagc 720
   ggtgagctga atgaggacac catgctctcc attatgctgg agcagaagaa accggaaaaa 780
   aacgacatca ccctttccgg tgaaaagctc cgcaaatact tcccgcgttc ctataccccg 840
   ttccagatcg agaacaccat cttcaaatta ctggacgcat ggcagaaaaa gcgccagcgc 900
   gaccagagcc gttga 915
<210> 398
   <211> 327
   <212> **DNA**
   <213> Roseburia inulinivorans
<400> 398
   atgttcatcc tgaaaattct ctttgcgccg ctgtcgctta tcctgtccct gttcgtttgg 60
   ctgtgcgcgg ggctgctgtc ctgttccggc ttcgtgttca agctggcaag cggactgctc 120
   tccttgctgg ctttcgccgt gctgattacc tactccgtaa agaacggcat catcctcctt 180
   aggctggcgt ttctgatcag cccgatggga cttccgctgc tggctgtgca ggggcttggc 240
   aagctgcaag acgtgaactc tgccatgaaa aacttcattc atagtaaaaa cacaagccgg 300
   ggcgaacgcc tcggcttgct tttctaa 327
<210> 399
   <211> 621
   <212> DNA
   <213> Roseburia inulinivorans
<400> 399
   gacgccatgt atatcaacac attcaaatat acccccaaag atgtgagctg ccagctctgt 60
   accgagtatg tgaaaaagct gggctgtacc gccctgcgct gcccgtggct ggccgagcgc 120
   atcgaggccg gtgtggtcgg ctaccgcgag gcagtcttag agacatttcc ccatgaacgc 180
   cgcctgttcc agcgcctgaa cctgctgatc aagcactatc ccggaagcct ttggagcaac 240
   gagcagcatg agcgcaggat gcagtatcag tgcgccgtgc agggctatcg ccgccgcaga 300
   gacaccaacg cctactatgc ggccatgtac ctgctgacct ctaacgacga catttaccgc 360
   cgcacggcaa attgcttctg caaggacggg atcgagttcg gctatgcggt gctgaaaaac 420
   acctcgccgc acaactacgc actgttcatg gctgcgcgcg acctctgcga caagacggag 480
   gcggtcacga tggcggactt agcggaaccg gaggtcattg acccggaggc gctgcgcctg 540
   atcgtcaatg ccactctgat cgcccgctac gggcttgccg cctttcagat cagagcgcga 600
   ggtgccgaat atgaacgctg a 621
<210> 400
   <211> 165
   <212> DNA
   <213> Roseburia inulinivorans
<400> 400
   ctgcacgcac ggtgtggagc cggggaaaag ctggagatca tatcaaatgc ttacctatgg 60
   ctatctggtg ctaaatctca gggcatgaag cagctgatgg ccggtggcgg ggtggctttg 120
   attggcatta ccctcgtgcc gctgctgtcc ggtctgttcg gctaa 165
<210> 401
   <211> 3036
   <212> **DNA**
   <213> Unknown
<220>
   <223> Unknown gut bacteria
<400> 401
   cccggcagca ccgaacccct gaaggacggc gaagaagcgc tggcaaaggc catcgagttt      60
   ggcttcccca tcatcctcaa ggcggcagcc ggcggcggcg gccgtggtat gcgtcgctgc     120
   gacaagcctg aagaagtcgt ggcagcctat gacatggttc acagcgaagc cctgaaggcg     180
   ttcggcaacg gcgacatctt cattgaaaag ttcctggaac gtcccaagca cattgagatc     240
   caggttctgg ctgaccagta cggcaatgtc gtccacctgt tcgagcgtga ctgctctttg     300
   cagcgccgtt accagaaggt cgtggaatac acccccgctt tctctgtgga tcagtccatc     360
   cgcaacgccc tgtatgaaga cgccatcaag atctccaagg ccgttggctt cgtcaacgcc     420
   ggcaccctgg aattcctggt ggacagcaag ggcaaccatt acttcatcga gatgaacccc     480
   cgtatccagg tggaacatac cgtcaccgaa atggtcaccg gcatcgacct ggttcgttcc     540
   cagatcctca ttgcggaagg ccgtcccctg agcgatcccg ccattggcat ctccagccag     600
   gctgacgtga ccacccgcgg ctactccatc cagtgccgtg tcaccaccga ggatcctgcc     660
   aacaactttg caccggacac cggtaagatc accgcttatc gtaccagcgg cggtttcggc     720
   atccgtctgg acgcaggcaa cacctatgtg ggcgcagaga tctctcccta ctacgacagc     780
   ctgctggtta agatcaccac cctggacaac acctttgagg gcgcttgccg caaggcactg     840
   cgtgccctga gtgagaccag aatccgcggc gtcaagacca acatctcctt cgtcagcaac     900
   atcctgtcca atccggtctt ccaggctggc cagtgctaca ccaagttcat tgatgacaca     960
   cctgagctgt tcaccatcag ccagtctcag aaccgtgcca acaagatgct ggaatacatc    1020
   ggcaacatcg ttgtcaatga ccccaccgca ggtcagaagc tgtatgagac gccccgcttc    1080
   cctgacgtac ccgaagggga tcccaagccc ggcctgaagc agtacctgga tcagaacggc    1140
   cccgaagccc tgagcaagtg ggtgctggat cagaagaagc tgctggtcac tgataccacc    1200
   tgccgtgacg cccatcagag cctgctggcc acccgtatgc gtacccgtga tatggtcaag    1260
   tgcgcaccgg ctatggccaa gatcctggca gactgcttct ccctggagat gtggggcggc    1320
   gctaccttcg atgtggcata ccgtttcctg aacgaatctc cctgggaacg tctggattgg    1380
   ctccgtcgtg agatccccaa tatccccttc cagatgctgc tgcgcggctc caacaccgtg    1440
   ggctacacca gctatcccga taacgtcgtc cgtgaattcg tccgtctgtc cgccgagggc    1500
   ggcattgacg tgttccgtat cttcgactcc ctgaactggc tgcccagcat ggaagtcgcc    1560
   attgacgaag ctctgaagca gaacaaattc gtagaaggca ccatctgcta cactggcgag    1620
   atcaccgata cttctcgtaa ctacaccttg aagtactatg ttgacatggc aaaggaactg    1680
   aaaaagatgg gctgccactc cattgccatc aaggatatgt ccggcctgct caagccttac    1740
   tccgcaaagg ttttggtctc cggcctgaag gacgctctgg atatccccat ccacctgcac    1800
   acccacgaca ccaccggcaa ccaggtggcc accaccttga tggcagccga agccggcgtg      1860
   gacatcgcag accttgccat ctcctccatg agctccctga ccagccagcc ctctatgaac      1920
   gctgtggtcg ccgctctgga aggacaggaa cgggataccg gcctgagcct gatggatctg      1980
   cagaagctga ccgattactg ggcagacgtc cgggaacgct atgtggactt cgagtccgac      2040
   ctgaagacgc ctgtcaccga tatctatcgt tacgagattc ctggcggtca gtataccaac      2100
   ttgaagccgc aggtggagag cctgaacctg ggacaccgtt tcctggaagt caaggaaatg      2160
   tacaagaagg tcaacgatat gctgggcgac atcgtcaagg tcacgccctc ctccaagatg      2220
   gtgggcgacc tggccatctt catggtgcag aacgacctga cccccgaaaa catcgtggaa      2280
   aagggcgcag ctctgagcta tcctgactcc gttgtcagct acttcaaggg catgatgggt      2340
   cagcctgcat ggggcttccc caaggatctg caaaagatcg tcctgaaggg cgaagaaccc      2400
   atctccgtcc gtcccggctc cctgctgcct cccgctgact tcgaggctgc taaggaacac      2460
   ctgcgcaagg tctgcggcta tgaaccttct gagcgtgacg tcatttcctg gctgacctat      2520
   ccgaaggtgc tggaggacta cttcaagaac cgtgcggaat acggctacat cacccgtttg      2580
   ggcagccatg tgtacttcca cggcctggca gtgggcgaga ccaaccgcgt caacatcgaa      2640
   gacggcaaga cgctggtcat caagtatctg ggtctgggcg accgcaacaa cgacggtacc      2700
   atcaacgtcc agtttgagct gaacggcatg cgccgtgaga tcaacgtcca ggataagcac      2760
   gccaaggctt ccgtccacgc agtggaaaag gccgatcctg acgatgtgat gcaggtcggt      2820
   gcttccatcc ccggcgcagt cagcaaggtg ctggtgaaga agggcgatgc agtgaaggaa      2880
   aacgacgtcc tgctcatcat cgaagccatg aagatggaga ccagcgtggt cgcccgtgca      2940
   gatggcgttg tcagcgacat caaggtggaa gcaggcgaca gtgtggttgc cggtcagctg      3000
   ctgatgaagc tgaagaaagc agaggacgaa gactga                                3036
<210> 402
   <211> 2121
   <212> DNA
   <213> Unknown
<220>
   <223> Unknown gut bacteria
<400> 402
   atgaaaaatg accgttggtc aaaatggaag gaagggatca cggtgctgaa attcggcgaa 60
   aagatcgtca aactgcgtat acccattctc atcgtcagta tcctgctgct cattccggca 120
   gcgatcggat acttcaacac ccgtatcaac tacgacatcc tgtactatct gcccaaggac 180
   atcgacacca tgcaggggca ggacgtcttg atggatgaat ttgggaaagg cgcctatgcg 240
   ctgtttgtct gtgagggcat ggagtacaag gacgtcgcca acctcaaaac cgacattgaa 300
   aacgtagacc atgtagcaca ggttatctgg tatgactctg ttgccgacct gtccgttccg 360
   gtggatgtgc tgccggataa ggtcaaggat gtgttcaatt ccaaggacgg ggacgccacc 420
   ttgatggcca tcttcttcga caccaccact tcggcggacg agaccatgga tgccattgag 480
   gagattcgga gcatctccgg gaagcagtgc ttcctgtcca gtatgtccgc tatcgtcacg 540
   gataccaaga acctggtcaa ccaggagctg gttccttatg tgctcatcgc tgtggtgctc 600
   tgctgcatcg tcctggcggt gaccatggac tcctttgtca tcccagtgct gttcatgctg 660
   agcattggca tggctatcat ctacaacctg ggcaccaacg tgttcaaagg agagatctcc 720
   ttcatcacca tgtccctggt ggctgtcctc cagctgggcg tgaccatgga ctactccatc 780
   ttcctatggg gcagttacaa ggaacagcgg gagaccatag cggataagaa cgaggccatg 840
   gctcacgcca tcgccgctac catcacctcg gtgaccggtt cttccctgac caccatcgct 900
   ggcttcatcg ccctgtgctt catgagcttc accttgggac tggatatggg cgtggtcatg 960
   gccaagggcg tcctgctggg cgtcatctgc tgtgtcaccg tgctgccttc cttgatcttg 1020
   gtctttgata aggccatctg gaagactgct cacaagaccg tccacctgcc cactgacggc 1080
   ttgagcgagt tcgtcatgaa gcactacaag ctgtttgcaa tcattatggt agtgctgtgg 1140
   attccggctc tgtacggcaa cgccaactac aacgtctact acaagctgga caacagcttg 1200
   ccagattacc tgccctccgt gcaggccaac caggagctga acaaaaagtt cgatatgaac 1260
   tccgtggaaa tgatcctctg tcgggatgac ctgtcccaga aggaggtcaa ggccatgctg 1320
   aaagagattc agaacgtgga cggcatcaac ttcgccctgg gtctggactc cctcaccggt 1380
   gacctggtgc ctgatgagct gattccccag tcggcacggg agaaaatgga gagcggcggc 1440
   tatcagctgc tgatgctcag ctccaaatat gaagttgcca ccgatgaggt caatgcccag 1500
   tgcaacaagg tggagcagat cctgaagaag tacgacgaaa aggggatgct catcggggag 1560
   gcaccctgta ctcgtgacct gatcaagatc accgaccatg acttcatggt ggtcagtgcg 1620
   gtctccatcc tggccatctt cgtcatcatc ctgctggtgc tgaaatccat ctccctgccg 1680
   gtcatcctgg tggccgtcat cgagctggcc atctacatca acatggctct ggcctacttc 1740
   accggtacca cgctgccctt catcgcctct atctgtatcg gcaccatcca gttgggcgct 1800
   accgtggact acgccatcct gatgaccacc cgttacaaaa aggagcgtat ggctggctgt 1860
   tccaaggagg aggccgtcaa gattgccttg tctacctcta tccactccat catgtccagc 1920
   gctctgggct tcttcgccgc caccatcggc gtggggcttt actccgatgt ggacttgatc 1980
   ggctccctgt gcctgctcat ggcacgcggc gccatcatca gtatgtttgt tgtcatcttt 2040
   atcatgccca gtatgtatat gctgcttgat aaactcatct gtaaaaccac cctcggctta 2100
   cgcaatacgg tggatgtcta a 2121
<210> 403
   <211> 2352
   <212> DNA
   <213> Unknown
<220>
   <223> Unknown gut bacteria
<400> 403
   atgggagcaa actactttac accggaaaac gcttggcggc atctgttctt catccacggc 60
   tgtaccgcag atgagttcta caccgatggc ggtctaaagc tgaatatcga gcagctgacc 120
   cagctggagc tgaagcgcag cggctatgcg cgggtggtct tttacgacaa ggatcagaag 180
   ctctactgct acgatgacga atccttcgcc ctcctgcggg ggcggagcgg tgcgggcaaa 240
   gaatcggctc ccgcaccgga aggcgctgcg cccacccatc ggaagggcgg cctgaaacgg 300
   ggcaagctgg gcaaaaaggc ggctgcgtca gcagagcctg tccagccggt ggaggcggaa 360
   gccgttcccg accagccgga gcagaccaat ggaacgtgga cgcccggcgc cgcctccggc 420
   atcggcatcc gccggatggg cgatggctcg ctccacctgg gcatggtgga caacaccttc 480
   gtcaagcgcc agatagacgc ctatatgtat gacgccagca tcaagacggc ggtggtcatc 540
   aatgatccgg atgacttcct gcgggagttc ggcgatgacc ctatgcacgc cctcaccgcc 600
   ggatacgagc ggatggggac ggagaaccag aacatcatgg tctttcttta caccgataaa 660
   cgcttgggca acgtctacca ggtcgcccag ttcgaccccc aggggaagaa cgtcaacgat 720
   attcagatcg gctgccccaa tgtggccgag ctgcgcaacc tgctcaccta tctgcgggtg 780
   cgccacggct tgcggatgtc ccttcgggat ttgacgccca acgccctggc cttgcgccag 840
   gccatgtctc tcagcgagca ggaggtgcgc atcaaggatc tatacacccg cctgcggacc 900
   tttggcgatc ggcagcctct gacaccggca gcctgctatg agctgttggg cgtgaaacag 960
   cctaccccag ccaaggaaca gctggcatcg ctcatcggga tgcagtccct gaaggatgct 1020
   ttggctcagt ttgacaccgg tgacgccgac caggctggcg cactggccta cttgactgcc 1080
   tcccgcctcc agcctgacct gccccatccc aagcccaagg aggagatgat ccacttcctt 1140
   ctcaccggca accccggcac tggcaagaac accaccgccc acctcatcgg acagctattc 1200
   tacgagatgg gctatctgga gactggacat gtggtggaga ctgaccgttc ccagctggtg 1260
   agcaaccatg tgggcgagag cgccctgtgg acccgacaga aggtgcagga ggccatgggc 1320
   ggtgtgctgt tcatcgacga agcctatacc ctcaagcgca atcgcgacag cggcaacgac 1380
   tttggccagg aggtcattga cacgctggtg aagctgatgg atcaatacaa aggcaaattc 1440
   atcttagttg cggcaggcta ccccagggag atggagctgt ttgagaagtc caaccccggt 1500
   ctggccggac ggttccgcca cctgcacatc agcgactaca ctccggcgga gatggaacag 1560
   atcgtggcct tccacacacg gaagaacaac gccgtcctct ctgacgaact ccaggcagca 1620
   ctgcccaact tctgtgagaa ctgggtgaac ctggcgggca cggagtggaa caacgctaga 1680
   gaagcggtga acctgctgga tgaaatgctg cggaactgga aacaggatcc ggaagcccag      1740
   tcggtgaccg atgagacggg ggtcacccac gccctcctgg aaccacgcca cattccggaa      1800
   gccctgtccg gcaacctgaa accggtggcg gagatgcagg cggaagcgct gaaccggctt      1860
   aacagcctga ccggtctcaa tggcgtgaag gcgaccgtag agcggcttcg ccgccggatg      1920
   ctggcaggcg atcagaagga gccgggacac tacctgttca ccggcaaccc cggcacaggc      1980
   aagaccaccg tggcacggta catggggcag atcctgcgca acctggggat gctgaagcgg      2040
   gggcacctgg tggagttcac cgcttcggaa ttgatgtccc aggtgttcaa cgaggagcat      2100
   cagggggact tctatgagac ggtgaggcac gccatggatg gagtgctgtt cattgatgag      2160
   gcctatcagc tgaccaccga caccaccggc cgtggccgtc ccatcctgga tgcgctgctg      2220
   ccctttatgg agaacaatcg caacaatctc tgcgtcatcg tagcaggcta tgaggacgag      2280
   atggacgact tcttgaagta caattccggc ttccagagcc ggttcaccga gagcgtacac      2340
   tttgacaact ac                                                          2352
<210> 404
   <211> 2076
   <212> DNA
   <213> Unknown
<220>
   <223> Unknown gut bacteria
<400> 404
   atggctgatt ataacaagac catcaatctg cccaagaccg acttccctat gcgtgccaag      60
   ctggcacagc gggaaccggg gatgctggaa gaaatgaatc gcaaggatct gtaccgtaag     120
   gtgatggaac ggaacaagga taagactccg tttatcctcc acgatggccc tccctatgcc     180
   aacggggata ttcacattgg caccgccatg aataagatcc tcaaggatat gatcatccgc     240
   tacaagaata tgaccggcca ctatgcgcct tatgttcctg gctgggacac ccacggtatg     300
   cccatcgaga ccgccattca gaagaagggc gtcaagcgca acaccatgtc cgtgccagaa     360
   ttccgggaca agtgccggaa ctttgccctg gatttcctca accgtcagcg ggaacagttc     420
   cgccgcctgg ggggcattgg cgattgggag catccctaca tcaccctgaa gcctgaattt     480
   gaagccaagc agatcaaggt attcggcgca atggcagaaa aaggctatat ctaccagggg     540
   ctcaagcccg tctactggtg ccccacctgc gagaccgctc tggcagaagc tgaggtggaa     600
   tatgctgatg accccgtcac caccatttac gtcaagttca aggttcatga tgacaagggc     660
   aagctggcac agtacggcga tctggacaag atgtacttcg tcatctggac gaccaccacc     720
   tggactctgc ccggcaacat ggccatctgc ctcaatcccc agttggagta ctctctggtc     780
   aagaccaatg gcgagatcct catcatggcc accgatctgg tggacagcgt catgaaggat     840
   tccggcatca ctgagtatga gacggtaggc accctgtttg gggacgagtt cgaatacatg 900
   accgcctatc atccctttat gaatcgggat tccctcatca tccggggcga ccacgtcacc 960
   ctggatgccg gttccggctg cgttcacacc gctccctcct tcggcctgga cgacttctat 1020
   gtgtgccagc gctatgacga gatccccacc ggcgtggaga tggaagtctc cgtcaacgca 1080
   caggggttcc tgaaccagtt tgccggcccc tatgaagggc tgcatgtgac caaggcacat 1140
   ccggtcatct tcaaggatct ggtggacatg ggtgccatcc tgtccagtgc ggatatcgtt 1200
   catagctatc cccactgctg gcgctgcaag aagcccatca tcttccgtgc cacccagcag 1260
   tggttcgcca gcgtggatgc catcaaggat gccgctgtgg aagcctgcga cgacatcact 1320
   tggaagcccg aatggggcaa ggaacggatg atctccatga tccgcgaacg ttccgattgg 1380
   tgtatctccc gtcagcgtac ttggggcgtg cccatcccca tcttcttctg caaggactgc 1440
   ggcaagccct actgcacgcc ggaatccatc gaaaagattt ctgagatctt cggcgctgaa 1500
   ggctccaacg cctggtgggc gaaggaagcg gaggaactga tgccggaagg cgctgtctgc 1560
   gaatgtggct gcaaggaatt caccaaggag accgacatca tggacgtctg gttcgattcc 1620
   ggctccacct gggctgccgt ctgcgaccac aatccggaac atcctgaact ggtctatccc 1680
   accgacttgt acctggaagg cggcgaccag tatcgcggct ggttccagtc ctctatgctc 1740
   acctctattg ccgcccgtgg tgtggcaccc tataagcaca tcatcaccca cggctggacc 1800
   gttgacggcc agggcaaggc catgcacaag tccttgggca acaccgtcgc cccgcaggac 1860
   atcatcaagg actacggcgc tgacatcctc cgtctgtggg tctcctccag cgactacact 1920
   caggatatgc gcatctccaa gcccatcctg aagcagctgt ctgacgccta cctgaagatc 1980
   cgcaacacct gccgttatat cctgggcaac ctgaccgggt tcgacctggc tgccgacctg 2040
   gtggagtaca aggatctgat ggaactggat cagtgg 2076
<210> 405
   <211> 939
   <212> DNA
   <213> Unknown
<220>
   <223> Unknown gut bacteria
<400> 405
   atgaaatacc ttttgaaaaa agtattcact ctcattatta cattgtttat cgtttccctt      60
   ctcgccttcc ttgcgtttca ggtgatctcg gatccggcca ctgccatttt aggcaccagc     120
   gccaccccgg aatctctggc ggcgctgcgg gaggagatgg ggctgaaccg tcccattttt     180
   gtccaatacg gaagctggct tttgggcttc ctccaagggg attttggcac gtcctacagc     240
   taccatatac cagtagcgga aatgatcgcc caaaagctcc ccatcaccgc catgctcacc     300
   ctgttctcct tcctgctcat gatcctggtg tccatccctt tgggcatctg gacggcacgg     360
   aaagagggcg gcctgctgga tcggagcctg acggtcatca accaaatctt tatgtccgtc     420
   ccgcccttcc tcttgggcgt ggcctgtact tatatcttcg gcgtaggtct gcgcatcttt     480
   gtccccggac agttcgtgtc ctatacccag tcctggccga aattcctagt ctacctcatc     540
   ttcccagcca tctccattgg catctcacgg atcgccatga cggtgaaaat gctccgtgcc     600
   tccatcctga accagatgga gcgggactac gttcgcactg cccggagtcg ggggagtacg      660
   aaggatcgca tcctgagaaa acacgtcctg cgcaatgcca tcatcccagt catcacgttc     720
   attgcaacgt ccatggcgga gatcgtggcc agcagcatcg tcatcgagca ggtctttgcc     780
   atccctggca ttggcgtcct cctgctctcg tccatcaaca accgtgactt cccggtggtg     840
   caagccctgg tgatgatcct ggttctctgg gtggttctgg tgaacttcct ggccgacctg     900
   ctctaccagt acatggatcc ccgtattcgg ttacgctga                            939
<210> 406
   <211> 1599
   <212> DNA
   <213> Unknown
<220>
   <223> Unknown gut bacteria
<400> 406
   aagaccaaga aggtcaacgg caagaacgtg accatctatc agcccttcct gatggtctcc 60
   ggcctcctgg tggacaacga taagatgtcc aacgtggaag tcaccaacgg caaagtcatc 120
   aacagcggcg acaagaccgt ggtcgtgggc atggcactgc ctggcctgga ggagagcttg 180
   ggtctggctg atctgaccga cagcgacggc aagaagattg acattgatgt gcctcagaag 240
   gtcaccatca aagcggatgt ctccgacttc tccctgctca ccaccgtcac catggcgtcc 300
   aacagcgcac tggaagaact gaatctggac gatgtggact cggttgacga cctgaaggac 360
   tccctgaaaa agctgggcga cagctccaaa gagctggtgg acggcaccaa gaaactggaa 420
   gacggcgtag gcgagctcaa cgacaagagc ggcgacctgg ccgatggcgt ggacaaagtg 480
   aatcagggtg caaaggattt ggataagggt gccaaagacc tgaacaacgg cgcaaaggat 540
   ttggatgacg gcaccaacga gctggccacc caggtcaagg atctgcccag ctctgcccag 600
   cagttgttgg acggcgccaa gagcatcaag tccgccctgg gcaacgccga cagcaagacc 660
   cagaccatct acaccggcgc cgctgccatc gaggaaggcg cacagcagat gtccgataac 720
   ctgaagaaca ccaacggcaa gagcatctac gaagccgcag ctggcatcca ggaaggtgcc 780
   gcaaaggtca ccagtggcgc agcaaagctg tctgacgggc tgaacaccgc agcagctaac 840
   ttggatgaat ccatcaagct gctgaacgcc gcagtgggtc agctggaaga tttgcagaag 900
   actacggaag acaccacaac gctgcaaacc tatggcgcgc tcatcaaaga gatcaagggc 960
   agcatccagt atcagcaggg cgtgaagcag gcactcacaa gcaccttaca gtctggtatc 1020
   accagcatca aggggggcgc aaccctcatc gacacctacg ccggtaagat cgccgatggc 1080
   tctgtgaagc tggtggaagg cgcagcaaag ctggaggctg gcgcaaaggc catccagagc 1140
   ggcgtagaca ctatgatctc cggcaacaac ggccagaacc tgaacgctat gatcgccggc 1200
   ctgagcatgc tgagccagca gagtggcaag ctggtggctg gcactcagaa gttggcggac 1260
   ggctccggta aactgctggc aggtaccgat accctgaccg aaggcaccgg caagctggca 1320
   agcggcaccg gcgaactgtc tgatggtacc gacaagctca tcagcggcat cggcgagctg 1380
   ctggacggct ccaaggatct ggcggaaggc atggccaagt tcgatgagga aggcatccag 1440
   aagctgacca gcctcactga cggcgacttg ggcgacatcg tagatcgcct ggaggcggtg 1500
   caggactatg ccgaggaata caagtcttac ggcggctgcc ctgctggcct ggagtgcagc 1560
   accaagttca tctacaagac cgattccatc ggggaatga 1599
<210> 407
   <211> 1851
   <212> DNA
   <213> Unknown
<220>
   <223> Unknown gut bacteria
<400> 407
   atgattgacg caaagctaga gcgcattttg ttgcgcgtac aaaagcccgc ccgctacacc        60
   ggcggggaat acaacgagat catcaaggat aaggcagcgg tagatgtgcg ctttgccatg       120
   tgctttccgg acacttacga aattggaatg tccaacctgg gactgcgcat cctctatggc       180
   tccatgaacc aggcggaggg cgtctggtgt gagcgtgcct ttgctccctg gggggacatg       240
   gaggaggaga tgcgcaaaga acatatcccc ctctacgccc tggagagcgg cgacagccta       300
   aaaaacttcg attttgtggg cttttccctg ggctacgaga tggcctatac caacgtgctg       360
   aatatgctgg atctggctca catccccctt cacagcgacc agcgggggga ggacgatccc       420
   atcatcgtgg ccggcggcac ctgcgtgtac aacggggagc cgttggcgga cttcatcgac       480
   attttctcct tgggtgaagg ggaggacgtg accctggaga tgatcgacct gtaccggaag       540
   gggaagcagg ctggttggag ccgggcggag tttttgcaaa atgccgccca ggtgcctggc       600
   ctgtatgtac ccagcctgta tgaaatttcc taccaccagg acggcaccgt tcaggccatc       660
   acccccaccc acggtgcgcc ggagcacatc cgcaagcgca tcgtggcaga cctggacaag       720
   tcctatttcc ccgtcaagac catcatcccc tccaccgaca tcgtccacga ccgggtgatg       780
   ctggaggtgt tccgaggctg tatccgaggt tgtcgattct gccaagctgg gtattgctac       840
   cgtccggtgc ggcccaagag cgtagacctg ctggtgcagc agggcatcgc atcctgcaag       900
   gactccggct accaggagat gaccctctcc agcctgtcca ccagcgacta ccgccccctg       960
   accgagctgt gcgatggcct gttggagtac tgcgaccccc gcaacatcaa catcaacctg     1020
   ccctcccttc gggcggacgg gttttccatg ggcatcatgc agcggctgca gcgggtgcgc     1080
   aagaccagca tcacctttgc ccccgaggcg ggcactcagc ggatgcggga caccatcaac     1140
   aagaacgtca aggaagaaga tttattacag tcctgccgca ccgccttcga gggcggctgc     1200
   aatgcggtga agctgtattt catgctgggt ttgcccaccg agacggatga ggacgtcatc     1260
   ggcatcgccc gactggcgga gaaggtgtac tggacgtgga aaaattacgc cgtcaacaag     1320
   agccgcggcc tgcgcattac cgtctccacc tccttctttg tgcccaagcc ctttacgcca     1380
   ttccaatggg aggcgcaaat tacgagagat gagtaccacc ggcgggtgga cttgctgcga     1440
   ggctccatca aaaacaagtc catccagtac aactggcacg agatggagac cagctacatc     1500
   gaagcagtgc tggctcgtgg cgaccggcgg ctggggaagc tgctggaaac ggtctggcgc     1560
   aagggcgggc ggctggactc ttggagcgag tatttctcct tcgagcgctg gatggaagcc     1620
   tttgccgaga ccggcatcga cccagatttc tacgccctgc gggagcgccc caaggacgaa     1680
   atcttgccct ggagcatgat cgacattggc gttcacccgg aatacctgtg gcgggaacgg     1740
   gagcaggcat atcgctcggt catcaccccg gactgtcggg tgcagtgcat gggctgcggt     1800
   gccaactgcc tgctgccgga cggcgtgttc tgtgacgaag atcggaggta a              1851
<210> 408
   <211> 1632
   <212> DNA
   <213> Unknown
<220>
   <223> Unknown gut bacteria
<400> 408
   atgagcaaga agcaaaaaag ggtgctctat cgcctcatcc tcgctgctgt cctcctggtg 60
   gcggcatccc tgctgccagt gactggtgtt tggaagtttt tgtgcttcct ggtgccctat 120
   tttatcattg gctatgacgt tctgctgggc gctctccatg gcatcgtcaa cgggcagatc 180
   tttgacgaga acttcctcat ggccatcgcc actgtaggcg ctatgggcgt gggggactac 240
   aaagagagcg tggccgttct gctgttctac cagctaggcg agctgttcca gagctttgcc 300
   gtgggcaaga gccgtcagag cattacccag ctgatggaca tccatccgga gtacgccaac 360
   gtgaccgatg agaacggtca gttggcgcag gtggatccgg aagaagtgga agtggggacg 420
   gagatcaccg tgctgcctgg ggagcgcatc cccatcgacg gggtcatcct cacgggcacc 480
   tctaccctga acaccagcgc actcaccggt gagagtctgc cccgttcggc agtgccgggg 540
   gatgcggtca tcagtggctg cgtgaatttg gatggtgtgc tggtcatcca aaccaccaag 600
   ctgtatgagg actccaccgt agctcgtatc ttggagctga cggagaattc cgccatgaag 660
   aaagctaggg cggaacggtt catcacccgt tttgcccact actatacccc gatcgtctgt 720
   atctctgcct tggtgctggc catcctgccg ccggtcatcc ggttggccat ggggttcagc 780
   ggggaatggg gcacatggat ctatcgtgcc ctgaccttcc tggtcatcag ctgtccctgc 840
   gctctggtca tctccattcc gctgaccttc ttcggcggca ttggcggtgc ttcccgtcac 900
   ggcatcctgg tcaagggtgc caacgacctg gagactttgg ccggtacccg ctgcatggtc 960
   tttgacaaga ccggcactct gactcagggc gtgttctcgg tcaccgaagt ccatccggca 1020
   gaggggtggg acaaggaaca agttttgcgc ctggctgcgc tggcggagca gttctccaac 1080
   catcccatca gccgcagcat ccaggcagct tgcactgatg acctgaccgg cgtggaagtg 1140
   acggatgtcc aagagcgtgg cggtcacggc atcaccgctc aggcggatgg gcacaccatc 1200
   gccgccggta atcagcggct gatggatgcg ctgaacatcg ctgtgccttc tgacctgaat 1260
   ctgaatggta ccactgtttt ggtggcctgt gacggcgtgt atgtgggcgt catcctggtg 1320
   gccgaccagg tgaaggcagg tgcgaaggaa gcgctggcca agctgaaggc agtgggcgtg 1380
   ggcaagaccg tcatgctcac cggcgacggg gatgctccgg ctgccgctgt ggccaaggag 1440
   ctgggtgtgg acgagtacca cagcaagctg ctgccggcgg acaaagtgac gctgctagaa 1500
   cagctgttgg agcaggaacg tccggtggcc tttgtaggcg acggcgtaaa cgacgcaccg 1560
   gtgctcaccc gtgcggatgt gggtatcgct atgggcgcga tgggctcgga cgccgccatt 1620
   gaagcggcgg ac 1632
<210> 409
   <211> 1593
   <212> DNA
   <213> Unknown
<220>
   <223> Unknown gut bacteria
<400> 409
   tgccaggtga gctatcagga ttccactggc acctacaacg gctatatgcg ggaagaacac      60
   gtggacaccg ataacaccga tgccgctgat ctgaccggtg tggtcaagac caagaaaaag     120
   tatatcaaca tccgttccgc taagagcgct gatgccgagg tggtctgcaa ggtgcccgcc     180
   ggcaagagcg tgtccatcat cggtgcgctg gatgaggagg gttggtacca ggtcagctac     240
   aagaatgaca agggtaccca taagggctac atgatggctc agtatatctc catccccagc     300
   atctcctctg gcaccatcgg cacctccagc gccgttctcc gctctgcggc ggacagcagt     360
   tccgatatgt tctgtgtcat ccccgaaggc aacaaagtct ccgtcctgga tgccctgggc     420
   gactggtatc aggtagaata caacgaccag gtgggctatg tggacgcaag ctgcgtggaa     480
   tccgaagccc tgagcaagag caagggctat ggcaccatct ctgttgacac cctcaacgtc     540
   cgtgccaagc ggaacaccga gtccaacgtg gtcaccaaga tgagcaaggg cactaccttc     600
   cgcatcacca gcgtcaagga cggctggtat ggcgtgacct acaacggtgt ggacggctat     660
   gcaaaggcag aatatgtcac cgctaccgac accattgacg ctggttatat ccaggtcact     720
   tctcagacct tgaagctgcg ctccggcgcc ggcactgact atgaccagct ggccatcgtg     780
   ccttatggca ctgtcctgaa catccgcggc tctctgggca gctggtatca ggtgaagtat     840
   aacggcaagg aaggctatgt gtgcggtgat tacacctccg ccaccacctc cggcggttat     900
   aaggcttatc ccgatttcgc caaggtcact gcttctgctc tgggtctgcg tgcaaagccc     960
   ggcaccgaca gcgacaagac cttcaccatc cccaacggca ctgtggtgtc cgtccgcggg    1020
   atgcaggatg gctggtataa ggtcgttgtc tacggcaccc atactggtta tatcgatccg    1080
   gcttacaccg agcgcagctc cggtccggct accgtgcctc aggtcaccac gaccaccact    1140
   ggtacgactg gcaccacgac caccggcacc accggcacca ccggttcctc caccacttcc    1200
   agtgcatcct ctagttcctc cagcagcacc tcctccggtt cctccggcgg cggcagcggc    1260
   gcggcagtcc tggcctatgg acagcggttc gtgggcaacc cctacaagtg gggcggcacc    1320
   agcctgacca acggcgctga ctgctccggc ttcgttcaga gcgtctacgc acacttcgga    1380
   aagagcctgc ctcatagctc ctacgcagac gcacatgtag gccgcggggt cagcaagtcc    1440
   gatatgcgtc ctggtgacat cgttgtttac ggcggtcatg tgggcatcta cgccggcggc    1500
   ggcaagctgc tgagcgcact gggcaagaag tatggcatca cctattgcag cgtcaactac    1560
   aagcccattc aggcggtacg ccgtctgttc taa                                 1593
<210> 410
   <211> 1482
   <212> DNA
   <213> Unknown
<220>
   <223> Unknown gut bacteria
<400> 410
   atgatgcaca agaatcgaaa caaaccggtt tatttgttca gcagctcccc cagcaaccgc      60
   ggggaggaac tgaaacgccg taccaaacgc gcaaaccagc gcactgtgac ctttttcctg     120
   gtctttctct acctgttcac tctgatctcc tttatcattc ccctgcgccc cacctattcg     180
   gatctggaac agcggaacct gactaagttc cccaccccca ccctggccac catcagcaac      240
   ggggagttct tcgagggggt caacacctgg tttgccgacc ccttcccctt ccgggagcag     300
   tttttgaagc tggaaacagg cgtcaccggc ctgtacggcg tcaaaacctc ccaggtggtg     360
   ggcgatgtga agcaggggga cgacattccc gatgccccca tgacgccgga tgacagcgac     420
   agctccgctg acgcctctgg cgatgcgtcc tcctccggtg gtgcttcttc ctctggcgat     480
   gccagcgctt ccacgcctgc tgacagcaat cagaccaagc ccaaggatga cgaaagtgac     540
   gccacgatcc cagagttgga taagaacgcc aaatccgaaa ccctgggcgc cgtactggtc     600
   attgatgacg cagcctatga atacttcaat ttcgatcaga aggccgccaa cagctatatc     660
   aagatgatga acaaggctgc caagaaactc aaaggcaaag ccaacttgta tgacctgatc     720
   gtacccacca gcatggatgt gtgcgtgccg gactccgtcc ggaaaggctt gaacacctcc     780
   agccaagaga aagccatcga gtatatcttt ggcagcatgg gcgatggagt tcattccatc     840
   aaggtgctgg acaccctcat caagcacaat gccgatggcg agtatgtcta cttccgcacc     900
   gaccatcatt ggacggctct gggcgcttat cacgcttatg aacgcttctg cgaagcctcc     960
   ggccagacag cagcttctct ggacagcttt aagaaggtga cctacaagaa cttccacggc    1020
   tccttctatc gggacactaa gtccaccgcc ctaaagaatc atccagataa gattgtcgcc    1080
   tatattccgc cctccaccaa cgacatctcc atcaccaata tggacggctc caagatggat    1140
   tggaatatca tcaccgacgt gagcacctgg aactccaccg ccaagtattc caccttcatc    1200
   ggcagtgaca accccttcag tgtggtcaag aatcccaagg tgaagaatgg caaatccgtc    1260
   atgctcatca aggagtcctt cggcaacgcc tttgccccct tcctggtgga gaactacgag    1320
   aatgtctaca tcgtggacta tcgctacatc aagaatgtag ataagcgttc tctgacccag    1380
   atggtggaca aatacaagat tgatgacgtc ctctttgtca acaacgtcag cgctgtccgc    1440
   aacgaaagcg ccgtgaagct gatgtccgac tttgtgggct ga                       1482
<210> 411
   <211> 1137
   <212> DNA
   <213> Unknown
<220>
   <223> Unknown gut bacteria
<400> 411
   atgaacatga ccgagcgcag aaagcgcatt ctccgtgcgg tagtggagca ttacattgaa      60
   acggccgaac cggtaggttc caagatcatc gctgaccttg cccagttgga ttgttcctct     120
   gccaccatcc gaaacgagat ggcgaccctg gagaagatgg ggtatctgga acagccccac     180
   acctccgccg gacgcatccc ctcccctgct ggctaccggt tctatgtgaa cgagctgatg     240
   gaaaagcaca agctgtccat gcaggagacg gaacggatca acgaggcgat gcatttgaag     300
   atgcgagagt tggatcgggt cattgaccaa gccggcaagc tggtcagcca gttcaccaac     360
   taccctgcct tcgccctcac cgccggtcgg gatcgcatct atatctctcg gtacgacctg     420
   ctgatggtgg atcccaacag ctttatcgtg gtggccatga ctaacaccaa tgtggtcaaa     480
   aacaagctgg tgcatctgcc cagcaacatc acagagccac agctccagct gctcaacgca     540
   ttgctgaaca cctccttcac tggcaagaca ctacaggaaa ttacgccgga cttgatgcag     600
   gtggcagaaa aggcggcagg cagttcctac gggctgatct ccctggtggt ttccttcgcc     660
   atggaggtgc tggatgacct ggaacacagc agcatccaca ccgcaggagc cagtcagctc     720
   ctgaatcacc cggaatacca ggacgtagac aaagcgcata agctgctgac ttatctgtcc     780
   gatgacccaa atttggctca cctaaccgct cccatgatga atgagggcaa ggatgagggg     840
   ccaaaggtgt tcatcggacc agagaatgtg gcagaagagt tgaaggacac cagcgtggtg     900
   ctggccagct acgacatcgg cgacggcatg aaaggcgtca tcggtgtggt ggggcccacc     960
   cgaatggact acgccaaggt gaccgccaag ctgtcctatc tggcggataa cctgggcaag    1020
   ctgttcaatg gaatgtccgg caacgccctg gctccccctg cgcagaaggc attggagctg    1080
   aatccgccct ctgagcttga cccaagtaaa aacgactcca acgatcaagg aggctga       1137
<210> 412
   <211> 1359
   <212> DNA
   <213> Unknown
<220>
   <223> Unknown gut bacteria
<400> 412
   atgacagtaa aagaattgat cgcacgatcc aaggccgcac aggaagcgtt tgcctttgca      60
   acgcaggaac aggcagatgc agctgcaaga gccatctgca aggtcgtata tgacaacgct     120
   gaaaagctgg gccccatggc cgcagaagaa tcccgtatgg gcagcgaaca ggacaagatt     180
   gcaaaatgcc gcaataagtc cgcactgatc tgggagagcc tgaaggacaa gaagtcctgc     240
   gacatcatca accggattga agaacgccgc atgatcgaag tggcaaagcc tgttggcgtg     300
   gtcgcttcca tcatcccctc caccaacccc gtcgtcaccc ccatgagcaa cggcgcattt     360
   gcactgaagt gccgcaactc tgtcatcttt gctccccatc cccgcgcagt caagtgtacc     420
   aagctgctgg tggagatgtt ccgcgcagag ctgaagaagc tgggtctgcc ggaagatctg     480
   gtgctgggcc tggaagaagt ctccaacgac ctgacctccg aactgatgag cgccgctgac     540
   atggtggttg ctaccggcgg catgggcatg gtcaaggcag cttactcctc cggcaagccc     600
   gccatcggtg ttggccaggg caacgtccag tgcatcatcg atgaagatgc tgacatcgct     660
   gacgctgttg gcaagatcat catcggccgc gcattcgaca acggcctgat ctgcctgggc     720
   gaacagaccg tgttcgtacc cgaagataag gaagcagagt ttgtcgctga gatgaagaag     780
   cagggcggct atctggtgga aggcgaagaa aacgtggaca agctgcggga aggtctgttc     840
   cccggcggcg atgcattcag ccgtgtggtc gttggtatgt tcgcagacca ggtttccgat     900
   gagatcggcc tgggcgcacc caagggcacc cgcgttctgt tcgccaaggc caaggctttg     960
   ggcgcagggg atgtgctgtg ccgcgaaaag ctctgcccgg tcatcgctta catgacctac    1020
   aagaccttca aggaaggcgt tgccatgatg gaagccaacc tggagcacga aggcaagggc    1080
   cacagcatcg ctgtccactc caacaccccc gaaaacgtgg aatacgccgc catccgctgc    1140
   tgcgtttccc gtgtggttgt caatcagccc tccggcacca ccggcggcgg cagccccacc    1200
   aacggcttca cccccaccac cacgctgggc tgcggcacct ggggcaacaa cagcttcgct    1260
   ggtaatctga actacgaaca gttcatcaac atcacccgcg ttggctatcc ctacgatgaa    1320
   tcctatctgc ccgataccgc taaggctttc gaagactga                           1359
<210> 413
   <211> 1629
   <212> DNA
   <213> Unknown
<220>
   <223> Unknown gut bacteria
<400> 413
   gcctgccgcg ccctctttgg cggcatccgc ttcagctccc tgcggctcat cgtccgctgt 60
   ccccaggtgc agggcggcag ctggactggg gtcaccctga cccaccggta caagctggaa 120
   tctgaccggc aggagtattt cttccccctg aaaaagctga aatgggacaa agaccactac 180
   atcgccatcg ccagcgtccc cctaaagcag ttcgccttct cccccctcta ttgggacatc 240
   cgcatgacct ttgaaaagga cggcgtccag tactggagct cggtgaaagc ccccttccgc 300
   tccacccagc actattggaa ggatctggcc tggaacgtag gcatcaaggg tctgttcttc 360
   ggggacagtc tccgcctgga caaggacaac cagctctttc tctaccgcac ggtgggcaac 420
   cggttcgccc tggtctatca ggagtactcc ccctacaccg gcttccgctt ccgctgcaag 480
   gaacggctgg ccatggctct gtactacctg cggcggaaat ctctgctgaa gcagagcatc 540
   tacctgacgt acgaaaaata ctgctgtatg gcgcaggaca acgggttcta cttcttccaa 600
   tactgcatgg agcacaacat ggagcaggtc atgaatcgga agatctattt catcatggac 660
   aaaaaggcca ccgactacca aaacctcctg ccctatcggg atcacgtcat ccagttcatg 720
   agcctcaagc acatgatcta catcctggcc gcccgcctgc tcatctccag cgactccaaa 780
   gcccacgcct acgcctggcg tgccaaggag agcgtcatcc tgccccaggt gaccaaaaat 840
   aagcggctgg tgttcctcca gcacggggtc atcgccctga aacgggtgga gttctcccgc 900
   tccggcacca acagcgtcaa cctgttcatc acctccaatc agcgggagca tgacatcatc 960
   atcaacgagc tgggctacct gccggaggaa gtggtcatca ccggcctggc tcgctgggat 1020
   gtgctccgag acaaatccgc ccagcaggaa aagacccaca tcctggtcat gcccacctgg 1080
   cggaattggt tggaggaagt ctccgatgag actttcgtgg ccagcgacta ctatcgcaac 1140
   tatatggcgt tgctcaactc ccatcggctg gctcaattcc tggaacagta cgacgtgtac 1200
   ctggacttct acatccaccc gaaatttcgg gagtacatca agaacttctc catctccggc 1260
   gaccgagtgg cgctcatccc cttcggctcc cagcccctga acgagctgat gatggagtgc 1320
   aagctgctgg tcaccgacta ttccagcgtc tgctgggacg tgtactacca gggcaaaccg 1380
   gtcatcttct accagttcga cctggaacag tacaacgaga cccaaggctc ctacatcgac 1440
   atggagaccg agctgttcgg cgaccgtgcc acggaaccag agcagctgtt cgccctgttg 1500
   gaggagacgg cagccaacgg gttccagctg aaggagaaat acgcccagat gcgggaaagt 1560
   atgtacgcat accttgacca caataatagt cagagaactt gtgacgagat ccaaaaacga 1620
   aattggtga 1629
<210> 414
   <211> 1188
   <212> DNA
   <213> Unknown
<220>
   <223> Unknown gut bacteria
<400> 414
   gtgaatcagg ctttaaatcg tctgttacaa gagaacaaaa ccgcctttta tgtctttgat      60
   atagcgaaac tgaaagcccg catcgcccac ttgcaggcgg cactgccgcc ggaggtggtg     120
   ctgtgctacg cggtgaaggc caacaccttc atcaccaagg agctcatcga cactatccac     180
   cgctttgaag tttgctcccc cggcgaggcg gagatctgcc gtgcagtggg ggtgcccagc     240
   gagaacatgg tcatctccgg cgtgtataag acgccggagg tcatggaggc catggtagct     300
   gacctcgatt tcagccccat ctatacggtg gagtctttgg agcagtacga gctgttccgg     360
   cggctggcgc agcagtatga ccgcaccctg cccctgctgc tgcggctgac caatgggagt     420
   cagtttggca tcaacaaggg ggacatcgaa gccatcgtcg cccggagaga ggagacgccg     480
   gaggttgatt tattgggcat ccagttcttc tccggcaccc aaaagacctc tctgaaaaag     540
   ctgaaacggg aggtggggaa gctggacgac ctgctgaccc tgctggaaga aaaatatggg     600
   tttgttgccc aggagctgga gtacggccct ggctttccgg tgaactactt ccagtcggat     660
   gagctagacg aggcggaact gctggctggc ttctcggagc tgctgcacga catgacccac     720
   caccccaaga tcaccttgga gctggggcgc agcatcgccg cttcctgcgg cacctactac     780
   acccacattg tggacttgaa gcggaaccag gagcagaact acgccctggt ggacggcggg     840
   atgcaccacc tggtgtattt cggccagcac atggccatga agcagccctt tttccatgtg     900
   tgcggcaagg agcatctgcc ggccacagat cagtggaacc tctgcggatc cctgtgcagc     960
   atgaacgatg tgatgaccaa gcaaagcccg ctgcccgact tggcgctggg cgacgtgctc    1020
   gcttttgaaa acgccggagc ctattgcatg accgagggca tctccttgtt cctcagccgt    1080
   gacattccgg cggtctacct gctgttggaa aacggcactg ccgcccaagt gcggaaggca    1140
   tttgatacgg ccaccttgaa tcaaccaaat tacggaaagg atgtatga                 1188
<210> 415
   <211> 1188
   <212> DNA
   <213> Unknown
<220>
   <223> Unknown gut bacteria
<400> 415
   atgaaaacac cctttgtaac caaagatcag ctggaggcga tctgccagca atatcccacc      60
   cccttccacc tctacgatga agctggcatc cgccagaccg cgcgggaagt gaaccaggcg     120
   tttgcctgga acccgggctt tcgggaatat ttcgccgtca aggccacacc gacccccggc     180
   atcctcaagg tgctgaaaga agaaggctgc ggtgtggact gctcctcgct gaccgagctg     240
   atcatgagcg acaagtgcgg tttcaccggc cacgagatca tgttctcctc caatgagact     300
   ccggcggaag aatatgtcca ggcggacaag cagaacgcca tcatcaacct ggatgacttc     360
   acccacatcg acttcctgaa agagaccatc ggttatatcc ccgaaaccat ctcctgccgc     420
   tacaatcctg gcggcgtgtt caccctgggg gagagcgaag aaggcttcca ggttatggat     480
   aaccccggcg aggccaagta cggcttcacc aaggaacaga tcatcgaggg ctacaagaag     540
   ctcaagtcca tgggcgcaaa atatttcggc atccacgcct tcctggcctc caacaccatt     600
   tccgacgact actatcccac cctggctggt gaactgttcg agctggcggt ggaactgaag     660
   gaaaagacag gctgccacat caagttcatc aacctgtccg gcggcgtggg cgtcccctat     720
   cgtccggagc agcgcaagaa cgacatcgca gccatcggcg ctggcgtcaa ggaacagtat     780
   gagaagatcc tggtgcctgc cggcatggat gacgtgagca tctacaccga gatgggtcgt     840
   ttcatgctgg cgccccacgg cctgctggtc acccgtgcca tccatgaaaa gcacatttat     900
   aaggaataca ttggcgtgga cgcctgcgca gccaacctga tgcgtccggc gatctatggc     960
   gcataccatc atattacggt catgggcaag gaaacagaac cttgtgacca caagtacgat    1020
   gtggtcggtt ccctgtgcga gaacagcgat aagtttgcca tcgaccggat gctgcccaag    1080
   atcgacaggg gcgacctgct ggttatccac gacaccggcg cccacggctt cgccatgggg    1140
   tataactaca acggtcgtct gcgcagcgca gagatcttgc tgaaggag                 1188
<210> 416
   <211> 741
   <212> DNA
   <213> Unknown
<220>
   <223> Unknown gut bacteria
<400> 416
   atgaagatat ggggtctcac cgaccgggga acggtcagag agcaaaatca agacaccttc      60
   cacctctgga aggaggacaa cacagccgcc ctcctggtct gcgatggaat gggcggtgca     120
   cgggcaggca atgtggccag cgccctggcg gcacaggcgt tcacggaagc catccagcac      180
   agcgatgccc agccagatca gcggctgacc gcagccctgg cacaggccaa cgagcaggtg      240
   taccagcgtg cccatcgtga tccggactgc cgagggatgg ggacaacgct ggtggctgca      300
   ctggtagagg gcaacctggc tcacatcatc aacgtgggcg acagccgtgc ctactacctg      360
   tcccaggacg gcatccggca gatcaccatc gaccactccc tggtacagga tctgctccaa      420
   cgggggcaca tcaccccaga agaagccagg aaccatccca acaaaaacgt cattacccgt      480
   gcgttgggag tggacaacaa gctggaaggg gatctgttca cccaggagct ggagccgggc      540
   agtatgctgc tgctgtgcag tgatggcctg agcaacgtgc tggaggatga ggaactgttg      600
   gaggtggcac tccacggcgg tacgccggag gaaggctgtc aacggctcat tgggcttgcg      660
   ctggaacggg gcgcaacgga caacgtaacc gcagtgcttt tggcgtggga cgcgccggag      720
   gcaaaggaat ctgaggagta a                                                741
<210> 417
   <211> 1215
   <212> DNA
   <213> Unknown
<220>
   <223> Unknown gut bacteria
<400> 417
   gccaatatgg cgcagaagta tgagaaggtc aacggcgtgg tggactatgc cgaagccctg 60
   gtgggcccca aatacggcta ctacatgggc tggttcatga ccaccatcta ctacccctcc 120
   atgacctcgg ttctggcttg gctcagcgct cgatataccc tggtgttcat caactctgtc 180
   aatccggatt ttaccatgat tatccccgcc tctgagggcg gctgtatcat cggcccggaa 240
   tgtatggcca tgaccatgtt cttcctctgt tgcgcctaca ccatcaacgc cctgtccccc 300
   aagctggcag gcaagatcca gaccacaacc accgtcatca agctcatccc gctggctctg 360
   atggcagtgg tcggcatcat ctatggcctg atgaatggga tgaccgctgc gaactttacg 420
   acgatggcaa atgtaggcga cgccgcctcc ggcaatccct tgctggccgc tgtctgcgcc 480
   acctccttcg cctatgaggg ctggatcatc gccaccgcca tcaacgcaga gctgaaggat 540
   tccaaaaaga acctgcctcg tgccttggtc atcggcggta tcatcatcat cgccgtctac 600
   atcgcctatt acgttggtgt cgccggcggc gctaccaacc aggagctgat cgacaacggt 660
   gcgactgtgg cctttgtcaa catcttcggc aacgtcctgg gcaacatcct gaatctgttc 720
   atcgccatct cctgcatggg caccatgaac ggtctcatgc tgggctgctg ccgtggtatg 780
   tactccgtgg ccgcccgaaa ggaaggccct tgccccgatg tgttcagtca ggtggataag 840
   gcgaccaacc tgcccaataa ttccgctgtc ctgggtttgt tcgtctgcgc cgcttggggg 900
   ctgtacttct tcctggccaa cctggccaac tcccccctgg tgggcaagtt cgtctttgac 960
   tcctccgaga tccccatcgt caccatctat gccatgtacc tgcccatctt tgtcatgtgg 1020
   atgaagaagg cgaaggacga gaacattggc agacggttta tcatccccat cctggccttc 1080
   tgcggcgcag cttttatggt ctacgcctgc atcatcggtc ataagttcgc caatgtctac 1140
   tatctgatcg tctttgcggt catcatggcc atcggcggca tcttccggaa gaacaagaag 1200
   ggggctgcgg tgtga 1215
<210> 418
   <211> 1605
   <212> DNA
   <213> Unknown
<220>
   <223> Unknown gut bacteria
<400> 418
   atgtctgaaa acaagcaacg caaagatacc ttttttggcg gcgcagccat tttggccgct        60
   ggcattatcg tggtgaaggt catcagcgcc gtctacaaga tgcccctggt caacatcctg       120
   ggcagcagcg gctatgcgga cttcaccgcc gccttcaata tttttaacat cctcctgacc       180
   gtctccactg ccggcattcc ggtggccatg tccaagatga tctccgaggc caacgcctcc       240
   cacaggcata agcagaccca caagatcttt cgggtctccc tgttcttctt cggcgcagtg       300
   ggcatcttct gctccttggt catgttcttc ggcgccaact tcttcgccgg catcatgaat       360
   gacaccaaag cagccgaatg tatgcgggtg ctggcgccat cggtggcgct ggtgtctgga       420
   ctgtcggtgt tccgtgggta cgcccagggg tacagcaaca tgaccccctc atcggtgtct       480
   cagatcatcg aggcgctgtt caagctcatc gtcggcttga ccctggcctg gtacttcatc       540
   cgggtgctgg ggaagccgga ctacattggc gcagcaggcg ccatcgctgg cgtcaccgtc       600
   tccgagcttg ccgctctgct ctatatggcc tgggactatc tgcgcaccca gcgccacacc       660
   ccttcgccgg cgccttcgga gcggccggac agcgccaagc ggatcctgaa aacctgtctg       720
   ctgctggcta tccctatcac catcaccgcc gcctccacct ccatcatgac cgctgtggac       780
   aacgccctgg tgctgggtcg gctgcaaaac gccggactgc tgctcagcga ggatgccgcc       840
   cgaagcctga tgggcaacta cactggggtg cagacagtct atcagatccc tgccgccctg       900
   atggtagcca tcaccgcctc cgtcatcccc gccgtgacca tctgcttcac ccagaaggat       960
   cggacgggcg cggccaagat cgttggctcc tcactgaaaa ctgccgctct gctggccttc      1020
   ccctccggca tcgggatgct ggtgctgggc aagcgcatcg tacagctgct cttccccagc      1080
   ctggacgcag acatcgccgg caccattctg tccatcctgg gcatcgccaa catcttcgtc      1140
   tgcctcatgc tggtggcaac ttctgtactg caagcctata acatcctctc cctgcccatt      1200
   ttcaccatgc tggcaggggg cgtggtcatg gtgctctttg actacttcat ggtaggcacg      1260
   gtgcaattca acatctacgg ctccccggta ggcacctgcc tgtgcttcgg tctgacctgc      1320
   gggctggatc tgcacctggt gcgccgtgtg gttcccggct gccccaatta tgcacagctc      1380
   tttggaaaaa ccctggccgc ctctatcatc atgggtgccg ccgcttgggc ggtgtacggg      1440
   ctgaccagcc acctcattgg cgtaaagctg gccgtcctgc tggccatcct gctggcggtg      1500
   gtgctgtatg ctgtactgct ggtactgctc caggtgctaa ccaaggatga tctttccctg      1560
   atgcccaagg gagataagat cgcaaaatta ctgcacatca actga                     1605
<210> 419
   <211> 816
   <212> DNA
   <213> Unknown
<220>
   <223> Unknown gut bacteria
<400> 419
   atgagcaaga gagcatatac tgtgctgccg ccggacagag aggagaaggt gctggggatt      60
   tttgtgaggg acgcatcctt ctaccgcacc ttcttccccc tgctcttggt catcgtcctc     120
   cagcagctgg cggcgctggc ggtgaacttg gcggacaaca tcatgctggg gcgctactcc     180
   gagctggcat tgtctggggc gtccctggtc aatcagctcc agtttaccct ccagcagatc     240
   gccgccggca tcggcatggg catcgtggtg ttagccgccc agtattgggg gcagaagcgg     300
   acggagccca tcaagaagat catctccctg ggggtcaagt gcggcttttt cagcggcgtc      360
   ctcttttttg ccatctctca gctggctccg gcgggagtgc tggggctgtt caccagggat      420
   acggaggtca tcgcagaagg agtgcgctat ctgaaggtca tctgctggac ctatccggtg     480
   ttcggcctgt ccaactccct catgtacgcc ctccagagcg tggagaccgc catgatcggc     540
   acggtcatgt cctgctccac catctgcatc aacgtctgcc tgaactactg tctcatctac     600
   ggcaacttcg gcgcgccgga gctgggcatc gtaggtgctg ccgtggccac cctggtcagc     660
   cggttggtgg agctgggcat catcctggtg tatatcctgt tccgcgacaa aaagctccac     720
   atgaagctgt gggagctttt gcggtgggac ggcacctatt tgccggatta tttgaaggtc     780
   tccaccccca tcgtcatctc cggcctgctc tggggg                               816
<210> 420
   <211> 1194
   <212> DNA
   <213> Unknown
<220>
   <223> Unknown gut bacteria
<400> 420
   atgaagaaaa aactgctgat cttaggcgct ggtatttatc aggtgccgct gatcgagaca      60
   gccaagcgga tgggcttgga gaccatcgtg gccagcatcc ccggcaacta tccaggcttt 120
   gcgctggcag acaaagtggt ctatgagaac acggtggaca aggaagccat gctggcgctg 180
   gccaaggcag agcaggtggc tggggtctgc gtgtgcggca ccgatgtttg cgtacctgcc 240
   caggggtatg tgtgcgacca gttggggctg aaaggcccca gcgaggcggc ggcagagcgt 300
   gcccaggata aggcgctgat gaaggcggcc tttgagaagg gcggcgtgcg gacggcgcgg 360
   ttcatccatg tggatatgga taacgctaac ccagtggacg tttgtcagga gattggctat 420
   ccggtcatct tcaagagtgt ggactcctct ggcagtcggg gtatcacccg agtcaatgga 480
   ccagaggaca tcgcctacgc ctatgaacag gtgaaggaga acacccatgc tgaccactac 540
   ctgatcgagg agtttctcgt gggtgaggag ttcggcgccc aggcgtttgt gctggatggg 600
   gaagtgcagt ttatcctgcc ccacggagac tatgtgttcc agggggatac tggggtgccc 660
   attgggcatt ttgcccccta tgacatgggg gcagacatcg tggcggatgc agaggaacag 720
   ctgcgccgag cggtaaagag cctggaggtg gacaactgcg ccatcaatgc agacttcatc 780
   ttgtgcaagg gcaagaccta tgtgctggag atcggcgcac gggcgggtgc tacctgtctg 840
   gtggagatga ccagcttgta ctatggctat gactactatg agaagatcat ccagtgcaac 900
   ctgggggaaa agccagactt tacgcctcag gcagcgcctc agcccaacgc cgaaatgctg 960
   ttccaggcgc cggtgacggg gaagatcacg gagattgact tgagcggcgt gacgccggat 1020
   ccgcacatca tcaacctgtc catggattac caggtgggtg atgctgtgcg gcagttccgc 1080
   aaggggccgg atcgcatcgg acagatcatc gccattgggg acagtgtgtc tcaggcaaag 1140
   gaacggatcg accgagcgat ggcgcaggtg aagattacca tcgacccgga ataa 1194
<210> 421
   <211> 1875
   <212> DNA
   <213> Unknown
<220>
   <223> Unknown gut bacteria
<400> 421
   ggccgtaaga gcgccgcagt ccgcaacacc atctcattta tcatcacggg cggggctatg      60
   gttctcatgt tcctgctcat caagccgatc atgatggatg gcgaagtcat tgcttattgg     120
   ctgggcgact gggcaccggt gtccggctgg gccatcggta tcggcatcga agtggacgcg     180
   ctgagcctgt tcttcggcct gatcgtcacg gtcgccgtgt ttgcctcctg catctatgcc     240
   ttcacctata tggcacacga tgacagccag gccaactact tcagcctgtt cctcatgctc     300
   tccggctccg tgctgggtct ggtgctctct ggcgacttgt tcaacatctt cgtcatgatc     360
   gagatcatga ccttctccgc cgtggcgctg accgcattcc ggaacgacta tgaaggcgcc     420
   atcgaaggtg cgttcaagta tctgaccgtc ggctccctgg gttccactgc tgtcctggtg     480
   gggattacca tcctctacaa ccagctgcac accctgaacc tggctcagat ctctgccatg 540
   ctggcggaaa agggcaacac ccccatcctg atggtggcct tcggcctgct attcatcggc 600
   ttcggcacca aggcgttcat cttccccttc caccccctgg cagccgatgc ccacgctgtg 660
   gcacctgctt ccatctccct gatgatctcc ggcgtcctca ccaagtgcgg cgtctatggc 720
   atcatccgtc tggtctatgc gctgttccag aacatccagg ttcccgcagt gcagtatctg 780
   gtggtcggct ttggcaccat cagtatgttc gtctgcgtca ccatggcgtt caatcagcat 840
   aacttcaagc ggctgctggc cttccattcc atctctcagg tgggctacgt catcaccgct 900
   ctgggtctgg gcactgccct gggtctcagc tccggcctgt tccacgccat gaaccacacc 960
   ctgttcaagg gcctgctgtt cctgactgct ggcgctgtcc agcacgccac cggcagcctg 1020
   gatctggata agctgggtgg tctggcaaag aagatgcccc gcacctgcat cctgttcctc 1080
   atcggcgccg cttccatcag cggcctgccc cccttcaacg gctttgcctc caagtggtac 1140
   atctaccagg cagcctttga aaagggcgct gagagcaaca acttcttctt catctttgtc 1200
   tgcatcgttg cgctgatcac ctccgtgctg accctggctt ccttcatcaa ggtctcccag 1260
   agcgttttct tcggccagct caaccccgaa ttcaaggatg cgaaagaagt ttccctgggt 1320
   atgcgcatcc ccatgtggat tctggccgtc ctgtgtatcc tcaccggcct gctgcccggc 1380
   aaagtcaccg attacctcct cgcacctgca accgccgcag cttccaacgc tgtgaactat 1440
   gtggataaga tgatgggtga cggctacgca gccgctcacg gcgtcaccaa caccctgggc 1500
   gacaccgcca tcaacttcgc cggcacttgg gatcccatcg tttggctgct cctgctgttc 1560
   tgcgtcactt gcgctgtggc tctggtcgca atcctgggct ccaacaagaa ggtcggcacc 1620
   ctggaaatgc cggctgagat cgcacaggca gatcaggttg ctccggctga cgccaagtat 1680
   gaacccttct tctccggtga agaaagcgtt tattctcagg ttggcggctc cgacctgttc 1740
   tggggcttca agcataacct gaaaaagtac ttcagctata tgcatgactg gcacagcggt 1800
   atcgttaacg actacgctct gtacggcgtg gtggcgatcg cctttgtcct ggtcttctgc 1860
   gtcgcgtttc tgtaa 1875
<210> 422
   <211> 1485
   <212> DNA
   <213> Unknown
<220>
   <223> Unknown gut bacteria
<400> 422
   aaccccgcca ccgcctttga gacgaatgag atcttggcca tctcggacga gaagctggaa 60
   cagttttatc aggatgttcc ggcgctggag ggctatcgcc gtcacctgga tgagatccgc 120
   cgccgccgtg cccacatcct ctctccggcg gaggaacggc tgctggccat ggctggggag 180
   ctgtctgaca atcccgacaa tatcttctcc atgctcaatg acgccgacct gacctttcct 240
   gacgccattg acgcccaggg ggcggcgcac catgtgaccc atggttccta tatccccatg 300
   atgtacggcg ccgatcgcac cctgcgcaaa tccgcgttcg agtccctgta cggggtctac 360
   gagcgccaca agaacaccct ggccgccacc ctggccgccc aggccaaaca gctgtggttc 420
   ttcgcccagg cacggaacta tccctccgcc ctggccgctg ccctggatcg gacggaggtg 480
   ccggagagcg tctatcacag cctgatcgac gcagtgcggg agaatctgcc caagctgcac 540
   cggtacacca gcctgcgcaa gaagctgctg gggtggaggg agctgcattt ctacgacctg     600
   tacgccaata tggtggggga tgtggagatg aagttcacct ttgcggaagc caaggagctg     660
   gtgctcaagg cgctggcgcc tttgagcgag aactatctga acggtctgcg ggcgggcttt 720
   gaaggccggt ggatcgacgt gtacgagaac gtgggcaagc gctctggcgc ttacagctcc 780
   ggcgctcgcc cccacccctt tgtgctgctc aacttccagg gcaccctcaa cgatgtattc 840
   accctggcac acgagatggg gcacgctatg cactctgaaa tgtccaacgt gaaccagccg 900
   gtggtctatt ccgattacgt tatctttgta gccgaggtgg cttccacctg taatgaagcc 960
   ctgctgatgg aatacctgct caaccacacc accgacaagc gggaaaaggc ttatctcatc 1020
   aactacttcc tggaacagtt ccgcaccacc ctgtatcgtc agaccatgtt tgcggaattc 1080
   gagttgcaga tcaatgagct ggcacagcag ggacagggca tcaccgccga ccggtgctgt 1140
   gagatctatc ggaagctcaa cgaggactac tttggcaagg acatcgtggt ggatgaccag 1200
   attgccctgg agtgggcgcg catccctcac ttctactaca actactatgt ctaccagtac 1260
   gccaccggct acgccgctgc cattgccatc agccgccgta tcctgaagga aggccagccg 1320
   gctgtggacg attatttcaa gttcctgtcc ggcggctgct ccatgccgcc catcgagctg 1380
   ctgaagctgg cgggcgtgga tctgtccact ccggcaccca tccaccaggc gctggagctg 1440
   tttgaccagc tcatcgacca gttggatgag ctgttggcgg aataa 1485
<210> 423
   <211> 852
   <212> DNA
   <213> Unknown
<220>
   <223> Unknown gut bacteria
<400> 423
   atgaagagga ataagctgtt ctccatcccc tatatcatct ggatggctct gtttgtagta        60
   gcgcccatcg tcctggtggt gctctacgcc ttcactgccg ccgatggcgg cttcaccttt       120
   gccaacttca cccgaatggg caattacttt ggggtctttg tccgcagctt ctggctggca       180
   ctcatcgcca ctgccatctg cctggtcatt ggctacccca tcgcctatct catctccaag       240
   gagagcaagc ggttccagcg catcagcatg gcgctgatca tgctgcccat gtggatgaac       300
   ttcctgctgc gtacctacgc ttggatgtcc attttggaga ataccggtct gctcaaccgc       360
   ttttttagcg ccattggcct catcgacctg gtcaacggca tcctgggcac caacatgacc       420
   cacttcaaca tgatcaacac cccaggcgct gtggtgttgg gcatggtgta taatttcctg       480
   cccttcatga tcctgcccat ccactcggtc attcaaaaaa ttgaccggaa cctcatcgag       540
   gccgcgcagg atctgggttc caacaactac cacgtcttta ccaaagtcat cttcccgctg       600
   agcctgcctg gcgtcctgtc cggcatcacc atggtgttcg tgcctgccgt gagcaccttt       660
   gccatctcca agctgctggg gggcagcaag gatttattgc tgggcgacct catcgagatt       720
   caattcctag gctctgccta caatccccat ctgggctccg ccatcagcct ggtcatgatg       780
   ctcattgttg tgatctgcat ggcggtcatg aaccggttcg gtgaagggga agaaacggcg       840
   gttatgctat ga                                                           852
<210> 424
   <211> 729
   <212> DNA
   <213> Unknown
<220>
   <223> Unknown gut bacteria
<400> 424
   ttgagtgctt ggtttgcaga agtgcaacag aaatttattg cggactttat cgaaaaagac        60
   cgttggactt acatcactga cggcctgaag atcaccatca tcgtcaccct gctggcgttg       120
   atcgttggcc tgatcctggg gatcctcatc gccatggtgc gctgtaccca cgaccagacc       180
   cgcccgaagt ggttccgttc gcccggcaac ttcctgctga agctggctga cgccatctgt       240
   cggctctaca tcaccgttat ccgcggcacg cccaccttgg tgcagctgct gatcatcaac       300
   ttcgtcatcc tggtctccgc ccagaaaatc accgttgccg tcatcacctt cggcatcaac       360
   tccggcgcct atgtggccga gatcatccga gggggcatca tgggcgtgga caagggacag       420
   atggaggctg gacgctccct gggcatgagc tatgtgacca ccatgaagga catcatcctg       480
   ccccaggcca tgaaaagcgc cctgcccgcc ctgatcaacg agatgatcac cctgctgaaa       540
   gagacgtcca tctgcggcta catcggcctg aatgagctga cccgcggcgg cgacatcatc       600
   cgtggtatca ccttcgatgc ccttttcccc ctgttcactg tggcgctgat ctatctcgtc       660
   atggtgctct ttatccagtg gatcatgggc aaagtggaaa ggaggctgcg cagcagtgat       720
   tatcgttaa                                                               729
<210> 425
   <211> 1218
   <212> DNA
   <213> Unknown
<220>
   <223> Unknown gut bacteria
<400> 425
   atgaagcgaa aacccattgc actgttactg atcctctcca tgctgctggc cttgtccctg 60
   accggctgcg gcaaggacac cgccctgacg gtctacgccg aaggcaattt catccgtcag 120
   gatctcatcg acgccttcac ggaggagacg ggcatcgagg tcaactacat tatcggcacc 180
   cgctctccgg cagagaccaa ggccaagacc gatgatgcca acagcaccaa gaccgattcc 240
   agtgcggacg aagaagtggt gctgccggaa gaatcctcgg tggacgtcct gtccgaagtt 300
   cgggcgtgga aggaggagga cacccaggcg cgggcggaaa gctcggaatc tgatggcgcc 360
   gtctgcgatt atgacgtgat cctcaccgac caggacatcc tctcccagct gaaggaggag 420
   gattgcctga tggagctgga caccaagaag gtgtccaact atgacaacat cgccaagaaa 480
   tatcgcaagc tcaacggaga tggcgctctg tacggtatcc ccgccctgtg gggcatcgcc 540
   ggcatcgtct gggacaccaa gctggtggat acccaagtga gcagctggga cattttgtgg 600
   gatgagactt acaagggcaa aatcgtcatg cccgctaccg cgcgggaatg tgccgctgtg 660
   gcgctgaagc gccagggcaa ggacgtgaac accacggaca aaaaggagct ggaggacgcc 720
   tacaagctgc tggatgaaca gaaggatctg gctgccggat acaccagccg agtggcctac 780
   accatgatga ccaaccaatc cgccgccctg gctgtggctg actccggtgc cgccattgac 840
   atgatgagca acaatgcaaa ccttgccttc gccctgcctg acgagggcag ctggcgggtg 900
   tgctactcct attgtgtccc cgctgggacg gagtggccgg aggaagcgca gaagttcatc 960
   aactatatgt gctccgccaa caacctggcc aagaacgccg tgtacagcaa atactccacc 1020
   acctctgggg aagcgctgaa gaagatgaac cagagctggc acgacaatcc gctggcctat 1080
   ccggacgaga ccatccgcaa gcagtccccc ttcctcaccg gcctgcccgc tgaaaccgag 1140
   acccttcacg gctccctgtt cgcccagctc accggcgcgg acacagccgc agacaacacc 1200
   aacaaccctc agtcctga 1218
<210> 426
   <211> 2283
   <212> **DNA**
   <213> Unknown
<220>
   <223> Unknown gut bacteria
<400> 426
   atgctccgcc gcgcgctgtt ccttcttacc gcgtgcggca ttgcggcgtt tgccgtattg 60
   atgctccagc ttttccggct gcaaatcctg cgccacgacg agctggagag cgctgccttg 120
   cagcagcagc tccgccgcac ggcgatcccc gcctcgcgcg ggacgatcta tgaccgcgag 180
   gggcgcgtgc ttgcgatgag cgcgacaacg tataccgtgt acgtgagccc cgcggagata 240
   tccatgaacg gcgaggacgc cgagcgcatt gcgtccggtc tttccgagat cctcggcgta 300
   gaccgcgaaa agatcctcac gatgaccgcc gaccggcgca gctggtacaa aactgtcaag 360
   cgccagatcg aggaagccga ggcggacgcg gtgcgcgcct ttaaaaacga gtacgacctg 420
   cagggcgtga agctcgagcc ggataccaag cgctattacc cgtatgcttc gctcgcctcg 480
   catgtcatcg gcttcgtcgg cgcggacaat accggccttt cgggactgga gtttacgcag 540
   aacgagctcc tgaccggaac cgccggaagc atcctccggc tgaagaacag cgccggaacg 600
   gatatgatcc tcacaagcta tgaggattac gtcgatgcgc aggacggact caatctccac 660
   ttgacaatcg ataccaccgt ccagtactat ctggagaagc agctttccca ggcggtgcag 720
   gactttgata tacaaaacgg cgccgccggc atcatcatgg acccgaatac gggcgcaatt 780
   ttggcgatgg cgtcgctcgg aaactttgat ctcaacaact accagcgcgt ctccgccgac 840
   atccaatcgg agatcgattc gaccgccgat ccggacaccg cggcggagat gttgaaagac 900
   gcgcagcgtg cccagtggcg caacaaggca atttccgata cctacgagcc gggctccaca 960
   tttaagatca tcacgcttgc catcgcgctc gaagagggcg ttgccgcgcc gtccgacagc 1020
   ttttactgcg gcggcagcat gacggtgcag ggacgcggca agccgctcaa gtgttggaaa 1080
   acgatcggtc acggtcccca gacacttacg caggcggcgc agcattcatg caacgtagca 1140
   tttgccacgc tggggctccg catcggcgag gaaacgttct accgctactg cgagagcttc 1200
   ggctttttcc gcgcgagcga aaatccggac gcttctctca cggggaagac cggcatttcg 1260
   cttccgggcg agagcggcag catctggtgg ccgcgcagcg tcttctgcaa cagcgaaaac 1320
   ttttcgcagc ttgcggcggc gtcgttcggc cagacgttca acatcacgcc gctccagctc 1380
   atcacggctg tgagcgcctg ctgcaacggc gggtatctta tgaagccgta catcgtcgat 1440
   tccgtcacgg acggcaccgg cgcgcttata tcgaaaaccg agccggaggt gctccggcag 1500
   gtcatttccg agaagacgag cgcgcaggtg aacgctgttc tggagcaggt cgtgtgcgat 1560
   acgaagcagg gcaccggcaa aaacgcctat gttgccgggt atcacatcgc cggcaagacc 1620
   ggcacctccg aaaaggtggc gcaggacgcc gccggcggaa aaaaggaata catcgtatcc 1680
   ttcatcggct acgcgcccgc ggacgatccg caggtcgtgt gcctcgtttt gatggatacg 1740
   ccctcaaacg agagcggtat ctatatctcc ggcgggcaga tggccgcgcc cgtcgtcggg 1800
   cgcatcctcg gcgaagtgct gccgtatctc ggcgtacagc cgcaatacag cgaagcggag 1860
   gaaaaataca tcgaccgcgc cgtgccgccg ctcaccggca aaacgccgga ggaagcggta 1920
   aagctgctcc gcgaggcagg gctcgccgcc cggatcgaag tgaccggcga cattgtcacc 1980
   ggccagctgc cggggaaggg aacggtcgtt gcgagcggca caacggtgct catctatacc 2040
   ggtgagatac cggaggcaga agaggagacc gtgccggagc tcacggggct cacctatacc 2100
   gaggcgcggg aagcgctctt ttcacgaagg ctgttcctcc gctcggacag cacgatcctc 2160
   gccgacagcg atacggtgcg cgttgtgttc cagagcaccg gcgcggggga gagcgtcccc 2220
   acgggcagcg taattgaggt cagcatcgtc aacgacgata acgatacata cggccggtat 2280
   tga 2283
<210> 427
   <211> 1743
   <212> DNA
   <213> Unknown
<220>
   <223> Unknown gut bacteria
<400> 427
   atgaaacgaa ccaataaagc aaacggcatc cgctccgcgg tgccgcgcct tacgggctat 60
   atgcgctcaa gcatgggcgt catcatcctg gcgctgatcc tcgccgcgct cagcgcggtg 120
   atgacgatcc ttgggccgaa ccagatcggc cggatcgcca cgctgatgtc gaacggcctc 180
   accggaaaca tcgatctcgc ggcgatcgcc aaagtcggca ttttccttgc tgtcatctat 240
   gtgctcagtg cagttttcag ctttatccag cactacacga tgtctgtcgt cacgctgaaa 300
   atgtcctacc gcatgcgcgg cgagctttcc gccaagatca accgcgtgcc gcagaagtat 360
   ttcggcacga cctcgcaggg cgacattctc agccgcatca caaacgacgt ttccaccctg 420
   cagcagggac tcacgaacag cctgcccacg atcatcagcg cggccacgca gtttgtcggc 480
   tgtctcctta tgatgttcgt caccgagtgg cggctcgcgc tcgtttccat cggcgtgacg 540
   atcctcggca tgctgctgac ggtgctgatt ctgtccagat cgcagcggta tttcgccgcg 600
   cggcagaaaa gtctcggcgc gctgaacggg tatgtggagg aaatgtactc cgggcatgaa 660
   gtcgtgcggc tcagccgcgc cgggcggaag atcggcgcgc agttcgatac gctcaacgcc 720
   gccgtttacg acgcgaactg gagatcgcag tttctctccg gcatgatgca gccgcttatg 780
   aacattgtcg gcaacatcgc ctatgtcgcg gtgtgcgttc tcggctcgat tctcgcgatg 840
   aacggcacga tcgagttcgg cgtgatcgtc tcgttcattc tgtatgtccg gcttttcacc 900
   tcgccgctga cgcagatggt gcagggcatg acgaacatgc agaccgcctc cgcctccgcg 960
   caccgcatct tcgattttct cgaaagcgag gagatgcccg acgagagcga aaagaccgcg 1020
   aagctcaccg atgtgcgcgg cagggtctgc tttgaccatg tccggttcag ctatccgagt 1080
   gacccggaca agatcatcat ccgggacttt tccgctgagg tgctccccgg acagaaagtc 1140
   gccatcgtcg gcccgaccgg cgcgggcaag acaacgatgg tcaatcttct catgcggttc 1200
   tttgagatca acagcggcag tataacgatc gacggcgtac ccatctccga aatgcgccgg 1260
   gaggatgtac acggtctctt cagcatggtt ttgcaggaca cctggctctt tgagggaacg 1320
   gtgcgggaga atcttgtgta caacatggag ggcatcaccg acgaacagct cgaagcggtc 1380
   tgccgcgcgt gcggactcga caagctcatc cattcgctgc cgcaggggtt tgatacggtg 1440
   ctctccgaga gcgccggcat ttccgccggg cagaagcagc ttctcaccat tgcccgcgct 1500
   atgctgcaaa atgcgcccat gctgatcctc gacgaggcga cctcctccgt cgatacccgc 1560
   acggaagttt tgatccagcg ggccatggac gagctcacga aggggcgcac cagctttgtg 1620
   atcgcgcacc gcctgtccac gatcaaaaac gcggacaaga tcctcgtcct gcgcgacggc 1680
   gacgtggtcg aaagcggcac gcacgaagag ctcatgcagc gcggcggctt ctacgccgag 1740
   ctc 1743
<210> 428
   <211> 1362
   <212> DNA
   <213> Unknown
<220>
   <223> Unknown gut bacteria
<400> 428
   atgcaaactt ttttgaagaa cggctatatc gtctccatgg atcaaaacga caccgtgttt      60
   gacggcggcg gggtgctcgt ggaaaacgac cgcatcacgg cggtcggccg ggtcgatccg     120
   gcgaagatct cgcccgacgc ggaggtcatc gatctgcgcg gaaaatatgt tctgcccgga     180
   tttgtgaata cccacgtcca cacctcgcag cagatatccc gtggtgtcgg cgacgatgtg     240
   gattttatct gctggctgca cgagagaatg tggccctttg agagcaatat gacggaggag     300
   gattcctacg tttccacact catgacgagc ctggagctga tccgctcggg cgtaacgtcg     360
   tttgcagagc cgggcggtca gttcgtctcc ggcatggcgc gggccacgga gcagtccggc     420
   ctgcgcggaa agctcgccaa gtcggtgatg gactgcggtg aaggcctccc gccgatctgg     480
   cagcgcacga tggagcagga gcttgagcag caggtcgcgg atatcgaaaa atatcacaat     540
   accgctgacg ggcgcgtgca ggtatggttt ggcctgcgga cgatattcaa caacaccgat     600
   gagctttgcg tccgcacaaa agagcttgcc gacaagtacg gcgtcggcat tcacatgcac     660
   gtggcggaag caaaggaaga aaaggaatac acctatgcgc gctggggcga ggggacggta     720
   aagcacctcg aacggctcgg tgtgctggat aaaaatctcc tggcagtaca cacggtctgg     780
   ctgaccgatg aagagctgga gctcttccaa aagcgggaag tgaagatctc gcacaatccc     840
   gcgtcggcca tgcgggtgct gggttttgcc cgcgttccga aaatgctgca gatgggactg     900
   atgccgtcca tcggcacaga cggcgcttcg tcctccaatc acatggacat ggtggatgaa     960
   atgtggctca ccagccttat tcataagggg tggcggctcg atccgacggt ggtcccgtca    1020
   caggatatac tgcgcatggc gacgaagtgg ggcgcgcgtg cgcttctcga tgaaaatctg    1080
   tatggcagtc tggaagccgg aaaaaaggcc gatttgatcg ttattgatcc gcacggacca    1140
   tccatgatgc cggtcaacga taaaattgcc gcgctggtca cggcgatgca ttcggccaat    1200
   atcgaatcca cgatgtgcga cggcaagtgg ctcatgcgtg aaagaaaaat cctgacgctt    1260
   gatgaggagg caattttgaa agaggcttgt gaacgggccg cggccattta taagcgtgcg    1320
   ggcatcgttc tgccggaccg ttttccggtc gtaaaagtct aa                       1362
<210> 429
   <211> 630
   <212> DNA
   <213> Unknown
<220>
   <223> Unknown gut bacteria
<400> 429
   atcggcagcc acgcctgcgt agagcttctg aacgccggac attcggtcgt tgtggtggac 60
   aatctcgtca acgccaacgc ggagtgcctg cggcgcgtcg agcgcatcac cggaaagacg 120
   ctcgaatacc acgaaatgga tcttttgaac cgcgcagctc tcaatgagct tttcgatacg 180
   caccggtttg actgcgtgct ccactttgcc ggtctcaagg cggtagggga gtcggtgcag 240
   cagccgctgc ggtactacaa caacaacctc ggcacgacca tccatctctg ccagacgatg 300
   gcggcgcacg gcgtgaagaa gatcctcttc tcctcgtccg cgacggtgta ttcgccggac 360
   aatacgatgc ccgtggacga gagcgcccgc accggcggct gcacgaatcc ctacggctgg 420
   acgaagtaca tgagcgagca gatcctctcc gatacggcga aggccgacgg cgaatggacc 480
   gttatgctgc tgcggtactt caaccccgtc ggcgcgcatc cgagcggact catcggcgag 540
   gatccgcgcg gcatcccgaa caatctgatg ccgtacatcg cccagaccgc cgccggacgg 600
   cgcgagtatc tgagcgtatt cggcaacgac 630
<210> 430
   <211> 1644
   <212> DNA
   <213> Unknown
<220>
   <223> Unknown gut bacteria
<400> 430
   atgagatctt ccaaaacccg catatggtgg gtcatcgcgg cgctcgtgct cttgctcgcg 60
   gctgccgccg tatggttttt ctgctcgtac agcgtctccg acgggcggct gatcgcccga 120
   aaggcggacg tgatcgatct tcgcggtgag gacatctccg cgcaggaagc cgaagcgctc 180
   gccgcgaagt accccgataa gaccgttctc tgggatgtgc cgctctccgg cggcagccgt 240
   ccgagcagcg cggaaagtct gagcctctct tcgctctcgg cggacgacat cccgctcttt 300
   gcctgctttc cgaatttgaa aagcatcgat gcgtccggct gcgcggatta tgaggcgctg 360
   ctcgcgctgc gaagcgccta tccggcgctt gatctgaccg cgttcgtgac cgtcggcggc 420
   aaggccgtgc cgatggacgc gcagcgcgcc gtgctgcgcg ccgatacgcc gcttgcggag 480
   attctggaaa cactgccctt tcttcccgag ctttacaggg tatcctttac gggcggcgcg 540
   gtatcctatg aaatgcagga tgcgctcacc gcagcgtttc cggcgctttc cttccgctgg 600
   gacgtggaag tgctcggcgt gaccgcttcc tccgcggcaa aggagctctc ctttgccggg 660
   caggcgctca ccgcgcagga cgtgaacacg ctctgcgata accttttccg tttcccggct 720
   ctcgagcgca tcgacctcac cggctgcggc cttacgccgg agcagatcaa agccgtccgc 780
   tccgcgagcg gcgccgttac cgtatacgag ctggagcttt tcggcaagac cgtttcgacg 840
   gacgctctcg agctggactt cagcggcata cgcatgggac tttccggcgc agccgtcatt 900
   gaggaagcgc tcggcgagtt tccgctgctt gaaaaggtcg tgctgtgcga ctgcggcata 960
   tccaacgagg acatggccgc gctcaacgac cggcatgaga atgtccggtt cgtctggcgc 1020
   gtatactttt ccgaattctc cctgcgcacg gacgatacga acttcatcgc cgcgcgcgtg 1080
   cgcaacgagt ttcccatcta cagctacgaa ctggaagtgc tcaaatactg ccccgacctg 1140
   caggcgcttg atctcggcca taaaaacatc acgagcctgg actttctccg ctacgtcccg 1200
   catctcaagt atctcatcat cgtcgaaaac gacgtgaacg acatcacgcc catcggcgag 1260
   ctgcaggagc tcacctacct tgaaatgttc tggacgaagt gcgaggatat ctccccgctg 1320
   caaaactgca aggcgctcac cgacctgaac atttcctatg tctactgccg ccccgccaaa 1380
   tgccttgaaa cgcttgtcaa catgccgcag ctcgaacggc tgtggtactg cggcaacaat 1440
   ctcaataccg aacagctcga agagctgcaa acggcgctgc cggacaccga aatgtatctc 1500
   gccgcgcgcg gcgagccgac cggcagcacc tggcgcgagc acccgcatta ctttgaaatg 1560
   cgcgactttt tcggcatgta ctacatggcc ggcggcacga acggcgtgga cgaaaacggc 1620
   aaccagatca ttatcaccgg gtga 1644
<210> 431
   <211> 1443
   <212> DNA
   <213> Unknown
<220>
   <223> Unknown gut bacteria
<400> 431
   atgcgcgtgc tcggcacgct gcttgagggc agcggaaaat acttcgccgt gtgcatcctt 60
   ctgggactgc tgctcacggt ggttgaaatg atcgcgccgc aggtcatccg cgtaacggtg 120
   gactcggtga tcgactcgca gccgatggat ctgcccgcgt gggcgctccg gtgggtggag 180
   tcgctcggcg gcgtggcgtt tctgcgcgcg aacatgtggt tcatcgcgct cattcttgcg 240
   cttgcgggtc tcgtcgcggc gctgctgcgc tatggcgcga acatgttcaa cgccaaagcg 300
   ggcgaaacgc tcgtcaagac cgcgcgcgac aggctcttcc accacattga atgcctgccg 360
   tggaagtggc acgccgagca tccgaccggc gatatcatcc agcgctgcac gagcgacgtc 420
   gagcgcatta aaaacttttt ccaggagcag ttcatctccg ttttccagat catcgtgcgc 480
   attatccttg cgctgacgct tatgacgctg atgaacgcga agctctccat cgtcgcgtgg 540
   gtcatcgcgc cgatcatcgt gctctactcc gtgctcttcc ataacaagat ccgcgcgcgc 600
   tttaccgagt gcgacgagaa cgagggcgtt ctctccacga tcgcgcagga aaacctcaca 660
   ggcgtgcgcg tcgtgcgcgc gttcgggtgc gaaaagtttg agcgcgaccg gttcgaggcg 720
   cagaacaaca agtataccga gtgctggatc agcctgaccc ggacgctctc cgatttctgg 780
   gcggtgggcg attttacgag ctgcctgcag atcctgctcg tggtcgtgct cggcacggtc 840
   tcgtgcgtga acggcaatct gacggcgggc gaatttgtgt cgttctcgct ttataacacg 900
   atgctcatcg gccctgtgcg ccgtctcggc cgtatcattt ccgaaatgag caaggcgggc 960
   gtctccatcg accgtctcgg cgaggttttg gaagccgaga ccgagcagga cgcgcccaac 1020
   gccgccgaag cgccgatgga caaggacatc gcctttgaaa acgtcagctt ccattacggc 1080
   gaggacggcc cggacgtgct cgaaaacgtc agcttcaccg tacccgccgg aaagagcttc 1140
   ggcattctcg gcggcaccgg ctccggcaag agcagcctgc tgcttctgct ctgccgtctg 1200
   tacgcgccca cggagggcaa gatcaccgtc ggcggcgtgg acattgccga tatgcctgcg 1260
   cgttatgtgc gcgagaacgt cggcgtagtg ctgcaggagc cgttcctctt ctcccgctcg 1320
   gtgcaggaga acatcggcat ctgcggcgcg agcgacgcgg agatccgtgc cgccgctgtt 1380
   accgcctgcg tggacgggga tatcacccgt ttttcgcagg gctacgacac catggtcggt 1440
   gag 1443
<210> 432
   <211> 1344
   <212> DNA
   <213> Unknown
<220>
   <223> Unknown gut bacteria
<400> 432
   atgctggagc atctgcctct tatcgtgttt ctggtcacat tcgtgctcat attcgtgctg 60
   cgtatgccga tccctgcggg catgatcgcc gcatgtacgc tgtacttcct gaccgacggt 120
   cttatcaagg gcgtaggctt cggcgcgctc atcaccggcg cgggcggcag cctgcatctc 180
   gtggcgcaga agaccatcga agcgatattt accaacggca ccatcgtcgc ggtaccattg 240
   tttatctttg ccgcgaacgt aatgaacgcc ggtaagatca ccgagtacgt tttcggcacc 300
   tgcatgggtc tcgtcggccg cttccgcggc ggtctggcgc acgtcaacgt cctcgcctct 360
   ctcgttttct ccggcatgac cggctccgcc atcgccgacg cctccggtct cggcaagctc 420
   gagatcgacg ccatgcgcaa agagggctac gatgacggct tctcctgcgc catcacggcg 480
   gccagtgcca ccatcggccc gatcttcccg ccgtccatcc ccatggtcgt ttacgcgatg 540
   ctcacgggcg cctccgtcgg cgcgctgttc atggcgggca tggtgcccgg cgtgctgctc 600
   gccgtcgcgc tgtgcgttta tgtgtacttc gtcgccaaaa agagaaacta cccccgcacc 660
   gcgcccccga agctgcgcga ctatctcctg tatctgacga agtccatctg ggcgatgctc 720
   accccggtca tcctgctcgt cggcatctat accggcatca tgaccccgac cgaggccggc 780
   gccatcgccg gactgtacgc gcttctcgtc tccttcctga tctaccggct catgtcctgg 840
   aggacgctca agcaggtcct cgtagacaca atgaagggta ccggctccgt ttccatcatg 900
   atcggcgccg cctcggccat ttccttcatc tttgccaaag agcagatcgg cacgatcctc 960
   ggcaactggc tgctcagtgt cacggccaac aagtacgtct tcctgtttgc cgtcaacatc 1020
   atcatcctga tccttggctg cctggtcgat acctcggtca tccagcttgt catgatcccg 1080
   atcctatggc cggtcgcgga ggcgatgggc atcaacatca tccatttcgg cctcatcatc 1140
   tgcttcaaca tgatggtcgg tctgtccacg ccgccgttcg gcatgtgcct gttcatcacc 1200
   tcgggcatat ccggcacgcc gctcaaggat atcattcggg agatctggtg gccgatcatc 1260
   gtcatgctga tcgtgctcgc cgtgatcacc tacatccccg acgtggtgct gttcctgcca 1320
   aagctcaccg gtatgatggc ctga 1344
<210> 433
   <211> 1125
   <212> DNA
   <213> Unknown
<220>
   <223> Unknown gut bacteria
<400> 433
   gtgaaagcta tcaatcccgg acactttctt ctgacctgcc ggctggacaa ctgggtgcat      60
   ctgctggaag agaaccactt ccatatcgcc cgcgaccggc tgccgcaggc gctgtacatt     120
   tccgcaacgg cgctcgcgct cgcgcccgcg gcggcggcgg aatcgctcat ttaccgcaag     180
   cgcatccgtg aaacaaagat cgaaaaggac ccgatcttca ttctcggcca ctggcgcagc     240
   ggcacaacgt atctgcagaa cgtcctctcg cgcgacgagc agtttggctg gttcgatccc     300
   gtgaacacga tcggcctgcc ctactcgctg ctgctcggcc ggctcatcca gccgccgatc     360
   gaaaagggca tccggaacgg ccgcccgcag gataacgtgc agtactcgct cgatctcccg     420
   atggaggaga cattcggcgt gctgacgatc tcgccgtatt cgatcatcca catgatcgcg     480
   ttcccggaaa attataaaaa gtacatcgaa ggcgcgttcg tgagcgacct tcccgtggag     540
   gaactgcgca aatgggagcg cagctacgat tacgccgtcc gcaagctcac ctacatcaaa     600
   aagggcaagc agctcattct caaaagcccg gacaacaccg cgcgcatccg cgagctgtgg     660
   ggcatgtatc cggacgcgcg gttcatcaac atccaccgcg acccctacaa gaccgtccgc     720
   tccacgatcc acatgttccg caccgagatg gactcgctcc ggctgaccga agagccggat     780
   aacatcgacg agctcatcga gaacacggtc atcgatattt tcgagcgcat gtaccgcgag     840
   cttttcgagc ttgagggctt tttcccgaag aaccggtacg tagacatcgc gtacaccgat     900
   ttctgccgcg ccccggtcga tacgctgcgg gatatttacc ggcgtctgga gctctccggc     960
   tttgaggcag ccgcgccgcg ctttcaggcg tatgtcgatt cgcagcgcgg ataccagaag    1020
   aataaattcg acatttcgcc ccggcttgtg cgcaagatca acgcgaagct gggcttctac    1080
   atggaacact acggctacga aatgcgggag gttgaggaag aatga                   1125
<210> 434
   <211> 1188
   <212> DNA
   <213> Unknown
<220>
   <223> Unknown gut bacteria
<400> 434
   atgcagaaaa tggtaaaacc catccggctc gttgtgctcg ccgtcgtctt tgtgctgctg      60
   ctcggcgttt acttcgtcgc gctttataag ctgcagatca tcggcgggcg cgaatactac     120
   gccgtagggg aaacggagac gaaagaagac atcaccatcg ccgcggcgcg agggaacatt     180
   ctcgaccgct acgggcgtct gctcgtgtcc aactcgctct gcaacaacct gtctatcaac     240
   gtaacggagc tcttcagtca ggacgacccg aacgcggtca ttctcgagct gtgcagcgcc     300
   gtggagcgct tcggtgacac ctataccgac acgctgccca tcacgaaggc cccgcccttt     360
   gagttcaccg agatggacac gctgcagaag agcatgctcg acgcctggct caaggcgaac     420
   gacatgccgc tcgactccag cgccgtggag atcctcgcgg cgatgcgtac gcgctacagc     480
   gtcggcagcg agtataacgc cgaggacgcc cggcgcatcg tcggcgtccg gtacgagatc     540
   aacgaccgct tcaacatcaa caccacgccc tacgttttcg ccgaggacgt ttcgatgccg     600
   ctgctcacct atctcatgga gcaggacatc cccggctttg agctttcctc gtcctatgtg     660
   cgcgaataca aaacgcagta cgccgcccat ctgctcggct acaccggcaa gctgacggcg     720
   gaggaatggg cgacctacgc cggcaaggac tactcgctcg acgcctacgt cggcaaggat     780
   ggcgtggaat acgcctttga ggattatctc cacggcgtgg acggcgaggc ggaggtctac     840
   tccaatctcg acggcgtcac gctccgcacc aagtacacca aggagccgca gcccggcggg     900
   catgtctatc tgacgctgga tatcggcctg caggaggtgt gcgaggaggc gctcgccgcg     960
   cacatcgagt cgatcaacga agagcgcgcc cgcatcattg cggaagccga aatgtacggc    1020
   accgagagca ccgaaatgat ctccggcggc gcgctgtgcg tcgtgaaggt cgataccggc    1080
   gagccgctcg cgctcgtgag ctatccgacc tacgacgccg ccacgatgct ggagaattac    1140
   gaaacgctcg tcgcggacga gaacgccccg ctctataacc gcgcgctc                 1188
<210> 435
   <211> 1383
   <212> DNA
   <213> Unknown
<220>
   <223> Unknown gut bacteria
<400> 435
   atggaggaaa caaacagcct ggcccggtcc cggcgcatgg gcgaaatgcc ggagggaaag        60
   ctgctgctgt caatggcagt gcccatgatg ctctcgatgc tggtgcaggc tctctataat       120
   attgtggaca gcgtatatgt ggcgaaggtg tcggaggact gcctttcggc gctctcactc       180
   gcgttcccgg cgcagaacat tctcatcggt cttgccaccg gcacgggcgt cggcgtttcg       240
   acgatgctct cccgcgctct cggcagccgg gacaacaagc tcgccaaccg cgtggcggga       300
   aacagcctgt tcctctgcgt gtgctgctgg attgttatgg cgatcttcgg tattttcttt       360
   gccgacgcct tcatccactc gcagacggat atcgagtcca tccgcagcta cggagattcg       420
   tatctgcgca tcgttacgat cggctcggta ttcgtgtatt ttgagatcaa ctttgagcgc       480
   cttctgcagt ccacgggcca gacgaagctc tccatgtggg cgcagataat cggcgcggtg       540
   gcaaacatcc ttcttgaccc gttctttatc tacggctggt gcggcctgcc cgcgttgggc       600
   actgcgggcg ccgccatcgc cacggtcatc ggccaggcgt tcggcgcgct ggcgggcttt       660
   ctgtttcacc acaaacataa taaagaagtc aatatccgtc tttccgactg ccgcccggac       720
   gggcacatca tcctcgacat ctatcgcatc ggctttccgt ccatcctcat ggtcggtatc       780
   ggttccctta cgaactatct catgaaccgc atcctcatgg cgttcacgtc cacggcggtc       840
   gcgttgtacg gcgcatactt caagctccag agcttcttct tcatgccggt gttcggactg       900
   aataacggca ttgtccccat cgtcggctat aactacggcg cgcggaaaaa ggaacgcatt       960
   taccggacga tccgctacgg cgtgctgtat gccgtgtgca tccttgctgt cggcctgctg      1020
   ctctttgaag cgatccccgg cgtgctgctg caggcgttct cgccgtcgga gcacatgctt      1080
   gccatcggca ttcccgcgct gcgcaaaatt gccatccatt tcccgatcgc ggcgttctgc      1140
   atcgtgggcg ggtcggtctg tcaggcgctc ggaaagagcc tctattcgtt cttcacgtcg      1200
   gtgctgcgtc aggtcgttgc gctcattccg gcggcgtatc ttctgtcgct gacagggaac      1260
   gtcggcaatg tatggtggtg cttcccgatc gcggaggtcg tatcggcgtg cgcaacggcg      1320
   tttttcctgc gccgcgcgct gcgaaatatg gaccgggagc tgacgctcgc ggaggggcaa      1380
   tga                                                                    1383
<210> 436
   <211> 1431
   <212> DNA
   <213> Unknown
<220>
   <223> Unknown gut bacteria
<400> 436
   atggaaaatc atacttccga ttaccgcgta gaaaaagact ccatcggcac gaaggacgtg      60
   ccggcaaacg tatattacgg cgtgcagtcg ctgcgcgccg cggaaaactt ccccatcacg     120
   gggctgcgga tgaacccgga gctcatccgc agtcttgcct acatcaaaaa ggcggcggca     180
   ctcacgaacc ttgaggccgg actgctgaac gagcgtatcg caaacgccat cgtgcgcgcc     240
   tgcgacgaga tcctcgcggg aaagctgcgt gagcagttca ttgtcgatcc cgtgcagggc     300
   ggcgccggca catccatcaa catgaacgcc aacgaggtca ttgccaaccg cgccatcgag     360
   ctgctcggcg gcgtcaaggg cgactactcg ctcgtcaacc cgaacgacca cgtcaactgc     420
   ggccagtcca ccaacgatgt cattcccact gccggaaaga tgacgacgct gcgccttctg     480
   aaaaagctgg agaccgagct ggaccggctg cgtgccgcgt ttgaggacaa atcccgcgaa     540
   tttaaccacg tcatcaaaat gggccgcacg cagatgcagg acgccgtgcc gatccggctg     600
   gggcaggagt ttcgcgccta tgccgacgcc gtgcggcgcg ataccgtgcg catgggcaag     660
   gccatggacg agatgcgctc gctcaatatg ggcggcacgg ccatcggcac cggcatcaac     720
   gcggacgaaa cgtacctgcg gcgcatcgtc cccgtgctga gcgaggtatc cggcatggag     780
   ctcacgcagg cgggcgatct catcgactcc acgcagaacc cggacccgtt tgtggcggtg     840
   tcgggcgctg tcaaaacgtg cgcggtgacg ctctcgaaaa ttgccaatga ccttcggctt     900
   atgtcgtccg ggccgcgcac cggcttcggc gagatcaatc tcccggcgcg gcagaacggc     960
   tcgtccatca tgccgggcaa ggtgaacccg gtcattccgg aggtcatcaa ccaggtggcg    1020
   ttcctcatca tcggcaacga catgaccgtc accatggccg ccgagggcgg gcagctcgag    1080
   ctcaacgcct ttgagccgat catcttctac tgcctgttcc agtcgatcgg cacgctgacc    1140
   tacgccgtgc acacgttcgt ggacaactgc gtcaccggca tcaccgcgaa cgaggagcgc    1200
   tgccgggagc ttgtcgagag cagcgtcggc gtcatcacgg cgctcacgcc gcacatcggc    1260
   tatcagcacg ccgcggacat cgccaagcgc gccatcgtca ccgggcagag cgtccggaag    1320
   ctcatactgc aggaaaagct tctcacggaa gaggagatcg acacgatcct cgatcccatg    1380
   aatctaacaa agccgggcat ccccggcaaa gagcttctcg cgcacaagta a             1431
<210> 437
   <211> 1434
   <212> **DNA**
   <213> Unknown
<220>
   <223> Unknown gut bacteria
<400> 437
   atgattgtca agaacagcaa gaaagaaaaa aacaccgtta cctttgatgt cgagctcgac      60
   gccgccgagt ttgaaaagta tgtcaacggt gcatatctga aggctcgcag ccgcatcatg     120
   gtccccggct tccgcaaggg caaggcgccc cgcatggtca ttgagggcat gtacggcaag     180
   gacgtgttct atgatgacgc ctttgataac gccgcgaacg acgcttttgc gttcggcgtc     240
   gctgagggca agctcgagac cgtcggccgt ccgaccatga ccgattccaa ggtcaccgag     300
   gagaacggcg cgcttctgag cttcagcacc gacgtttacc ccgaggtcac cctcggccag     360
   tataagggcc ttgaggcgga gaagccctcc gttgaggtca ccgacgaaga ggttgacggc     420
   cacatcgagc gcctgcgcaa gcagaacgcc cgcctcatca ccgtggagcg tcccgcgcag     480
   gacggcgaca ccgtgaacat caactacgcc ggcatgctcg acggcatccc gttcgacggc     540
   ggcacggcgg agaaccagaa tctcgttctc ggctccaaca ccttcgttcc cggctttgag     600
   agccagctcg tcggcatttc cgccggtgag gagcgcgatc ttgacattac tttcccggag     660
   aactacggcc acgatctcgg cggcaaggcc gtcgtgttcc acgtcaagtg caacgaggtc     720
   aagtttgaag agctgcccga gctggacgac gagttcgcga aggacaacga ctttgacacg     780
   ctcgccgatc tcaaggccga catccgcgcc aagtccgttg aggagaagga aaaggccgcg     840
   aagaacgcct ttgacgacgc cctcgtcgat caggctgtgg agaacatgac cgttgatatg     900
   cccgaaggca tgatcgaaga gcgcatggac ggcattgtcc gcgagtacgc gcagtacatg     960
   gccaaccagg gcatccgcat cgacgattat ctcaagatga tcggcaccga tatgaagacc    1020
   ttccgcgaga gcatgcgcgc caccgccgaa aagcaggccc gcaccgaggt cctcctcgcc    1080
   gccgtcgccg aagccgagaa tgtccagatc tctgaagagg atctgcaggc cgagtatgag    1140
   aagctcggtg aagcctacgg catgaaggcc gaggacgtgg cgaaggccat cgatgtcgcc    1200
   ggtctgaccg ctgatctgcg ccgtgagcgc gctatgagca tcatctccga gagcggcgtt    1260
   gccgtggaga agaaggccga ggagcccgcc aagacggagg aagcgtccaa ggccaagaag    1320
   aagcccgccg cgaagaaggc cgccaagacc gaagagaccg agggcgagga agcgcccaag    1380
   gccaagaaga ccacccgcaa gagcaccaag accgaggaga agacggagga gtga          1434
<210> 438
   <211> 1470
   <212> DNA
   <213> Unknown
<220>
   <223> Unknown gut bacteria
<400> 438
   cgcgatatcc cggccggcgg cagctacggc cgtctaaacg gggagaacgg gtatttcttt 60
   tttgcgcaga gcagccgcgg cgcggacaac ggcacggggt accgcatggt ggcggcaaag 120
   cccacggtcg atatcgccgc gggcgtgtac gacgacgccg agagccttac gctgacgatc 180
   accggcgaga acgtgcacta tacgctcgac ggcagcgacc cgacggcgga cgatccggcc 240
   tataccgcgc ccatcacgat cacggaaacg acgatcgtcc gcgccgcgag cttcccggcg 300
   gacgcgctgc cgggcaaagc ggcgacatgg tcgtatttcc tgcgcgagaa cagcacgctg 360
   cccgtcgtca gtcttgtgac cgacccggac aatctcaccg gcgcgcaggg catttactcc 420
   aaccacgaac gcgcgtggag cgagaagtgg gagcgcgagg cgaccgtcgc catgtacgag 480
   gacggcggcg agttctccat tgactgcggc atccgcatgc acggccgcac gagccgccgt 540
   gtgagcgaaa agaagtcgtt cacgctcaaa ttccgcggcc ggtacggcgg ggatctcaat 600
   tatgacgtgt tcggcgacgg cgtcgtgacg gacttttcct cgctgctgct gcgcgcgagc 660
   gtggaggata cctacacctc ctacatgcgc gacgagttct tcgcccgcat cgccatggac 720
   tatacggatg tgcccgcgca gaattaccgg tatgtttcgc tcttcctcaa cggcgagtac 780
   tggggcattt acgccatccg cgagcaccac agcgcggaat acttcgcctc gcacaagggc 840
   gtggacgccg acaccgtcga tatgcagacg ggcgagtttg aaggccagac cgcgtggagc 900
   gagatcttga actacgcgcg ctacaactcg ctgagcacgc cggagggctg ggcgtacatc 960
   caggagcatg tggacatccc ggagatgatt gactggctga ttctcgagtg ctggtcgggc 1020
   gatatcgacg tatacgaaaa cgtccgcttc tacgcgagcc ccgagtacga aaacgggaag 1080
   tacatttacg gtctcgcgga catggatctc acgatgatgg gcatggactc gatgagcgtc 1140
   ggcttcaaca acttccagct ccacggcatc attccctcgg cgctgcgtta caacgaagag 1200
   ttccgcgata tgttcctgac gcgtctcggc gaaatgctgc gcggcccgct ctcggacgag 1260
   aacgcgcaga aaacgctcga tgagctgcgc gcgatcgttg agccggagag cgcgcgtgat 1320
   ctcgcccgct ggggcaagtc gtccacgttg tttgccacgc agatggcgaa tctccgcggc 1380
   ttcgtctccg gccgcgccgc gcggatgcag aacgaggccc tcgccttctt cggcgtctcc 1440
   gccgaggatg ccgccaaata cttcgggtaa 1470
<210> 439
   <211> 1986
   <212> **DNA**
   <213> Unknown
<220>
   <223> Unknown gut bacteria
<400> 439
   gtgcgccgcg tcgtcatcac ggcgaaggac ggcaagtatc agctgacgct cacgctcaac 60
   ggcaccagct acgattacct ctaccccggc accggcacgc aggccgacgc cgttgaccgg 120
   agcgagtggg ctcccgccaa ggtcgtgagc ggcacgatcg acggcgagac gcgcgacaac 180
   tggcacacct acaccatcac ggtggataag ctgacgaacc cgctggccgt cacctctcac 240
   agccagaaga acgacaagtg gtacgaccgc agcatcacgc tggacatcgg aaatctcaag 300
   cgcaccgccg aggacggcaa gtattccgcc gcggccgagt gcgacgcggc gatgttcaag 360
   atcatcgaca cgaagctcaa cgttgtgaac ggcgcgatca tggccacgct gacgctcagc 420
   ggcaccggct acgattacct ctaccccggc acaggcgacg aggccgagaa ggacaaggac 480
   aactgggcgc ccttcgccgt ggacgaaaac ggcaagtaca cctacaccat ccccgtgtct 540
   gagctcgaca agcccctcat catctcctcg cacagcctga aaaacaacat ctggtacgac 600
   cgcacggtga cgttccgctc cgattcgctc aagaagatcg gcgacgcggc cgaccctgtc 660
   actccgacac ccacgccgac cccgacgccg acgcctaccc cgaaccccac caatccggac 720
   aacgaatccg atcacgaaac cgacaccagc ggctccacct cccgcgtgga taacagcacg 780
   acgctcaagg acggcgtcta cgcgcccgac agcttctcct tctccggcgg ctccggccgc 840
   gcgcgcatcg tgtgcgacaa ggtcacggtg aagaacggcc aggcgtacgc taccatccgc 900
   ttcgtgagca gcagcggcaa cccgccgcag tactcctatg tgaaggcctc cggcaacacc 960
   tattactcca acgacggcag caccttcacg atccccgtgg agctcaacaa gaacaaccgc 1020
   attctcggca tgaccaccaa gatgtcctcc gcgcacgaga tcgagtacat catcttcgtg 1080
   cagctcaaga cgaacgcgaa caaccagaag gacaacaccg acggcaaaac gctcagcacc 1140
   gaagcgcccg agatcatggg tctcacgttc caggaggaga tcaagctcga gcacgcgcag 1200
   tatctcaagc tcttccgcta tgagggcaac atcgtcctcg cggagatcga cgtcataagc 1260
   gacacggtgc tcgacaccga cgaggcgaag gccgagatcg ccaaggcgga cagcgccgcc 1320
   gcgtccgacg gcacggaaga agccgtcgcc gaggaagaga gcgccgacaa cgcgcagatc 1380
   aaggccgact acgtcgccgg gctctataag cagaacgtcc tgcgctatct gttcgttccg 1440
   gaaggaacgg agctccccgc gggccttgaa aagcagctca tcatcgtgac gctgccggcg 1500
   aagagcatct atctgatgga cgaaacgccc gtcgatacct ttgagatctt tgaagcgctg 1560
   aaccttgtca aggtgttcgg cgcggagacg agcgagaacg agatcctcag caagggtctt 1620
   gccgacggca gcgtgctgta cggcggcagc tgggataagc ccgagtacaa gaagctcgtg 1680
   cagaacaaga ccgatctcgc gatcacgtcc ggcaagctcc tgccgctgag cggggaaacg 1740
   ctcaccgagc gcaagcagac cgcccccgct gacgcggaga gcctgacggc ggtggagtat 1800
   cgcgaccgtc tgtacgatct ggccgaccgc ttcgccgtgc tcgatatccc gatgctcatc 1860
   gaccgctccg ccgacgaggc cgacgctctc gcgcaggccg actggctgcg gctctacggc 1920
   atcctcttcg gcgacgagac gaccgccgag cgcatgatcc gcgaaattac ctcccgggag 1980
   ggttaa 1986
<210> 440
   <211> 792
   <212> DNA
   <213> Unknown
<220>
   <223> Unknown gut bacteria
<400> 440
   atgcaaaatt caaccgacag aaggaaaatc atgagcgaac cgatcgtcag ttcccttgcc 60
   aaagcgctcc gcgttctgga atgcttcacg gtcagcgagc cggagctcgg cgtcaccgag 120
   atctccaaaa agctcgggat cggcaagtcc aacgcgcaca acatcatatc gacatactgc 180
   cagatgggtt atctgcagca gcttcccaac cggaaataca cgctcggcta caagctgctc 240
   gagcacgcgt tcatcatcaa ccagcatctc ggctatccgc acgcggtgta cgatctcctg 300
   cttgaggccg ccgagcgcac cgggcaggtc gtctacttcg gcatcccgta cgaaacggac 360
   gttctgtatc tgtatgtcgc ccatccgatc gcgcggatga aggagctgcc gtaccgcgag 420
   atctgcggcg agcacgcccc gctgtgtacc acgggcatcg gcaaggcgat cctcgccaat 480
   ctccctccgg aggaatggct ctcccgcctg cccgagatcg ttcccgcgca cacaccgcag 540
   acgatcaccg ataaggacaa gatcctgcag gagctgcaga gcacccgcga gcgcggcttc 600
   gccatcgaca actgcgagca cgagctgaac gtccgctgcg tcggcgtgcc ggtctataac 660
   gcgagaggcg ctcttgtcgc cggcatcagc tcctccggcg cggtgcaggt catgaccgac 720
   cggaaggtca tggaatgcgc cgacatcctg atgggtatca cgcagcgcat caaggaaagg 780
   ctgtacgcct ga 792
<210> 441
   <211> 1434
   <212> DNA
   <213> Unknown
<220>
   <223> Unknown gut bacteria
<400> 441
   atgacactgc ggccaaagcc cttccagccg atcaaaatgc tgcagatcat cggcggcgtt 60
   ttcttcgcgt atctcttctt cgggtttatg ctgctgccgt gcctgaacac gctcaccagc 120
   attttcaccg tcaagaacgc cgccggggaa accgatcccc tcgcggtcat ccgtttcttc 180
   ctcgccgggt ccatgtccca gtatgtgtgg aactcgctca agctggcgct gtgccttgtc 240
   atcacggtca acgtcgtcgg catttccatc gtgctgctga ccgagtactt tgacatcaag 300
   ggcgcaaaga tcctccgtct cggctatatg acgacgctca tctactccgg cgtcgcgctc 360
   gtcacgggct acatgttcct gtacgccagc gacggcatca tcaccacgca gctcaaggac 420
   gcgttcccga acatcaatgt caactggttc accggcttca acgcggtgct cttcacgatg 480
   acgttcgcct gcacgagcaa ccacgcgctc ttcctgcgca acgccatccg cggcatcgac 540
   tacaacaccg ttgaggccgc ccgcaacatg ggcgcgaagc ccttcaaggt gctctggaag 600
   gtcgtgttcc cgacgcttct gccgacgctc ttctcgctca cggtcatgac gtttatcacg 660
   ggcctgtgcg ccatgagcgc gccgacgttg ctcggctatg actccatcaa ccccgagatc 720
   gtccgtctgg ccggttcgtc cgtggcggac gaggcgttcc cgcaggcgcg cgcggcgctg 780
   ctttcgatca tcctcgcgtg cttcacgatc ctcctgctca ccgtcctctc gtcctatgag 840
   cgcaaggggc attacctctc cgtttccaag acaaaggcga agctccagaa gcagaagatc 900
   acgaaccccg tggcgaacgt cctcgcgcat atctacgcct acgtcctgtt catcatctac 960
   atgacgccgg tcgtgatgat cgtcatcttc tcgttccaga cgttcagcgc cgtgcgtatg 1020
   cgcaagcttg atctttcgca gtggacactc gtgaactact tcggcaagca ggactattcg 1080
   tatctcacgt cccgcggcac ctacaagatc cgcgagggct cgatctccgg cgtgttctcc 1140
   aacgaggcga cgctcagcgg catccagatg agctttgtca tgtccgtcat cgccgcggcg 1200
   ctcgcgtgcg tgatcgtcgt tgtggcctgc aactacatct tcaagcacaa gaagaccggc 1260
   acgatcctgg agtacgcgct gctcttcccg tggctgctgc cgacgatcct catctgctat 1320
   tcttaccgca cgttcttcaa cagcagcgac gtctggtatg tgttcggcca gaacctgtac 1380
   tatgccgacc gcgtgaggat attgatcgtt atcgcgtata cggtagtaaa gctg 1434
<210> 442
   <211> 1032
   <212> DNA
   <213> Unknown
<220>
   <223> Unknown gut bacteria
<400> 442
   atgagcctta tcacactcat cctcgtcctt gcggcggcgg ccgtgctcat cgtgctcggc      60
   accacgcgca gaaaggacgg cggcgacggg aaaaagaccc tgccgcgcac gatcgtgacg     120
   gccgcggcgg tgctcgtgct cgtcggcgcg ctgggcttcg gctcggtgta ccagctgcag     180
   gaggacgaat acgccgttat caccacgttc ggcaagccgt ccgtcgtgag cacgtccggc     240
   ctcaagttca agatccccat catccagcag aaaaccatcg tcagcaaggc gacgcagggg     300
   tttgctctcg gctacgatct gcgcacgaac gtgaacaacg aggacgaatc gctcatgatc     360
   agcgtggact tcaacttcgt caacgtggat ttctatgtgg aataccgcgt ggacgacccg     420
   gtgaagtacc tttataacag cgaagagccg gaagccattt tgaaaatgct ctgccagagc     480
   tacatccgcg atacgatcgg cctttacggc gtggacgata tcatcacgac cggcaaggcg     540
   gagatccagg gcgtcatccg cgacaagatc agcacccgtc tcgagcagga ggatatcggt     600
   ctcgtgctcg tgaacatcgc cctgcaggac gccgagcccc ccacgctcga ggtgcagcag     660
   gcgttcaagg cggtggagac ctccaagcag gaggcggaga ccgccatcaa caatgcgaac     720
   aagtacgcta acgagcagct gcccgccgcc aaggcgaacg cggacgaaat tctgcagcag     780
   gccgaggcct acaaggaatc ccgcatcgcc gaggccgagg gtcaggcctc gcgcttcacg     840
   gagctgtacg ccgaatactc caagtatccg gagatcacgc gccagcgcct gttctatgaa     900
   atgatcgcca aggtgttccc ggatatgaag atcgttatca ccggcaggga cggcagcacg     960
   ctgcagaccg ttctcccgct cgagagcttc accggcacga acacaacgac cacgacaggg    1020
   gaggagccgt aa                                                        1032
<210> 443
   <211> 1428
   <212> DNA
   <213> Unknown
<220>
   <223> Unknown gut bacteria
<400> 443
   atgaaagcaa acattcttgt ttatgatgtc ggcagcacct atacgaaggg cgcggccttt      60
   gcccttgcgg acgggaaact gacgttcctt gcccgcggcc agcacccgac gacgctggag     120
   aacatcagcg acggcgcgcg cggcgccgag gcggagatcc gcgccaaggg cgtggaattc     180
   gcgcccgata tggttcggtt cagctcgtgc agcgccgcgg gcggactgcg catggtcgcc     240
   atggggtaca tgccccgcgt gaccgcgaag gccgcgaagg aagttgccat gaccgccggt     300
   gcgcgtgtca tggaggtcgt ctccgcggac gagccgcccg agttccgcga ggaagtgctg     360
   cgcgagatca agcccgacat catcctgctc tccggcggta cggacggcgg cgacatggcc     420
   aacgtgctcg aaaacgcgga catcatctgc aaggtgcacg gcgaaggcac ggtcattctc     480
   ggctgcaaca agttcgccca gcgcgaggcg gcggagaaat tccgcgccgc gggcattgac     540
   tgcatccgcg tgccgaatat cctgccgacg atccacgagc tgaacgtgaa gcccgcgcgc     600
   atcgccatcc acgagcagtt catcaaccag atcacccatg cgaagggctt gcaggagttc     660
   cgcgattcgc tcgcggatca gaccgtcatc ccgacgccgg gcgccgtgct ccttgcgagc     720
   gagctgcttg ccaagggcag ctacgagcag gagggcgtcg gcggcgtgat cctcgtggat     780
   atcggcggcg ctacgaccga catccattcc gctctgcccg agctcatcga catgaacatc     840
   gaagagcgcg gacttgttgt gagcaacgaa aagcagtttt cctaccgcac cgtcgagggc     900
   aatctcggtc tgcgcgtgag cgcgaccggc attcccgagg ccgtgggcga caaggccgtt     960
   ctccgcgcca tggaccacga ttacggtctg acgccggagg atgtggaaaa ctatgtcgat    1020
   ctgctcgaag cgcacaacga tcacattccc gccgacgaga aggaggcctg tctcgaccgc    1080
   gcgcttgccg cgtgcgccgc ggacgttgcc atccgccgtc acgccggata ttacgcgaag    1140
   gaggccgatc ccgtcatggg catcatggcc ggcgctccga tggggcgcga tcttcgcaac    1200
   gtgcgccagg tgctgtgcgt gggcggtatt ttcctccact gccacggcga ggagcgcgag    1260
   aacatcgtgc gccgcacgtt cgataacccc ggcatctccc tgctgccgct caacgacccg    1320
   gacatctggt tcgacgagga ctacatcctc tacgccatgg gcgttctggc gcagcacttc    1380
   cccgaagaag ttctttcgta catgaaagag catgtgataa agaaataa                 1428
<210> 444
   <211> 1296
   <212> DNA
   <213> Unknown
<220>
   <223> Unknown gut bacteria
<400> 444
   atgaataatt caaacgaaac gaaagaaagg aaaaagctgg atctcaaacg aatcacggac 60
   agccgtgttt tctggatgat cctctcgctg cttgcggcgt cgcttctctg gatgtatgtg 120
   acgacgaccg agggcgttga ggtggaaaaa acgctcaccg gcgtaaagat cgagtttctc 180
   ggcgcggacg cgatgcgcgc gtcgagcgat ctcatcgtca ccgagcagga ccgcaccacc 240
   gttaacctga cgctctccgg cacgcgccgc gtgctctcca agctcacaaa ctccaacgtg 300
   acggcaacgg tgaatctcag caacgttgtg gcggacgggc ggtattcggt ttccttcgat 360
   atcgcctatc ccgccggcgt gaacgcgaac gaaataacgc tcgtccacac gtcatcggac 420
   gtcgtgaatt tctatgtgga caagctagcc cgcaagacca ttgaggtcaa gggcgcgttc 480
   accggcaaca cggcggacgg atatcttgcc gacgagagta tggtgttcga tcctctggtc 540
   gttgccatca gcgggccgaa gacggtcatc tccaaagtcg accatgcgtt tgtggccatt 600
   acgcgtgagg acgtggaccg cacgctgagc tattccacga cgtattctct cgtcgattcc 660
   gacgataacc cgatcgaaga ctcttccatc cagcgtgaaa cggaagaagt caccgtgacg 720
   ctgaacgtgc tttccacgaa ggaagtgccg cttgacgtaa cgatcatcga tggcggcggc 780
   gcaacgcgcg acgccaacac ccgcattgac attgaaccga agacgatcat gctcgcggga 840
   gatgcctccg ccatcgacag cacgacgaag ctcattctcg gcacgatcaa tctttcgagc 900
   ttcgccacgg acttctccgc cacctacacc atcatcccgc cgaacggcac cgagaatatg 960
   accggcgtta ccgaggcaac ggtgagagta tccatcacgg gcgttgccac gaagtccttc 1020
   tccgtgaatc cggaaaatat cgtctgcatc aacgttcccg acggctacac cgcggagaac 1080
   atcacgcagg cgcttaacgt aacggtccgc gccccgcagg aagtccttga ccagatcagc 1140
   gaggtaaatc tccgcgctgt ggccgatctc tccaatctcg gcacgaacgc ctccggcgtt 1200
   ttcaccccga cggtcgagat caagatcgac ggcttccccg aagcgggctg cgtcggcgaa 1260
   tataagatct atatcacgct caaggaaaag cattaa 1296
<210> 445
   <211> 1659
   <212> DNA
   <213> Unknown
<220>
   <223> Unknown gut bacteria
<400> 445
   atgaaataca gaaagatctt tgctctgctg ctcgccgcgg cgacgctctt ctcgcttgcc 60
   gcgtgcggcg agaccgccgc cgggccggag gcaacgcccg atcccgcgct ggacgagagc 120
   gccgcgaccg tctccggcca gacgctcacc ttcgccggcg ccgcggacga tgtgttctcg 180
   ctctcgctcg actacgagga gagcctcaac cccgtcaaga cgcagagcac gctcaatcag 240
   ctcgtggact gcctcgtgta cgaccggctt ttcgaggtcg atgagaactt caacgtgacg 300
   tcgcgcatcc tttcggactg gtattactcc aagaacgaag acagtgcggg cgtgtgggtg 360
   ctcaaggtga gggagggcat ccagatgcac gacggctcca cgctcaccgc gcaggacgtg 420
   gcgtattccg tgtcctgcat cttcacgagc gagagacaca agcatctgca gcagcaggtc 480
   gggcgcgtct attcgagcgc gtacaacggc gaggtgtatc tcgccgtgga aggcgcggac 540
   aatgcgcagc tcccgcagcg catgagcatc ccgatcatca agtccggctc cgtcggcgac 600
   gaaatccccg tcggatcggg cccgtatatg tataatgaag accgcacggc gctcgtgaag 660
   tttgacgggt atgaaaacag cgagagtctc ccgctcgacg aggtgcagct gcgcgcctac 720
   cgcggcatgg aggagctcat caccgagttt gaaagcggtc tcgtcgatct cgtcgtgaac 780
   gacccgaccg gcatttacaa catgggctac ggcggcaaaa acgaaaagcg caccatcacc 840
   accacgaatc tccacttcat cggcttcaac agccagagcc gcttcttcca gtatgacgcc 900
   taccgctacg cgatgaactg gatcgtgaac cgcgacaaga tcgtatcgga cacgctcgac 960
   ggcaacgccg tggcgacggt gctccccgtc cacccgaaca gcgcgctgta cgatacctcg 1020
   atcgcctcgc agttttccta cgatccatcg cgctgcctca ccgagctcga gcgcggcggc 1080
   tgccgcgatc tggacgcgga cggcatgctt gaatttgcgc tctccggcat gaaggtggac 1140
   attgagctca acttcgccgt ctgcgccgac aacgccgcca aggtgcagga ggcgcgccgg 1200
   atcgccgagg atctggaagc catcggcatc ggcgtcaacc tgcgcgagct tacatggaag 1260
   gactatctcg ccgcgctgcg ctccggatac atcgacttcg atcaggagga gcccgagatc 1320
   gttatggata tgtatctcgc cgaggtcgcg ctgaccggcg actggaacat cctcacgctg 1380
   ttctccggcg actgggagga tgacaacacc ctcaactacg gcggctggga caacagcgag 1440
   ctggaggagc tcaccgaggc ctatctcggc gcaaaggacg acgaccgcgc gcaggccgta 1500
   aacgactggc tgcagctcct cacgacgacc acggtgatcc tgccggtgtg ctttgagacg 1560
   cgcgaggtta tctcccatct cggcgtgatc tccggcatgt cgcccaacca gtacgacgta 1620
   ttcaataata ttaccgcctg gacagtcaat atcggctga 1659
<210> 446
   <211> 1602
   <212> DNA
   <213> Unknown
<220>
   <223> Unknown gut bacteria
<400> 446
   atgacaaacg caaaagagaa aaagaagatc gtcctctggc tgatcgtact ggcaatactc      60
   gccgcggcgg cgttcacggt cacggccatt gtgcgccata accagcgccc ggcgtgggac     120
   ggcggctaca gcgtccacat cagcgaggtc atgaccgata ataagacctg cccgaacggc     180
   gagggtgtcc tctgtgattg gatcgagatc gaaaacacgt cctcgaagga cttctccatc     240
   ggcggatact atctctctga cgagacgggc aaaggcaaat actgcttccc ggcggggacg     300
   gtcgtcccgg cgcgggggta tctcgtcgtg tggtgctcgc cggacgagga gggcgactat     360
   gcgccctttg ccctgcgcaa ggcaggcggg gagacggtgt gccttatgaa cgaaaaccgc     420
   acggtgctcg acagcgccgt taccgctgcg tgccggagcg ggcagtcgct tgtccgcggc     480
   agcgacggcg cggtcattcc tgccgatacg ccgacgcccg gctacccgaa caacgaagcg     540
   ggcgcaaagg cgtggcagga ggcgctcgcg cagcgcagcg gcggcacgct tgagctcagc     600
   gagatccagt cctcaaacac gctctatgcc gcgcccgacg ggaatttcta cgactggatc     660
   gaggtacata acccctcgga ttcgccggta tctctctccg gctacaagct ctcggaccgc     720
   acgaacaaaa cgaagtacgc cttcccggac gaaatgctcg gcgcgggcga atataagatc     780
   atatggtgcg cccccggtgt ggagggcgcg gaatatgcgt cctttgcgct ggcgagcgcg     840
   ggcggggaaa cggtcgtgct cacagcgccc tccggcgcgg agagcgagag cgtggaactg     900
   ccgatgctgg agaagaacac ggcctatgcc aaggaaaacg gcgaatggac ggtgtctcac     960
   cgcccgacgc cgggatattc caacgacgac gcggggtatg aggcgtggct gcgttccggc    1020
   ggctacgaaa acgacgaaat ttacatcacc gaggtcatgg cgcaaaaccg cggtacgctc    1080
   gccgacagcg acggcgacct ctccgactgg atcgagatcg caaacctcgg cgagacgagc    1140
   gtcgatcttg cgggctggta tctctcggac gatccggacg tgccggacga atggaagatc    1200
   ccgtcgctga cgcttgcgcc gggcgagtat gcggtgatct tcgcctccgg caagaaccgc    1260
   accgagggtg agcttcacac gagcttttcg ctcaccgacg gcgacacggt cattctctcc    1320
   gccggaaacg gcgcggagat ccgcagcgtg aagatcgagg ccgtgccgga ggggtcctcg    1380
   ctcgcgatgc agccggacgg cagcttcgcc gtgaccgact tcccgacgcc ggggcttgaa    1440
   aataccgccg agggctacgc cgacttctgc gatgccgaca cgcgcacctc gccgcttctg    1500
   ctgtgggaga tcgtcgtgta cgaggcgggt tcgcccgact gggtggagct caaaaacgtg    1560
   tcgggcgagt cgatcgatct ttccggatac acgatctcgg at                       1602
<210> 447
   <211> 1410
   <212> DNA
   <213> Unknown
<220>
   <223> Unknown gut bacteria
<400> 447
   atgaagaaat ttatcggaaa caaagacttt tacaagctgg tcgcggcgct cgcgctgccg      60
   ctgctgctgc agaacgccgt atcgacgttt gtgaacctgc tcgataacct tatggtcggg     120
   cggatcggca cggaatcgat gtcgggcgtg tccatcgtca accagcttct gttcgtatat     180
   aacctctgtc tcttcggcgg cacgggcggc gcgggcattt tcggcgcgca gtttttcggc     240
   cgcggagatt ataacggcgt ccggcatacg ctgcgcttca atatcctgct ctgcgccggg     300
   tttacagtga tcgccacggt gctgctcgcg ctcatcccgg atacgctcat cggtgcgttc     360
   ctgcacgagg caggcaccgg cggcgatctc gcgctcacgc tctcggaagg caaggactac     420
   cttcgcatca tgctcttcgg tctgccggcg ttctgcctta cgacggcgta tgtgagcata     480
   ctgcgcgtca ccggcgacaa ccgcctgccg atgaaggcga gcatcgcggc gatcttcgtc     540
   aatctcgttt tcaactatct gctcatctac ggcagcctcg gcttcccgat gctcggtgtc     600
   cgcggcgcag ccattgccac ggtgctctcg cgctatgtgg agttcggcct gatcctgctc     660
   ggcacccacg ggaagaaaac gccgccgcgc tttatccgcg ggctgtaccg cggctttgcg     720
   atccccggcc atctcgccgg acagatcgtc cgcaagggca tgccgctgct cgtgaacgag     780
   atgttctggt cgctcggcat gacgatgctc acgcagtgct actcctaccg cgggttggac     840
   gccattgcca cgctgaacat caactccacg gtgctcaatc tcttcaatac cgttatcttc     900
   acgatgggca ataccgtcgg catcatcatc ggcaacctgc tcggcgcgga ggagtatgac     960
   cgcgcgcgga actactgccc gaagctcgcc gcgctcagct gcgcggccag cgcggcggtc    1020
   ggcgttgtga tgttcctcgt cgcgccgtat gccccgcggc tttacaacac ctcccacgag    1080
   atcatgacgc tcgccgcgtc gctcatgcgc atctgcgctc tcatgctgcc ggtccacgcc    1140
   gtggtaaacg ccgcgtactg gaccatccgc gccggaggcc ggacgtacat caccatgctt    1200
   tcggacagcg tgttcacctg ggtcgtgtcg gtatcgatcg cgtgggtgct catccataaa    1260
   acgggtctgc cgctcgtgga ggcgtacctc tttgtgaatc tcgccgacct tatcaaaatg    1320
   gtcatcggca tcgtgcttct ccgccgcggc atctgggtgc gcaacatcgt caccggcgag    1380
   gagatcgcgg ccgggaatgc tgcgggctga                                    1410
<210> 448
   <211> 909
   <212> DNA
   <213> Unknown
<220>
   <223> Unknown gut bacteria
<400> 448
   atggaaaatc ctgcgcttat tatcatggcc gccggtatgg gcagccgctt cggcgggctc      60
   aagcagatca cgcccgtggg cgacaacggc gagatcatca tggacttctc gctgtacgac     120
   gcttaccagg cgggcttccg caaggtcgtg ttcgtcatca aaaaggaacg cgaggcggat     180
   ttccgcgcca tcgtcggcag ccatgtggaa agccggatgg acgtgcagta tgtgttccag     240
   tcgccggacg atgtgccgga gtggttcacc gttcccgaag ggcgcgaaaa gccctggggc     300
   acggcgcacg ccgtgcgcgc ctgccggaac gtgatcgacg gcccgttcgc cgtcatcaac     360
   gccgacgatt tttacggcgc aggggcattc cgcgcgctct atgattttct cgccgagccg     420
   atggcggaga gcgagaacgc catggtcggg tatcgcatgc gcaataccgt taccgaaaac     480
   gggtatgtcg cgcgcggcgt gtgcgaaacg gcaaacggct ttctcacctc gatcaccgag     540
   cggacgcaca ttgaaaagcg cggcgaccac gccgcctaca cgaccgatgg cgagactttc     600
   atcgatcttc ccggcgatac gctcgtatcg atgaacttct ggggcttccg caagcggatg     660
   atgcgcgcgt tcgacgagcg gtttgaggat ttcgtgcgcg agatcctgcc gaaaaatccg     720
   ctcaaggcgg agtacttcct gccgtgggtc gcggataagg agatgcacgc aggccttgcg     780
   cgcgtgcagg tgctgccatg cgaggagacc tggtacggcg tgacgtaccg cgaggatctc     840
   ccgcgcgtcc aggccgccgt agcggacatg aaggcgaagg gcgtgtaccc gaagaagctc     900
   tgggaataa                                                             909
<210> 449
   <211> 1098
   <212> DNA
   <213> Unknown
<220>
   <223> Unknown gut bacteria
<400> 449
   atggcacaca gaccaaacat tcttgcgctc gcatccaaga tcagtctcga aagtctgacg      60
   tacaccggca ttacctacgg cgatcccgag tacagaatcc ttgatcccat cgtcgatgac     120
   gacatgtgtt ccgttatgat gcgcatgcgt ctcgaaaccg acttctccgc ggaggatctc     180
   gccaaaaaga cgagaaagag cctcgatttc gtgcagacgc agtgcgacaa gctcgttaaa      240
   gcgggcgtca tccgcacgcg cgtccgcagc ggcgttctct gctactatta cccgatctgg      300
   gtacccggca tcatggaggg cattctctcc aaccgcgagc agtgcgacgc gcaccccgag      360
   ctcggcgcgt gttttgagga gtatacccgc atccgcgtcg gcatgctcgc ccctatgctc      420
   ggcaacggcg tgaatttcat gcgcgttatg cccgttatga gcgccatcga aaacgacacg      480
   cacaaagcct cctatgacga gatctccacg ctgatcgaaa aggcgtgggc aatctccgtc      540
   ggcccctgct cctgccgaag atcgcgccgt ctgatgggcg aaggctgcgg ccatctggaa      600
   gaggatatgt gcctctatct caacgacaac gccatcagct tctccaagaa cggcgagcac      660
   cgcctcatca ccaaggaaga agcgtacgag atcctccggc gcgccgagga caacggcctt      720
   gtccacgagg tgaaccaggc cctcggcttt gacgacgtca acgccatctg caactgctgc      780
   ggctgctcgt gctacgcgct gcgcatcgcc gagctgttcc gttcgccgaa cggcgtcagc      840
   tccaacttca ttgccagagt ggacaaggat aagtgcgtcg cctgcggcca gtgcgtggaa      900
   aactgccaga cgaacgccgt tcgtctcggc cagaagcact gtctcaccga ttcgcacatc      960
   tccgacgcct acgcatccga caaagagatc ccctgggata agaaaagcta caacattgat     1020
   taccgcacca cccgtaccga caccatgccc tccggcaccg cgccgtgcaa ggcggagtgt     1080
   ccggcgcatg ttccggtg                                                   1098
<210> 450
   <211> 2565
   <212> DNA
   <213> Unknown
<220>
   <223> Unknown gut bacteria
<400> 450
   atgaacgaac gcctgctttt gtcatccgaa ggcaggcgtt ctttacgcaa ttttaagacg        60
   gtctttattg aaaaagagcg aaaaaatggt atactgatat ataacgcttt tttcggggga       120
   gggcttggcg ctttgaagct gttccgcaga aaaacagaag aggaagccgt ccggcaggag       180
   atcgccgtga aggagaccgc gcccttcgag ggtctcaccg aggccgaggc cgccgcgcgc       240
   gccgccgcgg gctgggacaa tcgcccggtt gaaccgccca cgcgcaccac ggggcagatc       300
   gtgcgggaga atcttctcac gtactttaac cttgtgtttc ttgtgctcgc gctgtgcctt       360
   gtggcggtga aggcgtcgat tttgaacctt tcgttcgtca tcgtcgtcat tattaacgcc       420
   gtgatcggca tcgtgcagga gctgcgcagc aaaaaggcgc tcgatgcgct gaatatattg       480
   accgcgccgc aatgcgccgt tgtgcgcgag gggcgcgaaa agcgcgtgga cgtgtcctcg        540
   ctcgtgcggg acgatatcgt cctcttttcg accggcgagc aggtctgcgc cgatgccgtc       600
   gtggtggacg ggacgtgcct tgtcaatgag gcgctcgtca ccggcgaggc ggacgagatc       660
   cgcaaagcgc cgggcgatac gctgctctcg ggcagctttc ttgtctccgg catgtgccgc 720
   gcgcggctca ccgccgtggg cgcggattcg ttcgtcagcc gtctgacgct cgacgcgaaa 780
   gcgcagaaaa agcgtcaggc caagggtatg atgaaggcgc tgaacgatct tgtcaaatgg 840
   atcggcatcg gcatcatccc gatgggcgtg ctgctcgtgg taaaggaagt ctgctggctc 900
   ggccggagca tccccgcagg cgtcacgtcc acggtcggcg cgctcgttgg catgatcccg 960
   gagggcgtgt atctgctcac gagccttgcg cttgtggcgg gtgtgctgcg tctggcacag 1020
   aaaaagacga tgtgccgcga gatggactgc atcgaaacgc tcgcgcgcgt cgatacgctc 1080
   tgcgtggaca agaccggcac ggtgacggaa aacaaaatga tcgtggacga tatcgtcccg 1140
   ctctgccccg accggttcat cgccgaggat atccacatga tcatggcgga ctactgcgcg 1200
   gcgatgcgcg gcgataacga caccatggcc gccatgcgca aattcttcac cggcgaggtg 1260
   aatcagcgcg cgatcgccgc gctcccgttc acgagcgcga agaaatacgg cggcgtttcg 1320
   ttccatgagg atgagaccta tcttctcggc gcgccggagg tcatcctcgg cggcgatttt 1380
   gacgcctatc gggaaaagat cgatcactac agcgcgaagg gctgccgcgt gctgctcctc 1440
   gcgctctacg acgggcagct caccgatgaa gcactcaccg ccgagcgact gcccatctcg 1500
   ctcatattgc tctccaacaa gatccgcgcc gaggcgccgg atacgttcgc gtggtttgcg 1560
   cgcgagggcg tttccgtgcg cgtgatctcc ggcgacaacc cgctcacggt ttccgaggtc 1620
   gcccgccgcg cggggatccc ggacgcggac cgggcggtgg acgcccgcac gctcgaaacc 1680
   gacgagcagg tcgaaaaggc ggcgcgcgaa tgcgtcgtat tcggccgcgt gacgccggag 1740
   atgaagcggc ggctcgtcct tgcgctcaaa aaacaggggc acacggtcgc catgaccggc 1800
   gacggcgtaa acgacgtgct cgcgctcaag gaagccgact gctccatcgc cgtggcctcg 1860
   ggaagcgacg ttgcctgtca ggtatcgcag atcgtgctca cagactcgaa tttcgcctcg 1920
   ctcccggacg tcgtcgccga ggggcggcgc gtcatcaaca acatcgagcg ctccgcctcg 1980
   ctttattttg tgaagaacat gttctcgatc gcgatggcga tcatcagtct gatcttcact 2040
   ctgccgtacc cgctcagccc cgcacagctc ggccttgtga actgcatgac gatcggcgtt 2100
   ccggcgttca tcctcgccat ggagccgaac cgctcgctcg tgcagggcaa attcctcaaa 2160
   aacgtggtgc ggcgcgcgct cccggcggcg ctcacggata tcctgctcgt cggcggcgtg 2220
   atgctcttct atctggccgc cgatctgccg gacaccgcca tgagcacgat ctgtgccggc 2280
   gtgatgggcg ttgtgggact catgatggtg caccagacgg cgaaaccgta caacaagctg 2340
   cgcatcgcga tgatctgcgt actcgcgctc ggctttgcgg ggagctttct gttcctcaag 2400
   gcgcttttca accttgtggc gctcgatttt ctcagcgcgc tcgtgcttgc ggtgttcgcg 2460
   ctgcttgcct ggccggtgat gaatgtgatg tacaagctgc tggataaact cagcgcattc 2520
   gtgcagcggc ggaaaaagac gcgctgcgcg gcgaaagaac tgtaa 2565

## Description

The present invention relates to methods to determine the risk of obesity-related co-morbidities in overweight subjects based on the gut bacterial microbiome, and methods to increase bacterial richness in individuals in need thereof, particularly in overweight individuals.

Overweight and obesity are defined as abnormal or excessive fat accumulation that presents a risk to health. A crude population measure of obesity is the body mass index (BMI), a person's weight (in kilograms) divided by the square of his or her height (in metres). A person with a BMI equal to or more than 25 is considered overweight. A person with a BMI of 30 or more is generally considered obese.

Overweight and obesity are major risk factors for a number of chronic diseases, or so called obesity-related co-morbidities, including diabetes, cardiovascular diseases and cancer. A substantial literature supports that overweight and obesity are major causes of co-morbidities which can lead to further morbidity and mortality. In a recent review, 18 co-morbidities were shown to be statistically correlated with overweight and obesity: cancer (kidney, colorectal, prostate, ovarian, uterine/endometrial, esophageal, pancreatic, and postmenopausal breast), type II diabetes, cardiovascular disease risk (hypertension, coronary artery disease, congestive heart failure, pulmonary embolism, stroke), gallbladder disease, chronic back pain, osteoarthritis and asthma (Guh D. et al., BMC Public Health.; 9: 88; 2009).

This raises serious concern as regards to public health, both from the point of view of the patients, but also from an economic standpoint. Indeed, the above-mentioned diseases most often require extensive medical treatments, or, in the case of chronic diseases, continuous monitoring.

While it has been long recognized that the most efficient strategy to prevent those diseases is weight loss, many different weight loss strategies can be considered, depending on the patient. While classical low calorie diets have been recommended by practitioners for a long time, drastic approaches such as surgery can be necessary in some cases. There is still on-going discussion as to what kind of weight loss strategy is more efficient to alleviate co-morbidity risks. One of the possibilities that seem to surface from different studies is that specific weight loss strategies may need to be tailored to the overweight or obese individual, depending on several factors such as genetic background and hormonal profile. Yet, probably because of the diversity of cases, no clear markers have been identified that could serve as decision-making help for practitioners.

Recent research however points toward another aspect of the issue at hand, which is that the human microbiota plays a crucial role in both the predispositions of different diseases (Clemente et al.,Cell.,148(6):1258-70, 2012).

The human microbiota comprises thousands of bacterial species, among which commensal, beneficial or pathogen bacteria. Humans host microbiota in multiple locations such as skin, lung, vagina, mouth, and gut. Those microbiota are different in their location and in their bacterial composition. The gut microbiota is the largest in its composition. It is generally considered that it comprises thousands of bacterial species, weighs about 1.5 kg and constitutes a rich gene repertoire on its own, also called gut microbiome, 100 times larger than the human nuclear genome.

The gut microbiota has been shown to play a role in the development of metabolic disorders such as obesity, metabolic syndrome, and diabetes. While normbiosis, qualifying the normal state of the microbiota, seems to guaranty homeostasis, disbiosis, which is the distortion from normbiosis, correlates with a long list of diseases.

Recent studies show that the human gut microbiota may be altered in obese relative to lean individuals, even if somewhat inconsistent changes have been reported. An increase in the phylum *Firmicutes* and a decrease of *Bacteroidetes* associated with obesity was observed in some, but not all studies, with the inverse also reported. An increase of *Actinobacteria* in obese was reported as well. Mouse gut microbiota obesity-related alterations are characterized by changes in the *Firmicutes* to *Bacteroidetes* ratio, increased in the obese animals. These changes are likely not a mere consequence of obesity, since the obese phenotype can be transmitted by gut microbiota transplantation in mice, indicating that gut microbial populations may have an active role in obesity pathogenesis, and thus probably with the associated co-morbidities.

However, not all of the bacterial species of the gut microbiota have been identified and sequenced, mostly because most of them cannot be cultured. In addition, most bacteria are only present at a low copy number in the gut microbiota, which makes them difficult to detect (Hamady and Knight, Genome Res., 19: 1141-1152, 2009). Therefore, most sequences in the gut bacterial DNA are not yet taxonomically assigned, which restrains the use as biomarkers to taxonomically known species and genes.

There is thus still a need for markers that would allow the determination of the risk for an overweight subject to develop obesity-related co-morbidities, and moreover to differentiate between overweight subjects in order to adapt the weight-loss strategy to each individual.

### FIGURE LEGEND

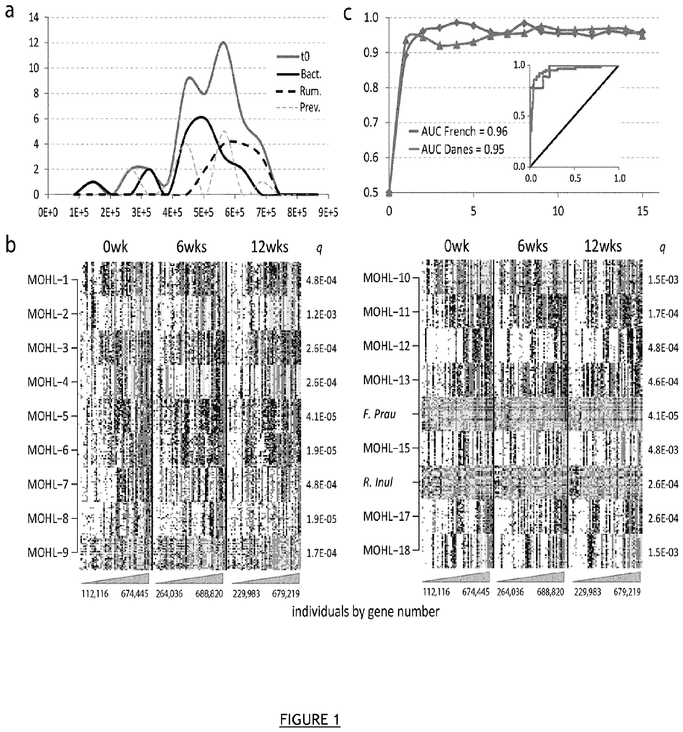

Figure 1. Gut microbial composition of LGC (n=18) and HGC (n=27) subjects.
   a)Gene count distribution in all individuals (red line) or enterotypes (black lines) at baseline, upon downsizing the data to 7 millions of uniquely matched reads. b) Presence and frequency of 25 tracer genes for 18 bacterial species. Rows correspond to genes and the relative frequency of each gene is indicated by color, increasing from light grey to intense grey; white denotes that a gene has not been detected. Columns correspond to individuals, who are ordered by increasing gene number for the 3 time points of the study. Values on the right side of the figure give the Mann-Whitney probability (q: adjusted by Benjamini-Hochberg method) that a species is differentially abundant among the low and high gene individuals; the abundance of a species in an individual was computed as the mean of frequencies of the tracer genes. *F. prau* & *R. inul* stand for *F. prausnitzii* and R. *inulinivorans,* respectively. c) AUC values obtained for the best combinations of 1 to 15 species in a ROC analysis of 45 individuals of our cohort (red); Inset: AUC for the best combination of 6 species. AUC French and AUC Danes refer to French (Micro-obese) and the Danish (MetaHIT) cohorts, respectively.

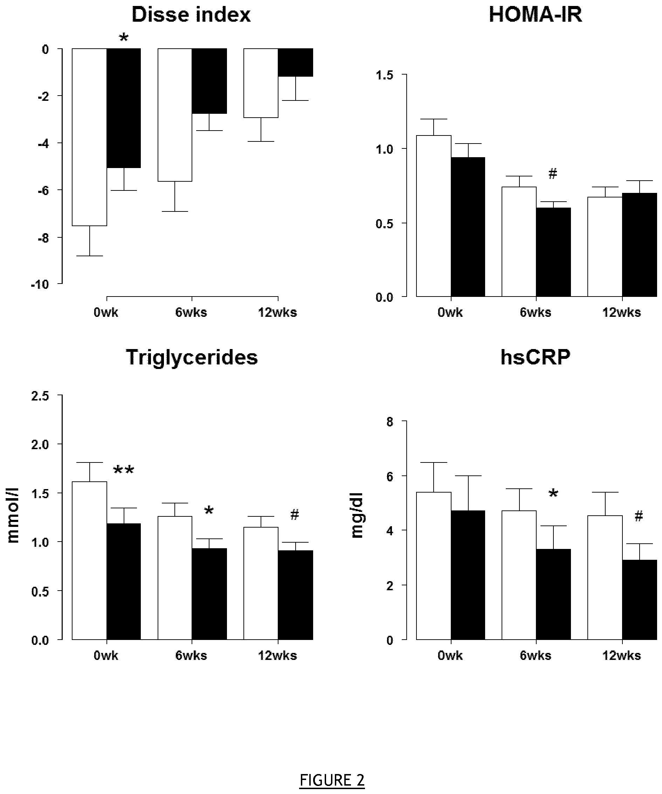

Figure 2. Differences between LGC (n=18) and HGC (n=27) subjects in bioclinical variables.
   White and black bars refer to LGC & HGC groups, respectively; standard errors of the mean are indicated. 0wk: baseline, 6wks: end of the energy restriction period, and 12wks: end of stabilization period. ^{#:} p-value<0.1, *: p-value<0.05, **: p-value<0.01 by Mann-Whitney tests. "Disse index" is calculated by combining lipid and insulin values (see supplementary material).

### DESCRIPTION

The inventors have established that the clinical traits linked to obesity-related co-morbidities are significantly correlated to a reduced gut bacterial diversity in overweight subjects. They moreover designed a method to accurately determine whether an overweight subject has a reduced bacterial diversity. It is thereby possible to determine with a high sensitivity whether an overweight subject is at risk to develop obesity-related co-morbidities.

The present invention is directed to a method for determining whether an overweight subject has a reduced gut bacterial diversity is at risk to develop obesity-related co-morbidities. Such a determination is particularly useful to assess if an overweight subject is at risk of developing obesity-related co-morbidities. The inventors have shown that it is possible to discriminate between overweight subjects having reduced gut bacterial diversity and those having normal gut bacterial diversity by simply assessing the presence of a small number of bacterial species in the gut

The inventors have found a set of specific bacterial species, which presence or absence in the bacterial DNA of the faeces of an overweight subject significantly correlates with reduced gut bacterial diversity. These species are not limited to the ones which have already been known from prior art.

By "reduced gut bacterial diversity", it is herein referred to a gut microbiota in which the number of bacterial species is reduced compared to the average normal gut microbiota.

For example, the comparison between a test microbiota and a normal gut microbiota can be achieved by the genotyping of sequences obtained from the biological samples for example with massively parallel DNA sequencing. In that case, a subject with reduced bacterial diversity can have a microbiome comprising less than 480 000 bacterial gene counts, wherein said counts were obtained by sequencing gut microbial DNA obtained from a sample of 200 mg of faeces with Illumina-based high throughput sequencing, mapping the sequences obtained onto a reference set of bacterial genome (as described in Arumugam et al., Nature., 473(7346):174-80, 2011), removing human contamination, discarding reads mapping at multiple positions, and based on the total amount of remaining matched reads.

According to the invention, a subject has either a reduced gut bacterial diversity, or a normal bacterial diversity. The skilled person would then understand easily that when the method of the invention does not determine that the overweight subject has a reduced gut bacterial diversity, said subject obviously has a normal gut bacterial diversity. By "normal gut bacterial diversity", it is herein referred to a gut microbiota in which the number of bacterial species is around the number found in the average normal gut microbiota, that is to say between 10% inferior and 10% superior to the the number of bacterial species found in the average normal gut microbiota.

By "microbiota", it is herein referred to microflora and microfauna in an ecosystem such as intestines, mouth, vagina, or lungs. In microbiology, flora (plural: floras or floræ) refers to the collective bacteria and other microorganisms in an ecosystem (e.g., some part of the body of an animal host). The "gut microbiota" consists of all the bacterial species constituting the microbiota present in the gut of an individual.

A bacterial species according to the disclosure encompasses not only known bacterial species, but also species which have not yet been taxonomically described. Indeed, whether they already have been taxonomically described or not, bacterial species can be characterized by their genome. For example, methods for characterizing bacteria using genetic information have been described in Vandamme et al. (Microbiol. Rev. 1996, 60(2):407).

It will be obvious to the person skilled in the art that the genes of a bacterial species are physically linked as a unit rather than being independently distributed between individuals, i. e. the genome of said bacterial species comprises gene sequences which are always present or absent together among individuals. Bacterial species can therefore be defined by parts of their genome, and sequencing the entire genome of bacterial species is not necessary for proper bacterial species identification.

For instance, a method for the identification of bacterial species in a microbial composition, based on bacterial DNA sequencing and using marker genes as taxonomic references has been described in Liu et al. (BMC genomics, 12(S2):S4, 2011). The person skilled in the art may further refer to Arumugam et al. (Nature, 473(7346):174-80, 2011) or Qin et al. (Nature, 490(7418):55-60, 2012) for detailed methods for the identification of bacterial species based on bacterial DNA sequencing.

According to the present invention a "bacterial species" is a group of bacterial genes from the gut microbiome, which abundance level varies in the same proportion among different individual samples. In other words, a bacterial species according to the disclosure is a cluster of bacterial gene sequences which abundance levels in samples from distinct subjects are statistically linked rather than being randomly distributed. It will be immediately apparent to the skilled person that such a cluster thus corresponds to a bacterial species.

Genes of the microbiome can be ascribed to a bacterial species by several statistical methods known to the person skilled in the art. Preferably, a statistical method for testing covariance is used for testing whether two genes belong to the same cluster. To this end, the skilled person may use non-parametrical measures of statistical dependence, such as the Spearman's rank correlation coefficient for example. Most preferably, a bacterial species according to the disclosure is a cluster that comprises gut bacterial genes and that is determined by the method used in Qin et al. (Nature, 490(7418): 55-60, 2012) for identifying metagenomic linkage groups.

By "subject", it is herein referred to a vertebrate, preferably a mammal, and most preferably a human. By "overweight subject", it is herein referred to a human being having a body mass index superior to 25 kg/m². The Body mass index is defined as the individual's body mass divided by the square of his or her height. The formulae universally used in medicine produce a unit of measure of kg/m2.

There are several ways to obtain samples of the said subject's gut microbial DNA (Sokol et al., Inflamm. Bowel Dis., 14(6): 858-867, 2008). For example, it is possible to prepare mucosal specimens, or biopsies, obtained by coloscopy. However, coloscopy is an invasive procedure which is ill-defined in terms of collection procedure from study to study. Likewise, it is possible to obtain biopies through surgery. However, even more than coloscopy, surgery is an invasive procedure, which effects on the microbial population are not known. Preferred is the fecal analysis, a procedure which has been reliably been used in the art (Bullock et al., Curr Issues Intest Microbiol.; 5(2): 59-64, 2004; Manichanh et al., Gut, 55: 205-211, 2006; Bakir et al., Int J Syst Evol Microbiol, 56(5): 931-935, 2006; Manichanh et al., Nucl. Acids Res., 36(16): 5180-5188, 2008; Sokol et al., Inflamm. Bowel Dis., 14(6): 858-867, 2008). An example of this procedure is described in the Methods section of the Experimental Examples. Feces contain about 1011 bacterial cells per gram (wet weight) and bacterial cells comprise about 50 % of fecal mass. The microbiota of the feces represents primarily the microbiology of the distal large bowel. It is thus possible to isolate and analyze large quantities of microbial DNA from the feces of an individual. By "gut microbial DNA", it is herein understood the DNA from any of the resident bacterial communities of the human gut. The term "gut microbial DNA" encompasses both coding and non-coding sequences; it is in particular not restricted to complete genes, but also comprises fragments of coding sequences. Fecal analysis is thus a non-invasive procedure, which yields consistent and directly-comparable results from patient to patient.

As explained above, "gut microbiome", as used herein, refers to the set of bacterial genes from the species constituting the microbiota present in the gut of said subject. The sequences of the microbiome of the disclosure comprise at least gene sequences from the bacterial gene catalogue published by Qin et al. (Nature, 464: 59-65, 2010). The gene sequences from the catalogue are available from the EMBL (http:///www.bork.embl.de/∼arumugam/Qin_et_al_2010/) and BGI (http://gutmeta.genomics.org.cn) websites.

These species are not limited to the ones which have already been known from prior art. Importantly, these specific bacterial species show a high correlation coefficient with reduced gut bacterial diversity. It is thus possible to determine whether a subject has reduced gut bacterial diversity with a high sensitivity. The sensitivity of a method is the proportion of actual positives which are correctly identified as such, and can be estimated by the area under the ROC (Receiver Operating Characteristic) curve, also called AUC. A receiver operating characteristic (ROC), or simply ROC curve, is a graphical plot which illustrates the performance of a binary classifier system as its discrimination threshold is varied. It is created by plotting the fraction of true positives out of the positives (TPR = true positive rate) vs. the fraction of false positives out of the negatives (FPR = false positive rate), at various threshold settings. TPR is also known as sensitivity, and FPR is one minus the specificity or true negative rate. Area Under the Curve (AUC) is a measure of a classifier/test performance across all possible values of the thresholds. The higher the AUC, the better the performance of the test.

The inventors have found that it is not necessary to determine the presence or the absence of every single of the said bacterial species in order to assess the diversity of the gut bacterial population. Rather, said diversity can be evaluated with a high degree of confidence and accuracy by examining a very small subset of bacterial species. As shown in the experimental part, a very small number of species is a good marker of the said diversity. Indeed, even when the presence or absence of only one bacterial species is assessed, the method of the invention enables the detection of reduced bacterial diversity in a subject with an AUC of at least 0.74, and can be up to 0.89, depending of the bacterial species chosen.

In comparison, a random method usually has an AUC of 0.5. Moreover, when inflammatory bowel disease, one of the pathologies associated with reduced bacterial diversity, is assessed by 16S rRNA sequencing of fecal samples, the AUC is of only 0.83 (Papa et al; PLoS One. 2012;7(6):e39242. 2012).

According to a first embodiment of the invention, the method for determining whether an overweight subject has a reduced gut bacterial diversity comprises a step of detecting from a gut microbial DNA sample obtained from said subject whether at least one gene from at least one bacterial species from Table 1 is absent in said sample.

The bacterial species of the disclosure are chosen from the list consisting in the bacterial species of Table 1. More precisely, the bacterial species of the disclosure are chosen from the list consisting in MOHL-1, MOHL-2, MOHL-3, MOHL-4, MOHL-5, MOHL-6, MOHL-7, MOHL-8, MOHL-9, MOHL-10, MOHL-11, MOHL-12, MOHL-13, MOHL-14, MOHL-15, MOHL-16, MOHL-17, MOHL-18.

By "at least one bacterial species", it is herein meant that the absence of one unique species or of more than one species is assessed. In a preferred embodiment, the method of the invention includes the detection of the absence of 1, 2, 2, 4, or 5 bacterial species. Even more preferably, the said method includes the detection of the absence of more than 5 bacterial species. Most preferably, the said method includes the detection of the absence of 18 bacterial species.

Most intestinal commensals cannot be cultured. Genomic strategies have been developed to overcome this limitation (Hamady and Knight, Genome Res, 19: 1141-1152, 2009). These strategies have allowed the definition of the microbiome as the collection of the genes comprised in the genomes of the microbiota (Turnbaugh et al., Nature, 449: 804-8010, 2007; Hamady and Knight, Genome Res., 19: 1141-1152, 2009). The existence of a small number of species shared by all individuals constituting the human intestinal microbiota phylogenetic core has been demonstrated (Tap et al., Environ Microbiol., 11(10): 2574-2584, 2009). Recently, a metagenomic analysis has led to the identification of an extensive catalogue of 3.3 million non-redundant microbial genes of the human gut, corresponding to 576.7 gigabases of sequence (Qin et al., Nature, 464(7285): 59-65, 2010).

It will be immediately apparent to the person of skills in the art that the presence of a bacterial species can be easily determined by detecting a nucleic acid sequence specific of the said species. The presence of gut bacterial species is usually determined by detecting 16S rRNA gene sequences. However, this method is limited to known bacterial species.

By contrast, in the method of the invention, no prior identification of the bacterial species the said gene belongs to is required. The inventors have determined a minimum set of 25 bacterial gene sequences that are non-redundant sequences for each bacterial species of Table 1, and that can be used as tracer genes.

**Table 1: bacterial species absent in overweight subjects with reduced bacterial gut diversity, and sequences comprised therein.**

| Bacterial species | Sequences |
|---|---|
| MOHL-1 | SEQ ID NO. 1 to 25 |
| MOHL-2 | SEQ ID NO. 26 to 50 |
| MOHL-3 | SEQ ID NO. 51 to 75 |
| MOHL-4 | SEQ ID NO. 76 to 100 |
| MOHL-5 | SEQ ID NO. 101 to 125 |
| MOHL-6 | SEQ ID NO. 126 to 150 |
| MOHL-7 | SEQ ID NO. 151 to 175 |
| MOHL-8 | SEQ ID NO. 176 to 200 |
| MOHL-9 | SEQ ID NO. 201 to 225 |
| MOHL-10 | SEQ ID NO. 226 to 250 |
| MOHL-11 | SEQ ID NO. 251 to 275 |
| MOHL-12 | SEQ ID NO. 276 to 300 |
| MOHL-13 | SEQ ID NO. 301 to 325 |
| MOHL-14 | SEQ ID NO. 326 to 350 |
| MOHL-15 | SEQ ID NO. 351 to 375 |
| MOHL-16 | SEQ ID NO. 376 to 400 |
| MOHL-17 | SEQ ID NO. 401 to 425 |
| MOHL-18 | SEQ ID NO. 426 to 450 |

It will be obvious to the person skilled in the art that the number of bacteria from a given bacterial species in a sample directly correlate with the number of copies of at least one gene sequence detected in said sample. It is thereby possible to determine the absence of at least one of the bacterial species from Table 1, simply by detecting the absence of at least one bacterial gene from said species.

The invention therefore enables assessing reduced gut bacterial diversity in an overweight subject, and thereby the risk for said subject to develop obesity-related co-morbidities without the need for complex and tedious statistical analysis. Moreover, because the method of the invention can rely on as little as one bacterial gene as a marker, it may be implemented by any known technique of DNA amplification or sequencing, and is not limited to a specific method or apparatus.

Another embodiment of the invention is a method for determining whether an overweight subject has a reduced gut bacterial diversity, said method comprising:
a) detecting from a gut microbial DNA sample obtained from said subject whether at least one of the sets of 25 bacterial genes from at least one bacterial species from Table 1 is absent in said sample, and
b) determining that the subject has a reduced gut bacterial diversity, if at least one of the sets of 25 bacterial genes from at least one at least one bacterial species from Table 1 is absent in said sample.
In a preferred embodiment, the bacterial genes sequences of the bacterial species according to the invention are chosen in the list consisting of sequence SEQ ID NO.1 to sequence SEQ ID NO. 450

Depending on the size of the sample and of the occurrence of the bacterial genes of interest, certain bacterial genes may be difficult to detect in a sample. The skilled person would thus easily conceive that, to increase the confidence of the results, it is advantageous to determine the absence of a bacterial species by detecting the average abundance of several bacterial genes from a bacterial species.

In an embodiment, detecting whether at least one bacterial gene from at least a bacterial species from Table 1 is absent in said sample comprises determining the number of copies of at least 1, 2, 3, 4 or 5 bacterial gene from said bacterial species in the sample. In a preferred embodiment, detecting whether at least one bacterial gene from at least one bacterial species from Table 1 is absent in said sample comprises determining the number of copies of at least 10, 15, 20 or at least 25 bacterial genes from said bacterial species in the sample.

Moreover, among all of the bacterial genes, some bacterial species are more significantly correlated with reduced gut bacterial diversity. For example, as shown in Table 3 of the experimental part, the detection of the presence or absence of one of the bacterial species among MO_HL_5, MO_HL_6, MO_HL_14, MO_HL_9, MO_HL_11, MO_HL_13, MO_HL_3, MO_HL_8, MO_HL_16, MO_HL_4, MO_HL_17, MO_HL_1, or MO_HL_7 enables the detection of reduced bacterial diversity in a subject with an AUC superior to 0.83.

It is thereby possible to increase the sensitivity of the method of the invention, simply by assessing the presence or absence of those specific bacterial species, or of a least one gene from the specific bacterial species they belong to.

In an advantageous embodiment, the method of the invention comprises a step of detecting from a gut microbial DNA sample obtained from said subject whether at least one gene from a bacterial species chosen from the list consisting in MO_HL_5, MO_HL_6, MO_HL_14, MO_HL_9, MO_HL_11, MO_HL_13, MO_HL_3, MO_HL_8, MO_HL_16, MO_HL_4, MO_HL_17, MO_HL_1, or MO_HL_7 from Table 1 is absent in said sample.

In a particularly advantageous embodiment, the method of the invention comprises a step of detecting from a gut microbial DNA sample obtained from said subject whether at least one gene from the bacterial species MOHL-5 from Table 1 is absent in said sample. The person skilled in the art knows that the more distinct bacterial species from Table 1 are present in the bacterial DNA from the feces of the subject, the higher the probability that the subjects has a reduced gut bacterial diversity. It would then be obvious to the skilled person that the sensitivity of the method of the invention can be increased by assessing the absence of bacterial genes from several different bacterial species from Table 1. It is then possible to increase the sensitivity of the method by using bacterial genes from a linear combination of 2, 3, 4, 5 or more different bacterial species. For example, the combinations of 2 bacterial species from Table 1 enable an AUC between around 0.775 and 0.948, the combinations of 3 bacterial species from Table 1 enable an AUC between around 0.813 and 0.993, and the combinations of 4 bacterial species from Table 1 and enable an AUC between around 0.827 and 0.987. However, the inventors have surprisingly discovered that the detection of specific combinations of 2, 3 or 4 bacterial species enables for very high AUC. The more advantageous combinations of 2, 3 and 4 bacterial species are indicated in Table 3, 4 and 5 respectively.

In a preferred embodiment, the method comprises a step of detecting from a gut microbial DNA sample obtained from said subject whether at least one gene from each of the bacterial species of any of the bacterial species combinations indicated in Table 3, 4 and/or 5 is absent in said sample.

The inventors have additionally selected bacterial species combinations of 2 to 18 bacterial species that enables for particularly important AUC, indicated in Table 6, ranging from around 0.89 to 0.99.

In an advantageous embodiment, the method of the invention comprises a step of detecting from a gut microbial DNA sample obtained from said subject whether:
- at least one of the set of 25 bacterial genes from each of the bacterial species MO_HL_12 and MO_HL_11 from Table 1 are absent in said sample, or;
- at least one of the set of 25 bacterial genes from each of the bacterial species MO_HL_11, MO_HL_7 and MO_HL_12 from Table 1 are absent in said sample, or;
- at least one of the set of 25 bacterial genes from each of the bacterial species MO_HL_11, MO_HL_5, MO_HL_7 and MO_HL_12 from Table 1 are absent in said sample, or;
- at least one of the set of 25 bacterial genes from each of the bacterial species MO_HL_11, MO_HL_8, MO_HL_12, MO_HL_7 and MO_HL_2from Table 1 are absent in said sample, or;
- at least one of the set of 25 bacterial genes from each of the bacterial species MO_HL_17, MO_HL_5, MO_HL_12, MO_HL_7, MO_HL_11and MO_HL_8 from Table 1 are absent in said sample, or;
- at least the set of 25 bacterial genes from each of the bacterial species MO_HL12, MO_HL_11, MO_HL_2, MO_HL_5, MO_HL_8, MO_HL_13 and MO_HL_7 from Table 1 are absent in said sample, or;
- at least one of the set of 25 bacterial genes from each of the bacterial species MO_HL_12, MO_HL_2, MO_HL_17, MO_HL_11, MO_HL_13, MO_HL_5, MO_HL_8 and MO_HL_7 from Table 1 are absent in said sample, or;
- at least one gene from each of the bacterial species MO_HL_4, MO_HL_11, MO_HL_6, MO_HL_13, MO_HL_2, MO_HL_8, MO_HL_12, MO_HL_7 and MO_HL_17 from Table 1 are absent in said sample, or;
- at least one of the set of 25 bacterial genes from each of the bacterial species MO HL_8, MO HL_11, MO_HL_6, MO_HL_4, MO_HL_13, MO_HL_5, MO_HL_2, MO_HL_7, MO_HL_17 and MO_HL_12 from Table 1 are absent in said sample;
- at least one of the set of 25 bacterial genes from each of the bacterial species MO_HL_5, MO_HL_13, MO_HL_4, MO_HL_12, MO_HL_15, MO_HL_17, MO_HL_6, MO_HL_2, MO_HL_8, MO_HL_7 and MO_HL_11 from Table 1 are absent in said sample;
- at least one of the set of 25 bacterial genes from each of the bacterial species MO_HL_11, MO_HL_6, MO_HL_17, MO_HL_4, MO_HL_3, MO_HL_12, MO_HL_5, MO_HL_10, MO_HL_8, MO_HL_7, MO_HL_2 and MO_HL_13 from Table 1 are absent, in said sample;
- at least one of the set of 25 bacterial genes from each of the bacterial species MO HL_18, MO_HL_15, MO_HL_11, MO_HL_10, MO_HL_5, MO_HL_4, MO_HL_6, MO_HL_8, MO_HL_12, MO_HL_7, MO_HL_2, MO_HL_13 and MO_HL_3 from Table 1 are absent in said sample, or;
- at least one of the set of 25 bacterial genes from each of the bacterial species MO_HL_6, MO_HL_17, MO_HL_3, MO_HL_2, MO_HL_4, MO_HL_18, MO_HL_5, MO_HL_13, MO_HL_10, MO_HL_15, MO_HL_7, MO_HL_1, MO_HL_8 and MO_HL_12from Table 1 are absent in said sample, or;
- at least one of the set of 25 bacterial genes from each of the bacterial species MO HL_10, MO_HL_18, MO_HL_2, MO_HL_13, MO_HL_7, MO_HL_1, MO_HL_11, MO_HL_3, MO_HL_4, MO_HL_15, MO_HL_5, MO_HL_6, MO_HL_17, MO_HL_12 and MO_HL_8 from Table 1 are absent, in said sample;
- at least one of the set of 25 bacterial genes from each of the bacterial species MO_HL_7, MO_HL_4, MO_HL_13, MO_HL_1, MO_HL_5, MO_HL_6, MO_HL_11, MO_HL_12, MO_HL_8, MO_HL_3, MO_HL_18, MO_HL_2, MO_HL_10, MO_HL_17, MO_HL_15 and MO_HL_9 from Table 1 are absent in said sample, or;
- at least one of the set of 25 bacterial genes from each of the bacterial species MO_HL_10, MO_HL_8, MO_HL_1, MO_HL_16, MO_HL_18, MO_HL_5, MO_HL_15, MO_HL_2, MO_HL_7, MO_HL_6, MO_HL_13, MO_HL_11, MO_HL_17, MO_HL_3, MO_HL_14, MO_HL_4 and MO_HL_12 from Table 1 are absent, in said sample;
- at least one of the set of 25 bacterial genes from each of the bacterial species MO_HL15, MO_HL_17, MO_HL_4, MO_HL_14, MO_HL_16, MO_HL_10, MO_HL_7, MO_HL_6, MO_HL_11, MO_HL_9, MO_HL_5, MO_HL_3, MO_HL_8, MO_HL_1, MO_HL_12, MO_HL_18, MO_HL_13 and MO_HL_2from Table 1 are absent, in said sample.

A bacterial gene is absent from the sample when its number of copies in the sample is inferior to a certain threshold value. Accordingly, a bacterial gene is present in the sample when its number of copies in the sample is inferior to a certain threshold value.

According to the present invention, a "threshold value" is intended to mean a value that permits to discriminate samples in which the number of copies of the bacterial gene of interest is low or high.

In particular, if a number of copies of a bacterial gene of interest is inferior or equal to the threshold value, then the number of copies of this bacterial gene in the sample is considered low, whereas if the number of copies is superior to the threshold value, then the number of copies of this bacterial gene in the sample is considered high. A low copy number means that the bacterial gene is absent from the sample, whereas a high number of copies means that the bacterial gene is present in the sample.

For each gene, and depending on the method used for measuring the number of copies of the bacterial gene, the optimal threshold value may vary. However, it may be easily determined by a skilled person based on the analysis of the microbiome of several individuals in which the number of copies (low or high) is known for this particular bacterial gene, and on the comparison thereof with the number of copies of a control gene. Such a comparison may be facilitated by using the same amount of bacterial DNA for each of the analyzed samples, or by dividing the number of copies of the bacterial gene obtained, by the initial amount of bacterial DNA used in the test. Indeed, it is well known from the skilled person that the total amount of bacteria in the gut of a subject, and consequently in its feces, remains the same even in the case of reduced bacterial diversity. It is also possible to use a reference such as a gut bacterial species whose abundance is known not to vary between individuals with reduced and normal bacterial diversity.

According to the invention, determining the number of copies of at least one of the set of 25 bacterial genes from at least one bacterial species as disclosed in Tab. 1 in a sample obtained from the subject can be achieved by any technique capable of detecting and quantifying nucleic acids sequences, and include *inter alia* hybridization with a labelled probe, PCR amplification, sequencing, and all other methods known to the person of skills in the art.

In a first embodiment, determining the number of copies of at least one bacterial gene in a sample obtained from the subject is performed using sequencing. Optionally, DNA is be fragmented, for example by restriction nuclease prior to sequencing. Sequencing is done using any technique known in the state of the art, including sequencing by ligation, pyrosequencing, sequencing-by-synthesis or single-molecule sequencing. Sequencing also includes PCR-Based techniques, such as for example quantitative PCR or emulsion PCR.

Sequencing is performed on the entire DNA contained in the biological sample, or on portions of the DNA contained in the biological sample. It will be immediately clear to the skilled person that the said sample contains at least a mixture of bacterial DNA and of human DNA from the host subject. However, though the overall bacterial DNA is likely to represent the major fraction of the total DNA present in the sample, each bacterial species may only represent a small fraction of the total DNA present in the sample.

To overcome this difficulty, the skilled person can use a method that allows the quantitative genotyping of sequences obtained from the biological sample with high precision. In one embodiment of this approach, the precision is achieved by analysis of a large number (for example, millions or billions) of polynucleotides. Furthermore, the precision can be enhanced by the use of massively parallel DNA sequencing, such as, but not limited to that performed by the Illumina Genome Analyzer platform (Bentley et al. Nature; 456: 53-59, 2008), the Roche 454 platform (Margulies et al. Nature; 437: 376-380, 2005), the ABI SOLiD platform (McKernan et al., Genome Res; 19: 1527-1541, 2009), the Helicos single molecule sequencing platform (Harris et al. Science; 320: 106-109, 2008), real-time sequencing using single polymerase molecules (Science; 323: 133-138, 2009), Ion Torrent sequencing (WO 2010/008480; Rothberg et al., Nature, 475: 348-352, 2011) and nanopore sequencing (Clarke J et al. Nat Nanotechnol.; 4: 265-270, 2009).

When the skilled person relies on sequencing methods to detect the presence or absence of certain bacterial genes, the information collected from sequencing is used to determine the number of copies of nucleic acid sequences of interest via bioinformatics procedures. For example, in an embodiment, the nucleic acid sequences of said bacterial species in the gut bacterial DNA sample are identified in the global sequencing data by comparison with the nucleic acid sequences SEQ ID NO.1 to SEQ ID NO. 450. This comparison is advantageously based on the level of sequence identity with the sequences SEQ ID NO.1 to SEQ ID NO. 450.

Thus, a nucleic acid sequence displaying at least 90 %, at least 95 %, at least 96 %, at least 97 %, at least 98 %, at least 99 %, or 100 % identity with at least one of the nucleic acid sequences SEQ ID NO. 1 to SEQ ID NO. 450 is identified as a sequence comprised in one of the bacterial species of the disclosure. Thus, in a preferred embodiment, detecting whether at least one bacterial species from Table 1 is absent in said sample comprises determining the number of nucleic acid sequences in the gut bacterial DNA sample having at least 90 %, at least 95 %, at least 96 %, at least 97 %, at least 98 %, at least 99 %, or 100 % identity with at least one of the nucleic acid sequences SEQ ID NO. 1 to SEQ ID NO. 450.

The term "sequence identity" herein refers to the identity between two nucleic acids sequences. Identity between sequences can be determined by comparing a position in each of the sequences which may be aligned for the purposes of comparison. When a position in the compared sequences is occupied by the same base, then the sequences are identical at that position. A degree of sequence identity between nucleic acid sequences is a function of the number of identical nucleotides at positions shared by these sequences.

To determine the percent identity of two amino acids sequences, the sequences are aligned for optimal comparison. For example, gaps can be introduced in the sequence of a first nucleic acid sequence for optimal alignment with the second nucleic acid sequence. The nucleotides at corresponding nucleotide positions are then compared. When a position in the first sequence is occupied by the same nucleotide as the corresponding position in the second sequence, the molecules are identical at that position. The percent identity between the two sequences is a function of the number of identical positions shared by the sequences. Hence % identity = number of identical positions/total number of overlapping positions X 100.

In this comparison the sequences can be the same length or can be different in length. Optimal alignment of sequences for determining a comparison window may be conducted by the local homology algorithm of Smith and Waterman (J. Theor. Biol., 91(2): 370-380, 1981), by the homology alignment algorithm of Needleman and Wunsch (J. Mol. Biol, 48(3): 443-453, 1972), by the search for similarity via the method of Pearson and Lipman (Proc. Natl. Acad. Sci. U.S.A., 85(5): 2444-2448, 1988), by computerized implementations of these algorithms (GAP, BESTFIT, FASTA and TFASTA in the Wisconsin Genetics Software Package Release 7.0, Genetic Computer Group, 575, Science Drive, Madison, Wisconsin) or by inspection. The best alignment (i.e. resulting in the highest percentage of identity over the comparison window) generated by the various methods is selected.

The term "sequence identity" thus means that two polynucleotide sequences are identical (i.e. on a nucleotide by nucleotide basis) over the window of comparison. The term "percentage of sequence identity" is calculated by comparing two optimally aligned sequences over the window of comparison, determining the number of positions at which the identical nucleic acid base (e.g. A, T, C, G, U, or I) occurs in both sequences to yield the number of matched positions, dividing the number of matched positions by the total number of positions in the window of comparison (i.e. the window size) and multiplying the result by 100 to yield the percentage of sequence identity. The same process can be applied to polypeptide sequences. The percentage of sequence identity of a nucleic acid sequence or an amino acid sequence can also be calculated using BLAST software (Version 2.06 of September 1998) with the default or user defined parameter.

In another preferred embodiment, PCR-based techniques are used to determine the number of copies of at least one bacterial gene. Preferably, the PCR technique used quantitatively measures starting amounts of DNA, cDNA, or RNA. Examples of PCR-based techniques according to the invention include techniques such as, but not limited to, quantitative PCR (Q-PCR), reverse-transcriptase polymerase chain reaction (RT-PCR), quantitative reverse-transcriptase PCR (QRT-PCR), rolling circle amplification (RCA) or digital PCR. These techniques are well known and easily available technologies for those skilled in the art and do not need a precise description. In a preferred embodiment, the determination of the copy number of the bacterial genes used in the invention is performed by quantitative PCR.

Amplification primers specific for the genes to be tested are thus also very useful for performing the methods according to the invention. The present disclosure thus also encompasses primers for amplifying at least one gene selected from the genes of sequence SEQ ID NO. 1-450.

In another preferred embodiment, the presence or absence of the bacterial genes according to the disclosure is detected by the use of a nucleic microarray. A "nucleic microarray" consists of different nucleic acid probes that are attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes can be nucleic acids such as cDNAs ("cDNA microarray") or oligonucleotides ("oligonucleotide microarray"), and the oligonucleotides may be about 25 to about 60 base pairs or less in length.

To determine the copy number of a target nucleic sample, said sample is labelled, contacted with the microarray in hybridization conditions, leading to the formation of complexes between target nucleic acids that are complementary to probe sequences attached to the microarray surface. The presence of labelled hybridized complexes is then detected. Many variants of the microarray hybridization technology are available to the man skilled in the art.

In a specific embodiment, the nucleic microarray is an oligonucleotide microarray comprising at least one oligonucleotide specific for at least one gene having a sequence selected from SEQ ID NOs 1-450. Preferably, the said microarray comprises at least 18 oligonucleotides, each oligonucleotide being specific for one gene of a distinct cluster of the invention. More preferably, the microarray of the invention consists of 450 oligonucleotides specific for each of the genes of sequences SEQ ID NOs. 1-450.

In another embodiment, the nucleic microarray is an oligonucleotide microarray comprising at least one oligonucleotide specific for at least one gene of each of the bacterial species MO_HL_12 and MO_HL_11. Preferably, the nucleic microarray is an oligonucleotide microarray comprising or consisting in oligonucleotides specific for at least 2, 3, 4, 5, 10, 20, or 25 genes of each of the bacterial species MO_HL_12 and MO_HL_11.

In another embodiment, the nucleic microarray is an oligonucleotide microarray comprising at least one oligonucleotide specific for at least one gene of each of the bacterial species MO_HL_11, MO_HL_7 and MO_HL_12. Preferably, the nucleic microarray is an oligonucleotide microarray comprising or consisting in oligonucleotides specific for at least 2, 3, 4, 5, 10, 20, or 25 genes of each of the bacterial species MO_HL_11, MO_HL_7 and MO_HL_12.

In another embodiment, the nucleic microarray is an oligonucleotide microarray comprising at least one oligonucleotide specific for at least one gene of each of the bacterial species MO_HL_11, MO_HL_5, MO_HL_7 and MO_HL_12. Preferably, the nucleic microarray is an oligonucleotide microarray comprising or consisting in oligonucleotides specific for at least 2, 3, 4, 5, 10, 20, or 25 genes of each of the bacterial species MO_HL_11, MO_HL_5, MO_HL_7 and MO_HL_12.

In another embodiment, the nucleic microarray is an oligonucleotide microarray comprising at least one oligonucleotide specific for at least one gene of each of the bacterial species MO_HL_11, MO_HL_8, MO_HL_12, MO_HL_7 and MO_HL_2. Preferably, the nucleic microarray is an oligonucleotide microarray comprising or consisting in oligonucleotides specific for at least 2, 3, 4, 5, 10, 20, or 25 genes of each of the bacterial species MO_HL_11, MO_HL_8, MO_HL_12, MO_HL_7 and MO_HL_2

In another embodiment, the nucleic microarray is an oligonucleotide microarray comprising at least one oligonucleotide specific for at least one gene of each of the bacterial species MO_HL_17, MO_HL_5, MO_HL_12, MO_HL_7, MO_HL_11and MO_HL_8. Preferably, the nucleic microarray is an oligonucleotide microarray comprising or consisting in oligonucleotides specific for at least 2, 3, 4, 5, 10, 20, or 25 genes of each of the bacterial species MO_HL_17, MO_HL_5, MO_HL_12, MO_HL_7, MO_HL_1 land MO_HL_8.

In another embodiment, the nucleic microarray is an oligonucleotide microarray comprising at least one oligonucleotide specific for at least one gene of each of the bacterial species MO_HL_12, MO_HL_11, MO_HL_2, MO_HL_5, MO_HL_8, MO_HL_13 and MO_HL_7. Preferably, the nucleic microarray is an oligonucleotide microarray comprising or consisting in oligonucleotides specific for at least 2, 3, 4, 5, 10, 20, or 25 genes of each of the bacterial species MO_HL_12, MO_HL_11, MO_HL_2, MO_HL_5, MO_HL_8, MO_HL_13 and MO_HL_7.

In another embodiment, the nucleic microarray is an oligonucleotide microarray comprising at least one oligonucleotide specific for at least one gene of each of the bacterial species MO_HL_12, MO_HL_2, MO_HL_17, MO_HL_11, MO_HL_13, MO_HL_5, MO_HL_8 and MO_HL_7. Preferably, the nucleic microarray is an oligonucleotide microarray comprising or consisting in oligonucleotides specific for at least 2, 3, 4, 5, 10, 20, or 25 genes of each of the bacterial species MO_HL_12, MO_HL_2, MO_HL_17, MO_HL_11, MO_HL_13, MO_HL_5, MO_HL_8 and MO_HL_7.

In another embodiment, the nucleic microarray is an oligonucleotide microarray comprising at least one oligonucleotide specific for at least one gene of each of the bacterial species MO_HL_4, MO_HL_11, MO_HL_6, MO_HL_13, MO_HL_2, MO_HL_8, MO_HL_12, MO_HL_7 and MO_HL_17. Preferably, the nucleic microarray is an oligonucleotide microarray comprising or consisting in oligonucleotides specific for at least 2, 3, 4, 5, 10, 20, or 25 genes of each of the bacterial species MO_HL_4, MO_HL_11, MO_HL_6, MO_HL_13, MO_HL_2, MO_HL_8, MO_HL_12, MO_HL_7 and MO_HL_17.

In another embodiment, the nucleic microarray is an oligonucleotide microarray comprising at least one oligonucleotide specific for at least one gene of each of the bacterial species MO_HL_8, MO_HL_11, MO_HL_6, MO_HL_4, MO_HL_13, MO_HL_5, MO_HL_2, MO_HL_7, MO_HL_17 and MO_HL_12. Preferably, the nucleic microarray is an oligonucleotide microarray comprising or consisting in oligonucleotides specific for at least 2, 3, 4, 5, 10, 20, or 25 genes of each of the bacterial species MO_HL_8, MO_HL_11, MO_HL_6, MO_HL_4, MO_HL_13, MO_HL_5, MO_HL_2, MO_HL_7, MO_HL_17 and MO_HL_12.

In another embodiment, the nucleic microarray is an oligonucleotide microarray comprising at least one oligonucleotide specific for at least one gene of each of the bacterial species MO_HL_5, MO_HL_13, MO_HL_4, MO_HL_12, MO_HL_15, MO_HL_17, MO_HL_6, MO_HL_2, MO_HL_8, MO_HL_7 and MO_HL_11. Preferably, the nucleic microarray is an oligonucleotide microarray comprising or consisting in oligonucleotides specific for at least 2, 3, 4, 5, 10, 20, or 25 genes of each of the bacterial species MO_HL_5, MO_HL_13, MO_HL_4, MO_HL_12, MO_HL_15, MO_HL_17, MO_HL_6, MO_HL_2, MO_HL_8, MO_HL_7 and MO_HL_11.

In another embodiment, the nucleic microarray is an oligonucleotide microarray comprising at least one oligonucleotide specific for at least one gene of each of the bacterial species MO_HL_11, MO_HL_6, MO_HL_17, MO_HL_4, MO_HL_3, MO_HL_12, MO_HL_5, MO_HL_10, MO_HL_8, MO_HL_7, MO_HL_2 and MO_HL_13. Preferably, the nucleic microarray is an oligonucleotide microarray comprising or consisting in oligonucleotides specific for at least 2, 3, 4, 5, 10, 20, or 25 genes of each of the bacterial species MO_HL_11, MO_HL_6, MO_HL_17, MO_HL_4, MO_HL_3, MO_HL_12, MO_HL_5, MO_HL_10, MO_HL_8, MO_HL_7, MO_HL_2 and MO_HL_13.

In another embodiment, the nucleic microarray is an oligonucleotide microarray comprising at least one oligonucleotide specific for at least one gene of each of the bacterial species MO_HL_18, MO_HL_15, MO_HL_11, MO_HL_10, MO_HL_5, MO_HL_4, MO_HL_6, MO_HL_8, MO_HL_12, MO_HL_7, MO_HL_2, MO_HL_13 and MO_HL_3 Preferably, the nucleic microarray is an oligonucleotide microarray comprising or consisting in oligonucleotides specific for at least 2, 3, 4, 5, 10, 20, or 25 genes of each of the bacterial species MO_HL_18, MO_HL_15, MO_HL_11, MO_HL_10, MO_HL_5, MO_HL_4, MO_HL_6, MO_HL_8, MO_HL_12, MO_HL_7, MO_HL_2, MO_HL_13 and MO_HL_3.

In another embodiment, the nucleic microarray is an oligonucleotide microarray comprising at least one oligonucleotide specific for at least one gene of each of the bacterial species MO_HL_6, MO_HL_17, MO_HL_3, MO_HL_2, MO_HL_4, MO_HL_18, MO_HL_5, MO_HL_13, MO_HL_10, MO_HL_15, MO_HL_7, MO_HL_1, MO_HL_8 and MO_HL_12. Preferably, the nucleic microarray is an oligonucleotide microarray comprising or consisting in oligonucleotides specific for at least 2, 3, 4, 5, 10, 20, or 25 genes of each of the bacterial species MO_HL_6, MO_HL_17, MO_HL_3, MO_HL_2, MO_HL_4, MO_HL_18, MO_HL_5, MO_HL_13, MO_HL_10, MO_HL_15, MO_HL_7, MO_HL_1, MO_HL_8 and MO_HL_12.

In another embodiment, the nucleic microarray is an oligonucleotide microarray comprising at least one oligonucleotide specific for at least one gene of each of the bacterial species MO_HL_10, MO_HL_18, MO_HL_2, MO_HL_13, MO_HL_7, MO_HL_1, MO_HL_11, MO_HL_3, MO_HL_4, MO_HL_15, MO_HL_5, MO_HL_6, MO_HL_17, MO_HL_12 and MO_HL_8 Preferably, the nucleic microarray is an oligonucleotide microarray comprising or consisting in oligonucleotides specific for at least 2, 3, 4, 5, 10, 20, or 25 genes of each of the bacterial species MO_HL_10, MO_HL_18, MO_HL_2, MO_HL_13, MO_HL_7, MO_HL_1, MO_HL_11, MO_HL_3, MO_HL_4, MO_HL_15, MO_HL_5, MO_HL_6, MO_HL_17, MO_HL_12 and MO_HL_8.

In another embodiment, the nucleic microarray is an oligonucleotide microarray comprising at least one oligonucleotide specific for at least one gene of each of the bacterial species MO_HL_7, MO_HL_4, MO_HL_13, MO_HL_1, MO_HL_5, MO_HL_6, MO_HL_11, MO_HL_12, MO_HL_8, MO_HL_3, MO_HL_18, MO_HL_2, MO_HL_10, MO_HL_17, MO_HL_15 and MO_HL_9.Preferably, the nucleic microarray is an oligonucleotide microarray comprising or consisting in oligonucleotides specific for at least 2, 3, 4, 5, 10, 20, or 25 genes of each of the bacterial species MO_HL_7, MO_HL_4, MO_HL_13, MO_HL_1, MO_HL_5, MO_HL_6, MO_HL_11, MO_HL_12, MO_HL_8, MO_HL_3, MO_HL_18, MO_HL_2, MO_HL_10, MO_HL_17, MO_HL_15 and MO_HL_9.

In another embodiment, the nucleic microarray is an oligonucleotide microarray comprising at least one oligonucleotide specific for at least one gene of each of the bacterial species MO_HL_10, MO_HL_8, MO_HL_1, MO_HL_16, MO_HL_18, MO_HL_5, MO_HL_15, MO_HL_2, MO_HL_7, MO_HL_6, MO_HL_13, MO_HL_11, MO_HL_17, MO_HL_3, MO_HL_14, MO_HL_4 and MO_HL_12 .Preferably, the nucleic microarray is an oligonucleotide microarray comprising or consisting in oligonucleotides specific for at least 2, 3, 4, 5, 10, 20, or 25 genes of each of the bacterial species MO_HL_10, MO_HL_8, MO_HL_1, MO_HL_16, MO_HL_18, MO_HL_5, MO_HL_15, MO_HL_2, MO_HL_7, MO_HL_6, MO_HL_13, MO_HL_11, MO_HL_17, MO_HL_3, MO_HL_14, MO_HL_4 and MO_HL_12.

In another embodiment, the nucleic microarray is an oligonucleotide microarray comprising at least one oligonucleotide specific for at least one gene of each of the bacterial species MO_HL_15, MO_HL_17, MO_HL_4, MO_HL_14, MO_HL_16, MO_HL_10, MO_HL_7, MO_HL_6, MO_HL_11, MO_HL_9, MO_HL_5, MO_HL_3, MO_HL_8, MO_HL_1, MO_HL_12, MO_HL_18, MO_HL_13 and MO_HL_2.Preferably, the nucleic microarray is an oligonucleotide microarray comprising or consisting in oligonucleotides specific for at least 2, 3, 4, 5, 10, 20, or 25 genes of each of the bacterial species MO_HL_15, MO_HL_17, MO_HL_4, MO_HL_14, MO_HL_16, MO_HL_10, MO_HL_7, MO_HL_6, MO_HL_11, MO_HL_9, MO_HL_5, MO_HL_3, MO_HL_8, MO_HL_1, MO_HL_12, MO_HL_18, MO_HL_13 and MO_HL_2.

Said microarray may further comprise at least one oligonucleotide for detecting at least one gene of at least one control bacterial species. A convenient bacterial species may be e.g. a bacterial species whose abundance does not vary between individuals with a reduced bacterial diversity and individuals with normal bacterial diversity. Preferably, the oligonucleotides are about 50 bases in length.

SuiTable microarray oligonucleotides specific for any gene of SEQ ID NOs. 1-450 may be designed, based on the genomic sequence of each gene, using any method of microarray oligonucleotide design known in the art. In particular, any available software developed for the design of microarray oligonucleotides may be used, such as, for instance, the OligoArray software (available at http://berry.engin.umich.edu/oligoarray/), the GoArrays software (available at http://www.isima.fr/bioinfo/goarrays/), the Array Designer software (available at http://www.premierbiosoft.com/dnamicroarray/index.html), the Primer3 software (available at http://frodo.wi.mit.edu/primer3/primer3_code.html), or the Promide software (available at http://oligos.molgen.mpg.de/).

The disclosure further concerns a kit for the *in vitro* determination of the reduced gut bacterial diversity phenotype, comprising at least one reagent for the determination of the copy number of at least one gene having a sequence selected from SEQ ID NOs. 1-450. By "a reagent for the determination of the copy number of at least one gene", it is meant a reagent which specifically allows for the determination of the copy number of the said gene, i.e. a reagent specifically intended for the specific determination of the copy number of at least one gene having a sequence selected from SEQ ID NOs. 1-450. This definition excludes generic reagents useful for the determination of the expression level of any gene, such as Taq polymerase or an amplification buffer, although such reagents may also be included in a kit according to the disclosure Such a reagent for the determination of the copy number of at least one gene can be for example a dedicated microarray as described above or amplification primers specific for at least one gene having a sequence selected from SEQ ID NOs. 1-450. The present disclosure thus also relates to a kit for the *in vitro* determination of the reduced gut bacterial diversity phenotype, said kit comprising a dedicated microarray as described above or amplification primers specific for at least one gene having a sequence selected from SEQ ID NOs. 1-450. Here also, when the kit comprises amplification primers, while said kit may comprise amplification primers specific for other genes, said kit preferably comprises at most 100, at most 75, 50, at most 40, at most 30, preferably at most 25, at most 20, at most 15, more preferably at most 10, at most 8, at most 6, even more preferably at most 5, at most 4, at most 3 or even 2 or one or even zero couples of amplification primers specific for other genes than the genes of sequences SEQ ID NOs 1-450. For example, said kit may comprise at least a couple of amplification primers for at least one gene in addition to the primers for at least one gene having a sequence selected from SEQ ID NOs. 1-450.

Such a kit for the in vitro determination of the reduced gut bacterial diversity phenotype may further comprise instructions for detection of the presence or absence of a responsive phenotype.

As the inventors have shown, overweight subject with a reduced gut bacterial diversity present a higher risk of obesity-associated co-morbidities. Determining that an overweight subject has a reduced gut bacterial diversity is therefore extremely useful in the determination of such risk, particularly as it is realized entirely in vitro.

Another object of the invention is therefore a method for determining if an overweight subject is at risk to develop obesity related co-morbidities, said method comprising the steps of:
a) determining that said subject has a a reduced gut bacterial diversity with a method according to claims 1 to 9;
b) if said subject has a reduced gut bacterial diversity, determining that said overweight subject is at is at risk to develop obesity related co-morbidities.

Obesity related co-morbidities are well known of the skilled person. They have been thoroughly studied in scientific literature, and a comprehensive list of them may be found in Guh D. et al, BMC Public Health., 9:88, 2009. The most significant of them are type II diabetes, all cancers except esophageal , pancreatic and prostate cancer, all cardiovascular diseases (except congestive heart failure), asthma, gallbladder disease, osteoarthritis and chronic back pain.

Thus, in an embodiment, the method of the invention is for determining if an overweight subject is at risk to develop type II diabetes, all cancers except esophageal , pancreatic and prostate cancer, all cardiovascular diseases (except congestive heart failure), asthma, gallbladder disease, osteoarthritis and chronic back pain.

Moreover, the inventors have found that the gut bacterial microbiome of overweigth subject with a reduced gut bacterial diversity can be enriched by modifications of their diet, that is to say by implementing a specific diet. Indeed, they discovered that gut bacterial diversity, can be increased with a low calorie, protein-rich diet. The inventors showed that the gut bacterial diversity increase due to the implementation of said diet is more significant in overweight subjects with a reduced bacterial diversity. Those subjects are therefore particularly prone to benefit from dietary intervention.

Another object of the invention is therefore a method for determining if an overweight subject is in need of a diet, said method comprising the steps of :
a) determining that said subject has a reduced gut bacterial diversity with a method of claims 1 to 9,
b) if said subject has a reduced gut bacterial diversity, determining that said subject is in need of a diet.

In a preferred embodiment, the diet of the invention is a low calorie protein rich diet. In a more preferred embodiment, the diet of the invention comprises diets of between 1,200 and 1,500 Kcal per day, wherein the proteins represent 35% of the total amount of calories, the lipids represent 25% of the total amount of calories, the carbohydrates represent 44% of the total amount of calories. Advantageously, if the overweight subject is a woman, the diet comprises 1,200 Kcal per day and if the overweight subject is a man, the diet comprises 1,500 Kcal per day.

Importantly, this enrichment in gut bacterial species is correlated with an alleviation of the clinical traits linked to obesity-related co-morbidities, and thus the risk to develop obesity-related co-morbidities, except for low grade inflammation.

Another object of the invention is a method for alleviating the risks to develop obesity-related co-morbidities, said method comprising the steps of:
a) determining that said subject has a reduced gut bacterial diversity with a method of claims 1 to 9;
b) if said subject has a reduced gut bacterial diversity, determining that said subject is in need of a diet.

It will be immediately apparent to the person skilled in the art that overweight subjects with a reduced gut bacterial diversity that also present low grade inflammation may only partially benefit from implementing a diet. It is however particularly important to treat low-grade inflammation as soon as possible and therefore to be able to distinguish between overweight subjects for which low grade inflammation can efficiently be alleviated by dietary intervention only, and those whose low-grade inflammation requires additional treatments. The method of the invention allows for an early detection of those subjects that will require low-grade inflammation treatments in addition to the implementation of the diet.

Another object of the invention is therefore a method for determining if an overweight subject is in need of a low-grade inflammation treatment, preferably in addition to the implementation of a diet, comprising the steps of:
a) determining that said subject has a reduced gut bacterial diversity with a method of claims 1 to 9;
b) if said subject has a reduced gut bacterial diversity, determining that said subject is in need of a low-grade inflammation treatment, preferably in addition to the implementation of a diet.

The additional low-grade inflammation treatments can be any conventional anti-inflammation treatment such as the lipid lowering 3-hydroxy-3-methylglutaryl coenzyme A (HMG-Co A) reductase inhibitors (statins) and also the insulin sensitizing peroxisome proliferator activated receptor γ activators (thiazolidinediones).

The evolution of the bacterial diversity with implementation of those modifications of the diet can easily be monitored with the method of the invention.

Another object of the invention is therefore a method for monitoring the efficiency of a diet in increasing gut bacterial microbiome richness in an overweight subject in need thereof, said method comprising the steps of :
a) determining that said subject has a reduced gut bacterial diversity with a method of claims 1 to 9;
b) implementing said diet;
c) determining from a second gut microbial DNA sample obtained from said subject if said subject has a reduced gut bacterial diversity with a method of the invention;
d) if said subject has a reduced gut bacterial diversity, determining that the diet is efficient in increasing gut bacterial microbiome richness in said subject

The practice of the invention employs, unless other otherwise indicated, conventional techniques or protein chemistry, molecular virology, microbiology, recombinant DNA technology, and pharmacology, which are within the skill of the art. Such techniques are explained fully in the literature. (See Ausubel et al., Current Protocols in Molecular Biology, Eds., John Wiley & Sons, Inc. New York, 1995; Remington's Pharmaceutical Sciences, 17th ed., Mack Publishing Co., Easton, Pa., 1985; and Sambrook et al., Molecular cloning: A laboratory manual 2nd edition, Cold Spring Harbor Laboratory Press - Cold Spring Harbor, NY, USA, 1989). The nomenclatures used in connection with, and the laboratory procedures and techniques of, molecular and cellular biology, protein biochemistry, enzymology and medicinal and pharmaceutical chemistry described herein are those well-known and commonly used in the art.

Having generally described this invention, a further understanding of characteristics and advantages of the invention can be obtained by reference to certain specific examples and figures which are provided herein for purposes of illustration only and are not intended to be limiting unless otherwise specified.

### EXAMPLES

### Clinical investigation

Obese (n=38) and overweight (n=11) subjects, 8 men and 41 women, were recruited for a 12-week controlled dietary intervention at the Center of Research in Human Nutrition, Pitié-Salpêtrière Hospital, Paris, France. The subjects included in the study had no chronic pathology except body weight excess. Their body weight was stable within 3 months prior to the study. No antibiotics or drugs were taken within 2 months before or during the course of the study. The Ethical Committee of Pitié-Salpêtrière Hospital approved the clinical study and subjects gave written informed consent. In the first 6-week phase, subjects consumed an energy restricted high protein diet (1,200 Kcal/d for women and 1,500 for men: 35% proteins, 25% lipids, 44% carbohydrates) with low glycaemic index carbohydrates and enrichment with soluble fibers. This phase was followed by a second 6-week body weight stabilization period with 20% increase in total energy intake, above their resting energy metabolic rate (indirect calorimetry, Deltatrac, Datex, France). At 0, 6 and 12 weeks, blood and fecal samples were collected and anthropometric measurements were performed. Subjects filled a 7-day dietary record and were interviewed by a registered dietitian. On the visit day, the dietitian checked the information and clarified any ambiguities regarding detail of food consumed. All records were analyzed by the registered dietitian using the computer software program PROFILE DOSSIER V3 (Audit Conseil en Informatique Médicale, Bourges, France), which has a dietary database initially made up of 400 food items representative of the French diet as described previously². A nutrient analysis was generated for each subject. Body composition was determined by dual-energy X-ray absorptiometry (DEXA). Blood samples were obtained after 12 hr of fasting to measure total cholesterol, high-density lipoprotein (HDL) cholesterol, triglycerides, insulin, glucose, and inflammatory markers (highly sensitive C-reactive protein (hs-CRP) and interleukin 6 (IL-6)) as previously described3. Insulin resistance was estimated using homeostasis model assessment of insulin resistance (HOMA-IR) and "Disse" index^{4,5}. Subcutaneous abdominal adipose tissue samples were obtained at all time points by needle biopsy from the periumbilical area under local anesthesia (1% xylocaine) for measuring the adipocytes diameter⁶ and for immunohistochemical studies (HAM56+ stained macrophages in adipose tissue). Paired Wilcoxon tests were performed to analyze changes in these variables between various time points (p-value<0.05). P-values were adjusted for multiple testing using the Benjamini-Hochberg procedure⁷.

### Metagenomic sequencing

Intestinal bacterial gene content of 49 obese and overweight individuals at 3 time-points (baseline, week 6 and week 12) was determined by high throughput ABI SOLiD sequencing technology of total fecal DNA. An average of 76.5 ± 36.5 (mean ± sd) million 35 base-long single reads were determined for each sample (a total of 393 Gb of sequence) . By using corona lite (v4.0r2.0), an average of 24.8 ± 14.3 million reads per individuals were mapped on the reference catalog of 3.3 million genes⁸ with a maximum of 3 mismatches. Reads mapping at multiple positions were discarded and an average of 14.2 ± 8.1 million uniquely mapped reads per individuals were retained for estimating the abundance of each reference gene by using METEOR⁹. Abundance of each gene in an individual was normalized with METEOR by dividing the number of reads that uniquely mapped to the gene of interest by the gene length. After that, normalized gene abundances were transformed in frequencies by dividing them with the total number of uniquely mapped reads for a given sample. The resulting set of gene frequencies, termed as microbial gene profile of an individual, was used for further analyses.

### Differentially abundant species between LGC and HGC

Two groups of patients with low gene count (LGC) and high gene count (HGC) were defined using the gene richness distribution (Figure 1a). Genes significantly different in groups of individuals were identified by Mann-Whitney tests, at a p-value threshold of 0.0001. They were clustered by an abundance-based binning strategy, using the covariance of their gene frequency profiles among the individuals of the cohort. Spearman correlations coefficients were determined pairwise and all the genes that correlated above a rho coefficient of 0.85 were assigned to the same cluster. Abundance of a given species in each individual was estimated as a mean abundance of 25 arbitrarily selected 'tracer' genes for each cluster. The values were very close to the mean frequency of all the genes of a cluster.

### Receiver-Operator Characteristic (ROC) analysis

The analyses were carried out to distinguish between HGC and LGC individuals by a combination of bacterial species. For each combination, only a single decision model was considered, computed as a sum of abundance of species more frequent in HGC than in LGC individuals. As opposed to the infinite number of regression models, such models are finite and can be exhaustively explored. To select the best models, the cross-validated area under the ROC curve (CV-AUC) criterion¹¹ was used, which is well adapted to classification models for binary outcome data.

### Correlations between microbial and clinical variables

Mann-Whitney tests were used to compare bioclinical variables, food items and gene clusters between LGC and HGC gene class at each time point. Associations between quantitative basal gene richness and bioclinical/food variables or deltas of bioclinical/food variables were investigated using linear models. For deltas, models were adjusted by the initial values of the variables. A p-value threshold of 0.05 was applied for statistical significance. No adjustment for multiple testing was used due to the highly correlated bioclinical and food variables. However, similar findings obtained in the accompanying paper¹⁰ support the results.

### Taxonomical annotation

To obtain a precise and updated taxonomical annotation using BLASTN (BLAST 2.2.24, default parameters), the 3.3 million genes of the reference catalog were compared to the iMOMi database¹² (may 2011 release) containing 3,340 complete and draft microbial genomes downloaded from NCBI databanks. Following taxonomical assignation parameters described by Arumugam¹³, each gene was assigned with the taxonomy of the best-hit covering ≥80% of the gene length and according to the identity threshold for the taxonomic rank (≥65% for phylum, ≥85% for genus and ≥90% for species).

### Results

Forty-nine obese or overweight subjects were recruited and subjected to a 6-week energy restricted high-protein diet followed by a 6-week weight-maintenance diet the compliance was good, as indicated by a PCA analysis of 35 nutrients over time.Bioclinical characteristics and detailed qualitative and quantitative features of individuals' food intake were obtained at baseline, 6 and 12 weeks . The 35% decrease in energy intake after the first 6-weeks was associated with a reduction in body fat mass, adipocyte diameter and improvements of insulin sensitivity and markers of metabolism and inflammation. During the weight-maintenance phase, intake of nutrients tended to return to baseline values, while dietary total energy, carbohydrate and lipid intake remained lower than at beginning of the of the intervention Serum lipid variables tended to return to their basal levels as well, while a progressive reduction occurred in systemic inflammation markers.

The gut microbial composition of the study population was examined first at baseline. A bimodal distribution of bacterial gene number was observed (Figure 1), similar to the one found in a cohort of 292 Danish individuals¹⁰, albeit somewhat less distinct, possibly due to a lower cohort size. At a threshold of 480,000 genes, defined visually and corresponding to that from the accompanying manuscript¹⁰, there were 18 (40%) low gene count (LGC) and 27 (60%) high gene count (HGC) individuals, harboring on average 379,436 and 561,499 genes, respectively, a one third difference. The enterotypes of the individuals in our cohort¹³ were determined. It was found that the *Bacteroides-* and *Ruminococcus-driven* enterotypes were prevalent among the LGC and HGC individuals, respectively, whereas the *Prevotella-*driven enterotype was distributed more uniformly (Figure 1).

The baseline phenotypes of the study population were then examined. The LGC group had significantly higher insulin-resistance and fasting serum triglyceride levels, as well as a tendency towards higher inflammation than the HGC group (Figure 2). It appears that in two European countries, the individuals of the LGC group, who have low gut bacterial richness, present phenotypes which expose them to an increased risk of obesity-associated co-morbidities. Antibiotic treatments, which lower the diversity, have been reported to improve the hormonal, metabolic and inflammatory status of obese mice; this apparent contradiction may be due to a restoration of a balance of the pro- and inflammatory bacterial species in mice. Interestingly, LGC subjects appeared to consume less fruits and vegetables and less fishery products than HGC subjects (Table 7), raising the possibility that the long-term dietary habits may affect gene richness and the associated phenotypes.

We next searched for the bacterial species differentially abundant in the LGC and the HGC groups. To this aim, the genes that had significantly different frequencies in the LGC and HGC groups were first identified. The genes from the same species were then clustered by a frequency-based covariance analysis, the genes that vary in a coordinated way are likely to belong to the same species¹⁰. 6,230 genes were identified that were different at Mann-Whitney p<0.0001; 4,462 (72%) were grouped into 112 clusters containing at least 2 genes at a Spearman correlation coefficient >0.85. A vast majority of these genes (3,966; 89%) were found in only 18 clusters, which are likely to originate from species differentially abundant in the LGC and HGC groups. Only two (*Faecalibacterium prausnitzii* and *Roseburia inulinivorans)* had a clear taxonomic assignment.. The relative abundance of the 18 species in each individual was computed as a median frequency of their genes; all were significantly more abundant among the HGC individuals (Figure 1).

To test whether the LGC and HGC individuals could be distinguished by the 18 species, an exhaustive ROC analysis of all species combinations was carried out, with 10-fold cross validation, using 90% of individuals for computation and the remaining 10% for test. The best AUC values for combination of different numbers of species are shown in Figure 1; they ranged between 0.96 and 0.99 for 2 to 9 species combinations, indicating an almost perfect stratification of LGC and HGC individuals.

Interestingly, 14 of the 18 species (78 %) were also identified as differentially abundant among the LGC and HGC individuals in a larger Danish cohort¹¹. Not surprisingly, the species combinations yielding the best AUC values for our cohort also efficiently stratified LGC and HGC Danes (Figure 1). This indicates that the LGC and HGC individuals from two European countries differ in a similar way, not only by their clinical phenotypes but also by specific features of their gut microbiota.

Very interestingly, gene richness increased significantly in the LGC group after the energy-restricted diet and remained higher than at the baseline after the stabilization phase, even if a slight downwards trend was apparent, whereas it did not change significantly during intervention in the HGC group. Thus, a dietary intervention can correct a putative loss of richness in the LGC group, albeit partially, as the difference between the LGC and HGC groups remained significant at the end of the intervention. The intervention led to an improvement of metabolic variables in LGC individuals, bringing them closer to that of the HGC individuals: the tendency towards higher insulin resistance disappeared while the difference in triglyceride levels was attenuated (Figure 2). Importantly, although the inflammation was decreased in all individuals, the difference between LGC and HGC individuals was not attenuated (Figure 2).

To investigate the correlations between gene richness and dietary intervention further, those were studied in a quantitative way. For this purpose, we downsized the data set to 4.5 million uniquely mapping reads, enabling us to include at each time point 45 individuals. Prior to dietary intervention, gene richness was not related to age, sex, and BMI, but was associated with higher consumption of fruits and vegetables and lower triglyceride and cholesterol levels, confirming the LGC/HGC group comparisons. After 6 weeks of low-calorie diet, subjects with high gene richness at baseline had lower insulin-resistance and serum triglycerides as well as diminished adipose tissue inflammatory cells than the subjects with lower gene richness; the difference was significant for triglycerides and systemic inflammation after the weight maintenance phase, at 12 weeks. These observations indicate that gene richness affects the efficacy of the dietary intervention, even if the difference between the LGC and HGC individuals was globally attenuated. This conclusion was strengthened by the associations between higher gene richness at baseline and better improvement of adipose tissue and systemic inflammation (delta changes at 6 and 12 weeks, respectively). Gene richness appears to be predictive of the efficacy of dietary intervention in overweight/obese individuals.

**Table 2: AUC obtained when determining low gut diversity by detecting the presence or absence of set of 25 bacterial genes from the given bacterial species.**

| bacterial species | AUC |
|---|---|
| MO_HL_ 5 | 0.890946502057613 |
| MO_HL_ 6 | 0.890946502057613 |
| MO_HL_ 14 | 0.872427983539095 |
| MO_HL_ 9 | 0.845679012345679 |
| MO_HL_ 11 | 0.843621399176955 |
| MO_HL_ 13 | 0.831275720164609 |
| MO_HL_ 3 | 0.82716049382716 |
| MO_HL_ 8 | 0.826131687242798 |
| MO_HL_ 16 | 0.825102880658436 |
| MO_HL_ 4 | 0.820987654320988 |
| MO_HL_ 17 | 0.812757201646091 |
| MO_HL_ 1 | 0.808641975308642 |
| MO_HL_ 7 | 0.800411522633745 |
| MO_HL_ 12 | 0.79835390946502 |
| MO_HL_ 2 | 0.777777777777778 |
| MO_HL_ 10 | 0.765432098765432 |
| MO_HL_ 15 | 0.742798353909465 |
| MO_HL_18 | 0.740740740740741 |

**Table 3: AUC above 0.9 are obtained when determining low gut diversity by detecting the presence or absence of set of 25 bacterial genes from the given combination of 2 bacterial species.**

| Combination of bacterial species | AUC | Combination of bacterial species | AUC |
|---|---|---|---|
| MO_HL_5 and MO_HL_6 | 0.948559670781893 | MO_HL_3 and MO_ HL_13 | 0.874485596707819 |
| MO_HL_11 and MO_HL_12 | 0.948559670781893 | MO_HL_13 and MO_HL_14 | 0.874485596707819 |
| MO_HL_6 and MO_HL_7 | 0.94238683127572 | MO_HL_14 and MO_HL_15 | 0.874485596707819 |
| MO_HL_5 and MO_HL_11 | 0.938271604938272 | MO_HL_14 and MO_HL_17 | 0.874485596707819 |
| MO_HL_6 and MO_HL_8 | 0.932098765432099 | MO_HL_4 and MO_HL_8 | 0.872427983539095 |
| MO_HL_7 and MO_HL_11 | 0.932098765432099 | MO_HL_4 and MO_HL_14 | 0.872427983539095 |
| MO_HL_4 and MO_HL_13 | 0.92798353909465 | MO_HL_9 and MO_HL_15 | 0.872427983539094 |
| MO_HL_5 and MO_HL_12 | 0.921810699588477 | MO_HL_1 and MO_HL_3 | 0.87037037037037 |
| MO_HL_6 and MO_HL_13 | 0.921810699588477 | MO_HL_1 and MO_HL_5 | 0.87037037037037 |
| MO_HL_13 and MO_HL_17 | 0.919753086419753 | MO_HL_1 and MO_ HL_13 | 0.868312757201646 |
| MO_HL_5 and MO_HL_17 | 0.91358024691358 | MO_HL_3 and MO_ HL_16 | 0.868312757201646 |
| MO_HL_6 and MO_HL_12 | 0.91358024691358 | MO_HL_11 and MO_HL_15 | 0.868312757201646 |
| MO_HL_5 and MO_HL_7 | 0.911522633744856 | MO_HL_13 and MO_HL_15 | 0.864197530864198 |
| MO_HL_5 and MO_HL_8 | 0.909465020576132 | MO_HL_3 and MO_ HL_12 | 0.864197530864197 |
| MO_HL_8 and MO_HL_11 | 0.909465020576132 | MO_HL_5 and MO_HL_9 | 0.864197530864197 |
| MO_HL_3 and MO_HL_9 | 0.907407407407407 | MO_HL_6 and MO_HL_9 | 0.864197530864197 |
| MO_HL_5 and MO_HL_13 | 0.907407407407407 | MO_HL_7 and MO_HL_18 | 0.864197530864197 |
| MO_HL_14 and MO_HL_16 | 0.905349794238683 | MO_HL_2 and MO_HL_15 | 0.863168724279835 |
| MO_HL_12 and MO_HL_13 | 0.904320987654321 | MO_HL_1 and MO_HL_11 | 0.862139917695473 |
| MO_HL_2 and MO_HL_7 | 0.901234567901235 | MO_HL_3 and MO_HL_6 | 0.862139917695473 |
| MO_HL_2 and MO_HL_14 | 0.89917695473251 | MO_HL_8 and MO_HL_17 | 0.862139917695473 |
| MO_HL_4 and MO_HL_5 | 0.89917695473251 | MO_HL_13 and MO_HL_18 | 0.862139917695473 |
| MO_HL_11 and MO_HL_17 | 0.89917695473251 | MO_HL_7 and MO_HL_9 | 0.860082304526749 |
| MO_HL_4 and MO_HL_11 | 0.898148148148148 | MO_HL_8 and MO_HL_18 | 0.860082304526749 |
| MO_HL_7 and MO_HL_12 | 0.896090534979424 | MO_HL_9 and MO_HL_16 | 0.860082304526749 |
| MO_HL_1 and MO_HL_7 | 0.895061728395062 | MO_HL_1 and MO_ HL_17 | 0.858024691358025 |
| MO_HL_1 and MO_HL_8 | 0.895061728395062 | MO_HL_5 and MO_HL_15 | 0.858024691358025 |
| MO_HL_8 and MO_HL_12 | 0.895061728395062 | MO_HL_3 and MO_HL_10 | 0.858024691358024 |
| MO_HL_2 and MO_HL_5 | 0.893004115226337 | MO_HL_1 and MO_ HL_15 | 0.8559670781893 |
| MO_HL_2 and MO_HL _13 | 0.893004115226337 | MO_HL_9 and MO_HL_10 | 0.8559670781893 |
| MO_HL_3 and MO_ HL_14 | 0.893004115226337 | MO_HL_8 and MO_HL_13 | 0.854938271604938 |
| MO_HL_6 and MO_HL_11 | 0.890946502057613 | MO_HL_2 and MO_HL_16 | 0.853909465020576 |
| MO_HL_9 and MO_HL_14 | 0.890946502057613 | MO_HL_8 and MO_HL_9 | 0.853909465020576 |
| MO_HL_6 and MO_HL_17 | 0.888888888888889 | MO_HL_4 and MO_HL_18 | 0.851851851851852 |
| MO_HL_2 and MO_HL_4 | 0.887860082304527 | MO_HL_9 and MO_HL_12 | 0.851851851851852 |
| MO_HL_4 and MO_HL_6 | 0.886831275720165 | MO_HL_9 and MO_HL_17 | 0.851851851851852 |
| MO_HL_7 and MO_HL_13 | 0.885802469135803 | MO_HL_10 and MO_HL_13 | 0.849794238683127 |
| MO_HL_4 and MO_HL_7 | 0.885802469135802 | MO_HL_11 and MO_HL_18 | 0.849794238683127 |
| MO_HL_2 and MO_HL_6 | 0.88477366255144 | MO_HL_1 and MO_HL_2 | 0.847736625514403 |
| MO_HL_2 and MO_HL_8 | 0.88477366255144 | MO_HL_3 and MO_ HL_17 | 0.847736625514403 |
| MO_HL_2 and MO_HL_9 | 0.88477366255144 | MO_HL_1 and MO_HL_6 | 0.845679012345679 |
| MO_HL_3 and MO_HL_7 | 0.88477366255144 | MO_HL_3 and MO_ HL_18 | 0.845679012345679 |
| MO_HL_3 and MO_HL_8 | 0.88477366255144 | MO_HL_4 and MO_HL_9 | 0.845679012345679 |
| MO_HL_6 and MO_HL_15 | 0.88477366255144 | MO_HL_8 and MO_HL_15 | 0.845679012345679 |
| MO_HL_9 and MO_HL_13 | 0.88477366255144 | MO_HL_1 and MO_ HL_16 | 0.843621399176955 |
| MO_HL_1 and MO_HL_9 | 0.882716049382716 | MO_HL_3 and MO_HL_11 | 0.843621399176954 |
| MO_HL_2 and MO_HL_3 | 0.882716049382716 | MO_HL_12 and MO_HL_17 | 0.842592592592593 |
| MO_HL_2 and MO_HL_11 | 0.882716049382716 | MO_HL_2 and MO_HL_12 | 0.842592592592592 |
| MO_HL_10 and MO_HL_14 | 0.882716049382716 | MO_HL_2 and MO_HL_17 | 0.84156378600823 |
| MO_HL_11 and MO_HL_13 | 0.882716049382716 | MO_HL_7 and MO_HL_10 | 0.84156378600823 |
| MO_HL_3 and MO_HL_4 | 0.880658436213992 | MO_HL_1 and MO_HL_12 | 0.839506172839506 |
| MO_HL_5 and MO_HL_18 | 0.880658436213992 | MO_HL_3 and MO_HL_5 | 0.839506172839506 |
| MO_HL_7 and MO_HL_8 | 0.880658436213992 | MO_HL_3 and MO_ HL_15 | 0.837448559670782 |
| MO_HL_7 and MO_HL_17 | 0.880658436213992 | MO_HL_7 and MO_HL_15 | 0.83641975308642 |
| MO_HL_9 and MO_HL_11 | 0.878600823045268 | MO_HL_6 and MO_HL_18 | 0.835390946502058 |
| MO_HL_6 and MO_HL_14 | 0.878600823045267 | MO_HL_7 and MO_HL_16 | 0.835390946502058 |
| MO_HL_1 and MO_HL_14 | 0.876543209876543 | MO_HL_8 and MO_HL_16 | 0.835390946502058 |
| MO_HL_5 and MO_HL_14 | 0.876543209876543 | MO_HL_13 and MO_HL_16 | 0.835390946502058 |
| MO_HL_7 and MO_HL_14 | 0.876543209876543 | MO_HL_4 and MO_HL_16 | 0.835390946502057 |
| MO_HL_8 and MO_HL_14 | 0.876543209876543 | MO_HL_8 and MO_HL_10 | 0.835390946502057 |
| MO_HL_9 and MO_HL_18 | 0.876543209876543 | MO_HL_6 and MO_HL_10 | 0.833333333333333 |
| MO_HL_11 and MO_HL_14 | 0.876543209876543 | MO_HL_6 and MO_HL_16 | 0.833333333333333 |
| MO_HL_12 and MO_HL_14 | 0.876543209876543 | MO_HL_1 0 and MO_HL_16 | 0.833333333333333 |
| MO_HL_14 and MO_HL_18 | 0.876543209876543 | MO_HL_11 and MO_HL_16 | 0.833333333333333 |
| MO_HL_2 and MO_HL_10 | 0.874485596707819 | MO_HL_1 and MO_HL_4 | 0.831275720164609 |
| | | MO_HL_15 and MO_HL_17 | 0.831275720164609 |

**Table 4: AUC above 0.95 are obtained when determining low gut diversity by detecting the presence or absence of set of 25 bacterial genes from the given combination of 3 bacterial species.**

| Combination of bacterial species | AUC | |
|---|---|---|
| MO_HL_7, MO_HL_11 and MO_HL_12 | 0.993827160493827 | |
| MO_HL_5, MO_HL_11 and MO_HL_12 | 0.977366255144033 | |
| MO_HL_8, MO_HL_11 and MO_HL_12 | 0.967078189300411 | |
| MO_HL_4, MO_HL_7 and MO_HL_11 | 0.965020576131687 | |
| MO_HL_5, MO_HL_7 and MO_HL_11 | 0.965020576131687 | |
| MO_HL_7, MO_HL_11 and MO_HL_17 | 0.958847736625514 | |
| MO_HL_5, MO_HL_6 and MO_HL_8 | 0.954732510288066 | |
| MO_HL_7, MO_HL_12 and MO_HL_13 | 0.954732510288066 | |
| MO_HL_5, MO_HL_6 and MO_HL_12 | 0.952674897119342 | |
| MO_HL_6, MO_HL_7 and MO_HL_12 | 0.952674897119342 | |
| MO_HL_7, MO_HL_8 and MO_HL_11 | 0.952674897119342 | |
| MO_HL_ 4, MO_HL_5 and MO_HL_11 | 0.950617283950617 | |
| MO_HL_6, MO_HL_8 and MO_HL_11 | 0.950617283950617 | |

**Table 5: AUC above 0.96 are obtained when determining low gut diversity by detecting the presence or absence of set of 25 bacterial genes from the given combination of 4 bacterial species**

| Combination of bacterial species | AUC |
|---|---|
| MO_HL_5, MO_HL_7, MO_HL_11 and MO_HL_12 | 0.987654320987654 |
| MO_HL_7, MO_HL_8, MO_HL_11 and MO_HL_12 | 0.981481481481482 |
| MO_HL_7, MO_HL_11, MO_HL_12 and MO_HL_17 | 0.981481481481481 |
| MO_HL_6, MO_HL_8, MO_HL_11 and MO_HL_12 | 0.977366255144033 |
| MO_HL_2, MO_HL_7, MO_HL_11 and MO_HL_12 | 0.975308641975309 |
| MO_HL_7, MO_HL_11, MO_HL_12 and MO_HL_15 | 0.975308641975309 |
| MO_HL_2, MO_HL_8, MO_HL_11 and MO_HL_12 | 0.973251028806584 |
| MO_HL_5, MO_HL_11, MO_HL_12 and MO_HL_17 | 0.973251028806584 |
| MO_HL_6, MO_HL_7, MO_HL_11 and MO_HL_12 | 0.973251028806584 |
| MO_HL_5, MO_HL_8, MO_HL_11 and MO_HL_12 | 0.97119341563786 |
| MO_HL_8, MO_HL_11, MO_HL_12 and MO_HL_17 | 0.97119341563786 |
| MO_HL_7, MO_HL_11, MO_HL_12 and MO_HL_13 | 0.969135802469136 |
| MO_HL_5, MO_HL_6, MO_HL_8 and MO_HL_11 | 0.967078189300411 |
| MO_HL_7, MO_HL_8, MO_HL_11 and MO_HL_17 | 0.967078189300411 |
| MO_HL_2, MO_HL_5, MO_HL_11 and MO_HL_12 | 0.965020576131687 |
| MO_HL_4, MO_HL_7, MO_HL_11 and MO_HL_12 | 0.965020576131687 |
| MO_HL_5, MO_HL_7, MO_HL_8 and MO_HL_11 | 0.965020576131687 |
| MO_HL_7, MO_HL_8, MO_HL_12 and MO_HL_13 | 0.965020576131687 |
| MO_HL_7, MO_HL_12, MO_HL_13 and MO_HL_17 | 0.965020576131687 |
| MO_HL_2, MO_HL _4, MO_HL_7 and MO_HL_11 | 0.962962962962963 |
| MO_HL_2, MO_HL_7, MO_HL_8 and MO_HL_13 | 0.962962962962963 |
| MO_HL_2, MO_HL_7, MO_HL_12 and MO_HL_13 | 0.962962962962963 |
| MO_HL_ 4, MO_HL_5, MO_HL_7 and MO_HL_11 | 0.962962962962963 |
| MO_HL_5, MO_HL_6, MO_HL_12 and MO_HL_13 | 0.962962962962963 |
| MO_HL_5, MO_HL_11, MO_HL_12 and MO_HL_13 | 0.962962962962963 |
| MO_HL_6, MO_HL_8, MO_HL_12 and MO_HL_13 | 0.962962962962963 |
| MO_HL_2, MO_HL_7, MO_HL_8 and MO_HL_11 | 0.960905349794239 |
| MO_HL_5, MO_HL_6, MO_HL_7 and MO_HL_8 | 0.960905349794239 |
| MO_HL_5, MO_HL_6, MO_HL_11 and MO_HL_12 | 0.960905349794239 |
| MO_HL_6, MO_HL_7, MO_HL_8 and MO_HL_11 | 0.960905349794239 |
| MO_HL_6, MO_HL_7, MO_HL_8 and MO_HL_12 | 0.960905349794239 |
| MO_HL_6, MO_HL_7, MO_HL_12 and MO_HL_13 | 0.960905349794239 |
| MO_HL_7, MO_HL_8, MO_HL_11 and MO_HL_15 | 0.960905349794239 |
| MO_HL_11, MO_HL_12, MO_HL_13 and MO_HL_17 | 0.960905349794239 |

**Table 6: bacterial species and combinations of 2 to 18 bacterial species giving the best AUC in the method of the invention.**

| Bacterial species and combination thereof | AUC |
|---|---|
| MO_HL_5 | 0.890946502 |
| MO_HL_12 and MO_HL_11 | 0.948559671 |
| MO_HL_11, MO_HL_7 and MO_HL_12 | 0.99382716 |
| MO_HL_11, MO_HL_5, MO_HL_7 and MO_HL_12 | 0.987654321 |
| MO_HL_11, MO_HL_8, MO_HL_12, MO_HL_7 and MO_HL_2 | 0.983539095 |
| MO_HL_17, MO_HL_5, MO_HL_12, MO_HL_7, MO_HL_11 and MO_HL_8 | 0.981481481 |
| MO_HL_12, MO_HL_11, MO_HL_2, MO_HL_5, MO_HL_8, MO_HL_13 and MO_HL_7 | 0.983539095 |
| MO_HL_12, MO_HL_2, MO_HL_17, MO_HL_11, MO_HL_13, MO_HL_5, MO_HL_8 and MO_HL_7 | 0.983539095 |
| MO_HL _4, MO_HL_11, MO_HL_6, MO_HL_13, MO_HL_2, MO_HL_8, MO_HL_12, MO_HL_7 and MO_HL_17 | 0.979423868 |
| MO_HL_8, MO_HL_11, MO_HL_6, MO_HL_4, MO_HL_13, MO_HL_5, MO_HL_2, MO_HL_7, MO_HL_17 and MO_HL_12 | 0.981481481 |
| MO_HL_5, MO_HL_13, MO_HL_4, MO_HL_12, MO_HL_15, MO_HL_17, MO_HL_6, MO_HL_2, MO_HL_8, MO_HL_7 and MO_HL_11 | 0.977366255 |
| MO_HL_11, MO_HL_6, MO_HL_17, MO_HL_4, MO_HL_3, MO_HL_12, MO_HL_5, MO_HL_10, MO_HL_8, MO_HL_7, MO_HL_2 and MO_HL_13 | 0.975308642 |
| MO_HL_18, MO_HL_15, MO_HL_11, MO_HL_10, MO_HL_5, MO_HL_4, MO_HL_6, MO_HL_8, MO_HL_12, MO_HL_7, MO_HL_2, MO_HL_13 and MO_HL_3 | 0.973251029 |
| MO_HL_6, MO_HL_17, MO_HL_3, MO_HL_2, MO_HL_4, MO_HL_18, MO_HL_5, MO_HL_13, MO_HL_10, MO_HL_15, MO_HL_7, MO_HL_1, MO_HL_8 and MO_HL_12 | 0.971193416 |
| MO_HL_10, MO_HL_18, MO_HL_2, MO_HL_13, MO_HL_7, MO_HL_1, MO_HL_11, MO_HL_3, MO_HL_ 4, MO_HL_15, MO_HL_5, MO_HL_6, MO_HL_17, MO_HL_12 and MO_HL_8 | 0.971193416 |
| MO_HL_7, MO_HL _4, MO_HL_13, MO_HL_1, MO_HL_5, MO_HL_6, MO_HL_11, MO_HL_12, MO_HL_8, MO_HL_3, MO_HL_18, MO_HL_2, MO_HL_10, MO_HL_17, MO_HL_15 and MO_HL_9 | 0.946502058 |
| MO_HL_10, MO_HL_8, MO_HL_1, MO_HL_16, MO_HL_18, MO_HL_5, MO_HL_15, MO_HL_2, MO_HL_7, MO_HL_6, MO_HL_13, MO_HL_11, MO_HL_17, MO_HL_3, MO_HL_14, MO_HL_4 and MO_HL_12 | 0.942386831 |
| MO_HL_15, MO_HL_17, MO_HL_4, MO_HL_14, MO_HL_16, MO_HL_10, MO_HL_7, MO_HL_6, MO_HL_11, MO_HL_9, MO_HL_5, MO_HL_3, MO_HL_8, MO_HL_1, MO_HL_12, MO_HL_18, MO_HL_13 and MO_HL_2 | 0.940329218 |

### References

1. Rizkalla, S. W. et al. Differential effects of macronutrient content in 2 energy-restricted diets on cardiovascular risk factors and adipose tissue cell size in moderately obese individuals: a randomized controlled trial. Am. J. Clin. Nutr. 95, 49-63 (2012).
2. Bouché, C. et al. Five-week, low-glycemic index diet decreases total fat mass and improves plasma lipid profile in moderately overweight nondiabetic men. Diabetes Care 25, 822-828 (2002).
3. Tordjman, J. et al. Structural and inflammatory heterogeneity in subcutaneous adipose tissue: Relation with liver histopathology in morbid obesity. Journal of Hepatology (2012).doi:10.1016/j.jhep.2011.12.015
4. Disse, E. et al. A lipid-parameter-based index for estimating insulin sensitivity and identifying insulin resistance in a healthy population. Diabetes Metab. 34, 457-463 (2008).
5. Antuna-Puente, B. et al. Evaluation of insulin sensitivity with a new lipid-based index in non-diabetic postmenopausal overweight and obese women before and after a weight loss intervention. Eur. J. Endocrinol. 161, 51-56 (2009).
6. Prat-Larquemin, L. et al. Adipose angiotensinogen secretion, blood pressure, and AGT M235T polymorphism in obese patients. Obes. Res. 12, 556-561 (2004).
7. Benjamini, Y. & Hochberg, Y. Controlling the false discovery rate: a practical and powerful approach to multiple testing. Journal of the Royal Statistical Society. Series B. Methodological 57, 289-300
8. Qin, J. et al. A human gut microbial gene catalogue established by metagenomic sequencing. Nature 464, 59-65 (2010).
9. Pons, N. et al. METEOR, a platform for quantitative metagenomic profiling of complex ecosystems. JOBIM (2010).
10. Le Chatelier, E. et al. Richness of human gut microbial communities correlates with metabolic markers. submitted in Nature
11. Jiang, D., Huang, J. & Zhang, Y. The Cross-Validated AUC for MCP-Logistic Regression with High-Dimensional Data. Stat Methods Med Res (2011).doi:10.1177/0962280211428385
12. Pons, N., Batto, J.-M., Ehrlich, S. D. & Renault, P. Development of software facilities to characterize regulatory binding motifs and application to streptococcaceae. J. Mol. Microbiol. Biotechnol. 14, 67-73 (2008).
13. Arumugam, M. et al. Enterotypes of the human gut microbiome. Nature 473, 174-180 (2011).

## Claims

1. A method for determining whether an overweight subject has a reduced gut bacterial diversity, said method comprising:
a) detecting from a gut microbial DNA sample obtained from said subject whether at least one of the sets of 25 bacterial genes from at least one bacterial species as disclosed in Table 1 is absent in said sample, and
b) determining that the subject has a reduced gut bacterial diversity, if at least one of the sets of 25 bacterial genes from at least one bacterial species from Table 1 is absent in said sample.

2. The method according to claim 1, **characterized in that** it comprises a step of detecting from a gut microbial DNA sample obtained from said subject whether at least one of the sets of 25 bacterial genes from a bacterial species chosen from the list consisting in MO_HL_5, MO_HL_6, MO_HL_14, MO_HL_9, MO_HL_11, MO_HL_13, MO_HL_3, MO_HL_8, MO_HL_16, MO_HL_4, MO_HL_17, MO_HL_1, or MO_HL_7 as disclosed in Table 1 is absent in said sample.

3. The method according to claim 1 or 2, **characterized in that** it comprises a step of detecting from a gut microbial DNA sample obtained from said subject whether at least the set of 25 bacterial genes from the bacterial species MOHL-5 as disclosed in Table 1 is absent in said sample.

4. The method according to any of claims 1 to 3, **characterized in that** it comprises a step of detecting from a gut microbial DNA sample obtained from said subject whether at least the set of 25 bacterial genes from each of the bacterial species of any of the bacterial species combinations indicated in Table 3, 4 and/or 5 is absent in said sample.

5. The method according to any of claims 1 to 3, **characterized in that** it comprises a step of detecting from a gut microbial DNA sample obtained from said subject whether:
- at least the set of 25 bacterial genes from each of the bacterial species MO_HL_12 and MO_HL_11 as disclosed in Table 1 is absent in said sample, or;
- at least the set of 25 bacterial genes from each of the bacterial species MO_HL_11, MO_HL_7 and MO_HL_12 as disclosed in Table 1 is absent in said sample, or;
- at least the set of 25 bacterial genes from each of the bacterial species MO_HL_11, MO_HL_5, MO_HL_7 and MO_HL_12 as disclosed in Table 1 is absent in said sample, or;
- at least the set of 25 bacterial genes from each of the bacterial species MO_HL_11, MO_HL_8, MO_HL_12, MO_HL_7 and MO_HL_2 as disclosed in Table 1 is absent in said sample, or;
- at least the set of 25 bacterial genes from each of the bacterial species MO_HL_17, MO_HL_5, MO_HL_12, MO_HL_7, MO_HL_11 and MO_HL_8 as disclosed in Table 1 is absent in said sample, or;
- at least the set of 25 bacterial genes from each of the bacterial species MO_HL_12, MO_HL_11, MO_HL_2, MO_HL_5, MO_HL_8, MO_HL_13 and MO_HL_7 as disclosed in Table 1 is absent in said sample, or;
- at least the set of 25 bacterial genes from each of the bacterial species MO_HL_12, MO_HL_2, MO_HL_17, MO_HL_11, MO_HL_13, MO_HL_5, MO_HL_8 and MO_HL_7 as disclosed in Table 1 is absent in said sample, or;
- at least the set of 25 bacterial genes from each of the bacterial species MO_HL_4, MO_HL_11, MO_HL_6, MO_HL_13, MO_HL_2, MO_HL_8, MO_HL_12, MO_HL_7 and MO_HL_17 as disclosed in Table 1 is absent in said sample, or;
- at least the set of 25 bacterial genes from each of the bacterial species MO_HL_8, MO_HL_11, MO_HL_6, MO_HL_4, MO_HL_13, MO_HL_5, MO_HL_2, MO_HL_7, MO_HL_17 and MO_HL_12 as disclosed in Table 1 is absent in said sample;
- at least the set of 25 bacterial genes from each of the bacterial species MO_HL_5, MO_HL_13, MO_HL_4, MO_HL_12, MO_HL_15, MO_HL_17, MO_HL_6, MO_HL_2, MO_HL_8, MO_HL_7 and MO_HL_11 as disclosed in Table 1 is absent in said sample;
- at least the set of 25 bacterial genes from each of the bacterial species MO_HL_11, MO_HL_6, MO_HL_17, MO_HL_4, MO_HL_3, MO_HL_12, MO_HL_5, MO_HL_10, MO_HL_8, MO_HL_7, MO_HL_2 and MO_HL_13 as disclosed in Table 1 is absent, in said sample;
- at least the set of 25 bacterial genes from each of the bacterial species MO_HL_18, MO_HL_15, MO_HL_11, MO_HL_10, MO_HL_5, MO_HL_4, MO_HL_6, MO_HL_8, MO_HL_12, MO_HL_7, MO_HL_2, MO_HL_13 and MO_HL_3 as disclosed in Table 1 is absent in said sample, or;
- at least the set of 25 bacterial genes from each of the bacterial species MO_HL_6, MO_HL_17, MO_HL_3, MO_HL_2, MO_HL_4, MO_HL_18, MO_HL_5, MO_HL_13, MO_HL_10, MO_HL_15, MO_HL_7, MO_HL_1, MO_HL_8 and MO_HL_12 as disclosed in Table 1 is absent in said sample, or;
- at least the set of 25 bacterial genes from each of the bacterial species MO_HL_10, MO_HL_18, MO_HL_2, MO_HL_13, MO_HL_7, MO_HL_1, MO_HL_11, MO_HL_3, MO_HL_4, MO_HL_15, MO_HL_5, MO_HL_6, MO_HL_17, MO_HL_12 and MO_HL_8 as disclosed in Table 1 is absent, in said sample;
- at least the set of 25 bacterial genes from each of the bacterial species MO_HL_7, MO_HL_4, MO_HL_13, MO_HL_1, MO_HL_5, MO_HL_6, MO_HL_11, MO_HL_12, MO_HL_8, MO_HL_3, MO_HL_18, MO_HL_2, MO_HL_10, MO_HL_17, MO_HL_15 and MO_HL_9 as disclosed in Table 1 is absent in said sample, or;
- at least the set of 25 bacterial genes from each of the bacterial species MO_HL_10, MO_HL_8, MO_HL_1, MO_HL_16, MO_HL_18, MO_HL_5, MO_HL_15, MO_HL_2, MO_HL_7, MO_HL_6, MO_HL_13, MO_HL_11, MO_HL_17, MO_HL_3, MO_HL_14, MO_HL_4 and MO_HL_12 as disclosed in Table 1 is absent, in said sample;
- at least the set of 25 bacterial genes from each of the bacterial species MO_HL_15, MO_HL_17, MO_HL_4, MO_HL_14, MO_HL_16, MO_HL_10, MO_HL_7, MO_HL_6, MO_HL_11, MO_HL_9, MO_HL_5, MO_HL_3, MO_HL_8, MO_HL_1, MO_HL_12, MO_HL_18, MO_HL_13 and MO_HL_2 as disclosed in Table 1 is absent, in said sample.

6. The method according to any of claims 1 to 5, **characterized in that** it comprises detecting the number of copies of at least one of said sets of 25 bacterial genes from said bacterial species in the sample.

7. The method according to claim 6, **characterized in that** said bacterial genes are chosen in the list consisting of sequence SEQID 1 to sequence SEQ ID 450.

8. The method according to any of claims 1 to 7, **characterized in that** the presence or absence of the bacterial genes according to the invention is detected by the use of a nucleic microarray which is preferably an oligonucleotide microarray or a cDNA microarray.

9. The method according to claim 8, **characterized in that** the nucleic microarray is an oligonucleotide microarray comprising at least one oligonucleotide specific for at least each of said 25 bacterial genes having a sequence selected from SEQ ID NOs 1-450.

10. The method according to any of claims 1 to 9, **characterized in that** said gut microbial DNA sample is obtained from a feces sample.

11. A method for determining if an overweight subject is at risk to develop obesity related co-morbidities selected from type II diabetes; cancers except esophageal, pancreatic and prostate cancer; cardiovascular diseases except congestive heart failure; asthma; gallbladder disease; osteoarthritis; and chronic back pain, said method comprising the steps of:
a) determining that said subject has a reduced gut bacterial diversity with a method according to anyone of claims 1 to 10;
b) if said subject has a reduced gut bacterial diversity, determining that said overweight subject is at is at risk to develop obesity related co-morbidities.

12. A method for determining if an overweight subject is in need of a diet, said method comprising the steps of:
a) determining that said subject has a reduced gut bacterial diversity with a method according to anyone of claims 1 to 10;
b) if said subject has a reduced gut bacterial diversity, determining that said subject is in need of a diet.

13. A method for alleviating the risks to develop obesity-related co-morbidities selected from type II diabetes; cancers except esophageal, pancreatic and prostate cancer; cardiovascular diseases except congestive heart failure; asthma; gallbladder disease; osteoarthritis; and chronic back pain, said method comprising the steps of:
a) determining that said subject has a reduced gut bacterial diversity with a method according to anyone of claims 1 to 10;
b) if said subject has a reduced gut bacterial diversity, determining that said subject is in need of a diet.

14. A method for determining if an overweight subject is in need of a low-grade inflammation treatment, preferably in addition to the implementation of a diet, comprising the steps of:
a) determining that said subject has a reduced gut bacterial diversity with a method according to anyone of claims 1 to 10;
b) if said subject has a reduced gut bacterial diversity, determining that said subject is in need of a low-grade inflammation treatment, preferably in addition to the implementation of a diet.

15. A method for monitoring the efficiency of a diet in increasing gut bacterial microbiome richness in an overweight subject in need thereof, said method comprising the steps of:
a) determining that said subject has a reduced gut bacterial diversity with a method according to anyone of claims 1 to 10;
b) implementing said diet;
c) determining from a second gut microbial DNA sample obtained from said subject if said subject has a reduced gut bacterial diversity with a method according to anyone of claims 1 to 10;
d) if said subject has a reduced gut bacterial diversity, determining that the diet is efficient in increasing gut bacterial microbiome richness in said subject.

## Patentansprüche

1. Verfahren zur Ermittlung, ob ein übergewichtiger Proband eine reduzierte Darmbakterienvielfalt hat, wobei das Verfahren Folgendes umfasst:
a) Nachweis aus einer mikrobiellen Darm-DNA-Probe, die von dem Probanden erhalten wurde, ob zumindest einer der Sätze von 25 bakteriellen Genen von zumindest einer Bakterienart wie in Tabelle 1 offenbart in der Probe fehlt, und
b) Ermittlung, dass der Proband eine reduzierte Darmbakterienvielfalt aufweist, wenn zumindest einer der Sätze von 25 bakteriellen Genen von zumindest einer Bakterienart der Tabelle 1 in der Probe fehlt.

2. Verfahren nach Anspruch 1, **dadurch gekennzeichnet, dass** es einen Schritt mit Nachweis aus einer mikrobiellen Darm-DNA-Probe, die von dem Probanden erhalten wurde, umfasst, ob zumindest einer der Sätze von 25 bakteriellen Genen von einer Bakterienart, die aus der Liste mit MO_HL_5, MO_HL_6, MO_HL_14, MO_HL_9, MO_HL_11, MO_HL_13, MO_HL_3, MO_HL_8, MO_HL_16, MO_HL_4, MO_HL_17, MO_HL_1 oder MO_HL_7 wie in Tabelle 1 offenbart gewählt wurde, in der Probe fehlt.

3. Verfahren nach Anspruch 1 oder 2, **dadurch gekennzeichnet, dass** es einen Schritt mit Nachweis aus einer mikrobiellen Darm-DNA-Probe, die von dem Probanden erhalten wurde, umfasst, ob zumindest der Satz von 25 bakteriellen Genen der Bakterienart MOHL-5 wie in Tabelle 1 offenbart in der Probe fehlt.

4. Verfahren nach einem der Ansprüche 1 bis 3, **dadurch gekennzeichnet, dass** es einen Schritt mit Nachweis aus einer mikrobiellen Darm-DNA-Probe, die von dem Probanden erhalten wurde, umfasst, ob zumindest der Satz von 25 bakteriellen Genen jeder der Bakterienarten jeglicher der Kombinationen von Bakterienarten wie in Tabelle 3, 4 und/oder 5 angegeben in der Probe fehlt.

5. Verfahren nach einem der Ansprüche 1 bis 3, **dadurch gekennzeichnet, dass** es einen Schritt mit Nachweis aus einer mikrobiellen Darm-DNA-Probe umfasst, die von dem Probanden erhalten wurde, ob:
- zumindest der Satz von 25 bakteriellen Genen von jeder der Bakterienarten MO_HL_12 und MO_HL_11 wie in Tabelle 1 offenbart in der Probe fehlt, oder;
- zumindest der Satz von 25 bakteriellen Genen von jeder der Bakterienarten MO_HL_11, MO_HL_7 und MO_HL_12 wie in Tabelle 1 offenbart in der Probe fehlt, oder;
- zumindest der Satz von 25 bakteriellen Genen von jeder der Bakterienarten MO_HL_11, MO_HL_5, MO_HL_7 und MO_HL_12 wie in Tabelle 1 offenbart in der Probe fehlt, oder;
- zumindest der Satz von 25 bakteriellen Genen von jeder der Bakterienarten MO_HL_11, MO_HL_8, MO_HL_12, MO_HL_7 und MO_HL_2 wie in Tabelle 1 offenbart in der Probe fehlt, oder;
- zumindest der Satz von 25 bakteriellen Genen von jeder der Bakterienarten MO_HL_17, MO_HL_5, MO_HL_12, MO_HL_7, MO_HL_11 und MO_HL_8 wie in Tabelle 1 offenbart in der Probe fehlt, oder;
- zumindest der Satz von 25 bakteriellen Genen von jeder der Bakterienarten MO_HL_12, MO_HL_11, MO_HL_2, MO_HL_5, MO_HL_8, MO_HL_13 und MO_HL_7 wie in Tabelle 1 offenbart in der Probe fehlt, oder;
- zumindest der Satz von 25 bakteriellen Genen von jeder der Bakterienarten MO_HL_12, MO_HL_2, MO_HL_17, MO_HL_11, MO_HL_13, MO_HL_5, MO_HL_8 und MO_HL_7 wie in Tabelle 1 offenbart in der Probe fehlt, oder;
- zumindest der Satz von 25 bakteriellen Genen von jeder der Bakterienarten MO_HL_4, MO_HL_11, MO_HL_6, MO_HL_13, MO_HL_2, MO_HL_8, MO_HL_12, MO_HL_7 und MO_HL_17 wie in Tabelle 1 offenbart in der Probe fehlt, oder;
- zumindest der Satz von 25 bakteriellen Genen von jeder der Bakterienarten MO_HL_8, MO_HL_11, MO_HL_6, MO_HL_4, MO_HL_13, MO_HL_5, MO_HL_2, MO_HL_7, MO_HL_17 und MO_HL_12 wie in Tabelle 1 offenbart in der Probe fehlt;
- zumindest der Satz von 25 bakteriellen Genen von jeder der Bakterienarten MO_HL_5, MO_HL_13, MO_HL_4, MO_HL_12, MO_HL_15, MO_HL_17, MO_HL_6, MO_HL_2, MO_HL_8, MO_HL_7 und MO_HL_11 wie in Tabelle 1 offenbart in der Probe fehlt;
- zumindest der Satz von 25 bakteriellen Genen von jeder der Bakterienarten MO_HL_11, MO_HL_6, MO_HL_17, MO_HL_4, MO_HL_3, MO_HL_12, MO_HL_5, MO_HL_10, MO_HL_8, MO_HL_7, MO_HL_2 und MO_HL_13 wie in Tabelle 1 offenbart in der Probe fehlt;
- zumindest der Satz von 25 bakteriellen Genen von jeder der Bakterienarten MO_HL_18, MO_HL_15, MO_HL_11, MO_HL_10, MO_HL_5, MO_HL_4, MO_HL_6, MO_HL_8, MO_HL_12, MO_HL_7, MO_HL_2, MO_HL_13 und MO_HL_3 wie in Tabelle 1 offenbart in der Probe fehlt, oder;
- zumindest der Satz von 25 bakteriellen Genen von jeder der Bakterienarten MO_HL_6, MO_HL_17, MO_HL_3, MO_HL_2, MO_HL_4, MO_HL_18, MO_HL_5, MO_HL_13, MO_HL_10, MO_HL_15, MO_HL_7, MO_HL_1, MO_HL_8 und MO_HL_12 wie in Tabelle 1 offenbart in der Probe fehlt, oder;
- zumindest der Satz von 25 bakteriellen Genen von jeder der Bakterienarten MO_HL_10, MO_HL_18, MO_HL_2, MO_HL_13, MO_HL_7, MO_HL_1, MO_HL_11, MO_HL_3, MO_HL_4, MO_HL_15, MO_HL_5, MO_HL_6, MO_HL_17, MO_HL_12 und MO_HL_8 wie in Tabelle 1 offenbart in der Probe fehlt;
- zumindest der Satz von 25 bakteriellen Genen von jeder der Bakterienarten MO_HL_7, MO_HL_4, MO_HL_13, MO_HL_1, MO_HL_5, MO_HL_6, MO_HL_11, MO_HL_12, MO_HL_8, MO_HL_3, MO_HL_18, MO_HL_2, MO_HL_10, MO_HL_17, MO_HL_15 und MO_HL_9 wie in Tabelle 1 offenbart in der Probe fehlt, oder;
- zumindest der Satz von 25 bakteriellen Genen von jeder der Bakterienarten MO_HL_10, MO_HL_8, MO_HL_1, MO_HL_16, MO_HL_18, MO_HL_5, MO_HL_15, MO_HL_2, MO_HL_7, MO_HL_6, MO_HL_13, MO_HL_11, MO_HL_17, MO_HL_3, MO_HL_14, MO_HL_4 und MO_HL_12 wie in Tabelle 1 offenbart in der Probe fehlt;
- zumindest der Satz von 25 bakteriellen Genen von jeder der Bakterienarten MO_HL_15, MO_HL_17, MO_HL_4, MO_HL_14, MO_HL_16, MO_HL_10, MO_HL_7, MO_HL_6, MO_HL_11, MO_HL_9, MO_HL_5, MO_HL_3, MO_HL_8, MO_HL_1, MO_HL_12, MO_HL_18, MO_HL_13 und MO_HL_2 wie in Tabelle 1 offenbart in der Probe fehlt.

6. Verfahren nach einem der Ansprüche 1 bis 5, **dadurch gekennzeichnet, dass** es den Nachweis der Anzahl Kopien von zumindest einem der Sätze von 25 bakteriellen Genen der Bakterienart in der Probe umfasst.

7. Verfahren nach Anspruch 6, **dadurch gekennzeichnet, dass** die bakteriellen Gene aus der Liste mit der Sequenz SEQ ID 1 bis Sequenz SEQ ID 450 gewählt werden.

8. Verfahren nach einem der Ansprüche 1 bis 7, **dadurch gekennzeichnet, dass** das Vorhandensein oder Fehlen der bakteriellen Gene gemäß der Erfindung mit Verwendung eines Nuklein-Microarray nachgewiesen wird, das bevorzugt ein Oligonukleotid-Microarray oder ein cDNA Microarray ist.

9. Verfahren nach Anspruch 8, **dadurch gekennzeichnet, dass** das Nuklein-Microarray ein Oligonukleotid-Microarray ist, das zumindest ein für zumindest jeder der 25 bakteriellen Gene spezifisches Oligonukleotid umfasst, die eine aus den SEQ ID NOs 1-450 gewählte Sequenz haben.

10. Verfahren nach einem der Ansprüche 1 bis 9, **dadurch gekennzeichnet, dass** die mikrobielle Darm-DNA-Probe aus einer Stuhlprobe gewonnen wurde.

11. Verfahren zur Ermittlung, ob ein übergewichtiger Proband Gefahr läuft, fettleibigkeitsbedingte Komorbiditäten zu entwickeln, die aus Typ II Diabetes ausgewählt wurden; Krebserkrankungen außer Speiseröhren-, Bauchspeicheldrüsen- und Prostatakrebs; Herz-Kreislauf-Erkrankungen außer kongestiver Herzinsuffizienz; Asthma, Gallenblasenerkrankung; Osteoarthritis; und chronischen Rückenschmerzen, wobei das Verfahren die folgenden Schritte umfasst:
a) Ermittlung, ob ein Proband eine reduzierte Darmbakterienvielfalt aufweist, und zwar mit einem Verfahren nach einem der Ansprüche 1 bis 10;
b) wenn der Proband eine reduzierte Darmbakterienvielfalt aufweist, Ermittlung, ob der übergewichtige Proband Gefahr läuft, fettleibigkeitsbedingte Komorbiditäten zu entwickeln.

12. Verfahren zur Ermittlung, ob ein übergewichtiger Proband eine Diät benötigt, wobei das Verfahren die folgenden Schritte umfasst:
a) Ermittlung, ob der Proband eine reduzierte Darmbakterienvielfalt aufweist, und zwar mit einem Verfahren nach einem der Ansprüche 1 bis 10;
b) wenn der Proband eine reduzierte Darmbakterienvielfalt aufweist, Ermittlung, ob der Proband eine Diät benötigt.

13. Verfahren zur Verringerung der Gefahr, fettleibigkeitsbedingte Komorbiditäten zu entwickeln, die aus Typ II Diabetes ausgewählt wurden; Krebserkrankungen außer Speiseröhren-, Bauchspeicheldrüsen- und Prostatakrebs; Herz-Kreislauf-Erkrankungen außer kongestiver Herzinsuffizienz; Asthma, Gallenblasenerkrankung; Osteoarthritis; und chronischen Rückenschmerzen, wobei das Verfahren die folgenden Schritte umfasst:
a) Ermittlung, ob der Proband eine reduzierte Darmbakterienvielfalt aufweist, und zwar mit einem Verfahren nach einem der Ansprüche 1 bis 10;
b) wenn der Proband eine reduzierte Darmbakterienvielfalt aufweist, Ermittlung, ob der Proband eine Diät benötigt.

14. Verfahren zur Ermittlung, ob ein übergewichtiger Proband eine Low-Grade-Entzündungsbehandlung, vorzugsweise zusätzlich zur Durchführung einer Diät benötigt, das die folgenden Schritte umfasst:
a) Ermittlung, ob der Proband eine reduzierte Darmbakterienvielfalt aufweist, und zwar mit einem Verfahren nach einem der Ansprüche 1 bis 10;
b) wenn der Proband eine reduzierte Darmbakterienvielfalt aufweist, Ermittlung, ob der Proband eine Low-Grade-Entzündungsbehandlung, vorzugsweise zusätzlich zur Durchführung einer Diät benötigt.

15. Verfahren zur Überwachung der Wirksamkeit einer Diät bei der Erhöhung des Mikrobiom-Reichtums an Darmbakterien in einem übergewichtigen Probanden, der diese benötigt, wobei das Verfahren die folgenden Schritte umfasst:
a) Ermittlung, ob der Proband eine reduzierte Darmbakterienvielfalt aufweist, und zwar mit einem Verfahren nach einem der Ansprüche 1 bis 10;
b) Umsetzung dieser Diät;
c) Ermittlung aus einer zweiten mikrobiellen Darm-DNA-Probe, die von dem Probanden erhalten wurde, ob der Proband eine reduzierte Darmbakterienvielfalt aufweist, und zwar mit einem Verfahren nach einem der Ansprüche 1 bis 10;
d) wenn der Proband eine reduzierte Darmbakterienvielfalt aufweist, Ermittlung, dass die Diät wirksam ist, da sie den Mikrobiom-Reichtum an Darmbakterien im Probanden erhöht.

## Revendications

1. Méthode permettant de déterminer si un sujet en surpoids a une diversité bactérienne intestinale réduite, ladite méthode comprenant :
a) la détection dans un échantillon d'ADN microbien intestinal provenant dudit sujet si au moins un des ensembles de 25 gènes bactériens d'au moins une espèce bactérienne décrite dans le Tableau 1 est absent dans ledit échantillon, et
b) la détermination selon laquelle le sujet a une diversité bactérienne intestinale réduite, si au moins un des ensembles de 25 gènes bactériens d'au moins une espèce bactérienne décrite dans le Tableau 1 est absent dans ledit échantillon.

2. Méthode selon la revendication 1, **caractérisée en ce qu'**elle comprend une étape de détection dans un échantillon d'ADN microbien intestinal provenant dudit sujet si au moins un des ensembles de 25 gènes bactériens d'une espèce bactérienne choisie dans la liste constituée par MO_HL_5, MO_HL_6, MO_HL_14, MO_HL_9, MO_HL_11, MO_HL_13, MO_HL_3, MO_HL_8, MO_HL_16, MO_HL_4, MO_HL_17, MO_HL_1, ou MO_HL_7 décrite dans le Tableau 1 est absent dans ledit échantillon.

3. Méthode selon la revendication 1 ou 2, **caractérisée en ce qu'**elle comprend une étape de détection dans un échantillon d'ADN microbien intestinal provenant dudit sujet si au moins l'ensemble de 25 gènes bactériens de l'espèce bactérienne MO_HL_5 décrite dans le Tableau 1 est absent dans ledit échantillon.

4. Méthode selon l'une quelconque des revendications 1 à 3, **caractérisée en ce qu'**elle comprend une étape de détection dans un échantillon d'ADN microbien intestinal provenant dudit sujet si au moins l'ensemble de 25 gènes bactériens de chacune des espèces bactériennes dans l'une quelconque des combinaisons d'espèces bactériennes indiquées dans le Tableau 3, 4 et/ou 5 est absent dans ledit échantillon.

5. Méthode selon l'une quelconque des revendications 1 à 3, **caractérisée en ce qu'**elle comprend une étape de détection dans un échantillon d'ADN microbien intestinal provenant dudit sujet si :
- au moins l'ensemble de 25 gènes bactériens de chacune des espèces bactériennes MO_HL_12 et MO_HL_11 décrites dans le Tableau 1 est absent dans ledit échantillon, ou ;
- au moins l'ensemble de 25 gènes bactériens de chacune des espèces bactériennes MO_HL_11, MO_HL_7 et MO_HL_12 décrites dans le Tableau 1 est absent dans ledit échantillon, ou ;
- au moins l'ensemble de 25 gènes bactériens de chacune des espèces bactériennes MO_HL_11, MO_HL_5, MO_HL_7 et MO_HL_12 décrites dans le Tableau 1 est absent dans ledit échantillon, ou ;
- au moins l'ensemble de 25 gènes bactériens de chacune des espèces bactériennes MO_HL_11, MO_HL_8, MO_HL_12, MO_HL_7 et MO_HL_2 décrites dans le Tableau 1 est absent dans ledit échantillon, ou ;
- au moins l'ensemble de 25 gènes bactériens de chacune des espèces bactériennes MO_HL_17, MO_HL_5, MO_HL_12, MO_HL_7, MO_HL_11 et MO_HL_8 décrites dans le Tableau 1 est absent dans ledit échantillon, ou ;
- au moins l'ensemble de 25 gènes bactériens de chacune des espèces bactériennes MO_HL_12, MO_HL_11, MO_HL_2, MO_HL_5, MO_HL_8, MO_HL_13 et MO_HL_7 décrites dans le Tableau 1 est absent dans ledit échantillon, ou ;
- au moins l'ensemble de 25 gènes bactériens de chacune des espèces bactériennes MO_HL_12, MO_HL_2, MO_HL_17, MO_HL_11, MO_HL_13, MO_HL_5, MO_HL_8 et MO_HL_7 décrites dans le Tableau 1 est absent dans ledit échantillon, ou ;
- au moins l'ensemble de 25 gènes bactériens de chacune des espèces bactériennes MO_HL_4, MO_HL_11, MO_HL_6, MO_HL_13, MO_HL_2, MO_HL_8, MO_HL_12, MO_HL_7 et MO_HL_17 décrites dans le Tableau 1 est absent dans ledit échantillon, ou ;
- au moins l'ensemble de 25 gènes bactériens de chacune des espèces bactériennes MO_HL_8, MO_HL_11, MO_HL_6, MO_HL_4, MO_HL_13, MO_HL_5, MO_HL_2, MO_HL_7, MO_HL_17 et MO_HL_12 décrites dans le Tableau 1 est absent dans ledit échantillon;
- au moins l'ensemble de 25 gènes bactériens de chacune des espèces bactériennes MO_HL_5, MO_HL_13, MO_HL_4, MO_HL_12, MO_HL_15, MO_HL_17, MO_HL_6, MO_HL_2, MO_HL_8, MO_HL_7 et MO_HL_11 décrites dans le Tableau 1 est absent dans ledit échantillon ;
- au moins l'ensemble de 25 gènes bactériens de chacune des espèces bactériennes MO_HL_11, MO_HL_6, MO_HL_17, MO_HL_4, MO_HL_3, MO_HL_12, MO_HL_5, MO_HL_10, MO_HL_8, MO_HL_7, MO_HL_2 et MO_HL_13 décrites dans le Tableau 1 est absent, dans ledit échantillon ;
- au moins l'ensemble de 25 gènes bactériens de chacune des espèces bactériennes MO_HL_18, MO_HL_15, MO_HL_11, MO_HL_10, MO_HL_5, MO_HL_4, MO_HL_6, MO_HL_8, MO_HL_12, MO_HL_7, MO_HL_2, MO_HL_13 et MO_HL_3 décrites dans le Tableau 1 est absent dans ledit échantillon, ou ;
- au moins l'ensemble de 25 gènes bactériens de chacune des espèces bactériennes MO_HL_6, MO_HL_17, MO_HL_3, MO_HL_2, MO_HL_4, MO_HL_18, MO_HL_5, MO_HL_13, MO_HL_10, MO_HL_15, MO_HL_7, MO_HL_1, MO_HL_8 et MO_HL_12 décrites dans le Tableau 1 est absent dans ledit échantillon, ou ;
- au moins l'ensemble de 25 gènes bactériens de chacune des espèces bactériennes MO_HL_10, MO_HL_18, MO_HL_2, MO_HL_13, MO_HL_7, MO_HL_1, MO_HL_11, MO_HL_3, MO_HL_4, MO_HL_15, MO_HL_5, MO_HL_6, MO_HL_17, MO_HL_12 et MO_HL_8 décrites dans le Tableau 1 est absent, dans ledit échantillon ;
- au moins l'ensemble de 25 gènes bactériens de chacune des espèces bactériennes MO_HL_7, MO_HL_4, MO_HL_13, MO_HL_1, MO_HL_5, MO_HL_6, MO_HL_11, MO_HL_12, MO_HL_8, MO_HL_3, MO_HL_18, MO_HL_2, MO_HL_10, MO_HL_17, MO_HL_15 et MO_HL_9 décrites dans le Tableau 1 est absent dans ledit échantillon, ou ;
- au moins l'ensemble de 25 gènes bactériens de chacune des espèces bactériennes MO_HL_10, MO_HL_8, MO_HL_1, MO_HL_16, MO_HL_18, MO_HL_5, MO_HL_15, MO_HL_2, MO_HL_7, MO_HL_6, MO_HL_13, MO_HL_11, MO_HL_17, MO_HL_3, MO_HL_14, MO_HL_4 et MO_HL_12 décrites dans le Tableau 1 est absent, dans ledit échantillon ;
- au moins l'ensemble de 25 gènes bactériens de chacune des espèces bactériennes MO_HL_15, MO_HL_17, MO_HL_4, MO_HL_14, MO_HL_16, MO_HL_10, MO_HL_7, MO_HL_6, MO_HL_11, MO_HL_9, MO_HL_5, MO_HL_3, MO_HL_8, MO_HL_1, MO_HL_12, MO_HL_18, MO_HL_13 et MO_HL_2 décrites dans le Tableau 1 est absent, dans ledit échantillon.

6. Méthode selon l'une quelconque des revendications 1 à 5, **caractérisée en ce qu'**elle comprend la détection du nombre de copies d'au moins un desdits ensembles de 25 gènes bactériens desdites espèces bactériennes dans l'échantillon.

7. Méthode selon la revendication 6, **caractérisée en ce que** lesdits gènes bactériens sont choisis dans la liste constituée de la séquence SEQ ID 1 à la séquence SEQ ID 450.

8. Méthode selon l'une quelconque des revendications 1 à 7, **caractérisée en ce que** la présence ou l'absence des gènes bactériens selon l'invention est détectée à l'aide d'une micropuce d'acide nucléique qui est de préférence une micropuce d'oligonucléotide ou une micropuce d'ADNc.

9. Méthode selon la revendication 8, **caractérisée en ce que** la micropuce d'acide nucléique est une micropuce d'oligonucléotide comprenant au moins un oligonucléotide spécifique d'au moins chacun desdits 25 gènes bactériens ayant une séquence choisie parmi SEQ ID NOs 1-450.

10. Méthode selon l'une quelconque des revendications 1 à 9, **caractérisée en ce que** ledit échantillon d'ADN microbien intestinal provient d'un échantillon de selles.

11. Méthode permettant de déterminer si un sujet en surpoids est à risque de développer des co-morbidités liées à l'obésité choisies parmi le diabète de type II ; les cancers sauf le cancer de l'oesophage, du pancréas et de la prostate ; les maladies cardiovasculaires sauf l'insuffisance cardiaque congestive ; l'asthme ; les troubles vésiculaires ; l'arthrose ; et la douleur dorsale chronique, ladite méthode comprenant les étapes suivantes :
a) la détermination selon laquelle ledit sujet a une diversité bactérienne intestinale réduite à l'aide d'une méthode selon l'une quelconque des revendications 1 à 10 ;
b) si ledit sujet a une diversité bactérienne intestinale réduite, la détermination selon laquelle ledit sujet en surpoids est à risque de développer des co-morbidités liées à l'obésité.

12. Méthode permettant de déterminer si un sujet en surpoids a besoin d'un régime, ladite méthode comprenant les étapes suivantes :
a) la détermination selon laquelle ledit sujet a une diversité bactérienne intestinale réduite à l'aide d'une méthode l'une quelconque des revendications 1 à 10 ;
b) si ledit sujet a une diversité bactérienne intestinale réduite, la détermination selon laquelle ledit sujet a besoin d'un régime.

13. Méthode permettant de réduire les risques de développer des co-morbidités liées à l'obésité choisies parmi le diabète de type II ; les cancers sauf le cancer de l'oesophage, du pancréas et de la prostate ; les maladies cardiovasculaires sauf l'insuffisance cardiaque congestive ; l'asthme ; les troubles vésiculaires ; l'arthrose ; et la douleur dorsale chronique, ladite méthode comprenant les étapes suivantes :
a) la détermination selon laquelle ledit sujet a une diversité bactérienne intestinale réduite à l'aide d'une méthode selon l'une quelconque des revendications 1 à 10 ;
b) si ledit sujet a une diversité bactérienne intestinale réduite, la détermination selon laquelle ledit sujet a besoin d'un régime.

14. Méthode permettant de déterminer si un sujet en surpoids a besoin d'un traitement pour inflammation chronique, de préférence en plus de la mise en oeuvre d'un régime, comprenant les étapes suivantes :
a) la détermination selon laquelle ledit sujet a une diversité bactérienne intestinale réduite à l'aide d'une méthode selon l'une quelconque des revendications 1 à 10 ;
b) si ledit sujet a une diversité bactérienne intestinale réduite, la détermination selon laquelle ledit sujet a besoin d'un traitement pour inflammation chronique, de préférence en plus de la mise en oeuvre d'un régime.

15. Méthode permettant de surveiller l'efficacité d'un régime à accroître la richesse du microbiome bactérien intestinal chez un sujet en surpoids en ayant besoin, ladite méthode comprenant les étapes suivantes :
a) la détermination selon laquelle ledit sujet a une diversité bactérienne intestinale réduite à l'aide d'une méthode selon l'une quelconque des revendications 1 à 10 ;
b) la mise en oeuvre dudit régime ;
c) la détermination à partir d'un second échantillon d'ADN microbien intestinal provenant dudit sujet si ledit sujet a une diversité bactérienne intestinale réduite à l'aide d'une méthode selon l'une quelconque des revendications 1 à 10 ;
d) si ledit sujet a une diversité bactérienne intestinale réduite, la détermination selon laquelle le régime est efficace à accroître la richesse du microbiome bactérien intestinal chez ledit sujet.
